# EUROPÄISCHE PATENTSCHRIFT

(11) **EP 3 083 586 B1**
(45) Veröffentlichungstag und Bekanntmachung des Hinweises auf die Patenterteilung: **18.04.2018**
(21) Anmeldenummer: 14824414.8
(22) Anmeldetag: 16.12.2014
(51) Int. Cl.: C07D 401/12, C07D 401/14, C07D 417/12, C07D 409/12, A61K 31/4439, A61K 31/444, A61P 37/00, A61P 29/00

(54) **NEUE INDAZOLCARBOXAMIDE, VERFAHREN ZU IHRER HERSTELLUNG, PHARMAZEUTISCHE PRÄPARATE DIE DIESE ENTHALTEN, SOWIE DEREN VERWENDUNG ZUR HERSTELLUNG VON ARZNEIMITTELN**
NOVEL CARBOXAMIDES, METHOD FOR THE PRODUCTION THEREOF, PHARMACEUTICAL PREPARATIONS COMPRISING THEM, AND USE THEREOF FOR PRODUCING MEDICAMENTS
NOUVEAUX INDAZOLCARBOXAMIDES, LEUR PROCÉDÉ DE FABRICATION, PRÉPARATIONS PHARMACEUTIQUES QUI LES CONTIENNENT ET LEUR UTILISATION POUR LA PRÉPARATION DE MÉDICAMENTS

(30) Priorität: 19.12.2013 EP 13198463; 16.10.2014 EP 14189216
(43) Veröffentlichungstag der Anmeldung: 26.10.2016
(73) Patentinhaber: Bayer Pharma Aktiengesellschaft, 13353 Berlin (DE)
(72) Erfinder: BOTHE, Ulrich, 13187 Berlin (DE); SIEBENEICHER, Holger, 10557 Berlin (DE); SCHMIDT, Nicole, San Francisco, CA 94103 (US); ROTGERI, Andrea, 13503 Berlin (DE); BÖMER, Ulf, 16548 Glienicke (DE); RING, Sven, 07749 Jena (DE); IRLBACHER, Horst, 10405 Berlin (DE); GÜNTHER, Judith, 13187 Berlin (DE); STEUBER, Holger, 10115 Berlin (DE); LANGE, Martin, 10115 Berlin (DE); SCHÄFER, Martina, 13465 Berlin (DE)
(74) Vertreter: BIP Patents
(86) Internationale Anmeldenummer: PCT/EP2014/077877
(87) Internationale Veröffentlichungsnummer: WO 2015/091426

(56) Entgegenhaltungen:
- WO-A1-2009/019167
- WO-A1-2013/042137

## Beschreibung

Die vorliegende Anmeldung betrifft neue Indazolcarboxamide, Verfahren zu ihrer Herstellung, Indazolcarboxamide zur Verwendung zur Behandlung und/oder Prophylaxe von Krankheiten sowie ihre Verwendung zur Herstellung von Arzneimitteln zur Behandlung und/oder Prophylaxe von Krankheiten, insbesondere von proliferativen Erkrankungen, von Autoimmun- und inflammatorischen Erkrankungen wie z.B. Rheumatoider Arthritis, Chronisch obstruktiver Lungenerkrankung (englisch chronic obstructive pulmonary disease, Abkürzung: COPD), Multipler Sklerose, Endometriose und entzündungsinduziertem oder chronischem Schmerz sowie von Lymphomen.

IRAK4 spielt eine Schlüsselrolle bei der Aktivierung des Immunsystems, insbesondere bei der angeborenen Immunität. Die angeborene Immunität beruht darauf, dass Mikroorganismen wie Bakterien und Viren bestimmte inhärente Merkmale aufweisen, durch die sie vom Immunsystem erkannt werden und dieses aktivieren. Erkannt werden bestimmte Pathogen-assoziierte Muster (pathogen associated molecular pattern, PAMPs). PAMPs werden von den "Pattern recognition receptors" (PRR) erkannt, zu denen auch Toll-Like Rezeptoren (TLR) gehören (Janeway und Medzhitov, Annu. Rev. Immunol., 2002). Vom Menschen sind zehn verschiedene TLRs bekannt. TLR1 und TLR6 sind Korezeptoren für TLR2. TLR2 erkennt u.a. Lipoproteine und Lipopeptide. TLR3 erkennt doppelsträngige RNA. TLR4 erkennt u.a. LPS (Lipopolysaccharide) von gramnegativen und Lipoteichonsäure von gram-positiven Bakterien. TLR5 erkennt Flagellin. CpG-Motive in bakterieller DNA werden von TLR9 erkannt (Miggin, O'Neill, J. Leukoc. Biol., 2006). Zusätzliche Moleküle können die Erkennungsfähigkeiten von TLRs weiter verändern (Akashi-Takamura and Miyake, Current Opinion in Immunology, 2008). Neben der Erkennung von PAMPs können auch sogenannte DAMPs (damage-associated molecular pattern) durch TLRs erkannt werden. Hierbei handelt es sich um körpereigene, von Zellen stammende Moleküle, die infolge eines Traumas, einer Ischämie oder anderen gewebezerstörenden Prozessen in Abwesenheit einer offensichtlichen Infektion entstehen. DAMPs können Bestandteile des Zytoplasmas als auch des Zellkerns sein. Sie werden sekretiert, wie z.B. HMGB1 (high-mobility group box 1 protein), welches durch TLR2 und TLR4 erkannt wird. Andere DAMPs werden de novo freigesetzt oder reichern sich beispielsweise in der äußeren Plasmamembran an, z.B. HSP90 (heat shock protein 90), wo sie durch TLR2 und TLR4 erkannt werden. Andere werden wiederum als Endabbauprodukte während des Zelltodes produziert (Krysko, Garg, et al., Nat Rev Cancer, 2012). Neben den TLRs spielen auch andere Komponenten wie z.B. Zytokine eine wichtige Rolle bei der angeborenen Immunität. Hierbei sei insbesondere die Interleukin (IL)-1 Familie mit den Interleukinen IL-1, IL-18 und IL-33 zu erwähnen. Sie werden von unterschiedlichen Immunzellen in Gegenwart von Infektionen oder Zell-und Gewebestress produziert und freigesetzt. Durch die Bindung an den jeweiligen Rezeptor kommt es dann zur Immunantwort (Dinarello, Annu. Rev. Immunol., 2009).
TLRs (außer TLR3) als auch die Rezeptoren der IL-1 Familie (IL-1R (Rezeptor), IL-18R und IL-33R) weisen dieselbe Signalkaskade auf, welche durch die Bindung des jeweiligen Liganden an seinen Rezeptor aktiviert wird. Die Liganden-Rezeptor-Bindung führt hierbei zur Rekrutierung des Adaptormoleküls MyD88 [Myeloid differentiation primary response gene (88)] zum Rezeptor durch eine TIR/TIR Domänen-Interaktion, die Bestandteil der Rezeptoren als auch von MyD88 ist. Neben der sogenannten TIR Domäne besitzt MyD88 eine N-terminale "death domain"(DD), die in Interaktion mit der DD Domäne der Interleukin-1 Receptor Associated Kinase-4 (IRAK4) tritt. IRAK4 zählt zu einer Serin/ Threonin Kinase Familie, zu welcher auch die strukturähnlichen Kinasen IRAK1, IRAK2 und IRAK-M zählen (Cao et al., Science, 1996; Muzio et al., Science, 1997; Wesche, Gao, et al., Journal of Biological Chemistry, 1999; Li, Strelow, et al., PNAS, 2002). Mit Ausnahme von IRAK-M, welches nur in Monozyten und Makrophagen exprimiert wird, werden IRAK4, IRAK1 und IRAK2 ubiquitär exprimiert (Flannery and Bowie, Biochemical Pharmacology, 2010). Infolge des Aktivierungsprozesses bildet sich aus mehreren MyD88- und IRAK4 Molekülen ein Multikomplex, der als "myddosome" bezeichnet wird (Precious et al., J. Biol. Chem., 2009). Dieses "myddosome" interagiert nun mit IRAK1 oder IRAK2 über DD-DD Interaktionen und bildet hierbei einen größeren Komplex (Lin, Lo, et al., Nature, 2010). Die Entstehung dieses Komplexes löst nun die Autophoshorylierung von IRAK4 aus, was im Folgenden zur Phosphorylierung von IRAK1 oder IRAK2 führt. Infolge der Aktivierung von IRAK1 oder IRAK2 findet eine Autophosphorylierung dieser Kinasen statt (Kollewe, Mackensen, et al., Journal of Biological Chemistry, 2004). Das aktivierte IRAK1 oder IRAK2 interagiert mit TRAF6 (tumor-necrosis factor-receptor-associated factor 6), welches mit dem Ubiquitin-Enzym-Komplex (E2) als Ubiquitin-Protein-Ligase fungiert, wobei es zu einer K62-verknüpften Ubiquitinierung von TRAF6 kommt. Dieser Prozess führt dann wiederum mit anderen Proteinen zu einer weiteren Komplexbildung. Dieser Komplex induziert die Aktivierung von TAK1 (Xia, Sun, et al., Nature, 2009). Aktiviertes TAK1 vermittelt die Aktivierung des NF (nuclear factor)-kB Signalweges und des MAPK (Mitogen-activated protein kinase) Signalweges (Wang, Deng, et al., Nature, 2001). Beim erstgenannten Signalweg, führt TAK1 zur Aktivierung des IKK Komplexes, wodurch das inhibierende IkB Protein phosphoryliert und durch das Proteasom abgebaut wird. NF-kB, welches zuvor durch IkB blockiert wurde, wandert nun vom Zytoplasma in den Zellkern, wo es an ein spezifisches DNA-Motiv, dem kB Motiv, bindet und zur Transkription von unterschiedlichen Genen führt (Gasparini and Feldmann, Curr Pharm Des, 2012).
Beim MAPK Signalweg phosphoryliert TAK1 verschiedene Mitglieder der MAPK Familie wie MKK3, -4, -6 und -7 (Wang, Deng, et al., Nature, 2001). Die Aktivierung dieser Kinasen führt zur Aktivierung von p38 und JNK (c-Jun N-terminal kinase) (Ono and Han, Cellular Signalling, 2000; Davis, Cell, 2000). Die Aktivierung des NF-kB Signalweges als auch des MAPK Signalweges führen zu unterschiedlichen Prozessen, die mit verschiedenen Immunprozessen assoziiert sind. So kommt es zu einer erhöhten Expression von unterschiedlichen inflammatorischen Signalmolekülen und Enzymen, wie z.B. Zytokine, Chemokine und COX-2, und zu einer erhöhten mRNA Stabilität von bestimmten Genen (Holtmann, Enninga, et al., Journal of Biological Chemistry, 2001; Datta, Novotny, et al., The Journal of Immunology, 2004). Des Weiteren können diese Prozesse mit der Proliferation und der Differenzierung von bestimmten Zelltypen einhergehen (Wan, Chi, et al., Nat Immunol, 2006; McGettrick and J. O'Neill, British Journal of Haematology, 2007).

Die zentrale Bedeutung von IRAK4 bei TLR- (außer TLR3) und IL-1 Rezeptorfamilie-vermittelten immunologischen Prozessen zeigt sich bei der Deletion von IRAK4. Zellen, die von Patienten isoliert wurden, bei denen ein Nichtvorhandensein von IRAK4 nachgewiesen wurde, zeigen keine Aktivität nach Stimulation verschiedener TLRs (außer TLR3) und der IL-1β Familie (Davidson, Currie, et al., The Journal of Immunology, 2006; Ku, von Bernuth, et al., JEM, 2007). Des Weiteren entwickeln Mäuse mit einer IRAK4 Deletion keine Antwort auf eine IL-1β-Stimulation und unterschiedliche TLR Stimulationen mit Ausnahme von TLR3 (Suzuki, Suzuki, et al., Nature, 2002). Hierbei spielt insbesondere die Kinaseaktivität von IRAK4 eine entscheidende Rolle (Kim, Staschke, et al., JEM, 2007). Im Gegensatz hierzu führt die Deletion von IRAK1 oder IRAK2 nur zu einem Aktivitätsverlust des Signalweges nach Stimulation (Thomas, Allen, et al., The Journal of Immunology, 1999; Swantek, Tsen, et al., The Journal of Immunology, 2000; Kawagoe, Sato, et al., Nat Immunol, 2008). Mäuse, die eine Deletion von IRAK2 und IRAK1 aufweisen, zeigen wiederum einen vergleichbaren Phänotyp wie Tiere mit einer IRAK4 Deletion (Kawagoe, Sato, et al., Nat Immunol, 2008). Die zentrale Rolle von IRAK4 in der Pathologie von unterschiedlichen inflammatorischen Erkrankungen, welche mit dem beschriebenen Signalweg assoziiert sind, konnte bereits durch den direkten Vergleich von Wildtyp (WT) Mäusen mit genetisch veränderten Tieren mit einer Kinase-inaktiven Form des IRAK4 (IRAK4 KDKI) gezeigt werden. IRAK4 KDKI Tiere weisen ein verbessertes Krankheitsbild im Tiermodell für multiple Sklerose, Atherosklerose, Herzinfarkt und Alzheimer auf (Rekhter, Staschke, et al., Biochemical and Biophysical Research Communication, 2008; Maekawa, Mizue, et al., Circulation, 2009; Staschke, Dong, et al., The Journal of Immunology, 2009; Kim, Febbraio, et al., The Journal of Immunology, 2011; Cameron, Tse, et al., The Journal of Neuroscience, 2012). Des Weiteren zeigte sich, dass die Deletion von IRAK4 im Tiermodell vor einer viral-induzierten Myokarditis infolge einer verbesserten antiviralen Reaktion bei gleichzeitig verringerter systemischer Inflammation schützt (Valaperti, Nishii, et al., Circulation, 2013).

Durch die zentrale Rolle von IRAK4 in der MyD88-vermittelten Signalkaskade von TLRs (außer TLR3) und der IL-1 Rezeptorfamilie kann die Inhibition von IRAK4 zur Prophylaxe und/oder Behandlung von durch die genannten Rezeptoren vermittelte Erkrankungen genutzt werden. TLRabhängige Prozesse sind mit einer Vielzahl unterschiedlicher Erkrankungen assoziiert. So hat es sich gezeigt, dass TLRs in der Pathogenese der Multiplen Sklerose, Rheumatoiden Arthritis, des Metabolischen Syndroms, der Diabetes, der Osteoarthritis, des Sjögren-Syndroms und der Sepsis involviert sind (Scanzello, Plaas, et al. Curr Opin Rheumatol, 2008; Roger, Froidevaux, et al, PNAS, 2009; Gambuzza, Licata, et al., Journal of Neuroimmunology, 2011; Fresno, Archives Of Physiology And Biochemistry, 2011; Goh and Midwood, Rheumatology, 2012; Dasu, Ramirez, et al., Clinical Science, 2012; Ramirez and Dasu, Curr Diabetes Rev, 2012; Li, Wang, et al., Pharmacology & Therapeutics, 2013). Hauterkrankungen wie Psoriasis, atopische Dermatitis, Acne inversa und Acne vulgaris sind mit dem IRAK4-vermittelten TLR Signalweg assoziiert.
Die genannten Erkrankungen sind durch eine erhöhte Expression bestimmter TLRs charakterisiert und deren pathologische Immunreaktionen werden durch bestimmte TLR-assoziierte Inflammationsprozesse vermittelt (Gilliet, Conrad, et al., Archives of Dermatology, 2004; Niebuhr, Langnickel, et al., Allergy, 2008; Miller, Adv Dermatol, 2008; Terhorst, Kalali, et al., Am J Clin Dermatol, 2010; Dispenza, Wolpert, et al., J Invest Dermatol, 2012; Selway, Kurczab, et al., BMC Dermatology, 2013; Wollina, Koch, et al. Indian Dermatol Online, 2013).
Auch bei pulmonalen Erkrankungen wie Lungenfibrose, obstruktiver Lungenerkrankung (COPD), akutem Atemnot-Syndrom (ARDS), akuter Lungenschädigung (ALI), interstitieller Lungenerkrankung (ILD), Sarkoidose und pulmonaler Hypertonie zeigt sich eine Assoziation mit verschiedenen TLR-vermittelten Signalwegen. Bei der Pathogenese der pulmonalen Erkrankungen kann es sich sowohl um infektiös vermittelte als auch um nicht-infektiös vermittelte Prozesse handeln (Ramirez Cruz, Maldonado Bernal, et al., Rev Alerg Mex, 2004; Jeyaseelan, Chu, et al., Infection and Immunity, 2005; Seki, Tasaka, et al., Inflammation Research, 2010; Xiang, Fan, et al., Mediators of Inflammation, 2010; Margaritopoulos, Antoniou, et al., Fibrogenesis & Tissue Repair, 2010; Hilberath, Carlo, et al., The FASEB Journal, 2011; Nadigel, Prefontaine, et al., Respiratory Research, 2011; Kovach and Standiford, International Immunopharmacology, 2011; Bauer, Shapiro, et al., Mol Med, 2012; Deng, Yang, et al., PLoS One, 2013; Freeman, Martinez, et al., Respiratory Research, 2013; Dubaniewicz, A., Human Immunology, 2013). Beispielsweise ist HMGB1 (high-mobility group box 1 protein) - ein endogener Ligand von TLR2 und TLR4 - bei Patienten mit Lungenfibrose erhöht. Die Blockierung dieser TLR-Signalwege führt zu einer verringerten Entzündung im Tiermodell (Yang, Cui, et al., The Journal of Immunology, 2009; Entezari, Weiss, et al., Mol Med, 2012). Die Beteiligung TLR2-vermittelter Prozesse in der Pathogenese von Sarkoidose wurde erst kürzlich mittels in vitro und in vivo Untersuchungen aufgezeigt (Chen, Song, et al., American Journal of Respiratory and Critical Care Medicine, 2010; Gabrilovich, Walrath, et al., Clinical & Experimental Immunology, 2013).
TLRs sind auch in die Pathogenese anderer inflammatorischer Erkrankungen wie Behçet-Krankheit, Gicht und Transplantatabstoßung involviert, so dass die Inhibition von IRAK4 hier ein geeigneter therapeutischer Ansatz ist (Liu-Bryan, Scott, et al., Arthritis & Rheumatism, 2005; Shi, Mucsi, et al., Immunological Reviews, 2010; Leventhal and Schroppel, Kidney Int, 2012; Kreisel and Goldstein, Transplant International, 2013; Li, Wang, et al., Pharmacology & Therapeutics, 2013). Auch Läsionen und peritoneale Makrophagen von Endometriose-Patientinnen weisen im Vergleich zu gesunden Probanden eine verstärkte Immunantwort infolge einer LPS-(Lipopolysaccharide) Stimulation auf (Allhorn, Boing, et al., Reproductive Biology and Endocrinology, 2008; Khan, Kitajima, et al., Journal of Obstetrics and Gynaecology Research, 2013).
Patienten mit Lupus erythematodes und Adult Morbus Still-Krankheit weisen eine erhöhte Expression von TLR7, MyD88 und IRAK4 auf (Chen, Lin, et al., Arthritis Res Ther, 2013). Die Inhibition von TLR7, 8 und 9 als auch die Verwendung von Tieren mit einer Deletion von TLR7 und/oder TLR9 führen im Krankheitsmodell für Lupus zu einer verbesserten Pathogenese (Christensen, Shupe, et al., Immunity, 2006; Nickerson, Christensen, et al., The Journal of Immunology, 2010; Zhu, Jiang, et al., Autoimmunity, 2013). Patienten mit chronisch entzündlicher Darmerkrankung, wie Kolitis ulcerosa und Morbus Crohn, weisen nicht nur Polymorphismen in verschiedenen TLR Genen auf. In verschiedenen Tiermodellen konnten auch gezeigt werden, dass bestimmte TLRs in die Pathogenese dieser Darmerkrankungen involviert sind (Rakoff-Nahoum, Hao, et al., Immunity, 2006; Heimesaat, Fischer, et al., PLoS ONE, 2007; Cario, Inflammatory Bowel Diseases, 2010; Walsh, Carthy, et al., Cytokine & Growth Factor Reviews, 2013).
Neben den bereits aufgeführten Erkrankungen werden IRAK4-vermittelte TLR Prozesse in der Pathogenese von Augenerkrankungen wie Keratitis, allergisch bedingte Konjunktivitis, Keratoconjunctivitis sicca, Makuladegeneration und Uveitis beschrieben (Kaarniranta and Salminen, J Mol Med (Berl), 2009; Sun and Pearlman, Investigative Ophthalmology & Visual Science, 2009; Redfern and McDermott, Experimental Eye Research, 2010; Kezic, Taylor, et al., J Leukoc Biol, 2011; Chang, McCluskey, et al., Clinical & Experimental Ophthalmology, 2012; Guo, Gao, et al., Immunol Cell Biol, 2012; Lee, Hattori, et al., Investigative Ophthalmology & Visual Science, 2012).

Die Funktion von TLRs in Arteriosklerose wurde nicht nur durch die Analyse von humanen Proben sondern auch mit Hilfe verschiedener Tiermodelle untermauert (Seneviratne, Sivagurunathan, et al., Clinica Chimica Acta, 2012; Falck-Hansen, Kassiteridi, et al., International Journal of Molecular Sciences, 2013).
Aufgrund der zentralen Rolle von IRAK4 in TLR-vermittelten Prozessen wird durch die Inhibition von IRAK4 auch die Behandlung und/oder Prävention von kardiovaskulären und neurologischen Erkrankungen wie z.B. myokardialem Reperfusionsschaden, Myokardinfarkt, Hypertonie (Oyama, Blais, et al., Circulation, 2004; Timmers, Sluijter, et al., Circulation Research, 2008; Fang and Hu, Med Sci Monit, 2011; Bijani, International Reviews of Immunology, 2012; Bomfim, Dos Santos, et al., Clin Sci (Lond), 2012; Christia and Frangogiannis, European Journal of Clinical Investigation, 2013; Thompson and Webb, Clin Sci (Lond), 2013) sowie Alzheimer, Schlaganfall und Parkinson möglich (Carty and Bowie, Biochemical Pharmacology, 2011; Lim, Kou, et al., The American Journal of Pathology, 2011; Beraud and Maguire-Zeiss, Parkinsonism & Related Disorders, 2012; Noelker, Morel, et al., Sci. Rep., 2013; Wang, Wang, et al., Stroke, 2013). Neuronen wie auch Mikroglia und Astrocyten exprimieren einen Großteil der bekannten TLRs.
Die Deletion von TLR7 im Tiermodell schützt vor unterschiedlichen Auslöser für Juckreiz (Nicotra, Loram, et al., Experimental Neurology, 2012; Liu and Ji, Pflugers Arch., 2013). Neben der Rolle von TLRs bei Juckreiz, konnte auch die Involvierung in Schmerzprozesse durch verschiedene Tiermodelle gezeigt werden, (Kim, Lee, et al., Toll-like Receptors: Roles in Infection and Neuropathology, 2009; Guerrero, Cunha, et al., European Journal of Pharmacology, 2012; Nicotra, Loram, et al., Experimental Neurology, 2012; David, Ratnayake, et al., Neurobiology of Disease, 2013). Untersuchungen bei Schmerzpatienten untermauern diese Erkenntnisse (Kwok, Hutchinson, et al., PLoS ONE, 2012; Chopra and Cooper, J Neuroimmune Pharmacol, 2013).

Aufgrund der Vermittlung der TLR Signale über IRAK4 ist von einer therapeutischen Wirkung in den genannten Indikationen durch die Inhibierung von IRAK4 auszugehen.

Dies gilt auch für einige onkologischen Erkrankungen. Bestimmte Lymphome weisen eine aktivierende MyD88-Mutation auf, die durch einen IRAK4 Inhibitor behandelt werden kann (Ngo, Young, et al., Nature, 2011; Treon, Xu, et al., New England Journal of Medicine, 2012; Choi, Kim, et al., Human Pathology, 2013). Auch chronisch lymphatische Leukämie, Melanome und Leberzellkarzinom sind mit Mutationen in MyD88 oder Veränderungen in der MyD88-Aktivität assoziiert (Puente, Pinyol, et al., Nature, 2011; Srivastava, Geng, et al., Cancer Research, 2012; Liang, Chen, et al., Clinical Cancer Research, 2013). Des Weiteren spielt MyD88 eine wichtige Rolle in Ras-abhängigen Tumore, so dass IRAK4 Inhibitoren auch zu deren Behandlung geeignet sind (Kfoury, A., K. L. Corf, et al., Journal of the National Cancer Institute, 2013).

Neben der Vermittlung von MyD88- und TLR- (außer TLR3)-assoziierten Prozessen vermittelt IRAK4 auch die Signale der IL-1 Rezeptorfamilie. Inflammatorische Erkrankungen wie CAPS (Cryopyrin-assoziierte periodische Syndrome) inklusive FCAS (familiäre Kälteurtikaria), MWS (Muckle-Wells-Syndrom), NOMID- (neonatal-onset multisystem inflammatory disease) und CONCA- (chronic infantile, neurological, cutaneous, and articular) Syndrom; FMF (familiäres Mittelmeerfieber), HIDS (Hyper-IgD-Syndrom), TRAPS (Tumornekrosefaktor-Rezeptor 1-assoziiertes periodisches Syndrom), juvenile idiopathische Arthritis, Adult Morbus Still-Krankheit, Morbus Adamantiades-Behçet, rheumatoide Arthritis, Osteoarthritis, Keratoconjunctivitis sicca und Sjögren Syndrome werden durch die Blockierung des IL-1 Signalweges behandelt, so dass auch hier ein IRAK4 Inhibitor zur Behandlung der genannten Krankheiten geeignet ist (Narayanan, Corrales, et al., Cornea, 2008; Henderson and Goldbach-Mansky, Clinical Immunology, 2010; Dinarello, European Journal of Immunology, 2011; Gul, Tugal-Tutkun, et al., Ann Rheum Dis, 2012; Pettersson, Annals of MedicinePetterson, 2012; Ruperto, Brunner, et al., New England Journal of Medicine, 2012; Nordström, Knight, et al., The Journal of Rheumatology, 2012; Vijmasi, Chen, et al., Mol Vis, 2013; Yamada, Arakaki, et al., Opinion on Therapeutic Targets, 2013). Insbesondere IL-18 ist mit der Pathogenese von rheumatoider Arthritis, der Adult Morbus Still-Krankheit, Typ-1-Diabetes, Multipler Sklerose und Lupus erythematodes assoziiert, so dass aufgrund des Wirkmechanismus IRAK4 Inhibitoren zur Behandlung und/oder Prävention der genannten Erkrankungen eingesetzt werden können (Volin and Koch, J Interferon Cytokine Res, 2011; Sedimbi, Hagglof, et al., Cell Mol Life Sci, 2013; Yap and Lai, Nephrology, 2013). Weiterhin eignen sich IRAK4 Inhibitoren zur Behandlung von Typ-2-Diabetes und den Folgen eines Myokardinfarktes, da es Hinweise gibt, dass die Inhibierung des IL-1-Signalweges ein erfolgreicher Therapieansatz ist (Abbate, Kontos, et al., The American Journal of Cardiology, 2010; Akash, Shen, et al., Journal of Pharmaceutical Sciences, 2012; Abbate, Van Tassell, et al., The American Journal of Cardiology, 2013). Mehrere Komponenten der IL-1 Rezeptorfamilie sind mit unterschiedlichen pulmonalen Erkrankungen wie Asthma, COPD, idiopathischer interstitieller Pneumonie und akutem Atemnot-Syndrom (ARDS) assoziiert und die Rolle in dessen Pathogenese konnte durch verschiedene Tiermodelle untermauert werden (Kang, Homer, et al., The Journal of Immunology, 2007; Imaoka, Hoshino, et al., European Respiratory Journal, 2008; Couillin, Vasseur, et al., The Journal of Immunology, 2009; Lloyd, Current Opinion in Immunology, 2010; Pauwels, Bracke, et al., European Respiratory Journal, 2011; Yin, Li, et al., Clinical & Experimental Immunology, 2012; Alexander-Brett, et al., The Journal of Clinical Investigation, 2013; Bunting, Shadie, et al., BioMed Research International, 2013; Byers, Alexander-Brett, et al., The Journal of Clinical Investigation, 2013; Kawayama, Okamoto, et al., J Interferon Cytokine Res, 2013; Martinez-Gonzälez, Roca, et al., American Journal of Respiratory Cell and Molecular Biology, 2013; Qiu, Li, et al., Immunology, 2013).

Des Weiteren haben unterschiedliche Studien gezeigt, dass ein Zusammenhang zwischen der Menge an IL-1 β und dessen Rezeptor, IL-18 und IL-33, und der Erkrankung Endometriose besteht (Akoum, Lawson, et al., Human Reproduction, 2007; Lawson, Bourcier, et al., Journal of Reproductive Immunology, 2008; Sikora, Mielczarek-Palacz, et al., American Journal of Reproductive Immunology; Santulli, Borghese, et al., Human Reproduction, 2013). Außerdem konnte im Tiermodell das Wachstum von humanem endometrialem Gewebe durch die Gabe des endogenen IL-1β Inhibitors, IL-1R2, blockiert werden (Khoufache, Bondza, et al., The American Journal of Pathology, 2012). Durch den Wirkmechanismus ist ein IRAK4 Inhibitor auch hier effektiv. Chronisch-entzündliche Darmerkrankungen wie Morbus Crohn und Kolitis ulcerosa sind mit einer Dysregulation der IL-1 Rezeptorfamilie assoziiert (Kobori, Yagi, et al., J Gastroenterol, 2010; Hao, Liu, et al., Curr Opin Gastroenterol, 2013). Neben den genannten Indikationen sind IRAK4 Inhibitoren auch zur Behandlung und/oder Prävention von IL-1 Rezeptorfamilie-vermittelten neurologischen Erkrankungen wie Hirnschlag, Alzheimer, Schlaganfall, Schädel-Hirn-Trauma und Schmerz wie Krebsschmerzen, postoperativer Schmerz, entzündungsinduzierter und chronischer Schmerz geeignet (Wolf, Livshits, et al., Brain, Behavior, and Immunity, 2008; Brough, Tyrrell, et al., Trends in Pharmacological, 2011; SciencesDenes, Kitazawa, Cheng, et al., The Journal of Immunology, 2011; Pinteaux, et al., Cerebrovascular Diseases, 2011; del Rey, Apkarian, et al., Annals of the New York Academy of Sciences, 2012; Denes, Wilkinson, et al., Disease Models & Mechanisms, 2013; Han, Zhao, et al., Neuroscience, 2013; Zhao, Zhang, et al., Neuroscience, 2013). Aufgrund der Weiterleitung von IL1 Rezeptorfamilie-vermittelter Prozesse durch IRAK4 wirken IRAK4 Inhibitoren in dermatologischen Erkrankungen wie Psoriasis, atopischer Dermatitis und allergischer Kontaktdermatitis. Die IL1 Rezeptorfamilie ist in die Pathogenese der genannten Erkrankungen involviert (Viguier, Guigue, et al., Annals of Internal Medicine, 2010; Cevikbas, Steinhoff, J Invest Dermatol, 2012; Minkis, Aksentijevich, et al., Archives of Dermatology, 2012; Mattii, Ayala, et al., Experimental Dermatology, 2013; Sedimbi, Hagglof, et al., Cell Mol Life Sci, 2013).

Die Assoziation von IRAK4 mit einer Vielzahl unterschiedlicher Erkrankungen durch die Vermittlung unterschiedlicher Signale via TLRs (außer TLR3) und der IL1 Rezeptorfamilie zeigt, dass durch die Inhibition von IRAK4 eine Vielzahl von Erkrankungen positiv beeinflusst werden können.

Die in der vorliegenden Erfindung beschriebenen Verbindungen sind nun in der Lage, IRAK 4 zu inhibieren. Dies wird auch dadurch belegt, dass die erfindungsgemäßen Verbindungen in TLR- und IL1-vermittelten Prozessen eine hemmende Wirkung ausüben.

Aufgabe der vorliegenden Erfindung ist somit die Bereitstellung neuer Verbindungen, welche in der oben beschriebenen Weise als Inhibitoren der Interleukin-1 Receptor Associated Kinase-4 (IRAK4) wirken. Die neuen IRAK4-Inhibitoren sollen insbesondere zur Behandlung und zur Prävention von proliferativen und entzündlichen Erkrankungen geeignet sein, die durch ein überreagierendes Immunsystem charakterisiert sind. Besonders genannt seien hier entzündlichen Hauterkrankungen, Herz-Kreislauf-Erkrankungen, Lungenerkrankungen, Augenerkrankungen, Autoimmunerkrankungen und Krebs-Erkrankungen.

Aus dem Stand der Technik sind eine Vielzahl von IRAK4 Inhibitoren bekannt. IRAK4 Inhibitoren werden zum Beispiel beschrieben in G. C. Harriman et al. in US20130231328 sowie in L. D. Romero et al. US20120283238.
IRAK4 Modulatoren basierend auf einem Pyrazol[1,5a]pyrimidin-Gerüst werden von N. Arora et al. in US20120015962 beschrieben.
Außerdem wird von V. R. Paidi et al. in WO2013106641 über Thiazolyl- oder Thiadiazolylsubstituierte Pyridinderivate und von S. D. Dodd et al. in WO2013106614 über Triazolylsubstituierte Pyridinderivate berichtet. Weitere Pyridinderivate findet man in WO2013106612.
Aminopyrimidone, die als IRAK4 Inhibitoren wirken, werden von W. M. Seganish et al. in WO2013066729 beschrieben, zusätzlich werden von W. T. Mcelroy et al in WO 2012129258 Amidopyrazole ebenfalls als IRAK Inhibitoren beschrieben.
Von G. Buckeley et al. wird in Bioorg. Med. Chem. Lett. 18 (2008), 3291 - 3295 sowie in Bioorg. Med. Chem. Lett. 18 (2008), 3656 - 3660 jeweils über Imidazol[1,2-a]pyridine berichtet. Weiterhin wird von A. D. Frenkel et al. in US20070037803 über Benzimidazolderivate als IRAK4 Inhibitoren berichtet.

Weitere IRAK Inhibitoren mit 2-Aminoimidazol- sowie mit 2-Aminobenzimididazolstruktur werden von A. D. Frenkel et al. in US2007/0037803 beansprucht.

IRAK4 Inhibitoren, die wie die erfindungsgemäßen Verbindungen auf einer Indazolstruktur basieren, werden von K. Guckian et al. in US8293923 beschrieben. Diese Indazolderivate weisen eine Substitution an der Position 3 des Indazols mit einer Benzimidazol-2-ylamino-Gruppe auf. US8293923 offenbart keine 2-substituierten Indazole.
Über weitere IRAK4 Inhibitoren basierend auf einer Indazolstruktur wird von C. Jorand-Lebrun et al. in US20130274241 berichtet. Dabei handelt es sich um Indazolderivate mit einem Triazolhaltigen Substituenten an Position 3 des Indazols. US20130274241 beschreibt keine 2-Substitution der offenbarten Indazole.

In WO2011043371 werden Oxazolcarboxamide verknüpft mit monocyclischen aromatischen Heterocyclen als IRAK4 Inhibitoren beschrieben. Oxazolcarboxamide, verknüpft mit einer Indazolstruktur wie sie die erfindungsgemäßen Verbindungen aufweisen, werden in WO2011043371 nicht beschrieben.
Bicyclische Heterocyclen mit einer Carboxamid-Struktur als IRAK4 Inhibitoren wie zum Beispiel die Substanz **L1** werden von B. Anima et al. in WO2013042137 beschrieben. Es werden jedoch nur Benzimidazol-, Benzoxazol- sowie Benzothiazolderivate, jedoch keine Indazolderivate, beschrieben.

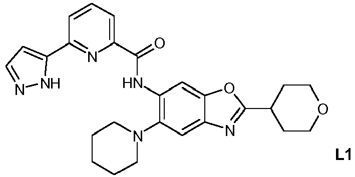

G. M. Buckley et al. berichten in Bioorg. Med. Chem. Lett. 18 (2008), 3211 - 3214 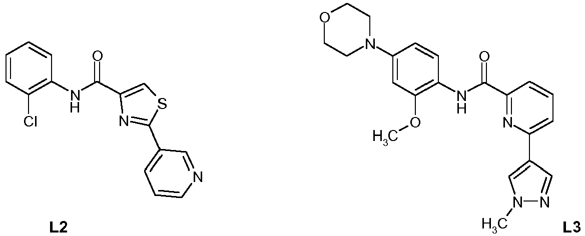 über Carboxamidderivate als IRAK4 Inhibitoren. Beispielsweise werden die Moleküle **L2** und **L3** beschrieben. Es werden keine Indazolderivate beschrieben.

In WO2009019167 werden von A. Bombrun et al. 6-Amino-pyrimidin-4-carboxamide mit einer 2-substituierten Indazolstruktur wie zum Beispiel **L4** beschrieben. Es wird berichtet, dass die beschriebenen Substanzen an den Sphingosin-1-phosphat Rezeptors binden. Eine hemmende Wirkung auf die IRAK4 Kinase wird in WO2009019167 nicht beschrieben.

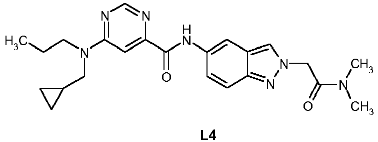

In US20080058341 werden Azaindazole mit Carboxamid-Struktur als CCR1 Antagonisten beschrieben. 2-substituierte Indazolderivate mit zusätzlicher Carboxamid-Struktur werden nicht offenbart.
Von A. J. Souers et al. werden in US20050137187 2-substituierte Indazole als Antagonisten von MCH (melanin-concentrating hormone) beschrieben. Der 2-Substituent am Indazol beinhaltet jedoch keine Carboxamid-Struktur.

Gegenstand der vorliegenden Erfindung sind Verbindungen der allgemeinen Formel (I) 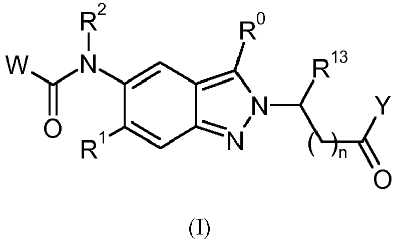 in der:
- R⁰: für Wasserstoff oder C₁-C₄-Alkyl steht, wobei der C₁-C₄-Alkylrest gegebenenfalls ein- oder mehrfach, gleich oder verschieden mit Hydroxy oder Halogen substituiert sein kann;
- R¹: für Wasserstoff, Halogen, Cyano, C(=O)OH, C(=O)OR^{a}, C(=O)NH₂, C(=O)N(H)R^{a}, C(=O)N(R^{a})R^{b}, C(=O)R^{d}, Hydroxy oder C₁-C₆-Alkyl steht, wobei der C₁-C₆-Alkylrest gegebenenfalls ein- oder mehrfach, gleich oder verschieden substituiert sein kann mit
Hydroxy, Halogen, Cyano, C(=O)OH, C(=O)OR^{a}, S(=O)₂-C₁-C₆-Alkyl, NH₂, NHR^{a}, N(R^{a})R^{b}, einem gegebenenfalls ein- oder mehrfach, gleich oder verschieden mit Halogen substituiertem C₁-C₆-Alkoxy, einem gegebenenfalls ein- oder mehrfach, gleich oder verschieden mit Halogen substituiertem C₃-C₈-Cycloalkoxy, einem ein- oder mehrfach, gleich oder verschieden mit R^{c} substituiertem Heterocycloalkyl,
oder für C₁-C₆-Alkoxy steht, wobei der C₁-C₆-Alkoxyrest gegebenenfalls ein- oder mehrfach, gleich oder verschieden substituiert sein kann mit
Hydroxy, Halogen, Cyano, C(=O)OH, C(=O)OR^{a}, S(=O)₂-C₁-C₆-Alkyl, NH₂, NHR^{a}, N(R^{a})R^{b}, einem gegebenenfalls ein- oder mehrfach, gleich oder verschieden mit Halogen substituiertem C₃-C₈-Cycloalkyl, einem gegebenenfalls ein- oder mehrfach, gleich oder verschieden mit Halogen substituiertem C₁-C₆-Alkoxy, einem gegebenenfalls ein- oder mehrfach, gleich oder verschieden mit Halogen substituiertem C₃-C₈-Cycloalkoxy, einem gegebenenfalls ein- oder mehrfach, gleich oder verschieden mit R^{c} substituiertem Heterocycloalkyl, einem gegebenenfalls ein- oder mehrfach, gleich oder verschieden mit R^{c} subsituiertem Aryl oder einem gegebenenfalls ein- oder mehrfach, gleich oder verschieden mit R^{c} substituiertem 5- oder 6-gliedrigen Heteroaryl,
oder für C₃-C₈-Cycloalkoxy oder Heterocycloalkoxy steht, welche gegebenenfalls ein- oder mehrfach, gleich oder verschieden substituiert sein können mit Hydroxy, Halogen, Cyano oder C₁-C₆-Alkyl,
oder für Aryloxy oder 5- oder 6-gliedriges Heteroaryloxy steht, worin Aryloxy und 5- oder 6-gliedriges Heteroaryloxy gegebenenfalls ein- oder mehrfach, gleich oder verschieden, substituiert sein können mit Hydroxy, Halogen, Cyano, C(=O)OH, C(=O)OR^{a}, C₁-C₆-Alkyl oder C₁-C₆-Alkoxy,
oder für C₃-C₈-Cycloalkyl oder Heterocycloalkyl steht, welche gegebenenfalls ein- oder mehrfach, gleich oder verschieden substituiert sein können mit Hydroxy, Halogen, Cyano oder C₁-C₆-Alkyl,
oder für C₂-C₆-Alkenyl oder C₂-C₆-Alkinyl steht,
oder für Aryl, 5- bis 10-gliedriges Heteroaryl, Aryl-C₁-C₄-alkyl oder 5- oder 6-gliedriges Heteroaryl-C₁-C₄-alkyl steht, worin Aryl und Heteroaryl gegebenenfalls ein- oder mehrfach, gleich oder verschieden substituiert sein können mit Halogen, Hydroxy, Cyano, C(=O)OH, C(=O)OR^{a}, C₁-C₆-Alkyl, C₃-C₈-Cycloalkyl oder C₁-C₆-Alkoxy;
- R^{a}: für C₁-C₆-Alkyl, C₃-C₁₀-Cycloalkyl, Heterocycloalkyl, Aryl oder Heteroaryl steht, worin Alkyl, Cycloalkyl, Heterocycloalkyl, Aryl und Heteroaryl gegebenenfalls ein- oder mehrfach, gleich oder verschieden substituiert sein können mit Halogen, Hydroxy, Cyano, C₁-C₃-Alkyl, C₁-C₃-Alkoxy, Heterocycloalkyl, -C(=O)O-C₁-C₆-Alkyl oder S(=O)₂-C₁-C₆-Alkyl;
- R^{b}: für C₁-C₆-Alkyl oder C₃-C₁₀-Cycloalkyl steht;
oder R^{a} und R^{b} bilden gemeinsam mit dem Stickstoffatom einen 5- oder 6-gliedrigen Heterocyclus, welcher gegebenenfalls ein- oder mehrfach, gleich oder verschieden substituiert sein kann mit Hydroxy, Halogen, Cyano oder C₁-C₆-Alkyl;
- R^{c}: für Hydroxy, Halogen, Cyano, C₁-C₃-Alkyl oder C₁-C₃-Alkoxy steht;
- R^{d}: für Wasserstoff, C₁-C₆-Alkyl oder C₃-C₁₀-Cycloalkyl steht;
- R²: für Wasserstoff, C₁-C₆-Alkyl oder C₃-C₆-Cycloalkyl steht;
- R¹³: für Wasserstoff oder C₁-C₆-Alkyl steht;
- W: für 5-gliedriges Heteroaryl steht, welches ein bis drei Heteroatome ausgewählt aus der Gruppe N, O und S enthält und gegebenenfalls einfach mit R³ sowie gegebenenfalls ein- oder mehrfach, gleich oder verschieden mit R⁴ substituiert sein kann oder
- W: für Pyridyl, Pyrazinyl, Pyridazinyl, 1,2,4-Triazinyl oder 1,3,5-Triazinyl steht, welche gegebenenfalls einfach mit R³ und gegebenenfalls ein- oder mehrfach, gleich oder verschieden mit R⁴ substituiert sein können;
- R³: für Wasserstoff, Halogen, Cyano, C(=O)R^{a}, NH₂, NHR^{a}, N(R^{a})R^{b}, N(H)C(=O)R^{a} oder C₁-C₆-Alkyl steht, wobei
C₁-C₆-Alkyl gegebenenfalls ein- oder mehrfach, gleich oder verschieden substituiert sein kann mit Hydroxy, Halogen, Cyano, C(=O)R^{a}, C(=O)OH, C(=O)OR^{a}, S(=O)₂-C₁-C₆-Alkyl, NH2, NHR^{a}, N(R^{a})R^{b}, C₁-C₆-Alkoxy, C₃-C₈-Cycloalkoxy,
worin C₁-C₆-Alkoxy und C₃-C₈-Cycloalkoxy gegebenenfalls ein- oder mehrfach, gleich oder verschieden substituiert sein können mit Halogen;
oder C₁-C₆-Alkyl ist gegebenenfalls ein- oder mehrfach, gleich oder verschieden substituiert mit C₃-C₆-Cycloalkyl oder Heterocycloalkyl,
worin C₃-C₆-Cycloalkyl und Heterocycloalkyl optional ein-, zwei- oder dreimal, gleich oder verschieden, substituiert sein können mit Halogen, Cyano, C₁-C₃-Alkyl oder C₁-C₃-Alkoxy,
oder C₁-C₆-Alkyl ist gegebenenfalls ein- oder mehrfach, gleich oder verschieden substituiert mit Aryl oder 5- oder 6-gliedrigem Heteroaryl,
worin Aryl und 5- oder 6-gliedriges Heteroaryl optional ein-, zwei- oder dreimal, gleich oder verschieden, substituiert sein können mit Halogen, Cyano, C₁-C₃-Alkyl oder C₁-C₃-Alkoxy,
oder
- R³: für C₁-C₆-Alkoxy steht, wobei C₁-C₆-Alkoxy gegebenenfalls ein- oder mehrfach, gleich oder verschieden substituiert sein kann mit Hydroxy, Halogen, Cyano, C(=O)OR^{a}, S(=O)₂-C₁-C₆-Alkyl, N(R^{a}) R^{b}, C₃-C₈-Cycloalkyl, C₁-C₄-Alkoxy, C₃-C₈-Cycloalkoxy,
oder für C₃-C₆-Cycloalkyl, Heterocycloalkyl oder C₅-C₁₁-Spirocycloalkyl steht, wobei Cycloalkyl, Heterocycloalkyl und Spirocycloalkyl gegebenenfalls ein- oder mehrfach, gleich oder verschieden substituiert sein können mit Hydroxy, Halogen, Cyano, C(=O)R^{a}, C(=O)OH, C(=O)OR^{a}, C₁-C₆-Alkyl oder C₁-C₄-Alkoxy;
oder für Aryl oder 5- bis 10-gliedriges Heteroaryl steht, worin
Aryl und Heteroaryl gegebenenfalls ein- oder mehrfach, gleich oder verschieden substituiert sein können mit Halogen, Hydroxy, Cyano, C(=O)OR^{a}, S(=O)₂-C₁-C₆-Alkyl, NO₂, NH2, NHR^{a}, N(R^{a})R^{b}, N(H)C(=O)R^{a}, C₃-C₈-Cycloalkyl, C₁-C₃-Alkoxy oder C₁-C₃-Alkyl, wobei
C₁-C₃-Alkyl gegebenenfalls ein- oder mehrfach, gleich oder verschieden substituiert sein kann mit Halogen;
- R⁴: für Halogen, Hydroxy, Cyano oder C₁-C₆-Alkyl steht, worin C₁-C₆-Alkyl gegebenenfalls ein- oder mehrfach, gleich oder verschieden substituiert sein kann mit Halogen, C₁-C₆-Alkoxy, worin C₁-C₆-Alkoxy gegebenenfalls ein- oder mehrfach, gleich oder verschieden substituiert sein kann mit Halogen, C₂-C₆-Alkenyl, C₂-C₆-Alkinyl, C₃-C₁₀-Cycloalkyl, 3- bis 10-gliedriges Heterocycloalkyl oder Aryl, wobei Aryl gegebenenfalls ein- oder mehrfach, gleich oder verschieden mit R substituiert sein kann, oder
- R⁴: für Aryl oder Heteroaryl steht, welche gegebenenfalls ein- oder mehrfach, gleich oder verschieden substituiert sein können mit R, oder
- R⁴: für C(=O)R^{a}, C(=O)NH₂, C(=O)N(H)R^{a}, C(=O)N(R^{a})R^{b}, C(=O)OR^{a}, NH₂, NHR^{a}, N(R^{a})R^{b}, N(H)C(=O)R^{a}, N(R^{a})C(=O)R^{a}, N(H)C(=O)NH₂, N(H)C(=O)NHR^{a}, N(H)C(=O)N(R^{a})R^{b}, N(R^{a})C(=O)NH₂, N(R^{a})C(=O)NHR^{a}, N(R^{a})C(=O)N(R^{a})R^{b}, N(H)C(=O)OR^{a}, N(R^{a})C(=O)OR^{a}, NO₂, N(H)S(=O)R^{a}, N(R^{a})S(=O)R^{a}, N(H)S(=O)₂R^{a}, N(R^{a})S(=O)₂R^{a}, N=S(=O)(R^{a})R^{b}, OC(=O)R^{a}, OC(=O)NH₂, OC(=O)NHR^{a}, OC(=O)N(R^{a})R^{b}, SH, SR^{a}, S(=O)R^{a}, S(=O)₂R^{a}, S(=O)₂NH₂, S(=O)₂NHR^{a}, S(=O)₂N(R^{a})R^{b} oder S(=O)(=N-R^{a})R^{b} steht;
- R: für Halogen, Cyano, C₁-C₆-Alkyl, C₂-C₆-Alkenyl,C₂-C₆-Alkinyl, C₃-C₁₀-Cycloalkyl, 3- bis 10-gliedriges Heterocycloalkyl, Aryl, Heteroaryl, C(=O)R^{a}, C(=O)NH₂, C(=O)N(H)R^{a}, C(=O)N(R^{a})R^{b}, C(=O)OR^{a}, NH₂, NHR^{a}, N(R^{a})R^{b}, N(H)C(=O)R^{a}, N(R^{a})C(=O)R^{a}, N(H)C(=O)NH₂, N(H)C(=O)NHR^{a}, N(H)C(=O)N(R^{a})R^{b}, N(R^{a})C(=O)NH₂, N(R^{a})C(=O)NHR^{a}, N(R^{a})C(=O)N(R^{a})R^{b}, N(H)C(=O)OR^{a}, N(R^{a})C(=O)OR^{a}, NO₂, N(H)S(=O)R^{a}, N(R^{a})S(=O)R^{a}, N(H)S(=O)₂R^{a}, N(R^{a})S(=O)₂R^{a}, N=S(=O)(R^{a})R^{b}, OH, C₁-C₆-Alkoxy, OC(=O)R^{a}, OC(=O)NH₂, OC(=O)NHR^{a}, OC(=O)N(R^{a})R^{b}, SH, SR^{a}, S(=O)R^{a}, S(=O)₂R^{a}, S(=O)₂NH₂, S(=O)₂NHR^{a}, S(=O)₂N(R^{a})R^{b} oder S(=O)(=NR^{a})R^{b} steht;
- n: für 0 oder 1 steht;
- Y: für eine Gruppe steht, ausgewählt aus: 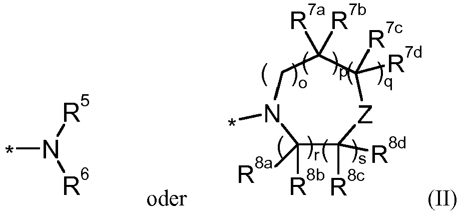
wobei * für die Verknüpfungsstelle der Gruppe mit dem Rest des Moleküls steht;
- R⁵: für Wasserstoff, C₁-C₆-Alkyl oder C₃-C₁₀-Cycloalkyl steht, wobei
C₁-C₆-Alkyl gegebenenfalls ein- oder mehrfach, gleich oder verschieden substituiert sein kann mit Hydroxy, Halogen, Cyano, C(=O)OH, C(=O)OR^{a}, S(=O)₂-C₁-C₆-Alkyl, N(R^{a})R^{b}, C₁-C₄-Alkoxy oder C₃-C₈-Cycloalkyl;
- R⁶: für Wasserstoff oder C₁-C₆-Alkyl steht, wobei
C₁-C₆-Alkyl gegebenenfalls ein- oder mehrfach, gleich oder verschieden substituiert sein kann mit Hydroxy, Halogen, Cyano, C₃-C₁₀-Cycloalkyl, C(=O)R^{a}, C(=O)OH, C(=O)OR^{a}, S(=O)₂-C₁-C₆-Alkyl, N(R^{a})R^{b}, C₁-C₄-Alkoxy oder C₃-C₈-Cycloalkoxy,
oder für C₃-C₁₀-Cycloalkyl steht, wobei
C₃-C₁₀-Cycloalkyl gegebenenfalls ein- oder mehrfach, gleich oder verschieden substituiert sein kann mit Hydroxy, Halogen, Cyano oder C₁-C₆-Alkyl, wobei C₁-C₆-Alkyl gegebenenfalls mit Hydroxy substituiert sein kann,
oder für Heterocycloalkyl steht, worin
Heterocycloalkyl gegebenenfalls ein- oder mehrfach, gleich oder verschieden substituiert sein kann mit Halogen, Cyano, C₁-C₃-Alkyl oder C₁-C₃-Alkoxy,
oder für Aryl oder 5- oder 6-gliedriges Heteroaryl steht, worin
Aryl und 5- oder 6-gliedriges Heteroaryl gegebenenfalls ein- oder mehrfach, gleich oder verschieden substituiert sein können mit Halogen, Cyano, C₁-C₃-Alkyl, C₁-C₃-Alkoxy, S(=O)₂NH₂, S(=O)₂NHR^{a} oder S(=O)₂N(R^{a})R^{b};
- R^{7a}: für Wasserstoff, Halogen, N(R^{a})R^{b}, C₁-C₆-Alkyl oder C₃-C₁₀-Cycloalkyl steht, wobei
C₁-C₆-Alkyl gegebenenfalls ein- oder mehrfach, gleich oder verschieden substituiert sein kann mit Hydroxy, Halogen, Cyano, C(=O)OH, C(=O)OR^{a}, S(=O)₂-C₁-C₆-Alkyl, N(R^{a})R^{b}, C₁-C₄-Alkoxy, C₃-C₈-Cycloalkyl oder Heterocycloalkyl;
- R^{7b}: für Wasserstoff, Halogen oder C₁-C₆-Alkyl steht, wobei
C₁-C₆-Alkyl gegebenenfalls ein- oder mehrfach, gleich oder verschieden substituiert sein kann mit Hydroxy, Halogen, Cyano, C(=O)OH, C(=O)OR^{a}, S(=O)₂-C₁-C₆-Alkyl, N(R^{a})R^{b}, C₁-C₄-Alkoxy, C₃-C₈-Cycloalkyl oder Heterocycloalkyl;
oder R^{7a} und R^{7b} bilden gemeinsam mit dem Kohlenstoffatom ein C₃-C₆-Cycloalkyl, welches gegebenenfalls ein- oder mehrfach, gleich oder verschieden substituiert sein kann mit Hydroxy, Halogen, Cyano oder C₁-C₆-Alkyl,
oder R^{7a} und R^{7b} stehen gemeinsam für eine Oxo-Gruppe;
- R^{7c}: für Wasserstoff, Halogen, N(R^{a})R^{b}, C₁-C₆-Alkyl oder C₃-C₁₀-Cycloalkyl steht, wobei
C₁-C₆-Alkyl gegebenenfalls ein- oder mehrfach, gleich oder verschieden substituiert sein kann mit Hydroxy, Halogen, Cyano, C(=O)OH, C(=O)OR^{a}, S(=O)₂-C₁-C₆-Alkyl, N(R^{a})R^{b}, C₁-C₄-Alkoxy, C₃-C₈-Cycloalkyl oder Heterocycloalkyl;
- R^{7d}: für Wasserstoff, Halogen oder C₁-C₆-Alkyl steht, wobei
C₁-C₆-Alkyl gegebenenfalls ein- oder mehrfach, gleich oder verschieden substituiert sein kann mit Hydroxy, Halogen, Cyano, C(=O)OH, C(=O)OR^{a}, S(=O)₂-C₁-C₆-Alkyl, N(R^{a})R^{b}, C₁-C₄-Alkoxy, C₃-C₈-Cycloalkyl oder Heterocycloalkyl;
oder R^{7c} und R^{7d} bilden gemeinsam mit dem Kohlenstoffatom ein C₃-C₆-Cycloalkyl, welches gegebenenfalls ein- oder mehrfach, gleich oder verschieden substituiert sein kann mit Hydroxy, Halogen, Cyano oder C₁-C₆-Alkyl,
oder R^{7c} und R^{7d} stehen gemeinsam für eine Oxo-Gruppe;
- R^{8a}: für Wasserstoff, Halogen, N(R^{a})R^{b}, C₁-C₆-Alkyl oder C₃-C₁₀-Cycloalkyl steht, wobei
C₁-C₆-Alkyl gegebenenfalls ein- oder mehrfach, gleich oder verschieden substituiert sein kann mit Hydroxy, Halogen, Cyano, C(=O)OH, C(=O)OR^{a}, S(=O)₂-C₁-C₆-Alkyl, N(R^{a})R^{b}, C₁-C₄-Alkoxy, C₃-C₈-Cycloalkyl oder Heterocycloalkyl;
- R^{8b}: für Wasserstoff, Halogen oder C₁-C₆-Alkyl steht, wobei
C₁-C₆-Alkyl gegebenenfalls ein- oder mehrfach, gleich oder verschieden substituiert sein kann mit Hydroxy, Halogen, Cyano, C(=O)OH, C(=O)OR^{a}, S(=O)₂-C₁-C₆-Alkyl, N(R^{a})R^{b}, C₁-C₄-Alkoxy, C₃-C₈-Cycloalkyl oder Heterocycloalkyl;
oder R^{8a} und R^{8b} bilden gemeinsam mit dem Kohlenstoffatom ein C₃-C₆-Cycloalkyl, welches gegebenenfalls ein- oder mehrfach, gleich oder verschieden substituiert sein kann mit Hydroxy, Halogen, Cyano oder C₁-C₆-Alkyl,
- R^{8c}: für Wasserstoff, Halogen, N(R^{a})R^{b}, C₁-C₆-Alkyl oder C₃-C₁₀-Cycloalkyl steht, wobei
C₁-C₆-Alkyl gegebenenfalls ein- oder mehrfach, gleich oder verschieden substituiert sein kann mit Hydroxy, Halogen, Cyano, C(=O)OH, C(=O)OR^{a}, S(=O)₂-C₁-C₆-Alkyl, N(R^{a})R^{b}, C₁-C₄-Alkoxy, C₃-C₈-Cycloalkyl oder Heterocycloalkyl;
- R^{8d}: für Wasserstoff, Halogen oder C₁-C₆-Alkyl steht, wobei
C₁-C₆-Alkyl gegebenenfalls ein- oder mehrfach, gleich oder verschieden substituiert sein kann mit Hydroxy, Halogen, Cyano, C(=O)OH, C(=O)OR^{a}, S(=O)₂-C₁-C₆-Alkyl, N(R^{a})R^{b}, C₁-C₄-Alkoxy, C₃-C₈-Cycloalkyl oder Heterocycloalkyl;
oder R^{8c} und R^{8d} bilden gemeinsam mit dem Kohlenstoffatom ein C₃-C₆-Cycloalkyl, welches gegebenenfalls ein- oder mehrfach, gleich oder verschieden substituiert sein kann mit Hydroxy, Halogen, Cyano oder C₁-C₆-Alkyl,
oder R^{8c} und R^{8d} stehen gemeinsam für eine Oxo-Gruppe;
- o: für 0, 1 oder 2 steht,
- p: für 0, 1 oder 2 steht,
- q: für 0, 1 oder 2 steht,
- r: für 0, 1 oder 2 steht,
- s: für 0, 1 oder 2 steht,
wobei o, p, q, r und s nicht gleichzeitig 0 bedeuten;
- Z: für eine Gruppe steht ausgewählt aus C(=O), CR⁹R¹⁰, NR¹¹, O, S, S(=O) oder S(=O)₂;
- R⁹: für Wasserstoff oder C₁-C₆-Alkyl steht,
- R¹⁰: für Wasserstoff, Halogen, Cyano, C(=O)R^{a}, C(=O)OH, C(=O)OR^{a}, C(=O)NH₂, C(=O)N(H)R^{a}, C(=O)N(R^{a})R^{b}, N(H)C(=O)R^{a}, N(R^{b})C(=O)R^{a}, S(=O)₂R^{a}, Hydroxy, N(R^{a})R^{b} oder C₁-C₆-Alkyl steht, wobei
C₁-C₆-Alkyl gegebenenfalls ein- oder mehrfach, gleich oder verschieden substituiert sein kann mit Hydroxy, Halogen, Cyano, C(=O)R^{a}, C(=O)OH, C(=O)OR^{a}, S(=O)₂-C₁-C₆-Alkyl, N(R^{a})R^{b}, C₁-C₄-Alkoxy oder C₃-C₈-Cycloalkoxy,
oder für C₁-C₆-Alkoxy steht, wobei
C₁-C₆-Alkoxy gegebenenfalls ein- oder mehrfach, gleich oder verschieden substituiert sein kann mit Hydroxy, Halogen, Cyano, C(=O)OH, C(=O)OR^{a}, S(=O)₂-C₁-C₆-Alkyl, N(R^{a})R^{b}, C₃-C₈-Cycloalkyl, C₁-C₄-Alkoxy, C₃-C₈-Cycloalkoxy, Heterocycloalkyl, Aryl oder 5- oder 6-gliedriges Heteroaryl, worin
Aryl und 5- oder 6-gliedriges Heteroaryl gegebenenfalls ein- oder mehrfach, gleich oder verschieden substituiert sein können mit Halogen, Cyano, C₁-C₃-Alkyl oder C₁-C₃-Alkoxy,
oder für Aryloxy oder 5- oder 6-gliedriges Heteroaryloxy steht, worin
Aryloxy und 5- oder 6-gliedriges Heteroaryloxy gegebenenfalls ein- oder mehrfach, gleich oder verschieden substituiert sein können mit Hydroxy, Halogen, Cyano, C(=O)OH, C(=O)OR^{a}, C₁-C₃-Alkyl oder C₁-C₃-Alkoxy,
oder für C₃-C₈-Cycloalkyl, C₃-C₈-Cycloalkyl-C₁-C₄-alkyl, Heterocycloalkyl oder Heterocycloalkyl-C₁-C₄-alkyl steht, welche gegebenenfalls ein- oder mehrfach, gleich oder verschieden substituiert sein können mit Hydroxy, Halogen, Cyano, C(=O)R^{a}, C(=O)OH, C(=O)OR^{a}, C₁-C₆-Alkyl oder C₁-C₆-Alkoxy, wobei
C₁-C₆-Alkoxy gegebenenfalls ein- oder mehrfach, gleich oder verschieden substituiert sein kann mit Halogen oder einer Oxo-Gruppe,
oder für C₂-C₆-Alkenyl oder C₂-C₆-Alkinyl steht,
oder für Aryl, 5- bis 10-gliedriges Heteroaryl, Aryl-C₁-C₄-alkyl oder 5- oder 6-gliedriges Heteroaryl-C₁-C₄-alkyl steht, worin
Aryl und Heteroaryl gegebenenfalls ein- oder mehrfach, gleich oder verschieden substituiert sein können mit Halogen, Hydroxy, Cyano, C(=O)OH, C(=O)OR^{a}, NHR^{a}, N(R^{a})R^{b}, C₁-C₃-Alkyl, C₃-C₈-Cycloalkyl oder C₁-C₃-Alkoxy;
oder R⁹ und R¹⁰ bilden gemeinsam mit dem Kohlenstoffatom ein C₃-C₈-Cycloalkyl oder einen 4- bis 6-gliedrigen Heterocyclus, wobei
der C₃-C₈-Cycloalkylrest oder der 4- bis 6-gliedrige Heterocyclus gegebenenfalls ein- oder mehrfach, gleich oder verschieden substituiert sein können mit Hydroxy, Halogen, Cyano, C₁-C₆-Alkyl, C(=O)R^{a} oder einer Oxo-Gruppe;
- R¹¹: für Wasserstoff, C(=O)R^{a}, C(=O)OR^{a}, C(=O)NH₂, C(=O)N(H)R^{a}, C(=O)N(R^{a})R^{b}, S(=O)₂R^{a}, S(=O)₂N(R^{a})R^{b} oder C₁-C₆-Alkyl steht, wobei
C₁-C₆-Alkyl gegebenenfalls ein- oder mehrfach, gleich oder verschieden substituiert sein kann mit Hydroxy, Halogen, Cyano, C(=O)R^{a}, C(=O)OR^{a}, C(=O)NH₂, C(=O)N(H)R^{a}, C(=O)N(R^{a})R^{b}, S(=O)₂-C₁-C₆-Alkyl, N(R^{a})R^{b}, C₃-C₈-Cycloalkyl, C₁-C₄-Alkoxy oder C₃-C₈-Cycloalkoxy, worin
C₃-C₈-Cycloalkyl, C₁-C₄-Alkoxy und C₃-C₈-Cycloalkoxy gegebenenfalls ein- oder mehrfach, gleich oder verschieden substituiert sein können mit Hydroxy oder Halogen,
oder für C₃-C₈-Cycloalkyl, Heterocycloalkyl oder Heterocycloalkyl-C₁-C₄-alkyl steht, welche gegebenenfalls ein- oder mehrfach, gleich oder verschieden substituiert sein können mit Hydroxy, Halogen, Cyano, C₁-C₆-Alkyl, C₁-C₆-Alkoxy, wobei Alkyl und Alkoxy gegebenenfalls ein- oder mehrfach, gleich oder verschieden substituiert sein können mit Halogen oder einer Oxo-Gruppe,
oder für C₂-C₆-Alkenyl oder C₂-C₆-Alkinyl steht,
oder für Aryl, 5- bis 10-gliedriges Heteroaryl, Aryl-C₁-C₄-alkyl oder 5- oder 6-gliedriges Heteroaryl-C₁-C₄-alkyl steht, worin
Aryl und Heteroaryl gegebenenfalls ein- oder mehrfach, gleich oder verschieden substituiert sein können mit Halogen, Hydroxy, Cyano, C(=O)OH, C(=O)OR^{a}, C₁-C₃-Alkyl, C₃-C₈-Cycloalkyl oder C₁-C₃-Alkoxy;
und ihre Diastereomere, Enantiomere, ihre Salze, ihre Solvate oder die Solvate ihrer Salze.

Wenn bei den im Folgenden beschriebenen Synthese-Intermediaten und Ausführungsbeispielen der Erfindung eine Verbindung in der Form eines Salzes der korrespondierenden Base bzw. Säure aufgeführt ist, so ist die exakte stöchiometrische Zusammensetzung eines solchen Salzes, wie es nach dem jeweiligen Herstell- und/oder Reinigungsverfahren erhalten wurde, in der Regel nicht bekannt. Sofern nicht genauer spezifiziert, sind daher Namens- und Strukturformel-Zusätze wie beispielsweise "Hydrochlorid", "Trifluoracetat", "Natrium-Salz" bzw. "x HCl", "x CF₃COOH", "x Na⁺" bei solchen Salzen nicht stöchiometrisch zu verstehen, sondern haben allein deskriptiven Charakter bezüglich der enthaltenen salzbildenden Komponenten.

Sinngemäß gleiches gilt für den Fall, dass Synthese-Intermediate oder Ausführungsbeispiele oder Salze hiervon nach den beschriebenen Herstell- und/ oder Reinigungsverfahren in Form von Solvaten, wie beispielsweise Hydraten, erhalten wurden, deren stöchiometrische Zusammensetzung (sofern definierter Art) nicht bekannt ist.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.
Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind, aber beispielsweise für die Isolierung oder Reinigung der erfindungsgemäßen Verbindungen verwendet werden können.
Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.
Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.
Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.
Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie 2H (Deuterium), 3H (Tritium), 13C, 14C, 15N, 17O, 18O, 32P, 33P, 33S, 34S, 35S, 36S, 18F, 36Cl, 82Br, 123I, 124I, 129I und 131I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoffverteilung im Körper; aufgrund der vergleichsweise leichten Herstell- und Detektierbarkeit sind hierfür insbesondere mit 3H- oder 14C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vor-liegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den für die Ausführungsbeispiele angegebenen Vorschriften, indem entsprechende isotopische Modifikationen der jeweiligen Reagentien und/oder Ausgangsverbindungen eingesetzt werden.

Ein weiterer Gegenstand der vorliegenden Erfindung sind alle möglichen kristallinen und polymorphen Formen der erfindungsgemäßen Verbindungen, wobei die Polymorphe entweder als einzelne Polymorphe oder als Gemisch mehrerer Polymorphe in allen Konzentrationsbereichen vorliegen können.

Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgenden Bedeutungen:
Alkyl steht im Rahmen der Erfindung für einen linearen oder verzweigten Alkylrest mit der jeweils angegebenen Anzahl an Kohlenstoffatomen. Beispielhaft seien Methyl, Ethyl, n-Propyl, Isopropyl, n-Butyl, iso-Butyl, 1-Methylpropyl, tert.-Butyl, n-Pentyl, 1-Ethylpropyl, 1-Methylbutyl, 2-Methylbutyl, 3-Methylbutyl, n-Hexyl, 1-Methylpentyl, 2-Methylpentyl, 3-Methylpentyl, 4-Methylpentyl, 1-Ethylbutyl und 2-Ethylbutyl genannt. Bevorzugt sind Methyl, Ethyl, n-Propyl, n-Butyl und 1-Methylpropyl sowie tert.-Butyl.
Alkenyl steht im Rahmen der Erfindung für einen linearen oder verzweigten, monovalenten Kohlenwasserstoffrest mit mindestens einer Doppelbindung und mit der jeweils angegebenen Anzahl an Kohlenstoffatomen. In der Regel sind das 2 bis 6 Kohlenstoffatome, bevorzugt 2 bis 4, und besonders bevorzugt 2 oder 3 Kohlenstoffatomen.
   Im Falle von mehreren Doppelbindungen können diese isoliert oder konjugiert vorliegen, wobei isolierte Doppelbindungen bevorzugt sind.
   Beispielhaft seien genannt:
   Vinyl, Allyl, (*E*)-2-Methylvinyl, (*Z*)-2-Methylvinyl, Homoallyl, (*E*)-But-2-enyl, (*Z*)-But-2-enyl, (*E*)-But-1-enyl, (*Z*)-But-1-enyl, Pent-4-enyl, (*E*)-Pent-3-enyl, (*Z*)-Pent-3-enyl, (*E*)-Pent-2-enyl, (Z)-Pent-2-enyl, (*E*)-Pent-1-enyl, (*Z*)-Pent-1-enyl, Hex-5-enyl, (*E*)-Hex-4-enyl, (Z)-Hex-4-enyl, (*E*)-Hex-3-enyl, (*Z*)-Hex-3-enyl, (*E*)-Hex-2-enyl, (*Z*)-Hex-2-enyl, (*E*)-Hex-1-enyl, (*Z*)-Hex-1-enyl, Isopropenyl, 2-Methylprop-2-enyl, 1-Methylprop-2-enyl, 2-Methylprop-1-enyl, (*E*)-1-Methylprop-1-enyl, (*Z*)-1-Methylprop-1-enyl, 3-Methylbut-3-enyl, 2-Methylbut-3-enyl, 1-Methylbut-3-enyl, 3-Methylbut-2-enyl, (*E*)-2-Methylbut-2-enyl, (*Z*)-2-Methylbut-2-enyl, (*E*)-1-Methylbut-2-enyl, (*Z*)-1-Methylbut-2-enyl, (*E*)-3-Methylbut-1-enyl, (*Z*)-3-Methylbut-1-enyl, (*E*)-2-Methylbut-1-enyl, (*Z*)-2-Methylbut-1-enyl, (*E*)-1-Methylbut-1-enyl, (*Z*)-1-Methylbut-1-enyl, 1,1-Dimethylprop-2-enyl, 1-Ethylprop-1-enyl, 1-Propylvinyl, 1-Isopropylvinyl, 4-Methylpent-4-enyl, 3-Methylpent-4-enyl, 2-Methylpent-4-enyl, 1-Methylpent-4-enyl, 4-Methylpent-3-enyl, (*E*)-3-Methylpent-3-enyl, (*Z*)-3-Methylpent-3-enyl, (*E*)-2-Methylpent-3-enyl, (*Z*)-2-Methylpent-3-enyl, (*E*)-1-Methylpent-3-enyl, (*Z*)-1-Methylpent-3-enyl, (*E*)-4-Methylpent-2-enyl, (*Z*)-4-Methylpent-2-enyl, (*E*)-3-Methylpent-2-enyl, (Z)-3-Methylpent-2-enyl, (*E*)-2-Methylpent-2-enyl, (*Z*)-2-Methylpent-2-enyl, (*E*)-1-Methylpent-2-enyl, (*Z*)-1-Methylpent-2-enyl, (*E*)-4-Methylpent-1-enyl, (*Z*)-4-Methylpent-1-enyl, (*E*)-3-Methylpent-1-enyl, (*Z*)-3-Methylpent-1-enyl, (*E*)-2-Methylpent-1-enyl, (*Z*)-2-Methylpent-1-enyl, (*E*)-1-Methylpent-1-enyl, (*Z*)-1-Methylpent-1-enyl, 3-Ethylbut-3-enyl, 2-Ethylbut-3-enyl, 1-Ethylbut-3-enyl, (*E*)-3-Ethylbut-2-enyl, (*Z*)-3-Ethylbut-2-enyl, (*E*)-2-Ethylbut-2-enyl, (*Z*)-2-Ethylbut-2-enyl, (*E*)-1-Ethylbut-2-enyl, (*Z*)-1-Ethylbut-2-enyl, (*E*)-3-Ethylbut-1-enyl, (*Z*)-3-Ethylbut-1-enyl, 2-Ethylbut-1-enyl, (*E*)-1-Ethylbut-1-enyl, (*Z*)-1-Ethylbut-1-enyl, 2-Propylprop-2-enyl, 1-Propylprop-2-enyl, 2-Isopropylprop-2-enyl, 1-Isopropylprop-2-enyl, (*E*)-2-Propylprop-1-enyl, (*Z*)-2-Propylprop-1-enyl, (*E*)-1-Propylprop-1-enyl, (*Z*)-1-Propylprop-1-enyl, (*E*)-2-Isopropylprop-1-enyl, (*Z*)-2-Isopropylprop-1-enyl, (*E*)-1-Isopropylprop-1-enyl, (*Z*)-1-Isopropylprop-1-enyl, (*E*)-3,3-Dimethylprop-1-enyl, (*Z*)-3,3-Dimethylprop-1-enyl, 1-(1,1-Dimethylethyl)ethenyl, Buta-1,3-dienyl, Penta-1,4-dienyl, Hexa-1,5-dienyl, Methylhexadienyl. Besonders bevorzugt ist Vinyl oder Allyl.
Alkinyl steht im Rahmen der Erfindung für einen linearen oder verzweigten, monovalenten Kohlenwasserstoffrest mit mindestens einer Dreifachbindung und mit der jeweils angegebenen Anzahl an Kohlenstoffatomen. Das sind in der Regel 2 bis 6 Kohlenstoffatome, bevorzugt 2 bis 4, und besonders bevorzugt 2 oder 3 Kohlenstoffatomen.
   Beispielhaft seien genannt:
   Ethinyl, Prop-1-inyl, Prop-2-inyl, But-1-inyl, But-2-inyl, But-3-inyl, Pent-1-inyl, Pent-2-inyl, Pent-3-inyl, Pent-4-inyl, Hex-1-inyl, Hex-2-inyl, Hex-3-inyl, Hex-4-inyl, Hex-5-inyl, 1-Methylprop-2-inyl, 2-Methylbut-3-inyl, 1-Methylbut-3-inyl, 1-Methylbut-2-inyl, 3-Methylbut-1-inyl, 1-Ethylprop-2-inyl, 3-Methylpent-4-inyl, 2-Methylpent-4-inyl, 1-Methylpent-4-inyl, 2-Methylpent-3-inyl, 1-Methylpent-3-inyl, 4-Methylpent-2-inyl, 1-Methylpent-2-inyl, 4-Methylpent-1-inyl, 3-Methylpent-1-inyl, 2-Ethylbut-3-inyl, 1-Ethylbut-3-inyl, 1-Ethylbut-2-inyl, 1-Propylprop-2-inyl, 1-Isopropylprop-2-inyl, 2,2-Dimethylbut-3-inyl, 1,1-Dimethylbut-3-inyl, 1,1-Dimethylbut-2-inyl oder 3,3 -Dimethylbut-1 -inyl.
   Besonders bevorzugt ist Ethinyl, Prop-1-inyl oder Prop-2-inyl.
Cycloalkyl steht im Rahmen der Erfindung für einen monocyclischen, gesättigten Alkylrest mit der jeweils angegebenen Anzahl an Kohlenstoffatomen. Beispielhaft und vorzugsweise seien Cyclopropyl, Cyclobutyl, Cyclopentyl, Cyclohexyl und Cycloheptyl genannt.
Alkoxy steht im Rahmen der Erfindung für einen linearen oder verzweigten Alkoxyrest mit der jeweils angegebenen Anzahl an Kohlenstoffatomen. 1 bis 6 bzw. 1 bis 4 Kohlenstoffatome sind bevorzugt. Beispielhaft seien Methoxy, Ethoxy, n-Propoxy, Isopropoxy, 1-Methylpropoxy, n-Butoxy, iso-Butoxy, tert.-Butoxy, n-Pentoxy, iso-Pentoxy, 1-Ethylpropoxy, 1-Methylbutoxy, 2-Methylbutoxy, 3-Methylbutoxy und n-Hexoxy genannt. Bevorzugt ist ein linearer oder verzweigter Alkoxyrest mit 1 bis 4 Kohlenstoffatomen. Beispielhaft und vorzugsweise seien Methoxy, Ethoxy, n-Propoxy, 1-Methylpropoxy, n-Butoxy und iso-Butoxy genannt.
Cycloalkoxy steht im Rahmen der Erfindung für einen monocyclischen, gesättigten Alkylrest mit der jeweils angegebenen Anzahl an Kohlenstoffatomen, der über ein Sauerstoffatom gebunden ist. Beispielhaft und vorzugsweise seien genannt: Cyclopropoxy, Cyclobutoxy, Cyclopentoxy, Cyclohexoxy und Cycloheptoxy.
Aryl steht im Rahmen der Erfindung für ein monovalentes, mono- bis tricyclisches, aromatisches carbocyclisches Ringsystem mit in der Regel 6 bis 14 Kohlenstoffatomen. Beispielhaft seien genannt: Phenyl, Naphthyl und Phenanthryl. Bevorzugt ist Phenyl.
Heterocyclyl bzw. Heterocyclus bzw. Heterocycloalkyl steht im Rahmen der Erfindung für einen mono- oder polycyclischen, vorzugsweise mono- oder bicyclischen, nicht-aromatischen, gesättigten oder teilweise ungesättigten Heterocyclus mit in der Regel 3 bis 10, vorzugsweise 3 bis 7 Ringatomen und bis zu 3, vorzugsweise bis zu 1 oder 2 Heteroatomen und/oder Heterogruppen aus der Reihe N, O, S, SO und/oder SO₂ enthält. Bevorzugt sind 3- bis 7-gliedrige, monocyclische gesättigte Heterocyclylreste mit bis zu zwei Heteroatomen aus der Reihe O, N und S. Beispielhaft seien genannt: Azetidinyl, Oxetanyl, Pyrrolidinyl, Pyrazolidinyl, Tetrahydrofuranyl, Piperidinyl, Piperazinyl, Tetrahydropyranyl, Morpholinyl, Thiomorpholinyl, Dioxidothiomorpholinyl, Dihydroindolyl und Dihydroisoindolyl. Bevorzugt sind: Azetidinyl, Pyrrolidinyl, Piperidinyl, Piperazinyl und Morpholinyl.
Heteroaryl ist ein monovalentes, aromatisches mono- oder bicyclisches Ringsystem mit in der Regel 5 bis 10, vorzugsweise 5 bis 6 Ringatomen und vorzugsweise 1 bis 3 Heteroatomen. Als Heteroatome können Stickstoffatome, Sauerstoffatome und/oder Schwefelatome vorkommen. Die Bindungsvalenz kann an einem beliebigen aromatischen Kohlenstoffatom oder an einem Stickstoffatom sein.

Heteroarylreste mit 5 Ringatomen umfassen beispielsweise die Ringe:
Thienyl, Thiazolyl, Furyl, Pyrrolyl, Oxazolyl, Imidazolyl, Pyrazolyl, Isoxazolyl, Isothiazolyl, Oxadiazolyl, Triazolyl, Tetrazolyl und Thiadiazolyl.

Heteroarylreste mit 6 Ringatomen umfassen beispielsweise die Ringe:
Pyridyl, Pyridazinyl, Pyrimidinyl, Pyrazinyl und Triazinyl.

Ein bicyclischer Heteroarylrest gemäß der vorliegenden Erfindung hat 9 oder 10 Ringatome.

Heteroarylreste mit 9 Ringatomen umfassen beispielsweise die Ringe:
Phthalidyl, Thiophthalidyl, Indolyl, Isoindolyl, Indazolyl, Benzothiazolyl, Benzofuryl, Benzothienyl, Benzimidazolyl, Benzoxazolyl, Azocinyl, Indolizinyl, Purinyl, Indolinyl.

Heteroarylreste mit 10 Ringatomen umfassen beispielsweise die Ringe:
Isochinolinyl, Chinolinyl, Chinolizinyl, Chinazolinyl, Chinoxalinyl, Cinnolinyl, Phthalazinyl, 1,7- oder 1,8-Naphthyridinyl, Pteridinyl, Chromanyl.

Unter C₅-C₁₁-Spirocycloalkyl bzw. Heterospirocycloalkyl mit einem Ersatz von 1-4 Kohlenstoffatomen durch Stickstoff, Sauerstoff und/oder Schwefel, einschließlich seiner beiden oxidierten Formen SO oder SO₂ und seiner als Sulfoximin abgewandelter Derivate, ist eine Fusion aus zwei Ringsystemen zu verstehen, die sich ein gemeinsames Atom teilen. Beispiele sind Spiro[2,2]pentan, Spiro[2,3]hexan, Azaspiro[2.3]hexan, Spiro[3,3]heptan, Azaspiro[3,3]heptan, Oxaazaspiro[3.3]heptan, Thiaazaspiro[3,3]heptan, Oxaspiro[3,3]heptan, Oxazaspiro[5,3]nonan, Oxazaspiro[4,3]oktan, Oxazaspiro[5,5]undekan, Diazaspiro[3,3]heptan, Thiazaspiro[3,3]heptan, Thiazaspiro[4,3]oktan, Azaspiro[5,5]dekan, sowie die weiteren homologen Spiro[3,4]-, Spiro[4,4]-, Spiro[5,5]-, Spiro[6,6]-, Spiro[2,4]-, Spiro[2,5]-,Spiro[2,6]-, Spiro[3,5]-, Spiro[3,6]-, Spiro[4,5]-, Spiro[4,6]- und Spiro[5,6]-Systeme inklusive der durch Heteroatome modifizierten Varianten gemäß Definition.

Halogen steht im Rahmen der Erfindung für Fluor, Chlor und Brom. Bevorzugt sind Fluor und Chlor.

Hydroxy steht im Rahmen der Erfindung für OH.

Eine Oxo-Gruppe steht im Rahmen der Erfindung für ein Sauerstoffatom, das über eine Doppelbindung an ein Kohlenstoffatom gebunden ist.

Ein Symbol * an einer Bindung bedeutet die Verknüpfungsstelle im Molekül.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt.

Bevorzugt sind Verbindungen der Formel (I), in der
- W: für 5-gliedriges Heteroaryl steht, welches ein bis drei Heteroatome ausgewählt aus der Gruppe N, O und S enthält und gegebenenfalls einfach mit R³ und gegebenenfalls ein- oder mehrfach, gleich oder verschieden mit R⁴ substituiert sein kann, wobei ein Ring-Heteroatom neben dem Ring-Kohlenstoffatom steht, an dem sich die Verknüpfung der Gruppe mit dem Rest des Moleküls befindet
- oder: für Pyridyl, Pyrazinyl, Pyridazinyl, 1,2,4-Triazinyl oder 1,3,5-Triazinyl steht, welche gegebenenfalls einfach mit R³ und gegebenenfalls ein- oder mehrfach, gleich oder verschieden mit R⁴ substituiert sein können;, wobei ein Ring-Heteroatom neben dem Ring-Kohlenstoffatom steht, an dem sich die Verknüpfung der Gruppe mit dem Rest des Moleküls befindet.

Weiterhin bevorzugt sind Verbindungen der Formel (I), in der
- W: für eine Gruppe steht, ausgewählt aus den folgenden allgemeinen Formeln (III) bis (IX): 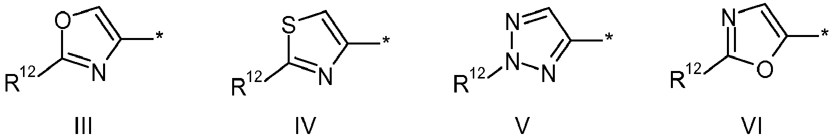 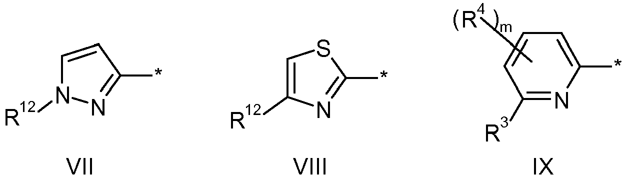 worin
R¹² für Wasserstoff, Halogen, ein gegebenenfalls ein- oder mehrfach, gleich oder verschieden mit Halogen substituiertes C₁-C₆-Alkyl, ein gegebenenfalls ein- oder mehrfach, gleich oder verschieden mit Halogen substituiertes C₃-C₆-Cycloalkyl, ein gegebenenfalls ein- oder mehrfach, gleich oder verschieden mit R^{c} subsituiertes Aryl, ein gegebenenfalls ein- oder mehrfach, gleich oder verschieden mit R^{c} substituiertes 5- oder 6-gliedriges Heteroaryl oder NHR^{a} steht;
m für 0, 1, 2 oder 3 steht und
R³ und R⁴ die weiter oben angegebenen Bedeutungen haben und
* für die Verknüpfungsstelle der Gruppe mit dem Rest des Moleküls steht.

Besonders bevorzugt sind außerdem Verbindungen der allgemeinen Formel (I), in der W für eine Gruppe der allgemeinen Formel (IX) steht, 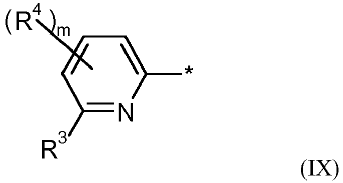
worin m = 0 oder 1 bedeutet und
R³ ein gegebenenfalls ein- oder mehrfach mit Halogen und/oder Hydroxy substituierter C₁-C₆-Alkylrest, ein Halogen, Cyano oder ein gegebenenfalls ein- oder mehrfach mit Halogen und/oder Hydroxy substituierter C₃-C₆-Cycloalkylrest ist.
Bevorzugt ist R³ ein unsubstituierter oder ein- oder mehrfach mit Hydroxy und/oder Halogen substituierter C₁-C₃-Alkylrest.

Besonders bevorzugte C₁-C₃-Alkylreste für R³ im Sinne der Erfindung sind Methyl und Ethyl. Bevorzugt ist R³ ein gegebenenfalls einfach mit Hydroxy und/ oder ein- bis dreifach mit Fluor substituierter C₁-C₆-Alkylrest.
Besonders bevorzugt ist R³ ein gegebenenfalls einfach mit Hydroxy und/ oder ein- bis dreifach mit Fluor substituierter C₁-C₃-Alkylrest.
Bevorzugte substituierte C₁-C₃-Alkylreste für R³ sind Trifluor-C₁-C₃-alkyl, Hydroxymethyl, 1-Hydroxyethyl, 2-Hydroxypropan-2-yl und 2,2,2-Trifluor-1-hydroxyethyl. Hierbei ist ein Trifluormethylrest besonders bevorzugt.
Außerdem ist für R³ ein Cyclopropylrest bevorzugt.

Beispielhaft für W seien hier genannt:
1-Ethyl-1H-pyrazol-3-yl, 2,4'-Bipyridin-6-yl, 2-(4-Fluorphenyl)-1,3-thiazol-4-yl, 2-(4-Methoxyphenyl)-1,3-thiazol-4-yl, 2-(Azetidin-3-ylamino)-1,3-thiazol-4-yl, 2-(Pyridin-3-yl)-1,3-thiazol-4-yl, 2-(Pyridin-4-yl)-1,3-thiazol-4-yl, 2-(Trifluormethyl)-1,3-thiazol-4-yl, 2-Brom-1,3-thiazol-4-yl, 2-Cyclopropyl-1,3-oxazol-4-yl, 2-Methyl-1,3-oxazol-4-yl, 2-Methyl-1,3-oxazol-5-yl, 2-Phenyl-2H-1,2,3-triazol-4-yl, 2-{[1-(tert-Butoxycarbonyl)azetidin-3-yl]amino}-1,3-thiazol-4-yl, 4'-Methyl-2,3'-bipyridin-6-yl, 4-(Trifluormethyl)-1,3-thiazol-2-yl, 5'-Methyl-2,3'-bipyridin-6-yl, 5-Fluor-6-(1-methyl-1H-pyrazol-4-yl)pyridin-2-yl, 5-Fluorpyridin-2-yl, 6'-Acetamido-2,3'-bipyridin-6-yl, 6'-Amino-2,3'-bipyridin-6-yl, 6'-Methoxy-2,3'-bipyridin-6-yl, 6'-Methyl-2,3'-bipyridin-6-yl, 6-(1,3-Dimethyl-1H-pyrazol-4-yl)pyridin-2-yl, 6-(1-Methyl-1H-pyrazol-4-yl)pyridin-2-yl, 6-(1-Methyl-1H-pyrazol-5-yl)pyridin-2-yl, 6-(1H-1,2,4-Triazol-1-yl)pyridin-2-yl, 6-(1H-Pyrazol-1-yl)pyridin-2-yl, 6-(3-Hydroxyazetidin-1-yl)pyridin-2-yl, 6-(3-Methyl-1H-pyrazol-4-yl)pyridin-2-yl, 6-(4-Chlor-1H-pyrazol-1 -yl)pyridin-2-yl, 6-(4H-1,2,4-Triazol-4-yl)pyridin-2-yl, 6-(Azetidin-3 -ylamino)-pyridin-2-yl, 6-(Cyclopropylmethoxy)pyridin-2-yl, 6-(Dimethylamino)pyridin-2-yl, 6-(Morpholin-4-yl)pyridin-2-yl, 6-(Morpholin-4-yl)pyridin-2-yl, 6-(Trifluormethyl)pyridin-2-yl, 6-[1-Hydroxyethyl]pyridin-2-yl, 6-[(2R,6S)-2,6-Dimethylmorpholin-4-yl]pyridin-2-yl, 6-[3-(Methylsulfonyl)-phenyl]pyridin-2-yl, 6-[3-(Trifluormethyl)-1H-pyrazol-4-yl]pyridin-2-yl, 6-Acetamidopyridin-2-yl, 6-Aminopyridin-2-yl, 6-Brompyridin-2-yl, 6-Brompyridin-2-yl, 6-Chlorpyridin-2-yl, 6-Cyclo-propylpyridin-2-yl, 6-Ethoxypyridin-2-yl, 6-Ethylpyridin-2-yl, 6-Fluorpyridin-2-yl, 6-Methoxy-pyridin-2-yl, 6-Methylpyridin-2-yl, 6-{[(2S)-Azetidin-2-ylmethyl]amino}pyridin-2-yl und 6-(2-Hydroxypropan-2-yl)pyridin-2-yl.

Bevorzugt für W sind folgende Reste:
2-(4-Fluorphenyl)-1,3-thiazol-4-yl, 2-(4-Methoxyphenyl)-1,3-thiazol-4-yl, 2-(Azetidin-3-ylamino)-1,3-thiazol-4-yl, 2-(Pyridin-4-yl)-1,3-thiazol-4-yl, 2-Cyclopropyl-1,3-oxazol-4-yl, 5-Fluor-6-(1-methyl-1H-pyrazol-4-yl)pyridin-2-yl, 6-(1-Methyl-1H-pyrazol-4-yl)pyridin-2-yl, 6-(1H-1,2,4-Triazol-1-yl)pyridin-2-yl, 6-(1H-Pyrazol-1 -yl)pyridin-2-yl, 6-(3-Methyl-1H-pyrazol-4-yl)pyridin-2-yl, 6-(Azetidin-3-ylamino)pyridin-2-yl, 6-(Dimethylamino)pyridin-2-yl, 6-(Morpholin-4-yl)-pyridin-2-yl, 6-(Trifluormethyl)pyridin-2-yl, 6-[1-Hydroxyethyl]pyridin-2-yl, 6-[3-(Trifluormethyl)-1H-pyrazol-4-yl]pyridin-2-yl, 6-Cyclopropylpyridin-2-yl, 6-Methylpyridin-2-yl und 6-(2-Hydroxypropan-2-yl)pyridin-2-yl.

Wenn W für eine Gruppe der allgemeinen Formel (IX) steht und m = 1 ist, dann befindet sich R⁴ bevorzugt in ortho-Position zu R³:

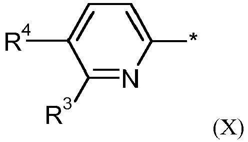

Wenn W für eine Gruppe der allgemeinen Formel (X) steht, dann ist R⁴ bevorzugt ein Wasserstoff, ein C₁-C₃-Alkyl, Fluor, Chlor, Brom, Cyano oder Trifluormethyl.

Besonders bevorzugt steht W für eine Gruppe der allgemeinen Formel (X), in der R⁴ ein Wasserstoff ist.

Besonders bevorzugt für W sind folgende Reste:
6-(1 -Methyl-1H-pyrazol-4-yl)pyridin-2-yl, 6-(1H-1,2,4-Triazol-1-yl)pyridin-2-yl, 6-(1H-Pyrazol-1-yl)pyridin-2-yl, 6-(3-Methyl-1H-pyrazol-4-yl)pyridin-2-yl, 6-(Azetidin-3-ylamino)pyridin-2-yl, 6-(Dimethylamino)pyridin-2-yl, 6-(Morpholin-4-yl)pyridin-2-yl, 6-(Trifluormethyl)pyridin-2-yl, 6-[1-Hydroxyethyl]pyridin-2-yl, 6-[3-(Trifluormethyl)-1H-pyrazol-4-yl]pyridin-2-yl, 6-Cyclopropylpyridin-2-yl, 6-Methylpyridin-2-yl und 6-(2-Hydroxypropan-2-yl)pyridin-2-yl.

Des Weiteren sind Verbindungen bevorzugt, bei denen R¹ Wasserstoff, Halogen, Hydroxy, Cyano, C₁-C₆-Alkoxy, C₁-C₆-Alkyl, C₃-C₈-Cycloalkyl, Aryloxy oder Heteroaryloxy ist.
C₁-C₆-Alkoxy im Sinne von R¹ ist in einer bevorzugten Ausführungsform ein Methoxy, Ethoxy, iso-Propoxy oder auch iso-Butoxy. C₁-C₆-Alkoxy kann ein- oder mehrfach substituiert sein, bevorzugt mit einem oder mehreren Halogenen oder auch mit einem ein- oder mehrfach, gleich oder verschieden mit Halogen substituiertem C₃-C₈-Cycloalkyl.
Wenn für R¹ ein ein- oder mehrfach halogeniertes C₁-C₆-Alkoxy steht, ist hierbei Fluor bevorzugt. Beispielhaft seien hier Trifluormethoxy, Difluormethoxy und 2,2,2-Trifluorethoxy genannt. Trifluormethoxy und 2,2,2-Trifluorethoxy sind ganz besonders bevorzugt.

Wenn R¹ für einen ein- oder mehrfach mit C₃-C₈-Cycloalkyl substituierten C₁-C₆-Alkoxyrest steht, ist ein C₃-C₅-Cycloalkyl, insbesondere ein Cyclopropylrest bevorzugt. Beispielhaft hierfür sei ein Cyclopropylmethoxy genannt.

Wenn R¹ ein mit einer Arylgruppe substituierter C₁-C₆-Alkoxyrest ist, sind Arylgruppen mit 6 Kohlenstoffatomen bevorzugt, zum Beispiel ein Benzyloxy.
Ist R¹ ein mit einer Heteroarylgruppe substituierter C₁-C₆-Alkoxyrest, so sind 6-gliedrige Heteroarylreste bevorzugt. Beispielhaft genannt für R¹ sei hier ein Pyrimidylmethoxyrest.

Weitere bevorzugte Ausführungsformen für R¹ im Sinne von C₁-C₆-Alkyl sind Methyl oder Ethyl. Ist R¹ ein Halogen, so sind Brom, Fluor oder Chlor bevorzugt. Besonders bevorzugt ist Chlor.

Weiterhin kann R¹ ein mit Hydroxy substituierter C₁-C₅-Alkylrest sein. Hierfür sind insbesondere 2-Hydroxypropan-2-yl oder 3-Hydroxypentan-3-yl zu nennen. Bevorzugt ist ein 2-Hydroxypropan-2-ylrest.

Wenn R¹ ein Halogen ist, so sind Fluor, Chlor und Brom bevorzugt. Besonders bevorzugt ist Chlor.

Ein weiterer Gegenstand der vorliegenden Erfindung sind auch Verbindungen der allgemeinen Formel (I), in der W für eine Gruppe der allgemeinen Formel (IX) oder (X) steht und R² Wasserstoff bedeutet.

Ebenfalls Gegenstand vorliegender Erfindung sind Verbindungen der allgemeinen Formel (I), in der W für eine Gruppe der allgemeinen Formel (IX) oder (X) steht und R⁰ Wasserstoff oder Methyl bedeutet.

Ebenfalls Gegenstand vorliegender Erfindung sind Verbindungen der allgemeinen Formel (I), in der W für eine Gruppe der allgemeinen Formel (IX) oder (X) steht und R¹³ Wasserstoff oder Methyl bedeutet.

Ebenfalls Gegenstand vorliegender Erfindung sind Verbindungen der allgemeinen Formel (I), in der W für eine Gruppe der allgemeinen Formel (IX) oder (X) steht und n 0 oder 1 bedeutet.

Ebenfalls Gegenstand vorliegender Erfindung sind Verbindungen der allgemeinen Formel (I), in der W für eine Gruppe der allgemeinen Formel (IX) oder (X) steht und R¹ Wasserstoff, Cyano, Isopropoxy, Trifluormethoxy, 2,2,2-Trifluorethoxy, Isobutoxy, Cyclopropylmethoxy, Pyridin-2-ylmethoxy, Benzyloxy, Brom, Chlor, Ethoxy, Fluor, Hydroxy, Methoxy oder ein 2-Hydroxypropan-2-yl bedeuten.

Ebenfalls Gegenstand vorliegender Erfindung sind Verbindungen der allgemeinen Formel (I), in der W für eine Gruppe der allgemeinen Formel (IX) steht 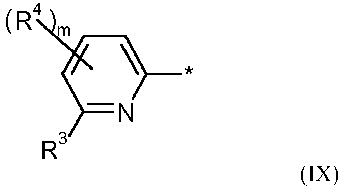 worin m 0 oder 1 bedeutet, R³ ein gegebenenfalls ein- oder mehrfach mit Halogen und/oder Hydroxy substituierter C₁-C₆-Alkylrest, Halogen, Cyano oder ein gegebenenfalls ein- oder mehrfach mit Halogen und/oder Hydroxy substituierter C₃-C₆-Cycloalkylrest ist und R⁴ ein C₁-C₃-Alkylrest, Fluor, Chlor, Brom, Cyano oder Trifluormethyl ist.

Ebenfalls Gegenstand vorliegender Erfindung sind Verbindungen der allgemeinen Formel (I), in der W für eine Gruppe der allgemeinen Formel (X) steht, 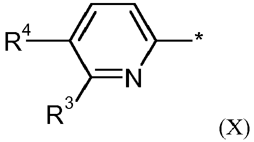 worin R⁴ Wasserstoff, C₁-C₃-Alkyl, Fluor, Chlor, Brom, Cyano oder Trifluormethyl ist und R³ ein gegebenenfalls ein- oder mehrfach mit Halogen und/oder Hydroxy substituierter C₁-C₆-Alkylrest, Halogen, Cyano oder ein gegebenenfalls ein- oder mehrfach mit Halogen und/oder Hydroxy substituierter C₃-C₆-Cycloalkylrest ist.

Besonders bevorzugt sind Verbindungen, in denen W für eine Gruppe der allgemeinen Formel (X) steht, in der R⁴ Wasserstoff bedeutet und R³ ein unsubstituierter oder ein- oder mehrfach mit Hydroxy und/oder Halogen substituierter C₁-C₃-Alkylrest ist.

Ganz besonders bevorzugt sind hier Verbindungen, in denen W für eine Gruppe der allgemeinen Formel (X) steht, in denen R⁴ Wasserstoff bedeutet und R³ ein gegebenenfalls einfach mit Hydroxy und/ oder ein- bis dreifach mit Fluor substituierter C₁-C₃-Alkylrest ist.
Hierfür sind insbesondere Verbindungen bevorzugt, in denen R⁴ Wasserstoff bedeutet und R³ ein Methyl, Ethyl, Trifluor-C₁-C₃-alkyl, Hydroxymethyl, 1-Hydroxyethyl, 2-Hydroxypropan-2-yl oder 2,2,2-Trifluor-1-hydroxyethyl ist.
Insbesondere bevorzugt ist R⁴ Wasserstoff und R³ ein Trifluormethyl- oder ein Cyclopropylrest.

Y steht entweder für einen Rest 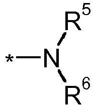 oder für eine Gruppe

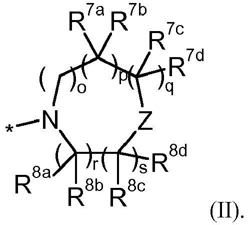

Wenn Y wie oben beschrieben NR⁵R⁶ bedeutet, ist R⁵ bevorzugt ein C₁-C₆-Alkyl, besonders bevorzugt ein Methyl oder Ethyl.
R⁶ ist ebenfalls ein C₁-C₆-Alkyl, das gegebenenfalls ein- oder mehrfach subsituiert sein kann, bevorzugt mit C₃-C₁₀-Cycloalkyl.
Besonders bevorzugt für R⁶ sind Methyl oder Ethyl, die gegebenenfalls mit C₃-C₁₀-Cycloalkyl substituiert sind. Cyclopropyl ist hier besonders bevorzugt.
Beispielhaft wird hierfür ein Cyclopropylmethyl genannt.

Weitere bevorzugte Ausführungsformen für R⁶ sind C₃-C₁₀-Cycloalkyl, Heterocycloalkyl, 5- oder 6-gliedriges Heteroaryl oder Aryl.
Besonders bevorzugt sind hier Pyridazinyl, Phenyl, Oxazolyl, Piperidinyl und Cyclohexyl.

Für den Fall, dass Y ein NR⁵R⁶ bedeutet, seien beispielhaft folgende Reste für Y genannt: (3-Sulfamoylphenyl)amino, [(3R)-Piperidin-3-ylamino]ethyl, 1,2-Oxazol-4-ylamino, [3-(Dimethylsulfamoyl)phenyl]amino, [trans-4-(2-Hydroxypropan-2-yl)cyclohexyl]amino, Pyridazin-4-ylamino, (Cyclopropylmethyl)(methyl)amino.

Wenn Y für eine Gruppe der allgemeinen Formel (II) 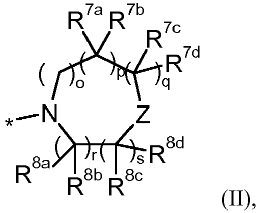 steht, mit
o = 0, 1 oder 2;
p = 0, 1, 2 oder 3;
q = 0 oder 1,
wobei die Summe aus o, p und q = 1, 2 oder 3 ist; und
r = 0 oder 1 bedeutet;
s = 0 oder 1 bedeutet; und
Z bedeutet CR⁹R¹⁰, NR¹¹, O, S oder S(=O)₂,
dann ist für p entweder 0 oder 1 oder 2 bevorzugt.

Insbesondere bevorzugt sind hierbei Verbindungen, in denen Y für eine Gruppe der allgemeinen Formel (II) steht 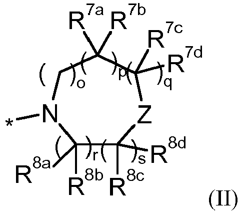 in der
o = 0 bedeutet,
p = 0 oder 1 bedeutet,
q, r und s = 1 bedeuten und
Z bedeutet CR⁹R¹⁰, NR¹¹, O, S oder S(=O)₂.

Wenn Y eine Gruppe der oben genannten allgemeinen Formel (II) bedeutet, seien beispielhaft folgende Gruppen genannt:
4-Benzoylpiperazin-1-yl, 4-(Pyrrolidin-1-yl)piperidin-1-yl, 4-(3-Hydroxy-2,2-dimethylpropanoyl)-piperazin-1-yl,4-(Methoxyacetyl)piperazin-1-yl, 4-(2-Hydroxypropan-2-yl)piperidin-1-yl, 4-(Cyclopropylmethyl)piperazin-1-yl, 4-Methylpiperazin-1-yl, 4-(Cyclopropylcarbonyl)piperazin-1-yl, Morpholin-4-yl, 4-(Ethoxycarbonyl)piperazin-1-yl, 3-(Dimethylamino)azetidin-1-yl, 3-(Piperidin-1-yl)azetidin-1-yl, 2- [4-(2-Hydroxy-2-methylpropyl)piperidin-1 -yl, 4-Hydroxy-1,4'-bipiperidin-1 '-yl, 4-(Dimethylamino)piperidin-1-yl, 4-(2,2,2-Trifluorethyl)piperazin-1-yl, 4-Ethyl-3-oxopiperazin-1-yl, 4-(4-Fluorbenzoyl)piperazin-1-yl, 4-(Pyridin-2-yl)piperazin-1-yl, 4-Cyclopentyl-3-oxopiperazin-1-yl, 3-Oxo-4-phenylpiperazin-1-yl, 4-(2,2-Dimethylpropanoyl)piperazin-1-yl, 4-(2-Hydroxy-2-methylpropanoyl)piperazin-1-yl, 4-(1-Phenylethyl)piperazin-1-yl]ethyl, 4-(Pyridin-3-ylcarbonyl)piperazin-1-yl, 4-Isonicotinoylpiperazin-1-yl, 4-(Morpholin-4-ylcarbonyl)-piperazin-1-yl,4-[2-(Methylamino)-2-oxoethyl]piperazin-1-yl, 4-(Pyrazin-2-yl)piperazin-1-yl, 4-(1-Hydroxyethyl)piperidin-1-yl, 2-(2-Methyl-2,8-diazaspiro[4.5]dec-8-yl, 6-Acetyl-2,6-diazaspiro[3.3]hept-2-yl, 3-Oxo-2,8-diazaspiro[4.5]dec-8-yl)ethyl, 6-Methyl-2,6-diazaspiro[3.5]-non-2-yl, 7-Oxa-2-azaspiro[3.5]non-2-yl, 1,4'-Bipiperidin-1'-yl, 2-[2-(Hydroxymethyl)piperidin-1-yl, 3-(Hydroxymethyl)piperidin-1-yl, 4-Carbamoylpiperidin-1-yl, 3-(Dimethylamino)piperidin-1-yl, 3-(Morpholin-4-ylmethyl)piperidin-1-yl, 4-[(Cyclopropylcarbonyl)amino]piperidin-1-yl, 4-[(5-Cyclopropyl-1,2,4-oxadiazol-3-yl)methyl]piperidin-1-yl, 4-(Pyrrolidin-1-ylcarbonyl)-piperidin-1-yl, 4-(4-Methylpiperazin-1-yl)piperidin-1-yl, 4-[2-(Morpholin-4-yl)ethyl]piperidin-1-yl, 4-[(5-Methyl-1,2,4-oxadiazol-3 -yl)methyl]piperidin-1 -yl, 3-(Pyrrolidin-1-ylmethyl)piperidin-1-yl, 4-(Methylsulfonyl)piperidin-1-yl, 4-[2-Oxo-2-(pyrrolidin-1-yl)ethyl]piperazin-1-yl, 4-(Phenylsulfonyl)piperidin-1-yl, 4-[Isonicotinoyl(methyl)amino]piperidin-1-yl, 4-[2-(Isopropylamino)-2-oxoethyl]piperazin-1-yl, 4-(1,1-Dioxidotetrahydrothiophen-3-yl)piperazin-1-yl, 4-[(Meth oxy-acetyl)(methyl)amino]piperidin-1-yl, 4-(Cyclohexylcarbonyl)piperazin-1-yl, 4-[2-(Cyclopropyl-amino)-2-oxoethyl]piperazin-1-yl, 2-Hydroxyethyl)piperidin-1-yl, 4-(1H-Pyrrol-1-yl)piperidin-1-yl, 4-(3-Hydroxypropyl)piperazin-1-yl, 4-Carbamoylpiperazin-1-yl, 4-(2-Oxopyrrolidin-1-yl)-piperidin-1-yl, 4-(2-Amino-2-oxoethyl)piperazin-1-yl, 1,1-Dioxidothiomorpholin-4-yl, 4-Isopropylpiperazin-1-yl, 4-(2-Thienylcarbonyl)piperazin-1-yl, 2-Cyclopropyl-2-oxoethyl)piperazin-1-yl, 4-[(1-Methyl-1H-pyrazol-4-yl)methyl]piperazin-1-yl, 4-[(1,5-Dimethyl-1H-pyrazol-3-yl)-carbonyl]piperazin-1-yl, 4-(Diethylcarbamoyl)piperazin-1-yl, Thiomorpholin-4-yl, 4-(2-Furyl-methyl)piperazin-1-yl, 4-(3-Thienylmethyl)piperazin-1-yl, 4'-Methyl-1,4'-bipiperidin-1'-yl, 6-Methyl-2,6-diazaspiro[3.3]hept-2-yl, 4-Cyclopentylpiperazin-1-yl, 4-[2-(2-Hydroxyethoxy)-ethyl]piperazin-1-yl, 4-(Pyridin-4-ylmethyl)piperazin-1-yl, 4-(Dimethylsulfamoyl)piperazin-1-yl, 4-(Pyridin-4-yl)piperazin-1-yl, 4-(Methylsulfonyl)piperazin-1-yl, {4-[2-(1H-Imidazol-1-yl)ethyl] piperazin-1-yl,4-(Diethylsulfamoyl)piperazin-1-yl, 4-(Pyridin-3-yl)piperazin-1-yl, 4-(Piperidin-1-ylsulfonyl)piperazin-1-yl, 4-[(1,5-Dimethyl-1H-pyrazol-4-yl)sulfonyl, 4-Ethylpiperazin-1-yl, 4-Methyl-4- [(4-methylpiperazin-1 -yl)carbonyl]piperidin-1 -yl, 4-(Cyclobutylcarbonyl)piperazin-1-yl, 4-(Cyclopentylcarbonyl)piperazin-1-yl, 4-[3-(Methylsulfonyl)benzoyl]piperazin-1-yl und 4-[2-Methoxy-5-(methylsulfonyl)benzoyl]piperazin-1-yl.

Insbesondere bevorzugt ist Y ein 4-Methylpiperazin-1-yl, ein 4-Ethylpiperazin-1-yl oder ein Morpholin-4-yl.

Ein bevorzugter Gegenstand vorliegender Erfindung sind Verbindungen der allgemeinen Formel (I), in der W für eine Gruppe der allgemeinen Formel (IX) steht, 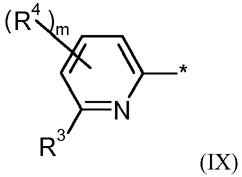 worin
m für 0 steht und R², R⁰ sowie R¹³ gleichzeitig Wasserstoff sowie R³ Trifluormethyl, Ethyl, Methyl, Cyclopropyl, 2,2,2-Trifluor-1-hydroxyethyl oder 1-Hydroxyethyl bedeuten; Y für 4-Methylpiperazin-1-yl, 4-Ethylpiperazin-1-yl oder Morpholin-4-yl steht, n für 0 und R¹ für Cyclopropylmethoxy, Trifluormethoxy, 2,2,2-Trifluorethoxy, Chlor, Ethoxy oder Methoxy steht.
Besonders bevorzugt steht R¹ hier für Cyclopropylmethoxy, Methoxy oder Ethoxy.
Besonders bevorzugt sind hierbei Verbindungen, in denen R³ ein Trifluormethyl- oder ein Cyclopropylrest ist.

Ebenfalls Gegenstand der vorliegenden Erfindung sind die folgenden Verbindungen:
N- {2-[2-(4-Benzoylpiperazin-1-yl)-2-oxoethyl]-6-methyl-2H-indazol-5-yl}-6-(trifluormethyl)pyridin-2-carboxamid
6-Ethyl-N-(6-methyl-2-{2-oxo-2-[4-(pyrrolidin-1-yl)piperidin-1-yl]ethyl}-2H-indazol-5-yl)pyridin-2-carboxamid
5-Fluor-N-(6-methyl-2-{2-oxo-2-[4-(pyrrolidin-1-yl)piperidin-1-yl]ethyl}-2H-indazol-5-yl)pyridin- 2-carboxamid
N-(2-{2-[4-(3-Hydroxy-2,2-dimethylpropanoyl)piperazin-1-yl]-2-oxoethyl}-6-methyl-2H-indazol-5-yl)-6-(trifluormethyl)pyridin-2-carboxamid
N-(2- {2-[4-(Methoxyacetyl)piperazin-1-yl]-2-oxoethyl}-6-methyl-2H-indazol-5-yl)-6-(trifluormethyl)pyridin-2-carboxamid
N-(2- {2-[4-(2-Hydroxypropan-2-yl)piperidin-1-yl]-2-oxoethyl}-6-methoxy-2H-indazol-5-yl)-6-(trifluormethyl)pyridin-2-carboxamid
N-(2- {2-[4-(2-Hydroxypropan-2-yl)piperidin-1-yl]-2-oxoethyl}-6-methoxy-2H-indazol-5-yl)-6-methylpyridin-2-carboxamid
N-(2-{2-[4-(Cyclopropylmethyl)piperazin-1-yl]-2-oxoethyl}-6-methoxy-2H-indazol-5-yl)-6-(trifluormethyl)pyridin-2-carboxamid
N-(2-{2-[4-(Cyclopropylmethyl)piperazin-1-yl]-2-oxoethyl}-6-methoxy-2H-indazol-5-yl)-6-methylpyridin-2-carboxamid
N-{2-[2-(4-Benzoylpiperazin-l-yl)-2-oxoethyl]-6-methoxy-2H-indazol-5-yl}-6-cyclopropylpyridin-2-carboxamid
N-{2-[2-(4-Benzoylpiperazin-1-yl)-2-oxoethyl]-6-methoxy-2H-indazol-5-yl}-6-(1-hydroxyethyl)pyridin- 2-carboxamid
N-{2-[2-(4-Methylpiperazin-1-yl)-2-oxoethyl]-6-(trifluormethoxy)-2H-indazol-5-yl}-6-(trifluormethyl)pyridin-2-carboxamid
6-Methyl-N-{2-[2-(4-methylpiperazin-1-yl)-2-oxoethyl]-6-(trifluormethoxy)-2H-indazol-5-yl}pyridin-2-carboxamid
tert-Butyl-3-{[4-({2-[2-(4-methylpiperazin-1-yl)-2-oxoethyl]-6-(trifluormethoxy)-2H-indazol-5-yl}carbamoyl)-1,3-thiazol-2-yl]amino}azetidin-1-carboxylat
N-{6-Brom-2-[2-(4-methylpiperazin-1-yl)-2-oxoethyl]-2H-indazol-5-yl}-6-(trifluormethyl)pyridin-2-carboxamid
N- {6-Brom-2- [2-(4-methylpiperazin-1 -yl)-2-oxoethyl] -2H-indazol-5-yl} -6-methylpyridin-2-carboxamid
N-{6-Brom-2-[2-(4-methylpiperazin-1-yl)-2-oxoethyl]-2H-indazol-5-yl}-2-cyclopropyl-1,3-oxazol-4-carboxamid
tert-Butyl-3-{[4-({6-brom-2-[2-(4-methylpiperazin-1-yl)-2-oxoethyl]-2H-indazol-5-yl}carbamoyl)-1,3 -thiazol-2-yl] amino} azetidin-1 -carboxylat
2-(Azetidin-3-ylamino)-N-{2-[2-(4-methylpiperazin-1-yl)-2-oxoethyl]-6-(trifluormethoxy)-2H-indazol-5-yl}-1,3-thiazol-4-carboxamid
N-{6-Cyan-2-[2-(4-methylpiperazin-1-yl)-2-oxoethyl]-2H-indazol-5-yl}-6-(trifluormethyl)pyridin-2-carboxamid
6'-Methyl-N-{2-[2-(4-methylpiperazin-1-yl)-2-oxoethyl]-2H-indazol-5-yl}-2,3'-bipyridin-6-carboxamid
5'-Methyl-N- {2-[2-(4-methylpiperazin-1-yl)-2-oxoethyl]-2H-indazol-5-yl}-2,3'-bipyridin-6-carboxamid
4'-Methyl-N- {2-[2-(4-methylpiperazin-1-yl)-2-oxoethyl]-2H-indazol-5-yl}-2,3'-bipyridin-6-carboxamid
6'-Methoxy-N-{2-[2-(4-methylpiperazin-1-yl)-2-oxoethyl]-2H-indazol-5-yl}-2,3'-bipyridin-6-carboxamid
6'-Acetamido-N-{2-[2-(4-methylpiperazin-1-yl)-2-oxoethyl]-2H-indazol-5-yl}-2,3'-bipyridin-6-carboxamid
N-{2-[2-(4-Methylpiperazin-1-yl)-2-oxoethyl]-2H-indazol-5-yl}-6'-nitro-2,3'-bipyridin-6-carboxamid
6'-Amino-N-{2-[2-(4-methylpiperazin-1-yl)-2-oxoethyl]-2H-indazol-5-yl}-2,3'-bipyridin-6-carboxamid
N-{2-[2-(4-Benzoylpiperazin-1-yl)-2-oxoethyl]-6-fluor-2H-indazol-5-yl}-6-(trifluormethyl)pyridin-2-carboxamid
N-(2-{2-[4-(Cyclopropylcarbonyl)piperazin-1-yl]-2-oxoethyl}-6-fluor-2H-indazol-5-yl)-6-(trifluormethyl)pyridin-2-carboxamid
N-{6-Fluor-2-[2-(4-methylpiperazin-1-yl)-2-oxoethyl]-2H-indazol-5-yl}-6-(trifluormethyl)pyridin-2-carboxamid
N-(2-{2-[4-(Cyclopropylcarbonyl)piperazin-1-yl]-2-oxoethyl}-6-fluor-2H-indazol-5-yl)-6-methylpyridin-2-carboxamid
N- {6-Fluor-2-[2-(4-methylpiperazin-1-yl)-2-oxoethyl]-2H-indazol-5-yl}-6-methylpyridin-2-carboxamid
N-(2-{2-[4-(Cyclopropylcarbonyl)piperazin-1-yl]-2-oxoethyl}-6-fluor-2H-indazol-5-yl)-6-(1-methyl-1H-pyrazol-4-yl)pyridin-2-carboxamid
N-{2-[2-(4-Benzoylpiperazin-1-yl)-2-oxoethyl]-6-fluor-2H-indazol-5-yl}-6-(1-methyl-1H-pyrazol-4-yl)pyridin-2-carboxamid
N-{6-Fluor-2-[2-(4-methylpiperazin-1-yl)-2-oxoethyl]-2H-indazol-5-yl}-6-(1-methyl-1H-pyrazol-4-yl)pyridin-2-carboxamid
N-{2-[2-(4-Benzoylpiperazin-1-yl)-2-oxoethyl]-6-fluor-2H-indazol-5-yl}-5-fluor-6-(1-methyl-1H-pyrazol-4-yl)pyridin-2-carboxamid
N-(2-{2-[4-(Cyclopropylcarbonyl)piperazin-1-yl]-2-oxoethyl}-6-fluor-2H-indazol-5-yl)-5-fluor-6-(1-methyl-1H-pyrazol-4-yl)pyridin-2-carboxamid
N-{2-[2-(4-Benzoylpiperazin-1-yl)-2-oxoethyl]-6-fluor-2H-indazol-5-yl}-6-(morpholin-4-yl)pyridin-2-carboxamid
N-(2-{2-[4-(Cyclopropylcarbonyl)piperazin-1-yl]-2-oxoethyl}-6-fluor-2H-indazol-5-yl)-6-(morpholin-4-yl)pyridin-2-carboxamid
N-{6-Fluor-2-[2-(4-methylpiperazin-1-yl)-2-oxoethyl]-2H-indazol-5-yl}-6-(morpholin-4-yl)pyridin-2-carboxamid
N-{6-(Benzyloxy)-2-[2-(4-methylpiperazin-1-yl)-2-oxoethyl]-2H-indazol-5-yl}-6-(trifluormethyl)pyridin-2-carboxamid
N-{6-Isobutoxy-2-[2-(4-methylpiperazin-1-yl)-2-oxoethyl]-2H-indazol-5-yl}-6-(trifluormethyl)pyridin-2-carboxamid
N-{6-Isobutoxy-2-[2-(morpholin-4-yl)-2-oxoethyl]-2H-indazol-5-yl}-6-(trifluormethyl)pyridin-2-carboxamid
N-(2-{2-[4-(2-Hydroxypropan-2-yl)piperidin-1-yl]-2-oxoethyl}-6-isobutoxy-2H-indazol-5-yl)-6-(trifluormethyl)pyridin-2-carboxamid
N-(2-{2-[(Cyclopropylmethyl)(methyl)amino]-2-oxoethyl}-6-isobutoxy-2H-indazol-H-yl)-6-(trifluormethyl)pyridin-2-carboxamid
N-{6-(Cyclopropylmethoxy)-2-[2-(4-methylpiperazin-1-yl)-2-oxoethyl]-2H-indazol-5-yl}-6-(trifluormethyl)pyridin-2-carboxamid
N-{6-(Cyclopropylmethoxy)-2-[2-(morpholin-4-yl)-2-oxoethyl]-2H-indazol-5-yl}-6-(trifluormethyl)pyridin-2-carboxamid
N-[6-(Cyclopropylmethoxy)-2-{2-[4-(2-hydroxypropan-2-yl)piperiin-1-yl]-2-oxoethyl}-2H-indazol-5-yl]-6-(trifluormethyl)pyridin-2-carboxamid
N-[6-(Cyclopropylmethoxy)-2-{2-[(cyclopropylmethyl)(methyl)amino]-2-oxoethyl}-2H-indazol-5-yl]-6-(trifluormethyl)pyridin-2-carboxamid
N-{2-[2-(4-Methylpiperazin-1-yl)-2-oxoethyl]-6-(pyridin-2-ylmethoxy)-2H-indazol-5-yl}-6-(trifluormethyl)pyridin-2-carboxamid
N-{2-[2-(Morpholin-4-yl)-2-oxoethyl]-6-(pyridin-2-ylmethoxy)-2H-indazol-5-yl}-6-(trifluormethyl)pyridin-2-carboxamid
N-[2-{2-[4-(2-Hydroxypropan-2-yl)piperidin-1-yl]-2-oxoethyl}-6-(pyridin-2-ylmethoxy)-2H-indazol-5-yl]-6-(trifluormethyl)pyridin-2-carboxamid
N-[2-{2-[(Cyclopropylmethyl)(methyl)amino]-2-oxoethyl}-6-(pyridin-2-ylmethoxy)-2H-indazol-5-yl]-6-(trifluormethyl)pyridin-2-carboxamid
N-{2-[2-(4-Benzoylpiperazin-1-yl)-2-oxoethyl]-6-chlor-2H-indazol-5-yl}-6-(trifluormethyl)pyridin-2-carboxamid
N-{6-Chlor-2-[2-(morpholin-4-yl)-2-oxoethyl]-2H-indazol-5-yl}-6-(trifluormethyl)pyridin-2-carboxamid
Ethyl-4-{[6-chlor-5-({[6-(trifluormethyl)pyridin-2-yl]carbonyl}amino)-2H-indazol-2-yl] acetyl}piperazin-1 -carboxylat
N-(6-Chlor-2-{2-oxo-2-[4-(pyrrolidin-1-yl)piperidin-1-yl]ethyl}-2H-indazol-5-yl)-6-(trifluormethyl)pyridin-2-carboxamid
N-(6-Chlor-2-{2-[4-(2-hydroxypropan-2-yl)piperidin-1-yl]-2-oxoethyl}-2H-indazol-5-yl)-6-(trifluormethyl)pyridin-2-carboxamid
N-(6-Chlor-2-{2-[4-(3-hydroxy-2,2-dimethylpropanoyl)piperazin-1-yl]-2-oxoethyl}-2H-indazol-5-yl)-6-(trifluormethyl)pyridin-2-carboxamid
N-(6-Chlor-2-{2-[3-(dimethylamino)azetidin-1-yl]-2-oxoethyl}-2H-indazol-5-yl)-6-(trifluormethyl)pyridin-2-carboxamid
N-(6-Chlor-2- {2-oxo-2-[3-(piperidin-1-yl)azetidin-1-yl]ethyl}-2H-indazol-5-yl)-6-(trifluormethyl)pyridin-2-carboxamid
N-(6-Chlor-2-{2-[4-(2-hydroxy-2-methylpropyl)piperidin-1-yl]-2-oxoethyl}-2H-indazol-5-yl)-6-(trifluormethyl)pyridin-2-carboxamid
N- {6-Chlor-2-[2-(4-hydroxy-1,4'-bipiperidin-1'-yl)-2-oxoethyl]-2H-indazol-5-yl}-6-(trifluormethyl)pyridin-2-carboxamid
N-{6-Methoxy-2-[2-(4-methylpiperazin-1-yl)-2-oxoethyl]-2H-indazol-5-yl}-6-(trifluormethyl)pyridin-2-carboxamid
N-{6-Methoxy-2-[2-(morpholin-4-yl)-2-oxoethyl]-2H-indazol-5-yl}-6-(trifluormethyl)pyridin-2-carboxamid
N-(2-{2-[4-(Dimethylamino)piperidin-1-yl]-2-oxoethyl}-6-ethoxy-2H-indazol-5-yl)-6-(trifluormethyl)pyridin-2-carboxamid
N-(6-Ethoxy-2- {2-oxo-2-[-yl)piperidin-1-yl]ethyl}-2H-indazol-5-yl)-6-(trifluormethyl)pyridin-2-carboxamid
N-{6-Ethoxy-2-[2-(4-methylpiperazin-1-yl)-2-oxoethyl]-2H-indazol-5-yl}-6-(trifluormethyl)pyridin-2-carboxamid
N-{2-[2-(4-Benzoylpiperazin-1-yl)-2-oxoethyl]-6-ethoxy-2H-indazol-5-yl}-6-(trifluormethyl)pyridin-2-carboxamid
N-(6-Ethoxy-2- {2-[4-(2-hydroxypropan-2-yl)piperidin-1-yl]-2-oxoethyl}-2H-indazol-5-yl)-6-(trifluormethyl)pyridin-2-carboxamid
N-{6-Ethoxy-2-[2-(morpholin-4-yl)-2-oxoethyl]-2H-indazol-5-yl}-6-(trifluormethyl)pyridin-2-carboxamid
N-{2-[2-(4-Benzoylpiperazin-1-yl)-2-oxoethyl]-3-methyl-2H-indazol-5-yl}-6-(trifluormethyl)pyridin-2-carboxamid
N-{2-[3-(4-Benzoylpiperazin-1-yl)-3-oxopropyl]-2H-indazol-5-yl}-6-(trifluormethyl)pyridin-2-carboxamid
N-(2-{2-[4-(Cyclopropylcarbonyl)piperazin-1-yl]-2-oxoethyl}-2H-indazol-5-yl)-2-(pyridin-3-yl)-1,3-thiazol-4-carboxamid
N-(2- {2-[4-(Cyclopropylcarbonyl)piperazin-1-yl]-2-oxoethyl}-2H-indazol-5-yl)-2-(pyridin-4-yl)-1,3-thiazol-4-carboxamid
N-(2-{2-[4-(Cyclopropylcarbonyl)piperazin-1-yl]-2-oxoethyl}-2H-indazol-5-yl)-6-(trifluormethyl)pyridin-2-carboxamid
6-(Azetidin-3-ylamino)-N-(2-{2-[4-(cyclopropylcarbonyl)piperazin-1-yl]-2-oxoethyl}-2H-indazol-5-yl)pyridin-2-carboxamid
N-{2-[2-(4-Methylpiperazin-1-yl)-2-oxoethyl]-2H-indazol-5-yl}-2-(pyridin-3-yl)-1,3-thiazol-4-carboxamid
N-{2-[2-(4-Methylpiperazin-1-yl)-2-oxoethyl]-2H-indazol-5-yl}-6-(1-methyl-1H-pyrazol-4-yl)pyridin-2-carboxamid
N-{2-[2-(4-Methylpiperazin-1-yl)-2-oxoethyl]-2H-indazol-5-yl}-6-(1H-pyrazol-4-yl)pyridin-2-carboxamid
6-(1,3-Dimethyl-1H-pyrazol-4-yl)-N-{2-[2-(4-methylpiperazin-1-yl)-2-oxoethyl]-2H-indazol-5-yl}pyridin-2-carboxamid
N- {2-[2-(4-Methylpiperazin-1-yl)-2-oxoethyl]-2H-indazol-5-yl}-6-[3-(trifluormethyl)-1H-pyrazol-4-yl]pyridin-2-carboxamid
6-Ethyl-N-{2-[2-(4-methylpiperazin-1-yl)-2-oxoethyl]-2H-indazol-5-yl}pyridin-2-carboxamid
6-(1-Methyl-1H-pyrazol-4-yl)-N-(2-{2-oxo-2-[4-(2,2,2-trifluorethyl)piperazin-1-yl]ethyl}-2H-indazol-5-yl)pyridin-2-carboxamid
N-(2-{2-Oxo-2-[4-(2,2,2-trifluorethyl)piperazin-1-yl]ethyl}-2H-indazol-5-yl)-6-(trifluormethyl)pyridin-2-carboxamid
N-{2-[2-(4-Ethyl-3-oxopiperazin-1-yl)-2-oxoethyl]-2H-indazol-5-yl}-6-(1-methyl-1H-pyrazol-4-yl)pyridin-2-carboxamid
N-{2-[2-(4-Benzoylpiperazin-1-yl)-2-oxoethyl]-2H-indazol-5-yl}-6-(trifluormethyl)pyridin-2-carboxamid
N-{2-[2-(4-Benzoylpiperazin-1-yl)-2-oxoethyl]-2H-indazol-5-yl}-6-methylpyridin-2-carboxamid
N-{2-[2-(4-Benzoylpiperazin-1-yl)-2-oxoethyl]-2H-indazol-5-yl}-6-(morpholin-4-yl)pyridin-2-carboxamid
N-{2-[2-(4-Benzoylpiperazin-1-yl)-2-oxoethyl]-2H-indazol-5-yl}-2-(pyridin-4-yl)-1,3-thiazol-4-carboxamid
N-{2-[2-(4-Benzoylpiperazin-1-yl)-2-oxoethyl]-2H-indazol-5-yl}-6-chlorpyridin-2-carboxamid
N-{2-[2-(4-Benzoylpiperazin-1-yl)-2-oxoethyl]-2H-indazol-5-yl}-2-methyl-1,3-oxazol-5-carboxamid
6-Amino-N-{2-[2-(4-benzoylpiperazin-1-yl)-2-oxoethyl]-2H-indazol-5-yl}pyridin-2-carboxamid
N-{2-[2-(4-Benzoylpiperazin-1-yl)-2-oxoethyl]-2H-indazol-5-yl}-2-methyl-1,3-oxazol-4-carboxamid
N-{2-[2-(4-Benzoylpiperazin-1-yl)-2-oxoethyl]-2H-indazol-5-yl}-6-methoxypyridin-2-carboxamid
N-{2-[2-(4-Benzoylpiperazin-1-yl)-2-oxoethyl]-2H-indazol-5-yl}-2-cyclopropyl-1,3-oxazol-4-carboxamid
N-{2-[2-(4-Benzoylpiperazin-1-yl)-2-oxoethyl]-2H-indazol-5-yl}-6-(4H-1,2,4-triazol-4-yl)pyridin-2-carboxamid
N-{2-[2-(4-Benzoylpiperazin-1-yl)-2-oxoethyl]-2H-indazol-5-yl}-2-phenyl-2H-1,2,3-triazol-4-carboxamid
N-{2-[2-(4-Benzoylpiperazin-1-yl)-2-oxoethyl]-2H-indazol-5-yl}-6-(1-methyl-1H-pyrazol-5-yl)pyridin-2-carboxamid
N-{2-[2-(4-Benzoylpiperazin-1-yl)-2-oxoethyl]-2H-indazol-5-yl}-2-(trifluormethyl)-1,3-thiazol-4-carboxamid
N-{2-[2-(4-Benzoylpiperazin-1-yl)-2-oxoethyl]-2H-indazol-5-yl}-6-(1H-pyrazol-1-yl)pyridin-2-carboxamid
N-{2-[2-(4-Benzoylpiperazin-1-yl)-2-oxoethyl]-2H-indazol-5-yl}-6-(1-methyl-1H-pyrazol-4-yl)pyridin-2-carboxamid
N-{2-[2-(4-Benzoylpiperazin-1-yl)-2-oxoethyl]-2H-indazol-5-yl}-1-ethyl-1H-pyrazol-3-carboxamid
N-{2-[2-(4-Benzoylpiperazin-1-yl)-2-oxoethyl]-2H-indazol-5-yl}-6-(4-chlor-1H-pyrazol-1-yl)pyridin-2-carboxamid
N-{2-[2-(4-Benzoylpiperazin-1-yl)-2-oxoethyl]-2H-indazol-5-yl}-4-(trifluormethyl)-1,3-thiazol-2-carboxamid
N-{2-[2-(4-Benzoylpiperazin-1-yl)-2-oxoethyl]-2H-indazol-5-yl}-6-(1,3-dimethyl-1H-pyrazol-4-yl)pyridin-2-carboxamid
N-{2-[2-(4-Benzoylpiperazin-1-yl)-2-oxoethyl]-2H-indazol-5-yl}-2,4'-bipyridin-6-carboxamid
N-{2-[2-(4-Benzoylpiperazin-1-yl)-2-oxoethyl]-2H-indazol-5-yl}-6-(1H-pyrazol-4-yl)pyridin-2-carboxamid
N-{2-[2-(4-Benzoylpiperazin-1-yl)-2-oxoethyl]-2H-indazol-5-yl}-5-fluor-6-(1-methyl-1H-pyrazol-4-yl)pyridin-2-carboxamid
N-{2-[2-(4-Benzoylpiperazin-1-yl)-2-oxoethyl]-2H-indazol-5-yl}-6-(3-methyl-1H-pyrazol-4-yl)pyridin-2-carboxamid
N-{2-[2-(4-Benzoylpiperazin-1-yl)-2-oxoethyl]-2H-indazol-5-yl}-6-(1H-1,2,4-triazol-1-yl)pyridin-2-carboxamid
N-{2-[2-(4-Benzoylpiperazin-1-yl)-2-oxoethyl]-2H-indazol-5-yl}-6-[3-(trifluormethyl)-1H-pyrazol-4-yl]pyridin-2-carboxamid
N-{2-[2-(4-Benzoylpiperazin-1-yl)-2-oxoethyl]-2H-indazol-5-yl}-6-ethoxypyridin-2-carboxamid
N-{2-[2-(4-Benzoylpiperazin-1-yl)-2-oxoethyl]-2H-indazol-5-yl}-6-(cyclopropylmethoxy)pyridin-2-carboxamid
N-{2-[2-(4-Benzoylpiperazin-1-yl)-2-oxoethyl]-2H-indazol-5-yl}-6-ethylpyridin-2-carboxamid
N-{2-[2-(4-Benzoylpiperazin-1-yl)-2-oxoethyl]-2H-indazol-5-yl}-2-(4-methoxyphenyl)-1,3-thiazol-4-carboxamid
N-{2-[2-(4-Benzoylpiperazin-1-yl)-2-oxoethyl]-2H-indazol-5-yl}-2-brom-1,3-thiazol-4-carboxamid
N-{2-[2-(4-Benzoylpiperazin-1-yl)-2-oxoethyl]-2H-indazol-5-yl}-2-(4-fluorphenyl)-1,3-thiazol-4-carboxamid
N-{2-[2-(4-Benzoylpiperazin-1-yl)-2-oxoethyl]-2H-indazol-5-yl}-6-fluorpyridin-2-carboxamid
N-{2-[2-(4-Benzoylpiperazin-1-yl)-2-oxoethyl]-2H-indazol-5-yl}-6-brompyridin-2-carboxamid
N-(2-{2-[4-(4-Fluorbenzoyl)piperazin-1-yl]-2-oxoethyl}-2H-indazol-5-yl)-6-(trifluormethyl)pyridin-2-carboxamid
N-(2-{2-Oxo-2-[4-(pyridin-2-yl)piperazin-1-yl]ethyl}-2H-indazol-5-yl)-6-(trifluormethyl)pyridin-2-carboxamid
N-(2-{2-[4-(Methoxyacetyl)piperazin-1-yl]-2-oxoethyl}-2H-indazol-5-yl)-6-(trifluormethyl)pyridin-2-carboxamid
N-{2-[2-(4-Cyclopentyl-3-oxopiperazin-1-yl)-2-oxoethyl]-2H-indazol-5-yl}-6-(trifluormethyl)pyridin-2-carboxamid
N-{2-[2-Oxo-2-(3-oxo-4-phenylpiperazin-1-yl)ethyl]-2H-indazol-5-yl}-6-(trifluormethyl)pyridin-2-carboxamid
N-(2-{2-[4-(2,2-Dimethylpropanoyl)piperazin-1-yl]-2-oxoethyl}-2H-indazol-5-yl)-6-(trifluormethyl)pyridin-2-carboxamid
N-(2- {2-[4-(Cyclopropylmethyl)piperazin-1-yl]-2-oxoethyl}-2H-indazol-5-yl)-6-(trifluormethyl)pyridin-2-carboxamid
N-{2-[2-Oxo-2-(pyridazin-4-ylamino)ethyl]-2H-indazol-5-yl}-6-(trifluormethyl)pyridin-2-carboxamid
N-(2-{2-[4-(2-Hydroxy-2-methylpropanoyl)piperazin-1-yl]-2-oxoethyl}-2H-indazol-5-yl)-6-(trifluormethyl)pyridin-2-carboxamid
N-(2-{2-Oxo-2-[4-(1-phenylethyl)piperazin-1-yl]ethyl} -2H-indazol-5-yl)-6-(trifluormethyl)pyridin-2-carboxamid
N-(2-{2-Oxo-2-[4-(pyridin-3-ylcarbonyl)piperazin-1-yl]ethyl}-2H-indazol-5-yl)-6-(trifluormethyl)pyridin-2-carboxamid
N-{2-[2-(4-Isonicotinoylpiperazin-1-yl)-2-oxoethyl]-2H-indazol-5-yl}-6-(trifluormethyl)pyridin-2-carboxamid
N-(2-{2-[4-(Morpholin-4-ylcarbonyl)piperazin-1-yl]-2-oxoethyl}-2H-indazol-5-yl)-6-(trifluormethyl)pyridin-2-carboxamid
N-[2-(2-{4-[2-(Methylamino)-2-oxoethyl]piperazin-1-yl}-2-oxoethyl)-2H-indazol-5-yl]-6-(trifluormethyl)pyridin-2-carboxamid
N-(2-{2-Oxo-2-[4-(pyrazin-2-yl)piperazin-1-yl]ethyl}-2H-indazol-5-yl)-6-(trifluormethyl)pyridin-2-carboxamid
N-(2-{2-[4-(1-Hydroxyethyl)piperidin-1-yl]-2-oxoethyl}-2H-indazol-5-yl)-6-(trifluormethyl)pyridin-2-carboxamid
N-{2-[2-(2-Methyl-2,8-diazaspiro[4.5]dec-8-yl)-2-oxoethyl]-2H-indazol-5-yl}-6-(trifluormethyl)pyridin-2-carboxamid
N-{2-[2-(6-Acetyl-2,6-diazaspiro[3.3]hept-2-yl)-2-oxoethyl]-2H-indazol-5-yl}-6-(trifluormethyl)pyridin-2-carboxamid
N-{2-[2-Oxo-2-(3-oxo-2,8-diazaspiro[4.5]dec-8-yl)ethyl]-2H-indazol-5-yl}-6-(trifluormethyl)pyridin-2-carboxamid
N-{2-[2-(6-Methyl-2,6-diazaspiro[3.5]non-2-yl)-2-oxoethyl]-2H-indazol-5-yl}-6-(trifluormethyl)pyridin-2-carboxamid
N-{2-[2-(7-Oxa-2-azaspiro[3.5]non-2-yl)-2-oxoethyl]-2H-indazol-5-yl}-6-(trifluormethyl)pyridin-2-carboxamid
N-{2-[2-(1,4'-Bipiperidin-1'-yl)-2-oxoethyl]-2H-indazol-5-yl}-6-(trifluormethyl)pyridin-2-carboxamid
N-(2-{2-[2-(Hydroxymethyl)piperidin-1-yl]-2-oxoethyl}-2H-indazol-5-yl)-6-(trifluormethyl)pyridin-2-carboxamid
N-(2-{2-[3-(Hydroxymethyl)piperidin-1-yl]-2-oxoethyl}-2H-indazol-5-yl)-6-(trifluormethyl)pyridin-2-carboxamid
N-{2-[2-(4-Carbamoylpiperidin-1-yl)-2-oxoethyl]-2H-indazol-5-yl}-6-(trifluormethyl)pyridin-2-carboxamid
N-(2-{2-[3-(Dimethylamino)piperidin-1-yl]-2-oxoethyl}-2H-indazol-5-yl)-6-(trifluormethyl)pyridin-2-carboxamid
N-(2-{2-[3-(Morpholin-4-ylmethyl)piperidin-1-yl]-2-oxoethyl}-2H-indazol-5-yl)-6-(trifluormethyl)pyridin-2-carboxamid
N-[2-(2-{4-[(Cyclopropylcarbonyl)amino]piperidin-1-yl}-2-oxoethyl)-2H-indazol-5-yl]-6-(trifluormethyl)pyridin-2-carboxamid
N-(2- {2-[4-(3-Ethyl-1,2,4-oxadiazol-5-yl)piperidin-1-yl]-2-oxoethyl}-2H-indazol-5-yl)-6-(trifluormethyl)pyridin-2-carboxamid
N-[2-(2-{4-[(5-Cyclopropyl-1,2,4-oxadiazol-3-yl)methyl]piperidin-1-yl}-2-oxoethyl)-2H-indazol-5-yl]-6-(trifluormethyl)pyridin-2-carboxamid
N-(2-{2-Oxo-2-[4-(pyrrolidin-1-ylcarbonyl)piperidin-1-yl]ethyl}-2H-indazol-5-yl)-6-(trifluormethyl)pyridin-2-carboxamid
N-(2-{2-[4-(4-Methylpiperazin-1-yl)piperidin-1-yl]-2-oxoethyl}-2H-indazol-5-yl)-6-(trifluormethyl)pyridin-2-carboxamid
N-[2-(2-{4-[2-(Morpholin-4-yl)ethyl]piperidin-1-yl}-2-oxoethyl)-2H-indazol-5-yl]-6-(trifluormethyl)pyridin-2-carboxamid
N-[2-(2-{4-[(5-Methyl-1,2,4-oxadiazol-3-yl)methyl]piperidin-1-yl}-2-oxoethyl)-2H-indazol-5-yl]-6-(trifluormethyl)pyridin-2-carboxamid
N-(2-{2-Oxo-2-[3-(pyrrolidin-1-ylmethyl)piperidin-1-yl]ethyl}-2H-indazol-5-yl)-6-(trifluormethyl)pyridin-2-carboxamid
N-[2-(2-{[3-(Dimethylsulfamoyl)phenyl]amino}-2-oxoethyl)-2H-indazol-5-yl]-6-(trifluormethyl)pyridin-2-carboxamid
N-{2-[2-(1,2-Oxazol-4-ylamino)-2-oxoethyl]-2H-indazol-5-yl}-6-(trifluormethyl)pyridin-2-carboxamid
N-(2-{2-[4-(Methylsulfonyl)piperidin-1-yl]-2-oxoethyl}-2H-indazol-5-yl)-6-(trifluormethyl)pyridin-2-carboxamid
N-[2-(2-Oxo-2-{4-[2-oxo-2-(pyrrolidin-1-yl)ethyl]piperazin-1-yl}ethyl)-2H-indazol-5-yl]-6-(trifluormethyl)pyridin-2-carboxamid
N-(2-{2-Oxo-2-[4-(phenylsulfonyl)piperidin-1-yl]ethyl}-2H-indazol-5-yl)-6-(trifluormethyl)pyridin-2-carboxamid
N-(2-{2-Oxo-2-[(3-sulfamoylphenyl)amino]ethyl}-2H-indazol-5-yl)-6-(trifluormethyl)pyridin-2-carboxamid
N-[2-(2-{4-[Isonicotinoyl(methyl)amino]piperidin-1-yl}-2-oxoethyl)-2H-indazol-5-yl]-6-(trifluormethyl)pyridin-2-carboxamid
N-[2-(2-{4-[2-(Isopropylamino)-2-oxoethyl]piperazin-1-yl}-2-oxoethyl)-2H-indazol-5-yl]-6-(trifluormethyl)pyridin-2-carboxamid
N-(2-{2-[4-(1,1-Dioxidotetrahydrothiophen-3-yl)piperazin-1-yl]-2-oxoethyl}-2H-indazol-5-yl)-6-(trifluormethyl)pyridin-2-carboxamid
N-[2-(2-{4-[(Methoxyacetyl)(methyl)amino]piperidin-1-yl}-2-oxoethyl)-2H-indazol-5-yl]-6-(trifluormethyl)pyridin-2-carboxamid
Ethyl-4- {[5-({[6-(trifluormethyl)pyridin-2-yl]carbonyl}amino)-2H-indazol-2-yl]acetyl}piperazin-1-carboxylat
N-(2-{2-[4-(Cyclohexylcarbonyl)piperazin-1-yl]-2-oxoethyl}-2H-indazol-5-yl)-6-(trifluormethyl)pyridin-2-carboxamid
N-[2-(2-{4-[2-(Cyclopropylamino)-2-oxoethyl]piperazin-1-yl}-2-oxoethyl)-2H-indazol-5-yl]-6-(trifluormethyl)pyridin-2-carboxamid
N-(2-{2-[2-(2-Hydroxyethyl)piperidin-1-yl]-2-oxoethyl}-2H-indazol-5-yl)-6-(trifluormethyl)pyridin-2-carboxamid
N-(2-{2-Oxo-2-[4-(pyrrolidin-1-yl)piperidin-1-yl]ethyl}-2H-indazol-5-yl)-6-(trifluormethyl)pyridin-2-carboxamid
N-(2-{2-Oxo-2-[4-(1H-pyrrol-1-yl)piperidin-1-yl]ethyl}-2H-indazol-5-yl)-6-(trifluormethyl)pyridin-2-carboxamid
N-(2- {2-[4-(3-Hydroxypropyl)piperazin-1-yl]-2-oxoethyl}-2H-indazol-5-yl)-6-(trifluormethyl)pyridin-2-carboxamid
4-{[5-({[6-(Trifluormethyl)pyridin-2-yl]carbonyl}amino)-2H-indazol-2-yl]acetyl}piperazin-1-carboxamid
N-(2-{2-Oxo-2-[4-(2-oxopyrrolidin-1-yl)piperidin-1-yl]ethyl}-2H-indazol-5-yl)-6-(trifluormethyl)pyridin-2-carboxamid
N-{2-[2-(Morpholin-4-yl)-2-oxoethyl]-2H-indazol-5-yl}-6-(trifluormethyl)pyridin-2-carboxamid
N-(2-{2-[4-(2-Amino-2-oxoethyl)piperazin-1-yl]-2-oxoethyl}-2H-indazol-5-yl)-6-(trifluormethyl)pyridin-2-carboxamid
N-{2-[2-(1,1-Dioxidothiomorpholin-4-yl)-2-oxoethyl]-2H-indazol-5-yl}-6-(trifluormethyl)pyridin-2-carboxamid
N-{2-[2-(4-Isopropylpiperazin-1-yl)-2-oxoethyl]-2H-indazol-5-yl}-6-(trifluormethyl)pyridin-2-carboxamid
N-(2-{2-Oxo-2-[4-(2-thienylcarbonyl)piperazin-1-yl]ethyl}-2H-indazol-5-yl)-6-(trifluormethyl)pyridin-2-carboxamid
N-(2-{2-[4-(2-Cyclopropyl-2-oxoethyl)piperazin-1-yl]-2-oxoethyl}-2H-indazol-5-yl)-6-(trifluormethyl)pyridin-2-carboxamid
N-[2-(2-{4-[(1-Methyl-1H-pyrazol-4-yl)methyl]piperazin-1-yl}-2-oxoethyl)-2H-indazol-5-yl]-6-(trifluormethyl)pyridin-2-carboxamid
N-[2-(2-{4-[(1,5-Dimethyl-1H-pyrazol-3-yl)carbonyl]piperazin-1-yl}-2-oxoethyl)-2H-indazol-5-yl]-6-(trifluormethyl)pyridin-2-carboxamid
N,N-Diethyl-4- {[5-({[6-(trifluormethyl)pyridin-2-yl]carbonyl}amino)-2H-indazol-2-yl]acetyl}piperazin-1-carboxamid
N-{2-[2-Oxo-2-(thiomorpholin-4-yl)ethyl]-2H-indazol-5-yl}-6-(trifluormethyl)pyridin-2-carboxamid
N-(2-{2-[4-(2-Furylmethyl)piperazin-1-yl]-2-oxoethyl}-2H-indazol-5-yl)-6-(trifluormethyl)pyridin-2-carboxamid
N-(2-{2-Oxo-2-[4-(3-thienylmethyl)piperazin-1-yl]ethyl}-2H-indazol-5-yl)-6-(trifluormethyl)pyridin-2-carboxamid
N-{2-[2-(4'-Methyl-1,4'-bipiperidin-1'-yl)-2-oxoethyl]-2H-indazol-5-yl}-6-(trifluormethyl)pyridin-2-carboxamid
N-{2-[2-(6-Methyl-2,6-diazaspiro[3.3]hept-2-yl)-2-oxoethyl]-2H-indazol-5-yl}-6-(trifluormethyl)pyridin-2-carboxamid
N-{2-[2-(4-Cyclopentylpiperazin-1-yl)-2-oxoethyl]-2H-indazol-5-yl}-6-(trifluormethyl)pyridin-2-carboxamid
N-[2-(2-{4-[2-(2-Hydroxyethoxy)ethyl]piperazin-1-yl}-2-oxoethyl)-2H-indazol-5-yl]-6-(trifluormethyl)pyridin-2-carboxamid
N-(2-{2-Oxo-2-[4-(pyridin-4-ylmethyl)piperazin-1-yl]ethyl}-2H-indazol-5-yl)-6-(trifluormethyl)pyridin-2-carboxamid
N-(2-{2-[4-(Dimethylsulfamoyl)piperazin-1-yl]-2-oxoethyl}-2H-indazol-5-yl)-6-(trifluormethyl)pyridin-2-carboxamid
N-(2-{2-Oxo-2-[4-(pyridin-4-yl)piperazin-1-yl]ethyl}-2H-indazol-5-yl)-6-(trifluormethyl)pyridin-2-carboxamid
N-(2-{2-[4-(Methylsulfonyl)piperazin-1-yl]-2-oxoethyl}-2H-indazol-5-yl)-6-(trifluormethyl)pyridin-2-carboxamid
Ameisensäure--N-[2-(2-{4-[2-(1H-imidazol-1-yl)ethyl]piperazin-1-yl}-2-oxoethyl)-2H-indazol-5-yl]-6-(trifluormethyl)pyridin-2-carboxamid(1:1)
N-(2- {2-[4-(Diethylsulfamoyl)piperazin-1-yl]-2-oxoethyl}-2H-indazol-5-yl)-6-(trifluormethyl)pyridin-2-carboxamid
N-(2- {2-Oxo-2-[-yl]ethyl}-2H-indazol-5-yl)-6-(trifluormethyl)pyridin-2-carboxamid
N-(2- {2-Oxo-2-[4-(piperidin-1-ylsulfonyl)piperazin-1-yl]ethyl}-2H-indazol-5-yl)-6-(trifluormethyl)pyridin-2-carboxamid
N-[2-(2-{4-[(1,5-Dimethyl-1H-pyrazol-4-yl)sulfonyl]piperazin-1-yl}-2-oxoethyl)-2H-indazol-5-yl]-6-(trifluormethyl)pyridin-2-carboxamid
N-(2-{2-[4-(Cyclopropylmethyl)piperazin-1-yl]-2-oxoethyl}-2H-indazol-5-yl)-6-(1-methyl-1H-pyrazol-4-yl)pyridin-2-carboxamid
N-(2-{2-[4-(2-Hydroxypropan-2-yl)piperidin-1-yl]-2-oxoethyl}-2H-indazol-5-yl)-6-(1-methyl-1H-pyrazol-4-yl)pyridin-2-carboxamid
6-(1-Methyl-1H-pyrazol-4-yl)-N-(2-{2-oxo-2-[4-(pyrrolidin-1-yl)piperidin-1-yl]ethyl}-2H-indazol-5-yl)pyridin-2-carboxamid
N-{2-[2-(4-Ethylpiperazin-1-yl)-2-oxoethyl]-2H-indazol-5-yl}-6-(1-methyl-1H-pyrazol-4-yl)pyridin-2-carboxamid
N-(2-{2-[4-(Dimethylamino)piperidin-1-yl]-2-oxoethyl}-2H-indazol-5-yl)-6-(1-methyl-1H-pyrazol-4-yl)pyridin-2-carboxamid
N-(2-{2-[(Cyclopropylmethyl)(methyl)amino]-2-oxoethyl}-6-methoxy-2H-indazol-5-yl)-6-methylpyridin-2-carboxamid
N-(2-{2-[(Cyclopropylmethyl)(methyl)amino]-2-oxoethyl}-6-ethoxy-2H-indazol-5-yl)-6-(trifluormethyl)pyridin-2-carboxamid
N-(2-{2-[(Cyclopropylmethyl)(methyl)amino]-2-oxoethyl}-6-methoxy-2H-indazol-5-yl)-6-(trifluormethyl)pyridin-2-carboxamid
6-Cyclopropyl-N-(2-{2-[4-(2-hydroxypropan-2-yl)piperidin-1-yl]-2-oxoethyl}-6-methoxy-2H-indazol-5-yl)pyridin-2-carboxamid
6-(1-Hydroxyethyl)-N-(2-{2-[4-(2-hydroxypropan-2-yl)piperidin-1-yl]-2-oxoethyl}-6-methoxy-2H-indazol-5-yl)pyridin-2-carboxamid
6-(Azetidin-3-ylamino)-N-{2-[2-(4-benzoylpiperazin-1-yl)-2-oxoethyl]-2H-indazol-5-yl}pyridin-2-carboxamid
6-[(Azetidin-2-ylmethyl)amino]-N-{2-[2-(4-benzoylpiperazin-1-yl)-2-oxoethyl]-2H-indazol-5-yl}pyridin-2-carboxamid
N-{2-[2-(4-Benzoylpiperazin-1-yl)-2-oxoethyl]-2H-indazol-5-yl}-6-(3-hydroxyazetidin-1-yl)pyridin-2-carboxamid
6-[(2R,6S)-2,6-Dimethylmorpholin-4-yl]-N-(6-methyl-2-{2-oxo-2-[4-(pyrrolidin-1-yl)piperidin-1-yl]ethyl}-2H-indazol-5-yl)pyridin-2-carboxamid
N-[2-(2-{4-Methyl-4-[(4-methylpiperazin-1-yl)carbonyl]piperidin-1-yl}-2-oxoethyl)-2H-indazol-5-yl]-6-(trifluormethyl)pyridin-2-carboxamid
N-(6-Chlor-2-{2-oxo-2-[(3R)-piperidin-3-ylamino]ethyl}-2H-indazol-5-yl)-6-(trifluormethyl)pyridin-2-carboxamid
N-(2-{2-[4-(Cyclopropylcarbonyl)piperazin-1-yl]-2-oxoethyl}-6-isopropoxy-2H-indazol-5-yl)-6-methylpyridin-2-carboxamid
N-(2-{2-[4-(Cyclopropylcarbonyl)piperazin-1-yl]-2-oxoethyl}-6-isopropoxy-2H-indazol-5-yl)-6-(trifluormethyl)pyridin-2-carboxamid
N-{2-[2-(4-Benzoylpiperazin-1-yl)-2-oxoethyl]-6-isopropoxy-2H-indazol-5-yl}-6-methylpyridin-2-carboxamid
N-{2-[2-(4-Benzoylpiperazin-1-yl)-2-oxoethyl]-6-isopropoxy-2H-indazol-5-yl}-6-(trifluormethyl)pyridin-2-carboxamid
N-{6-Isopropoxy-2-[2-(4-methylpiperazin-1-yl)-2-oxoethyl]-2H-indazol-5-yl}-6-(trifluormethyl)pyridin-2-carboxamid
N-{6-Isopropoxy-2-[2-(4-methylpiperazin-1-yl)-2-oxoethyl]-2H-indazol-5-yl}-6-methylpyridin-2-carboxamid
N-(2-{2-[4-(Cyclobutylcarbonyl)piperazin-1-yl]-2-oxoethyl}-2H-indazol-5-yl)-6-(trifluormethyl)pyridin-2-carboxamid
N-(2-{2-[4-(Cyclopentylcarbonyl)piperazin-1-yl]-2-oxoethyl}-2H-indazol-5-yl)-6-(trifluormethyl)pyridin-2-carboxamid
N-[2-(2-{4-[3-(Methylsulfonyl)benzoyl]piperazin-1-yl}-2-oxoethyl)-2H-indazol-5-yl]-6-(trifluormethyl)pyridin-2-carboxamid
N-[2-(2-{4-[2-Methoxy-5-(methylsulfonyl)benzoyl]piperazin-1-yl}-2-oxoethyl)-2H-indazol-5-yl]-6-(trifluormethyl)pyridin-2-carboxamid
6-Brom-N-(6-methyl-2-{2-oxo-2-[4-(pyrrolidin-1-yl)piperidin-1-yl]ethyl}-2H-indazol-5-yl)pyridin-2-carboxamid
2-(4-Methoxyphenyl)-N-(6-methyl-2-{2-oxo-2-[4-(pyrrolidin-1-yl)piperidin-1-yl]ethyl}-2H-indazol-5-yl)-1,3-thiazol-4-carboxamid
2-(4-Fluorphenyl)-N-(6-methyl-2-{2-oxo-2-[4-(pyrrolidin-1-yl)piperidin-1-yl]ethyl}-2H-indazol-5-yl)-1,3-thiazol-4-carboxamid
N-(6-Methyl-2- {2-oxo-2-[4-(pyrrolidin-1-yl)piperidin-1 -yl]ethyl}-2H-indazol-5-yl)-6-(trifluormethyl)pyridin-2-carboxamid
6-Brom-N-{2-[2-(4-methylpiperazin-1-yl)-2-oxoethyl]-2H-indazol-5-yl}pyridin-2-carboxamid
6-Brom-N-{2-[2-(4-methylpiperazin-1-yl)-2-oxoethyl]-6-(trifluormethoxy)-2H-indazol-5-yl}pyridin-2-carboxamid
N-{2-[2-(4-Methylpiperazin-1-yl)-2-oxoethyl]-6-(trifluormethoxy)-2H-indazol-5-yl}-6-(4H-1,2,4-triazol-4-yl)pyridin-2-carboxamid
2-Brom-N-{6-brom-2-[2-(4-methylpiperazin-1-yl)-2-oxoethyl]-2H-indazol-5-yl}-1,3-thiazol-4-carboxamid
N-{6-Hydroxy-2-[2-(4-methylpiperazin-1-yl)-2-oxoethyl]-2H-indazol-5-yl}-6-(trifluormethyl)pyridin-2-carboxamid
N-[6-(Benzyloxy)-2-{2-[4-(2-hydroxypropan-2-yl)piperidin-1-yl]-2-oxoethyl}-2H-indazol-5-yl]-6-methylpyridin-2-carboxamid
6-Brom-N-{6-brom-2-[2-(4-methylpiperazin-1-yl)-2-oxoethyl]-2H-indazol-5-yl}pyridin-2-carboxamid
N-{6-(Benzyloxy)-2-[2-(4-methylpiperazin-1-yl)-2-oxoethyl]-2H-indazol-5-yl}-6-methylpyridin-2-carboxamid
2-(Azetidin-3-ylamino)-N-{2-[2-(4-benzoylpiperazin-1-yl)-2-oxoethyl]-2H-indazol-5-yl}-1,3-thiazol-4-carboxamid
6-Acetamido-N-{6-methoxy-2-[2-(morpholin-4-yl)-2-oxoethyl]-2H-indazol-5-yl}pyridin-2-carboxamid
6-(Dimethylamino)-N-(2-{2-[4-(2-hydroxypropan-2-yl)piperidin-1-yl]-2-oxoethyl}-6-methoxy-2H-indazol-5-yl)pyridin-2-carboxamid
6-(Dimethylamino)-N-{6-methoxy-2-[2-(morpholin-4-yl)-2-oxoethyl]-2H-indazol-5-yl}pyridin-2-carboxamid
6-Acetamido-N-(2-{2-[4-(2-hydroxypropan-2-yl)piperidin-1-yl]-2-oxoethyl}-6-methoxy-2H-indazol-5-yl)pyridin-2-carboxamid
6-(Dimethylamino)-N-{6-methoxy-2-[2-(4-methylpiperazin-1-yl)-2-oxoethyl]-2H-indazol-5-yl}pyridin-2-carboxamid
N-{2-[2-(4-Methylpiperazin-1-yl)-2-oxoethyl]-2H-indazol-5-yl}-6-[3-(methylsulfonyl)phenyl]pyridin-2-carboxamid
N-{2-[1-(4-Benzoylpiperazin-1-yl)-1-oxopropan-2-yl]-2H-indazol-5-yl}-6-(trifluormethyl)pyridin-2-carboxamid
N-[6-Chlor-2-(2-{[trans-4-(2-hydroxypropan-2-yl)cyclohexyl]amino}-2-oxoethyl)-2H-indazol-5-yl]-6-(trifluormethyl)pyridin-2-carboxamid
6-(2-Hydroxypropan-2-yl)-N-{6-methoxy-2-[2-(morpholin-4-yl)-2-oxoethyl]-2H-indazol-5-yl}pyridin-2-carboxamid
N-{6-Chlor-2-[2-(3,3-difluorpyrrolidin-1-yl)-2-oxoethyl]-2H-indazol-5-yl}-6-(trifluormethyl)pyridin-2-carboxamid
N-{6-Chlor-2-[2-oxo-2-(pyrrolidin-1-yl)ethyl]-2H-indazol-5-yl}-6-(trifluormethyl)pyridin-2-carboxamid
N- {6-Chlor-2-[2-(2-oxa-7-azaspiro[3.5]non-7-yl)-2-oxoethyl]-2H-indazol-5-yl} -6-(trifluormethyl)pyridin-2-carboxamid
N-(6-Chlor-2-{2-[4-(2-hydroxy-2-methylpropyl)piperazin-1-yl]-2-oxoethyl}-2H-indazol-5-yl)-6-(trifluormethyl)pyridin-2-carboxamid
N-{6-Methoxy-2-[2-oxo-2-(pyrrolidin-1-yl)ethyl]-2H-indazol-5-yl}-6-(trifluormethyl)pyridin-2-carboxamid
N-{2-[2-(3,3-Difluorpyrrolidin-1-yl)-2-oxoethyl]-6-methoxy-2H-indazol-5-yl}-6-(trifluormethyl)pyridin-2-carboxamid
6-(Difluormethyl)-N-{6-methoxy-2-[2-(morpholin-4-yl)-2-oxoethyl]-2H-indazol-5-yl}pyridin-2-carboxamid
N-{2-[2-(3,3-Difluorpyrrolidin-1-yl)-2-oxoethyl]-6-methoxy-2H-indazol-5-yl}-6-methylpyridin-2-carboxamid
N-{6-Methoxy-2-[2-(morpholin-4-yl)-2-oxoethyl]-2H-indazol-5-yl}-6-methylpyridin-2-carboxamid
N-{6-Methoxy-2-[2-(morpholin-4-yl)-2-oxoethyl]-2H-indazol-5-yl}-2-(tetrahydro-2H-pyran-4-yl)-1,3-oxazol-4-carboxamid
N-{2-[2-(1,1-Dioxido-1-thia-6-azaspiro[3.3]hept-6-yl)-2-oxoethyl]-6-methoxy-2H-indazol-5-yl}-6-(trifluormethyl)pyridin-2-carboxamid
N-{6-Methoxy-2-[2-(2-oxa-6-azaspiro[3.3]hept-6-yl)-2-oxoethyl]-2H-indazol-5-yl}-6-(trifluormethyl)pyridin-2-carboxamid
N-{6-(3-Hydroxy-2,2-dimethylpropoxy)-2-[2-(morpholin-4-yl)-2-oxoethyl]-2H-indazol-5-yl}-6-(trifluormethyl)pyridin-2-carboxamid
6-Ethyl-N-{6-methoxy-2-[2-(morpholin-4-yl)-2-oxoethyl]-2H-indazol-5-yl}pyridin-2-carboxamid
6-Isobutyl-N-{6-methoxy-2-[2-(morpholin-4-yl)-2-oxoethyl]-2H-indazol-5-yl}pyridin-2-carboxamid
Methyl-2-[2-(morpholin-4-yl)-2-oxoethyl]-5-({[6-(trifluormethyl)pyridin-2-yl]carbonyl}amino)-2H-indazol-6-carboxylat
Methyl-5-{[(6-methylpyridin-2-yl)carbonyl]amino}-2-[2-(morpholin-4-yl)-2-oxoethyl]-2H-indazol-6-carboxylat
N-{6-Methoxy-2-[2-(morpholin-4-yl)-2-oxoethyl]-2H-indazol-5-yl}-6-(pyrrolidin-1-yl)pyridin-2-carboxamid
N-{6-Methoxy-2-[2-(morpholin-4-yl)-2-oxoethyl]-2H-indazol-5-yl}-6-(morpholin-4-yl)pyridin-2-carboxamid
6-(Cyclopropylamino)-N-{6-methoxy-2-[2-(morpholin-4-yl)-2-oxoethyl]-2H-indazol-5-yl}pyridin-2-carboxamid
6-(Butylamino)-N-{6-methoxy-2-[2-(morpholin-4-yl)-2-oxoethyl]-2H-indazol-5-yl}pyridin-2-carboxamid
N-{6-Methoxy-2-[2-(morpholin-4-yl)-2-oxoethyl]-2H-indazol-5-yl}-6-(propylamino)pyridin-2-carboxamid
6-(Isobutylamino)-N-{6-methoxy-2-[2-(morpholin-4-yl)-2-oxoethyl]-2H-indazol-5-yl}pyridin-2-carboxamid
R-N-{6-Methoxy-2-[2-(morpholin-4-yl)-2-oxoethyl]-2H-indazol-5-yl}-6-(2,2,2-trifluor-1-hydroxyethyl)pyridin-2-carboxamid
S-N-{6-Methoxy-2-[2-(morpholin-4-yl)-2-oxoethyl]-2H-indazol-5-yl}-6-(2,2,2-trifluor-1-hydroxyethyl)pyridin-2-carboxamid
6-(1-Hydroxyethyl)-N-{6-methoxy-2-[2-(morpholin-4-yl)-2-oxoethyl]-2H-indazol-5-yl}pyridin-2-carboxamid
6-(Cyclopropylamino)-N-{6-methoxy-2-[2-(4-methylpiperazin-1-yl)-2-oxoethyl]-2H-indazol-5-yl}pyridin-2-carboxamid
N-{6-Methoxy-2-[2-(4-methylpiperazin-1-yl)-2-oxoethyl]-2H-indazol-5-yl}-6-(propylamino)pyridin-2-carboxamid
6-(Isobutylamino)-N-{6-methoxy-2-[2-(4-methylpiperazin-1-yl)-2-oxoethyl]-2H-indazol-5-yl}pyridin-2-carboxamid
6-(1-Hydroxyethyl)-N-{6-methoxy-2-[2-(4-methylpiperazin-1-yl)-2-oxoethyl]-2H-indazol-5-yl}pyridin-2-carboxamid
N-{6-Methoxy-2-[2-(4-methylpiperazin-1-yl)-2-oxoethyl]-2H-indazol-5-yl}-4-methyl-6-(trifluormethyl)pyridin-2-carboxamid
N-{6-(Benzyloxy)-2-[2-(morpholin-4-yl)-2-oxoethyl]-2H-indazol-5-yl}-6-(trifluormethyl)pyridin-2-carboxamid
6-(Cyclopropylamino)-N-(2-{2-[4-(2-hydroxypropan-2-yl)piperidin-1-yl]-2-oxoethyl}-6-methoxy-2H-indazol-5-yl)pyridin-2-carboxamid
6-(Butylamino)-N-(2-{2-[4-(2-hydroxypropan-2-yl)piperidin-1-yl]-2-oxoethyl}-6-methoxy-2H-indazol-5-yl)pyridin-2-carboxamid
N-(2-{2-[4-(2-Hydroxypropan-2-yl)piperidin-1-yl]-2-oxoethyl}-6-methoxy-2H-indazol-5-yl)-6-[(2-methoxyethyl)amino]pyridin-2-carboxamid
N-(2-{2-[4-(2-Hydroxypropan-2-yl)piperidin-1-yl]-2-oxoethyl}-6-methoxy-2H-indazol-5-yl)-6-(propylamino)pyridin-2-carboxamid
N-(2-{2-[4-(2-Hydroxypropan-2-yl)piperidin-1-yl]-2-oxoethyl}-6-methoxy-2H-indazol-5-yl)-6-(isobutylamino)pyridin-2-carboxamid
5-Fluor-N-(2-{2-[4-(2-hydroxypropan-2-yl)piperidin-1-yl]-2-oxoethyl}-6-methoxy-2H-indazol-5-yl)-6-methylpyridin-2-carboxamid
N-{6-Hydroxy-2-[2-(morpholin-4-yl)-2-oxoethyl]-2H-indazol-5-yl}-6-(trifluormethyl)pyridin-2-carboxamid
N-{6-(3-Cyanpropoxy)-2-[2-(morpholin-4-yl)-2-oxoethyl]-2H-indazol-5-yl}-6-(trifluormethyl)pyridin-2-carboxamid
N-{2-[2-(Morpholin-4-yl)-2-oxoethyl]-6-(2,2,2-trifluorethoxy)-2H-indazol-5-yl}-6-(trifluormethyl)pyridin-2-carboxamid
N-{6-(Cyclohexylmethoxy)-2-[2-(morpholin-4-yl)-2-oxoethyl]-2H-indazol-5-yl}-6-(trifluormethyl)pyridin-2-carboxamid
N-{6-(2,2-Dimethylpropoxy)-2-[2-(morpholin-4-yl)-2-oxoethyl]-2H-indazol-5-yl}-6-(trifluormethyl)pyridin-2-carboxamid
N-{2-[2-(Morpholin-4-yl)-2-oxoethyl]-6-(tetrahydrofuran-2-ylmethoxy)-2H-indazol-5-yl}-6-(trifluormethyl)pyridin-2-carboxamid
N-{6-(Cyclopentyloxy)-2-[2-(morpholin-4-yl)-2-oxoethyl]-2H-indazol-5-yl}-6-(trifluormethyl)pyridin-2-carboxamid
N-{6-(Cyanmethoxy)-2-[2-(morpholin-4-yl)-2-oxoethyl]-2H-indazol-5-yl}-6-(trifluormethyl)pyridin-2-carboxamid
({2-[2-(Morpholin-4-yl)-2-oxoethyl]-5-({[6-(trifluormethyl)pyridin-2-yl]carbonyl}amino)-2H-indazol-6-yl} oxy)essigsäure
N-{6-(Cyclobutylmethoxy)-2-[2-(morpholin-4-yl)-2-oxoethyl]-2H-indazol-5-yl}-6-(trifluormethyl)pyridin-2-carboxamid
N-{2-[2-(Morpholin-4-yl)-2-oxoethyl]-6-[2-(pyrrolidin-1-yl)ethoxy]-2H-indazol-5-yl}-6-(trifluormethyl)pyridin-2-carboxamid
N-{6-[2-(Morpholin-4-yl)ethoxy]-2-[2-(morpholin-4-yl)-2-oxoethyl]-2H-indazol-5-yl}-6-(trifluormethyl)pyridin-2-carboxamid
N-{2-[2-(Morpholin-4-yl)-2-oxoethyl]-6-[2-(piperidin-1-yl)ethoxy]-2H-indazol-5-yl}-6-(trifluormethyl)pyridin-2-carboxamid
N-{6-(3-Hydroxypropoxy)-2-[2-(morpholin-4-yl)-2-oxoethyl]-2H-indazol-5-yl}-6-(trifluormethyl)pyridin-2-carboxamid
N-{6-(2-Hydroxypropoxy)-2-[2-(morpholin-4-yl)-2-oxoethyl]-2H-indazol-5-yl}-6-(trifluormethyl)pyridin-2-carboxamid
N-{6-(2-Hydroxyethoxy)-2-[2-(morpholin-4-yl)-2-oxoethyl]-2H-indazol-5-yl}-6-(trifluormethyl)pyridin-2-carboxamid
N-{6-(2-Methoxyethoxy)-2-[2-(morpholin-4-yl)-2-oxoethyl]-2H-indazol-5-yl}-6-(trifluormethyl)pyridin-2-carboxamid
Ethyl-({2-[2-(morpholin-4-yl)-2-oxoethyl]-5-({[6-(trifluormethyl)pyridin-2-yl]carbonyl}amino)-2H-indazol-6-yl}oxy)acetat
Methyl-4-({2-[2-(morpholin-4-yl)-2-oxoethyl]-5-({[6-(trifluormethyl)pyridin-2-yl]carbonyl}amino)-2H-indazol-6-yl}oxy)butanoat
Ethyl-2-({2-[2-(morpholin-4-yl)-2-oxoethyl]-5-({[6-(trifluormethyl)pyridin-2-yl]carbonyl}amino)-2H-indazol-6-yl}oxy)propanoat
Ethyl-3-methyl-2-({2-[2-(morpholin-4-yl)-2-oxoethyl]-5-({[6-(trifluormethyl)pyridin-2-yl]carbonyl}amino)-2H-indazol-6-yl}oxy)butanoat
2-({2-[2-(Morpholin-4-yl)-2-oxoethyl]-5-({[6-(trifluormethyl)pyridin-2-yl]carbonyl}amino)-2H-indazol-6-yl} oxy)propansäure
N-{6-(2-Hydroxypropan-2-yl)-2-[2-(morpholin-4-yl)-2-oxoethyl]-2H-indazol-5-yl}-6-(trifluormethyl)pyridin-2-carboxamid
N-{6-Chlor-2-[2-(4-methylpiperazin-1-yl)-2-oxoethyl]-2H-indazol-5-yl}-6-(difluormethyl)pyridin-2-carboxamid
N-{6-Chlor-2-[2-(morpholin-4-yl)-2-oxoethyl]-2H-indazol-5-yl}-6-(difluormethyl)pyridin-2-carboxamid

Ein weiterer Gegenstand der vorliegenden Erfindung ist ein Verfahren zur Herstellung von Zwischenprodukten der allgemeinen Fomrel (III) aus der Verbindung der Formel (II), 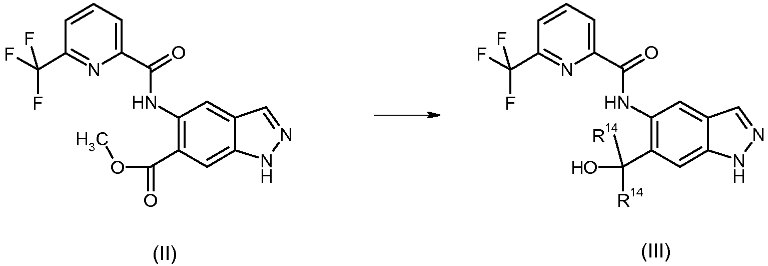 worin R¹⁴ entweder Methyl oder Ethyl ist.
Die Umsetzung des Zwischenproduktes der Formel (II) zu den Zwischenprodukten der Formel (III) erfolgt durch eine Grignardreaktion. Bevorzugt wird die Grignardreaktion mit Alkylmagnesiumbromid durchgeführt. Bevorzugt verwendet hierfür werden entweder Methylmagnesiumbromid oder Ethylmagnesiumbromid.

Weiterer Gegenstand der Erfindung sind hiermit Zwischenprodukte der allgemeinen Formel (II). Ein weiterer Gegenstand der Erfindung sind auch Zwischenprodukte der allgemeinen Formel (III), in der R¹⁴ entweder Methyl oder Ethyl bedeutet.

Die erfindungsgemäßen Verbindungen der Formel (I) wirken als Inhibitoren der IRAK4 Kinase, und zeigen ein nicht vorhersehbares, wertvolles pharmakologisches Wirkspektrum.

Daher ist neben den weiter oben genannten ein weiterer Gegenstand der vorliegenden Erfindung die erfindungsgemäßen Verbindungen zur Verwendung in der Behandlung und/oder Prophylaxe von Krankheiten bei Menschen und Tieren.

Die erfindungsgemäßen Verbindungen sind geeignet für die Prophylaxe und/ oder Behandlung von verschiedenen Erkrankungen und krankheitsbedingten Zuständen, insbesondere von TLR (außer TLR3) und/oder IL-1-Rezeptorfamilie-vermittelten Erkrankungen bzw. Erkrankungen, dessen Pathologie direkt durch IRAK4 vermittelt ist. Als IRAK4-assoziierte Erkrankungen sind multiple Sklerose, Atherosklerose, Herzinfarkt, Alzheimer, viral-induzierte Myokarditis, Gicht, Psoriasis und Arthritis zu benennen.

Die erfindungsgemäßen Verbindungen können ferner für die Prophylaxe und/oder Behandlung von MyD88 und TLR (außer TLR3)-vermittelte Erkrankungen eingesetzt werden. Dies umfasst Multiple Sklerose, Rheumatoide Arthritis, Metabolisches Syndrom, Diabetes, Osteoarthritis, Sjögren-Syndrom, Sepsis, Hauterkrankungen wie Psoriasis, atopische Dermatitis und Acne vulgaris, pulmonale Erkrankungen wie Lungenfibrose, chronisch obstruktive Lungenerkrankung (COPD), akutes Atemnot-Syndrom (ARDS), akute Lungenschädigung (ALI), interstitielle Lungenerkrankung (ILD), Sarkoidose und pulmonale Hypertonie.

Aufgrund des Wirkmechanismus der erfindungsgemäßen Verbindungen sind sie zur Prophylaxe und/ oder Behandlung der TLR-vermittelten Erkrankungen Behçet-Krankheit, Gicht, Endometriose, Transplantatabstoßung, Lupus erythematodes, Adult Morbus Still-Krankheit und chronisch entzündlichen Darmerkrankungen, wie Kolitis ulcerosa und Morbus Crohn geeignet.

Neben den bereits aufgeführten Erkrankungen, sind die erfindungsgemäßen Verbindungen auch zur Verwendung in der Behandlung und/oder Prävention folgender Erkrankungen geeignet: Augenerkrankungen wie Keratitis, allergisch bedingte Konjunktivitis, Keratoconjunctivitis sicca, Makuladegeneration und Uveitis; kardiovaskulären Erkrankungen wie Arteriosklerose, myokardialer Reperfusionsschaden, Myokardinfarkt, Hypertonie und neurologische Erkrankungen wie Alzheimer, Schlaganfall und Parkinson.

Weiterhin ist die Prophylaxe und/oder Behandlung von Juckreiz und Schmerz durch die erfindungsgemäßen Verbindungen gegeben. Aufgrund des Wirkmechanismus der erfindungsgemäßen Verbindungen sind sie zur Prophylaxe und/oder Behandlung von onkologischen Erkrankungen wie Lymphome, chronisch lymphatische Leukämie, Melanomen und Leberzellkarzinom und Ras-abhängige Tumore geeignet.

Außerdem sind die erfindungsgemäßen Verbindungen geeignet zur Behandlung und/oder Prävention von Erkrankungen, die über die IL-1 Rezeptorfamilie vermittelt sind. Diese Erkrankungen umfassen CAPS (Cryopyrin-assoziierte periodische Syndrome) inklusive FCAS (familiäre Kälteurtikaria), MWS (Muckle-Wells-Syndrom), NOMID- (neonatal-onset multisystem inflammatory disease) und CONCA- (chronic infantile, neurological, cutaneous, and articular) Syndrom, FMF (familiäres Mittelmeerfieber), HIDS (Hyper-IgD-Syndrom), TRAPS (Tumornekrosefaktor-Rezeptor 1-assoziiertes periodisches Syndrom), juvenile idiopathische Arthritis, Adult Morbus Still-Krankheit, Morbus Adamantiades-Behçet, rheumatoide Arthritis, Osteoarthritis, Keratoconjunctivitis sicca und Sjögren Syndrom, Multiple Sklerose, Lupus erythematodes, Typ-1-Diabetes, Typ-2-Diabetes und den Folgen eines Myokardinfarktes.

Pulmonale Erkrankungen wie Asthma, COPD, idiopathische interstitielle Pneumonie und ARDS, Endometriose, chronisch-entzündliche Darmerkrankungen wie Morbus Crohn und Kolitis ulcerosa sind mit einer Dysregulation der IL-1 Rezeptorfamilie assoziiert und für den therapeutischen und/oder prophylaktischen Einsatz der erfindungsgemäßen Verbindungen geeignet.

Die erfindungsgemäßen Verbindungen können ferner für Behandlung und/oder Prävention von IL-1 Rezeptorfamilie-vermittelten neurologischen Erkrankungen wie Hirnschlag, Alzheimer, Schlaganfall, Schädel-Hirn-Trauma. Schmerzerkrankungen wie Krebsschmerzen, postoperativer Schmerz, entzündungsinduzierter und chronischer Schmerz und dermatologische Erkrankungen wie Psoriasis, atopische Dermatitis, allergische Kontaktdermatitis eingesetzt werden.

Die Behandlung und/ oder Prophylaxe von entzündlichen Hauterkrankungen, Herz-Kreislauf-Erkrankungen, Lungenerkrankungen, Augenerkrankungen, Autoimmunerkrankungen und KrebsErkrankungen mit den erfindungsgemäßen IRAK4-Inhibitoren ist besonders bevorzugt.

Im Weiteren ist Gegenstand der vorliegenden Erfindung auch Verbindungen nach Anspruch 1 zur Verwendung in einem Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als Synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
Allgemein sind Wirkstoffe wie antibakterielle (z.B. Penicilline, Vancomycin, Ciprofloxacin), antivirale (z.B. Aciclovir, Oseltamivir) und antimykotische (z.B. Naftifin, Nystatin) Substanzen und Gammaglobuline, immunomodulatorische und immunosuppressive Verbindungen wie Cyclosporin, Tacrolimus, Rapamycin, Mycophenolate mofetil, Interferone, Corticosteroide (z.B. Prednisone, Prednisolone, Methylprednisolone, Hydrocortisone, Betamethason), Cyclophophamide, Azathioprine und Sulfasalazine; Paracetamol, non-steroidale anti-inflammatorische Substanzen (NSAIDS) (Aspirin, Ibuprofen, Naproxen, Etodolac, Celecoxib, Colchicine) zu nennen.

Für die Tumortherapie seinen zu nennen Immunotherapie, antiproliferative Substanzen wie beispielsweise aber nicht ausschließlich Trastuzumab, Rituximab, Tositumomab, Aromatase Inhibitoren (z.B. Letrozol, Anastrozol), Antiestrogene (z.B.Tamoxifen), Topoisomerase I Inhibitoren (z.B. Irinotecan, Topotecan), Topoisomerasen II Inhibitoren (z.B. Daunorubicin, Idarubicin, Mitoxantron), Mikrotubuli-aktive Substanzen (z.B. Vinblastin, Vincristin), Telomeraseinhibitoren (z.B. Imetelstat), alkylierende Substanzen und Histon-Deacetylasen Inhibitoren (z.B. Vorinostat, Romidepsin, Panobinostat); Substanzen, die Prozesse der Zelldifferenzierung beeinflussen wie MMP Inhibitoren (Peptidmimetika, Nicht-Peptidmimetika und Tetracycline wie z.B. Marimastat, BAY 12-9566, BMS-275291, Clodronate, Prinomastat, Doxycycline), mTOR Inhibitoren (z.B. Sirolimus, Everolimus, Temsirolimus, Zotarolimus), Antimetabolite (z.B. Methotrexat, 5-Fluoruracil, Cladribin, Fludarabin), Platinverbindungen (z.B. Carboplatin, Cisplatin, Cisplatinum); Anti-angiogene Verbindungen (z.B.. Bevacizumab), antiandrogene Verbindungen (z.B. Flutamid, Nilutamid, Bicalutamid, Cyproteronacetat), Proteasomeinhibitoren (z.B. Bortezomib, Carfilzomib, Oprozomib, ONYX0914), Gonadoliberin-Agonisten und -Antagonisten (z.B. Goserelin, Triptorelin, Degarelix), Methionine Aminopeptidase Inhibitoren (z.B. Bengamid-Derivate, TNP-470, PPI-2458), Heparanaseinhibitoren (z.B. SST0001, PI-88); Inhibitoren gegen genetisch verändertes Ras-Protein (z.B. Farnesyl-Transferase Inhibitoren wie Lonafarnib, Tipifarnib), HSP90 Inhibitoren (z.B.: Geldamycin-Derivate wie 17-Allylaminogeldanamycin, 17-demethoxygeldanamycin (17AAG), 17-DMAG, Retaspimycin Hydrochloride, IPI-493, AUY922, BIIB028, STA-9090, KW-2478), Kinesin Spindleprotein Inhibitoren (z.B. SB715992, SB743921, Pentamidine/Chlorpromazine), MEK (mitogen-activated protein kinase kinase) Inhibitoren (z.B. Trametinib, BAY 86-9766, AZD6244,), Kinase-Inhibitoren (z.B.: Sorafenib, Regorafenib, Lapatinib, Sutent, Dasatinib, Cetuximab BMS-908662, GSK2118436, AMG 706), Hedgehog Signalinhibitoren (z.B. Cyclopamin, Vismodegib), BTK (Bruton's Tyrosine Kinase) Inhibitor (z.B. Ibrutinib), JAK/pan-JAK (Janus Kinase) Inhibitor (z.B. SB-1578, Baricitinib, Tofacitinib, Pacritinib, Momelotinib, Ruxolitinib, VX-509, AZD-1480, TG-101348), PI3K Inhibitor (z.B. BAY 1082439, BAY 80-6946, ATU-027, SF-1126, DS-7423, GSK-2126458, Buparlisib, PF-4691502, BYL-719, XL-147, XL-765, Idelalisib), SYK (Spleen Tyrosine Kinase) Inhibitor (z.B. Fostamatinib, Excellair, PRT-062607), Bisphosphonate (z.B. Etridonat, Clodronat, Tiludronat, Pamidronat, Alendronsäure, Ibandronat, Risedronat, Zoledronat), Rituximab, Cyclophosphamide, Doxorubicin, Vincristine, Chlorambucil, Fludarabin, Dexamethasone, Cladribin, Prednisone.
Für die Tumortherapie geeignet ist auch eine Kombination aus nicht-medikamentöser Therapie wie Chemotherapie, Radiotherapie oder Phototherapie, die von einer medikamentösen Behandlung mit den erfindungsgemäßen IRAK4-Inhibitoren begleitet werden oder die nach Beendigung der nichtmedikamentösen Tumortherapie wie Chemotherapie, Radiotherapie oder Phototherapie durch eine medikamentöse Behandlung mit den erfindungsgemäßen IRAK4-Inhibitoren ergänzt werden.

Die erfindungsgemäßen IRAK4-Inhibitoren können außer mit den bereits genannten auch mit folgenden Wirkstoffen kombiniert werden:
Wirkstoffe für die Alzheimertherapie wie beispielsweise Acetylcholinesterase-Hemmern (z.B. Donepezil, Rivastigmine, Galantamin, Tacrin), NMDA (N-Methyl-D-Aspartat)-Rezeptorantagonisten (Z.B. Memantine); L-DOPA/Carbidopa (L-3,4-Dihydroxyphenylalanin), COMT (Catechol-O-Methyltransferase)-Hemmer (z.B. Entacapon), Dopaminagonisten (z.B. Ropinrol, Pramipexol, Bromocriptin), MAO-B (Monoaminooxidase-B)-Hemmer (z.B. Selegilin), Anticholinergika (z.B.. Trihexyphenidyl) und NMDA-Antagonisten (z.B. Amantadin) zur Behandlung von Parkinson; Beta-Interferon (IFN-beta) (z.B. IFN beta-lb, IFN beta-la Avonex® und Betaferon®), Glatirameracetat, Immunglobuline, Natalizumab, Fingolimod und Immunsuppressiva wie Mitoxantron, Azathioprin und Cyclophosphamid zur Behandlung der Multiplen Sklerose; Substanzen zur Behandlung von pulmonalen Erkrankungen wie beispielsweise Beta-2-Sympathomimetika (z.B. Salbutamol), Anticholinergika (z.B. Glycopyrronium), Methylxanthine (z.B. Theophyllin), Leukotrienrezeptor-Antagonist (z.B. Montelukast), PDE-4 (Phosphodiesterase Typ 4) -Hemmer (z.B. Roflumilast), Methotrexat, IgE Antikörper, Azathioprin und Cyclophosphamid, Kortisolhaltige Präparate; Substanzen zur Behandlung von Osteoarthritis wie non-steroidale anti-inflammatorische Substanzen (NSAIDs). Neben den zwei genannten Therapien sind für rheumatoide Erkrankungen wie rheumatoide Arthritis und juvenile idiopathische Arthritis Methotrexat und Biologika zur B-Zell- und T-Zell-Therapie (z.B. Rituximab, Abatacept) zu nennen. Neurotrophe Substanzen wie Acetylcholinesterase Inhibitoren (z.B. Donepezil), MAO (Monoaminooxidase) Inhibitoren (z.B. Selegilin), Interferone und Antikonvulsivum (z.B. Gabapentin); Wirkstoffe zur Behandlung von kardiovaskulären Erkrankungen wie beta-Blocker (z.B. Metoprolol), ACE Inhibitoren (z.B. Benazepril), Diuretika (z.B. Hydrochlorothiazid), Calciumkanal-Blocker (z.B. Nifedipin), Statine (z.B. Simvastatin); Anti-Diabetika wie z.B. Metformin und Glibenclamid, Sulfonylharnstoffe (z.B. Tolbutamid) und Insulintherapie zur Behandlung von Diabetes und metabolisches Syndrom. Wirkstoffe wie Mesalazin, Sulfasalazin, Azathioprin, 6-Mercaptopurin oder Methotrexat, probiotische Bakterien (Mutaflor, VSL#3®, Lactobacillus GG, Lactobacillus plantarum, L. acidophilus, L. casei, Bifidobacterium infantis 35624, Enterococcus fecium SF68, Bifidobacterium longum, Escherichia coli Nissle 1917), Antibiotika wie beispielsweise Ciprofloxacin und Metronidazol, Antidiarrhoika wie z.B. Loperamid oder Laxativa (Bisacodyl) zur Behandlung von chronisch-entzündlichen Darmerkrankungen. Immunsuppressiva wie Glucocorticoide und non-steroidale anti-inflammatorische Substanzen (NSAIDs), Cortison, Chloroquin, Cyclosporin, Azathioprin, Belimumab, Rituximab, Cyclophosphamid zur Behandlung von Lupus erythematodes. Beispielhaft aber nicht ausschließlich Calcineurinhemmer (z.B. Tacrolimus und Ciclosporin), Zellteilungshemmer (z.B. Azathioprin, Mycophenolat Mofetil, Mycophenolsäure, Everolimus oder Sirolimus), Rapamycin, Basiliximab, Daclizumab, Anti-CD3-Antikörper, Anti-T-Lymphozytenglobulin/Anti-Lymphozytenglobulin bei Organtransplantation. Vitamin D3-Analoga wie beispielsweise Calcipotriol, Tacalcitol oder Calcitriol, Salicylsäure, Harnstoff, Ciclosporin, Methotrexat, Efalizumab bei dermatologischen Erkrankungen.

Ein weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, über das Ohr oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### Herstellung der erfindungsgemäßen Verbindungen

Die Herstellung der erfindungsgemäßen Verbindungen wird durch die folgenden Syntheseschemata verdeutlicht.

Die in Syntheseschema 1 aufgeführten Intermediate 0 können analog zu Literatur- und Patentvorschriften zum Beispiel aus 4-substituierten 2-Fluor-5-nitrobenzaldehyden oder 2-Chlor-5-nitrobenzaldehyden hergestellt werden oder sind kommerziell erhältlich. Zur Herstellung werden 4-substituierte 2-Fluor-5-nitrobenzaldehyde mit Hydrazin umgesetzt (J. Med. Chem., 2013, 56, 4343). Die entstandenen 5-Nitro-indazole (Intermediate 0) können beispielsweise mit Palladium auf Kohle durch Hydrierung (US201228984, WO200671940, US2003153596, EP2045253) oder Transferhydrierung (Eur. J. Med. Chem., 2010, 45, 5520) oder durch Reaktion mit Eisen (J. Chem. Soc., 1955, 2412) oder Zinn(II)chlorid (Bioorg. Med. Chem., 2004, 12, 2115, US201215962) reduziert werden zu den entsprechenden 5-Aminoindazolen. Die Intermediate 1a können zu den Intermediaten 1b umgesetzt werden. Der Rest R² kann auf unterschiedlichen Wegen eingeführt werden, beispielsweise über eine Alkylierung mit Alkylhalogeniden (Bioorg. Med. Chem., 2010, 18, 4801) oder Alkylsufonaten oder über eine reduktive Aminierung durch Umsetzung mit Aldehyden (WO2009102498) beziehungsweise Ketonen (EP140325). Als Reduktionsmittel können verschiedene Hydrid-donoren wie zum Beispiel Natriumborhydrid, Natriumcyanoborhydrid oder Natriumtrisacetoxyborhydrid eingesetzt werden. Alternativ besteht auch die Möglichkeit, den anilinischen Stickstoff der Intermediate 1a durch Verwendung eines Acylhalogenides oder eines Carbonsäureanhydrides zunächst zu acylieren und das Amid dann unter Verwendung eines geeigneten Reduktionsmittels zu dem entsprechenden Amin zu reduzieren, wodurch ebenfalls Intermediate 1b erhalten werden. Als Reduktionsmittel können zum Beispiel Lithiumaluminiumtetrahydrid (J. Am. Chem. Soc., 1954, 76, 1384), Boran als Komplex mit Dimethylsulfid (Synthetic Communications, 1991, 21, 1579) beziehungsweise Tetrahydrofuran (Org. and Biomol. Chem., 2012, 10, 8692) oder Natriumbis(2-methoxyethoxy)aluminiumhydrid (WO200873461) eingesetzt werden.
Die Intermediate 1a und 1b können am anilinischen Stickstoff mit einer bekannten und in der Literatur beschriebenen Schutzgruppe versehen werden (Protecting Groups, Philip J. Kocienski, 3rd Revised edition (9. Februar 2005), Thieme, Kapitel 8; Greene's Protective Groups in Organic Synthesis, Peter G. M. Wuts, Theodora W. Greene, 4. Auflage (8. Dezember 2006), Wiley-Interscience, Kapitel 7), wodurch die Intermediate 2 erhalten werden. Bevorzugt ist als Schutzgruppe die tert-Butyloxycarbonylgruppe (BOC-Schutzgruppe). Die Einführung der BOC-Schutzgruppe erfolgt bevorzugt mit Di-tert-butyldicarbonat in Gegenwart einer Base wie zum Beispiel *N*,*N*-Diisopropylethylamin oder Triethylamin.
Die Intermediate 2 können mit im Carbonsäurebereich halogenierten Carbonsäureestern wie zum Beispiel Methylbromacetat, Ethylbromacetat, tert-Butylbromacetat, Benzylbromacetat, Ethyl-3-brompropanoat oder Ethyl-2-brompropanoat unter basischen Bedingungen zu einer Mischung der entsprechenden regioisomeren 1- und 2-alkylierten Indazolverbindungen umgesetzt werden (Organic Letters, 2009, 11, 5054; WO200474284; US2009286800; WO200919167; WO201297744; J. Med. Chem., 2007, 50, 3101; Molecules, 2006, 11, 86). Bevorzugt ist hierbei die Umsetzung mit *N*,*N*-Dicyclohexylmethylamin in Tetrahydrofuran oder *N,N*-Dimethylformamid zwischen 25°C und 100°C (J. Org. Chem. 2006, 71, 5392). Ebenfalls bevorzugt ist die Umsetzung in Gegenwart von Kaliumcarbonat in *N*,*N*-Dimethylformamid. Die Mischungen aus den regioisomeren 1- und 2-alkylierten Indazolverbindungen können durch Säulenchromatographie oder präparativer HPLC getrennt werden, wodurch die 2-alkylierten Indazolverbindungen (Intermediate 3) erhalten werden können.
Die Umsetzung der Intermediate 3 zu den Intermediaten 4 kann unter bekannten Bedingungen (Protecting Groups, Philip J. Kocienski, 3rd Revised edition (9. Februar 2005), Thieme, Kapitel 6; Greene's Protective Groups in Organic Synthesis, Peter G. M. Wuts, Theodora W. Greene, 4. Auflage (8. Dezember 2006), Wiley-Interscience, Kapitel 5; WO200919167 A1) erfolgen. Bevorzugt ist hierbei die Verseifung mit Lithiumhydroxid oder Lithiumhydroxid Monohydrat in einer Mischung aus Tetrahydrofuran und Wasser (J. Med. Chem., 2012 , 55, 1318, Bioorg. Med. Chem., 2009 , 17, 7113). Gegebenenfalls kann auch Ethanol oder Methanol zugesetzt werden.
Die Intermediate 4 können mit Aminen zu den entsprechenden Intermediaten 5 umgesetzt werden. Hierbei können verschiedene in der Literatur bekannte Kupplungsreagenzien eingesetzt werden (Amino Acids, Peptides and Proteins in Organic Chemistry. Vol.3 - Building Blocks, Catalysis and Coupling Chemistry, Andrew B. Hughes, Wiley, Kapitel 12 - Peptide-Coupling Reagents, 407-442; Chem. Soc. Rev., 2009, 38, 606). Der Einsatz von 1-(3-Dimethylaminopropyl)-3-ethylcarbodiimidhydrochlorid in Kombination mit 1-Hydroxy-1H-benzotriazol Hydrat ist bevorzugt (WO2012107475; Bioorg. Med. Chem. Let., 2008 , 18, 2093).
Die so erhaltenen Intermediate 5 können zu den Intermediaten 6 umgesetzt werden. Die Abspaltung der Schutzgruppe am anilinischen Stickstoff kann unter bekannten Reaktionsbedingungen durchgeführt werden (Protecting Groups, Philip J. Kocienski, 3rd Revised edition (9. Februar 2005), Thieme, Kapitel 8; Greene's Protective Groups in Organic Synthesis, Peter G. M. Wuts, Theodora W. Greene, 4. Auflage (8. Dezember 2006), Wiley-Interscience, Kapitel 7). Bevorzugt ist die Abspaltung der tert-Butyloxycarbonyl-Schutzgruppe mit Trifluoressigsäure in Dichlormethan (Bioorg. Med. Chem. Lett., 2011, 21, 6274; J. Med. Chem., 2008, 51, 1904; WO201353051).
Die Intermediate 6 können mit heterocyclischen Carbonsäuren zu den Verbindungen der allgemeinen Formel (I) umgesetzt werden mit Hilfe der bereits bei der Herstellung der Intermediate 5 erwähnten literaturbekannten Kupplungsreagenzien. Auch hier ist der Einsatz von 1-(3-Dimethylaminopropyl)-3-ethylcarbodiimidhydrochlorid in Kombination mit 1-Hydroxy-1H-benzotriazol Hydrat bevorzugt (US2006194801).

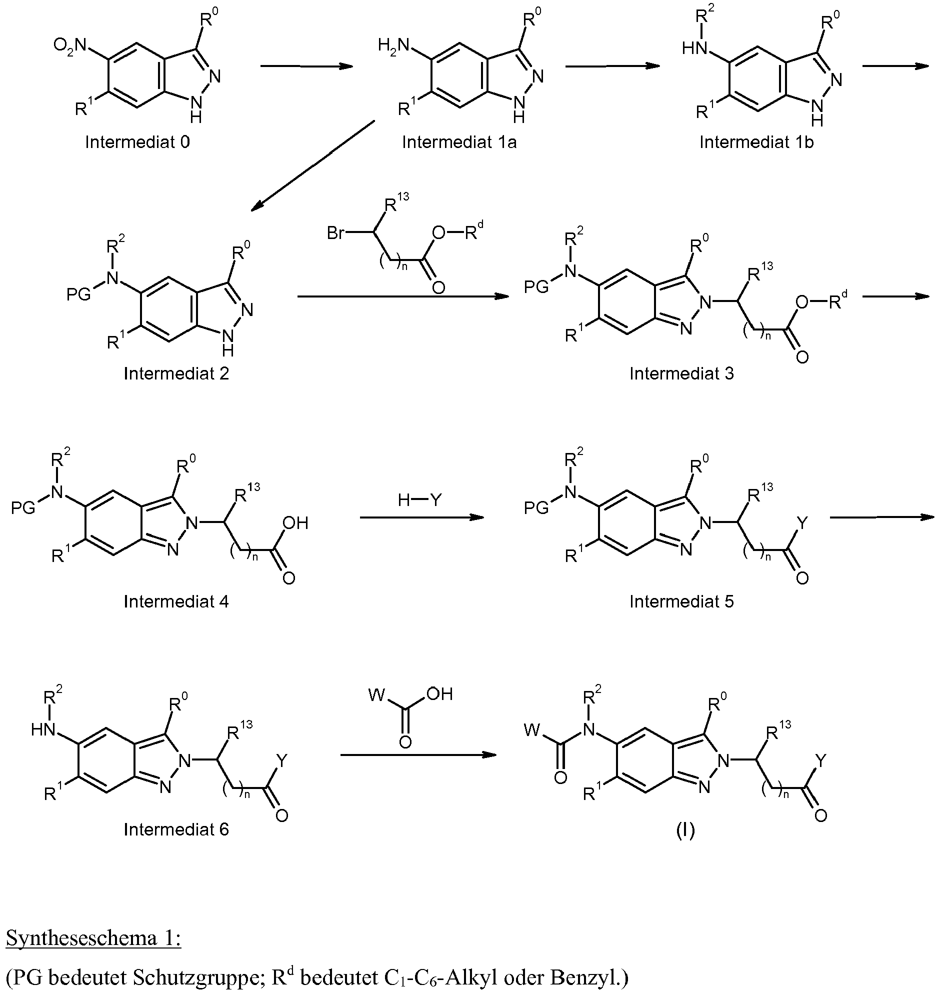

Alternativ können Intermediate 5 auch direkt aus Intermediaten 2 erhalten werden wie in Syntheseschema 1a verdeutlicht. Als Reagenzien werden halogenierte Carbonsäureamide verwendet. Die Reaktionsbedingungen sind identisch mit denen der Herstellung von Intermediat 3 aus Intermediat 2. Bevorzugt ist die Umsetzung mit 2-Bromacetamiden in Gegenwart der Base N,N-Dicyclohexylmethylamin. Besonders bevorzugt ist die Umsetzung mit 2-Brom-1-(morpholin-4-yl)ethanon.

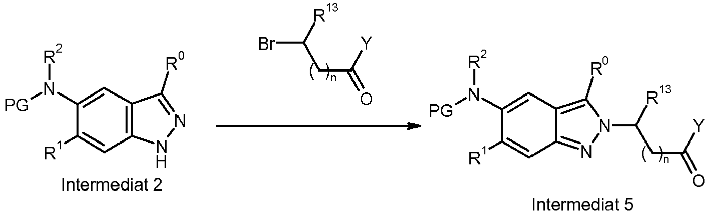

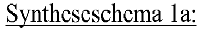

Wie in Syntheseschema 2 verdeutlicht wird, können die Intermediate 3 auch zunächst zu den Intermediaten 7 umgesetzt werden (J. Am. Chem. Soc., 2009, 131, 3342; EP2522657). Wenn PG tert-Butyloxycarbonyl bedeutet, wird bevorzugt Trifluoressigsäure in Dichlormethan eingesetzt (WO201062171). Die Intermediate 7 können mit heterocyclischen Carbonsäuren zu den Intermediaten 8 umgesetzt werden. Hierbei werden wie in Syntheseschema 1 Kupplungsreagenzien verwendet. Als Kupplungsreagenz wird bevorzugt 1-(3-Dimethylaminopropyl)-3-ethylcarbodiimidhydrochlorid in Kombination mit 1-Hydroxy-1H-benzotriazol Hydrat eingesetzt. Die Intermediate 8 können analog zu Syntheseschema 1 verseift werden, wobei die Verseifung mit Lithiumhydroxid oder Lithiumhydroxid Monohydrat in einer Mischung aus Tetrahydrofuran und Wasser bevorzugt ist. Gegebenenfalls kann auch Ethanol oder Methanol zugesetzt werden.
Die so gebildeten Intermediate 9 können zu den Verbindungen der allgemeinen Formel (I) umgesetzt werden. Die Kupplung mit Aminen erfolgt analog Syntheseschema 1 durch Einsatz literaturbekannter Kupplungsreagenzien. Die Verwendung von 1-(3-Dimethylaminopropyl)-3-ethylcarbodiimidhydrochlorid in Kombination mit 1-Hydroxy-1H-benzotriazol Hydrat ist bevorzugt.

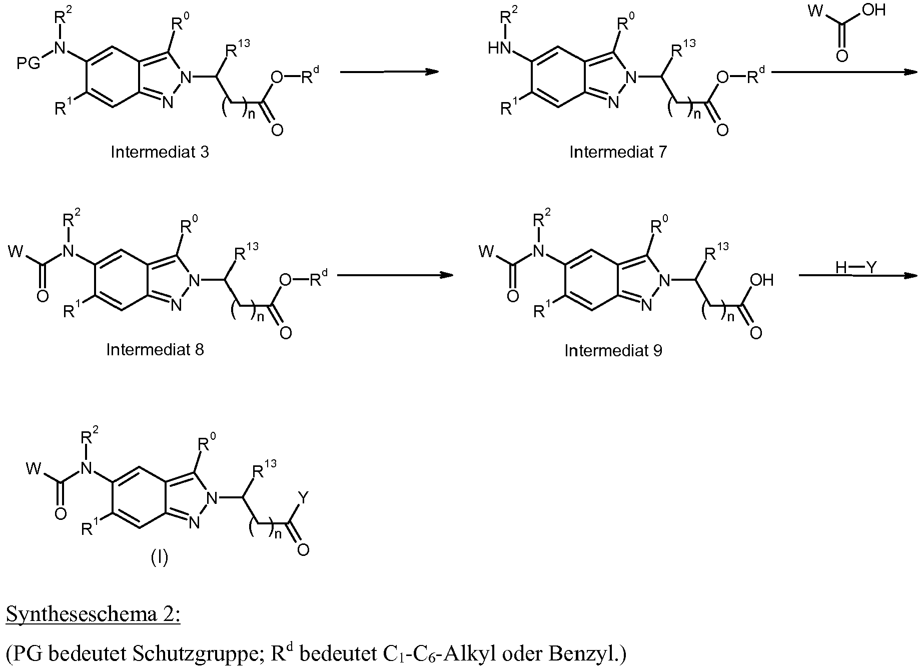

Die Intermediate 2b lassen sich wie in Syntheseschema 3 verdeutlicht herstellen. Durch Umsetzung der Intermediate 1a mit einem Überschuss Di-tert-butyldicarbonat wird eine Mischung der Intermediate 10 und 11 erhalten, welche selektiv an den Positionen 1 bzw. 2 verseift werden können, wodurch die Intermediate 2b erhalten werden. Die Abspaltung wird bevorzugt mit Natriumcarbonat in einer Mischung aus *N,N*-Dimethylformamid und Wasser zwischen 50°C und 100°C für 12 - 36 Stunden durchgeführt (Tet. Lett., 2006, 47, 8575).

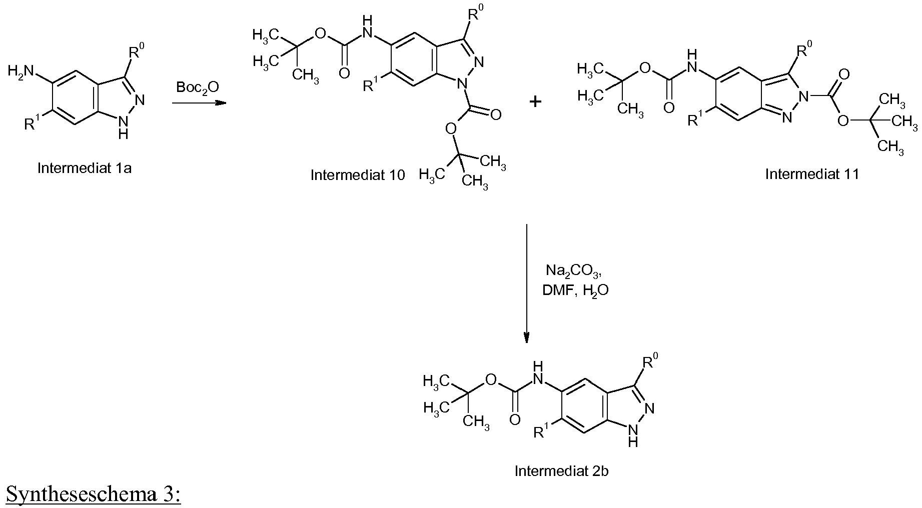

Die Intermediate 8a lassen sich wie in Syntheseschema 4 beschrieben ausgehend von den Intermediaten 1a zum Beispiel mit der Bedeutung R¹ = Cl in einer mehrstufigen Synthesesequenz herstellen. Hierzu wird zunächst eines der Stickstoffatome im Indazolring geschützt, bevorzugt das Stickstoffatom in der Position 1 (WO200958924). Bevorzugt ist als Schutzgruppe die tert-Butyloxycarbonylgruppe (BOC-Schutzgruppe). Die Einführung der BOC-Schutzgruppe erfolgt bevorzugt mit Di-tert-butyldicarbonat in Gegenwart einer Base wie zum Beispiel *N,N-*Diisopropylethylamin oder Triethylamin.
Die Intermediate 12 können unter den oben erwähnten Kupplungsbedingungen mit heterocyclischen Carbonsäuren acyliert werden, wodurch die Intermediate 13 erhalten werden. Bevorzugt ist der Einsatz von 1-(3-Dimethylaminopropyl)-3-ethylcarbodiimidhydrochlorid in Kombination mit 1-Hydroxy-1H-benzotriazol Hydrat.
Die Schutzgruppe der Intermediate 13 am Indazolring kann durch in der Literatur bekannte Reaktionsbedingungen (Protecting Groups, Philip J. Kocienski, 3rd Revised edition (9. Februar 2005), Thieme, Kapitel 8; Greene's Protective Groups in Organic Synthesis, Peter G. M. Wuts, , 4. Auflage (8. Dezember 2006), Wiley-Interscience, Kapitel 7) abgespalten werden. Bevorzugt ist der Einsatz von Trifluoressigsäure in Dichlormethan, um eine BOC-Schutzgruppe abzuspalten. Die Intermediate 14 können zu einer Mischung der entsprechenden regioisomeren 1- und 2-alkylierten Indazol-Verbindungen umgesetzt werden. Durch Trennung der Regioisomeren werden die gewünschten 2-alkylierten Indazolderivate (Intermediate 8a) erhalten (J. Org. Chem. 2006, 71, 5392). Dabei werden die Reaktionsbedingungen wie bei der Herstellung der Intermediate 3 aus den Intermediaten 2 verwendet (Syntheseschema 1). Der Einsatz von *N*,*N*-Dicyclohexylmethylamin in Tetrahydrofuran oder *N,N*-Dimethylformamid ist bevorzugt.

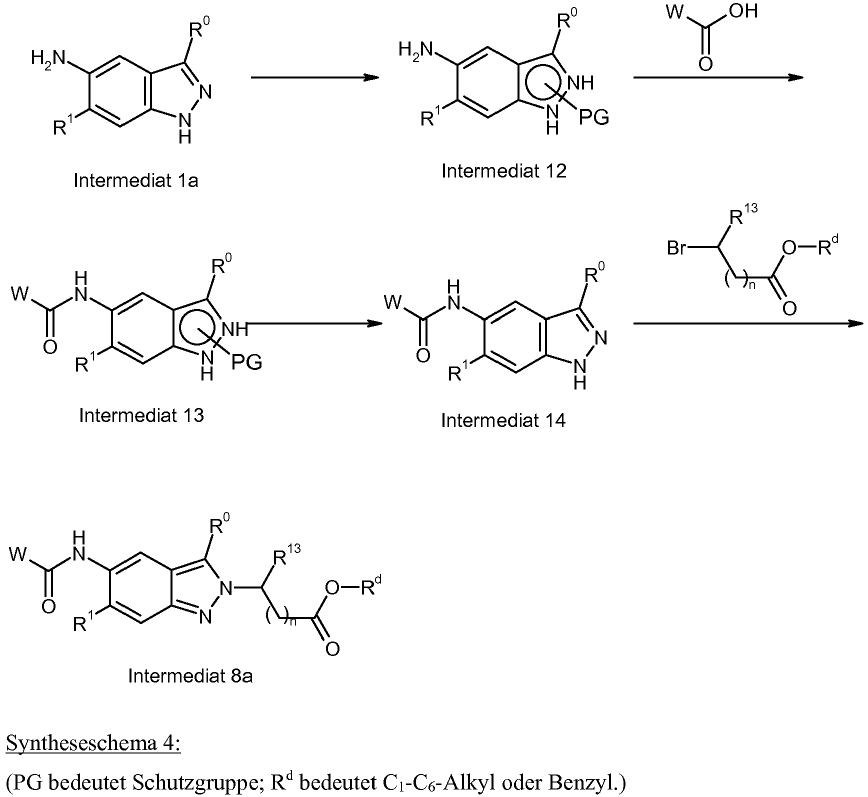

Die Intermediate 14 lassen sich in einigen Fällen auch wie in Syntheseschema 5 beschrieben herstellen. Die Intermediate 1a werden regioselektiv am anilinischen Stickstoff mit heterocyclischen Carbonsäuren acyliert. Hierbei werden die oben genannten Kupplungsreagenzien verwendet. Bevorzugt ist die Kombination aus 1-(3-Dimethylaminopropyl)-3-ethylcarbodiimidhydrochlorid und 1-Hydroxy-1H-benzotriazol Hydrat unter Verwendung von Triethylamin als Base (EP1403255; WO2005 82890; US2006194801; Bioorg. Med. Chem. Lett., 2007, 17, 3550).

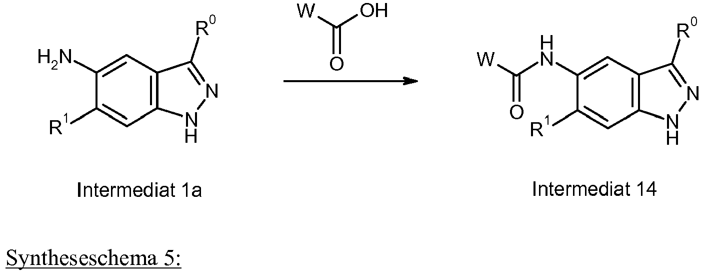

Gemäß Syntheseschema 5-1 können aus Intermediaten 14a mit der Bedeutung R^{1c} = -CO₂Me oder -CO₂Et, bevorzugt -CO₂Me, mit Hilfe einer Grignard-Reaktion (Organikum, 19. Auflage, Johann Ambrosius Barth Leipzig, S. 515 - 520) durch Verwendung von Methylmagnesiumbromid, Methylmagnesiumchlorid, Ethylmagnesiumbromid oder Ethylmagnesiumchlorid Intermediate 14b mit der Bedeutung R^{1d} = -C(CH₃)₂OH oder -C(CH₂CH₃)₂OH erhalten werden. Die Umsetzung mit Methylmagnesiumbromid ist bevorzugt um Intermediate mit der Bedeutung R^{1d} = -C(CH₃)₂OH zu erhalten. Die Intermediate mit der Formel 14b können dann analog zu Syntheseschema 4 und dann gemäß Syntheseschema 2 zu erfindungsgemäßen Verbindungen mit der Bedeutung R¹ = -C(CH₃)₂OH oder -C(CH₂CH₃)₂OH umgesetzt werden. Alternativ und bevorzugt können die Intermediate 14b auch durch Umsetzung mit 2-Chloracetamiden oder 2-Bromacetamiden zu erfindungsgemäßen Verbindungen der Formel (I) mit der Bedeutung R¹ = -C(CH₃)₂OH oder -C(CH₂CH₃)₂OH umgesetzt werden. Dabei können Reaktionsbedingungen wie bei Syntheseschema 1a verwendet werden. Der Einsatz von N,N-Dicyclohexylmethylamin in Tetrahydrofuran oder N,N-Dimethylformamid ist bevorzugt. Besonders bevorzugt ist die Verwendung von 2-Brom-1-(morpholin-4-yl)ethanon.

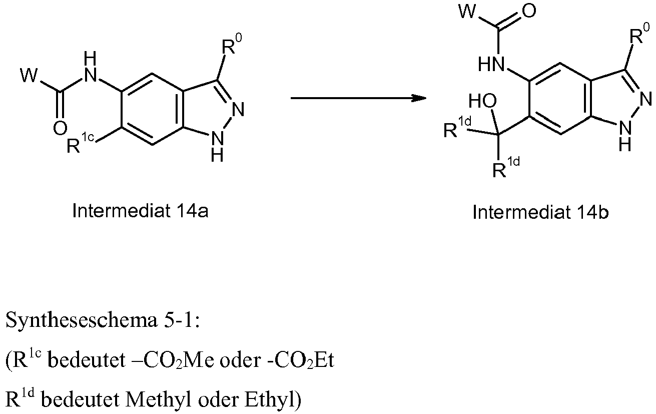

Eine Teilmenge der erfindungsgemäßen Verbindungen kann wie in Schema 6 verdeutlicht hergestellt werden. Die Edukte der allgemeinen Formel (Ia) werden in Gegenwart eines PalladiumKatalysators mit einer Organometallverbindung, welche den Rest R^{1b} überträgt, umgesetzt. Der Rest R^{1b} steht für C₁-C₆-Alkyl, C₃-C₈-Cycloalkyl, Heterocycloalkyl, C₃-C₈-Cycloalkyl-C₁-C₄-alkyl, Heterocycloalkyl-C₁-C₄-alkyl, C₂-C₆-Alkenyl, C₂-C₆-Alkinyl, Aryl, 5- bis 10-gliedriges Heteroaryl, Aryl-C₁-C₄-alkyl oder 5- oder 6-gliedriges Heteroaryl-C₁-C₄-alkyl, welches gegebenenfalls ein-oder mehrfach, gleich oder verschieden substituiert sein kann mit geschütztem Hydroxy, Halogen, Cyano, C(=O)OR^{a}, S(=O)₂-C₁-C₆-Alkyl, geschütztem NH₂, geschütztem NHR^{a} oder N(R^{a})R^{b}. Für die Umsetzung können die bekannten Kupplungsreaktionen unter Verwendung von Organomagnesiumverbindungen (Kumada-Reaktion: J. Organomet. Chem., 2002, 653, 288; Handbook of Organopalladium Chemistry for Organic Synthesis, 2002, 1, 335; Top. Curr. Chem., 2002, 219, 1), Organoborverbindungen (Suzuki-Reaktion: Pure Appl. Chem., 1985, 57, 1749; Chem. Rev., 1995, 95, 2457; Advances in Metal-Organic Chemistry, 1998, 6, 187; Angew. Chem., Int. Ed. Engl., 2004, 43, 2201, Top. Curr. Chem., 2002, Vol. 219, 248), Organozinnverbindungen (Stille-Reaktion: Angew. Chem., 1986, 98, 504; Synthesis, 1992, 803; Org. React., 1997, 50, 1; Angew. Chem., Int. Ed. Engl., 2004, 43, 4704; J. Organomet. Chem., 2002, 653, 50) oder Organozinkverbindungen (Negishi-Reaktion: Acc. Chem. Res., 1982, 15, 340; Metal-Catalyzed Cross-coupling Reactions, F. Diedrich, P. J. Stang, Wiley-VCH, 1998, 1; Aust. J. Chem. 2004, 57, 107; Handbook of Organopalladium Chemistry for Organic Synthesis, E.-I. Negishi, Y. Dumond, 2002, Vol. 1, 767) in Gegenwart einer Palladiumverbindung (z.B. Palladium(II)acetat, Tetrakis(triphenylphosphin)palladium, Allylchlor(1,3 -bis(2,6-di-isopropylphenyl)imidazol-2-yliden)palladium, Tris(dibenzylideneaceton)dipalladium(0), eines Liganden (z.B. 2,2'-Bis(diphenylphosphino)-1,1'-binaphthyl,Triphenylphosphin, 1,1'-Bis(diphenylphosphino)ferrocen, 2-Dicyclohexylphosphino-2',4',6'-triisopropylbiphenyl, 4,5-Bis(diphenylphosphino)-9,9-dimethylxanthen, 2-(Dicyclohexylphosphino)3,6-dimethoxy-2',4',6'-triisopropyl-1,1'-biphenyl, 1,1'-Bis(di-o-tolylphosphino)ferrocen) in einem Lösungsmittel (z.B. N,N-Dimethylformamid, Toluol, Xylol, Tetrahydrofuran, Dioxan, Dimethoxyethan, tert.-Butyl-methylether) mit einer Base (z.B. Natrium-tert.-butanolat, Kalium-tert.-butanolat, Natriumhydrid, Kaliumhydrid, Kaliumhexamethyldisilazid, Trikaliumphosphat, Cäsiumcarbonat) bei einer Temperatur von 40-200 °C verwendet werden. Die Temperatur ist auch vom Lösungsmittel abhängig. Alternativ zu den oben genannten Palladiumverbindungen können auch andere Palladiumverbindungen, welche so genannte Präkatalysatoren darstellen (z.B. Chloro[2-(dicyclohexylphosphino)-3,6-dimethoxy-2',4',6'-triisopropyl-1,1'-biphenyl][2-(2-aminoethyl)phenyl]palladium(II) oder (2-Dicyclohexylphosphino-2',4',6'-triisopropyl-1,1'-biphenyl)[2-(2-aminoethyl)phenyl)]palladium(II) chlorid) eingesetzt werden. Bevorzugt werden für die Umsetzungen Tetrakis(triphenylphosphin)palladium, Palladium(II)acetat mit 2,2'-Bis(diphenylphosphino)-1,1'-binaphthyl oder 4,5-Bis(diphenylphosphino)-9,9-dimethylxanthen oder Allylchlor(1,3-bis(2,6-diisopropylphenyl)imidazol-2-yliden)palladium eingesetzt. Der Einsatz von Tetrakis(triphenylphosphin)palladium ist besonders bevorzugt. Die Reste R^{a} und R^{b} können hierbei die in der allgemeinen Formel (I) beschriebenen Definitionen annehmen. Für den Fall, dass die Elektrophile geschützte Hydroxyfunktionen oder geschütztes NH₂ oder NHR^{a} tragen, kann in einem zusätzlichen Syntheseschritt nach einem gängigen und in der Literatur beschriebenen Verfahren diese Schutzgruppe wieder abgespalten werden (Protecting Groups, Philip J. Kocienski, 3rd Revised edition (9. Februar 2005), Thieme; Greene's Protective Groups in Organic Synthesis, Peter G. M. Wuts, Theodora W. Greene, 4. Auflage (8. Dezember 2006), Wiley-Interscience).
In dem Fall, dass R^{1b} Cyanid bedeutet, kann die Umsetzung der Edukte der allgemeinen Formel (Ia) in Gegenwart einer der oben beschriebenen Palladiumverbindungen und in Gegenwart von Zinkcyanid in einem der oben beschriebenen Lösungsmittel bei einer Temerpatur von 40 - 200°C erfolgen. Das Erhitzen der Reaktionsmischung kann hierbei entweder durch thermisches Erhitzen oder in der Mikrowelle erfolgen. Der Einsatz von Tetrakis(triphenylphosphin)palladium in *N,N-*Dimethylformamid bei einer Temperatur von 150°C in der Mikrowelle ist hierbei besonders bevorzugt.
Zusätzlich kann auch eine Umsetzung der Edukte der allgemeinen Formel (Ia) mit primären oder sekundären Aminen oder mit Alkoxiden erfolgen (Buchwald-Hartwig Reaktion: Chemtracts: Inorg. Chem., 1996, 8, 1; Curr. Org. Chem. 1997, 1, 287; Synlett 1997, 329; Angew. Chem., Int. Ed. Engl., 1998, 37, 2046; Pure Appl. Chem. 1999, 71, 1425; Top. Curr. Chem. 2002, 219, 131), wodurch Verbindungen der allgemeinen Formel (Ib) mit R^{1b} = NHR^{a}, NR^{a}R^{b}, NHC(=O)R^{a} oder OR^{a} erhalten werden können. Die Umsetzung erfolgt in Gegenwart einer Palladiumverbindung (z.B.Palladium(II)acetat ,Tris(dibenzylideneaceton)dipalladium(0) ), eines Liganden (z.B. 2,2'-Bis(diphenylphosphino)-1,1'-binaphthyl, Triphenylphosphin, 1,1'-Bis(diphenylphosphino)ferrocen, 2-Dicyclohexylphosphino-2',4',6'-triisopropylbiphenyl, 4,5-Bis(diphenylphosphino)-9,9-dimethylxanthen, 2-(Dicyclohexylphosphino)3,6-dimethoxy-2',4',6'-triisopropyl-1,1'-biphenyl, 1,1'-Bis(di-o-tolylphosphino)ferrocen) in einem Lösungsmittel (z.B. *N*,*N*-Dimethylformamid, Toluol, Xylol, Tetrahydrofuran, Dioxan, Dimethoxyethan, tert.-Butyl-methylether) mit einer Base (z.B. Natrium-tert.-butanolat, Kalium-tert.-butanolat, Lithiumhexamethyldisilazid, Natriumhexamethyldisilazid, Kaliumhexamethyldisilazid, Kaliumcarbonat, Cäsiumcarbonat) bei einer Temperatur von 40-200 °C. Die Temperatur ist auch vom Lösungsmittel abhängig. Alternativ zu den oben genannten Palladiumverbindungen können auch andere Palladiumverbindungen, welche so genannte Präkatalysatoren darstellen (z.B. Chloro[2-(dicyclohexylphosphino)-3,6-dimethoxy-2',4',6'-triisopropyl-1,1'-biphenyl][2-(2-aminoethyl)phenyl]palladium(II) oder (2-Dicyclohexylphosphino-2',4',6'-triisopropyl-1,1'-biphenyl)[2-(2-aminoethyl)phenyl)]palladium(II) chloride) eingesetzt werden. Die Umsetzung mit den letztgenannten Präkatalysatoren ist dabei bevorzugt.

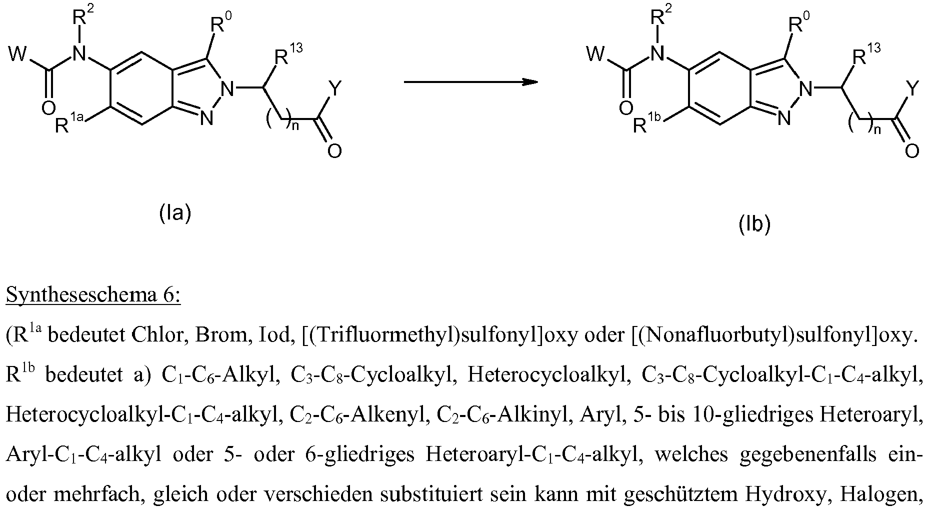

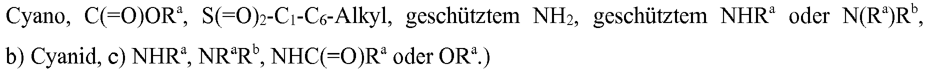

Eine Teilmenge der erfindungsgemäßen Verbindungen lässt sich wie in Syntheseschema 7 dargestellt herstellen, indem Edukte der allgemeinen Formel (Ic) mit Elektrophilen R^{e}-X wie Alkylhalogeniden, Alkylsufonaten, Arylhalogeniden, Arylsulfonaten, Hetarylhalogeniden oder Hetarylsulfonaten umgesetzt werden. X hat die Bedeutung von Chlor, Brom, Iod, O(S=O)₂CH₃, O(S=O)₂C₆H₄CH₃ oder O(S=O)₂CF₃, wobei X bevorzugt Chlor, Brom oder Iod und besonders bevorzugt Brom bedeutet.
R^{e} steht für C₁-C₆-Alkyl, C₃-C₈-Cycloalkyl, Heterocycloalkyl, C₃-C₈-Cycloalkyl-C₁-C₄-alkyl, Heterocycloalkyl-C₁-C₄-alkyl, C₂-C₆-Alkenyl, C₂-C₆-Alkinyl, Aryl, 5- bis 10-gliedriges Heteroaryl, Aryl-C₁-C₄-alkyl oder 5- oder 6-gliedriges Heteroaryl-C₁-C₄-alkyl, welche gegebenenfalls ein- oder mehrfach, gleich oder verschieden substituiert sein können mit Hydroxy (gegebenenfalls geschützt), Halogen, Cyano, C(=O)OR^{a}, S(=O)₂-C₁-C₆-Alkyl, geschütztem NH₂, geschütztem NHR^{a} oder NR^{a}R^{b}. Die Reste R^{a} und R^{b} können hierbei die in der allgemeinen Formel (I) beschriebenen Definitionen annehmen. Für den Fall, dass die Elektrophile geschützte Hydroxyfunktionen oder geschütztes NH₂ oder NHR^{a} tragen, kann in einem zusätzlichen Syntheseschritt nach einem gängigen und in der Literatur beschriebenen Verfahren diese Schutzgruppe wieder abgespalten werden (Protecting Groups, Philip J. Kocienski, 3rd Revised edition (9. Februar 2005), Thieme; Greene's Protective Groups in Organic Synthesis, Peter G. M. Wuts, Theodora W. Greene, 4. Auflage (8. Dezember 2006), Wiley-Interscience). Hat R^{e}-X die Bedeutung Alkylhalogenid oder Alkylsulfonat können geeignete Basen wie z.B. Natrium-tert.-butanolat, Kalium-tert.-butanolat, Natriumhydrid, Kaliumhydrid, Kaliumhexamethyldisilazid, Trikaliumphosphat, Natriumcarbonat, Kaliumcarbonat, Cäsiumcarbonat verwendet werden (WO2003101379 A2; Bioor. Med. Chem., 2008, 16, 1966; J. Med. Chem., 2012, 55, 7141). Ferner können weitere Zusätze wie z.B. Natriumiodid, Kaliumiodid, Cäsiumiodid für die Alkylierung verwendet werden. Die Umsetzung mit aktivierten Arylhalogeniden, Arylsulfonaten, Hetarylhalogeniden oder Hetarylsulfonaten (elektronenziehende Reste oder Heteroatome in der ortho- und/oder para-Position zum Halogenid bzw. Sulfonat) kann durch nukleophile aromatische Substitution am aktivierten Arylhalogeniden, Arylsulfonaten, Hetarylhalogeniden oder Hetarylsulfonaten erfolgen, wobei ebenfalls geeignete Basen wie z.B. Natrium-tert.-butanolat, Kalium-tert.-butanolat, Natriumhydrid, Kaliumhydrid, Kaliumhexamethyldisilazid, Trikaliumphosphat, Natriumcarbonat, Kaliumcarbonat oder Cäsiumcarbonat für die Umsetzung zum Einsatz kommen können (WO200795124 A2, EP2103620 A1). Ferner kann die Arylierung beziehungsweise Heteroarylierung der Edukte der allgemeinen Formel (Ic) in Syntheseschema 7 durch Umsetzung mit Arylhalogeniden, Arylsulfonaten, Hetarylhalogeniden oder Hetarylsulfonaten mit Hilfe eines in der Literatur bekannten Übergangsmetallkatalysators auf Basis von Kupfer (z.B. Kupfer(I)iodid, Kupfer(I)oxid, Kupfer(II)acetat) (Russ. Chem. Rev., 1974, 43, 1443; Tetrahedron, 2000, 56, 5054; Synlett, 2003, 2428; Angew. Chem., Int. Ed. Engl., 2003, 42, 5400; Angew. Chem., Int. Ed. Engl., 2004, 43, 1043) oder Palladium (z.B. Palladium(II)acetat, Tris(dibenzylideneaceton)dipalladium(0)) (Acc. Chem. Res., 1998, 31, 852; Angew. Chem., Int. Ed. Engl., 1998, 37, 2046; Top. Cur. Chem., 2002, 219, 131) in Gegenwart einer geeigneten Base (z.B. Natrium-tert.-butanolat, Kalium-tert.-butanolat, Natriumhydrid, Kaliumhydrid, Kaliumhexamethyldisilazid, Trikaliumphosphat, Natriumcarbonat, Kaliumcarbonat, Cäsiumcarbonat) und eines Liganden (z.B.2,2'-Bis(diphenylphosphino)-1,1'-binaphthyl , 4,5-Bis(diphenylphosphino)-9,9-dimethylxanthen , Triphenylphosphin, 1,1'-Bis(diphenylphosphino)ferrocen, 1,1'-Bis(di-o-tolylphosphino)ferrocen, 1,3-Di-tert-butyl-2-chlor-1,3,2-diazaphospholidin, 2'-(Dicyclohexylphosphino)-N,N-dimethylbiphenyl-2-amin) in einem Lösungsmittel (z.B. *N*,*N*-Dimethylformamid, Toluol, Xylol, Tetrahydrofuran, Dioxan, Dimethoxyethan, tert.-Butyl-methylether) bei einer Temperatur von 40-200 °C durchgeführt werden. Bevorzugt werden die 6-Hydroxyindazole mit Alkylhalogeniden unter Verwendung von Kaliumcarbonat als Base und *N,N*-Dimethylformamid als Lösungsmittel umgesetzt. Die Reaktionen werden bevorzugt 1 - 24 Stunden bei 70 - 150 °C in der Mikrowelle durchgeführt.

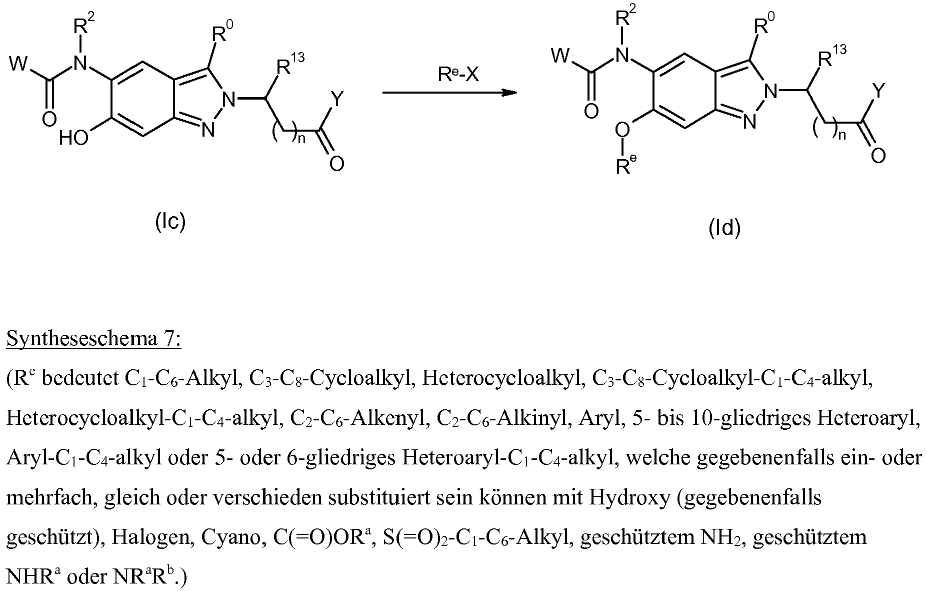

Die als Ausgangsmaterial zur Synthese einer Teilmenge der erfindungsgemäßen Verbindungen verwendeten Pyridincarbonsäuren (Intermediat 19) sind kommerziell erhältlich oder können auf literaturbekannten Wegen gemäß Syntheseschema 8 hergestellt werden. Einige der Intermediate 19 können ausgehend von Carbonsäureestern (Intermediat 17) durch Verseifung oder - in dem Fall, dass es sich um einen tert-Butylester handelt - durch Reaktion mit einer Säure wie zum Beispiel Chlorwasserstoff oder Trifluoressigsäure hergestellt werden. Gegebenenfalls können die Intermediate 19 als Salze (zum Beispiel als Kaliumsalz) hergestellt werden. Die Intermediate 17 sind kommerziell erhältlich, können auf literaturbekannten Wegen hergestellt werden oder sind aus den Intermediaten 16, welche als X¹ ein Chlor, Brom oder Iod tragen, durch eine Umsetzung in einer Kohlenmonoxid-Atmosphäre gegebenenfalls unter Überdruck in Gegenwart eines Phosphinliganden wie zum Beispiel 1,3-Bis(diphenylphoshino)propan, einer PalladiumVerbindung wie zum Beispiel Palladium(II)acetat und einer Base wie zum Beispiel Triethylamin unter Zusatz von Ethanol oder Methanol in einem Lösemittel wie zum Beispiel Dimethylsulfoxid erhältlich.
Der Rest R³ steht hierbei für Cyano, substituiertes oder unsubstituiertes C₁-C₆-Alkyl, substituiertes oder unsubstituiertes C₁-C₆-Alkoxy, substituiertes oder unsubstituiertes C₃-C₆-Cycloalkyl, Heterocycloalkyl, C₅-C₁₁-Spirocycloalkyl, substitiertes oder unsubstituiertes C₃-C₆-Cycloalkyl-C₁-C₄-Alkyl, substituiertes oder unsubstituiertes Aryl, 5- bis 10-gliedriges Heteroaryl, NH₂, NHR^{a}, N(R^{a})R^{b} oder N(H)C(=O)R^{a}.
Im besonderen Fall, dass R³ die Bedeutung substituiertes oder unsubstituiertes C₁-C₆-Alkoxy, NH₂, NHR^{a} oder N(R^{a})R^{b} hat, kann R³ durch Erhitzen der entsprechenden bishalogenierten Intermediate 15, bei denen X¹ und X² unabhängig voneinander für Chlor, Brom oder Iod stehen, mit Alkoholen oder Aminen eingebracht werden, wodurch die Intermediate 16 erhalten werden.
Falls R³ für substituiertes oder unsubstituiertes C₁-C₆-Alkyl (Eur. J. of Org. Chem., 2002, 327), substituiertes oder unsubstituiertes C₃-C₆-Cycloalkyl, Heterocycloalkyl, C₅-C₁₁-Spirocycloalkyl oder substitiertes oder unsubstituiertes C₃-C₆-Cycloalkyl-C₁-C₄-Alkyl steht, kann R³ durch Umsetzung der Intermediate 15 mit entsprechenden Organometallverbindungen eingeführt werden. Hierbei kommen Organolithiumverbinungen (Green Chemistry, 2011, 13, 1110), Organomagnesiumverbindungen oder Organokupferverbindungen (Angew. Chem., 2013 , 125, 6397) in Betracht. Im Falle von Amino- oder Hydroxy-substituierten Resten R³ trägt die funktionelle Gruppe eine in der Literatur bekannte und nach Ansicht des Fachmanns geeignete Schutzgruppe in der Organometallverbindung (Protecting Groups, Philip J. Kocienski, 3rd Revised edition (9. Februar 2005), Thieme; Greene's Protective Groups in Organic Synthesis, Peter G. M. Wuts, Theodora W. Greene, 4. Auflage (8. Dezember 2006), Wiley-Interscience). Diese Schutzgruppe kann in einem zusätzlichen Syntheseschritt nach einem gängigen und in der Literatur beschriebenen Verfahren wieder abgespalten werden (Protecting Groups, Philip J. Kocienski, 3rd Revised edition (9. Februar 2005), Thieme; Greene's Protective Groups in Organic Synthesis, Peter G. M. Wuts, Theodora W. Greene, 4. Auflage (8. Dezember 2006), Wiley-Interscience). Alternativ kann der Rest R³ auch über eine Palladium-katalysierte Suzuki-Kupplung (Pure Appl. Chem., 1985, 57, 1749; Chem. Rev., 1995, 95, 2457; Advances in Metal-Organic Chemistry, 1998, 6, 187; Angew. Chem., Int. Ed. Engl., 2004, 43, 2201, Top. Curr. Chem., 2002, Vol. 219, 248) eingeführt werden, wenn R³ ein substituiertes oder unsubstituiertes Aryl oder ein 5- bis 10-gliedriges Heteroaryl bedeutet. Dabei wird R³ über eine entsprechende Organoborverbindung in Gegenwart einer Palladiumverbindung (z.B. Palladium(II)acetat, Tris(dibenzylideneacetone)dipalladium(0), Tetrakis(triphenylphosphin)palladium), eines Liganden (z.B. 2,2'-Bis(diphenylphosphino)-1,1'-binaphthyl, Triphenylphosphin, 1,1'-Bis(diphenylphosphino)ferrocen, 2-Dicyclohexylphosphino-2',4',6'-triisopropylbiphenyl, 4,5-Bis(diphenylphosphino)-9,9-dimethylxanthene, 2-(Dicyclohexylphosphino)3,6-dimethoxy-2',4',6'-triisopropyl-1,1'-biphenyl, 1,1'-Bis(di-o-tolylphosphino)ferrocen) in einem Lösungsmittel (z.B. *N*,*N*-Dimethylformamid, Acetonitril Toluol, Xylol, Tetrahydrofuran, Dioxan, Dimethoxyethan, Methanol, Ethanol, Wasser) mit einer Base (z.B. Natriumcarbonat, Kaliumcarbonat, Cäsiumcarbonat, Trikaliumphosphat, Kaliumfluorid, Natriumhydroxid) und gegebenenfalls Zugabe von Lithiumchlorid bei einer Temperatur von 25 - 200 °C eingeführt. Der Einsatz von Tetrakis(triphenylphosphin)palladium ist bevorzugt.
Die Intermediate 17 können alternativ auch aus den Intermediaten 18 hergestellt werden. Zur Einführung des Restes R³ wird die oben beschriebene Suzuki-Reaktion mit entsprechenden Organoborverbindungen verwendet.

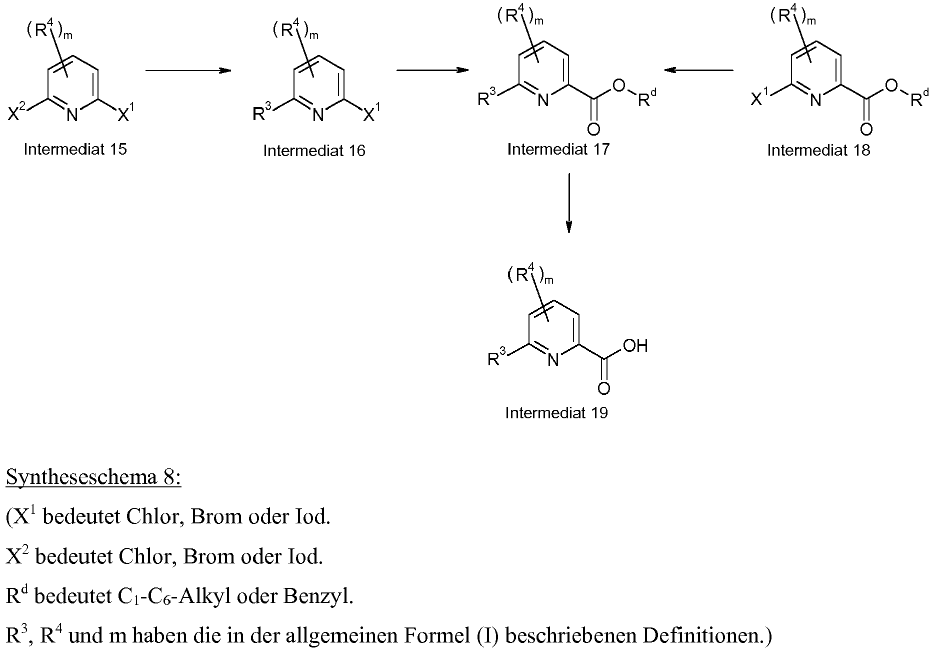

Gemäß Syntheseschema 9 können Intermediate 20, die gemäß Syntheseschema 2 hergestellt werden können, im Rahmen einer Negishi-Reaktion (Acc. Chem. Res., 1982, 15, 340; Metal-Catalyzed Cross-coupling Reactions, F. Diedrich, P. J. Stang, Wiley-VCH, 1998, 1; Aust. J. Chem. 2004, 57, 107; Handbook of Organopalladium Chemistry for Organic Synthesis, E.-I. Negishi, Y. Dumond, 2002, Vol. 1, 767) mit primären und sekundären Alkylzink-Reagenzien in Gegenwart eines Palladiumkatalysators umgesetzt werden, wodurch eine Teilmenge (Ie) der erfindungsgemäßen Verbindungen hergestellt werden kann mit der Bedeutung R^{g} = primäres oder sekundäres C₁-C₆-Alkyl. Bevorzugt ist die Umsetzung mit Diethylzink oder 2-Methylpropylzinkbromid.

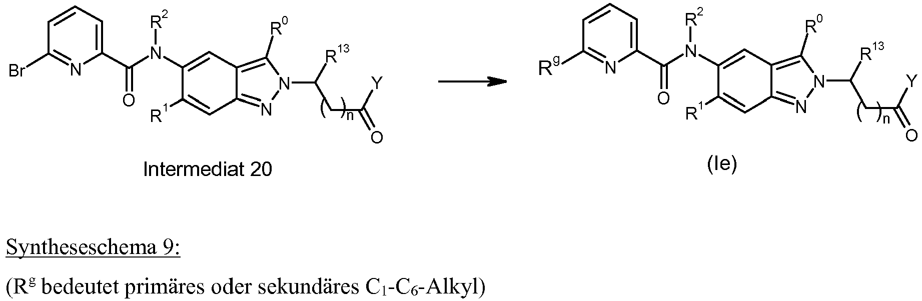

Weitere Intermediate können nach Syntheseschema 10 erhalten werden: Intermediate 9 können im Rahmen einer Amidkupplung wie bei Syntheseschema 1 beschrieben zu Intermediaten 21 umgesetzt werden. Der Einsatz von 1-(3-Dimethylaminopropyl)-3-ethylcarbodiimidhydrochlorid in Kombination mit 1-Hydroxy-1H-benzotriazol Hydrat ist bevorzugt. Intermediate 21 können dann durch Umsetzung mit Trifluoressigsäure zu Intermediaten 22 umgesetzt werden, die im Rahmen einer Amidkupplungsreaktion analog zu den bei Syntheseschema 1 beschriebenen Methoden zu Beispielverbindungen umgesetzt werden können.

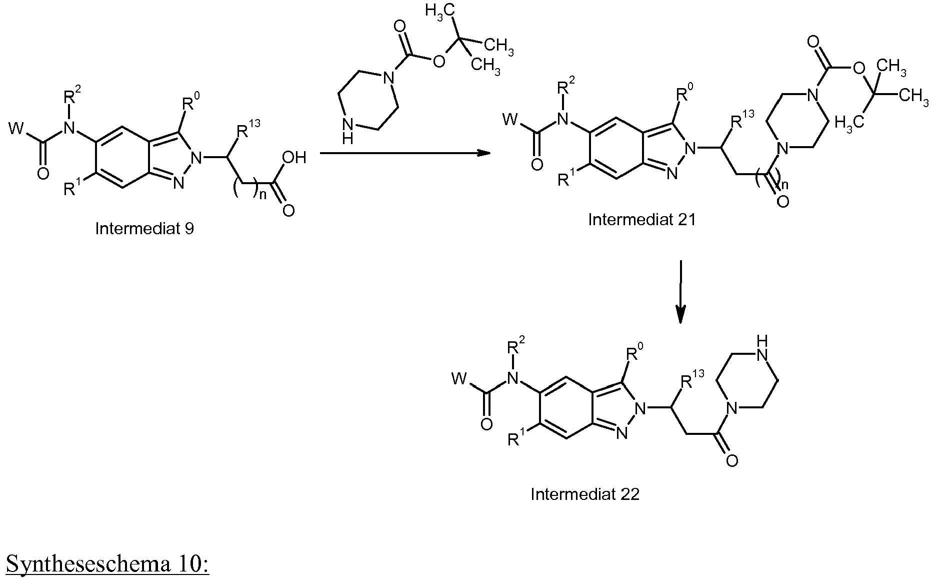

Intermediate des Typs 2b können nach Syntheseschema 11 erhalten werden: Intermediate 12b werden mit Benzylcarbonochloridat und N-Ethyl-N-isopropylpropan-2-amin in THF umgesetzt um Intermediate 23 zu erhalten. Die Umsetzung mit Trifluoressigsäure in Dichlormethan führt dann zu den Intermediaten 2b, die dann gemäß Syntheseschema 1 weiter zu erfindungsgemäßen Verbindungen umgesetzt werden.

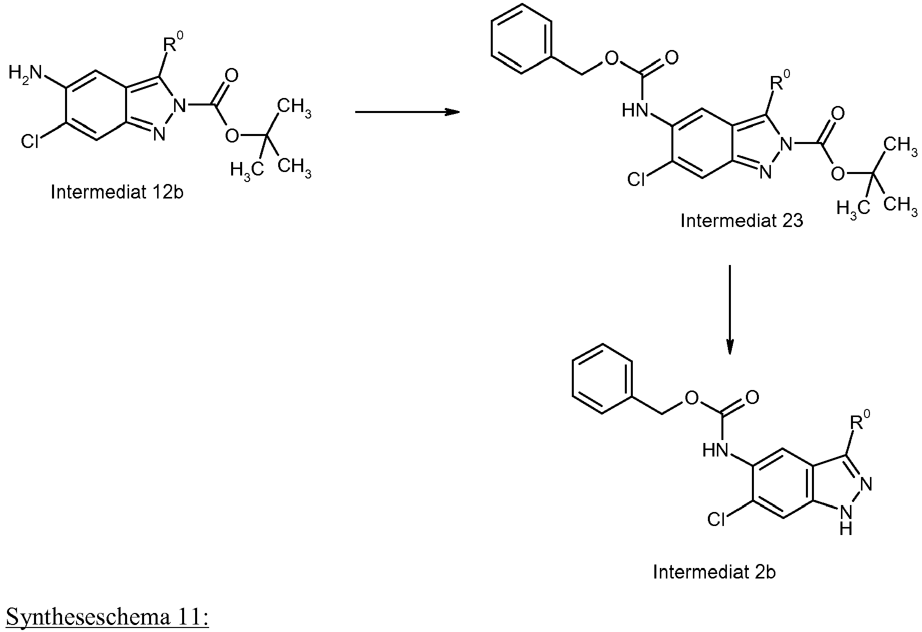

**Abkürzungen**

| | |
|---|---|
| DMF | *N*,*N-*Dimethylformamid |
| HPLC | Hochleistungsflüssigkeitschromatographie |
| HOBt | 1-Hydroxy-1H-benzotriazolhydrat |
| UPLC | Ultra- Hochleistungsflüssigchromatographie |
| DAD | Diodenarraydetektor |
| EDC | 1-(3-Dimethylaminopropyl)-3-ethylcarbodiimidhydrochlorid |
| ELSD | Verdampfungslichtstreudetektor |
| ESI | Elektronenspray-Ionisation |
| SQD | Single Quadrupole Detektor |
| PTFE | Polytetrafluorethylen |
| KPG | Kerngezogenes Präzisions-Glasgerät |
| SV | Säulenvolumen |
| BOC | tert - Butyloxycarbonyl |
| PG | Protecting group, Schutzgruppe |
| LG | Leaving group, Abgangsgruppe |

### Methoden

### Analytische HPLC-Methoden:

### Methode A1: UPLC (ACN-HCOOH):

Instrument: Waters Acquity UPLC-MS SQD 3001; Säule: Acquity UPLC BEH C18 1.7 50x2.1mm; Eluent A: Wasser + 0.1% Vol. Ameisensäure (99%), Eluent B: Acetonitril; Gradient: 0-1.6 min 1-99% B, 1.6-2.0 min 99% B; Fluss 0.8 ml/min; Temperatur: 60 °C.; Injektion: 2 µl; DAD scan: 210-400 nm; ELSD.

### Methode A2: UPLC (ACN-NH3):

Instrument: Waters Acquity UPLC-MS SQD 3001; Säule: Acquity UPLC BEH C18 1.7 50x2.1mm; Eluent A: Wasser + 0.2% Vol. Ammoniak (32%), Eluent B: Acetonitril; Gradient: 0-1.6 min 1-99% B, 1.6-2.0 min 99% B; Fluss 0.8 ml/min; Temperatur: 60 °C; Injektion: 2 µl; DAD scan: 210-400 nm; ELSD.

### Methode A3: (LC-MS)

Instrument: Agilent 1290 Infinity LC; Säule: Acquity UPLC BEH C18 1.7 50x2.1mm; Eluent A: Wasser + 0.05% Vol. Ameisensäure, Eluent B: Acetonitril + 0.05% Vol. Ameisensäure; Gradient: 0-1.7 min 2-90% B, 1.7-2.0 min 90% B; Fluss 1.2 ml/min; Temperatur: 60 °C; Injektion: 2 µl; DAD scan: 190-390 nm; MS: Agilent TOF 6230.

### Methode A4: (LC-MS)

Instrument: Waters Acquity; Säule: Kinetex (Phenomenex), 50x2 mm; Eluent A: Wasser + 0.05% Vol. Ameisensäure, Eluent B: Acetonitril + 0.05% Vol. Ameisensäure; Gradient: 0-1.9 min 1-99% B, 1.9-2.1 min 99% B; Fluss 1.5 ml/min; Temperatur: 60 °C; Injektion: 0.5 µl; DAD scan: 200 - 400 nm.

### Präparative HPLC-Methoden:

Methode P1: System: Waters Autopurificationsystem: Pump 2545, Sample Manager 2767, CFO, DAD 2996, ELSD 2424, SQD; Säule: XBrigde C18 5µm 100x30 mm; Eluent: A: Wasser + 0.1% Vol. Ameisensäure, Eluent B: Acetonitril; Gradient: 0-8 min 10-100% B, 8-10 min 100% B; Flow: 50 mL/min; Temperatuer: Raumtemp.; Lösung: Max. 250 mg / max. 2.5 mL DMSO o. DMF; Injektion: 1 x 2.5 mL; Detection: DAD scan range 210-400 nm; MS ESI+, ESI-, scan range 160-1000 m/z.

Methode P2: System: Waters Autopurificationsystem: Pump 254, Sample Manager 2767, CFO, DAD 2996, ELSD 2424, SQD 3100; Säule: XBrigde C18 5µm 100x30 mm; Eluent: A: Wasser + 0.2% Vol. Ammoniak (32%), Eluent B: Methanol; Gradient: 0-8 min 30-70% B; Fluss: 50 mL/min; Temperatur: Raumtemp.; Detektion: DAD scan range 210-400 nm; MS ESI+, ESI-, scan range 160-1000 m/z; ELSD.

Methode P3: System: Labomatic, Pump: HD-5000, Fraction Collector: LABOCOL Vario-4000, UV-Detector: Knauer UVD 2.1S; Säule: XBrigde C18 5µm 100x30 mm; Eluent A: Wasser + 0.2% Vol. Ammoniak (25%), Eluent B: Acetonitril; Gradient: 0-1 min 15% B, 1-6,3 min 15-55% B, 6,3-6,4min 55-100% B, 6,4-7,4min 100% B; Flow: 60 mL/min; Temperatur: Raumtemp.; Lösung: Max. 250 mg / 2mL DMSO; Injektion: 2 x 2mL; Detection: UV 218 nm; Software: SCPA PrepCon5.

Methode P4: System: Agilent: Prep 1200, 2xPrep Pump, DLA, MWD, Prep FC; Säule: Chiralpak IA 5µm 250x20 mm; Eluent A: Methanol, Eluent B: Ethanol; Gradient: isokratisch 50% B; Fluss: 15 mL/min; Temperatur: Raumtemp.; Detektion: UV 254 nm

Methode P5: System: Labomatic, Pump: HD-5000, Fraction Collector: LABOCOL Vario-4000, UV-Detector: Knauer UVD 2.1S; Säule: Säule: Chromatorex RP C18 10µm 125x30 mm, Eluent: A: Wasser + 0.1% Vol. Ameisensäure, Eluent B: Acetonitril; Gradient: 0 - 15 min 65 - 100% B; Fluss: 60 mL/min; Temperatur: Raumtemp.; Lösung: Max. 250 mg / 2mL DMSO; Injektion: 2 x 2mL; Detection: UV 254 nm; Software: SCPA PrepCon5.

### Mikrowelle

CEM Discover S-Class; Autosampler: CEM Explorer; Software: CEM Synergy; Methode: Dynamic heating mode, 300W, max. 18bar.

### Intermediate

### 2-Fluoro-5-nitro-4-(trifluoromethoxy)benzaldehyd

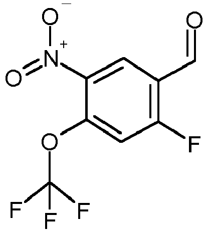

20.9 g (100.4 mmol) 2-Fluoro-4-(trifluoromethoxy)benzaldehyd wurden in 100 ml Schwefelsäure (W=96%) gelöst und in einem Dreihalskolben mit KPG-Rührer, Tropftrichter und Innenthermometer auf -15°C gekühlt. Zu dieser Lösung wurde innerhalb von 60min die vorher hergestellte und gekühlte Nitriersäure (28 ml Schwefelsäure (w=96%) in 14 ml Salpetersäure (w=65%)) zu getropft. Dabei bewegte sich die Innentemperatur zwischen -15°C und -12°C. Nachdem das Zutropfen beende war, wurde eine weitere Stunde nachgerührt (Innentemperatur bei - 13°C). Die Reaktionsmischung wurde auf Eis gegeben und drei Mal mit je 150 ml Ethylacetat extrahiert. Die vereinten org. Phasen wurden mit gesättiger Natriumchloridlösung gewaschen, über Natriumsulfat getrocknet, filtriert und eingeengt. Man erhielt 25.4 g (100 % d. Th.) der Titelverbindung.
¹H NMR (400 MHz, CHLOROFORM-d) δ = 7.34 (dd, 1 H), 8.57 (d, 1 H), 10.34 (s, 1 H).

### Intermediat 0-2

### 5-Nitro-6-(trifluoromethoxy)-1H-indazole

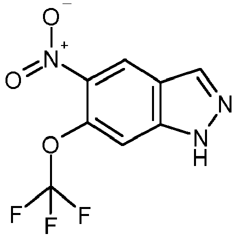

25.4 g (100.4 mmol) 2-Fluoro-5-nitro-4-(trifluoromethoxy)benzaldehyd wurden in 200 ml absolutem Ethanol vorgelegt und mit 25 ml (513.6 mmol) Hydrazinhydrat versetzt. Die Lösung färbte sich dunkel. Das Reaktionsgemisch wurde für 2 h refluxiert. Anschließend wurde die Reaktionsmischung auf 1.4 1 Wasser gegeben und 10 Minuten kräftig gerührt. Der Niederschlag wurde abgesaugt und drei Mal mit je 40 ml Wasser gewaschen. Der erhaltene Feststoff wurde über Nacht im Vakuumtrockenschrank bei +50°C getrocknet. Man erhielt 19.4 g (78 % d. Th.) der Titelverbindung.
UPLC-MS (Methode A1): Rₜ = 1.03min
MS (ESIpos): m/z = 248 (M+H)⁺
¹H NMR (400 MHz, DMSO-d6) δ = 7.86 (s, 1 H), 8.46 (s, 1 H), 8.82 (s, 1 H), 13.87 (br. s., 1 H).

### Intermediat 0-3

### 6-(Benzyloxy)-5-nitro-1H-indazol

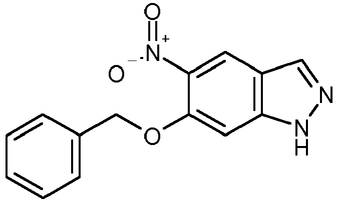

20.0 g (111.6 mmol) 5-Nitro-1H-indazol-6-ol (CAS-Nr. 1082041-56-2) wurden in 750 ml Tetrahydrofuran vorgelegt und mit 13.9 ml (134.0 mmol) Benzylalkohol und 35.1 g (134.0 mmol) Triphenylphosphin versetzt. Die Lösung wurde auf 0°C gekühlt und es wurden 26.03 ml (134.0 mmol) Diisopropylazodicarboxylat hinzugegeben. Das Reaktionsgemisch wurde für 1 h bei 0°C und anschließend für 24 h bei 25°C gerührt. Die Reaktionsmischung wurde anschließend mit Wasser versetzt und mit Ethylacetat extrahiert. Die vereinigten organischen Phasen wurden mit gesättigter Natriumchloridlösung gewaschen und eingeengt. Der Rückstand wurde in Dichlormethan aufgenommen, Isolute® HM-N (Biotage) hinzugegeben und unter Einengen auf Isolute adsorbiert. Die Isolute wurde auf eine mit Hexan vorequilibrierte Kartusche (750 g; KP-Sil) gegeben und mittels Isolera® Flash-Aufreinigungssystem (Biotage) die Chromatographie durchgeführt (Laufmittel: Hexan-Ethylacetat; Flussrate: 200 ml/mim; Gradient: isokratisch 88:12 (1 SV), 88:12->20:80 (10 SV), isokratisch 20:80 (2 SV)). Die vereinigten Produktfraktionen wurden eingeengt und getrocknet. Man erhielt 18.908g (63% der Theorie) der Titelverbindung erhalten.
UPLC-MS (Methode A1): Rₜ = 1.09 min
MS (ESIpos): m/z = 270 (M+H)⁺
¹H NMR (300 MHz, DMSO-d6) δ = 5.35 (s, 2 H), 7.25 - 7.57 (m, 6 H), 8.20 (s, 1 H), 8.45 (s, 1 H), 13.38 (br. s., 1 H).

### Intermediat 0-4

### 6-Ethoxy-5-nitro-1H-indazol

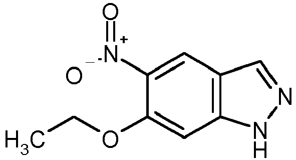

100 mg (0.56 mmol) 5-Nitro-1H-indazol-6-ol (CAS-Nr. 1082041-56-2) wurden in 668 µl *N,N-*Dimethylformamid vorgelegt und mit 93 mg (0.67 mmol) Kaliumcarbonat und 54 µl (0.67 mmol) Iodethan versetzt. Die Lösung wurde für 1 h bei 60°C in der Mikrowelle erhitzt. Die Reaktionsmischung wurde anschließend mit Wasser versetzt und mit Ethylacetat extrahiert. Die vereinigten organischen Phasen wurden mit gesättigter Natriumchloridlösung gewaschen, über einen wasserabweisenden Filter filtriert und eingeengt. Der Rückstand wurde in Dichlormethan aufgenommen, Isolute® HM-N (Biotage) hinzugegeben und unter Einengen auf Isolute adsorbiert. Die Isolute wurde auf eine mit Hexan vorequilibrierte Kartusche (25 g; KP-Sil) gegeben und mittels Isolera® Flash-Aufreinigungssystem (Biotage) die Chromatographie durchgeführt (Laufmittel: Hexan-Ethylacetat; Flussrate: 25 ml/mim; Gradient: isokratisch 88:12 (1 SV), 88:12->0:100 (10 SV), isokratisch 0:100 (2 SV)). Die vereinigten Produktfraktionen wurden eingeengt und getrocknet. Man erhielt 89 mg (77% der Theorie) der Titelverbindung erhalten.
UPLC-MS (Methode A1): Rₜ = 0.89 min
MS (ESIpos): m/z = 208 (M+H)⁺
¹H NMR (300 MHz, DMSO-d6) δ = 1.37 (t, 3 H), 4.23 (q, 2 H), 7.19 (s, 1 H), 8.17 (s, 1 H), 8.40 (s, 1 H), 13.31 (br. s., 1 H).

### Intermediat 0-5

### Methyl-5-nitro-1H-indazol-6-carboxylat

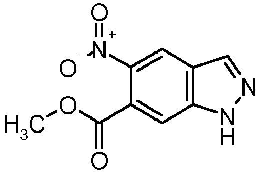

4.60 g (26.1 mmol) Methyl-1H-indazol-6-carboxylat wurden in 120 ml Schwefelsäure (W=96%) gelöst und in einem Dreihalskolben mit KPG-Rührer, Tropftrichter und Innenthermometer auf - 15°C gekühlt. Zu dieser Lösung wurde innerhalb von 15 Minuten die vorher hergestellte und gekühlte Nitriersäure (9.2 ml Schwefelsäure (w=96%) in 4 ml Salpetersäure (w=65%)) zugetropft. Dabei bewegte sich die Innentemperatur zwischen -15°C und -12°C. Nachdem das Zutropfen beende war, wurde eine weitere Stunde nachgerührt (Innentemperatur bei -5°C). Die Reaktionsmischung wurde auf Eis gegeben, der entstandene Niederschlag abgesaugt , mit Wasser gewaschen und im Trockenschrank bei 50°C unter Vakuum getrocknet. Man erhielt 5.49 g (91 % d. Th.) der Titelverbindung.
UPLC-MS (Methode A1): Rₜ = 1.21 min
MS (ESIpos): m/z = 471 (M+H)⁺
¹H-NMR (400MHz, DMSO-d6): δ = 3.85 (s, 3 H) 6.01 (s, 2 H) 6.98 (s, 1 H) 7.79 - 7.91 (m, 1 H) 7.99 (s, 1 H) 12.84 (br. s., 1 H).

### Intermediat 1-1

### 6-(Trifluoromethoxy)-1H-indazol-5-amin

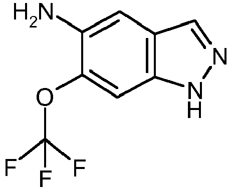

10.0 g (40.5 mmol) 5-Nitro-6-(trifluoromethoxy)-1H-indazol (Intermediat 0-2) wurden in 400 ml Methanol gelöst. Danach wurde entgast und mit Stickstoff gespült (Vorgang noch zwei Mal wiederholt). 2.48 g (2.0 mmol) Palladium auf Aktivkohle wurden zugegeben. Der Kolben wurde evakuiert und mit Wasserstoff gespült. Das Reaktionsgemisch wurde für 5 Stunden bei Raumtemperatur unter Normaldruck Wasserstoff hydriert. Das Reaktionsgemisch wurde über einen PTFE-Filter mit Celite filtriert und eingeengt. Man erhielt 7.2 g (74 % d. Th.) der Titelverbindung.
UPLC-MS (Methode A1): Rₜ = 0.75 min
MS (ESIpos): m/z = 218 (M+H)⁺
1H NMR (400 MHz, DMSO-d6) δ = 4.91 (s, 2 H), 7.04 (s, 1 H), 7.32 (s, 1 H), 7.83 (s, 1 H), 12.72 (br. s., 1 H).

### Intermediat 1-2

### 5-Amino-1H-indazol-6-ol

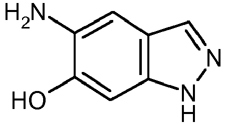

Analog zu Intermediat 1-1 wurden 6.5 g (36.3 mmol) 5-Nitro-1H-indazol-6-ol (CAS-Nr. 1082041-56-2) in 1,5 l Methanol gelöst und mit 193 mg (1.8 mmol) Palladium auf Aktivkohle für 5 h bei 25°C unter Normaldruck Wasserstoff hydriert. Man erhielt 5.28 g (98 % d. Th.) der Titelverbindung.
UPLC-MS (Methode A1): Rₜ = 0.26 min
MS (ESIpos): m/z = 150 (M+H)⁺
¹H NMR (400 MHz, DMSO-d6): δ = 4.37 (br. s., 2 H) 6.71 - 6.78 (m, 2 H) 7.59 (s, 1 H) 12.17 (br. s., 1 H).

### Intermediat 1-3

### 6-(Benzyloxy)-1H-indazol-5-amin

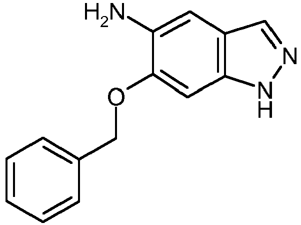

18.5 g (68.7 mmol) 6-(Benzyloxy)-5-nitro-1H-indazol (Intermediat 0-3) wurden in 500 ml Ethanol gelöst und in einem 1 l Dreihalskolben mit KPG-Rührer und Rückflusskühler vorgelegt und mit 100 ml Wasser versetzt. Anschließend wurden 19.2 g (343.5 mmol) Eisenpulver und 1.84 g (34.35 mmol) Ammoniumchlorid zugegeben. Die braune Suspension wurde für 4 h zum Rückfluß erhitzt. Das Reaktionsgemisch wurde mittels Wasserbades auf 25°C abgekühlt und über Celite filtriert (klares Filtrat). Der Filterkuchen wurde mit Ethanol gewaschen. Das Filtrat wurde eingeengt, bis noch etwa 200ml Lösemittel vorhanden waren. Das Reaktionsgemisch wurde auf 2 l Wasser gegeben. Die Suspension wurde gekühlt und der entstandene Niederschlag anschließend abgesaugt. Der Filterkuchen wurde zwei Mal mit je 150 ml Wasser und zwei Mal mit je 100 ml Diethylether gewaschen. Der Niederschlag wurde für 5 h bei 50°C im Vakuumtrockenschrank getrocknet und mit 193 mg (1.81 mmol) Palladium auf Aktivkohle für 5 h bei 25°C unter Normaldruck Wasserstoff nachhydriert. Man erhielt 15.28 g (92 % d. Th.) der Titelverbindung.
UPLC-MS (Methode A1): Rₜ = 0.66 min
MS (ESIpos): m/z = 240 (M+H)⁺
¹H NMR (300 MHz, DMSO-d6): δ = 4.54 (s, 2 H), 5.19 (s, 2 H), 6.84 (s, 1 H), 6.91 (s, 1 H), 7.22 - 7.45 (m, 3 H), 7.48 - 7.57 (m, 2 H), 7.66 (s, 1 H), 12.43 (br. s., 1 H).

### Intermediat 1-4

### 6-Isopropoxy-1H-indazol-5-amin

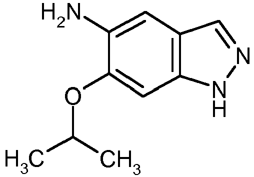

10 g (45.2 mmol) 6-Isopropoxy-5-nitro-1H-indazol (CAS-Nr. 1082041-56-2) wurden in 200 ml Ethanol gelöst und mit 1.20 g (1.13 mmol) Palladium auf Aktivkohle für 24 h bei 25°C unter Normaldruck Wasserstoff hydriert. Die Reaktionsmischung wurde über Celite abfiltriert, mit Ethanol gewaschen und das Filtrat eingeengt. Der Rückstand wurde mit wenig Ethanol im Ultraschallbad angelöst, Diethylether hinzugegeben und weiter im Ultraschallbad digeriert. Der Feststoff wurde abgesaugt und mit wenig Diethylether und Hexan gewaschen, wobei 4.69 g (54%) Produkt erhalten wurden. Das Filtrat wurde eingeengt und auf eine mit Hexan vorequilibrierte Biotage SNAP Kartusche (100 g; KP-Sil) gegeben und mittels Isolera® Flash-Aufreinigungssystem (Biotage) die Chromatographie durchgeführt (Laufmittel: Hexan-Ethylacetat; Gradient: 90:10->35:65 (9.2 SV), isokratisch 35:65 (1 SV)). Die vereinigten Produktfraktionen wurden eingeengt und der Rückstand mit einer Mischung aus Hexan und Dichlormethan (2:1) im Ultraschallbad digeriert. Der entstandene Feststoff wurde abfiltriert. Man erhielt zusätzlich 2.36 g (27 % d. Th.) der Titelverbindung.
UPLC-MS (Methode A1): Rₜ = 0.75 min
MS (ESIpos): m/z = 192 (M+H)⁺
¹H NMR (400 MHz, DMSO-d6): δ = 1.31 (s, 3 H), 1.33 (s, 3 H), 4.43 (s, 2 H), 4.57 - 4.68 (m, 1 H), 6.81 (s, 1 H), 6.83 (s, 1 H), 7.64 (s, 1 H), 12.34 (br. s., 1 H).

### Intermediat 1-5

### 6-Ethoxy-1H-indazol-5-amin

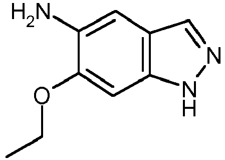

Analog zu Intermediat 1-1 wurden 65 mg (0.31 mmol) 6-Ethoxy-5-nitro-1H-indazol (Intermediat 0-4) in 4.1 ml Methanol gelöst und mit 33 mg (0.03 mmol) Palladium auf Aktivkohle für 5 h bei 25°C unter Normaldruck Wasserstoff hydriert. Man erhielt 54 mg (97 % d. Th.) der Titelverbindung.
UPLC-MS (Methode A1): Rₜ = 0.64 min
MS (ESIpos): m/z = 178 (M+H)⁺
¹H NMR (400 MHz, DMSO-d6): δ = 1.40 (t, 3 H), 4.07 (q, 2 H), 4.47 (br. s., 2 H), 6.81 (s, 2 H), 7.65 (s, 1H), 12.39 (br. s., 1 H).

### Intermediat 1-6

### Methyl-5-amino-1H-indazol-6-carboxylat

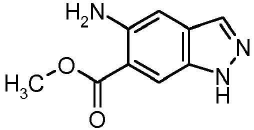

Analog zu Intermediat 1-1 wurden 5.48 g (24.8 mmol) Methyl-5-nitro-1H-indazol-6-carboxylat (Intermediat 0-9) in 293 ml Methanol gelöst und mit 1.32 g (1.24 mmol) Palladium auf Aktivkohle für 3 h bei 25°C unter Normaldruck Wasserstoff hydriert. Man erhielt 4.52 g (91 % d. Th.) der Titelverbindung.
UPLC-MS (Methode A1): Rₜ = 0.75 min
MS (ESIpos): m/z = 222 (M+H)⁺
¹H NMR (500 MHz, DMSO-d6): δ = 3.85 (s, 3 H), 6.05 (br. s., 2 H), 6.99 (d, 1 H), 7.85 (d, 1 H), 8.00 (s, 1 H), 12.83 (br. s., 1 H).

### Intermediat 2-1

### tert-Butyl-(6-methyl-1H-indazol-5-yl)carbamat

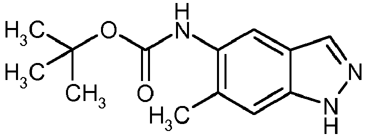

10.3 g (70.0 mmol) 6-Methyl-1H-indazol-5-amin (CAS-Nr: 81115-45-9) wurden in 150 ml Tetrahydrofuran suspendiert, 13.4 ml (80.0 mmol) *N*,*N*-Diisopropylethylamin zugegeben und das Gemisch auf 0°C gekühlt. Nach Zugabe von 5.52 g (25.3 mmol) Di-tert-butyldicarbonat bei 0°C wurde das Gemisch anschließend für 18 h bei 25°C gerührt. Man engte ein und erhielt 17.6 g eines Rohproduktes, welches ohne Reinigung eingesetzt wurde.
UPLC-MS (Methode A1): Rₜ = 1.01 min
MS (ESIpos): m/z = 248 (M+H)⁺.

### Intermediat 2-2

### tert-Butyl (6-methoxy-1H-indazol-5-yl)carbamat

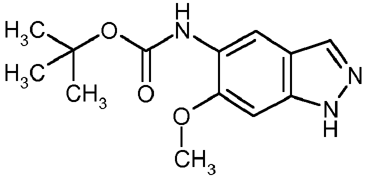

4.0 g (24.5 mmol) 6-Methoxy-1H-indazol-5-amin (CAS-Nr. 749223-61-8) wurden in 30 ml Tetrahydrofuran gelöst und 5.35 g (24.5 mmol) Di-tert-butyldicarbonat zugegeben. Die Reaktionsmischung wurde für 18 h bei 25°C gerührt. Anschließend wurde die Reaktionsmischung eingeengt und der Rückstand in 20 ml Dichlormethan suspendiert. Es wurden 200 ml Hexan hinzugegeben und die entstandene Suspension für 25 Minuten unter Eisbadkühlung gerührt. Die Niederschlag wurde abgesaugt, zweimal mit 25 ml Hexan gewaschen und getrocknet. Man erhielt 4.83 g (75 % d. Th.) der Titelverbindung.
UPLC-MS (Methode A2): Rₜ = 1.08 min
MS (ESIpos): m/z = 264 (M+H)⁺
¹H NMR (400 MHz, CHLOROFORM-d) δ = 1.56 (s, 9 H), 3.95 (s, 3 H), 6.88 (s, 1 H), 7.12 (br. s., 1 H), 7.94 (d, 1 H), 8.40 (br. s., 1 H).

### Intermediat 2-3

### tert-Butyl [6-(trifluoromethoxy)-1H-indazol-5-yl]carbamat

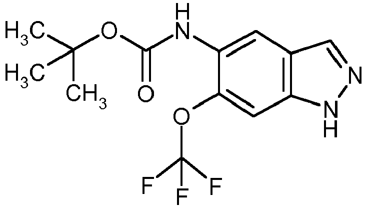

5.0 g (23.0 mmol) 6-(Trifluoromethoxy)-1H-indazol-5-amin (Intermediat 1-1) wurden in 100 ml Tetrahydrofuran suspendiert, 4.81 ml (27.6 mmol) *N*,*N*-Diisopropylethylamin zugegeben und das Gemisch auf 0°C gekühlt. Nach Zugabe von 5.52 g (25.3 mmol) Di-tert-butyldicarbonat bei 0°C wurde das Gemisch anschließend für 18h bei 25°C gerührt. Es wurden weitere 3.52 g (16.1mmol) Di-tert-butyldicarbonat zugegeben und für weitere 24 h bei 25°C gerührt. Die Reaktionsmischung wurde für weitere 24 h zum Rückfluß erhitzt. Anschließend wurde die Reaktionsmischung eingeengt, in Ethylacetat aufgenommen und mit 0.5 M Salzsäure, gesättigter Natriumhydrogencarbonatlösung und gesättigter Natriumchloridlösung gewaschen. Die vereinigten, organischen Phasen wurden über Natriumsulfat getrocknet und nach Filtration wurde die Lösung eingeengt. Der Rückstand wurde in Dichlormethan aufgenommen, Isolute® HM-N (Biotage) hinzugegeben und unter Einengen auf Isolute adsorbiert. Die Isolute wurde auf eine mit Hexan vorequilibrierte Biotage SNAP Kartusche (340 g; KP-Sil) gegeben und mittels Isolera® Flash-Aufreinigungssystem (Biotage) die Chromatographie durchgeführt (Laufmittel: Hexan-Ethylacetat; Gradient: isokratisch 90:10 (3 SV), 90:10->80:20 (2 SV), isokratisch 80:20 (7 SV), 80:20->75:25 (1 SV), isokratisch 75:25 (7 SV)). Die vereinigten Produktfraktionen wurden eingeengt und der bräunliche Feststoff im Vakuum getrocknet. Man erhielt 3.48 g (48 % d. Th.) der Titelverbindung.
UPLC-MS (Methode A2): Rₜ = 1.15 min
MS (ESIpos): m/z = 318 (M+H)⁺
¹H NMR (300 MHz, DMSO-d6) δ =1.44 (s, 9 H), 7.51 (s, 1 H), 7.83 (s, 1 H), 8.11 (s, 1 H), 8.80 (s, 1 H).

### Intermediat 2-4

### tert-Butyl (6-hydroxy-1H-indazol-5-yl)carbamat

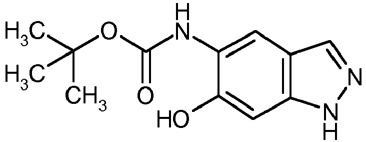

8.05 g (36.8 mmol) Di-tert-butyldicarbonat wurden in 125 ml Tetrahydrofuran suspendiert und unter Rühren mit 5.0 g (33.5 mmol) 5-Amino-1H-indazol-6-ol (Intermediat 1-2) portionsweise versetzt. Die Reaktionsmischung wurde für 24 h bei 25°C gerührt. Anschließend wurde die Reaktionsmischung eingeengt, der Rückstand mit Methanol aufgenommen, mit 2 ml 1 M Natronlauge und 2 ml Wasser versetzt. Es wurde für 30 min nachgerührt und anschließend das Methanol abdestilliert. Zum Rückstand wurde 1 M Salzsäure zugegeben, bis ein pH 7 erreicht war. Dann wurde mit Dichlormethan extrahiert, die vereinten org. Phasen über Natriumsulfat getrocknet, filtriert und eingeengt. Man erhielt 7.50 g (90 % d. Th.) der Titelverbindung.
UPLC-MS (Methode A2): Rₜ = 0.95 min
MS (ESIpos): m/z = 250 (M+H)⁺
¹H NMR (300 MHz, DMSO-d6): δ = 1.47 (s, 9 H) 6.88 (s, 1 H) 7.66 (s, 1 H) 7.82 (s, 1 H) 7.91 (s, 1 H) 10.19 (br. s., 1 H) 12.50 (s, 1 H).

### Intermediat 2-5

### tert-Butyl (6-fluoro-1H-indazol-5-yl)carbamat

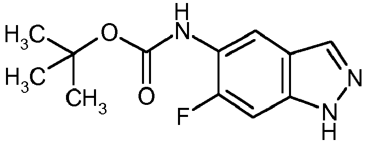

Analog zu Intermediat 2-2 wurden 4.96 g (32.8 mmol) 6-Fluoro-1H-indazol-5-amin (CAS Nr.: 709046-14-0), 7.16 g (32.8 mmol) Di-tert-butyldicarbonat und 6.28 ml (36 mmol) *N,N-*Diisopropylethylamin in 51 ml Tetrahydrofuran gelöst und 20 h bei 25°C gerührt. Man erhält 5.72 g (69 % d.Th.) der Titelverbindung.
UPLC-MS (Methode A1): Rₜ = 1.01 min
MS (ESIpos): m/z = 252 (M+H)⁺
¹H-NMR (300 MHz, DMSO-d6): δ = 1.45 (s, 9H), 7.35 (d, 1H), 7.81 (m, 1H), 8.03 (s, 1H), 8.80 (s, 1H), 13.08 (s, 1H).

### Intermediat 2-6

### tert-Butyl (6-brom-1H-indazol-5-yl)carbamat

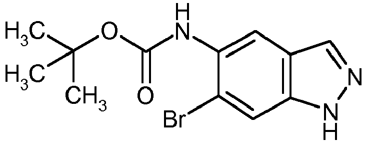

7.05 g (17.1 mmol) der Mischung aus tert-Butyl-6-brom-5-[(tert-butoxycarbonyl)amino]-1H-indazol-l-carboxylat und tert-Butyl-6-brom-5-[(tert-butoxycarbonyl)amino]-2H-indazol-2-carboxylat (Intermediat 10 und 11) wurden in 141 ml Dimethylformamid und mit 2.17 g (20.5 mmol) Natriumcarbonat in 71 ml Wasser gelöst. Die Reaktionsmischung wurde bei 85°C für 24 h erhitzt. Die Reaktionsmischung wurde mit Dichlormethan versetzt, mit 0.5 M Salzsäure und gesättigter Natriumchloridlösung gewaschen, über Natriumsulfat getrocknet und eingeengt. Das Produkt wurde im Vakuum getrocknet. Man erhielt 5.35 g (98 % d. Th.) Produkt.
UPLC-MS (Methode A2): Rₜ = 1.09 min
MS (ESIneg): m/z = 310 (M(⁷⁹Br)-H)⁺
¹H NMR (400 MHz, CHLOROFORM-*d*) δ = 1.57 (s, 9 H) 7.01 (br. s., 1 H) 7.83 (s, 1 H) 8.07 (s, 1 H) 8.50 (s, 1 H).

### Intermediat 2-7

### tert-Butyl [6-(benzyloxy)-1H-indazol-5-yl]carbamat

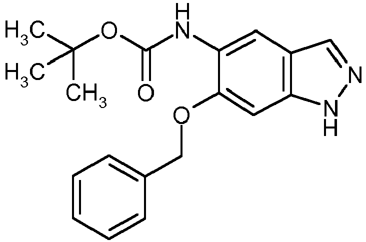

7.50 g (30.1 mmol) tert-Butyl (6-hydroxy-1H-indazol-5-yl)carbamat (Intermediat 2-4) wurde in 150 ml *N,N*-Dimethylformamid gelöst und unter Rühren mit 5.66 g (33.1 mmol) Benzylbromid und 8.32 g (60.2 mmol) Kaliumcarbonat versetzt. Die Reaktionsmischung wurde für 24 h bei 25°C gerührt. Anschließend wurde die Reaktionsmischung mit Wasser verdünnt und mit Ethylacetat extrahiert. Die vereinten organischen Phasen wurden mit gesättiger Natriumchloridlösung gewaschen, die Phasen getrennt und über einen wasserabweisenden Filter filtriert. Der Rückstand wurde in Dichlormethan aufgenommen und unter Einengen auf Isolute adsorbiert. Die Isolute wurde auf eine mit Hexan vorequilibrierte Biotage SNAP Kartusche (340 g; KP-Sil) gegeben und mittels Isolera® Flash-Aufreinigungssystem (Biotage) die Chromatographie durchgeführt (Laufmittel: Hexan-Ethylacetat; Gradient 100:0->60:40 (10 SV), isokratisch 60:40 (9 SV). Die vereinigten Produktfraktionen wurden eingeengt und im Vakuum getrocknet. Man erhielt 3.46 g (34 % d. Th.) Produkt.
UPLC-MS (Methode A2): Rₜ = 1.27 min
MS (ESIpos): m/z = 340 (M+H)⁺
1H NMR (300 MHz, CHLOROFORM-d): δ = 1.55 (s, 9 H) 5.20 (s, 2 H) 6.92 (s, 1 H) 7.14 (s, 1 H) 7.36 - 7.49 (m, 5 H) 7.94 (d, J=0.75 Hz, 1 H) 8.44 (s, 1 H).

### Intermediat 2-8

### tert-Butyl-1H-indazol-5-ylcarbama

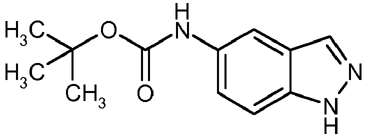

25.5 g (191.5 mmol) 1H-Indazol-5-amin (CAS-Nr. 19335-11-6) wurden in 300 ml Tetrahydrofuran vorgelegt, 37 ml *N*,*N*-Diisopropylethylamin zugegeben man addierte portionsweise 41.8 g (191.5 mmol) Di-tert-butyldicarbonat und rührte für 24 h bei 25°C. Man engte ein und erhielt 44.6 g (95 % d. Th.) der Titelverbindung.
UPLC-MS (METHODE A1): Rₜ = 0.96 min
MS (ESIpos): m/z = 234 (M+H)⁺.
¹H-NMR (300MHz, DMSO-d₆): δ = 1.44 (s, 9H), 7.24 - 7.46 (m, 2H), 7.84 (s, 1H), 7.92 (s, 1H), 9.24 (br. s., 1H), 12.86 (br. s., 1H).

### Intermediat 2-9

### tert-Butyl-(3-methyl-1H-indazol-5-yl)carbamat

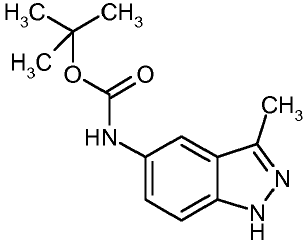

1.00 g (6.8 mmol) 3-Methyl-1H-indazol-5-amin wurden analog mit 1.48 g (6.8 mmol) Di-tert-butylcarbonat und 1.3 ml (7.5 mmol) *N*,*N*-Diisopropylethylamin in 15 ml THF über Nacht umgesetzt. Es wurden nach Einengen 1.70 g der Titelverbindung als Rohprodukt erhalten.
UPLC-MS (METHODE A1): Rt = 1.01 min
MS (ESIpos): m/z = 248 (M+H)+.

### Intermediat 2-10

### tert-Butyl-(6-isopropoxy-1H-indazol-5-yl)carbamat

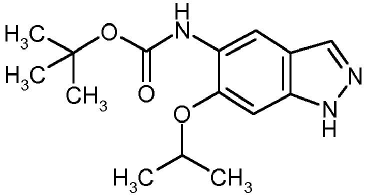

Analog zu Intermediat 2-2 wurden 2.2 g (11.6 mmol) 6-Isopropoxy-1H-indazol-5-amin Intermediat 1-4) mit 2.52 g (11.6 mmol) Di-tert-butyldicarbonat und 2.21 ml (12.7 mmol) *N,N-*Diisopropylethylamin umgesetzt. Man erhielt 2.72 g (81 % d.Th.) der Titelverbindung.
UPLC-MS (Methode A1): Rt = 1.20
MS (ESIpos): m/z = 292(M+H)⁺
1H-NMR (300 MHz, DMSO-d6): δ = 1.34 (d, 6H), 1.47 (s, 9H), 4.63- 4.74 (m, 1H), 6.98 (s, 1H), 7.68 (s, 1H), 7.88 (s, 1H), 7.94 (s, 1H), 12.68 (s, 1H).

### Intermediat 2-11

### Benzyl-(6-chlor-1H-indazol-5-yl)carbamat

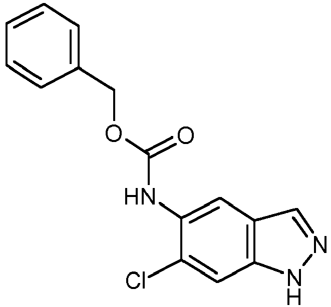

4.61 g tert-Butyl-5-{[(benzyloxy)carbonyl]amino}-6-chlor-2H-indazol-2-carboxylat (Intermediat 23-1, Rohprodukt) in 40 ml Dichlormethan wurden mit 6.1 ml Trifluoressigsäure versetzt und man rührte bei Raumtemperatur über Nacht. Man versetzte mit gesättigter wässriger Natriumhydrogencarbonatlösung, saugte den Feststoff ab, wusch mit Wasser und Diethylether und trocknete. Man erhielt 2.11 g eines hellbraunen Feststoffes (Rohprodukt).
¹H-NMR (400MHz, DMSO-d₆, ausgewählte Signale): δ [ppm]= 5.13 (s, 2H), 7.69 (s, 1H), 7.83 (s, 1H), 8.07 (s, 1H), 9.13 (br. s., 1H), 13.15 (br. s., 1H).

### Intermediat 3-1

### Ethyl- {5-[(tert-butoxycarbonyl)amino]-6-methyl-2H-indazol-2-yl} acetat

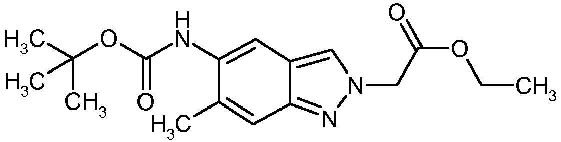

10.0 g (40.4 mmol) tert-Butyl-(6-methyl-1H-indazol-5-yl)carbamat (Intermediat 2-1) wurden mit 9.00 ml (80.9 mmol) Ethyl-bromacetat in 75 ml Tetrahydrofuran in Gegenwart von 17.1 ml (80.9 mmol) *N*,*N*-Dicyclohexylmethylamin 24 h bei 70°C gerührt. Der ausgefallene Feststoff wurde abfiltriert und zweimal mit Ethylacetat gewaschen. Das Filtrat wurde mit Wasser versetzt, man trennte die organische Phase ab und extrahierte zweimal mit Ethylacetat. Die vereinigten organischen Phasen wurden mit 1 M Salzsäurelösung, gesättigter Natriumhydrogencarbonatlösung und gesättigter Natriumchloridlösung gewaschen und eingeengt. Der Rückstand wurde in Dichlormethan aufgenommen, Isolute® HM-N (Biotage) hinzugegeben und unter Einengen auf Isolute adsorbiert. Die Isolute wurde auf eine mit Hexan vorequilibrierte Biotage SNAP Kartusche (340 g; KP-Sil) gegeben und mittels Isolera® Flash-Aufreinigungssystem (Biotage) die Chromatographie durchgeführt (Laufmittel: Hexan-Ethylacetat; Flussrate: 100 ml/mim; Gradient: isokratisch 100:10 (1 SV), 100:0->50:50 (20 SV), isokratisch 50:50 (0.2 SV)). Die vereinigten Produktfraktionen wurden eingeengt und getrocknet. Man erhielt 8.90 g (42% der Theorie) der Titelverbindung erhalten.

In einem zweiten Experiment erhielt man 213 mg der Titelverbindung analog ausgehend von 2.00 g tert-Butyl-(6-methyl-1H-indazol-5-yl)carbamat unter Verwendung von 2.24 g Kaliumcarbonat anstelle von *N*,*N*-Dicyclohexylmethylamin bei 80°C in *N,N*-Dimethylformamid und nachfolgender zweifacher Reinigung an Kieselgel (Hexan/Ethylacetat).
UPLC-MS (Methode A1): Rₜ = 1.14 min
MS (ESIpos): m/z = 334 (M+H)⁺
¹H-NMR (600MHz, DMSO-d6): δ = 1.21 (t, 3H), 1.46 (s, 9H), 2.28 (s, 3H), 4.16 (q, 2H), 5.34 (s, 2H), 7.38 (d, 1H), 7.57 (s, 1H), 8.25 (d, 1H), 8.40 (s, 1H).

### Intermediat 3-2

### Benzyl-{5-[(tert-butoxycarbonyl)amino]-6-methoxy-2H-indazol-2-yl}acetat

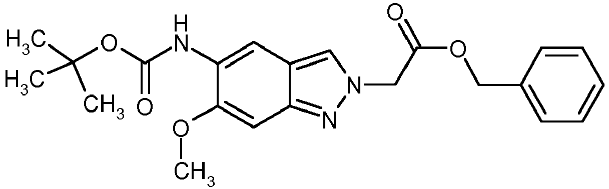

Analog zu Intermediat 3-1 wurden 4.17 g (15.8 mmol) tert-Butyl-(6-methoxy-1H-indazol-5-yl)carbamat (Intermediat 2-2) in 50 ml THF mit 2.51 ml (15.8 mmol) Bromessigsäurebenzylester und 3.36 ml (15.8 mmol) *N*,*N*-Dicyclohexylmethylamin für 4 h bei 65°C gerührt, bevor 2.51 ml (15.8 mmol) Bromessigsäurebenzylester und 3.36 ml (15.8 mmol) *N*,*N*-Dicyclohexylmethylamin addiert wurden und für weitere 18 h bei 65°C gerührt wurde. Nach Aufarbeitung und säulenchromatographischer Aufreinigung mittels Isolera® Flash-Aufreinigungssystem (Biotage) (Laufmittel: Hexan-Ethylacetat; Flussrate: 100 ml/mim; Gradient: isokratisch 100:10 (5 min), 100:0->75:25 (20 min), isokratisch 75:25 (5 min), 75:25->50:50 (15 min), isokratisch 50:50 (5 min) ), 50:50->20:80 (6 min)) erhielt man 3.22 g (47% der Theorie) der Titelverbindung.
UPLC-MS (Methode A1): Rₜ = 1.37 min
MS (ESIpos): m/z = 412 (M+H)⁺
¹H-NMR (500MHz, DMSO-d6): δ = 1.47 (s, 9H), 3.86 (s, 3H), 5.20 (s, 2H), 5.37 (s, 2H), 6.97 (s, 1H), 7.28 - 7.42 (m), 7.79 (s, 1H), 7.94 (br. s., 1H), 8.21 (s, 1H).

### Intermediat 3-3

### Ethyl {5-[(tert-butoxycarbonyl)amino]-6-(trifluoromethoxy)-2H-indazol-2-yl}acetat

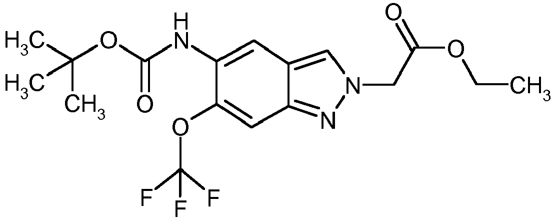

Analog zu Intermediat 3-1 wurden 3.17 g (10.0 mmol) tert-Butyl [6-(trifluoromethoxy)-1H-indazol-5-yl]carbamat (Intermediat 2-3), 5.54 ml (50 mmol) Ethyl-bromacetat und 10.7 ml (50 mmol) *N*,*N*-Dicyclohexylmethylamin in 20 ml Tetrahydrofuran für 24 h bei 70°C erhitzt. Nach Aufarbeitung und säulenchromatographischer Aufreinigung mittels Isolera® Flash-Aufreinigungssystem (Biotage) (Laufmittel: Hexan-Dichlormethan-Ethylacetat; Gradient: isokratisch 90:5:5 (5 SV), 90:5:5->85:7.5:7.5 (5 SV), isokratisch 85:7.5:7.5 (11 SV), 85:7.5:7.5->80:10:710 (3 SV), isokratisch 80:10:10 (9 SV)) erhielt man 512 mg (13 % d. Th.) Produkt.
UPLC-MS (Methode A2): Rₜ = 1.29 min
MS (ESIpos): m/z = 404 (M+H)⁺
¹H NMR (400 MHz, DMSO-d6) δ = 1.22 (t, 3 H), 1.44 (s, 9 H), 4.18 (q, 2 H), 5.42 (s, 2 H), 7.58 (s, 1 H), 7.82 (s, 1 H), 8.44 (d, 1 H), 8.75 (s, 1 H).

### Intermediat 3-4

### Ethyl {6-(benzyloxy)-5-[(tert-butoxycarbonyl)amino]-2H-indazol-2-yl}acetat

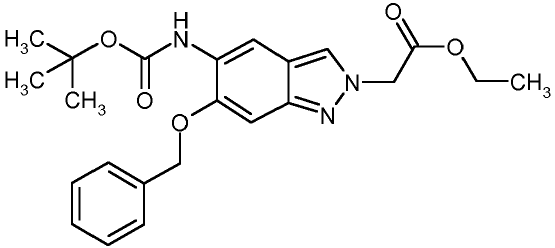

Analog zu Intermediat 3-1 wurden 3.46 g (10.2 mmol) tert-Butyl [6-(benzyloxy)-1H-indazol-5-yl]carbamat (Intermediat 2-7), 2.26 ml (20.3 mmol) Ethyl-bromacetat und 4.36 ml (20.3 mmol) N,N-Dicyclohexylmethylamin in 50 ml Tetrahydrofuran für 2 h bei 70°C erhitzt. Es wurden nochmals 2.26 ml (20.3 mmol) Ethyl-bromacetat und 4.36 ml (20.3 mmol) N,N-Dicyclohexylmethylamin hinzugegeben und für weitere 22 h bei 70°C gerührt. Nach Aufarbeitung und säulenchromatographischer Aufreinigung mittels Isolera® Flash-Aufreinigungssystem (Biotage) (Laufmittel: Hexan-Ethylacetat; Gradient 90:10->65:35 (10 SV), isokratisch 65:35 (5 SV), 65:35->50:50 (5 SV), isokratisch 50:50 (5 SV)) erhielt man 2.37 g (55 % d. Th.) der Titelverbindung.
UPLC-MS (Methode A1): Rₜ = 1.43 min
MS (ESIpos): m/z = 426 (M+H)⁺
¹H NMR (400 MHz, CHLOROFORM-d): δ = 1.28 (t, 3 H), 1.54 (s, 9 H), 4.25 (q, 2 H), 5.09 (s, 2 H), 5.19 (s, 2 H), 7.03 (s, 1 H), 7.25 (s, 1 H), 7.32 - 7.49 (m, 5 H), 7.82 (s, 1 H), 8.30 (s, 1 H).

### Intermediat 3-5

### Ethyl {5-[(tert-butoxycarbonyl)amino]-6-fluoro-2H-indazol-2-yl}acetat

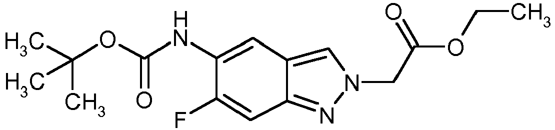

Analog zu Intermediat 3-1 wurden 5.44 g (21.6 mmol) tert-Butyl (6-fluoro-1H-indazol-5-yl)carbamat (Intermediat 2-5), 4.80 ml (43.3 mmol) Ethyl-bromacetat und 9.18 ml (43.3 mmol) *N*,*N*-Dicyclohexylmethylamin in 30 ml Tetrahydrofuran für 72 h gerührt, wobei noch zusätzlich jeweils nach 24 h und 48 h 0.96 ml (8.6 mmol) Ethyl-bromacetat und 1.84 ml (8.6 mmol) *N,N-*Dicyclohexylmethylamin zugegeben wurden. Es wurde eingeengt, in Dichlormethan aufgenommen mit Wasser gewaschen, getrocknet und eingeengt. Nach Aufarbeitung und säulenchromatographischer Aufreinigung mittels Isolera® Flash-Aufreinigungssystem (Biotage) (Laufmittel: Hexan-Dichlormethan-Ethylacetat; isokratisch 40:48:12 (8 SV)) erhielt man 3.75 g (47 % d. Th.) der Titelverbindung.
UPLC-MS (Methode A1): Rₜ = 1.15 min
MS (ESIpos): m/z = 338 (M+H)⁺
¹H-NMR (300 MHz, DMSO-d6): δ = 1.21 (t, 3H), 1.46 (s, 9H), 4.17 (q, 2H), 5.36 (s, 2H), 7.37 (d, 1H), 7.84 (d, 1H), 8.36 (s, 1H), 8.80 (s, 1H).

### Intermediat 3-6

### Ethyl {6-brom-5-[(tert-butoxycarbonyl)amino]-2H-indazol-2-yl}acetat

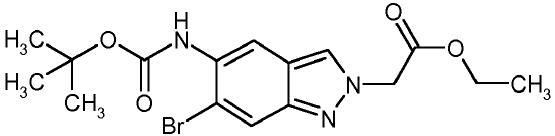

Analog zu Intermediat 3-1 wurden 4.85 g (15.5 mmol) tert-Butyl (6-brom-1H-indazol-5-yl)carbamat (Intermediat 2-6), 6.89 ml (62.1 mmol) Ethyl-bromacetat und 13.3 ml (62.1 mmol) *N*,*N*-Dicyclohexylmethylamin in 50 ml Tetrahydrofuran für 24 h bei 70°C gerührt. Nach Aufarbeitung und säulenchromatographischer Aufreinigung mittels Isolera® Flash-Aufreinigungssystem (Biotage) (Laufmittel: Hexan-Dichlormethan-Ethylacetat; Gradient: isokratisch 80:10:10 (16 SV), 80:10:10->75:12.5:12.5 (1 SV), isokratisch 75:12.5:12.5 (8 SV)) erhielt man 2.01 g (32 % d. Th.) der Titelverbindung.
UPLC-MS (Methode A2): Rₜ = 1.27 min
MS (ESIpos): m/z = 398 (M(⁷⁹Br)+H)⁺
¹H-NMR (400MHz, DMSO-d6): δ = 1.21 (t, 3H), 1.45 (s, 9H), 4.17 (q, 2H), 5.40 (s, 2H), 7.78 (s, 1H), 7.96 (s, 1H), 8.41 (d, 1H), 8.54 (s, 1H).

### Intermediat 3-7

### Ethyl-{5-[(tert-butoxycarbonyl)amino]-2H-indazol-2-yl}acetat

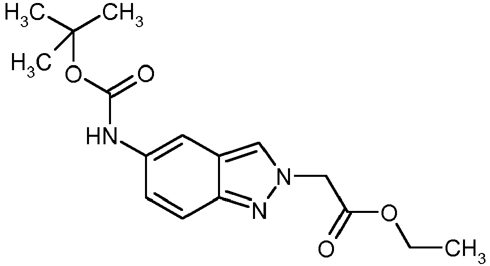

8.90 g (38.1 mmol) tert-Butyl-1H-indazol-5-ylcarbamat (Intermediat 2-8) in 80 ml *N,N-*Dimethylformamid wurden mit 10.5 g (76.3 mmol) Kaliumcarbonat und 4.67 ml (42.0 mmol) Ethyl-bromacetat versetzt und man rührte bei 80°C für 24 h. Man verdünnte mit Wasser, extrahierte mit Ethylacetat, wusch mit Wasser und gesättigter Natriumchloridlösung, trocknete die organische Phase, engte ein und reinigte den Rückstand durch Säulenchromatographie an Kieselgel (Hexan/Ethylacetat). Man erhielt 2.4 g der Titelverbindung als Hauptkomponente als Mischung mit tert-Butyl-1H-indazol-5-ylcarbamat (Ausgangsverbindung).
¹H-NMR (500MHz, CHLOROFORM-d, ausgewählte Signale): δ = 1.28 (t, 3H), 4.25 (q, 1H), 5.16 (s, 2H), 7.03 (dd, 1H), 7.62 (d, 1H).

### Intermediat 3-8

### Ethyl-{5-[(tert-butoxycarbonyl)amino]-3-methyl-2H-indazol-2-yl}acetat

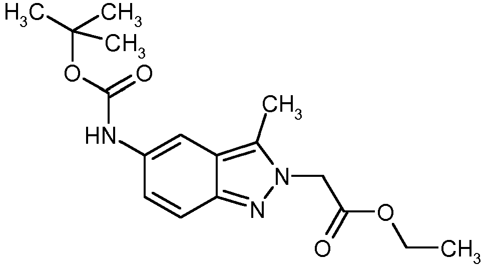

Man rührte eine Mischung aus 1.70 g tert-Butyl-(3-methyl-1H-indazol-5-yl)carbamat (Intermediat 2-9) (Rohprodukt) und 842 µl (7.6 mmol) Ethyl-bromacetat und 1.90 g (13.7 mmol) Kaliumcarbonat in 10 ml *N,N*-Dimethylformamid für 5 h bei 80°C. Man verdünnte mit Wasser, extrahierte dreimal mit Ethylacetat, wusch mit Wasser und gesättigter Natriumchloridlösung, trocknete und engte ein. Der Rückstand wurde durch säulenchromatographische Reinigung an Kieselgel (Hexan/Ethylacetat) gereinigt. Man erhielt 436 mg der Titelverbindung als Rohprodukt.
UPLC-MS (METHODE A1): Rₜ = 1.12 min
MS (ESIpos): m/z = 334 (M+H)⁺.

### Intermediat 3-9

### Ethyl-3-{5-[(tert-butoxycarbonyl)amino]-2H-indazol-2-yl}propanoat

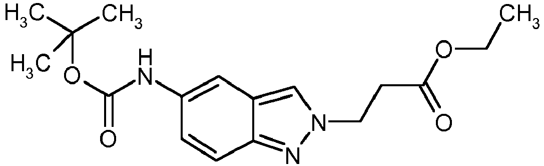

Es wurden 1.0 g (4.3 mmol) tert-Butyl-1H-indazol-5-ylcarbamat (Intermediat 2-8), 656 µl (5.1 mmol) Brompropionsäureethylester und 1.30 g (9.4 mmol) Kaliumcarbonat in 6.4 ml *N,N-*Dimethylformamid für 90 min bei 80°C erhitzt. Nach Aufarbeitung und säulenchromatographischer Aufreinigung mittels Isolera® Flash-Aufreinigungssystem (Biotage) (Laufmittel: Hexan-Ethylacetat; Gradient 100:0->80:20 (5 SV), 80:20->70:30 (5 SV), 70:30->60:40 (5 SV)) erhielt man 640 mg (45 % d. Th.) Produkt.
UPLC-MS (Methode A1): Rₜ = 1.12 min
MS (ESIpos): m/z = 334 (M+H)⁺
¹H-NMR (300 MHz, DMSO-d6): δ = 1.13 (t, 3H), 1.48 (s, 9H), 3.00 (t, 2H), 4.04 (q, 2H), 4.60 (t, 2H), 7.17 - 7.24 (m, 1H), 7.43 -7.50 (m, 1H), 7.82 (s, 1H), 8.21 (s, 1H), 9.23 (s, 1H).

### Intermediat 3-10

### Ethyl {5-[(tert-butoxycarbonyl)amino]-6-isopropoxy-2H-indazol-2-yl}acetat

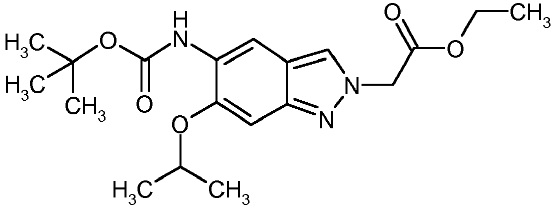

Analog zu Intermediat 3-5 wurden 2.72 g (9.3 mmol) tert-Butyl-(6-isopropoxy-1H-indazol-5-yl)carbamat (Intermediat 2-10), mit 3.10 ml (28.0 mmol) Ethyl-bromacetat umgesetzt. Man erhielt 1.84 g (52 % d.Th.) der Titelverbindung.
UPLC-MS (Methode A1): Rt = 1.32 min
MS (ESIpos): m/z = 378 (M+H)⁺
¹H NMR (600MHz, DMSO-d6): δ = 1.21 (t, 3H), 1.34(d, 6H), 1.48 (s, 9H), 4.16 (q, 2H), 4.68 - 4.75 (m, 1H), 5.27 (s,2H), 6.98 (s, 1H), 7.63 (s, 1H), 7.97 (s, 1H), 8.17 (s, 1H).

### Intermediat 3-11

### Ethyl-2-{5-[(tert-butoxycarbonyl)amino]-2H-indazol-2-yl}propanoat

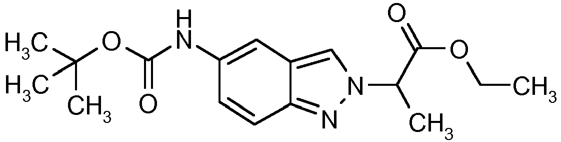

Eine Mischung aus 15.0 g (64.3 mmol) tert-Butyl-1H-indazol-5-ylcarbamat (Intermediat 2-8), 9.21 ml (70.7 mmol) Ethyl-2-brompropanoat und 17.8 g (128.6 mmol) Kaliumcarbonat in 100 ml *N,N-*Dimethylformamid wurde bei 80°C für 24 h gerührt. Man verdünnte mit Wasser, extrahierte mit Ethylacetat, wusch mit gesättigter Natriumchloridlösung und engte ein. Der Rückstand wurde durch Säulenchromatographie an Kieselgel (Hexan/Ethylacetat) gereinigt. Man erhielt 6.10 g (28 % d.Th.) der Titelverbindung.
¹H-NMR (400MHz, DMSO-d6): δ = 1.14 (t, 3H), 1.49(s, 9H), 1.77 (d, 3H), 4.07 - 4.17 (m, 2H), 5.52 (q, 1H), 7.23 (dd, 1H), 7.49 (d, 1H), 7.85 (br. s., 1H), 8.32 (s, 1H), 9.22 (s, 1H).

### Intermediat 3-12

### tert-Butyl-(5-{[(benzyloxy)carbonyl]amino}-6-chlor-2H-indazol-2-yl)acetat

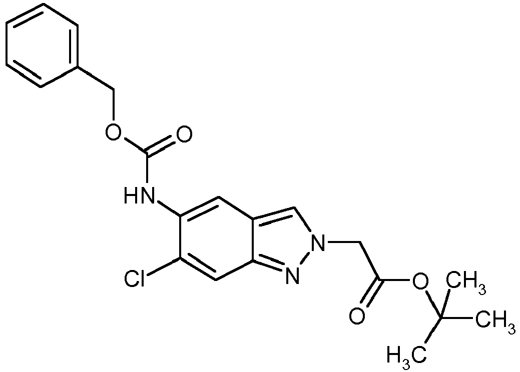

2.11 g Benzyl-(6-chlor-1H-indazol-5-yl)carbamat (Intermediat 2-11) wurden in 20 ml THF vorgelegt, man addierte 1.5 ml tert-Butyl-bromacetat und 2.2 ml *N,N*-Dicyclohexylmethylamin und rührte bei 65°C über Nacht. Man addierte erneut 0.75 ml tert-Butyl-bromacetat und 1.1 ml *N,N-*Dicyclohexylmethylamin und rührte bei 70°C über Nacht. Man filtrierte den Feststoff ab, wusch mit Ethylacetat nach, versetzte das Filtrat mit Wasser, extrahierte mit Ethylacetat, wusch mit 1M wässriger Salzsäurelösung, gesättigter Natriumhydrogencarbonatlösung, Kochsalzlösung, filtrierte über einen wasserabweisenden Filter und engte ein. Der Rückstand wurde säulenchromatographisch an Kieselgel gereinigt. Man erhielt 950 mg der Titelverbindung als gelben Schaum.
¹H-NMR (400MHz, DMSO-d6): δ = 1.43 (s, 9H), 5.14 (s, 2H), 5.29 (s, 2H), 7.29 - 7.47 (m, 5H), 7.80 (s, 1H), 7.84 (s, 1H), 8.41 (s, 1H), 9.09 (s, 1H).

### Intermediat 4-1

### {5-[(tert-Butoxycarbonyl)amino]-6-methyl-2H-indazol-2-yl} essigsäure

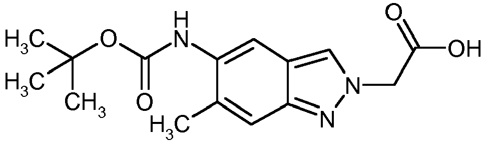

10.6 g (25.4 mmol, 80%) Ethyl-{5-[(tert-butoxycarbonyl)amino]-6-methyl-2H-indazol-2-yl}acetat (Intermediat 3-1) in 100 ml Tetrahydrofuran und 10 ml Ethanol wurden mit 10.7 g (254 mmol) Lithiumhydroxid Monohydrat gelöst in 50 ml Wasser versetzt und gerührt. Es fiel ein Feststoff aus. Nach 18 h wurde das Reaktionsgemisch mit Wasser verdünnt, mit 2M Salzsäure auf pH 4 angesäuert und der Feststoff abfiltriert, mit Wasser und Diethylether gewaschen und getrocknet. Man erhielt 6.98 g (87 % d. Th.) der Titelverbindung.
UPLC-MS (Methode A1): Rₜ = 0.92 min
MS (ESIpos): m/z = 306 (M+H)⁺
¹H-NMR (300MHz, DMSO-d6): δ = 1.44 (s, 9H), 2.25 (s, 3H), 4.78 (s, 2H), 7.32 (s, 1H), 7.49 (s, 1H), 8.10 (s, 1H), 8.35 (s, 1H).

### Intermediat 4-2

### {5-[(tert-Butoxycarbonyl)amino] -6-methoxy-2H-indazol-2-yl} essigsäure

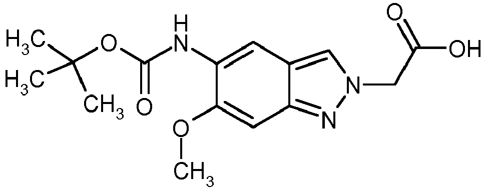

Analog zu Intermdiat 4-1 wurden ausgehend von 3.2 g Benzyl-{5-[(tert-butoxycarbonyl)amino]-6-methoxy-2H-indazol-2-yl}acetat (Intermediat 3-2) 1.91 g der Titelverbindung erhalten.
UPLC-MS (Methode A1): Rₜ = 1.04 min
MS (ESIpos): m/z = 322 (M+H)⁺
¹H-NMR (500MHz, DMSO-d6): δ = 1.47 (s, 9H), 3.86 (s, 3H), 5.16 (s, 2H), 6.96 (s, 1H), 7.78 (s, 1H), 7.93 (br. s., 1H), 8.16 (d, 1H), 13.13 (br. s., 1H).

### Intermediat 4-3

### {5-[(tert-butoxycarbonyl)amino]-6-(trifluoromethoxy)-2H-indazol-2-yl} essigsäure

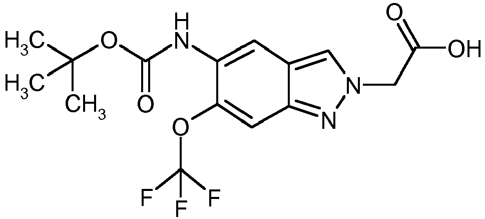

Analog zu Intermediat 4-1 wurden 530 mg (1.31 mmol) Ethyl {5-[(tert-butoxycarbonyl)amino]-6-(trifluoromethoxy)-2H-indazol-2-yl}acetat (Intermediat 3-3) in 20 ml Tetrahydrofuran suspendiert und dann mit einer Lösung von 157 mg (6.57 mmol) Lithiumhydroxid Monohydrat in 2.4 ml Wasser versetzt und 24 h bei 25°C gerührt. Nach Aufarbeitung erhielt man 437 mg (81 % d.Th.) der Titelverbindung.
UPLC-MS (Methode A1): Rₜ = 1.10 min
MS (ESIpos): m/z = 376 (M+H)⁺
¹H NMR (400 MHz, DMSO-d6): δ = 1.44 (s, 9 H), 5.29 (s, 2 H), 7.57 (s, 1 H), 7.81 (s, 1 H), 8.41 (d, 1 H), 8.74 (s, 1 H).

### Intermediat 4-4

### {6-Brom-5-[(tert-butoxycarbonyl)amino]-2H-indazol-2-yl} essigsäure

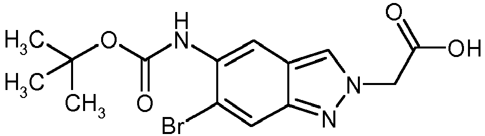

Analog zu Intermediat 4-1 wurden 1.00 g (2.5 mmol) Ethyl-{6-brom-5-[(tert-butoxycarbonyl)amino]-2H-indazol-2-yl}acetat (Intermediat 3-6) in 50 ml Tetrahydrofuran gelöst und dann mit einer Lösung von 301 mg (12.6 mmol) Lithiumhydroxid Monohydrat in 4.5 ml Wasser versetzt und 24 h bei 25°C gerührt. Nach Aufarbeitung erhielt man 844 mg (82 % d.Th.) der Titelverbindung.
UPLC-MS (Methode A1): Rₜ = 0.64 min
MS (ESIpos): m/z = 370 (M(⁷⁹Br)+H)⁺
¹H NMR (300 MHz, DMSO-d6): δ = 1.45 (s, 9 H), 3.35 (s br, 1 H), 5.28 (s, 2 H), 7.76 (s, 1 H), 7.95 (s, 1 H), 8.38 (s, 1 H), 8.52 (s, 1 H).

### Intermediat 4-5

### {5-[(tert-Butoxycarbonyl)amino]-2H-indazol-2-yl} essigsäure

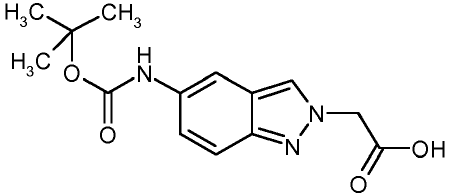

Analog zu Intermediat 4-1 wurden 5.00 g (15.6 mmol) Ethyl-{5-[(tert-butoxycarbonyl)amino]-2H-indazol-2-yl}acetat (Intermediat 3-7) in 50 ml Tetrahydrofuran und 5 ml Ethanol gelöst und dann mit einer Lösung von 6.57 g (15.6 mmol) Lithiumhydroxid Monohydrat in 20 ml Wasser versetzt und 24 h bei 25°C gerührt. Nach Aufarbeitung erhielt man 4.1 g (89 % d.Th.) der Titelverbindung.
UPLC-MS (Methode A1): Rₜ = 0.90 min
MS (ESIpos): m/z = 292 (M+H)+.

### Intermediat 4-6

### {5-[(tert-Butoxycarbonyl)amino] -3-methyl-2H-indazol-2-yl} essigsäure

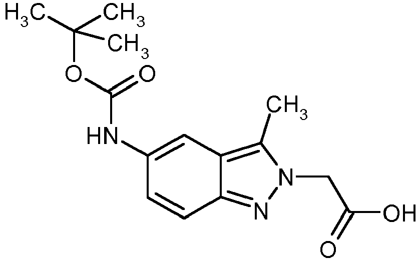

Analog zu Intermediat 4-1 wurden 436 mg (1.3 mmol) Ethyl-{5-[(tert-butoxycarbonyl)amino]-3-methyl-2H-indazol-2-yl}acetat (Intermediat 3-8) in 5 ml Tetrahydrofuran und 1 ml Ethanol gelöst und dann mit einer Lösung von 549 mg (13.1 mmol) Lithiumhydroxid Monohydrat in 2.5 ml Wasser versetzt und 24 h bei 25°C gerührt. Man erhielt nach der Zugabe von Zitronensäure einen Feststoff, der filtriert wurde, mit Wasser und Diethylether gewaschen und getrocknet wurde. Man erhielt 320 mg (70 % d.Th.) der Titelverbindung.
UPLC-MS (Methode A1): Rₜ = 0.92 min
MS (ESIpos): m/z = 306 (M+H)⁺.

### Intermediat 4-7

### 2- {5-[(tert-Butoxycarbonyl)amino]-2H-indazol-2-yl}propansäure

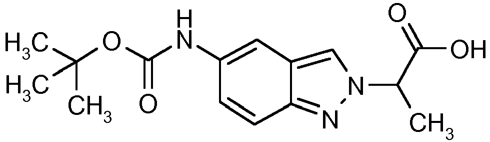

Analog zu Intermediat 4-1 wurden 5.77 g (17.3 mmol) Ethyl-2-{5-[(tert-butoxycarbonyl)amino]-2H-indazol-2-yl}propanoat (Intermediat 3-8) in 50 ml Tetrahydrofuran und 5 ml Ethanol gelöst und dann mit einer Lösung von 7.26 g (17.3 mmol) Lithiumhydroxid Monohydrat in 40 ml Wasser versetzt und 24 h bei 25°C gerührt. Nach Ansäuern mit 1 M Salzsäurelösung erhielt man einen Feststoff, der abfiltriert, mit Wasser und Diethylether gewaschen und getrocknet wurde. Man erhielt 4.2 (79 % d.Th.) der Titelverbindung.
¹H-NMR (300MHz, DMSO-d6): δ = 1.45 (s, 9H), 1.72 (d, 3H), 5.33 - 5.41 (m, 1H), 7.18 (dd, 1H), 7.45 (d, 1H), 7.82 (s, 1H), 8.26 (s, 1H), 9.20 (s, 1H), 13.13 (br. s., 1H).

### Intermediat 4-8

### {6-(Benzyloxy)-5-[(tert-butoxycarbonyl)amino]-2H-indazol-2-yl} essigsäure

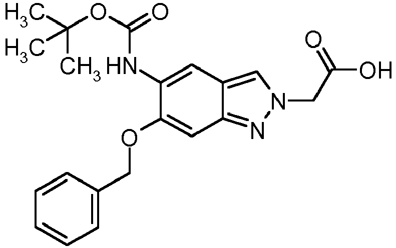

Analog zu Intermediat 4-1 wurden 14.15 g (33.3 mmol) Ethyl-{6-(benzyloxy)-5-[(tert-butoxycarbonyl)amino]-2H-indazol-2-yl}acetat (Intermediat 3-4) in 250 ml Tetrahydrofuran und 25 ml Ethanol gelöst und dann mit einer Lösung von 3.98 g (166.3 mmol) Lithiumhydroxid Monohydrat in 30 ml Wasser versetzt und 72 h bei 25°C gerührt. Nach Ansäuern mit 1 M Salzsäurelösung auf pH 3 wurde die Rekationsmischung eingengt, mit Wasser versetzt und der enstandene Feststoff abfiltriert, mit Wasser und Diethylether gewaschen und getrocknet. Man erhielt 13.05 g (33 % d.Th.) der Titelverbindung.
UPLC-MS (Methode A1): Rₜ = 1.25 min
MS (ESIpos): m/z = 398 (M+H)⁺
¹H-NMR (300MHz, DMSO-d6): δ = 1.45 (s, 9 H), 4.93 (s, 2 H), 5.20 (s, 2 H), 7.01 (s, 1 H), 7.26 - 7.45 (m, 3 H), 7.53 (d, 2 H), 7.80 - 7.91 (m, 2 H), 8.11 (s, 1 H).

### Intermediat 4-9

### (5-{[(Benzyloxy)carbonyl]amino}-6-chlor-2H-indazol-2-yl)essigsäure

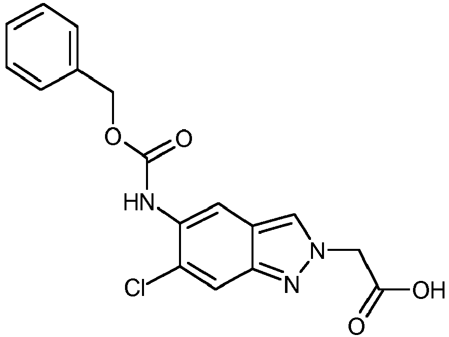

Eine Mischung aus 940 mg tert-Butyl-(5-{[(benzyloxy)carbonyl]amino}-6-chlor-2H-indazol-2-yl)acetat (Intermediat 3-12) in 10 ml Dichlormethan wurde mit 1.7 ml Trifluoressigsäure versetzt und man rührte bei Raumtemperatur über Nacht. Man versetzte mit gesättigter wässriger Natriumhydrogencarbonatlösung und saugte den Niederschlag ab, wusch mit Wasser und Ethylacetat und trocknete. Man erhielt 766 mg der Titelverbindung.
¹R-NMR (400MHz, DMSO-d₆): δ [ppm]= 4.66 (s, 2H), 5.12 (s, 2H), 7.26 - 7.45 (m, 5H), 7.69 (s, 1H), 7.75 (s, 1H), 8.22 (s, 1H), 9.01 (s, 1H).

### Intermediat 5-1

### tert-Butyl-{2-[2-(4-benzoylpiperazin-1-yl)-2-oxoethyl]-6-methyl-2H-indazol-5-yl}carbamat

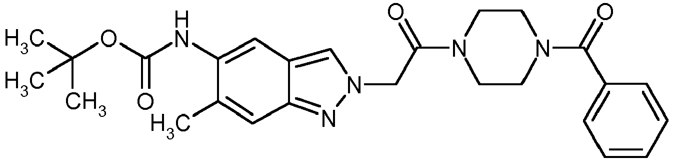

181 mg (0.59 mmol) {5-[(tert-Butoxycarbonyl)amino]-6-methyl-2H-indazol-2-yl}essigsäure (Intermediat 4-1) und 169 mg (0.89 mmol) Phenyl(piperazin-1-yl)methanon wurden in 5 ml Tetrahydrofuran und 0.5 ml *N,N*-Dimethylformamid vorgelegt. Man addierte 91 mg (0.59 mmol) 1-Hydroxy-1H-benzotriazol Hydrat, 227 mg (1.19 mmol) 1-(3-Dimethylaminopropyl)-3-ethylcarbodiimidhydrochlorid und 0.25 ml (1.79 mmol) Triethylamin und rührte 18 h bei 25°C. Man verdünnte mit Wasser und Ethylacetat, filtrierte den ausgefallenen Feststoff ab, wusch mit Wasser und Diethylether und trocknete im Vakuum. Man erhielt 248 mg (85% d.Th.) der Titelverbindung.
UPLC-MS (Methode A1): Rₜ = 1.07 min
MS (ESIpos): m/z = 478 (M+H)⁺
¹H-NMR (400MHz, DMSO-d6): δ = 1.42 (s, 9H), 2.24 (s, 3H), 3.32 - 3.82 (m, 8H), 5.41 (br. s., 2H), 7.33 (s, 1H), 7.38 - 7.48 (m, 5H), 7.52 (s, 1H), 8.12 - 8.16 (m, 1H), 8.35 (s, 1H).

### Intermediat 5-2

### tert-Butyl-(6-methyl-2-{2-oxo-2-[4-(pyrrolidin-1-yl)piperidin-1-yl]ethyl}-2H-indazol-5-yl)carbamat

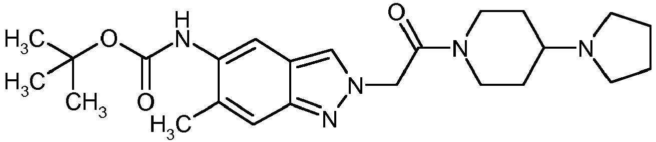

Analog zu Intermediat 5-1 wurden 2.00 g (6.55 mmol) {5-[(tert-Butoxycarbonyl)amino]-6-methyl-2H-indazol-2-yl}essigsäure (Intermediat 4-1) mit 1.31 g (8.52 mmol) 4-(Pyrrolidin-1-yl)piperidin umgesetzt. Man erhielt 2.59 g (90% d.Th.) der Titelverbindung.
UPLC-MS (Methode A1): Rₜ = 0.77 min
MS (ESIpos): m/z = 442(M+H)⁺
¹H-NMR (300MHz, DMSO-d6): δ = 1.18 - 1.52 (m, 11H, enthält Singulett bei 1.45 ppm), 1.66 (br. s., 4H), 1.83 (t, 2H), 2.16 - 2.30 (m, 4H), 2.76 - 2.90 (m, 1H), 3.08 - 3.22 (m, 1H), 3.80 - 3.92 (m, 1H), 4.01 - 4.14 (m, 1H), 5.31 - 5.46 (m, 2H), 7.35 (s, 1H), 7.53 (s, 1H), 8.15 (s, 1H), 8.39 (s, 1H).

### Intermediat 5-3

### tert-Butyl-(2-{2-[4-(3-hydroxy-2,2-dimethylpropanoyl)piperazin-1-yl]-2-oxoethyl}-6-methyl-2H-indazol-5-yl)carbamat

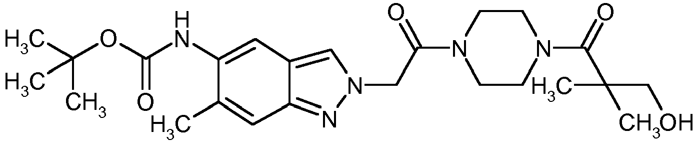

Analog zu Intermediat 5-1 wurden 300 mg (0.98 mmol) {5-[(tert-Butoxycarbonyl)amino]-6-methyl-2H-indazol-2-yl}essigsäure (Intermediat 4-1) mit 238 mg (1.28 mmol) 3-Hydroxy-2,2-dimethyl-l-(piperazin-l-yl)propan-l-on umgesetzt. Man erhielt 216 mg (46% d.Th.) der Titelverbindung.
UPLC-MS (Methode A2): Rₜ = 0.96 min
MS (ESIpos): m/z = 474 (M+H)⁺
¹H-NMR (400MHz, DMSO-d6): δ = 1.16 (s, 6H), 1.45 (s, 9H), 2.26 (s, 3H), 3.39 - 3.68 (m, 10H), 4.59 (t, 1H), 5.42 (s, 2H), 7.35 (s, 1H), 7.54 (s, 1H), 8.15 (s, 1H), 8.37 (s, 1H).

### Intermediat 5-4

### tert-Butyl-(2-{2-[4-(methoxyacetyl)piperazin-1-yl]-2-oxoethyl}-6-methyl-2H-indazol-5-yl)carbamat

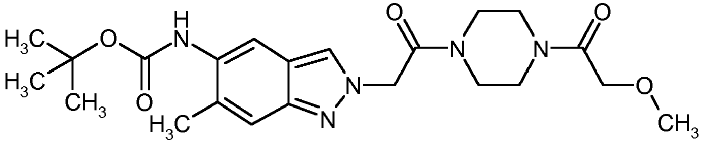

Analog zu Intermediat 5-1 wurden 300 mg (0.98 mmol) {5-[(tert-Butoxycarbonyl)amino]-6-methyl-2H-indazol-2-yl}essigsäure (Intermediat 4-1) mit 248 mg (1.28 mmol) 2-Methoxy-1-(piperazin-1-yl)ethanonhydrochlorid(1:1) umgesetzt. Man erhielt 144 mg der Titelverbindung als Rohprodukt.
UPLC-MS (Methode A2): Rₜ = 0.93 min
MS (ESIpos): m/z = 446 (M+H)⁺.

### Intermediat 5-5

### tert-Butyl-(2-{2-[4-(2-hydroxypropan-2-yl)piperidin-1-yl]-2-oxoethyl}-6-methoxy-2H-indazol-5-yl)carbamat

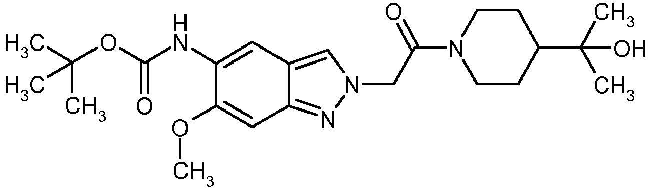

Analog zu Intermediat 5-1 wurden 266 mg (0.83 mmol) {5-[(tert-Butoxycarbonyl)amino]-6-methoxy-2H-indazol-2-yl}essigsäure (Intermediat 4-2) mit 154 mg (1.08 mmol) 2-(Piperidin-4-yl)propan-2-ol in 10 ml Tetrahydrofuran umgesetzt. Man erhielt 382 mg der Titelverbindung als Rohprodukt.
UPLC-MS (Methode A1): Rₜ = 1.10 min
MS (ESIpos): m/z = 447 (M+H)⁺.

### Intermediat 5-6

### tert-Butyl-(2- {2-[4-(cyclopropylmethyl)piperazin-1-yl]-2-oxoethyl}-6-methoxy-2H-indazol-5-yl)carbamat

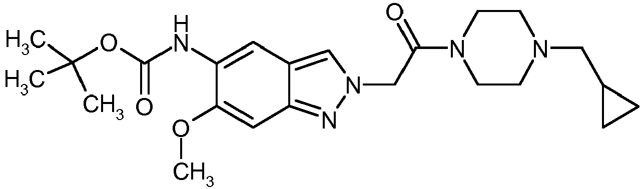

Analog zu Intermediat 5-1 wurden 250 mg (0.78 mmol) {5-[(tert-Butoxycarbonyl)amino]-6-methoxy-2H-indazol-2-yl}essigsäure (Intermediat 4-2) mit 164 mg (1.17 mmol) 1-(Cyclopropylmethyl)piperazin umgesetzt. Man erhielt 402 mg der Titelverbindung als Rohprodukt.
UPLC-MS (Methode A1): Rₜ = 0.85 min
MS (ESIpos): m/z = 444 (M+H)⁺.

### Intermediat 5-7

### tert-Butyl-{2-[2-(4-benzoylpiperazin-1-yl)-2-oxoethyl]-6-methoxy-2H-indazol-5-yl}carbamat

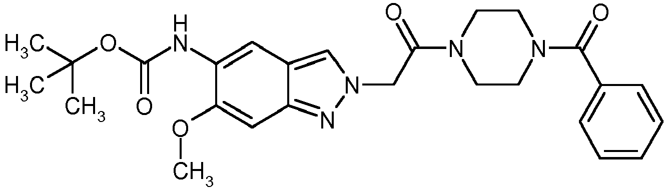

Analog zu Intermediat 5-1 wurden 548 mg (1.71 mmol) {5-[(tert-Butoxycarbonyl)amino]-6-methoxy-2H-indazol-2-yl}essigsäure (Intermediat 4-2) mit 389 mg (2.05 mmol) Phenyl(piperazin-1-yl)methanon umgesetzt. Man erhielt 808 mg der Titelverbindung als Rohprodukt.
UPLC-MS (Methode A1): Rₜ = 1.14 min
MS (ESIpos): m/z = 494 (M+H)⁺.

### Intermediat 5-8

### tert-Butyl {2-[2-(4-methylpiperazin-1-yl)-2-oxoethyl]-6-(trifluoromethoxy)-2H-indazol-5-yl}carbamat

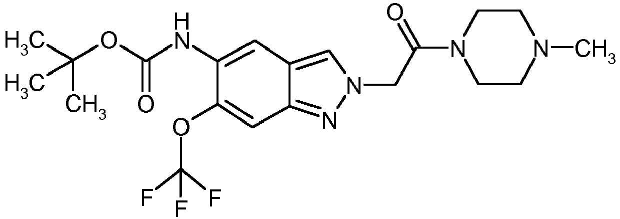

350 mg (0.85 mmol) tert-Butyl {2-[2-(4-methylpiperazin-1-yl)-2-oxoethyl]-6-(trifluoromethoxy)-2H-indazol-5-yl}carbamat (Intermediat 4-3) wurde mit 130 mg (0.85 mmol) 1-Hydroxy-1H-benzotriazol Hydrat und 325 mg (1.70 mmol) 1-(3-Dimethylaminopropyl)-3-ethylcarbodiimidhydrochlorid in 3.5 ml *N,N*-Dimethylformamidund und 473 µl (3.40 mmol) Triethylamin 30 min bei 25°C gerührt. Dann wurden 103 µL (0.93 mmol) 1-Methylpiperazin (CAS Nr.: 109-01-3) zugegeben und für 24 h bei 25°C gerührt. Es wurde auf 50 ml Wasser gegossen, abgesaugt, mit Wasser gewaschen und getrocknet. Man erhielt 305 mg (78 % d.Th.) der Titelverbindung.
UPLC-MS (Methode A2): Rₜ = 1.12 min
MS (ESIpos): m/z = 376 (M+H)⁺
¹H NMR (400 MHz, DMSO-d6): δ = 1.44 (s, 9 H), 2.23 (s, 3 H), 2.28 - 2.38 (m, 2 H), 2.41 (br. s., 2 H), 3.47 (br. s., 2 H), 3.55 (br. s., 2 H), 5.49 (s, 2 H), 7.54 (s, 1 H), 7.80 (s, 1 H), 8.34 (d, 1 H), 8.73 (s, 1 H), 9.93 (br. s., 1 H).

### Intermediat 5-9

### tert-Butyl {6-bromo-2-[2-(4-methylpiperazin-1-yl)-2-oxoethyl]-2H-indazol-5-yl}carbamat

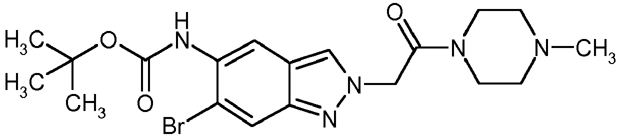

Analog zu Intermediat 5-8 wurden 800 mg (1.97 mmol) {6-Brom-5-[(tert-butoxycarbonyl)amino]-2H-indazol-2-yl} essigsäure (Intermediat 4-4) mit 246 µl (2.17 mmol) 1-Methylpiperazin umgesetzt Man erhielt 824 mg (93 % d.Th.) der Titelverbindung.
UPLC-MS (Methode A2): Rₜ = 1.07 min
MS (ESIpos): m/z = 452 (M(⁷⁹Br)+H)⁺
¹H NMR (300 MHz, DMSO-d6): δ = 1.45 (s, 9 H), 2.20 (s, 3 H), 2.25 - 2.34 (m, 2 H), 2.34 - 2.40 (m, 2 H), 3.43 - 3.49 (m, 2 H), 3.50 - 3.55 (m, 2 H), 5.47 (s, 2 H), 7.75 (s, 1 H), 7.93 (s, 1 H), 8.31 (s, 1 H), 8.54 (s, 1 H).

### Intermediat 5-10

### tert-Butyl-(2-{2-[4-(cyclopropylcarbonyl)piperazin-1-yl]-2-oxoethyl}-2H-indazol-5-yl)carbamat

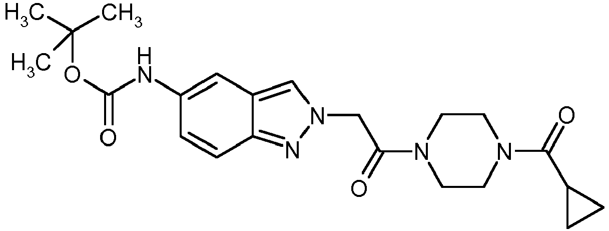

Analog zu Intermediat 5-1 wurden 2.00 g (4.3 mmol, 62%) {5-[(tert-Butoxycarbonyl)amino]-2H-indazol-2-yl}essigsäure (Intermediat 4-5) mit 1.14 g (6.0 mmol) Cyclopropyl(piperazin-1-yl)methanonhydrochlorid(1:1) umgesetzt. Man erhielt 2.3 g der Titelverbindung als Rohprodukt.
UPLC-MS (Methode A1): Rₜ = 0.97 min
MS (ESIpos): m/z = 428 (M+H)⁺.

### Intermediat 5-11

### tert-Butyl-{2-[2-(4-benzoylpiperazin-1-yl)-2-oxoethyl]-2H-indazol-5-yl}carbamat

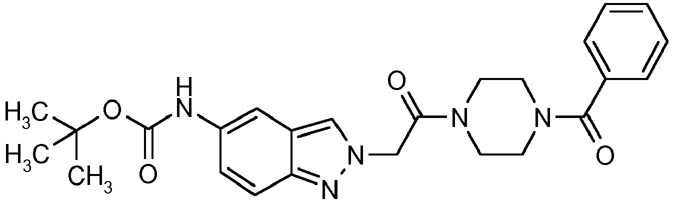

Analog zu Intermediat 5-1 wurden 2.53 g (8.7 mmol) {5-[(tert-Butoxycarbonyl)amino]-2H-indazol-2-yl}essigsäure (Intermediat 4-5) mit 1.98 g (10.4 mmol) Phenyl(piperazin-1-yl)methanon zu 3.8 g (93 % d.Th.) der Titelverbindung umgesetzt.
UPLC-MS (Methode A1): Rₜ = 1.05 min
MS (ESIpos): m/z = 464 (M+H)+.
¹H-NMR (400MHz, DMSO-d₆): δ = 1.45 (s, 9H), 3.30 - 3.78 (m, 8H), 5.41 (br. s., 2H), 7.18 (dd, 1H), 7.35 - 7.50 (m, 6H), 7.82 (br. s., 1H), 8.11 (s, 1H), 9.18 (s, 1H).

### Intermediat 5-12

### tert-Butyl- {2-[2-(4-methylpiperazin-1-yl)-2-oxoethyl]-2H-indazol-5-yl}carbamat

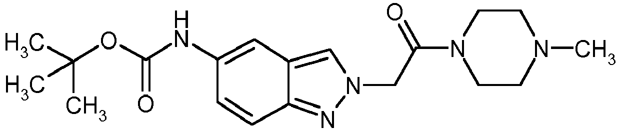

Analog zu Intermediat 5-1 wurden 1.00 g (3.4 mmol) {5-[(tert-Butoxycarbonyl)amino]-2H-indazol-2-yl}essigsäure (Intermediat 4-5) mit 0.41 g (4.1 mmol) 1-Methylpiperazin zu 916 mg (71 % d.Th.) der Titelverbindung umgesetzt.
UPLC-MS (Methode A1): Rt = 0.73 min
MS (ESIpos): m/z = 374 (M+H)+.

### Intermediat 5-13

### tert-Butyl-(2-{2-oxo-2-[4-(2,2,2-trifluorethyl)piperazin-1-yl]ethyl}-2H-indazol-5-yl)carbamat

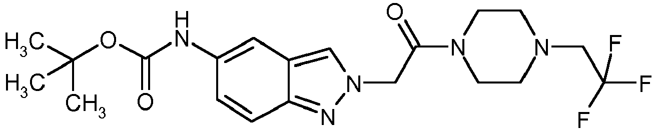

Analog zu Intermediat 5-1 wurden 1.01 g (3.5 mmol) {5-[(tert-Butoxycarbonyl)amino]-2H-indazol-2-yl}essigsäure (Intermediat 4-5) mit 1.00 g (4.2 mmol) 1-(2,2,2-Trifluorethyl)piperazindihydrochlorid zu 634 mg (42 % d.Th.) der Titelverbindung umgesetzt.
UPLC-MS (Methode A1): Rₜ = 1.11 min
MS (ESIpos): m/z = 442 (M+H)⁺.

### Intermediat 5-14

### tert-Butyl- {2-[2-(4-ethyl-3-oxopiperazin-1-yl)-2-oxoethyl]-2H-indazol-5-yl}carbamat

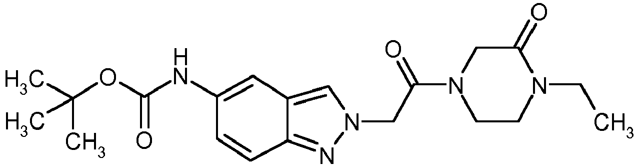

Analog zu Intermediat 5-1 wurden 2.38 g (3.5 mmol, 62%) {5-[(tert-Butoxycarbonyl)amino]-2H-indazol-2-yl}essigsäure (Intermediat 4-5) mit 1.00 g (6.1 mmol) 1-Ethylpiperazin-2-onhydrochlorid(1:1) zu 1.92 g (71 % d.Th.) der Titelverbindung als Rohprodukt umgesetzt.
UPLC-MS (Methode A1): Rₜ = 0.92 min
MS (ESIpos): m/z = 402 (M+H)⁺.

### Intermediat 5-15

### tert-Butyl-{2-[2-(4-benzoylpiperazin-1-yl)-2-oxoethyl]-3-methyl-2H-indazol-5-yl }carbamat

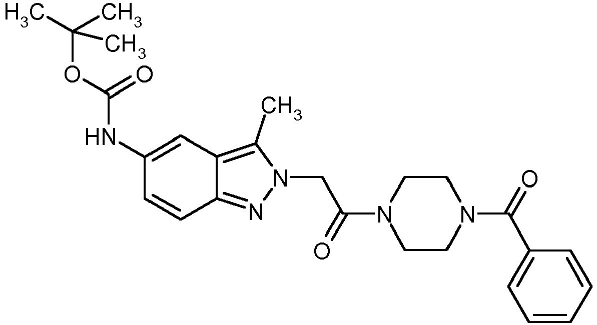

Analog zu Intermediat 5-1 wurden 160 mg (0.52 mmol) {5-[(tert-Butoxycarbonyl)amino]-3-methyl-2H-indazol-2-yl}essigsäure (Rohprodukt) (Intermediat 4-6) wurden analog mit 150 mg (0.79mmol) Phenyl(piperazin-1-yl)methanon umgesetzt. Nach Zugabe von Wasser und Ethylacetat fiel ein Feststoff aus, der mit Wasser und Diethylether gewaschen wurde und getrocknet wurde. Man erhielt 130 mg (52 % d.Th.) der Titelverbindung.
UPLC-MS (Methode A1): Rₜ = 1.07 min
MS (ESIpos): m/z = 478 (M+H)⁺.

### Intermediat 5-16

### tert-Butyl-{6-methoxy-2-[2-(morpholin-4-yl)-2-oxoethyl]-2H-indazol-5-yl}carbamat

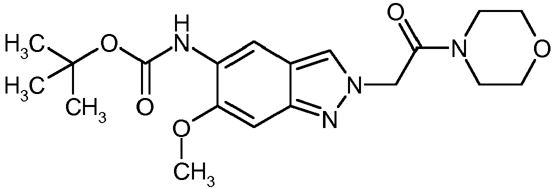

Analog zu Intermediat 5-1 wurden 1.00 g (3.11 mmol) {5-[(tert-Butoxycarbonyl)amino]-6-methoxy-2H-indazol-2-yl} essigsäure (Intermediat 4-2) mit 407 µL (4.67 mmol) 1-Methylpiperazin umgesetzt. Das Reaktionsgemisch wurde auf Wasser gegeben und mit Ethylacetat extrahiert. Die vereinigten, organischen Phasen wurden mit gesättigter Natriumchloridlösung gewaschen, mit Natriumsulfat getrocknet, filtriert, eingeengt und getrocknet. Man erhielt 1.16 g (95 % d.Th.) der Titelverbindung.
UPLC-MS (Methode A2): Rₜ = 1.03 min
MS (ESIpos): m/z = 391 (M+H)⁺
¹H NMR (300 MHz, CHLOROFORM-d): δ = 1.55 (s, 9 H), 3.58 (s, 4 H), 3.66 (s, 4 H), 3.93 (s, 3 H), 5.18 (s, 2 H), 6.94 (s, 1 H), 7.22 (s, 1 H), 7.81 - 7.90 (m, 1 H), 8.25 (s, 1 H).

### Intermediat 5-17

### tert-Butyl- {6-methoxy-2-[2-(4-methylpiperazin-1-yl)-2-oxoethyl]-2H-indazol-5-yl}carbamat

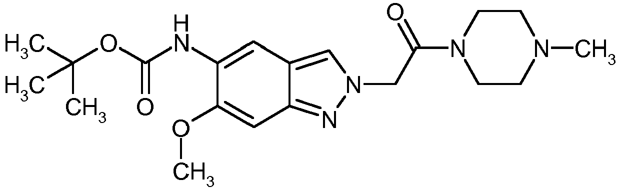

Analog zu Intermediat 5-16 wurden 1.00 g (3.11 mmol) {5-[(tert-Butoxycarbonyl)amino]-6-methoxy-2H-indazol-2-yl} essigsäure (Intermediat 4-2) mit 530 µL (4.67 mmol) 1-Methylpiperazin umgesetzt. Nach Aufarbeitung erhielt man 1.21 g (96 % d.Th.) der Titelverbindung.
UPLC-MS (Methode A1): Rₜ = 0.82 min
MS (ESIpos): m/z = 404 (M+H)⁺
¹H NMR (300 MHz, CHLOROFORM-d): δ = 1.55 (s, 9 H), 2.28 (s, 3 H), 2.30 - 2.34 (m, 2 H), 2.34 - 2.41 (m, 3 H), 3.52 - 3.61 (m, 2 H), 3.62 - 3.71 (m, 2 H), 3.93 (s, 3 H), 5.18 (s, 2 H), 6.94 (s, 1 H), 7.22 (s, 1 H), 7.85 (s, 1 H), 8.24 (s, 1 H).

### Intermediat 5-18

### tert-Butyl-(2-{2-[(cyclopropylmethyl)(methyl)amino]-2-oxoethyl}-6-methoxy-2H-indazol-5-yl)carbamat

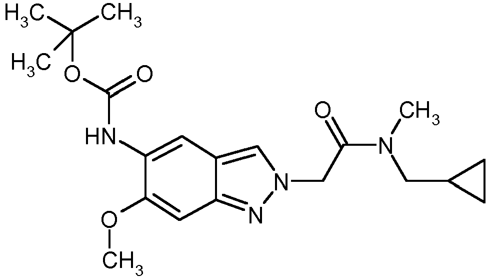

Analog zu Intermediat 5-1 wurden 250 mg (0.78 mmol) {5-[(tert-Butoxycarbonyl)amino]-6-methoxy-2H-indazol-2-yl}essigsäure (Intermediat 4-2) mit 86 mg (1.01 mmol) 1-Cyclopropyl-N-methylmethanamin für 24 h bei 25°C gerührt Man verdünnte mit Wasser, extrahierte dreimal mit Ethylacetat, wusch mit gesättigter Natriumchloridlösung und engte ein. Man erhielt 353 mg eines Rohproduktes.
UPLC-MS (Methode A1): Rₜ = 1.19 min
MS (ESIpos): m/z = 389 (M+H)⁺.

### Intermediat 5-19

### tert-Butyl- {2-[1 -(4-benzoylpiperazin-1 -yl)-1 -oxopropan-2-yl] -2H-indazol-5-yl} carbamat

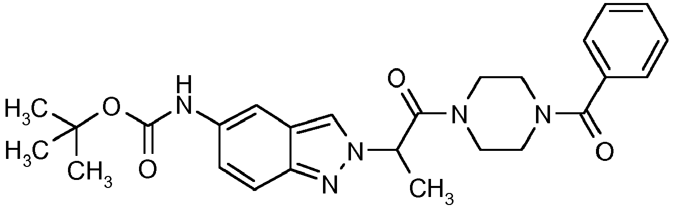

Analog zu Intermediat 5-1 wurden 2.00 g (6.55 mmol) 2-{5-[(tert-Butoxycarbonyl)amino]-2H-indazol-2-yl}propansäure (Intermediat 4-6) und 1.50 g (7.86 mmol) Phenyl(piperazin-1-yl)methanon für 24 h bei 25°C gerührt. Man erhielt 3.7 g der Titelverbindung als Rohprodukt.
UPLC-MS (Methode A1): Rₜ = 1.11 min
MS (ESIpos): m/z = 448 (M+H)⁺.

### Intermediat 5-20

### tert-Butyl-{6-(benzyloxy)-2-[2-(morpholin-4-yl)-2-oxoethyl]-2H-indazol-5-yl}carbamat

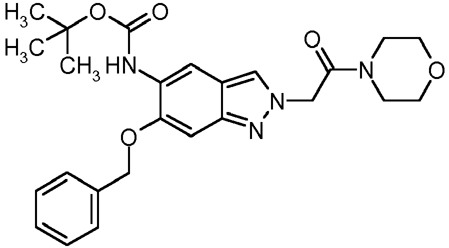

Analog zu Intermediat 5-1 wurden 3.50 g (8.81 mmol) {6-(Benzyloxy)-5-[(tert-butoxycarbonyl)amino]-2H-indazol-2-yl}essigsäure (Intermediat 4-8) mit 1.14 ml (13.2 mmol) Morpholin innerhalb 24 h bei 25°C umgesetzt. Nach Aufarbeitung erhielt man 3.67 g (89 % d.Th.) der Titelverbindung.
UPLC-MS (Methode A1): Rₜ = 1.25 min
MS (ESIpos): m/z = 467 (M+H)⁺
¹H-NMR (400MHz, DMSO-d6): δ = 1.45 (s, 9 H), 3.41 - 3.48 (m, 2 H), 3.51 - 3.60 (m, 4 H), 3.61 - 3.66 (m, 2 H), 5.21 (s, 2 H), 5.35 (s, 2 H), 7.01 (s, 1 H), 7.29 - 7.37 (m, 1 H), 7.38 - 7.44 (m, 2 H), 7.50 - 7.57 (m, 2 H), 7.87 (s, 2 H), 8.11 (s, 1 H).

### Intermediat 5-21

### Benzyl- {6-chlor-2-[2-(4-methylpiperazin-1-yl)-2-oxoethyl]-2H-indazol-5-yl}carbamat

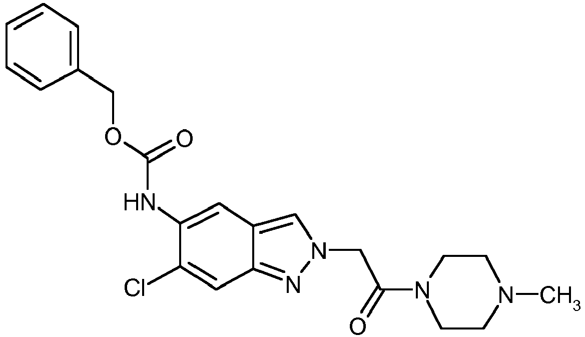

387 mg (5-{[(Benzyloxy)carbonyl]amino}-6-chlor-2H-indazol-2-yl)essigsäure (Intermediat 4-9) wurden analog zur Herstellung von Intermediat 5-1 mit 140 mg 1-Methylpiperazin umgesetzt. Nach der Reaktion verdünnte man mit Wasser, setzte Ethylacetat zu und gesättigte Kochsalzlösung. Der ausgefallene Feststoff wurde abfiltriert, mit Wasser und Diethylether gewaschen und getrocknet. Man erhielt 302 mg der Titelverbindung.
¹H-NMR (400MHz, DMSO-d₆): δ [ppm]= 2.19 (s, 3H), 2.23 - 2.41 (m, 4H), 3.41 - 3.48 (m, 2H), 3.48 - 3.56 (m, 2H), 5.13 (s, 2H), 5.46 (s, 2H), 7.28 - 7.45 (m, 5H), 7.75 (s, 1H), 7.81 (s, 1H), 8.32 (d, 1H), 9.07 (s, 1H).

### Intermediat 5-22

### Benzyl- {6-chlor-2-[2-(morpholin-4-yl)-2-oxoethyl]-2H-indazol-5-yl}carbamat

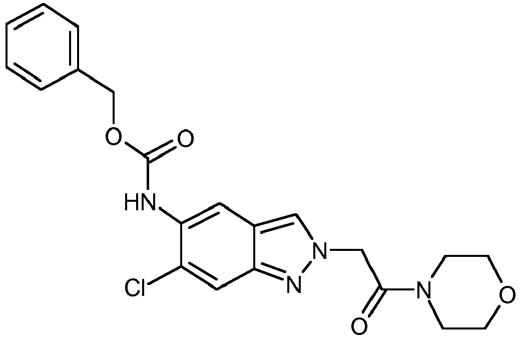

400 mg Benzyl-(6-chlor-1H-indazol-5-yl)carbamat (Intermediat 2-11) wurden in 5.0 ml Cyclopentylmethylether vorgelegt. Man addierte 265 mg 2-Brom-1-(morpholin-4-yl)ethanon und 0.22 ml N-Ethyl-N-isopropylpropan-2-amin und rührt 20 h bei 100°C. Man versetzte mit Wasser und erhielt einen Feststoff durch das Abkratzen von öligen Rückständen vom Kolbenrand. Der Feststoff wurde abgesaugt, mit Wasser und Diethylether gewaschen, aus Ethylacetat ausgerührt und getrocknet. Man erhielt 254 mg der Titelverbindung.
1H-NMR (500MHz, DMSO-d6): δ[ppm] = 3.47 (d, 2H), 3.56 (d, 2H),3.58 - 3.61 (m, 2H), 3.65 (d, 2H), 5.15 (s, 2H), 5.49 (s, 2H), 7.28-7.48 (m, 5H), 7.76 (s, 1H), 7.83 (s, 1H), 8.33 (s, 1H), 9.07 (s, 1H).

### Intermediat 6-1

### 2-(5-Amino-6-methyl-2H-indazol-2-yl)-1-(4-benzoylpiperazin-1 -yl)ethanon

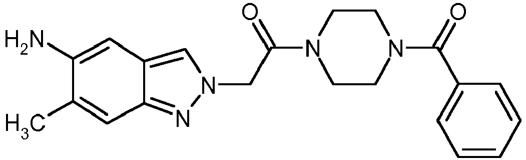

Man addierte 0.3 ml (3.89 mmol) Trifluoressigsäure zu 247 mg (0.52 mmol) tert-Butyl-{2-[2-(4-benzoylpiperazin-1-yl)-2-oxoethyl]-6-methyl-2H-indazol-5-yl}carbamat (Intermediat 5-1) in 5 ml Dichlormethan und rührte 18 h bei 25°C. Dann wurden erneut 0.3 ml (3.89 mmol) Trifluoressigsäure zugegeben, man rührte 18 h und goss auf gesättigte Natriumhydrogencarbonatlösung und extrahierte drei Mal mit Dichlormethan. Nach Einengen wurden 223 mg der Titelverbindung als Rohprodukt erhalten.
UPLC-MS (Methode A1): Rₜ = 0.61 min
MS (ESIpos): m/z = 378 (M+H)⁺
¹H-NMR (400MHz, DMSO-d6): δ = 2.15 (s, 3H), 3.29 - 3.75 (m, 8H), 4.53 (s, 2H), 5.28 (br. s., 2H), 6.63 (s, 1H), 7.17 (s, 1H), 7.37 - 7.47 (m, 5H), 7.75 - 7.79 (m, 1H).

### Intermediat 6-2

### 2-(5-Amino-6-methyl-2H-indazol-2-yl)-1-[4-(pyrrolidin-1-yl)piperidin-1-yl]ethanon

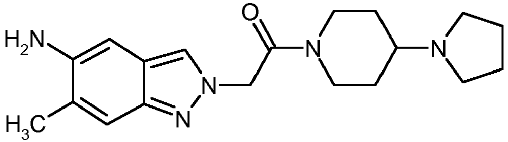

2.59 g (5.87 mmol) tert-Butyl-(6-methyl-2-{2-oxo-2-[4-(pyrrolidin-1-yl)piperidin-1-yl]ethyl}-2H-indazol-5-yl)carbamat (Intermediat 5-2) wurden in 30 ml Dichlormethan vorgelegt, man addierte 4.5 ml (58.7 mmol) Trifluoressigsäure und rührte 78 h bei 25°C. Das Reaktionsgemisch wurde eingeengt, man gab zwei Mal Toluol zu und engte jeweils ein. Der Rückstand wurde durch HPLC nach der Methode P2 (Gradient: 0-0,5 min 25 mL/min auf 70 mL/min 25% B; 0,5-5,5 min 25-55% B; Fluss: 70 mL/min) gereinigt. Man erhielt 1.04 g (52% der Theorie) der Titelverbindung.
UPLC-MS (Methode A2): Rₜ = 0.81 min
MS (ESIpos): m/z = 342 (M+H)⁺
¹H-NMR (300MHz, DMSO-d6): δ = 1.16 - 1.47 (m, 2H), 1.66 (br. s., 4H), 1.82 (br. s., 2H), 2.12 - 2.28 (m, 4H), 2.74 - 2.89 (m, 1H), 3.05 - 3.20 (m, 1H), 3.79 - 3.92 (m, 1H), 4.02 - 4.14 (m, 1H), 4.58 (br. s., 2H), 5.18 - 5.33 (m, 2H), 6.65 (s, 1H), 7.19 (s, 1H), 7.78 (d, 1H).

### Intermediat 6-3

### 1-{4-[(5-Amino-6-methyl-2H-indazol-2-yl)acetyl]piperazin-1-yl}-3-hydroxy-2,2-dimethylpropan-1-on

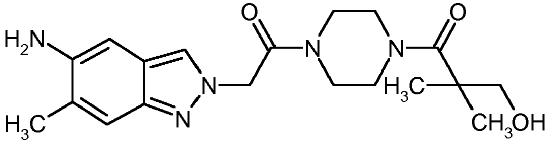

207 mg (0.44 mmol) tert-Butyl-(2-{2-[4-(3-hydroxy-2,2-dimethylpropanoyl)piperazin-1-yl]-2-oxoethyl}-6-methyl-2H-indazol-5-yl)carbamat (Intermediat 5-3) in 5 ml Dichlormethan wurden mit 0.34 ml (4.37 mmol) Trifluoressigsäure versetzt und man rührte 2 Tage bei 25°C. Man goss auf gesättigte Natriumhydrogencarbonatlösung, extrahierte dreimal mit Dichlormethan und engte ein. Man erhielt 184 mg der Titelverbindung als Rohprodukt.
UPLC-MS (Methode A1): Rₜ = 0.52 min
MS (ESIpos): m/z = 374 (M+H)⁺.

### Intermediat 6-4

### 2-(5-Amino-6-methyl-2H-indazol-2-yl)-1-[4-(methoxyacetyl)piperazin-1-yl]ethanon

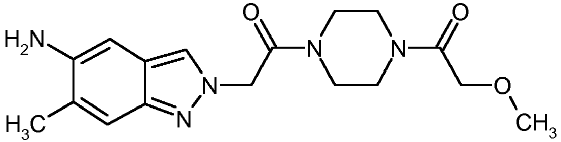

144 mg (0.32 mmol) tert-Butyl-(2-{2-[4-(methoxyacetyl)piperazin-1-yl]-2-oxoethyl}-6-methyl-2H-indazol-5-yl)carbamat (Intermediat 5-4) in 3 ml Dichlormethan wurden mit 0.25 ml (3.23 mmol) Trifluoressigsäure versetzt und man rührte bei 25°C für 24 h. Man engte ein und erhielt 219 mg der Titelverbindung als Rohprodukt.
UPLC-MS (Methode A1): Rₜ = 0.46 min
MS (ESIpos): m/z = 346 (M+H)⁺.

### Intermediat 6-5

### 2-(5-Amino-6-methoxy-2H-indazol-2-yl)-1-[4-(2-hydroxypropan-2-yl)piperidin-1-yl]ethanon

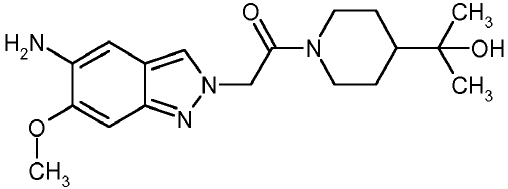

382 mg (0.86 mmol) tert-Butyl-(2-{2-[4-(2-hydroxypropan-2-yl)piperidin-1-yl]-2-oxoethyl}-6-methoxy-2H-indazol-5-yl)carbamat (Intermediat 5-5) in 5 m Dichlormethan wurden mit 261 µl (3.38 mmol) Trifluoressigsäure versetzt und die Mischung wurde 18 h bei 25°C gerührt. Man addierte erneut 609 µl (7.90 mmol) Trifluoressigsäure und rührte weiter bei 25°C bis zum vollständigen Umsatz. Man engte ein und addierte drei Mal Toluol und entfernte Toluol jeweils im Vakuum. Man erhielt 735 mg der Titelverbindung als Rohprodukt.
UPLC-MS (Methode A1): Rₜ = 0.57 min
MS (ESIpos): m/z = 347 (M+H)⁺.

### Intermediat 6-6

### 2-(5-Amino-6-methoxy-2H-indazol-2-yl)-1-[4-(cyclopropylmethyl)piperazin-1-yl]ethanon

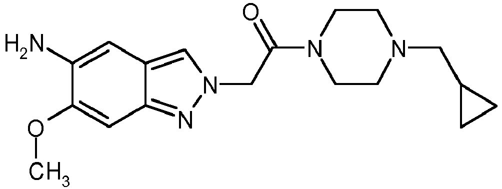

Analog zu Intermediat 6-5 wurden 402 mg (0.86 mmol) tert-Butyl-(2-{2-[4-(cyclopropylmethyl)piperazin-1-yl]-2-oxoethyl}-6-methoxy-2H-indazol-5-yl)carbamat (Intermediat 5-6) mit 663 µl (8.61 mmol) Trifluoressigsäure in 5 ml Dichlormethan umgesetzt. Man erhielt 822 mg der Titelverbindung als Rohprodukt.

### Intermediat 6-7

### 2-(5-Amino-6-methoxy-2H-indazol-2-yl)-1-(4-benzoylpiperazin-1-yl)ethanon

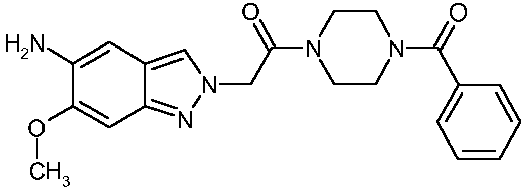

Analog zu Intermediat 6-3 wurden 808 mg (1.64 mmol) tert-Butyl-{2-[2-(4-benzoylpiperazin-1-yl)-2-oxoethyl]-6-methoxy-2H-indazol-5-yl}carbamat (Intermediat 5-7) mit 1,26 ml (16.37 mmol) Trifluoressigsäure in 10 ml Dichlormethan für 18 h bei 25C° gerührt. Nach Aufarbeitung erhielt man 649 mg (99% der Theorie) der Titelverbindung.
UPLC-MS (Methode A1): Rₜ = 0.63 min
MS (ESIpos): m/z = 394 (M+H)⁺
¹H-NMR (400MHz, DMSO-d6): δ = 3.33 - 3.79 (8H), 3.81 (s, 3H), 4.60 (s, 2H), 5.27 (br. s., 2H), 6.62 (s, 1H), 6.78 (s, 1H), 7.39 - 7.50 (m, 5H), 7.76 (s, 1H).

### Intermediat 6-8

### 2-[5-Amino-6-(trifluoromethoxy)-2H-indazol-2-yl]-1-(4-methylpiperazin-1-yl)ethanon

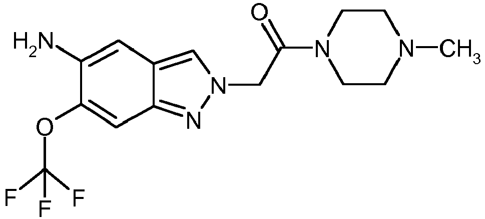

Analog zu Intermediat 6-4 wurden 484 mg (1.06 mmol) tert-Butyl {2-[2-(4-methylpiperazin-1-yl)-2-oxoethyl]-6-(trifluoromethoxy)-2H-indazol-5-yl}carbamat (Intermediat 5-8) mit 815 µL Trifluoressigsäure in 5 ml Dichlormethan umgesetzt. Nach Aufarbeitung erhielt man 320 mg (85 % d. Th.) der Titelverbindung.
UPLC-MS (Methode A2): Rₜ = 0.79 min
MS (ESIpos): m/z = 357 (M+H)⁺
¹H NMR (400 MHz, DMSO-d6): δ = 2.16 - 2.24 (m, 3 H), 2.28 (t, 2 H), 2.32 - 2.40 (m, 2 H), 3.41 - 3.49 (m, 2 H), 3.49 - 3.56 (m, 2 H), 4.95 (s, 2 H), 5.36 (s, 2 H), 6.88 (s, 1 H), 7.39 (s, 1 H), 7.98 (s, 1 H).

### Intermediat 6-9

### 2-(5-Amino-6-bromo-2H-indazol-2-yl)-1-(4-methylpiperazin-1-yl)ethanon

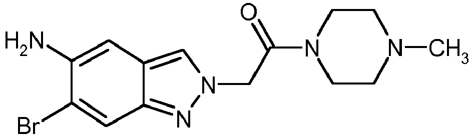

Analog zu Intermediat 6-4 wurden 293 mg (0.65 mmol) tert-Butyl {6-bromo-2-[2-(4-methylpiperazin-1-yl)-2-oxoethyl]-2H-indazol-5-yl}carbamat (Intermediat 5-9) mit 499 µL (6.48 mmol) Trifluoressigsäure in 3 ml Dichlormethan umgesetzt. Nach Aufarbeitung erhielt man 210 mg (92 % d. Th.) der Titelverbindung.
UPLC-MS (Methode A2): Rₜ = 0.70 min
MS (ESIpos): m/z = 352 (M(⁷⁹Br)+H)⁺
¹H NMR (400 MHz, DMSO-d6): δ = 2.20 (s, 3 H), 2.27 (t, 2 H), 2.31 - 2.40 (m, 2 H), 3.41 - 3.48 (m, 2 H), 3.49 - 3.56 (m, 2 H), 4.91 (s, 2 H), 5.34 (s, 2 H), 6.92 (s, 1 H), 7.77 (s, 1 H), 7.95 (d, 1 H).

### Intermediat 6-10

### 2-(5-Amino-2H-indazol-2-yl)-1-[4-(cyclopropylcarbonyl)piperazin-1-yl]ethanon

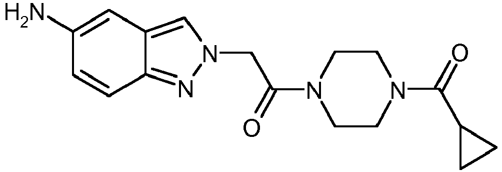

Analog zu Intermediat 6-4 wurden 2.3 g (5.38 mmol) tert-Butyl-(2-{2-[4-(cyclopropylcarbonyl)piperazin-1-yl]-2-oxoethyl}-2H-indazol-5-yl)carbamat (Intermediat 5-10) mit 4.1 ml (53.8 mmol) Trifluoressigsäure in 25 ml Dichlormethan zu 1.09 g (62 % d.Th.) der Titelverbindung als Rohprodukt umgesetzt.
UPLC-MS (methode A1): Rt = 0.47 min
MS (ESIpos): m/z = 328 (M+H)⁺.

### Intermediat 6-11

### 2-(5-Amino-2H-indazol-2-yl)-1-(4-benzoylpiperazin-1 -yl)ethanon

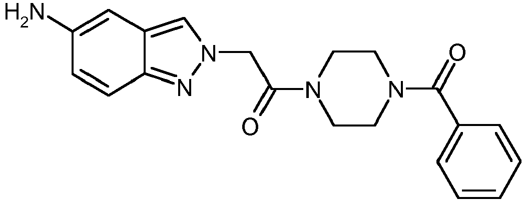

Analog zu Intermediat 6-4 wurden 4.20 g (9.06 mmol) tert-Butyl-{2-[2-(4-benzoylpiperazin-1-yl)-2-oxoethyl]-2H-indazol-5-yl}carbamat (Intermediat 5-11) wurden mit 6.98 ml (90.6 mmol) Trifluoressigsäure zu 3.27 g (99 % d.Th.)der Titelverbindung umgesetzt.
UPLC-MS (Methode A1): Rₜ = 0.57 min
MS (ESIpos): m/z = 364 (M+H)⁺
¹R-NMR (300MHz, DMSO-d₆): δ = 3.36 - 3.80 (m, 8H), 4.78 (s, 2H), 5.33 (br. s., 2H), 6.55 (d, 1H), 6.74 (dd, 1H), 7.30 (d, 1H), 7.38 - 7.53 (m, 5H), 7.81 (s, 1H).

### Intermediat 6-12

### 2-(5-Amino-2H-indazol-2-yl)-1-(4-methylpiperazin-1-yl)ethanon

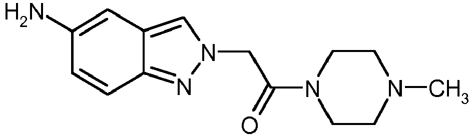

Analog zu Intermediat 6-4 wurden 916 mg (2.45 mmol) tert-Butyl-{2-[2-(4-methylpiperazin-1-yl)-2-oxoethyl]-2H-indazol-5-yl}carbamat (Intermediat 5-12) mit 1.89 ml (24.5 mmol) Trifluoressigsäure in Dichlormethan für 24 h bei 25°C gerührt. Man engte ein, das Rohprodukt wurde in 10 ml Tetrahydrofuran und 1 ml *N,N*-Dimethylformamid gelöst. Der ausgefallene Feststoff wurde abfiltriert und mit Diethylether gewaschen. Man löste den Feststoff in Methanol und engte zur Trockne ein. Man erhielt 1.2 g der Titelverbindung als Rohprodukt.

### Intermediat 6-13

### 2-(5-Amino-2H-indazol-2-yl)-1-[4-(2,2,2-trifluorethyl)piperazin-1-yl]ethanon

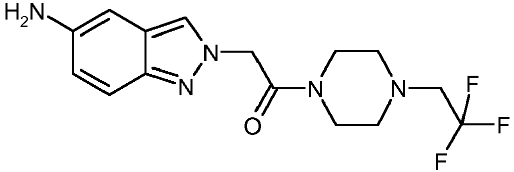

Analog zu Intermediat 6-4 wurden 634 mg (1.43 mmol) tert-Butyl-(2-{2-oxo-2-[4-(2,2,2-trifluorethyl)piperazin-1-yl]ethyl}-2H-indazol-5-yl)carbamat (Intermediat 5-13) in 5 ml Dichlormethan mit 1.1 ml (14.4 mmol) Trifluoressigsäure versetzt und man rührte für 24 h bei 25°C. Man engte ein, setzte je zwei Mal Toluol zu und evaporierte. Man erhielt 1.0 g eines Rohproduktes.
UPLC-MS (Methode A1): Rₜ = 0.59 min
MS (ESIpos): m/z = 342 (M+H)⁺.

### Intermediat 6-14

### 4-[(5-Amino-2H-indazol-2-yl)acetyl]-1-ethylpiperazin-2-on

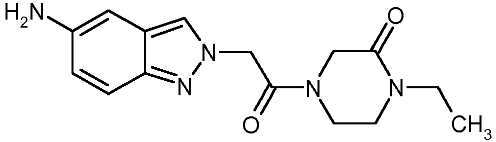

Analog zu Intermediat 6-4 wurden 1.92 g (3.59 mmol, 75%) tert-Butyl-{2-[2-(4-ethyl-3-oxopiperazin-1-yl)-2-oxoethyl]-2H-indazol-5-yl}carbamat (Intermediat 5-14) in 15 ml Dichlormethan mit 2.8 ml (35.9 mmol) Trifluoressigsäure versetzt und man rührte bei 25°C für 24 h. Man versetzte mit gesättigter Natriumhydrogencarbonatlösung, filtrierte, trennte die organische Phase und extrahierte mit Dichlormethan. Dabei fiel in der wässrigen Phase ein Niederschlag aus, der abgesaugt wurde und mit Wasser und Diethylether gewaschen wurde. Nach Trocknen erhielt man 636 mg (44 % d.Th.) der Titelverbindung als Rohprodukt.

### Intermediat 6-15

### 2-(5-Amino-3-methyl-2H-indazol-2-yl)-1-(4-benzoylpiperazin-1-yl)ethanon

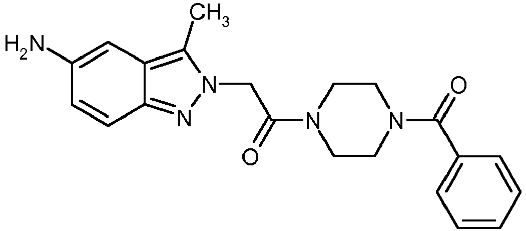

130 mg (0.27 mmol) tert-Butyl-{2-[2-(4-benzoylpiperazin-1-yl)-2-oxoethyl]-3-methyl-2H-indazol-5-yl}carbamat in 3 ml Dichlormethan wurden mit 0.21 ml (2.72 mmol) Trifluoressigsäure versetzt, man rührte bei 25°C für 24 h und engte ein. Man erhielt 204 mg der Titelverbindung als Rohprodukt.
UPLC-MS (Methode A1): Rₜ = 0.61 min
MS (ESIpos): m/z = 378 (M+H)⁺.

### Intermediat 6-16

### 2-(5-Amino-6-methoxy-2H-indazol-2-yl)-1-(morpholin-4-yl)ethanon

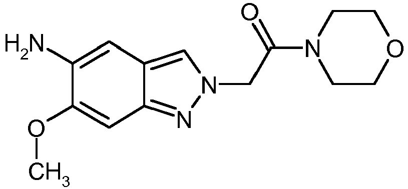

Analog zu Intermediat 6-4 wurden 1.16 g (2.97 mmol) tert-Butyl-{6-methoxy-2-[2-(morpholin-4-yl)-2-oxoethyl]-2H-indazol-5-yl}carbamat (Intermediat 5-16) mit 2.29 ml (29.7 mmol) Trifluoressigsäure in 20 ml Dichlormethan für 24 h bei 25°C gerührt. Es wurden weitere 1.15 ml (14.9 mmol) Trifluoressigsäure zugegeben und für weitere 24 h bei 25°C gerührt. Das Reaktionsgemisch wurde drei Mal mit Toluol eingeengt. Der Rückstand würde in Tetrahydrofuran gelöst und mit Diethylether versetzt. Der entstandene Niederschlag wurde abgesaugt, mit Diethylether gewaschen und getrocknet. Man erhielt 759 mg (88 % d. Th.) der Titelverbindung.
UPLC-MS (Methode A2): Rₜ = 0.60 min
MS (ESIpos): m/z = 291 (M+H)⁺
¹H NMR (400 MHz, DMSO-d6): δ = 3.45 (br. s., 2 H), 3.51 - 3.71 (m, 6 H), 3.93 (s, 3 H), 5.40 (s, 2 H), 7.10 (s, 1 H), 7.52 (s, 1 H), 8.21 (s, 1 H).

### Intermediat 6-17

### 2-(5-Amino-6-methoxy-2H-indazol-2-yl)-1-(4-methylpiperazin-1-yl)ethanon

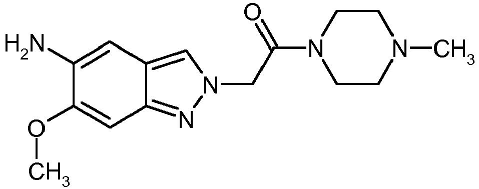

Analog zu Intermediat 6-16 wurden 1.25 g (3.10 mmol) tert-Butyl-{6-methoxy-2-[2-(4-methylpiperazin-1-yl)-2-oxoethyl]-2H-indazol-5-yl}carbamat (Intermediat 5-17) mit 2.39 ml (31.0 mmol) Trifluoressigsäure in 25 ml Dichlormethan für 5 h bei 25°C gerührt. Nach Aufarbeitung erhielt man 534 mg (57 % d. Th.) der Titelverbindung.
UPLC-MS (Methode A2): Rₜ = 0.61 min
MS (ESIpos): m/z = 304 (M+H)⁺
¹H NMR (400 MHz, DMSO-d6): δ = 2.19 (s, 3 H), 2.24 - 2.30 (m, 2 H), 2.30 - 2.37 (m, 2 H), 3.41 - 3.48 (m, 2 H), 3.49 - 3.54 (m, 2 H), 3.82 (s, 3 H), 4.61 (br. s., 2 H), 5.23 (s, 2 H), 6.63 (s, 1 H), 6.79 (s, 1 H), 7.76 (s, 1 H).

### Intermediat 6-18

### 2-(5-Amino-6-methoxy-2H-indazol-2-yl)-N-(cyclopropylmethyl)-N-methylacetamid

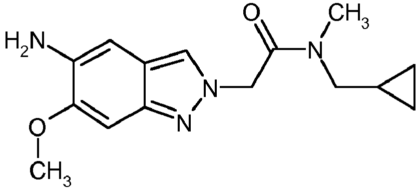

Analog zu Intermediat 6-4 wurden 353 mg (0.86 mmol, 95%) tert-Butyl-(2-{2-[(cyclopropylmethyl)(methyl)amino]-2-oxoethyl}-6-methoxy-2H-indazol-5-yl)carbamat (Intermediat 5-18) in 10 ml Dichlormethan vorgelegt. Man addierte 665 µl (8.63 mmol) Trifluoressigsäure, rührte für 24 h bei 25°C, addierte erneute 200 µl Trifluoressigsäure und rührte 3 h. Man engte ein, addierte zwei Mal Toluol und engte jeweils ein. Man erhielt 750 mg eines Rohproduktes.
UPLC-MS (Methode A1): Rₜ = 0.61 min
MS (ESIpos): m/z = 289 (M+H)⁺.

### Intermediat 6-19

### 2-(5-Amino-2H-indazol-2-yl)-1-(4-benzoylpiperazin-1 -yl)propan-1 -on

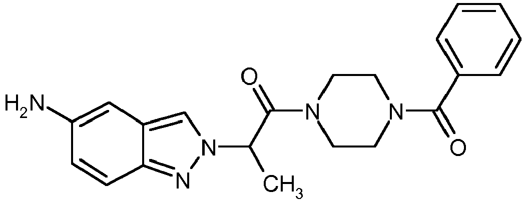

Analog zu Intermediat 6-4 wurden 3.70 g (7.75 mmol) tert-Butyl-{2-[1-(4-benzoylpiperazin-1-yl)-1-oxopropan-2-yl]-2H-indazol-5-yl}carbamat (Intermediat 5-19) (Rohprodukt) in 40 ml Dichlormethan vorgelegt. Man addierte 6.0 ml (77.4 mmol) Trifluoressigsäure und rührte bei 25°C für 24 h. Man goß vorsichtig auf gesättigte Natriumhydrogencarbonatlösung, extrahierte mit Dichlormethan und engte ein. Das Rohprodukt wurde mit Diethylether ausgerührt. Man erhielt 2.4 g (75 % d.Th.) der Titelverbindung als hellbraunen Feststoff.
¹H-NMR (300MHz, DMSO-d6): δ = 1.59 (d, 3H), 2.9 - 3.7 (breite Signale, überlagert), 4.78 (s, 2H), 5.74 (br. s, 1H), 6.52 (s, 1H), 6.71 (dd, 1H), 7.25 - 7.47 (m), 7.91 (s, 1H).

### Intermediat 6-20

### 2-[5-Amino-6-(benzyloxy)-2H-indazol-2-yl]-1-(morpholin-4-yl)ethanon

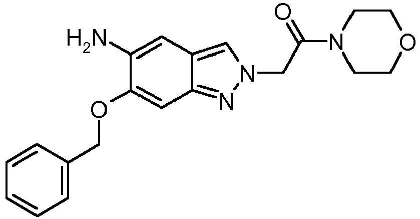

Analog zu Intermediat 6-4 wurden 3.65 g (7.82 mmol) tert-Butyl-{6-(benzyloxy)-2-[2-(morpholin-4-yl)-2-oxoethyl]-2H-indazol-5-yl}carbamat (Intermediat 5-20) in 50 ml Dichlormethan vorgelegt. Man addierte 6.0 ml (78.2 mmol) Trifluoressigsäure und rührte bei 25°C für 24 h. Man goß vorsichtig auf gesättigte Natriumhydrogencarbonatlösung, extrahierte mit Dichlormethan, wusch die vereinigten organischen Phasen mit gesättigter Natriumchloridlösung und engte ein. Man erhielt 2.72 g (95 % d.Th.) der Titelverbindung.
UPLC-MS (Methode A1): Rₜ = 0.85 min
MS (ESIpos): m/z = 312 (M+H)⁺
¹H-NMR (400 MHz, CHLOROFORM-d): δ = 3.59 (s, 4 H), 3.65 (d, 4 H), 5.15 (s, 4 H), 6.78 (s, 1 H), 6.98 (s, 1 H), 7.34 - 7.44 (m, 3 H), 7.46 - 7.50 (m, 2 H), 7.71 - 7.74 (m, 1 H).

### Intermediat 6-21

### 2-(5-Amino-6-chlor-2H-indazol-2-yl)-1-(4-methylpiperazin-1-yl)ethanon

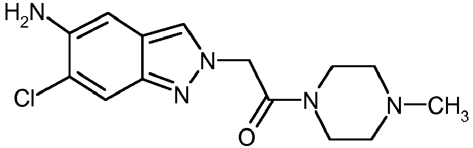

Zu 299 mg Benzyl-{6-chlor-2-[2-(4-methylpiperazin-1-yl)-2-oxoethyl]-2H-indazol-5-yl}carbamat (Intermediat 5-21) wurden 5.0 ml eiskalte Trifluoressigsäure gegeben und man rührte bei Raumtemperatur 3 Tage. Mann goss auf gesättigte wässrige Natriumhydrogencarbonatlösung, extrahierte mit Ethylacetat, wusch mit Kochsalzlösung, filtrierte über einen wasserabweisenden Filter und engte ein. Nach Reinigung durch präparative HPLC (Methode P2) erhielt man einen Feststoff, der mit Diethylether ausgerührt wurde. Nach Trocknen erhielt man 101 mg der Titelverbindung.
¹H-NMR (300MHz, DMSO-d₆): δ [ppm]= 2.18 (s, 3H), 2.22 - 2.39 (m, 4H), 3.38 - 3.56 (m), 4.96 (s, 2H), 5.33 (s, 2H), 6.88 (s, 1H), 7.57 (s, 1H), 7.94 (d, 1H).

### Intermediat 6-22

### 2-(5-Amino-6-chlor-2H-indazol-2-yl)-1-(morpholin-4-yl)ethanon

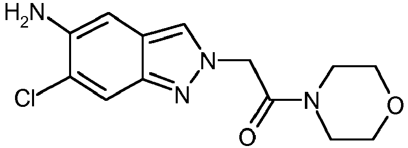

Analog zur Herstellung von Intermediat 6-21 wurden 254 mg Benzyl-{6-chlor-2-[2-(morpholin-4-yl)-2-oxoethyl]-2H-indazol-5-yl}carbamat (Intermediat 5-22) mit 3 ml Trifluoressigsäure 6 Tage bei Raumtemperatur gerührt. Nach analoger wässriger Aufarbeitung erhielt man 137 mg der Titelverbindung als Rohprodukt
UPLC-MS (Methode A1): Rt = 0.60 min (UV-Detector: TIC). Gefundene Masse 294.00.

### Intermediat 7-1

### Ethyl (5-amino-6-fluoro-2H-indazol-2-yl)acetat

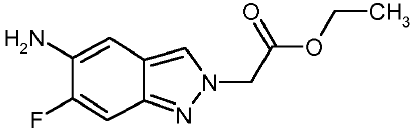

Analog zu Intermediat 6-4 wurden 1.1 g (3.3 mmol) Ethyl {5-[(tert-butoxycarbonyl)amino]-6-fluoro-2H-indazol-2-yl}acetat (Intermediat 3-5) mit 1.92 ml (24.9 mmol)Trifluoressigsäure in 11 ml Dichlormethan umgesetzt. Nach Aufarbeitung erhielt man 790 mg (100 % d. Th.) der Titelverbindung.
UPLC-MS (Methode A1): Rₜ = 0.67 min
MS (ESIpos): m/z = 238 (M+H)⁺
1H-NMR (300 MHz, DMSO-d6): δ = 1.21 (t, 3H), 4.16 (q, 2H), 4.93 (s, 2H), 5.24 (s, 2H), 6.81 (d, 1H), 7.21 (d, 1H), 8.80 (s, 1H).

### Intermediat 7-2

### Ethyl [5-amino-6-(benzyloxy)-2H-indazol-2-yl]acetat

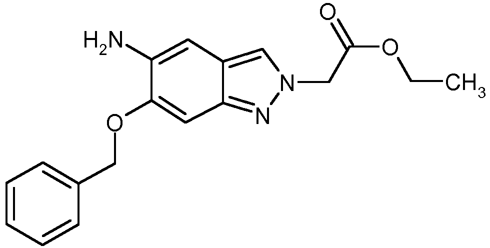

Analog zu Intermediat 6-4 wurden 2.37 g (5.56 mmol) Ethyl {6-(benzyloxy)-5-[(tert-butoxycarbonyl)amino]-2H-indazol-2-yl}acetat (Intermediat 3-4) mit 3.24 ml (41.8 mmol) Trifluoressigsäure in 25 ml Dichlormethan umgesetzt. Nach Aufarbeitung erhielt man 1.79 g (99 % d. Th.) der Titelverbindung.
UPLC-MS (Methode A1): Rₜ = 0.91 min
MS (ESIpos): m/z = 326 (M+H)⁺
¹H NMR (400 MHz, CHLOROFORM-d): δ = 1.29 (t, 3 H), 4.25 (q, 2 H), 5.07 (s, 2 H), 5.15 (s, 2 H), 6.81 (s, 1 H), 7.01 (s, 1 H), 7.31 - 7.45 (m, 3 H), 7.45 - 7.52 (m, 2 H), 7.67 (s, 1 H).

### Intermediat 7-3

### Ethyl-(5-amino-2H-indazol-2-yl)acetat

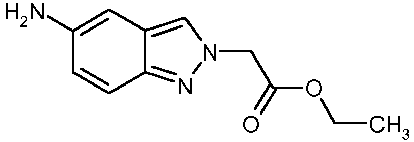

Analog zu Intermediat 6-4 wurden 5.00 g (15.7 mmol) tert-Butyl-{5-[(tert-butoxycarbonyl)amino]-2H-indazol-2-yl}acetat (Intermediat 3-7) in 75 ml Dichlormethan mit 9.0 ml (117.4 mmol) Trifluoressigsäure versetzt und man rührte für 24 h bei 25°C. Man goß auf gesättigte Natriumhydrogencarbonatlösung, trennte die organische Phase ab und extrahierte dreimal mit Dichlormethan. Die vereinigten organischen Phasen wurden mit Kochsalzlösung gewaschen, getrocknet und eingeengt. Man erhielt 3.4 g der Titelverbindung als braunen Feststoff.
UPLC-MS (METHODE A1): Rt = 0.47 min
MS (ESIpos): m/z = 220 (M+H)⁺
¹H-NMR (400MHz, DMSO-d₆): δ = 1.18 (t, 3H), 2.49 (br. s., 1H), 4.12 (q, 2H), 4.80 (s, 2H), 5.20 (s, 2H), 6.52 (dd, 1H), 6.73 (dd, 1H), 7.26 - 7.32 (m, 1H), 7.87 (d, 1H).

### Intermediat 7-4

### Ethyl-3-(5-amino-2H-indazol-2-yl)propanoat

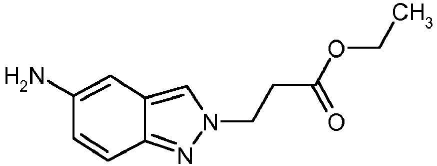

Analog zu Intermediat 7-1 wurden 640 mg (1.92 mmol) Ethyl-3-{5-[(tert-butoxycarbonyl)amino]-2H-indazol-2-yl}propanoat (Intermediat 3-9), mit 1.1 ml (14.4 mmol) Trifluoressigsäure umgesetzt. Man erhielt 391 mg (87 % d.Th.) der Titelverbindung.
UPLC-MS (Methode A1): Rt = 0.50 min
MS (ESIpos): m/z = 234 (M+H)⁺.

### Intermediat 7-5

### Ethyl (5-amino-6-isopropoxy-2H-indazol-2-yl)acetat

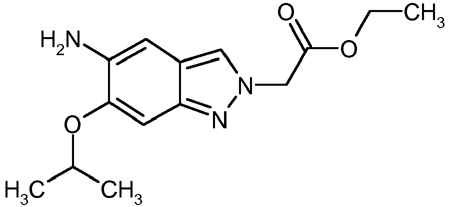

Analog zu Intermediat 7-1 wurden 1.8 g (4.84 mmol) Ethyl {5-[(tert-butoxycarbonyl)amino]-6-isopropoxy-2H-indazol-2-yl}acetat (Intermediat 3-10) mit 2.8 ml (36.3 mmol) Trifluoressigsäure umgesetzt. Man erhielt 1.3 g (100 % d.Th.) der Titelverbindung.
UPLC-MS (Methode A1): Rₜ = 0.69 min
MS (ESIpos): m/z = 278 (M+H)⁺
¹H-NMR (300 MHz, DMSO-d6): δ = 1.21 (t, 3H), 1.32 (d, 6H), 4.15 (q, 2H), 4.59 (s, 1H), 4.60 - 4.69 (m, 1H), 5.16 (s, 2H), 6.64 (s, 1H), 6.81 (d, 1H), 7.83 (s, 1H).

### Intermediat 7-6

### Benzyl-(5-amino-6-methoxy-2H-indazol-2-yl)acetat

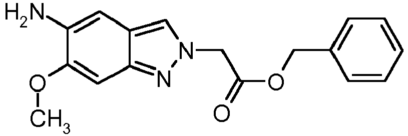

Analog zu Intermediat 7-1 wurden 25.7 g (60.1 mmol) Benzyl-{5-[(tert-butoxycarbonyl)amino]-6-methoxy-2H-indazol-2-yl}acetat (Intermediat 3-2) mit 23.1 ml (300.3 mmol) Trifluoressigsäure umgesetzt. Man erhielt 20.5 g (98 % d.Th.) der Titelverbindung.
UPLC-MS (Methode A1): Rₜ = 0.85 min
MS (ESIpos): m/z = 312 (M+H)⁺

### Intermediat 8-1

### Ethyl [6-fluoro-5-({[6-(trifluoromethyl)pyridin-2-yl]carbonyl}amino)-2H-indazol-2-yl]acetat

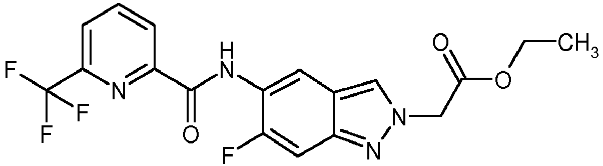

221 mg (1.16 mmol) 6-(Trifluoromethyl)pyridin-2-carbonsäure wurde mit 177 mg (1.16 mmol) 1-Hydroxy-1H-benzotriazol-Hydrat und 444 mg (2.32 mmol) 1-(3-Dimethylaminopropyl)-3-ethylcarbodiimidhydrochlorid in 5.5 ml Dimethylformamid 30 min bei 25°C gerührt. Es wurden 250 mg (1.05 mmol) Ethyl (5-amino-6-fluoro-2H-indazol-2-yl)acetat (Intermediat 7-1) zugegeben und 30 min bei 25°C gerührt. Es wurde auf 150 ml Wasser gegossen, abgesaugt, mit Wasser gewaschen und getrocknet. Man erhielt 366 mg (84 % d. Th.) der Titelverbindung.
UPLC-MS (Methode A1): Rₜ = 1.23 min
MS (ESIpos): m/z = 411 (M+H)⁺
¹H-NMR (300 MHz, DMSO-d6): δ = 1.22 (t, 3H), 4.18 (q, 2H), 5.41 (s, 2H), 7.55 (d, 1H), 8.21 (m, 1H), 8.36 - 8.51 (m, 4H), 10.27 (m, 1H).

### Intermediat 8-2

### Ethyl (6-fluoro-5- {[(6-methylpyridin-2-yl)carbonyl] amino}-2H-indazol-2-yl)acetat

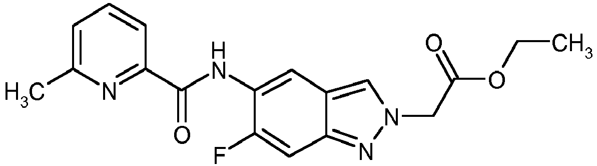

Analog zu Intermediat 8-1 wurden 159 mg (1.16 mmol) 6-Methylpyridin-2-carbonsäure mit 250 mg (1.05 mmol) Ethyl (5-amino-6-fluoro-2H-indazol-2-yl)acetat (Intermediat 7-1) umgesetzt. Nach Aufarbeitung erhielt man 316 mg (84 % d. Th.) der Titelverbindung.
UPLC-MS (Methode A1): Rₜ = 1.17 min
MS (ESIpos): m/z = 357 (M+H)⁺.

### Intermediat 8-3

### Ethyl [6-fluoro-5-({[6-(1-methyl-1H-pyrazol-4-yl)pyridin-2-yl]carbonyl}amino)-2H-indazol-2-yl]acetat

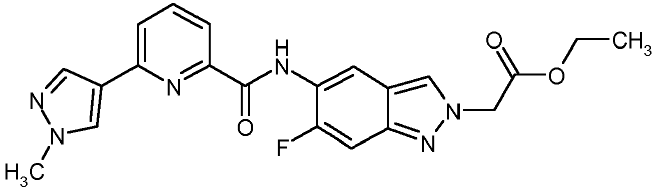

Analog zu Intermediat 8-1 wurden 235 mg (1.16 mmol) 6-(1-Methyl-1H-pyrazol-4-yl)pyridin-2-carbonsäure mit 250 mg (1.05 mmol) Ethyl (5-amino-6-fluoro-2H-indazol-2-yl)acetat (Intermediat 7-1) umgesetzt. Nach Aufarbeitung erhielt man 364 mg (82 % d. Th.) der Titelverbindung.
UPLC-MS (Methode A1): Rₜ = 1.05 min
MS (ESIpos): m/z = 423 (M+H)⁺.

### Intermediat 8-4

### Ethyl-[6-fluor-5-({[5-fluor-6-(1-methyl-1H-pyrazol-4-yl)pyridin-2-yl]carbonyl}amino)-2H-indazol-2-yl]acetat

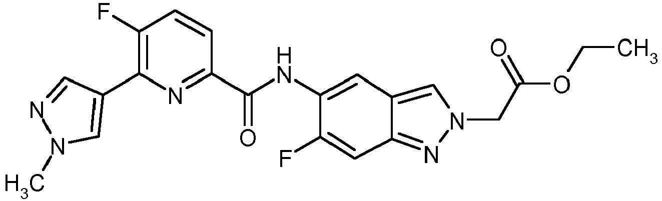

Analog zu Intermediat 8-1 wurden 235 mg (1.0 mmol) 5-Fluor-6-(1-methyl-1H-pyrazol-4-yl)pyridin-2-carbonsäure (Intermediat 19-5) mit 250 mg (1.05 mmol) Ethyl (5-amino-6-fluoro-2H-indazol-2-yl)acetat (Intermediat 7-1) umgesetzt. Nach Aufarbeitung erhielt man 326 mg (76 % d. Th.) der Titelverbindung.
UPLC-MS (Methode A1): Rₜ = 1.13 min
MS (ESIpos): m/z = 442 (M+H)⁺.

### Intermediat 8-5

### Ethyl-[6-fluor-5-({[6-(morpholin-4-yl)pyridin-2-yl]carbonyl}amino)-2H-indazol-2-yl]acetat

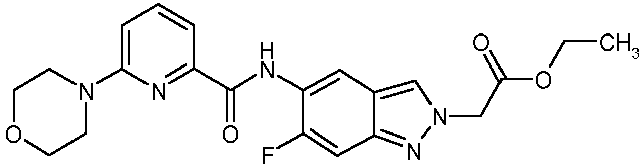

Analog zu Intermediat 8-1 wurden 222 mg (0.97 mmol) 6-(Morpholin-4-yl)pyridin-2-carbonsäure mit 230 mg (0.97 mmol) Ethyl (5-amino-6-fluoro-2H-indazol-2-yl)acetat (Intermediat 7-1) umgesetzt. Nach Aufarbeitung erhielt man 414 mg (100 % d. Th.) der Titelverbindung.
UPLC-MS (Methode A1): Rₜ = 1.12 min
MS (ESIpos): m/z = 428 (M+H)⁺.

### Intermediat 8-6

### Ethyl [6-(benzyloxy)-5-({[6-(trifluoromethyl)pyridin-2-yl]carbonyl}amino)-2H-indazol-2-yl]acetat

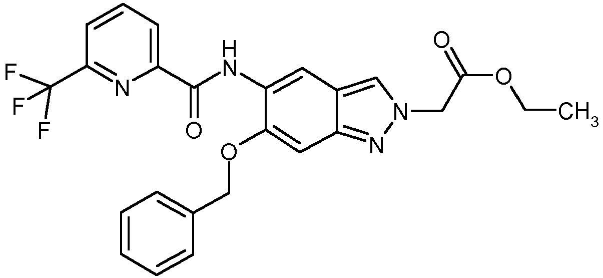

1.79 g (5.5 mmol) Ethyl [5-amino-6-(benzyloxy)-2H-indazol-2-yl]acetat (Intermediat 7-2) wurde mit 1.26 g (6.6 mmol) 6-(Trifluoromethyl)pyridin-2-carbonsäure, 842 mg (5.5 mmol) 1-Hydroxy-1H-benzotriazol-Hydrat und 2.11 g (11.0 mmol) 1-(3-Dimethylaminopropyl)-3-ethylcarbodiimidhydrochlorid und 2.3 ml (16,5 mmol) Triethylamin in 75 ml Tetrahydrofuran bei 25°C für 24 h. Die Reaktionsmischung wurde eingeengt und der Rückstand mit Wasser versetzt. Der erhaltene Feststoff wurde abgesaugt und zwei Mal mit Wasser und zwei Mal mit Diethylether gewaschen. Der gelbe Feststoff wurde im Vakuum getrocknet. Man erhielt 2.44 g (89 % d. Th.) Produkt.
UPLC-MS (Methode A1): Rₜ = 1.42 min
MS (ESIpos): m/z = 499 (M+H)⁺
¹H NMR (400 MHz, DMSO-d6): δ = 1.23 (t, 3 H), 4.18 (q, 2 H), 5.31 (s, 2 H), 5.33 (s, 2 H), 7.32 (s, 1 H), 7.34 - 7.47 (m, 3 H), 7.54 - 7.61 (m, 2 H), 8.18 (d, 1 H), 8.32 - 8.42 (m, 2 H), 8.43 - 8.52 (m, 1 H), 8.81 (s, 1 H), 10.47 (s, 1 H).

### Intermediat 8-7

### Ethyl [6-hydroxy-5-({[6-(trifluoromethyl)pyridin-2-yl]carbonyl}amino)-2H-indazol-2-yl]acetat

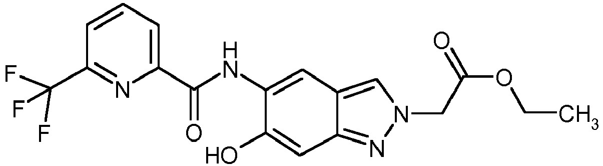

1.0 g (2.01 mmol) Ethyl [6-(benzyloxy)-5-({[6-(trifluoromethyl)pyridin-2-yl]carbonyl}amino)-2H-indazol-2-yl]acetat (Intermediat 8-6) wurden in 40 ml Ethanol gelöst und der Kolben evakuiert und anschließend mit Stickstoff gespült (Vorgang noch zwei Mal wiederholt). 213 mg (0.2 mmol) Palladium auf Kohle wurde zugegeben und der Kolben wurde evakuiert und mit Wasserstoff gespült. Das Reaktionsgemisch wurde für 6 h bei 25°C unter Normaldruck Wasserstoff hydriert. Anschließend wurde die Reaktionsmischung über einen PTFE-Filter mit Celite filtriert und eingeengt. Man erhielt 783 mg (96 % d. Th.) Produkt.
UPLC-MS (Methode A1): Rt = 1.08 min
MS (ESIpos): m/z = 409 (M+H)⁺
¹H NMR (400 MHz, DMSO-d6): δ = 1.22 (t, 3 H), 4.17 (q, , 2 H), 5.28 (s, 2 H) 6.92 (s, 1 H) 8.21 (d, 1 H), 8.27 (s, 1 H), 8.40 (t, 1 H), 8.47 (d, 1 H), 8.70 (s, 1 H), 10.55 (s, 1 H), 10.72 (s, 1 H).

### Intermediat 8-8

### Ethyl [6-isobutoxy-5-({[6-(trifluoromethyl)pyridin-2-yl]carbonyl}amino)-2H-indazol-2-yl]acetat

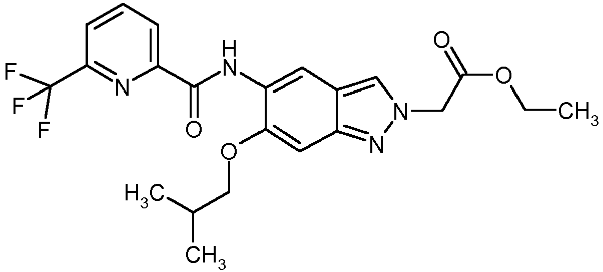

200 mg (0.49 mmol) Ethyl [6-hydroxy-5-({[6-(trifluoromethyl)pyridin-2-yl]carbonyl}amino)-2H-indazol-2-yl]acetat (Intermediat 8-7) wurde in 1,5 ml *N,N*-Dimethylformamid gelöst und unter Rühren mit 203 mg (1,47 mmol) Kaliumcarbonat versetzt. Die Suspension wurde 10 Minuten bei 25°C gerührt und anschließend wurden 80 µL (0.73 mmol) 1-Brom-2-methylpropan hinzugegeben. Die Reaktionsmischung wurde für 1 h bei 100°C in der Mikrowelle gerührt. Anschließend wurde die Reaktionsmischung mit Wasser verdünnt und mit Ethylacetat versetzt. Es entstand ein Feststoff, der abgesaugt und zewi Mal mit Wasser und zwei Mal mit Diethylether gewaschen wurde. Der grünliche Feststoff wurde für 3 h im Trockenschrank getrocknet. Man erhielt 200 mg (88 % d. Th.) Produkt.
UPLC-MS (Methode A1): Rₜ = 1.45 min
MS (ESIpos): m/z = 465 (M+H)⁺
¹H NMR (300 MHz, DMSO-d6): δ = 1.12 (d, 6 H), 1.22 (t, 3 H), 2.19 (dt, 1 H), 3.96 (d, 2 H), 4.17 (q, 2 H), 5.32 (s, 2 H), 7.09 (s, 1 H), 8.22 (d, 1 H), 8.32 (s, 1 H), 8.37 - 8.45 (m, 1 H), 8.46 - 8.51 (m, 1 H), 8.78 (s, 1 H), 10.58 (s, 1 H).

### Intermediat 8-9

### Ethyl [6-(cyclopropylmethoxy)-5-({[6-(trifluoromethyl)pyridin-2-yl]carbonyl}amino)-2H-indazol-2-yl] acetat

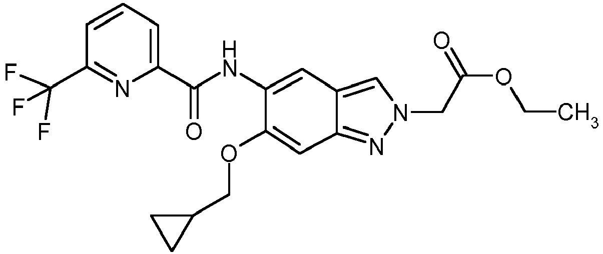

Analog zu Intermediat 8-5 wurden 200 mg (0.49 mmol) Ethyl [6-hydroxy-5-({[6-(trifluoromethyl)pyridin-2-yl]carbonyl}amino)-2H-indazol-2-yl]acetat (Intermediat 8-7) mit 71 µL (0.73 mmol) (Brommethyl)cyclopropan umgesetzt. Nach Aufarbeitung erhielt man 223 mg (99 % d. Th.) der Titelverbindung.
UPLC-MS (Methode A1): Rₜ = 1.38 min
MS (ESIpos): m/z = 463 (M+H)⁺
¹H NMR (300 MHz, CHLOROFORM-d): δ = 0.38 - 0.50 (m, 2 H), 0.69 - 0.84 (m, 2 H), 1.30 (t, 3 H), 1.45 (br. s., 1 H), 3.98 (d, 2 H), 4.27 (q, 2 H), 5.15 (s, 2 H), 6.98 (s, 1 H), 7.87 (d, 1 H), 7.93 (s, 1 H), 8.13 (t, 1 H), 8.51 (d, 1 H), 8.88 (s, 1 H), 10.91 (s, 1 H).

### Intermediat 8-10

### Ethyl [6-(pyridin-2-ylmethoxy)-5-({[6-(trifluoromethyl)pyridin-2-yl]carbonyl}amino)-2H-indazol-2-yl]acetat

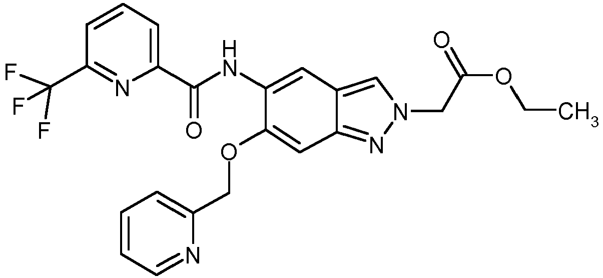

200 mg (0.49 mmol) Ethyl [6-hydroxy-5-({[6-(trifluoromethyl)pyridin-2-yl]carbonyl}amino)-2H-indazol-2-yl]acetat (Intermediat 8-7) wurde in 6,6 ml *N,N*-Dimethylformamid gelöst und unter Rühren mit 270 mg (1,96 mmol) Kaliumcarbonat versetzt. Die Suspension wurde 10 Minuten bei 25°C gerührt und anschließend wurden 185 mg (0.73 mmol) 2-(Brommethyl)pyridinhydrobromid hinzugegeben. Die Reaktionsmischung wurde für 1 h bei 100°C in der Mikrowelle gerührt. Anschließend wurde die Reaktionsmischung mit Wasser verdünnt und mit Ethylacetat versetzt. Es entstand ein Feststoff, der abgesaugt und zwei Mal mit Wasser und zwei Mal mit Diethylether gewaschen wurde. Der grünliche Feststoff wurde für 3 h im Trockenschrank getrocknet. Man erhielt 160 mg (65 % d. Th.) der Titelverbindung.
UPLC-MS (Methode A1): Rₜ = 1.24 min
MS (ESIpos): m/z = 500 (M+H)⁺
1H NMR (400 MHz, DMSO-d6): δ = 1.23 (t, 3 H), 4.18 (q, 2 H), 5.34 (s, 2 H), 5.36 (s, 2 H), 7.70 (d, 1 H), 7.82 - 7.91 (m, 1 H), 8.15 - 8.21 (m, 1 H), 8.36 (s, 1 H), 8.37 - 8.43 (m, 1 H), 8.45 - 8.50 (m, 1 H), 8.62 (d, 1 H), 8.82 (s, 1 H), 10.50 (s, 1 H).

### Intermediat 8-11

### Ethyl-[5-({[6-(1-methyl-1H-pyrazol-4-yl)pyridin-2-yl]carbonyl}amino)-2H-indazol-2-yl]acetat

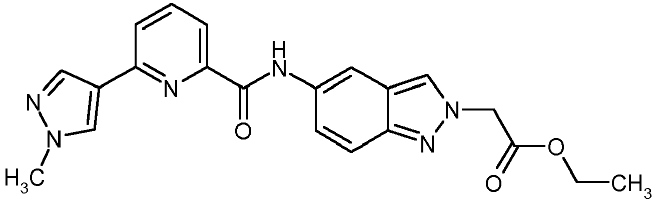

Analog zu Intermediat 8-1 wurden 1.00 g 6-(1-Methyl-1H-pyrazol-4-yl)pyridin-2-carbonsäure (Intermediat 19-2) (Rohprodukt) und 961 mg (4.39 mmol) Ethyl-(5-amino-2H-indazol-2-yl)acetat (Intermediat 7-3) in 10 ml Tetrahydrofuran für 24 h bei 25°C gerührt. Man versetzte mit Wasser, engte ein und saugte den ausfallenden Feststoff ab, wusch mit Wasser und Diethylether und trocknete im Vakuum. Man erhielt 1.45 g (80 % d.Th.) der Titelverbindung.
UPLC-MS (Methode A1): Rt = 1.01 min
MS (ESIpos): m/z = 405 (M+H)⁺.

### Intermediat 8-12

### Ethyl-[6-ethoxy-5-({[6-(trifluormethyl)pyridin-2-yl]carbonyl} amino)-2H-indazol-2-yl]acetat

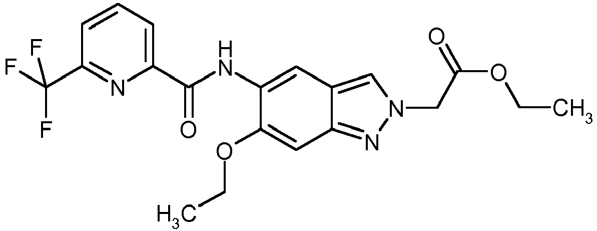

Analog zu Intermediat 3-1 wurden 1.30 g (3.71 mmol) N-(6-Ethoxy-1H-indazol-5-yl)-6-(trifluormethyl)pyridin-2-carboxamid (Intermediat 14-3) mit 826 µl (7.42 mmol) Ethyl-bromacetat und 1.54 ml (7.42 mmol) *N,N*-Dicyclohexylmethylamin in 20 ml Tetrahydrofuran bei 65°C für 18h gerührt. Es wurden nochmals Man addiert 413 µl (3.71 mmol) Ethyl-bromacetat und 770 µl (3.71 mmol) *N,N*-Dicyclohexylmethylamin hinzugegeben und für weitere 6 h bei 65°C gerührt. Nach Aufarbeitung erhielt man 143 mg der Titelverbindung als Rohprodukt.

Weitere 637 mg der Titelverbindung wurden durch Zugabe von Wasser zum Filtrat der Reaktion, Extraktion mit Ethylacetat, Waschen der organischen Phase mit 1M Salzsäurelösung, gesättigter Natriumhydrogencarbonatlösung, gesättigter Natriumchloridlösung, Trocknen, Einengen und Ausrühren des Rückstandes mit Ethylacetat erhalten.
UPLC-MS (Methode A1): Rₜ = 1.31 min
MS (ESIpos): m/z = 437 (M+H)⁺
¹H-NMR (400MHz, DMSO-d6): δ = 1.23 (t, 3H), 1.51 (t, 3H), 4.14 - 4.27 (m, 4H), 5.31 (s, 2H), 7.10 (s, 1H), 8.18 - 8.23 (m, 1H), 8.31 (s, 1H), 8.37 - 8.44 (m, 1H), 8.45 - 8.49 (m, 1H), 8.73 (s, 1H), 10.74 (s, 1H).

### Intermediat 8-13

### Ethyl-3-[5-({ [6-(trifluormethyl)pyridin-2-yl]carbonyl}amino)-2H-indazol-2-yl]propanoat

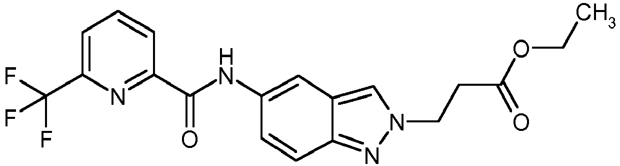

Analog zu Intermediat 8-1 wurden 194 mg (0.83 mmol) Ethyl-3-(5-amino-2H-indazol-2-yl)propanoat (Intermediat 7-4), mit 175 mg (0.91 mmol) 6-(Trifluoromethyl)pyridin-2-carbonsäure umgesetzt. Man erhielt 285 mg (84 % d.Th.) der Titelverbindung.
UPLC-MS (Methode A1): Rₜ = 1.17 min
MS (ESIpos): m/z = 407 (M+H)⁺.

### Intermediat 8-14

### tert-Butyl-[6-chlor-5-({[6-(trifluormethyl)pyridin-2-yl]carbonyl}amino)-2H-indazol-2-yl]acetat

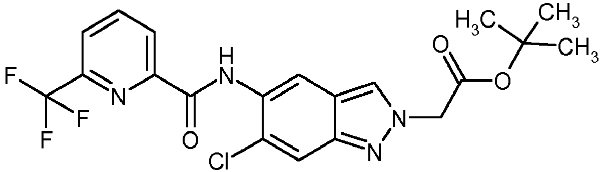

4.48 g (12.2 mmol) N-(6-Chlor-1H-indazol-5-yl)-6-(trifluormethyl)pyridin-2-carboxamid (Intermediat 14-1) wurden in 40 ml Tetrahydrofuran vorgelegt. Man addierte 3.61 ml (24.5 mmol) Bromessigsäure-tert-butylester und 5.19 ml (24.5 mmol) *N,N*-Dicyclohexylmethylamin und ließ bei 70°C 5.5 h rühren. Man addierte erneut 3.61 ml (24.5 mmol) Bromessigsäure-tert-butylester und 5.19 ml (24.5 mmol) *N,N*-Dicyclohexylmethylamin, rührte 18 h bei 65°C, addierte erneut 1.81 ml (12.3 mmol) Bromessigsäure-tert-butylester und 2.60 ml (12.3 mmol) *N,N-*Dicyclohexylmethylamin und ließ weitere 6 h bei 65°C rühren. Man filtrierte, versetzte das Filtrat mit Wasser und extrahierte dreimal mit Ethylacetat, wusch die vereinigten organischen Phasen mit 1M Salzsäure, gesättigter Natriumhydrogencarbonatlösung und gesättigter Natriumchloridlösung und engte ein. Nach Ausrühren des Rohproduktes mit Ethylacetat wurden nach Trocknung 1.45 g (26% der Theorie) der Titelverbindung erhalten.
UPLC-MS (Methode A1): Rₜ = 1.43 min
MS (ESIpos): m/z = 455 (M+H)⁺
1H-NMR (400MHz, DMSO-d6): δ = 1.45 (s, 9H), 5.32 (s, 2H), 7.95 (s, 1H), 8.23 (d, 1H), 8.38 - 8.44 (m, 1H), 8.45 - 8.49 (m, 1H), 8.49 (s, 1H), 8.66 (s, 1H), 10.5 (s, 1H).

### Intermediat 8-15

### tert-Butyl [6-methoxy-5-({[6-(trifluoromethyl)pyridin-2-yl]carbonyl} amino)-2H-indazol-2-yl]acetat

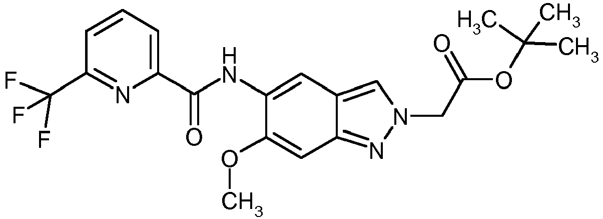

2.00 g (5.95 mmol) N-(6-Methoxy-1H-indazol-5-yl)-6-(trifluoromethyl)pyridine-2-carboxamid (Intermediat 14-2) wurden in 40 ml Tetrahydrofuran gelöst und bei 25°C mit 4.39 ml (29.7 mmol) Bromessigsäure-tert-butylester und 6.37 ml (29.7 mmol) *N,N*-Dicyclohexylmethylamin versetzt. Die Lösung wurde für 3 h bei 70°C gerührt. Es werden weitere 0.87 ml (5.95 mmol) Bromessigsäure-tert-butylester und 1.27 ml (5.95 mmol) *N,N*-Dicyclohexylmethylamin hinzugegeben und für weitere 24 h bei 70°C gerührt. Der Feststoff in der Reaktionsmischung wurde abfiltriert und mit Tetrahydrofuran zwei Mal gewaschen. Das regioisomerenreine Kristallisat wird für 3 h im Vakuumtrockenschrank bei 50°C getrocknet. Man erhielt 1.58 g (59 % d. Th.) Produkt.
UPLC-MS (Methode A1): Rₜ = 1.36 min
MS (ESIpos): m/z = 451 (M+H)⁺
¹H NMR (400 MHz, CHLOROFORM-d): δ = 1.50 (s, 9 H), 4.04 (s, 3 H), 5.04 (s, 2 H), 7.06 (s, 1 H), 7.86 (d, 1 H), 7.92 (s, 1 H), 8.12 (t, 1 H), 8.50 (d, 1 H), 8.84 (s, 1 H), 10.72 (s, 1 H).

### Intermediat 8-16

### tert-Butyl-[5-({[6-(trifluormethyl)pyridin-2-yl]carbonyl}amino)-2H-indazol-2-yl]acetat

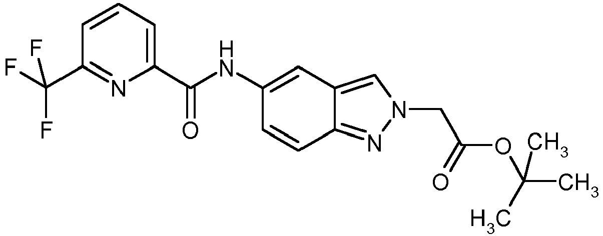

Eine Lösung aus 582 mg (1.90 mmol) N-(1H-Indazol-5-yl)-6-(trifluormethyl)pyridin-2-carboxamid (Intermediat 14-4) und 309 µl (2.09 mmol) Bromessigsäure-tert-butylester in 5 ml N,N-Dimethylformamid wurden mit 525 mg (3.80 mmol) Kaliumcarbonat versetzt und die Mischung wurde bei 80°C für 24 h gerührt. Man versetzte mit Wasser und extrahierte dreimal mit Ethylacetat. Aus der Ethylacetatphase fiel ein Feststoff aus, der abgesaugt wurde und mit Ethylacetat gewaschen wurde. Trocknen im Vakuum gab 72 mg (8% der Theorie) der Titelverbindung. Man engte die Ethylacetatphase ein und reinigte den Rückstand durch präparative HPLC. Dabei erhielt man weitere 151 mg (19% der Theorie) der Titelverbindung.
¹H-NMR (500MHz, DMSO-d6): δ = 1.45 (s, 9H), 5.27 (s, 2H), 7.56 - 7.61 (m, 1H), 7.61 - 7.64 (m, 1H), 8.17 (dd, 1H), 8.30 - 8.39 (m), 8.39 - 8.43 (m,1H), 10.38 (s, 1H).

### Intermediat 8-17

### Ethyl [6-isopropoxy-5-({[6-(trifluoromethyl)pyridin-2-yl]carbonyl}amino)-2H-indazol-2-yl]acetat

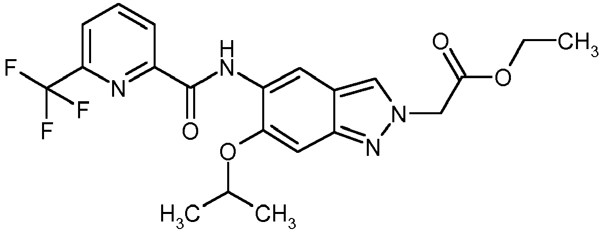

Analog zu Intermediat 8-1 wurden 300 mg (1.08 mmol) Ethyl (5-amino-6-isopropoxy-2H-indazol-2-yl)acetat (Intermediat 7-5) mit 227 mg (1.19 mmol) 6-(Trifluoromethyl)pyridin-2-carbonsäure umgesetzt. Man erhielt 487 mg (100 % d.Th.) der Titelverbindung.
UPLC-MS (Methode A1): Rₜ = 1.34 min
MS (ESIpos): m/z = 451 (M+H)⁺
¹H-NMR (300 MHz, DMSO-d6): δ = 1.23 (t, 3H), 1.41 (d, 6H), 4.18 (q, 2H), 4.79 - 4.92 (m, 1H), 5.32 (s, 2H), 7.18 (s, 1H), 8.22 (d, 1H), 8.33 (s, 1H), 8.37 - 8.50 (m, 2H), 8.75 (s, 1H), 10.75 (s, 1H).

### Intermediat 8-18

### Ethyl (6-isopropoxy-5- {[(6-methylpyridin-2-yl)carbonyl]amino} -2H-indazol-2-yl)acetat

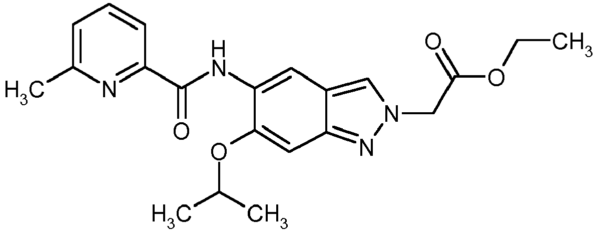

Analog zu Intermediat 8-2 wurden 0.3 g (1 mmol) Ethyl (5-amino-6-isopropoxy-2H-indazol-2-yl)acetat (Intermediat 7-5), mit 137 mg (1.2 mmol) 6-Methylpyridin-2-carbonsäure umgesetzt. Man erhielt 380 mg (89 % d.Th.) der Titelverbindung.
UPLC-MS (Methode A1): Rₜ = 1.28 min
MS (ESIpos): m/z = 397 (M+H)⁺
¹H-NMR (300 MHz, DMSO-d6): δ = 1.22 (t, 3H), 1.45 (d, 6H), 2.62 (s, 3H), 4.18 (q, 2H), 4.78 - 4.89 (m, 1H), 5.31 (s, 2H), 7.15 (s, 1H), 7.52 - 7.60 (m, 1H), 7.95 - 8.01 (m, 2H), 8.29 (s, 1H), 8.72 (s, 1H), 10.99 (s, 1H).

### Intermediat 8-19

### tert-Butyl-[6-(benzyloxy)-5-{[(6-methylpyridin-2-yl)carbonyl]amino}-2H-indazol-2-yl]acetat

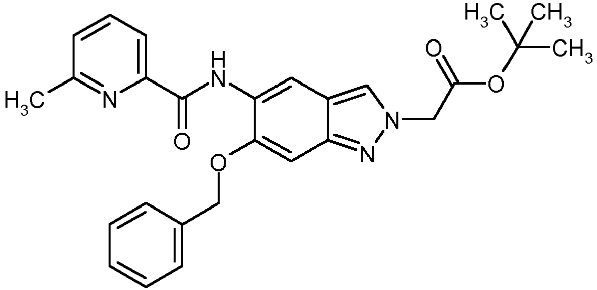

Analog zu Intermediat 8-15 wurden 1.00 g (2.79 mmol) N-[6-(Benzyloxy)-1H-indazol-5-yl]-6-methylpyridin-2-carboxamid (Intermediat 14-5) in 20 ml Tetrahydrofuran gelöst und bei 25°C mit 1.64 ml (11.2 mmol) Bromessigsäure-tert-butylester und 2.39 ml (11.2 mmol) *N,N-*Dicyclohexylmethylamin versetzt. Nach 3 h bei 70°C wurden nochmals weitere 1.64 ml (11.2 mmol) Bromessigsäure-tert-butylester und 2.39 ml (11.2 mmol) *N,N*-Dicyclohexylmethylamin hinzugegeben und für weitere 24 h bei 70°C gerührt. Der Feststoff in der Reaktionsmischung wurde abfiltriert und mit Tetrahydrofuran zwei Mal gewaschen. Das regioisomerenreine Kristallisat wird für 3 h im Vakuumtrockenschrank bei 50°C getrocknet. Man erhielt 971 mg (74 % d. Th.) Produkt.
UPLC-MS (Methode A1): Rₜ = 1.47 min
MS (ESIpos): m/z = 473 (M+H)⁺
1H-NMR (500MHz, DMSO-d6): δ = 1.45 (s, 9H), 2.43 (s, 3H), 5.20 (s, 2H), 5.31 (s, 2H), 7.29 (s, 1H), 7.39 - 7.43 (m, 1H), 7.45 - 7.53 (m, 3H), 7.63 - 7.68 (m, 2H), 7.93 - 7.97 (m, 1H), 7.97 - 8.00 (m, 1H), 8.29 (d, 1H), 8.78 (s, 1H), 10.87 (s, 1H).

### Intermediat 8-20

### Methyl-3-[6-methoxy-5-({[6-(trifluormethyl)pyridin-2-yl]carbonyl}amino)-2H-indazol-2-yl]-2-methylpropanoat

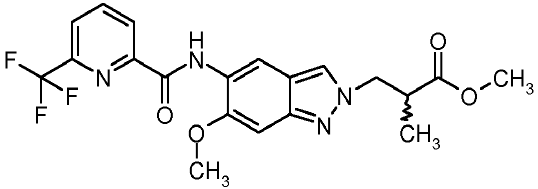

200 mg (0.60 mmol) N-(6-Methoxy-1H-indazol-5-yl)-6-(trifluoromethyl)pyridine-2-carboxamid (Intermediat 14-2) in 5 ml Acetonitril wurden mit 164 mg (1.19 mmol) Kaliumcarbonat und 83 µl (0.65 mmol) Methyl-(2R)-3-brom-2-methylpropanoat versetzt und man rührte bei 85°C für 24 h. Man verdünnte mit Wasser, extrahierte mit Ethylacetat, wusch mit gesättigter Natriumchloridlösung, filtrierte über einen wasserabweisenden Filter und engte ein. Das Rohprodukt wurde in 2,0 ml Dimethylsulfoxid gelöst und mit präparativer HPLC gereinigt. Die Produktfraktion wurde lyophilisiert. Man erhielt 25 mg (56 % d.Th.) der Titelverbindung.
UPLC-MS (Methode A1): Rₜ = 1.23 min
MS (ESIpos): m/z = 437 (M+H)⁺
1H-NMR (300MHz, DMSO-d6): δ = 1.08 (d, 3 H), 3.13 (q, 1 H), 3.55 (s, 3 H), 4.04 (s, 3 H), 4.48 (dd, 1 H), 4.62 (dd, 1 H), 7.40 (s, 1 H), 8.02 (s, 1 H), 8.17 - 8.26 (m, 1 H), 8.40 (t, 1 H), 8.47 (d, 1 H), 8.71 (s, 1 H), 10.42 (s, 1 H).

### Intermediat 8-21

### Benzyl-[5-({[6-(difluormethyl)pyridin-2-yl]carbonyl}amino)-6-methoxy-2H-indazol-2-yl]acetat

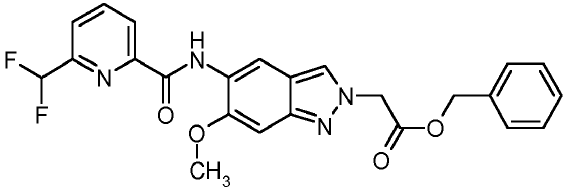

Analog zu Intermediat 8-6 wurden 400 mg (1.29 mmol) Benzyl-(5-amino-6-methoxy-2H-indazol-2-yl)acetat (Intermediat 7-6) mit 245 mg (1.41 mmol) 6-(Difluormethyl)pyridin-2-carbonsäure (CAS Nr: 1256824-41-5), 197 mg (1.29 mmol) 1-Hydroxy-1H-benzotriazol-Hydrat und 493 mg (2.57 mmol) 1-(3-Dimethylaminopropyl)-3-ethylcarbodiimidhydrochlorid und 537 µl (3.85 mmol) Triethylamin in 10 ml Tetrahydrofuran bei 25°C für 24 h gerührt. Die Reaktionsmischung wurde mit Wasser verdünnt und drei Mal mit Ethylacetat extrahiert. Die vereinigten organischen Phasen wurden mit gesättigter Natriumchloridlösung gewaschen und eingeengt. Das Rohprodukt wurde in Diethylether und etwas Wasser aufgenommen und für 30 Minuten gerührt. Der Feststoff wurde abgesaugt, drei Mal mit Diethylether gewaschen und im Trockenschrank getrocknet. Man erhielt 401 mg (48 % d.Th.) der Titelverbindung.
UPLC-MS (Methode A1): Rₜ = 1.29 min
MS (ESIpos): m/z = 467 (M+H)⁺.

### Intermediat 8-22

### Benzyl-[5-({[6-(2-hydroxypropan-2-yl)pyridin-2-yl]carbonyl}amino)-6-methoxy-2H-indazol-2-yl]acetat

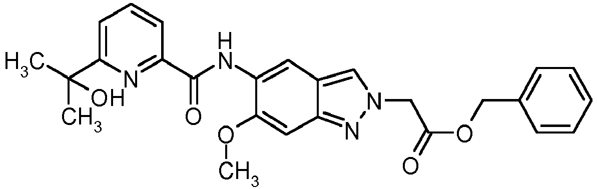

Analog zu Intermediat 8-6 wurden 300 mg (0.96 mmol) Benzyl-(5-amino-6-methoxy-2H-indazol-2-yl)acetat (Intermediat 7-6) mit 295 mg (1.16 mmol) Kalium-6-(2-hydroxypropan-2-yl)pyridin-2-carboxylat (Intermediat 19-11), 148 mg (0.96 mmol) 1-Hydroxy-1H-benzotriazol-Hydrat und 277 mg (1.45 mmol) 1-(3-Dimethylaminopropyl)-3-ethylcarbodiimidhydrochlorid und 403 µl (2.89 mmol) Triethylamin in 10 ml Tetrahydrofuran bei 25°C für 24 h gerührt. Die Reaktionsmischung wurde mit Wasser verdünnt und drei Mal mit Ethylacetat extrahiert. Die vereinigten organischen Phasen wurden mit gesättigter Natriumchloridlösung gewaschen und eingeengt. Das Rohprodukt wurde in 4 ml Dimethylsulfoxid gelöst und per präparativer HPLC nach der Methode P5 (Gradient: 0 - 15 min 30 - 70% B; Fluss: 150 ml/min) gereinigt. Die Produktfraktion wurden lyophilisiert. Man erhielt 209 mg (46 % d.Th.) der Titelverbindung.
UPLC-MS (Methode A1): Rₜ = 1.19 min
MS (ESIpos): m/z = 475 (M+H)⁺
¹H-NMR (300 MHz, DMSO-d6): δ 1.57 (s, 6 H), 4.00 (s, 3 H), 5.21 (s, 2 H), 5.41 (s, 2 H), 5.47 (s, 1 H), 7.13 (s, 1 H), 7.34 - 7.41 (m, 5 H), 7.94 (dd, 1 H), 7.99 - 8.12 (m, 2 H), 8.33 (s, 1 H), 8.69 (s, 1 H), 10.94 (s, 1 H).

### Intermediat 8-23

### Benzyl-(6-methoxy-5-{[(6-methylpyridin-2-yl)carbonyl]amino}-2H-indazol-2-yl)acetat

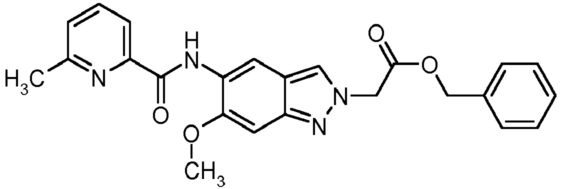

7.57 g (19.0 mmol) N-(6-Methoxy-1H-indazol-5-yl)-6-methylpyridin-2-carboxamid (Intermediat 14-6) wurden mit 6.03 ml (38.1 mmol) Bromessigsäurebenzylester in 100 ml Tetrahydrofuran in Gegenwart von 8.01 ml (38.1 mmol) *N,N-*Dicyclohexylmethylamin für 2.5 h bei 70°C und für 17 h bei 60°C gerührt. Man gab nochmals 3.02 ml (19.1 mmol) Bromessigsäurebenzylester und 4.01 ml (19.1 mmol) *N,N-*Dicyclohexylmethylamin hinzu und rührte für weitere 24 h bei 70°C. Der Feststoff wurde abgesaugt und mit Ethylacetat gewaschen. Das Filtrat wurde nochmals filtriert, zwei Mal mit Ethylacetat gewaschen und der Feststoff getrocknet. Das Filtrat wurde mit Wasser versetzt und nach Phasentrennung noch ein Mal mit Ethylacetat gewaschen. Die vereinigten organischen Phasen wurden mit gesättigter Natriumchloridlösung gewaschen, über einen wasserabweisenden Filter filtriert und eingeengt. Das Rohprodukt wurde mit Ethylacetat versetzt und 15 Minuten gerührt. Der Feststoff wurde abgesaugt und drei Mal mit Ethylacetat gewaschen und im Trockenschrank getrocknet. Insgesamt erhielt man 6.02 g (63% der Theorie) der Titelverbindung erhalten.
LC-MS (Methode A3): Rₜ = 1.25 min
MS (ESIpos): m/z = 431 (M+H)⁺
¹H-NMR (500MHz, DMSO-d6): δ = 2.63 (s, 3 H), 4.01 (s, 3 H), 5.21 (s, 2 H), 5.40 (s, 2 H), 7.11 (s, 1 H), 7.34 - 7.40 (m, 5 H), 7.55 (dd, 1 H), 7.93 - 8.02 (m, 2 H), 8.30 - 8.33 (m, 1 H), 8.73 (s, 1 H), 10.72 (s, 1 H).

### Intermediat 8-24

### tert-Butyl- [6-methoxy-5-({[2-(tetrahydro-2H-pyran-4-yl)-1,3-oxazol-4-yl]carbonyl} amino)-2H-indazol-2-yl] acetat

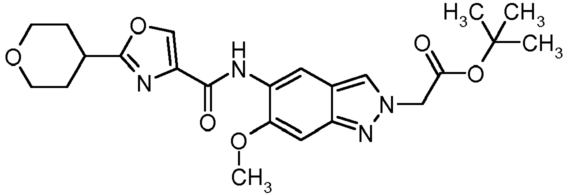

1.19 g (1.77 mmol) N-(6-Methoxy-1H-indazol-5-yl)-2-(tetrahydro-2H-pyran-4-yl)-1,3-oxazol-4-carboxamid (Intermediat 14-7) wurden mit 524 µl (3.55 mmol) Bromessigsäure-tert-butylester in 10 ml Tetrahydrofuran in Gegenwart von 752 µl (3.55 mmol) *N,N-*Dicyclohexylmethylamin für 2.5 h bei 70°C und für 17 h bei 60°C gerührt. Man gab nochmals 1.51 ml (9.5 mmol) Bromessigsäure-tert-butylester und 2.00 ml (9.5 mmol) *N,N-*Dicyclohexylmethylamin hinzu und rührte für weitere 6 h bei 70°C. Der Feststoff wurde abgesaugt und drei Mal mit Ethylacetat gewachen. Das Filtrat wurde mit Wasser versetzt und nach Phasentrennung noch ein Mal mit Ethylacetat gewaschen. Die vereinigten organischen Phasen wurden mit gesättigter Natriumchloridlösung gewaschen, über einen wasserabweisenden Filter filtriert und eingeengt. Das Rohprodukt wurde mit Ethylacetat versetzt, der Feststoff wurde abgesaugt und drei Mal mit Ethylacetat gewaschen und im Trockenschrank getrocknet. Insgesamt erhielt man 330 mg (41% der Theorie) der Titelverbindung erhalten.
UPLC-MS (Methode A1): Rₜ = 1.23 min
MS (ESIpos): m/z = 457 (M+H)⁺
¹H-NMR (500MHz, DMSO-d6): δ = 1.44 (s, 9 H), 1.72 - 1.86 (m, 2 H), 1.91 - 2.02 (m, 2 H), 3.17 - 3.27 (m, 1 H), 3.48 (td, 2 H), 3.92 (dt, 2 H), 3.97 (s, 3 H), 5.18 (s, 2 H), 7.10 (s, 1 H), 8.26 (d, 1 H), 8.57 (s, 1 H), 8.74 (s, 1 H), 9.41 (s, 1 H).

### Intermediat 8-25

### tert-Butyl-(5-{[(6-brompyridin-2-yl)carbonyl]amino}-6-methoxy-2H-indazol-2-yl)acetat

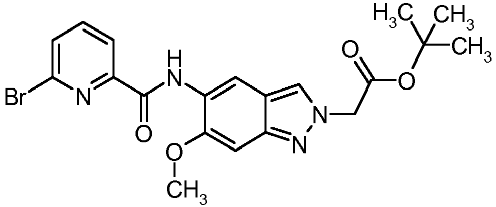

4.20 g (12.10 mmol) 6-Brom-N-(6-methoxy-1H-indazol-5-yl)pyridin-2-carboxamid (Intermediat 14-8) wurden mit 3.57 ml (24.20 mmol) Bromessigsäure-tert-butylester in 50 ml Tetrahydrofuran in Gegenwart von 5.18 ml (24.20 mmol) *N,N-*Dicyclohexylmethylamin für 2 h bei 70°C und für 17 h bei 60°C gerührt. Man gab nochmals 3.57 ml (24.20 mmol) Bromessigsäure-tert-butylester und 5.18 ml (24.20 mmol) *N,N-*Dicyclohexylmethylamin hinzu und rührte für weitere 24 h bei 70°C. Die Reaktionsmischung wurde mittels Eisbad gekühlt und der entstandene Feststoff wurde abgesaugt, mit Wasser und Diethylether gewaschen und im Vakuum getrocknet. Man erhielt 3.67 g (66% der Theorie) der Titelverbindung erhalten.
UPLC-MS (Methode A1): Rₜ = 1.33 min
MS (ESIpos): m/z = 461 (M+H)⁺
¹H-NMR (500MHz, DMSO-d6): δ = 1.44 (s, 9 H) 4.00 (s, 3 H) 5.20 (s, 2 H) 7.14 (s, 1 H) 7.90 - 8.10 (m, 2 H) 8.20 (dd, 1 H) 8.29 (s, 1 H) 8.68 (s, 1 H) 10.31 (s, 1 H).

### Intermediat 9-1

### [6-Fluor-5-({[6-(trifluoromethyl)pyridin-2-yl]carbonyl} amino)-2H-indazol-2-yl]essigsäure

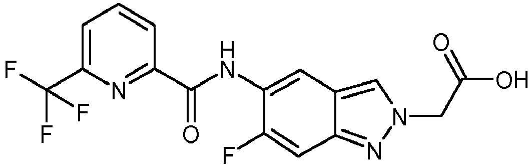

381 mg (0.93 mmol) Ethyl [6-fluoro-5-({[6-(trifluoromethyl)pyridin-2-yl]carbonyl}amino)-2H-indazol-2-yl]acetat (Intermediat 8-1) wurden in 9.2 ml Tetrahydrofuran und 0.45 ml Ethanol suspendiert und dann mit einer Lösung von 222 mg (9.3 mmol) Lithiumhydroxid in 2.3 ml Wasser versetzt. Es wurde 30 min bei 25°C gerührt und dann unter Eiskühlung auf pH 2 mit 2N Salzsäure angesäuert. Dann wurden 10 ml Wasser zugegeben und der Niederschlag abgesaugt. Man erhielt 332 mg (93 % d.Th.) der Titelverbindung.
UPLC-MS (Methode A1): Rₜ = 1.04 min
MS (ESIpos): m/z = 383 (M+H)⁺
1H-NMR (300 MHz, DMSO-d6): δ = 5.30 (s, 2H), 7.55 (d, 1H), 8.22 (m, 1H), 8.34 - 8.54 (m, 4H), 10.26 (m, 1H), 13.30 (s br, 1H).

### Intermediat 9-2

### (6-Fluor-5-{[(6-methylpyridm-2-yl)carbonyl]amino}-2H-indazol-2-yl)essigsäure

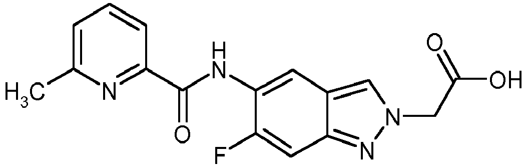

Analog zu Intermediat 9-1 wurden 316 mg (0.89 mmol) Ethyl (6-fluoro-5-{[(6-methylpyridin-2-yl)carbonyl]amino}-2H-indazol-2-yl)acetat (Intermediat 8-2) mit 212 mg (8.87 mmol) Lithiumhydroxid in 2.2 ml Wasser, 8.8 ml Tetrahydrofuran und 0.44 ml Ethanol umgesetzt. Nach Aufarbeitung erhielt man 302 mg der Titelverbindung als Rohprodukt.
UPLC-MS (Methode A1): Rₜ = 0.99 min
MS (ESIpos): m/z = 329 (M+H)⁺
¹H NMR (400 MHz, DMSO-d6) δ = 2.62 (s, 3 H), 5.28 (s, 2 H), 7.44 - 7.63 (m, 2 H), 7.90 - 8.06 (m, 2 H), 8.45 (s, 1 H), 8.56 (d, 1 H), 10.38 (d, J=1 H).

### Intermediat 9-3

### [6-Fluor-5-({[6-(1-methyl-1H-pyrazol-4-yl)pyridin-2-yl]carbonyl}amino)-2H-indazol-2-yl]essigsäure

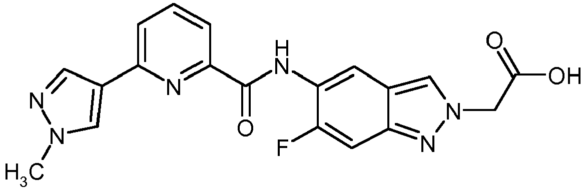

Analog zu Intermediat 9-1 wurden 364 mg (0.86 mmol) Ethyl [6-fluoro-5-({[6-(1-methyl-1H-pyrazol-4-yl)pyridin-2-yl]carbonyl}amino)-2H-indazol-2-yl]acetat (Intermediat 8-3) mit 206 mg (8.6 mmol) Lithiumhydroxid in 2.1 ml Wasser, 8.5 ml Tetrahydrofuran und 0.42 ml Ethanol umgesetzt. Nach Aufarbeitung erhielt man 302 mg (89 % d.Th.) der Titelverbindung als Rohprodukt.
UPLC-MS (Methode A1): Rₜ = 0.87 min
MS (ESIpos): m/z = 395 (M+H)⁺
¹H-NMR (300 MHz, DMSO-d6): δ = 3.93 (s, 3H), 5.30 (s, 2H), 7.55 (d, 1H), 7.92 (t, 2H), 8.03 (t, 1H), 8.21 (s, 1H), 8.39 (d, 1H), 8.46 (s, 1H), 8.52 (s, 1H), 10.51 (s, 1H), 13.26 (s br, 1H).

### Intermediat 9-4

### [6-Fluor-5-({[5-fluor-6-(1-methyl-1H-pyrazol-4-yl)pyridin-2-yl]carbonyl}amino)-2H-indazol-2-yl]essigsäure

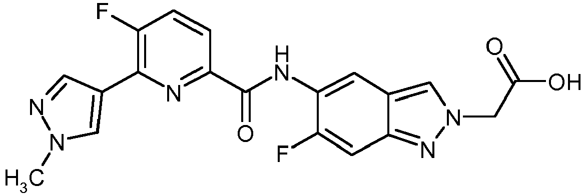

Analog zu Intermediat 9-1 wurden 326 mg (0.74 mmol) Ethyl-[6-fluor-5-({[5-fluor-6-(1-methyl-1H-pyrazol-4-yl)pyridin-2-yl]carbonyl}amino)-2H-indazol-2-yl]acetat (Intermediat 8-4) mit 177 mg (7.4 mmol) Lithiumhydroxid in 1.8 ml Wasser, 7.3 ml Tetrahydrofuran und 0.36 ml Ethanol umgesetzt. Nach Aufarbeitung erhielt man 305 mg (100 % d.Th.) der Titelverbindung als Rohprodukt.
UPLC-MS (Methode A1): Rₜ = 0.95 min
MS (ESIpos): m/z = 413 (M+H)⁺
¹H-NMR (300 MHz, DMSO-d6): δ = 3.96 (s, 3H), 5.30 (s, 2H), 7.54 (d, 1H), 7.98 (m, 2H), 8.27 (m, 2H), 8.46 (s, 1H), 8.53 (s, 1H), 10.42 (s, 1H), 13.29 (s br, 1H).

### Intermediat 9-5

### [6-Fluor-5-({[6-(morpholin-4-yl)pyridin-2-yl]carbonyl} amino)-2H-indazol-2-yl]essigsäure

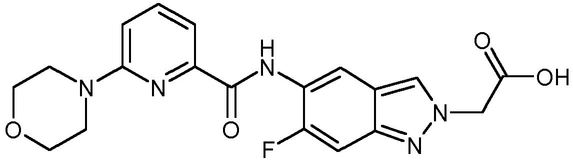

Analog zu Intermediat 9-1 wurden 436 mg (1.02 mmol) Ethyl-[6-fluor-5-({[6-(morpholin-4-yl)pyridin-2-yl]carbonyl}amino)-2H-indazol-2-yl]acetat (Intermediat 8-5) mit 244 mg (10.2 mmol) Lithiumhydroxid in 2.5 ml Wasser, 10 ml Tetrahydrofuran und 0.5 ml Ethanol umgesetzt. Nach Aufarbeitung erhielt man 295 mg (72 % d.Th.) der Titelverbindung als Rohprodukt.
UPLC-MS (Methode A1): Rₜ = 0.95 min
MS (ESIpos): m/z = 400 (M+H)⁺
¹H-NMR (300 MHz, DMSO-d6): δ = 3.59 (m, 4H), 3.75 (m, 4H), 5.26 (s, 2H), 7.15 (d, 1H), 7.42 - 7.59 (m, 2H), 7.82 (t, 1H), 8.40 - 8.51 (m, 2H), 10.28 (m, 1H).

### Intermediat 9-6

### [6-(benzyloxy)-5-({[6-(trifluoromethyl)pyridin-2-yl]carbonyl}amino)-2H-indazol-2-yl]essigsäure

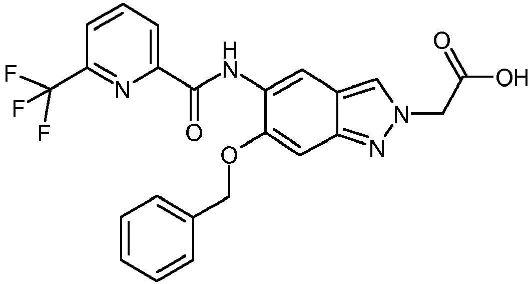

Analog zu Intermediat 9-1 wurden 75 mg (0.15 mmol) Ethyl [6-(benzyloxy)-5-({[6-(trifluoromethyl)pyridin-2-yl]carbonyl}amino)-2H-indazol-2-yl]acetat (Intermediat 8-6) mit 18 mg (0.75 mmol) Lithiumhydroxid in 271 µl Wasser und 2.5 ml Tetrahydrofuran umgesetzt. Nach Aufarbeitung erhielt man 59 mg (83 % d.Th.) der Titelverbindung.
UPLC-MS (Methode A1): Rₜ = 1.26 min
MS (ESIpos): m/z = 471 (M+H)⁺
¹H NMR (400 MHz, DMSO-d6): δ = 7.31 (s, 1 H), 7.33 - 7.47 (m, 3 H), 7.54 - 7.63 (m, 2 H), 8.12 - 8.22 (m, 1 H), 8.31 (s, 1 H), 8.39 (s, 1 H), 8.46 - 8.51 (m, 1 H), 8.80 (s, 1 H), 10.47 (s, 1 H).

### Intermediat 9-7

### [6-Isobutoxy-5-({[6-(trifluoromethyl)pyridin-2-yl]carbonyl} amino)-2H-indazol-2-yl]essigsäure

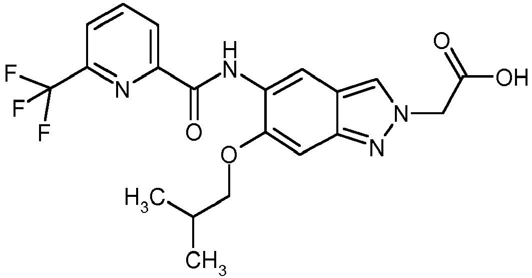

Analog zu Intermediat 9-1 wurden 200 mg (0.43 mmol) Ethyl [6-isobutoxy-5-({[6-(trifluoromethyl)pyridin-2-yl]carbonyl}amino)-2H-indazol-2-yl]acetat (Intermediat 8-8) mit 51 mg (2,15 mmol) Lithiumhydroxid in 776 µl Wasser und 10 ml Tetrahydrofuran umgesetzt. Nach Aufarbeitung erhielt man 64 mg (87 % d.Th.) der Titelverbindung.
UPLC-MS (Methode A1): Rₜ = 1.22 min
MS (ESIpos): m/z = 437 (M+H)⁺
¹H NMR (300 MHz, DMSO-d6): δ = 1.11 (s, 3 H), 1.13 (s, 3 H), 2.19 (dt, 1 H), 3.96 (d, 2 H), 5.21 (s, 2 H), 7.09 (s, 1 H), 8.22 (dd, 1 H), 8.31 (s, 1 H), 8.37 - 8.46 (m, 1 H), 8.46 - 8.52 (m, 1 H), 8.78 (s, 1 H), 10.58 (s, 1 H).

### Intermediat 9-8

### [6-(Cyclopropylmethoxy)-5-({[6-(trifluoromethyl)pyridin-2-yl]carbonyl} amino)-2H-indazol-2-yl]essigsäure

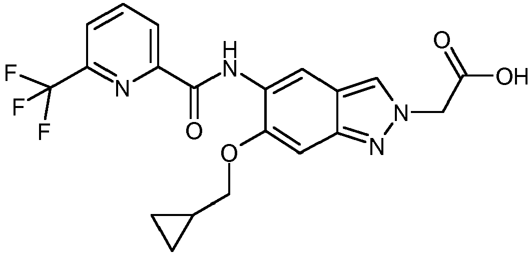

Analog zu Intermediat 9-1 wurden 220 mg (0.48mmol) Ethyl [6-(cyclopropylmethoxy)-5-({[6-(trifluoromethyl)pyridin-2-yl]carbonyl}amino)-2H-indazol-2-yl]acetat (Intermediat 8-9) mit 57 mg (2,38 mmol) Lithiumhydroxid in 857 µl Wasser und 10 ml Tetrahydrofuran umgesetzt. Nach Aufarbeitung erhielt man 181 mg (88 % d.Th.) der Titelverbindung.
UPLC-MS (Methode A1): Rₜ = 1.21 min
MS (ESIpos): m/z = 435 (M+H)⁺
¹H NMR (400 MHz, DMSO-d6): δ = 0.42 - 0.48 (m, 2 H), 0.63 - 0.69 (m, 2 H), 1.29 - 1.41 (m, 1 H), 4.03 (d, 2 H), 5.20 (s, 2 H), 7.07 (s, 1 H), 8.21 (dd, 1 H), 8.29 (s, 1 H), 8.37 - 8.44 (m, 1 H), 8.46 - 8.50 (m, 1 H), 8.76 (s, 1 H), 10.71 (s, 1 H).

### Intermediat 9-9

### [6-(Pyridin-2-ylmethoxy)-5-({[6-(trifluoromethyl)pyridin-2-yl]carbonyl}amino)-2H-indazol-2-yl]essigsäure

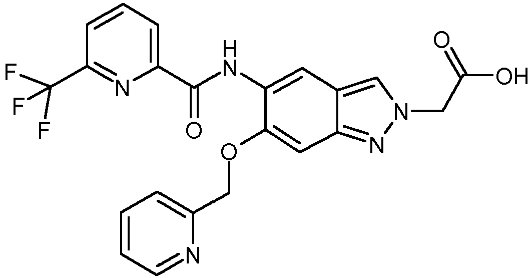

Analog zu Intermediat 9-1 wurden 160 mg (0.32mmol) Ethyl [6-(pyridin-2-ylmethoxy)-5-({[6-(trifluoromethyl)pyridin-2-yl]carbonyl}amino)-2H-indazol-2-yl]acetat (Intermediat 8-10) mit 38 mg (1.60 mmol) Lithiumhydroxid in 577 µl Wasser und 6.7 ml Tetrahydrofuran umgesetzt. Nach Aufarbeitung erhielt man 129 mg (85 % d.Th.) der Titelverbindung.
UPLC-MS (Methode A1): Rₜ = 1.02 min
MS (ESIpos): m/z = 472 (M+H)⁺
¹H NMR (300 MHz, DMSO-d6): δ = 5.02 (s, 2 H), 5.34 (s, 2 H), 7.30 (s, 1 H), 7.42 (dd, 1 H), 7.70 (d, 1 H), 7.80 - 7.92 (m, 1 H), 8.18 (d, 1 H), 8.27 (s, 1 H), 8.39 (t, 1 H), 8.44 - 8.53 (m, 1 H), 8.62 (d, 1 H), 8.80 (s, 1 H), 10.49 (s, 1 H).

### Intermediat 9-10

### [5-({[6-(1-Methyl-1H-pyrazol-4-yl)pyridin-2-yl]carbonyl}amino)-2H-indazol-2-yl]essigsäure

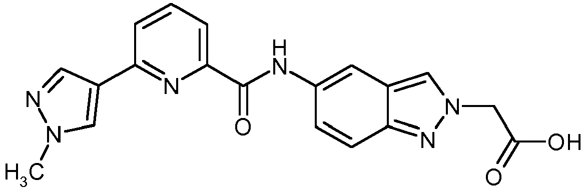

Analog zu Intermediat 9-1 wurden 1.2 g (3.11 mmol) Ethyl-[5-({[6-(1-methyl-1H-pyrazol-4-yl)pyridin-2-yl]carbonyl}amino)-2H-indazol-2-yl]acetat (Intermediat 8-11) (Rohprodukt) in 10 ml Tetrahydrofuran vorgelegt und 1.25 g (29.7 mmol) Litihumhydroxid Monohydrat in 3 ml Wasser und 2 ml Ethanol addiert. Man rührte 5 h bei 25°C. Man versetzte mit Wasser und addierte 10 prozentige Zitronensäure bis zu einem pH-Wert von 4. Man extrahierte drei Mal mit Ethylacetat und versetzte die wässrige Phase mit gesättigter Natriumchloridlösung. Aus der wässrigen Phase fiel ein Feststoff aus der abgesaugt, mit Wasser und Ethylacetat gewaschen und getrocknet wurde. Man erhielt 850 mg (54 % d.Th.) der Titelverbindung als braunen Feststoff.
UPLC-MS (Methode A1): Rₜ = 0.82 min
MS (ESIpos): m/z = 37 (M+H)+.
¹H-NMR (300MHz, DMSO-d₆): δ = 3.93 (s), 4.98 (s, 2H), 7.60 (s, 2H), 7.83 - 8.05 (m, 3H), 8.23 - 8.40 (m, 3H), 8.67 (s, 1H), 10.42 (s, 1H).

### Intermediat 9-11

### ([6-Chlor-5-({[6-(trifluormethyl)pyridin-2-yl]carbonyl}amino)-2H-indazol-2-yl]essigsäure

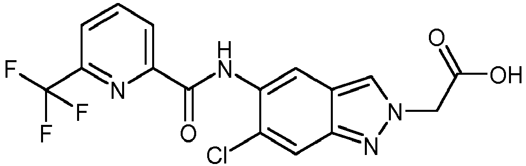

1.45 g (3.19 mmol) tert-Butyl-[6-chlor-5-({[6-(trifluormethyl)pyridin-2-yl]carbonyl}amino)-2H-indazol-2-yl]acetat (Intermediat 8-14) wurden in 15 ml Dichlormethan und bei 25°C mit 2.46 ml (31.9 mmol) Trifluoressigsäure versetzt. Die Lösung wurde für 18 h bei 25°C gerührt Man versetzte mit Wasser und saugte den ausgefallenen Niederschlag ab, wusch drei Mal mit Wasser, zwei Mal mit Diethylether und trocknete den Feststoff im Vakuum. Es wurden 1.28 g (98% der Theorie) der Titelverbindung erhalten.
UPLC-MS (Methode A1): Rₜ = 1.11 min
MS (ESIpos): m/z = 399 (M+H)⁺
1H-NMR (400MHz, DMSO-d6): δ = 5.31 (s, 2H), 7.93 (s, 1H), 8.22 (dd, 1H), 8.37 - 8.50 (m, 3H), 8.64 (s, 1H), 10.52 (s, 1H), 13.28 (br. s., 1H).

### Intermediat 9-12

### [6-Methoxy-5-({[6-(trifluoromethyl)pyridin-2-yl]carbonyl}amino)-2H-indazol-2-yl]essigsäure

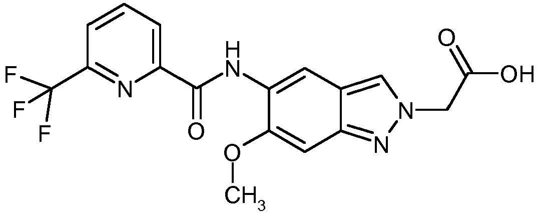

Analog zu Intermediat 9-11 wurden 1.1 g (2.44 mmol) tert-Butyl [6-methoxy-5-({[6-(trifluoromethyl)pyridin-2-yl]carbonyl}amino)-2H-indazol-2-yl]acetat (Intermediat 8-15) mit 3.76 ml (48.8 mmol) Trifluoressigsäure in 20 ml Dichlormethan für 24 h bei 25°C gerührt. Nach Aufarbeitung erhielt man 1.20 g (96 % d. Th.) der Titelverbindung.
UPLC-MS (Methode A1): Rₜ = 1.09 min
MS (ESIpos): m/z = 395 (M+H)⁺
¹H NMR (300 MHz, DMSO-d6): δ = 3.99 (s, 3 H), 5.22 (s, 2 H), 7.14 (s, 1 H), 8.22 (dd, 1 H), 8.31 (s, 1 H), 8.42 (d, 1 H), 8.46 (s, 1 H), 8.71 (s, 1 H), 10.51 (s, 1 H).

### Intermediat 9-13

### [6-Ethoxy-5-({[6-(trifluormethyl)pyridin-2-yl]carbonyl}amino)-2H-indazol-2-yl]essigsäure

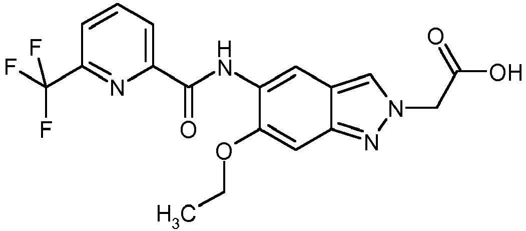

Analog zu Intermediat 9-1 wurden 774 mg (1.77 mmol) Ethyl-[6-ethoxy-5-({[6-(trifluormethyl)pyridin-2-yl]carbonyl}amino)-2H-indazol-2-yl]acetat (Intermediat 8-12) in 1 ml Ethanol und 25 ml Tetrahydrofuran vorgelegt und dann mit einer Lösung von 745 mg (17.74 mmol) Lithiumhydroxid Monohydrat gelöst in 5 ml Wasser versetzt und für 3 Tage bei 25°C gerührt. Nach Aufarbeitung erhielt man 698 mg (94% der Theorie) der Titelverbindung.
UPLC-MS (Methode A1): Rₜ = 1.13 min
MS (ESIpos): m/z = 409 (M+H)⁺
¹H-NMR (300MHz, DMSO-d6): δ = 1.49 (t, 3H), 4.20 (q, 2H), 5.17 (s, 2H), 7.09 (s, 1H), 8.21 (dd, 1H), 8.28 (s, 1H), 8.36 - 8.48 (m, 2H), 8.71 (s, 1H), 10.73 (s, 1H).

### Intermediat 9-14

### [5-({[6-(Trifluormethyl)pyridin-2-yl]carbonyl}amino)-2H-indazol-2-yl]essigsäure

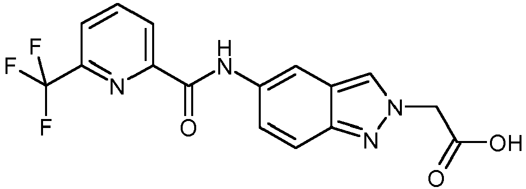

Eine Mischung aus 216 mg (2.02 mmol) tert-Butyl-[5-({[6-(trifluormethyl)pyridin-2-yl]carbonyl}amino)-2H-indazol-2-yl]acetat (Intermediat 8-16) in 3 ml Dichlormethan wurde mit 197 µl (2.57 mmol) Trifluoressigsäure versetzt. Man rührte 3 Tage bei 25°C, addierte nochmals 197 µl (2.57 mmol) Trifluoressigsäure und rührte bei 25°C. Das Reaktionsgemisch wurde mit Wasser versetzt. Man rührte 10 min und saugte den Feststoff ab, wusch mit Wasser und trocknete. Man erhielt 142 mg (76 % d.Th.) der Titelverbindung.
¹H-NMR (300MHz, DMSO-d₆): δ = 5.25 (s, 2H), 7.52 - 7.62 (m, 2H), 8.14 (dd, 1H), 8.26 - 8.41 (m, 4H), 10.37 (s, 1H).

### Intermediat 9-15

### 3-[5-({[6-(Trifluormethyl)pyridin-2-yl]carbonyl}amino)-2H-indazol-2-yl]propansäure

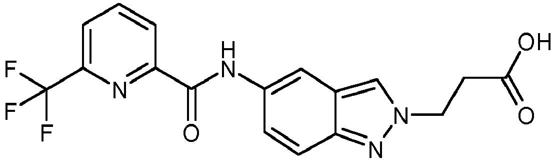

Analog zu Intermediat 9-1 wurden 285 mg (0.70 mmol) Ethyl-3-[5-({[6-(trifluormethyl)pyridin-2-yl]carbonyl}amino)-2H-indazol-2-yl]propanoat (Intermediat 8-13), mit 168 mg (7.0 mmol) Lithiumhydroxid umgesetzt. Man erhielt 253 mg (95 % d.Th.) der Titelverbindung.
UPLC-MS (Methode A1): Rₜ = 0.99 min
MS (ESIpos): m/z = 379 (M+H)⁺.

### Intermediat 9-16

### [6-Isopropoxy-5-({[6-(trifluoromethyl)pyridin-2-yl]carbonyl}amino)-2H-indazol-2-yl]essigsäure

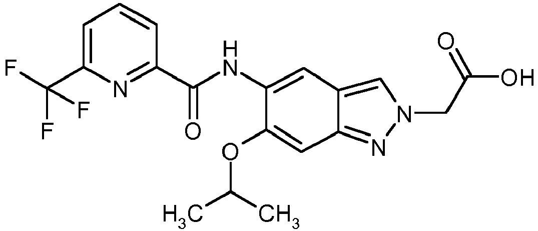

Analog zu Intermediat 9-1 wurden 490 mg (1.1 mmol) Ethyl [6-isopropoxy-5-({[6-(trifluoromethyl)pyridin-2-yl]carbonyl}amino)-2H-indazol-2-yl]acetat (Intermediat 8-17), mit 260 mg (11 mmol) Lithiumhydroxid umgesetzt. Man erhielt 367 mg (80 % d.Th.) der Titelverbindung.
UPLC-MS (Methode A1): Rt = 1.17 min
MS (ESIpos): m/z = 423 (M+H)⁺
1H-NMR (300 MHz, DMSO-d6): δ = 1.45 (d, 6H), 4.80 - 4.92 (m, 1H), 5.21 (s, 2H), 7.17 (s, 1H), 8.19 - 8.25 (m, 1H), 8.30 (s, 1H), 8.36 - 8.49 (m, 2H), 8.74 (s, 1H), 10.75 (s, 1H), 13.21 (s, 1H).

### Intermediat 9-17

### (6-Isopropoxy-5-{[(6-methylpyridin-2-yl)carbonyl]amino}-2H-indazol-2-yl)essigsäure

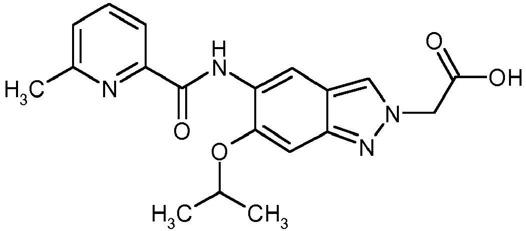

Analog zu Intermediat 9-1 wurden 370 mg (0.93 mmol) Ethyl (6-isopropoxy-5-{[(6-methylpyridin-2-yl)carbonyl]amino}-2H-indazol-2-yl)acetat (Intermediat 8-18), mit 223 mg (9.33 mmol) Lithiumhydroxid umgesetzt. Man erhielt 280 mg (81 % d.Th.) der Titelverbindung.
UPLC-MS (Methode A1): Rt = 1.11 min
MS (ESIpos): m/z = 369 (M+H)⁺
¹H-NMR (300 MHz, DMSO-d6): δ = 1.45 (d, 6H), 2.62 (s, 3H), 4.78 - 4.89 (m, 1H), 5.19 (s, 2H), 7.14 (s, 1H), 7.52 - 7.60 (m, 1H), 7.93 -8.02 (m, 2H), 8.27 (s, 1H), 8.72 (s, 1H), 10.99 (s, 1H), 13.19 (sbr, 1H).

### Intermediat 9-18

### [6-(Benzyloxy)-5-{[(6-methylpyridin-2-yl)carbonyl]amino}-2H-indazol-2-yl]essigsäure

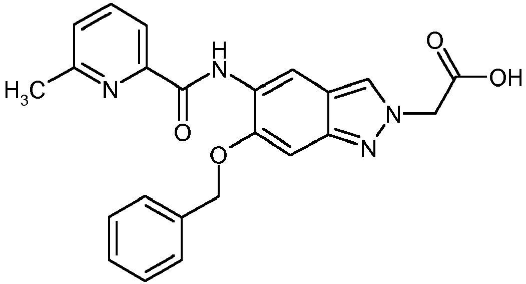

Analog zu Intermediat 9-14 wurden 100 mg (0.21 mmol) tert-Butyl-[6-(benzyloxy)-5-{[(6-methylpyridin-2-yl)carbonyl]amino}-2H-indazol-2-yl]acetat (Intermediat 8-19) in 6.7 ml Dichlormethan gelöst und mit 326 µl (4.23 mmol) Trifluoressigsäure für 24 h bei 25°C gerührt.. Nach Aufarbeitung erhielt man 67 mg (76 % d.Th.) der Titelverbindung.
UPLC-MS (Methode A1): Rₜ = 1.20 min
MS (ESIpos): m/z = 417 (M+H)⁺
¹H NMR (400 MHz, DMSO-d6): δ = 2.43 (s, 3 H), 5.22 (s, 2 H), 5.31 (s, 2 H), 7.29 (s, 1 H), 7.42 (d, 1 H), 7.44 - 7.54 (m, 3 H), 7.65 (d, 2 H), 7.91 - 8.02 (m, 2 H), 8.30 (s, 1 H), 8.78 (s, 1 H), 10.87 (s, 1 H).

### Intermediat 9-19

### (6-Methoxy-5-{[(6-methylpyridin-2-yl)carbonyl]amino}-2H-indazol-2-yl)essigsäure

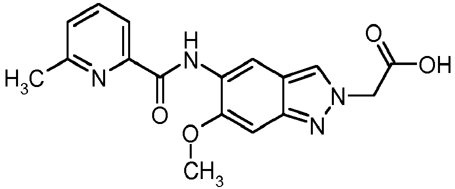

Analog zu Intermediat 9-1 wurden 2.28 g (3.92 mmol, 74%) Benzyl-(6-methoxy-5-{[(6-methylpyridin-2-yl)carbonyl]amino}-2H-indazol-2-yl)acetat (Intermediat 8-23) in 20 ml Tetrahydrofuran und 3.0 ml Methanol gelöst und dann mit einer Lösung von 1.65 g (39.2 mmol) Lithiumhydroxid Monohydrat in 3.0 ml Wasser versetzt. Man verdünnte mit Wasser, säuerte mit 10 prozentiger Zitronensäurelösung auf pH 4 an. Der ausgefallene Feststoff wurde abfiltriert, drei Mal mit Wasser und drei Mal mit Diethylether gewaschen und im Vakuum getrocknet. Man erhielt 2.43 g der Titelverbindung als Rohprodukt.
UPLC-MS (Methode A1): Rₜ = 1.00 min
MS (ESIpos): m/z = 341 (M+H)⁺.

### Intermediat 9-20

### [6-Methoxy-5-({[2-(tetrahydro-2H-pyran-4-yl)-1,3-oxazol-4-yl]carbonyl} amino)-2H-indazol-2-yl]essigsäure

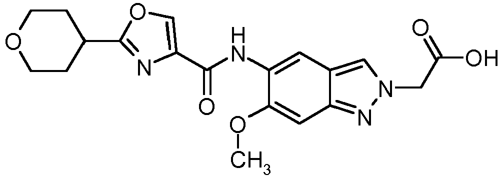

Analog zu Intermediat 9-11 wurden 325 mg (0.71 mmol) tert-Butyl-[6-methoxy-5-({[2-(tetrahydro-2H-pyran-4-yl)-1,3-oxazol-4-yl]carbonyl}amino)-2H-indazol-2-yl]acetat (Intermediat 8-24) in 5 ml Dichlormethan gelöst und mit 549 µl (7.12 mmol) Trifluoressigsäure für 21 h bei 25°C gerührt. Man gab nochmals 275 µl (3.56 mmol) Trifluoressigsäure hinzu und rührte für weitere 70 h bei 25°C. Man versetzte mit Wasser und saugte den ausgefallenen Niederschlag ab, wusch drei Mal mit Wasser, drei Mal mit Diethylether und trocknete den Feststoff im Vakuum. Man erhielt 313 mg der Titelverbindung als Rohprodukt.
UPLC-MS (Methode A1): Rₜ = 0.91 min
MS (ESIpos): m/z = 401 (M+H)⁺
¹H-NMR (300MHz, DMSO-d6): δ = 1.67 - 1.90 (m, 2 H), 1.98 (d, 2 H), 3.22 (ddd, 1 H), 3.40 - 3.54 (m, 2 H), 3.87 - 4.01 (m, 6 H), 5.20 (s, 2 H), 7.10 (s, 1 H), 8.27 (s, 1 H), 8.56 (s, 1 H), 8.75 (s, 1 H), 9.42 (s, 1 H).

### Intermediat 9-21

### (5-{[(6-Brompyridin-2-yl)carbonyl]amino}-6-methoxy-2H-indazol-2-yl)essigsäure

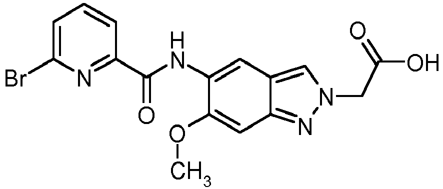

Analog zu Intermediat 9-11 wurden 3.50 g (7.59 mmol) tert-Butyl-(5-{[(6-brompyridin-2-yl)carbonyl]amino}-6-methoxy-2H-indazol-2-yl)acetat (Intermediat 8-25) in 100 ml Dichlormethan gelöst und mit 11.7 ml (15.54 mmol) Trifluoressigsäure für 24 h bei 25°C gerührt. Die Reaktionsmischung wurde vorsichtig auf gesättigte Natriumhydrogencarbonatlösung gegeben kurz gerührt, der entstandene Niederschlag abgesaugt und bei 50°C im Vakuumtrockenschrank getrocknet. Man erhielt 3.10 g der Titelverbindung als Rohprodukt.
UPLC-MS (Methode A1): Rₜ = 1.02 min
MS (ESIpos): m/z = 405 (M+H)⁺
¹H-NMR (300MHz, DMSO-d6): δ = 4.00 (s, 3 H) 5.21 (s, 2 H) 7.13 (s, 1 H) 7.95 (dd, 1 H) 8.04 (t, 1 H) 8.20 (dd, 1 H) 8.28 - 8.31 (m, 1 H) 8.68 (s, 1 H) 10.30 (s, 1 H).

### Intermediat 9-22

### 3-[6-Methoxy-5-({[6-(trifluormethyl)pyridin-2-yl]carbonyl}amino)-2H-indazol-2-yl]-2-methylpropansäure

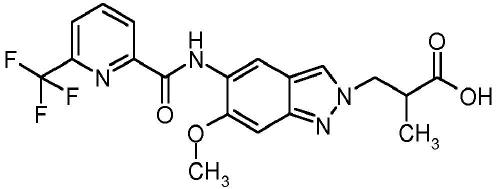

Analog zu Intermediat 4-1 wurden 37 mg (0.09 mmol) Methyl-3-[6-methoxy-5-({[6-(trifluormethyl)pyridin-2-yl]carbonyl}amino)-2H-indazol-2-yl]-2-methylpropanoat (Intermediat 8-20) in 2 ml Tetrahydrofuran und 0.1 ml Methanol gelöst und mit einer Lösung aus 36 mg (0.85 mmol) Lithiumhydroxid Monohydrat in 0.1 ml Wasser versetzt und die Mischung wurde 23.5 h bei 25°C gerührt. Man verdünnte mit Wasser, säuerte mit 10 prozentiger Zitronensäurelösung auf pH 4 an und extrahierte drei Mal mit Ethylacetat. Die vereinigten, organischen Phasen wurden mit gesättigter Natriumchloridlösung gewaschen, über einen wasserabweisenden Filter filtriert, eingeengt und im Vakuum getrocknet. Man erhielt 34 mg (94% der Theorie) der Titelverbindung.
UPLC-MS (Methode A1): Rₜ = 1.13 min
MS (ESIpos): m/z = 423 (M+H)⁺
¹H-NMR (300MHz, DMSO-d6): δ = 1.04 (d, 3 H), 3.00 - 3.13 (m, 2 H), 3.98 (s, 3 H), 4.37 (dd, 1 H), 4.59 (dd, 1 H), 7.15 (s, 1 H), 8.22 (dd, 1 H), 8.29 (s, 1 H), 8.35 - 8.44 (m, 1 H), 8.44 - 8.49 (m, 1 H), 8.68 (s, 1 H), 10.49 (s, 1 H).

### Intermediat 9-23

### 3-[6-Methoxy-5-({[6-(trifluormethyl)pyridin-2-yl]carbonyl}amino)-2H-indazol-2-yl]-2-methylpropansäure

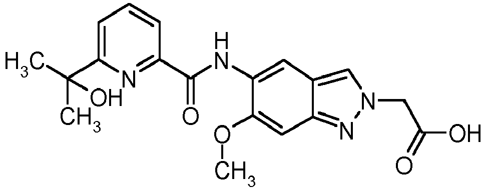

Analog zu Intermediat 4-1 wurden 206 mg (0.43 mmol) Benzyl-[5-({[6-(2-hydroxypropan-2-yl)pyridin-2-yl]carbonyl}amino)-6-methoxy-2H-indazol-2-yl]acetat (Intermediat 8-22) in 10 ml Tetrahydrofuran und 1.0 ml Methanol gelöst und mit einer Lösung aus 182 mg (4.33 mmol) Lithiumhydroxid Monohydrat in 1.5 ml Wasser versetzt und die Mischung wurde 24 h bei 25°C gerührt. Man verdünnte mit Wasser, säuerte mit 10 prozentiger Zitronensäurelösung auf pH 4 an und engte die Mischung ein. Der ausgefallene Feststoff wurde abfiltriet, ein Mal mit Wasser und drei Mal mit Diethylether gewaschen und im Vakuum getrocknet. Man erhielt 155 mg (93% der Theorie) der Titelverbindung.
UPLC-MS (Methode A1): Rₜ = 1.20 min
MS (ESIpos): m/z = 421 (M+H)⁺
¹H-NMR (400MHz, DMSO-d6): δ = 1.57 (s, 6 H), 3.99 (s, 3 H), 5.20 (s, 2 H), 5.47 (s, 1 H), 7.12 (s, 1 H), 7.93 (dd, J=7.5, 1.3 Hz, 1 H), 7.98 - 8.11 (m, 2 H), 8.28 (s, 1 H), 8.68 (s, 1 H), 10.93 (s, 1 H).

### Intermediat 9-24

### [5-({[6-(Difluormethyl)pyridin-2-yl]carbonyl}amino)-6-methoxy-2H-indazol-2-yl]essigsäure

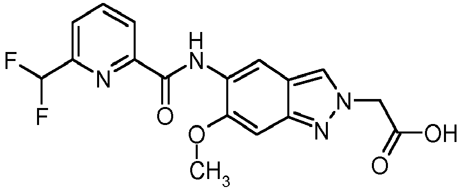

Analog zu Intermediat 4-1 wurden 613 mg Benzyl-[5-({[6-(difluormethyl)pyridin-2-yl]carbonyl}amino)-6-methoxy-2H-indazol-2-yl]acetat (Intermediat 8-21) mit 469 mg Lithiumhydroxid Monohydrat in 3 ml Wasser, 15 ml THF und 1 ml Methanol 3 h bei Raumtemperatur gerührt. Man erhielt nach analoger Aufarbeitung 378 mg der Titelverbindung.
UPLC-MS (Methode A1): Rt = 0.98 min, gefundene Masse (UV Detector TIC) 376.00.

### Intermediat 10 und Intermediat 11

### tert-Butyl-6-brom-5-[(tert-butoxycarbonyl)amino]-1H-indazol-1-carboxylattert-Butyl-6-brom-5-[(tert-butoxycarbonyl)amino]-2H-indazol-2-carboxylat

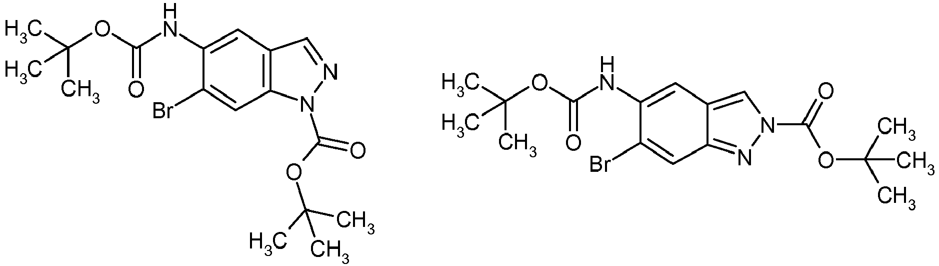

27.5 g (126.1 mmol) Di-tert-butyldicarbonat wurde in 53.5 ml Tetrahydrofuran gelöst und auf 0°C gekühlt. Nach Zugabe von 5.35 g (25.2 mmol) 6-Brom-1H-indazol-5-amin (CAS Nr: 1360928-41-1) bei 0°C wurde das Gemisch anschließend für 24 h bei 80°C gerührt. Die Reaktionsmischung wurde eingeengt, mit Dichlormethan versetzt, mit 0.5 M Salzsäure und gesättigter Natriumchloridlösung gewaschen, über Natriumsulfat getrocknet und während des Einengens auf Isolute® HM-N (Biotage) adsorbiert. Die Isolute wurde auf eine mit Hexan vorequilibrierte Biotage SNAP Kartusche (340 g; KP-Sil) gegeben und mittels Isolera® Flash-Aufreinigungssystem (Biotage) die Chromatographie durchgeführt (Laufmittel: Hexan-Ethylacetat; Gradient: isokratisch 80:20 (9 SV), Man erhielt 7.07 g (68 % d. Th.) des regioisomeren Produktgemisches. (Verhältnis: 1-Isomer/2-Isomer: 85%/15%)
UPLC-MS (Methode A2): Rₜ = 1.48 min
MS (ESIneg): m/z = 410 (M(⁷⁹Br)-H)⁺

### Intermediat 12-1

### tert-Butyl-5 -amino-6-chlor-1H-indazol-1-carboxylat

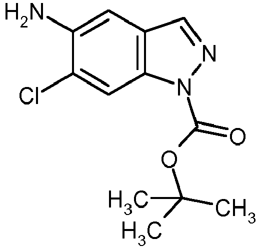

1.80 g (10.7 mmol) 6-Chlor-1H-indazol-5-amin (Cas Nr. 221681-75-0) in 18 ml Tetrahydrofuran wurden mit 2.1 ml (11.8 mmol) *N,N-*Diisopropylethylamin und 2.34 g (10.7 mmol) Di-tert-butyldicarbonat versetzt und man rührte 18 h bei 25°C. Man engte ein und der Rückstand wurde in Ethylacetat aufgenommen und unter Einengen auf Isolute adsorbiert. Die Isolute wurde auf eine mit Hexan vorequilibrierte Biotage SNAP Kartusche (100 g; KP-Sil) gegeben und mittels Isolera® Flash-Aufreinigungssystem (Biotage) die Chromatographie durchgeführt (Laufmittel: Hexan-Ethylacetat; Flussrate: 50 ml/min; Gradient: isokratisch 100:0 (5min), 100:0->75:25 (20 min), isokratisch 75:25 (5min), 75:25->50:50 (15 min), isokratisch 50:50 (5min), 50:50->0:100 (15 min)). Die vereinigten Produktfraktionen wurden eingeengt und im Vakuum getrocknet. Man erhielt 1.23 g (43% der Theorie) der Titelverbindung.
UPLC-MS (Methode A1): Rₜ = 1.16 min
MS (ESIpos): m/z = 268 (M+H)⁺

### Intermediat 12-2

### tert-Butyl-5-amino-6-chlor-2H-indazol-2-carboxylat

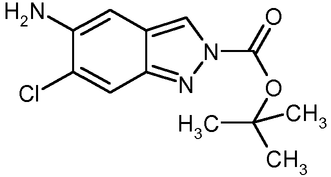

Analog zur Herstellung von Intermediat 12-1 wurden 7.5 g 6-Chlor-1H-indazol-5-amin (Cas Nr. 221681-75-0) umgesetzt. Durch Aufreinigung per säulenchromatographischer Reinigung an Kieselgel (Hexan/Ethylacetat) wurden 1.0 g der Titelverbindung isoliert.
¹H-NMR (500MHz, DMSO-d6): δ = 1.62 (s, 9H), 5.33 (s, 2H), 6.79 (s, 1H), 7.74 (s, 1H), 8.50 (d, 1H).

### Intermediat 13

### tert-Butyl-6-chlor-5-({[6-(trifluormethyl)pyridin-2-yl]carbonyl}amino)-1H-indazol-1-carboxylat

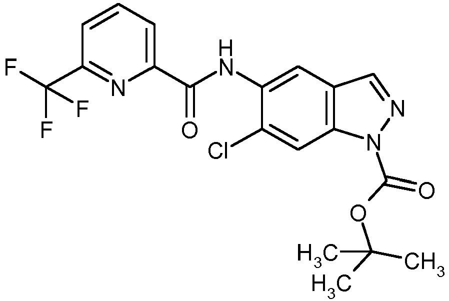

Analog zu Intermediat 5-1 wurden 1.23 g (4.59 mmol) tert-Butyl-5-amino-6-chlor-1H-indazol-1-carboxylat (Intermediat 12-1) in 20 ml *N,N*-Dimethylformamid mit 1.14 g (5.97 mmol) 6-(Trifluormethyl)pyridin-2-carbonsäure für 72 h bei 25°C gerührt. Man versetzte mit Wasser, ließ 15 min rühren, saugte den Feststoff ab und wusch den Feststoff drei Mal mit Wasser und trocknete im Vakuum. Es wurden 2.02 g (98% der Theorie) der Titelverbindung erhalten.
UPLC-MS (Methode A1): Rₜ = 1.57 min
MS (ESIpos): m/z = 441 (M+H)⁺
¹H-NMR (300MHz, DMSO-d6): δ = 1.65 (s, 9H), 8.19 - 8.27 (m, 2H), 8.37 - 8.53 (m, 3H), 8.75 (s, 1H), 10.59 (s, 1H).

### Intermediat 14-1

### N-(6-Chlor-1H-indazol-5-yl)-6-(trifluormethyl)pyridin-2-carboxamid

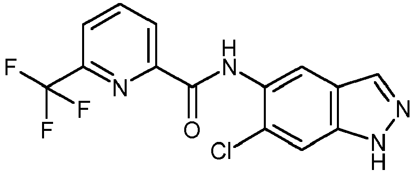

Analog zu Intermediat 6-1 wurden 3.85 g (8.73 mmol) tert-Butyl-6-chlor-5-({[6-(trifluormethyl)pyridin-2-yl]carbonyl}amino)-1H-indazol-1-carboxylat (Intermediat 13) in 40 ml Dichlormethan mit 6.7 ml (8.73 mmol) Trifluoressigsäure versetzt und die Mischung wurde bei 25°C für 18 h gerührt. Nach Aufarbeitung erhielt man 2.98 g (100% der Theorie) der Titelverbindung.
UPLC-MS (Methode A1): Rₜ = 1.18 min
MS (ESIpos): m/z = 341 (M+H)⁺
¹H-NMR (300MHz, DMSO-d6): δ = 7.83 (s, 1H), 8.14 - 8.27 (m, 2H), 8.36 - 8.49 (m, 2H), 8.60 (s, 1H), 10.50 (br. s., 1H), 13.25 (br. s., 1H).

### Intermediat 14-2

### N-(6-Methoxy-1H-indazol-5-yl)-6-(trifluoromethyl)pyridine-2-carboxamid

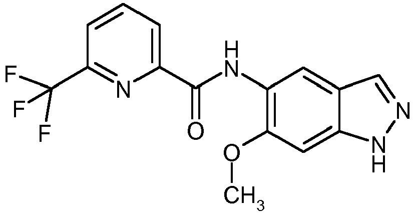

3.84 g (23.5 mmol) 6-Methoxy-1H-indazol-5-amin (CAS Nr.: 749223-61-8) und 4.95 g (25.9 mmol) 6-(Trifluoromethyl)pyridin-2-carbonsäure wurden in 150 ml Tetrahydrofuran gelöst und bei 25°C mit 3.60 g (23.5 mmol) 1-Hydroxy-1H-benzotriazol Hydrat, 9.02 g (47.1 mmol) 1-(3-Dimethylaminopropyl)-3-ethylcarbodiimidhydrochlorid und 9.84 ml (70.6 mmol) Triethylamin versetzt. Die Lösung wurde für 24 h bei 25°C gerührt. Nach dem Einengen der Lösung wurde in Ethylacetat aufgenommen, mit Wasser versetzt und die wässrige Phase drei Mal mit Ethylacetat extrahiert. Die vereinigten, organischen Phasen wurden mit gesättigter Natriumchloridlösung gewaschen, über Natriumsulfat getrocknet und nach Filtration wurde die Lösung eingeengt. Der Rückstand wurde in Dichlormethan aufgenommen, Isolute® HM-N (Biotage) hinzugegeben und unter Einengen auf Isolute adsorbiert. Die Isolute wurde auf eine mit Hexan vorequilibrierte Biotage SNAP Kartusche (340 g; KP-Sil) gegeben und mittels Isolera® Flash-Aufreinigungssystem (Biotage) die Chromatographie durchgeführt (Laufmittel: Hexan-Ethylacetat; Gradient 100:0->50:50 (9 SV), isokratisch 50:50 (4 SV) Die vereinigten Produktfraktionen wurden eingeengt und der beige Feststoff im Vakuum getrocknet. Man erhielt 3.75 g (47 % d. Th.) der Titelverbindung.
UPLC-MS (Methode A1): Rₜ = 1.12 min
MS (ESIpos): m/z = 337 (M+H)⁺
¹H-NMR (400 MHz, DMSO-d6): δ = 4.01 (s, 3 H), 7.13 (s, 1 H), 8.02 (s, 1 H), 8.21 (dd, 1 H), 8.40 (t, 1 H), 8.47 (d, 1 H), 8.74 (s, 1 H), 10.42 (s, 1 H), 12.91 (s, 1 H).

### Intermediat 14-3

### N-(6-Ethoxy-1H-indazol-5-yl)-6-(trifluormethyl)pyridin-2-carboxamid

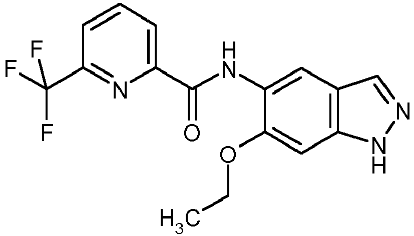

Analog zu Intermediat 5-1 wurden 1.00 g (5.64 mmol) 6-Ethoxy-1H-indazol-5-amin mit 1.29 g (6.77 mmol) 6-(Trifluormethyl)pyridin-2-carbonsäure in 50 ml Tetrahydrofuran für 18 h bei Raumtemperatur umgesetzt. Nach Aufarbeitung und säulenchromatographischer Aufreinigung mittels Isolera® Flash-Aufreinigungssystem (Biotage) (SNAP Kartusche (100 g; KP-Sil), Laufmittel: Hexan-Ethylacetat; Gradient: isokratisch 100:0 (1 SV), 100:0->50:50 (10 SV), isokratisch 50:50 (4.7 SV), 50:50->3:97 (9.4 SV)) erhielt man1.30 g (64 % d. Th.) der Titelverbindung.
UPLC-MS (Methode A1): Rₜ = 1.18 min
MS (ESIpos): m/z = 351 (M+H)⁺
¹H-NMR (500MHz, DMSO-d6): δ = 1.51 (t, 3H), 4.24 (q, 2H), 7.10 (s, 1H), 8.00 (s, 1H), 8.20 (dd, 1H), 8.39 - 8.43 (m, 1H), 8.46 - 8.48 (m, 1H), 8.79 (s, 1H), 10.67 (s, 1H), 12.87 (s, 1H).

### Intermediat 14-4

### N-(1H-Indazol-5-yl)-6-(trifluormethyl)pyridin-2-carboxamid

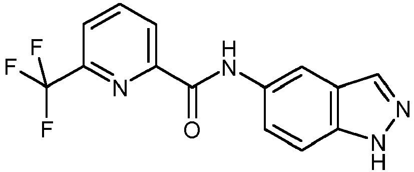

Analog zu Intermediat 5-1 wurden 4.43 g (33.3 mmol) 1H-Indazol-5-amin (CAS Nr.: 19335-11-6) wurden analog mit 7.00 g (36.6 mmol) 6-(Trifluormethyl)pyridin-2-carbonsäure umgesetzt. Man erhielt nach säulenchromatographischer Reinigung an Kieselgel (Hexan/Ethylacetat) 7.8 g (73 % d.Th.) der Titelverbindung.
¹H-NMR (300MHz, DMSO-d₆): δ = 7.51 (d, 1H), 7.68 (dd, 1H), 8.05 (s, 1H), 8.14 (dd, 1H), 8.25 - 8.41 (m, 3H), 10.42 (s, 1H), 13.04 (br. s., 1H).

### Intermediat 14-5

### N-[6-(Benzyloxy)-1H-indazol-5-yl]-6-methylpyridin-2-carboxamid

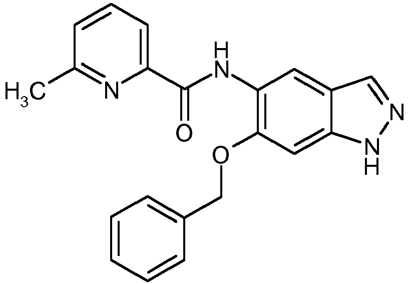

Analog zu Intermediat 14-2 wurden 1.00 g (4.18 mmol) 6-(Benzyloxy)-1H-indazol-5-amin (Intermediat 1-3) und 688 mg (5.02 mmol) 6-Methylpyridin-2-carbonsäure in 50 ml Tetrahydrofuran gelöst und mit 640 mg (4.18 mmol) 1-Hydroxy-1H-benzotriazol Hydrat, 1.60 g (8.36 mmol) 1-(3-Dimethylaminopropyl)-3-ethylcarbodiimidhydrochlorid und 1.75 ml (12.54 mmol) Triethylamin für 24h bei 25°C gerührt. Nach Einengen der Lösung wurde der entstandene Niederschlag mit Wasser versetzt, abgesaugt, mit Wasser und Diethylether gewaschen und im Vakuum getrocknet. Man erhielt 1.13 g (76 % d. Th.) der Titelverbindung.
UPLC-MS (Methode A1): Rₜ = 1.26 min
MS (ESIpos): m/z = 359 (M+H)⁺
¹H NMR (300 MHz, DMSO-d6): δ = 2.43 (s, 3 H), 5.34 (s, 2 H), 7.29 (s, 1 H), 7.35 - 7.57 (m, 4 H), 7.65 (d, 2 H), 7.86 - 8.07 (m, 3 H), 8.84 (s, 1 H), 10.82 (s, 1 H), 12.95 (s, 1 H).

### Intermediat 14-6

### N-(6-Methoxy-1H-indazol-5-yl)-6-methylpyridin-2-carboxamid

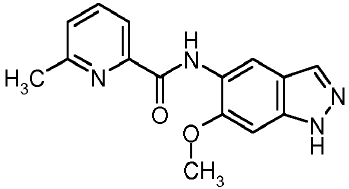

Analog zu Intermediat 14-2 wurden 5.00 g (30.64 mmol) 6-Methoxy-1H-indazol-5-amin (CAS-Nr. 749223-61-8) und 4.62 g (33.70 mmol) 6-Methylpyridin-2-carbonsäure in 100 ml Tetrahydrofuran gelöst und mit 4.69 g (30.64 mmol) 1-Hydroxy-1H-benzotriazol Hydrat, 11.74 g (61.28 mmol) 1-(3-Dimethylaminopropyl)-3-ethylcarbodiimidhydrochlorid und 21.35 ml (153.2 mmol) Triethylamin für 20 h bei 25°C gerührt. Die Reaktionsmischung wurde mit Wasser versetzt und eingeengt. Der entstandene Niederschlag wurde abgesaugt, drei Mal mit Wasser und drei Mal mit Diethylether gewaschen und im Trockenschrank getrocknet. Man erhielt 7.89 g (65 % d. Th.) der Titelverbindung.
UPLC-MS (Methode A1): Rₜ = 0.49 min
MS (ESIpos): m/z = 283 (M+H)⁺.

### Intermediat 14-7

### N-(6-Methoxy-1H-indazol-5-yl)-2-(tetrahydro-2H-pyran-4-yl)-1,3-oxazol-4-carboxamid

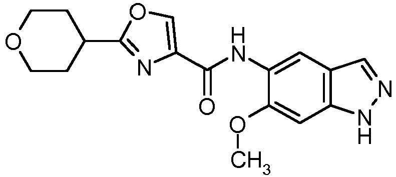

Analog zu Intermediat 14-2 wurden 782 mg (4.80 mmol) 6-Methoxy-1H-indazol-5-amin (CAS-Nr. 749223-61-8) und 1.04 g (5.27 mmol) 2-(Tetrahydro-2H-pyran-4-yl)-1,3-oxazol-4-carbonsäure (CAS-Nr. 955401-82-8) in 15 ml Tetrahydrofuran gelöst und mit 734 mg (4.80 mmol) 1-Hydroxy-1H-benzotriazol Hydrat, 1.84 g (9.59 mmol) 1-(3-Dimethylaminopropyl)-3-ethylcarbodiimidhydrochlorid und 3.34 ml (24.0 mmol) Triethylamin für 26 h bei 25°C gerührt. Die Reaktionsmischung wurde mit Wasser versetzt und eingeengt. Der entstandene Niederschlag wurde abgesaugt, drei Mal mit Wasser und drei Mal mit Diethylether gewaschen und im Trockenschrank getrocknet. Man erhielt 1.19 g (37 % d. Th.) der Titelverbindung.
UPLC-MS (Methode A1): Rₜ = 0.94 min
MS (ESIpos): m/z = 343 (M+H)⁺.

### Intermediat 14-8

### 6-Brom-N-(6-methoxy-1H-indazol-5-yl)pyridin-2-carboxamid

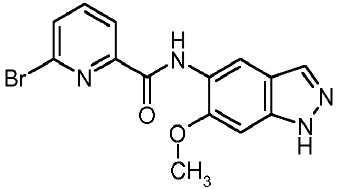

2.0 g (12.26 mmol) 6-Methoxy-1H-indazol-5-amin (CAS-Nr. 749223-61-8) wurden in 50 ml Tetrahydrofuran gelöst, mit 4.72 g (14.71 mmol) *O*-(Benzotriazol-1-yl)-*N,N,N',N'-*tetramethyluroniumtetrafluoroborat und 2.56 ml (14.71 mmol) N,N-Diisopropylethylamin versetzt und für 30 Minuten bei 25°C gerührt. Es wurden 2.56 ml (14.71 mmol) 6-Brompyridin-2-carbonsäure (CAS-Nr. 21190-87-4) hinzugegeben und für weitere 24 h bei 25°C gerührt. Die Reaktionsmischung wurde eingeengt und der Rückstand auf 400 ml Wasser gegeben. Der entstandene Niederschlag wurde abgesaugt, zwei Mal mit Wasser und zwei Mal mit Diethylether gewaschen und für 4 h bei 50°C im Vakuumtrockenschrak getrocknet. Man erhielt 4.18 g (98 % d. Th.) der Titelverbindung.
UPLC-MS (Methode A1): Rₜ = 0.93 min
MS (ESIpos): m/z = 347 (M+H)⁺
¹H NMR (300 MHz, DMSO-d6): δ = 4.02 (s, 3 H) 7.13 (s, 1 H) 7.89 - 8.10 (m, 3 H) 8.20 (dd, 1 H) 8.71 (s, 1 H) 10.22 (s, 1 H) 12.90 (br. s., 1 H).

### Intermediat 14-9

### Methyl-5-({[6-(trifluormethyl)pyridin-2-yl]carbonyl}amino)-1H-indazol-6-carboxylat

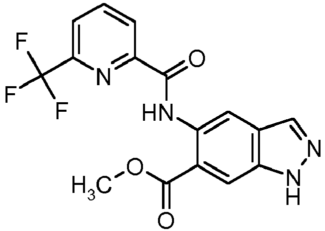

4.5 g (23.53 mmol) Methyl-5-amino-1H-indazol-6-carboxylat (Intermediat 1-6) wurden in 45 ml Tetrahydrofuran gelöst, mit 9.07 g (28.24 mmol) *O*-(Benzotriazol-1-yl)-*N,N,N',N'-*tetramethyluroniumtetrafluoroborat und 4.92 ml (28.24 mmol) N,N-Diisopropylethylamin versetzt und für 30 Minuten bei 25°C gerührt. Es wurden 4.95 g (25.89 mmol) 6-(Trifluormethyl)pyridin-2-carbonsäure (CAS-Nr. 21190-87-4) hinzugegeben und für weitere 24 h bei 25°C gerührt. Die Reaktionsmischung wurde über einen Membranfilter abgesaugt, mit Tetrahydrofuran und Wasser gewaschen und für 24 h bei 50°C im Vakuumtrockenschrank getrocknet. Das Filtrat wurde mit Acetonitril eingeengt und der dabei entstandene Niederschlag abgesaugt, gewaschen und getrocknet. Man erhielt 8.60 g (84 % d. Th.) der Titelverbindung.
UPLC-MS (Methode A1): Rₜ = 1.21 min
MS (ESIpos): m/z = 365 (M+H)⁺
¹H NMR (300 MHz, DMSO-d6): δ = 3.97 (s, 3 H), 8.13 - 8.27 (m, 2 H), 8.30 (s, 1 H), 8.33 - 8.45 (m, 1 H), 8.45 - 8.51 (m, 1 H), 9.15 (s, 1 H), 12.57 (s, 1 H), 13.44 (s, 1 H).

### Intermediat 14-10

### Methyl-5-{[(6-methylpyridin-2-yl)carbonyl]amino}-1H-indazol-6-carboxylat

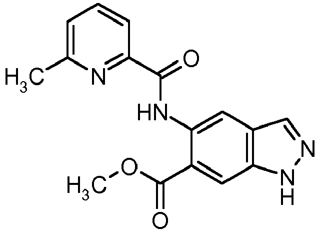

500 mg (2.62 mmol) Methyl-5-amino-1H-indazol-6-carboxylat (Intermediat 1-6) wurden in 5 ml Tetrahydrofuran gelöst, mit 1.01 g (3.14 mmol) *O*-(Benzotriazol-1-yl)-*N,N,N',N'-*tetramethyluroniumtetrafluoroborat und 547 µl (3.14 mmol) N,N-Diisopropylethylamin versetzt und für 30 Minuten bei 25°C gerührt. Es wurden 395 mg (2.88 mmol) 6-Methylpyridin-2-carbonsäure (CAS-Nr. 21190-87-4) hinzugegeben und für weitere 8 h bei 25°C gerührt. Die Reaktionsmischung wurde auf Wasser gegeben, 10 Minuten kräftig gerührt und der Niederschlag über einen Nylonfilter abgesaugt. Der Niederschlag wurde zwei Mal mit Wasser und zwei Mal mit Diethylether gewaschen. Der Feststoff wurde bei 50°C im Vakuumtrockenschrank für 3 h getrocknet. Man erhielt 790 mg (92 % d. Th.) der Titelverbindung.
UPLC-MS (Methode A1): Rₜ = 1.05 min
MS (ESIpos): m/z = 311 (M+H)⁺
¹H NMR (300 MHz, DMSO-d6): δ = 2.65 (s, 3 H), 4.00 (s, 3 H), 7.55 (dd, 1 H), 7.91 - 7.99 (m, 1 H), 7.99 - 8.04 (m, 1 H), 8.23 (s, 1 H), 8.29 (s, 1 H), 9.18 (s, 1 H), 12.65 (s, 1 H), 13.41 (s, 1 H).

### Intermediat 14-11

### N-[6-(2-Hydroxypropan-2-yl)-1H-indazol-5-yl]-6-(trifluormethyl)pyridin-2-carboxamid

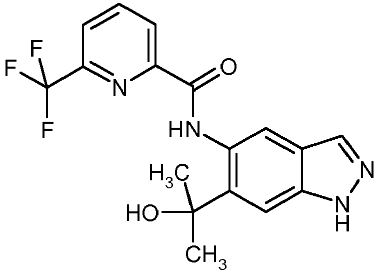

Zu einer eiskalten Lösung aus 1.50 g (4.12 mmol) Methyl-5-({[6-(trifluormethyl)pyridin-2-yl]carbonyl}amino)-1H-indazol-6-carboxylat (Intermediat 14-9) in 20 ml THF wurden vorsichtig 6.9 ml (5 Äquiv.) einer 3M Methylmagnesiumbromid-Lösung in Diethylether gegeben. Man rührte 1 h unter Eisbadkühlung und 19.5 h bei Raumtemperatur. Man gab nochmals 2 Äquiv. der Methylmagnesiumbromid-Lösung zu und ließ weitere 24 h bei Raumtemperatur rühren. Man versetzte mit gesättigter wässriger Ammoniumchloridlösung, rührte die Mischung um und extrahierte dreimal mit Ethylacetat. Die vereinigten organischen Phasen wurden mit Kochsalzlösung gewaschen, über einen wasserabweisenden Filter filtriert und eingeengt. Der Rückstand wurde säulenchromatographisch an Kieselgel (Hexan/Ethylacetat Gradient) gereinigt. Man erhielt 763 mg (45% d. Th.) der Titelverbindung.
¹H-NMR (400MHz, DMSO-d₆): δ [ppm]= 1.63 (s, 6H), 5.99 (s, 1H), 7.49 (s, 1H), 8.06 (s, 1H), 8.14 - 8.19 (m, 1H), 8.37 (t, 1H), 8.46 (d, 1H), 8.78 (s, 1H), 12.32 (s, 1H), 12.97 (s, 1H).

### Intermediat 16-1

### 6-Brom-N-isobutylpyridin-2-amin

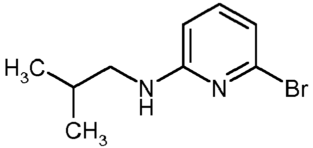

1.0 g 2,6-Dibrompyridin und 340 mg 2-Methylpropan-1-amin und 1.43 ml 2,2,6,6-Tetramethylpiperidin wurde in einem Druckreaktor 16 h bei 190°C gerührt. Man goß auf gesättigte Natriumhydrogencarbonatlösung, extrahierte mit Dichlormethan, wusch mit gesättigter Kochsalzlösung, trocknete über Natriumsulfat und engte ein. Der Rückstand wurde säulenchromatographisch an Kieselgel gereinigt. Man erhielt 920 mg der Titelverbindung.
¹H-NMR (300MHz, CHLOROFORM-d): δ = [ppm]= 1.00 (d, 6H), 1.81 - 1.98 (m, 1H), 3.05 (t, 2H), 4.76 (br. s., 1H), 6.29 (d, 1H), 6.72 (d, 1H), 7.22 - 7.35 (m, 2H).

### Intermediat 17-1

### Methyl-6-(1-hydroxyethyl)pyridin-2-carboxylat

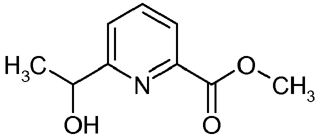

2.00 g 1-(6-Brompyridin-2-yl)ethanol (Telfer, Shane G.; Kuroda, Reiko, Chemistry A European Journal, 2005, 11, 57 - 68) wurden in 20 ml Methanol und 30 ml Dimethylsulfoxid suspendiert. Man addierte 265 mg 1,3-Bis(diphenylphoshino)propan, 140 mg Palladium(II)acetat und 3.2 ml Triethylamin, spülte dreimal mit Kohlenmonoxid und rührte in einer Kohlenmonoxidatmosphäre (12 bar 0.5 h, dann bei 16 bar über Nacht). Man versetzte mit Wasser, extrahierte mit Ethylacetat und engte ein. Man erhielt 1.7 g Methyl-6-(1-hydroxyethyl)pyridin-2-carboxylat als Öl (Rohprodukt).
¹H-NMR (400MHz, CHLOROFORM-d): δ = 1.57 (d, 3H), 4.02 (s, 3H), 5.03 (q, 1H), 7.56 (d, 1H), 7.88 (t, 1H), 8.05 (d, 1H).

### Intermediat 17-2

### Methyl-6-(2,2,2-trifluor-1-hydroxyethyl)pyridin-2-carboxylat

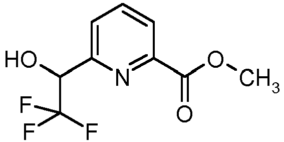

1.04 g (4.06 mmol) 1-(6-Brompyridin-2-yl)-2,2,2-trifluorethanol (CAS 1093880-21-7) wurden analog zu Intermediat 17-1 in einer Kohlenmonoxidatmosphäre umgesetzt. Nach analoger Aufarbeitung wurde das Rohprodukt durch präparative HPLC gereinigt. Man erhielt 696 mg der Titelverbindung.
¹H-NMR (300MHz, DMSO-d₆): δ = 3.89 (s, 3H), 5.15 - 5.28 (m, 1H), 7.18 - 7.25 (m, 1H), 7.86 (dd, 1H), 8.05 - 8.14 (m, 2H).

### Intermediat 17-3

### Methyl-6-(2-hydroxypropan-2-yl)pyridin-2-carboxylat

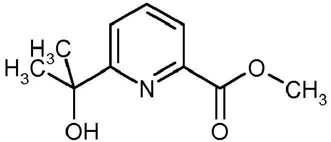

1.00 g 2-(6-Brompyridin-2-yl)propan-2-ol wurden analog zu Intermediat 17-1 in einer Kohlenmonoxidatomosphäre umgesetzt. Nach analoger Aufarbeitung wurde das Rohprodukt durch präparative HPLC gereinigt. Man erhielt 540 mg der Titelverbindung.
¹H-NMR (400MHz, DMSO-d₆): δ [ppm]= 1.44 (s, 6H), 3.86 (s, 3H), 5.34 (s, 1H), 7.86 - 7.99 (m, 3H).

### Intermediat 17-4

### Methyl-6- {[1 -(tert-butoxycarbonyl)azetidin-3 -yl] amino} pyridin-2-carboxylat

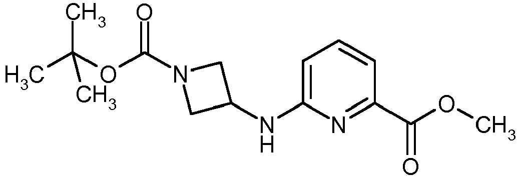

Ein Mischung aus 250 mg Methyl-6-fluorpyridin-2-carboxylat, 361 mg tert-Butyl-3-aminoazetidin-1-carboxylat (1.3 Äquivalente) und 0.84 ml N-Ethyl-N-isopropylpropan-2-amin in 3.0 ml 1-Methylpyrrolidin-2-on wurde bei 80°C gerührt. Man gab erneut 0.5 Äquivalente tert-Butyl-3-aminoazetidin-1-carboxylat zu und rührte bei 100°C über Nacht. Man addierte erneut 0.5 Äquivalente tert-Butyl-3-aminoazetidin-1-carboxylat und rührte bei Raumtemperatur 3 Tage. Man versetzte mit Wasser, extrahierte mit Ethylacetat, engte die organischen Phasen ein und reinigte den Rückstand durch präparative HPLC. Man erhielt 230 mg der Titelverbindung.
UPLC-MS (Methode A1): Rt = 1.07 min (UV-Detektor TIC), gefundene Masse 307.15.

### Intermediat 17-5

### Methyl-6-({[1-(tert-butoxycarbonyl)azetidin-2-yl]methyl}amino)pyridin-2-carboxylat

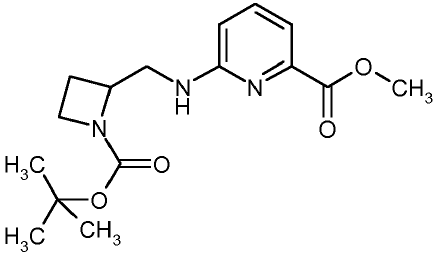

Ein Mischung aus 500 mg Methyl-6-fluorpyridin-2-carboxylat, 720 mg tert-Butyl-2-(aminomethyl)azetidin-1-carboxylat und 2.2 ml N-Ethyl-N-isopropylpropan-2-amin in 7.5 ml 1-Methylpyrrolidin-2-on wurde 30 min bei 100°C, 4 h bei 120°C und 3 h bei 140°C gerührt. Man engte ein und reinigte durch präparative HPLC (Säule: Reprospher C18-DE 5µm 125x30 mm, Lösemittelsystem: A = Wasser + 0.1% Vol. Ameisensäure (99%), B = Acetonitril, Gradient 0 - 5.5 min 40-80% B). Man erhielt 230 mg der Titelverbindung als Rohprodukt. Gefundene Masse (UV-Detektor TIC) 321.17.

### Intermediat 17-6

### Methyl-6-[(2R,6S)-2,6-dimethylmorpholin-4-yl]pyridin-2-carboxylat

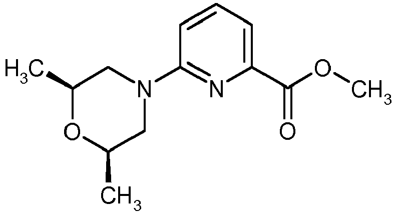

300 mg Methyl-6-fluorpyridin-2-carboxylat wurden analog mit 334 mg (2R,6S)-2,6-Dimethylmorpholin analog zu Intermediat 17-4 über Nacht bei 80°C umgesetzt. Man addiert erneut 0.5 Äquivalente (2R,6S)-2,6-Dimethylmorpholin und rührte 7 h bei 100°C. Nach wässriger Aufarbeitung erhielt man 875 mg eines Rohproduktes, welches noch 1-Methylpyrrolidin-2-on enthielt. UPLC-MS (Methode A1): Rt = 1.05 min (UV-Detektor TIC), gefundene Masse 250.00.

### Intermediat 17-7

### Methyl-6-(isobutylamino)pyridin-2-carboxylat

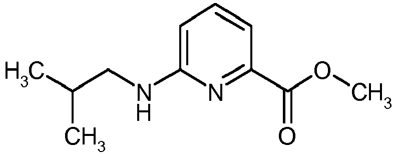

900 mg 6-Brom-N-isobutylpyridin-2-amin (Intermediat 16-1) wurden analog zu Intermediat 17-1 in einer Kohlenmonoxidatmosphäre umgesetzt. Das Rohprodukt wurde durch säulenchromatographische Reinigung an Kieselgel gereinigt. Man erhielt 796 mg der Titelverbindung.
¹H-NMR (300MHz, CHLOROFORM-d): d [ppm]= 1.02 (d, 3H), 1.83 - 1.98 (m, 1H), 3.08 (t, 2H), 3.97 (s, 3H), 4.97 (br. s., 1H), 6.58 (d, 1H), 7.42 (d, 1H), 7.58 (t, 1H).

### Intermediat 19-1

### Kalium-6-(1-hydroxyethyl)pyridin-2-carboxylat

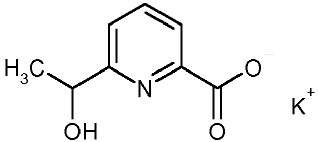

541 mg Methyl-6-(1-hydroxyethyl)pyridin-2-carboxylat (Intermediat 17-1, Rohprodukt) wurden in 5 ml Methanol vorgelegt, man addierte 120 mg Kaliumhydroxid und rührte bei 50°C über Nacht. Man addierte nochmals Kaliumhydroxid und rührte 5 h bei 50°C. Man engte ein und erhielt 625 mg Kalium-6-(1-hydroxyethyl)pyridin-2-carboxylat als Rohprodukt.

### Intermediat 19-2

### 6-(1-Methyl-1H-pyrazol-4-yl)pyridin-2-carbonsäure

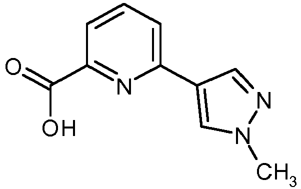

500 mg (2.31 mmol) Methyl-6-brompyridin-2-carboxylat, 578 mg (1.2 Äquiv.) 1-Methyl-4-(4,4,5,5-tetramethyl-1,3,2-dioxaborolan-2-yl)-1H-pyrazol und 192 mg Lithiumchlorid wurden in 5 ml Toluol und 3 ml Ethanol vorgelegt. Man addierte 162 mg Bis(triphenylphosphin)palladium(II)chlorid und 3.5 ml wässrige Natriumcarbonatlösung (2 M) und erwärmte bei 120°C in der Mikrowelle. Man säuerte mit 10 prozentiger Zitronensäurelösung auf pH 5 an, extrahierte dreimal mit Ethylacetat, wusch mit Kochsalzlösung, filtrierte und engte ein. Der Rückstand wurde durch präparative HPLC (Säule XBrigde C18 5µm 100x30 mm) gereinigt. Man erhielt 70 mg (15 % d. Th.) der Titelverbindung.
¹H-NMR (400MHz, DMSO-d₆): δ = 3.89 (s, 3H), 7.79 - 7.94 (m, 3H), 8.09 (s, 1H), 8.39 (s, 1H), (12.9 br. s, 1H).

### Intermediat 19-3

### 6-(1-Methyl-1H-pyrazol-5-yl)pyridin-2-carbonsäure

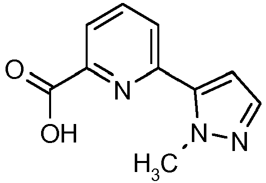

500 mg (2.31 mmol) Methyl-6-brompyridin-2-carboxylat wurden analog zur Synthese von Intermediat 19-2 mit 1-Methyl-5-(4,4,5,5-tetramethyl-1,3,2-dioxaborolan-2-yl)-1H-pyrazol bei 120°C innerhalb von 90 min in der Mikrowelle umgesetzt. Nach präparativer HPLC-Aufreinigung nach der Methode P1 wurden 34 mg (15 % d. Th.) der Titelverbindung erhalten.
¹H-NMR (400MHz, DMSO-d₆): δ = 4.22 (s, 3H), 6.89 (d, 1H), 7.50 (d, 1H), 7.96 - 8.10 (m, 3H), 13.29 (br. s., 1H).

### Intermediat 19-4

### 6-(1H-Pyrazol-4-yl)pyridin-2-carbonsäure

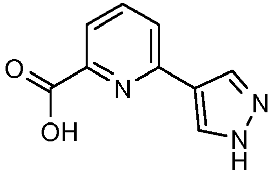

1 g (2.31 mmol) 6-Brompyridin-2-carbonsäure und 1.15 g 4-(4,4,5,5-Tetramethyl-1,3,2-dioxaborolan-2-yl)-1H-pyrazol wurden analog zur Synthese von Intermediat 19-2 bei 120°C innerhalb von 90 min in der Mikrowelle umgesetzt. Man addierte Ethylacetat und Wasser, filtrierte, trennte die organische Phase ab und extrahierte zweimal mit Ethylacetat. Die Ethylacetatphasen wurden verworfen. Man addierte zur wässrigen Phase 10 prozentige Zitronensäurelösung bis zu einem pH-Wert von 4, extrahierte dreimal mit Ethylacetat und engte die Ethylacetatphasen ein. Man erhielt einen Rückstand der durch präparative HPLC (Säule XBrigde C18) gereinigt wurde. Man erhielt 110 mg (12 % d. Th.) der Titelverbindung.
¹H-NMR (300MHz, DMSO-d₆): δ = 7.77 - 7.98 (m, 3H), 8.31 (s, 2H), 13.03 (br. s., 2H).

### Intermediat 19-5

### 5-Fluor-6-(1-methyl-1H-pyrazol-4-yl)pyridin-2-carbonsäure

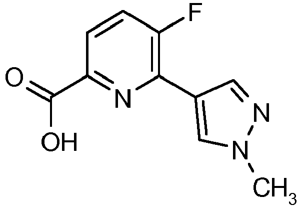

500 mg Methyl-6-brom-5-fluorpyridin-2-carboxylat wurden analog mit 533 mg (1.2 Äquiv.) 1-Methyl-4-(4,4,5,5-tetramethyl-1,3,2-dioxaborolan-2-yl)-1H-pyrazol 90 min bei 120°C in der Mikrowelle umgesetzt. Man erhielt 380 mg (80 % d. Th.) der Titelverbindung als Rohprodukt.
UPLC-MS (Methode A1): Rₜ = 0.72 min
MS (ESIpos): m/z = 222 (M+H)⁺

### Intermediat 19-6

### 6-(1,3-Dimethyl-1H-pyrazol-4-yl)pyridin-2-carbonsäure

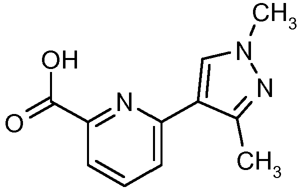

500 mg (2.31 mmol) Methyl-6-brompyridin-2-carboxylat wurden analog zur Synthese von Intermediat 19-2 mit 617 mg 1,3-Dimethyl-4-(4,4,5,5-tetramethyl-1,3,2-dioxaborolan-2-yl)-1H-pyrazolbei 120°C innerhalb von 90 min in der Mikrowelle umgesetzt. Nach Reinigung durch HPLC wurden 66 mg (13 % d. Th.) der Titelverbindung erhalten.
¹H-NMR (400MHz, DMSO-d₆): δ = 2.47 (s), 3.80 (s, 3H), 7.71 - 7.81 (m, 2H), 7.88 - 7.94 (m, 1H), 8.27 (s, 1H), 12.95 (br. s., 1H).

### Intermediat 19-7

### 6-(3-Methyl-1H-pyrazol-4-yl)pyridin-2-carbonsäure

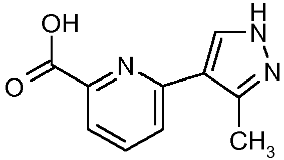

216 mg Methyl-6-brompyridin-2-carboxylat wurden analog zur Synthese von Intermediat 19-2 mit 250 mg 3-Methyl-4-(4,4,5,5-tetramethyl-1,3,2-dioxaborolan-2-yl)-1H-pyrazol 90 min bei 120°C in der Mikrowelle umgesetzt. Man erhielt nach Reinigung per HPLC 68 mg (33 % d. Th.) der Titelverbindung vermischt mit Methyl-6-(3-methyl-1H-pyrazol-4-yl)pyridin-2-carboxylat.
UPLC-MS (Methode A1): Rₜ = 0.50 min
MS (ESIpos): m/z = 204 (M+H)⁺

### Intermediat 19-8

### 6-[3-(Methylsulfonyl)phenyl]pyridin-2-carbonsäure

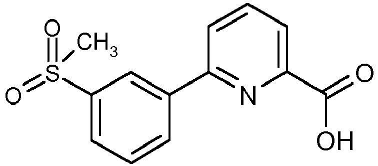

500 mg (2.31 mmol) Methyl-6-brompyridin-2-carboxylat, 694 mg (1.5 Äquiv.) [3-(Methylsulfonyl)phenyl]boronsäure und wurden in 10 ml DMSO vorgelegt. Man addierte 267 mg Tetrakis(triphenylphosphin)palladium(0), 736 mg Natriumcarbonat und 2 ml Wasser und erwärmte 2h bei 110°C in der Mikrowelle. Man verdünnte mit Wasser, säuerte mit 10 prozentiger Zitronensäurelösung auf pH 4 an, setzte Ethylacetat zu, filtriert, trennte die Phasen des Filtrates, extrahierte mit Ethylacetat, wusch mit Kochsalzlösung und engte ein. Man gab 2.5 ml Methanol und 917 mg Lithiumhydroxid Monohydrat in 10 ml Wasser zu und rührte bei Raumtemperatur 5 h. Man verdünnte mit Wasser, säuerte mit 10 prozentiger Zitronensäurelösung auf pH 4 an, extrahierte mit Ethylacetat, wusch mit Kochsalzlösung und engte ein. Man erhielt 776 mg der Titelverbindung als Rohprodukt.
UPLC-MS (Methode A1): Rₜ = 0.75 min
MS (ESIpos): m/z = 278 (M+H)⁺

### Intermediat 19-9

### 6-[3-(Trifluormethyl)-1H-pyrazol-4-yl]pyridin-2-carbonsäure

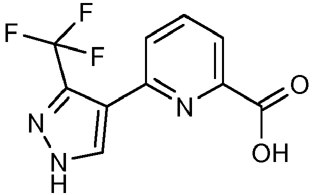

250 mg Methyl-6-brompyridin-2-carboxylat wurden analog zur Herstellung von Intermediat 19-8 mit 394 mg 4-(4,4,5,5-Tetramethyl-1,3,2-dioxaborolan-2-yl)-3-(trifluormethyl)-1H-pyrazol umgesetzt. Man erhielt 442 mg der Titelverbindung als Rohprodukt.
UPLC-MS (Methode A1): Rₜ = 0.82 min
MS (ESIpos): m/z = 258 (M+H)⁺

### Intermediat 19-10

### Kalium-6-(2,2,2-trifluor-1 -hydroxyethyl)pyridin-2-carboxylat

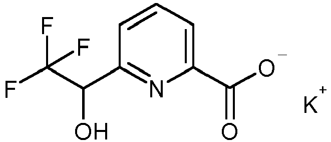

Zu 693 mg Methyl-6-(2,2,2-trifluor-1-hydroxyethyl)pyridin-2-carboxylat (Intermediat 17-2) in 5.0 ml Methanol wurden 165 mg Kaliumhydroxid gegeben und man rührte 20 h bei 50°C. Nach Einengen erhielt man 787 mg eines Feststoffes, der ohne weitere Aufreinigung weiter eingesetzt wurde.

### Intermediat 19-11

### Kalium-6-(2-hydroxypropan-2-yl)pyridin-2-carboxylat

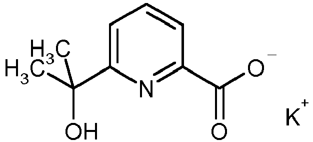

Analog zu Intermediat 19-10 wurden 535 mg Methyl-6-(2-hydroxypropan-2-yl)pyridin-2-carboxylat (Intermediat 17-3) mit 0.28 g Kaliumhydroxid in 6.0 ml Methanol bei 50°C umgesetzt. Man erhielt nach Eineingen 876 mg der Titelverbindung als Rohprodukt.

### Intermediat 19-12

### 6- {[1-(tert-Butoxycarbonyl)azetidin-3-yl]amino}pyridin-2-carbonsäure

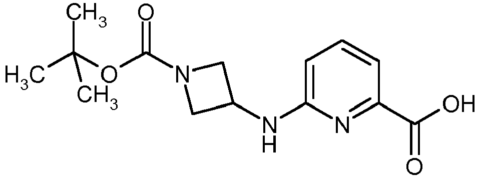

Zur einer Mischung aus 230 mg Methyl-6-{[1-(tert-butoxycarbonyl)azetidin-3-yl]amino}pyridin-2-carboxylat (Intermediat 17-4) in 4.0 ml THF addierte man 0.31 g Lithiumhydroxid Monohydrat gelöst in 1.0 ml Wasser und 0.5 ml Ethanol und rührte bei Raumtemperatur über Nacht. Man verdünnte mit Wasser, säuerte mit Zitronensäurelösung auf einen pH-Wert von 6 an, extrahierte mit Ethylacetat, wusch mit gesättigter Kochsalzlösung, filtrierte über einen wasserabweisenden Filter und engte ein. Man erhielt 202 mg eines Öles, welches ohne weitere Aufreinigung eingesetzt wurde.

### Intermediat 19-13

### Kalium-6-({[1-(tert-butoxycarbonyl)azetidin-2-yl]methyl}amino)pyridin-2-carboxylat

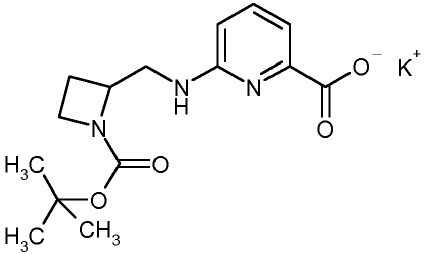

Zu 230 mg Methyl-6-({[1-(tert-butoxycarbonyl)azetidin-2-yl]methyl}amino)pyridin-2-carboxylat (Intermediat 17-5) in 3.0 ml Ethanol addierte man 24 mg Kaliumhydroxid und rührte bei 50°C über Nacht. Man engte ein und erhielt 265 mg eines Rohproduktes, welches ohne Reinigung weiter eingesetzt wurde.

### Intermediat 19-14

### 6-[(2R,6S)-2,6-Dimethylmorpholin-4-yl]pyridin-2-carbonsäure

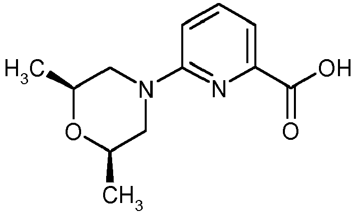

875 mg Methyl-6-[(2R,6S)-2,6-dimethylmorpholin-4-yl]pyridin-2-carboxylat (Intermediat 17-6) wurden in 5 ml THF und 1 ml Methanol vorgelegt, man addierte 698 mg Lithiumhydroxid Monohydrat in 2.5 ml Wasser und rührte bei Raumtemperatur über Nacht. Man versetzte zweimal mit Toluol und engte jeweils ein. Man versetzte mit Methanol, rührte und filtrierte den Feststoff ab, wusch mit Diethylether, engte das Filtrat ein und reinigte durch präparative HPLC (Methode P1). Man erhielt 224 mg der Titelverbindung.
UPLC-MS (Methode A1): Rt = 0.66 min (UV Detektor TIC), gefundene Masse 236.12.

### Intermediat 19-15

### Kalium-6-(isobutylamino)pyridin-2-carboxylat

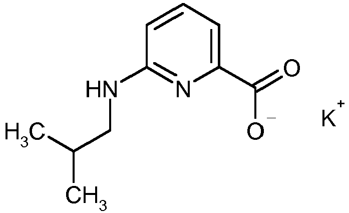

Man versetzte eine Lösung aus 790 mg Methyl-6-(isobutylamino)pyridin-2-carboxylat (Intermediat 17-7) in 3.4 ml Wasser, 32 ml THF und 3.2 ml Methanol mit 454 mg Lithiumhydroxid und rührt bei Raumtemperatur über Nacht. Man erhielt nach Einengen 1.15 g eines Feststoffes, der ohne weitere Reinigung eingesetzt wurde.
UPLC-MS (Methode A1): Rt = 0.58 min (UV Detektor TIC), gefundene Masse 194.00.

### Intermediat 20-1

### 6-Brom-N-{6-methoxy-2-[2-(morpholin-4-yl)-2-oxoethyl]-2H-indazol-5-yl}pyridin-2-carboxamid

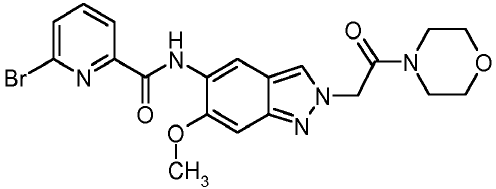

Analog zu Intermediat 8-6 wurden 1.00 g (2.47 mmol) (5-{[(6-Brompyridin-2-yl)carbonyl]amino}-6-methoxy-2H-indazol-2-yl)essigsäure (Intermediat 9-21) mit 258 µl (2.96 mmol) Morpholin, 378 mg (2.47 mmol) 1-Hydroxy-1H-benzotriazol-Hydrat und 946 mg (4.94 mmol) 1-(3-Dimethylaminopropyl)-3-ethylcarbodiimidhydrochlorid und 1.03 ml (7.40 mmol) Triethylamin in 35 ml Tetrahydrofuran bei 25°C für 24 h gerührt. Die Reaktionsmischung wurde eingeengt, mit Wasser versetzt und der enstandene Niederschlag abgesaugt, mit Wasser und Diethylether gewaschen und im Vakuum getrocknet. Man erhielt 586 mg (50 % d.Th.) der Titelverbindung.
UPLC-MS (Methode A1): Rₜ = 1.07 min
MS (ESIpos): m/z = 474 (M+H)⁺
¹H NMR (400 MHz, DMSO-d6): δ = 3.47 (d, 2 H), 3.58 (br. s., 4 H), 3.64 (d, 2 H), 4.00 (s, 3 H), 5.40 (s, 2 H), 7.93 - 7.99 (m, 1 H), 8.05 (t, 1 H), 8.14 - 8.29 (m, 2 H), 8.68 (s, 1 H), 10.31 (s, 1 H).

### Intermediat 21-1

### tert-Butyl-4- {[5-({[6-(trifluormethyl)pyridin-2-yl]carbonyl}amino)-2H-indazol-2-yl] acetyl} piperazin-1 -carboxylat

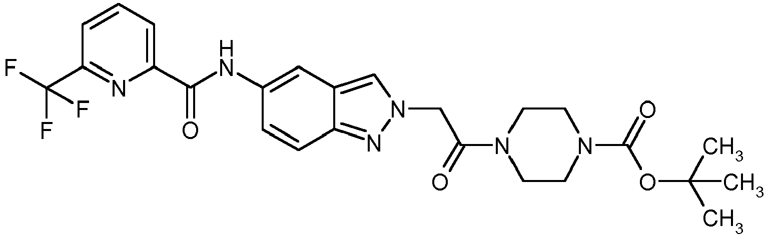

1.30 g (3.57 mmol) [5-({[6-(Trifluormethyl)pyridin-2-yl]carbonyl}amino)-2H-indazol-2-yl]essigsäure (Intermediat 9-14) wurde in 50 ml Tetrahydrofuran und 5.4 ml *N,N-*Dimethylformamid und für 30 Minuten bei 25°C gerührt. Anschließend wurden 997 mg (5.35 mmol) tert-Butylpiperazin-l-carboxylat, 546 mg (3.57 mmol) 1-Hydroxy-1H-benzotriazol Hydrat und 1.37 g (7.14 mmol) 1-(3-Dimethylaminopropyl)-3-ethylcarbodiimidhydrochlorid hinzugegeben und für weitere 24 h bei 25°C gerührt. Die Reaktionsmischung wurde auf Wasser gegeben. Der erhaltene Feststoff wurde abgesaugt und zwei Mal mit Wasser nachgewaschen. Der Feststoff wurde in Dichlormethan aufgenommen, über Natriumsulfat getrocknet, filtriert und eingeengt. Der gelbe Feststoff wurde im Vakuum getrocknet. Man erhielt 1.78 g (94 % d. Th.) tert-Butyl-4-{[5-({[6-(trifluormethyl)pyridin-2-yl]carbonyl}amino)-2H-indazol-2-yl]acetyl}piperazin-1-carboxylat.
UPLC-MS (Methode A1): Rₜ = 1.21 min
MS (ESIpos): m/z = 533 (M+H)⁺

### Intermediat 22-1

### N-{2-[2-Oxo-2-(piperazin-1-yl)ethyl]-2H-indazol-5-yl}-6-(trifluormethyl)pyridin-2-carboxamid

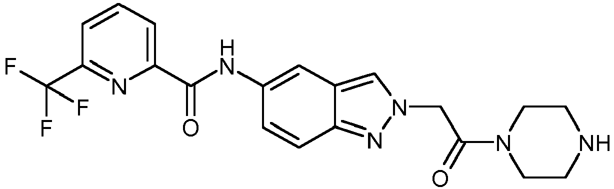

Man addierte 1.93 ml (25.08 mmol) Trifluoressigsäure zu 1.78 g (3.34 mmol) tert-Butyl-4-{[5-({[6-(trifluormethyl)pyridin-2-yl]carbonyl}amino)-2H-indazol-2-yl]acetyl}piperazin-1-carboxylat (Intermediat 21-1) in 11 ml Dichlormethan und rührte 24 h bei 25°C. Dann goss man auf gesättigte Natriumhydrogencarbonatlösung. Die entstandene Suspension wurde filtriert und mit 30 ml Wasser sowie 10 ml Diethylether nachgewaschen. Nach trocknen im Vakuum erhielt man 1.41 g (97 % d. Th.) N-{2-[2-Oxo-2-(piperazin-1-yl)ethyl]-2H-indazol-5-yl}-6-(trifluormethyl)pyridin-2-carboxamid als Rohprodukt erhalten.
UPLC-MS (Methode A1): Rₜ = 0.80 min
MS (ESIpos): m/z = 433 (M+H)⁺
¹H NMR (400 MHz, DMSO-d6): δ = 2.66 (br. s., 2 H), 2.73 (br. s., 2 H), 3.39 (br. s., 2 H), 3.47 (br. s., 2 H), 5.44 (s, 2 H), 7.47 - 7.68 (m, 2 H), 8.17 (d, J=7.1 Hz, 1 H), 8.30 (s, 2 H), 8.33 - 8.43 (m, 2 H), 10.37 (s, 1 H).

### Intermediat 23-1

### tert-Butyl-5-{[(benzyloxy)carbonyl]amino}-6-chlor-2H-indazol-2-carboxylat

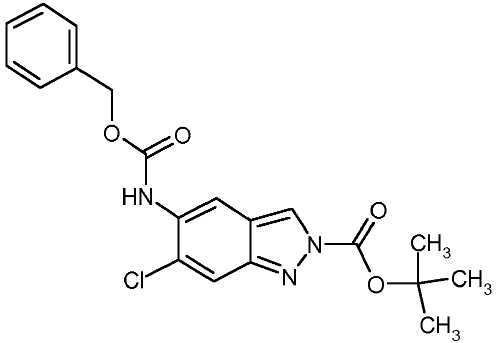

2.09 g tert-Butyl-5-amino-6-chlor-2H-indazol-2-carboxylat (Intermediat 12-2) in 15 ml THF wurden mit 1.50 ml N-Ethyl-N-isopropylpropan-2-amin und 1.11 ml Benzylcarbonochloridat versetzt und rührt bei Raumtemperatur über Nacht. Man addierte erneut 1.50 ml N-Ethyl-N-isopropylpropan-2-amin und 1.11 ml Benzylcarbonochloridat und rührte 3 Tage bei Raumtemperatur. Man gab erneut 1.50 ml N-Ethyl-N-isopropylpropan-2-amin und 1.11 ml Benzylcarbonochloridat zu und rührte bei Raumtemperatur über Nacht. Man versetzte mit Wasser, extrahierte mit Ethylacetat, wusch mit Kochsalzlösung und engte ein. Man erhielt 4.61 g eines Rohproduktes, welches ohne weitere Aufreinigung weiter eingesetzt wurde.
UPLC-MS (Methode A1): Rt = 1.40 min (UV-TIC), gefundene Masse 401.00.

Die chemische Bezeichnung der Beispiele wurde unter Verwendung der Software von ACD / LABS (Batch Version 12.01.) erstellt.

### Beispiele

### Allgemeine Versuchsvorschrift 1a

1.0 Äquivalente des jeweiligen Intermediates wurden mit 1.0 Äquivalenten 1-Hydroxy-1H-benzotriazol Hydrat, 2.0 Äquivalenten 1-(3-Dimethylaminopropyl)-3-ethylcarbodiimidhydrochlorid, 5.0 Äquivalenten Triethylamin und 1.5 Äquivalenten der jeweiligen Carbonsäure in Tetrahydrofuran 24 h bei 25°C gerührt. Die Reaktionsmischung wurde mit Wasser und Ethylacetat versetzt. Der entstandene Niederschlag wurde abfiltriert, mit Wasser und Diethylether gewaschen und getrocknet.

### Allgemeine Versuchsvorschrift 1b

1.0 Äquivalente des jeweiligen Intermediates wurden mit 1.0 Äquivalenten 1-Hydroxy-1H-benzotriazol Hydrat, 2.0 Äquivalenten 1-(3-Dimethylaminopropyl)-3-ethylcarbodiimidhydrochlorid, 3.0 Äquivalenten Triethylamin und 1.5 Äquivalenten der jeweiligen Carbonsäure in *N,N-*Dimethylformamid 24 h bei 25°C gerührt, wobei eine Suspension entsteht. Der entstandene Niederschlag wurde abfiltriert, zweimal mit N,N-Dimethylformamid und Diethylether gewaschen und getrocknet.

### Allgemeine Versuchsvorschrift 1c

1.0 Äquivalente des jeweiligen Intermediates wurden mit 1.0 Äquivalenten 1-Hydroxy-1H-benzotriazol Hydrat, 2.0 Äquivalenten 1-(3-Dimethylaminopropyl)-3-ethylcarbodiimidhydrochlorid, 5.0 Äquivalenten Triethylamin und 1.3 Äquivalenten der jeweiligen Carbonsäure in Tetrahydrofuran 24 h bei 25°C gerührt. Die Reaktionsmischung wurde mit Wasser versetzt und mehrmals mit Ethylacetat extrahiert. Die vereinigten organischen Phasen wurden eingeengt und der Rückstand mit präparativer HPLC nach Methode P1 gereinigt.

### Allgemeine Versuchsvorschrift 1d

1.0 Äquivalente des jeweiligen Intermediates wurden mit 1.0 Äquivalenten 1-Hydroxy-1H-benzotriazol Hydrat, 2.0 Äquivalenten 1-(3-Dimethylaminopropyl)-3-ethylcarbodiimidhydrochlorid, 3.0 Äquivalenten Triethylamin und 1.2 Äquivalenten der jeweiligen Carbonsäure in 1 ml *N,N-*Dimethylformamid 24 h bei 25°C gerührt. Die Reaktionsmischung wurde mit weiteren 1.5 ml *N,N*-Dimethylformamid verdünnt und mit präparativer HPLC nach Methode P1 gereinigt.

### Allgemeine Versuchsvorschrift 1e

1.0 Äquivalente des jeweiligen Intermediates wurden mit 1.0 Äquivalenten 1-Hydroxy-1H-benzotriazol Hydrat, 2.0 Äquivalenten 1-(3-Dimethylaminopropyl)-3-ethylcarbodiimidhydrochlorid, 4.0 Äquivalenten Triethylamin und 1.2 Äquivalenten der jeweiligen Carbonsäure in 1 ml Tetrahydrofuran 24 h bei 25°C gerührt. Die Reaktionsmischung wurde auf 25 ml Wasser gegossen. Der entstandene Niederschlag wurde abfiltriert, zweimal mit Diethylether gewaschen und im Trockenschrank getrocknet.

**Tabelle 1: Beispiele 1-18**

| Die Beispielverbindungen wurden aus den entsprechenden Intermediaten und Carbonsäuren nach den allgemeinen Versuchsvorschriften 1a - 1e hergestellt. | | | | |
|---|---|---|---|---|
| Bsp Nr. | Struktur/Name | Hergestellt aus | * siehe Legen de | ¹H-NMR / LC-MS |
| 1 | 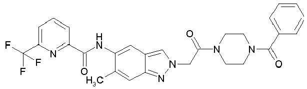 | Intermediat 6-1 und 6-(Trifluorme thyl)pyridin -2-carbonsäur e | la [a] (68%) | (400MHz, DMSO-d6): δ = 2.38 (s, 3H), 3.32 - 3.77 (8H), 5.45 (br. s., 2H), 7.39 - 7.49 (m, 6H), 8.15 - 8.21 (m, 2H), 8.24 (s, 1H), 8.33 - 8.43 (m, 2H), 10.11 (s, 1H). |
| | N-{2-[2-(4-Benzoylpiperazin-1-yl)-2-oxoethyl]-6-methyl-2H-indazol-5-yl}-6-(trifluormethy l)pyridin- 2-carboxamid | | | |
| | | | | UPLC-MS (Methode A1): Rt = 1.16 min |
| | | | | MS (ESIpos): m/z = 551 (M+H)+ |
| 2 | 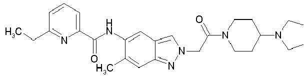 | Intermediat 6-2 und 6-Ethylpyridi n-2-carbonsäur e | la (91%) | (300MHz, DMSO-d6): δ = 1.21 - 1.49 (m, 5H), 1.67 (br. s., 4H), 1.84 (t, 2H), 2.14 - 2.31 (m, 1H), 2.79 - 2.95 (m, 3H), 3.17 (t, 1H), 3.80 - 3.93 (m, 1H), 4.03 - 4.16 (m, 1H), 5.34 - 5.49 (m, 2H), 7.49 (s, 1H), 7.53 - 7.59 (m, 1H), 7.94 - 8.01 (m, 2H), 8.24 (s, 1H), 8.39 (s, 1H), 10.37 (s, 1H). |
| | 6-Ethyl-N-(6-methyl-2-{2-oxo-2-[4-(pyrrolidin-1-yl)piperidin-1-yl]ethyl}-2H-indazol-5-yl)pyridin-2-carboxamid | | | |
| | | | | UPLC-MS (Methode A2): Rt = 1.18 min |
| | | | | MS (ESIpos): m/z = 475 (M+H)+ |
| 3 | 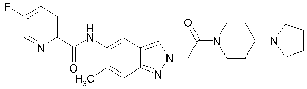 | Intermediat 6-2 und 5-Fluorpyridi n-2-carbonsäur e | 1b (36%) | (400 MHz, DMSO-d6): δ = 1.18 - 1.37 (m, 1 H), 1.37 - 1.51 (m, 1 H), 1.68 (br. s., 4 H), 1.76 - 1.94 (m, 2 H), 2.14 - 2.30 (m, 1 H), 2.39 (s, 3 H), 2.78 - 2.94 (m, 1 H), 3.18 (t, 1 H), 3.82 - 3.95 (m, 1 H), 4.03 - 4.16 (m, 1 H), 5.44 (d, 1 H), 5.39 (d, 1 H), 7.44 - 7.50 (m, 1 H), 7.99 (td, 1 H), 8.12 (s, 1 H), 8.20 - 8.29 (m, 2 H), 8.75 (d, 1 H), 10.15 (s, 1 H). |
| | 5-Fluor-N-(6-methyl-2-{2-oxo-2-[4-(pyrrolidin-1-yl)piperidin-1-yl]ethyl}-2H-indazol-5-yl)pyridine-2-carboxamid | | | |
| | | | | LC-MS (Methode A3): Rt = 0.79 min |
| | | | | MS (ESIpos): m/z = 465 (M+H)+ |
| 4 | 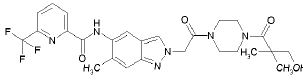 | Intermediat 6-3 und 6-(Trifluorme thyl)pyridin -2-carbonsäur e | la (53%) | (300MHz, DMSO-d6): δ = 1.16 (s, 6H), 2.40 (s, 3H), 3.39 - 3.52 (m, 4H), 3.52 - 3.70 (m, 6H), 4.62 (t, 1H), 5.47 (s, 2H), 7.49 (s, 1H), 8.17 - 8.29 (m, 3H), 8.34 - 8.45 (m, 2H), 10.15 (s, 1H). |
| | N-(2-{2-[4-(3-Hydroxy-2,2-dimethylpropanoyl)piperazin-1-yl]-2-oxoethyl}-6-methyl-2H-indazol-5-yl)-6-(trifluormethyl)pyridin-2-carboxamid | | | |
| | | | | UPLC-MS (Methode A1): Rt = 1.06 min |
| | | | | MS (ESIpos): m/z = 547 (M+H)+ |
| 5 | 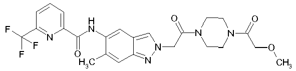 | Intermediat 6-4 und 6-(Trifluorme thyl)pyridin -2-carbonsäur e | la (12%) | (400MHz, DMSO-d6): δ = 2.40 (s, 3H), 3.36 - 3.64 (m, 8H), 4.12 (br. s., 2H), 5.47 (s, 2H), 7.49 (s, 1H), 8.18 - 8.28 (m, 3H), 8.35 - 8.45 (m, 2H), 10.13 (s, 1H). |
| | N-(2-{2-[4-(Methoxyacetyl)piperazin-1-yl]-2-oxoethyl}-6-methyl-2H-indazol-5-yl)-6-(trifluormethyl)pyridin-2-carboxamid | | | |
| | | | | UPLC-MS (Methode A1): Rt = 1.02 min |
| | | | | MS (ESIpos): m/z = 519 (M+H)+ |
| 6 | 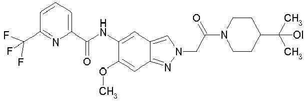 | Intermediat 6-5 und 6-(Trifluorme thyl)pyridin -2-carbonsäur e | 1c (51%) | (300MHz, DMSO-d6): δ = 0.94 - 1.29 (m, 8H, enthält Singulett bei 1.04 ppm), 1.35 - 1.51 (m, 1H), 1.74 (t, 2H), 2.53 (s, 1H), 2.98 (t, 1H), 3.91 - 4.10 (m, 4H), 4.17 (s, 1H), 4.41 (d, 1H), 5.28 - 5.44 (m, 2H), 7.10 (s, 1H), 8.18 - 8.27 (m, 2H), 8.35 - 8.50 (m, 2H), 8.69 (s, 1H), 10.50 (s, 1H). |
| | N-(2- {2-[4-(2-Hydroxypropan-2-yl)piperidin-1-yl]-2-oxoethyl}-6-methoxy-2H-indazol-5-yl)-6-(trifluormethyl)pyridin-2-carboxamid | | | |
| | | | | UPLC-MS (Methode A1): Rt = 1.13 min |
| | | | | MS (ESIpos): m/z = 520 (M+H)+ |
| 7 | 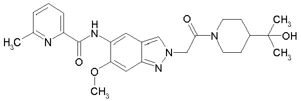 | Intermediat 6-5 und 6-Methylpyri din-2-carbonsäur e | 1c (41%) | (300MHz, DMSO-d6): δ = 0.97 - 1.29 (m, 8H, enthält Singulett bei 1.04 ppm), 1.36 - 1.50 (m, 1H), 1.74 (t, 2H), 2.53 (s, 1H), 2.61 (s, 3H), 2.98 (t, 1H), 3.95 - 4.08 (m, 4H), 4.17 (s, 1H), 4.41 (d, 1H), 5.28 - 5.42 (m, 2H), 7.08 (s, 1H), 7.51 - 7.59 (m, 1H), 7.92 - 8.02 (m, 2H), 8.20 (s, 1H), 8.70 (s, 1H), 10.70 (s, 1H). |
| | N-(2-{2-[4-(2-Hydroxypropan-2-yl)piperidin-1-yl]-2-oxoethyl} -6-methoxy-2H-indazol-5-yl)-6-methylpyridin-2-carboxamid | | | |
| | | | | UPLC-MS (Methode A1): Rt = 1.06 min |
| | | | | MS (ESIpos): m/z = 466 (M+H)+ |
| 8 | 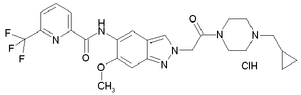 | Intermediat 6-6 und 6-(Trifluorme thyl)pyridin -2-carbonsäur e | 1a (17%) [b] | (400MHz, DMSO-d6): δ = 0.41 (br. s., 2H), 0.66 (d, 2H), 1.12 (br. s., 1H), 2.89 - 3.26 (m, 5H), 3.60 (br. s., 3H), 4.00 (s, 3H), 4.21 (d, 1H), 4.42 (d, 1H), 5.48 (d, 2H), 7.11 (s, 1H), 8.19 - 8.25 (m, 2H), 8.36 - 8.49 (m, 2H), 8.72 (s, 1H), 10.5 (s, 1H), 10.7 (s). |
| | N-(2-{2-[4-(Cyclopropylmethyl)piperazin-1-yl]-2-oxoethyl}-6-methoxy-2H-indazol-5-yl)-6-(trifluormethyl)pyridin-2-carboxamidhydrochlorid | | | |
| | | | | UPLC-MS (Methode A1): Rt = 0.94 min |
| | | | | MS (ESIpos): m/z = 517 (M+H)+ |
| 9 | 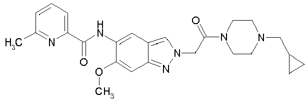 | Intermediat 6-6 und 6-Methylpyri din-2-carbonsäur e | 1c (26%) [c] | (300MHz, DMSO-d6, ausgewählte Signale, Probe enthielt Ameisensäureanteil): δ = 0.27 (br. s., 2H), 0.59 (br. s., 2H), 0.97 (br. s., 1H), 2.62 (s), 3.05 (br. s.), 3.53 (br. s.), 4.00 (s, 3H), 5.43 (br. s., 2H), 7.08 (s, 1H), 7.55 (dd, 1H), 7.93 - 8.01 (m, 2H), 8.19 (s, 1H), 8.72 (s, 1H), 10.71 (s, 1H). |
| | N-(2- {2-[4-(Cyclopropylmethyl)piperazin-1-yl]-2-oxoethyl}-6-methoxy-2H-indazol-5-yl)-6-methylpyridin-2-carboxamid | | | |
| | | | | UPLC-MS (Methode A1): Rt = 0.85 min |
| | | | | MS (ESIpos): m/z = 463 (M+H)+ |
| 10 | 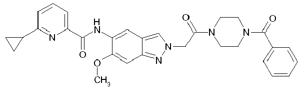 | Intermediat 6-7 und 6-Cyclopropy lpyridin-2-carbonsäur e | 1a (50%) [d] | (300MHz, DMSO-d6): δ = 1.04 - 1.15 (m, 4H), 2.21 - 2.33 (m, 1H), 3.39 - 3.87 (8H), 4.00 (s, 3H), 5.41 (br. s., 2H), 7.09 (s, 1H), 7.41 - 7.52 (m, 5H), 7.58 - 7.65 (m, 1H), 7.87 - 7.96 (m, 2H), 8.20 (s, 1H), 8.66 (s, 1H), 10.80 (s, 1H). |
| | N-{2-[2-(4-Benzoylpiperazin-1-yl)-2-oxoethyl]-6-methoxy-2H-indazol-5-yl}-6-cyclopropylpyridin-2-carboxamid | | | |
| | | | | UPLC-MS (Methode A1): Rt = 1.18 min |
| | | | | MS (ESIpos): m/z = 539 (M+H)+ |
| 11 | 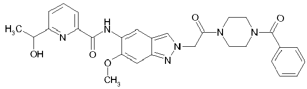 | Intermediat 6-7 und Intermediat 19-1 | 1c (49%) | (300MHz, DMSO-d6): δ = 1.51 (d, 3H), 3.38 - 3.91 (8H), 3.99 (s, 3H), 4.81 - 4.90 (m, 1H), 5.41 (br. s., 2H), 5.60 (d, 1H), 7.09 (s, 1H), 7.35 - 7.56 (m, 5H), 7.76 - 7.82 (m, 1H), 8.01 - 8.11 (m, 2H), 8.21 (s, 1H), 8.68 (s, 1H), 10.78 (s, 1H). |
| | N-{2-[2-(4-Benzoylpiperazin-1-yl)-2-oxoethyl]-6-methoxy-2H-indazol-5-yl}-6-(1-hydroxyethyl)pyridin-2-carboxamid | | | |
| | | | | UPLC-MS (Methode A1): Rt = 0.93 min |
| | | | | MS (ESIpos): m/z = 534 (M+H)+ |
| 12 | 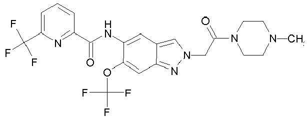 | Intermediat 6-8 und 6-(Trifluorme thyl)pyridin -2-carbonsäur e | 1d (47%) | (400 MHz, DMSO-d6) δ = 2.22 (s, 3 H) 2.27 - 2.36 (m, 2 H) 2.37 - 2.44 (m, 2 H) 3.44 - 3.52 (m, 2 H) 3.52 - 3.60 (m, 2 H) 5.52 (s, 2 H) 7.75 (s, 1 H) 8.23 (dd, 1 H) 8.38 - 8.50 (m, 3 H) 8.71 (s, 1 H) 10.40 (s, 1 H). |
| | N-{2-[2-(4-Methylpiperazin-1-yl)-2-oxoethyl]-6-(trifluoromethoxy)-2H-indazol-5-yl}-6-(trifluoromethyl)pyridine-2-carboxamid | | | |
| | | | | UPLC-MS (Methode A1): Rt = 0.96 min |
| | | | | MS (ESIpos): m/z = 531 (M+H)+ |
| 13 | 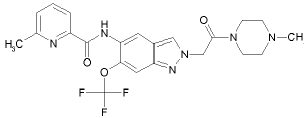 | Intermediat 6-8 und 6-Methylpyri din-2-carbonsäur e | 1d (49%) | (300 MHz, DMSO-d6) δ = 2.21 (s, 3 H), 2.25 - 2.34 (m, 2 H), 2.39 (br. s., 2 H), 2.61 (s, 3 H), |
| | 6-Methyl-N-{2-[2-(4-methylpiperazin-1-yl)-2-oxoethyl]-6-(trifluoromethoxy)-2H-indazol-5-yl}pyridine-2-carboxamid | | | 3.44 - 3.51 (m, 2 H), 3.51 - 3.61 (m, 2 H), 5.52 (s, 2 H), 7.59 (dd, 1 H), 7.76 (s, 1 H), 7.96 - 8.04 (m, 2 H), 8.45 (s, 1 H), 8.72 (s, 1 H), 10.65 (s, 1 H). |
| | | | | UPLC-MS (Methode A1): Rt = 0.92 min |
| | | | | MS (ESIpos): m/z = 477 (M+H)+ |
| 14 | 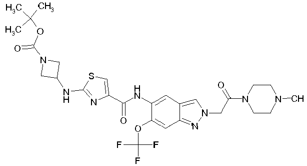 | Intermediat 6-8 und 2-{[1-(tert-Butoxycarb onyl)azetidi n-3-yl]amino}-1,3-thiazol-4-carbonsäur e** | 1d (39%) | (300 MHz, DMSO-d6) δ = 1.39 (s, 9 H), 2.21 (s, 3 H), 2.26 - 2.34 (m, 2 H), 2.38 (br. s., 2 H), 3.46 (br. s., 2 H), 3.54 (br. s., 2 |
| | tert-Butyl 3-{[4-({2-[2-(4-methylpiperazin-1-yl)-2-oxoethyl]-6-(trifluoromethoxy)-2H-indazol-5-yl}carbamoyl)-1,3-thiazol-2-yl]amino}azetidin-1-carboxylat | | | H), 3.77 (dd, 2 H), 4.20 (t, 2 H), 4.46 (d, 1 H), 5.50 (s, 2 H), 7.56 (s, 1 H), 7.73 (s, 1 H), 8.42 (s, 1 H), 8.57 (d, 1 H), 8.62 (s, 1 H), 9.54 (s, 1 H). |
| | | | | UPLC-MS (Methode A1): Rt = 0.63 min |
| | | | | MS (ESIpos): m/z = 639 (M+H)+ |
| 15 | 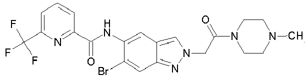 | Intermedia Intermediat 6-9 und 6-(Trifluorme thyl)pyridin -2-carbonsäur e | 1e (76%) | (300 MHz, DMSO-d6) δ = 2.21 (s, 3 H), 2.29 (br. s., 2 H), 2.38 |
| | N-{6-Bromo-2-[2-(4-methylpiperazin-1-yl)-2-oxoethyl]-2H-indazol-5-yl}-6-(trifluoromethyl)pyridin-2-carboxamid | | | (br. s., 2 H), 3.47 (br. s., 2 H), 3.54 (br. s., 2 H), 5.50 (s, 2 H), 8.09 (s, 1 H), 8.24 (d, 1 H), 8.35 - 8.50 (m, 3 H), 8.64 (s, 1 H), 10.54 (s, 1 H). |
| | | | | LC-MS (Methode A3): Rt = 0.93 min |
| | | | | MS (ESIpos): m/z = 525 (M(79Br)+H)+ |
| 16 | 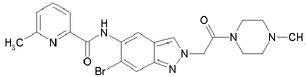 | Intermediat 6-9 und 6-Methylpyri din-2-carbonsäur e | 1e (93%) | (300 MHz, DMSO-d6) δ = 2.21 (s, 3 H), 2.25 - 2.35 (m, 2 H), 2.38 (br. s., 2 H), 2.64 (s, 3 H), 3.47 (br. s., 2 H), 3.54 (br. s., 2 H), 5.49 (s, 2 H), 7.58 (dd, 1 H), 7.97 - 8.04 (m, 2 H), 8.08 (s, 1 H), 8.39 (s, 1 H), 8.71 (s, 1 H), 10.77 (s, 1 H). |
| | N-{6-Brom-2-[2-(4-methylpiperazin-1-yl)-2-oxoethyl]-2H-indazol-5-yl}-6-methylpyridin-2-carboxamid | | | |
| | | | | LC-MS (Methode A3): Rt = 0.88 min |
| | | | | MS (ESIpos): m/z = 471 (M(79Br)+H)+ |
| 17 | 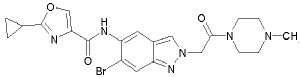 | Intermediat 6-9 und 2-Cyclopropy 1-1,3-oxazol-4-carbonsäur e | 1e (55%) | (300 MHz, DMSO-d6) δ = 1.01 - 107 (m, 2 H), 107 - 1.16 (m, 2 H), 2.17 - 2.26 (m, 4 H), 2.27 - 2.34 (m, 2 H), 2.37 (br. s., 2 H), 3.46 (br. s., 2 H), 3.54 (br. s., 2 H), 5.49 (s, 2 H), 8.03 (s, 1 H), 8.29 (s, 1 H), 8.37 (s, 1 H), 8.63 (s, 1 H), 9.61 (s, 1 H). |
| | N-{6-Brom-2-[2-(4-methylpiperazin-1-yl)-2-oxoethyl]-2H-indazol-5-yl}-2-cyclopropyl-1,3-oxazole-4-carboxamid | | | |
| | | | | LC-MS (Methode A3): Rt = 0.83 min |
| | | | | MS (ESIpos): m/z = 487 (M(79Br)+H)+ |
| 18 | 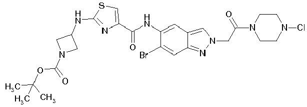 | Intermediat 6-9 und 2-{[1-(tert-Butoxycarb onyl)azetidi n-3-yl]amino}-1,3-thiazol-4-carbonsäur e** | 1e (41%) | (300 MHz, DMSO-d6) δ = 1.39 (s, 9 H), 2.21 (s, 3 H), 2.25 - 2.33 (m, 2 H), 2.37 (br. s., 2 H), |
| | tert-Butyl 3-{[4-({6-brom-2-[2-(4-methylpiperazin-1-yl)-2-oxoethyl]-2H-indazol-5-yl}carbamoyl)-1,3-thiazol-2-yl]amino}azetidin-1-carboxylat | | | 3.46 (br. s., 2 H), 3.54 (br. s., 2 H), 3.79 (dd, J=8.6, 5.4 Hz, 2 H), 4.23 (t, 2 H), 4.50 (d, 1 H), 5.47 (s, 2 H), 7.54 (s, 1 H), 8.05 (s, 1 H), 8.31 - 8.41 (m, 1 H), 8.54 (d, 1 H), 8.64 (s, 1 H), 9.82 (s, 1 H). |
| | | | | UPLC-MS (Methode A2): Rt = 1.09 min |
| | | | | MS (ESIpos): m/z = 633 (M(79Br)+H)+ |

| | | | | |
|---|---|---|---|---|
| * Hergestellt nach angegebener Vorschrift, in Klammern ist die Ausbeute in % angegeben [a]: Die Reaktion wurde in einer Mischung aus Tetrahydrofuran/*N*,*N*-Dimethylformamid (5:1) durchgeführt. Es wurden 3 Äquivalente Triethylamin eingesetzt. [b]: Es wurden 1.3 Äquivalente der Pyridincarbonsäure eingesetzt. [c]: Es wurden 1.5 Äquivalente der Pyridincarbonsäure eingesetzt. Das Produkt befand sich in der wäßrigen Phase. [d]: Die präparative HPLC wurde nach der Methode P1 durchgeführt. [e]: Das Produkt fiel direkt aus der Reaktionsmischung aus, wurde filtriert, mehrfach mit Wasser gewaschen und im Trockenschrank getrocknet. ** Die Herstellung von 2-{[1-(tert-Butoxycarbonyl)azetidin-3-yl]amino}-1,3-thiazol-4-carbonsäure erfolgte aus 3-Brom-2-oxopropansäure und tert-Butyl-3-aminoazetidin-1-carboxylat erfolgte analog zu Bioorganic and Medicinal Chemistry Letters, 1996, 6, 12, 1409 - 1414 und Chemical and Pharmaceutical Bulletin, 2005 , 53, 4, 437 - 440. | | | | |

### Beispiel 19

### 2-(Azetidin-3-ylamino)-N-{2-[2-(4-methylpiperazin-1-yl)-2-oxoethyl]-6-(trifluoromethoxy)-2H-indazol-5-yl}-1,3-thiazol-4-carboxamid

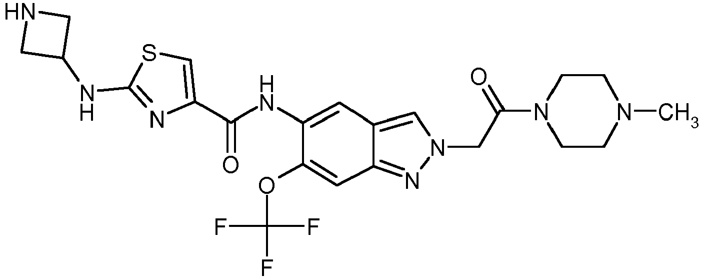

21 mg (0.03 mmol) tert-Butyl 3-{[4-({2-[2-(4-methylpiperazin-1-yl)-2-oxoethyl]-6-(trifluoromethoxy)-2H-indazol-5-yl}carbamoyl)-1,3-thiazol-2-yl]amino}azetidin-1-carboxylat (Beispiel 18) wurden in 1 ml Dichlormethan gelöst und mit 25 µl (0.03 mmol) Trifluoressigsäure versetzt. Die Reaktionsmischung wurde 24 h bei 25°C gerührt. Anschließend wurde mit weiterem Dichlormethan verdünnt und mit gesättigter Natriumhydrogencarbonatlösung sowie mit gesättigter Natriumchloridlösung gewaschen. Anschließend wurde über einen wasserabweisenden Filter filtriert und eingeengt. Der Rückstand wurde im Vakuum getrocknet. Man erhielt 7 mg (31 % d. Th.) der Titelverbindung.
UPLC-MS (Methode A2): Rₜ = 0.86 min
MS (ESIpos): m/z = 539 (M+H)⁺
¹H NMR (400 MHz, DMSO-d6): δ = 2.21 (s, 3 H), 2.29 (br. s., 2 H), 2.38 (br. s., 2 H), 3.42 - 3.49 (m, 4 H), 3.54 (br. s., 2 H), 3.69 - 3-73 (m, 1 H), 5.50 (s, 2 H), 7.49 (s, 1 H), 7.72 (s, 1 H), 8.42 (s, 1 H), 8.64 (s, 1 H), 9.58 (s, 1 H).

### Beispiel 20

### N-{6-Cyano-2-[2-(4-methylpiperazin-1-yl)-2-oxoethyl]-2H-indazol-5-yl}-6-(trifluoromethyl)pyridin-2-carboxamid

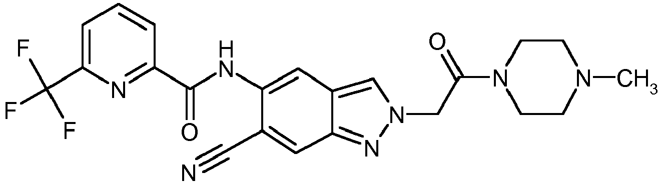

50 mg (0.10 mmol) N-{6-Bromo-2-[2-(4-methylpiperazin-1-yl)-2-oxoethyl]-2H-indazol-5-yl}-6-(trifluoromethyl)pyridin-2-carboxamid (Beispiel 15) wurden mit 5 mg (0.005 mmol) Tetrakis(triphenylphosphin)palladium(0) und 12 mg (0.10 mmol) Zinkcyanid in einem Mikrowellengefäß vorgelegt und in 1 ml *N*,*N*-Dimethylformamid suspendiert. Die Reaktionsmischung wurde 15 Minuten bei 150°C in der Mikrowelle gerührt. Da der Umsatz noch nicht vollständig war, wurden nochmal 5 mg (0.005 mmol) Tetrakis(triphenylphosphin)palladium(0) und 5.5 mg (0.05 mmol) Zinkcyanid zugegeben und weitere 30 Minuten in der Mikrowelle bei 150°C gerührt. Die Reaktionsmischung wurde mit Ethylacetat verdünnt und mit Wasser und gesättigter Natriumchloridlösung gewaschen.

Anschließend wurde die Lösung über einen wasserabweisenden Filter filtriert und eingeengt. Das Rohprodukt wurde in 2,5 ml *N*,*N*-Dimethylformamid gelöst und mit präparativer HPLC nach Methode P1 gereinigt. Die Produktfraktion wurde lyophilisiert. Man erhielt 25 mg (56 % d.Th.) der Titelverbindung.
LC-MS (Methode A3): Rₜ = 1.07 min
MS (ESIpos): m/z = 472 (M+H)⁺
¹H NMR (400 MHz, DMSO-d6): δ = 2.22 (s, 3 H), 2.27 - 2.33 (m, 2 H), 2.36 - 2.42 (m, 2 H), 3.44 - 3.50 (m, 2 H), 3.52 - 3.58 (m, 2 H), 5.59 (s, 2 H), 8.21 - 8.26 (m, 2 H), 8.37 - 8.43 (m, 2 H), 8.43 - 8.47 (m, 1 H), 8.51 (d, 1 H), 10.66 (s, 1 H).

### Beispiel 21

### 6'-Methyl-N-{2-[2-(4-methylpiperazin-1-yl)-2-oxoethyl]-2H-indazol-5-yl}-2,3'-bipyridin-6-carboxamid

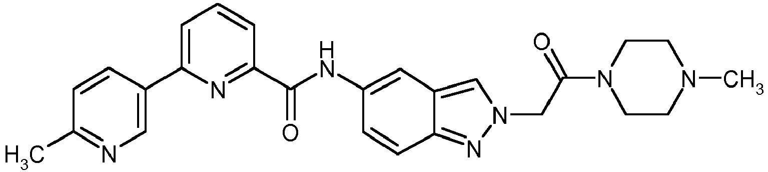

75 mg (0.16 mmol) 6-Brom-N-{2-[2-(4-methylpiperazin-1-yl)-2-oxoethyl]-2H-indazol-5-yl}pyridin-2-carboxamid (Beispiel 231) wurden in einer entgasten Mischung aus 1.73 ml Dioxan und 0.25 ml Wasser gelöst und mit 45 mg (0.33 mmol) (6-Methylpyridin-3-yl)boronsäure, 13 mg (0.02 mmol) 1,1'-Bis(diphenylphosphino)ferrocen-palladium(II)dichlorid und 52 mg (0.49 mmol) Natriumcarbonat versetzt. Die Reaktionsmischung wurde 90 Minuten bei 105°C in der Mikrowelle gerührt. Anschließend wurde die Reaktionsmischung filtriert und das Filtrat mit gesättigter Ammoniumchloridlösung und Dichlormethan versetzt. Die Phasen wurden getrennt und die organische Phase mit gesättigter Natriumchloridlösung gewaschen, über einen wasserabweisenden Filter filtriert und eingeengt. Das Rohprodukt wurde in 2,5 ml *N*,*N*-Dimethylformamid gelöst und mit präparativer HPLC nach Methode P1 gereinigt. Die Produktfraktion wurde lyophilisiert. Man erhielt 40 mg (52 % d.Th.) der Titelverbindung.
LC-MS (Methode A3): Rₜ = 0.46 min
MS (ESIpos): m/z = 470 (M+H)⁺
¹H NMR (300 MHz, DMSO-d6): δ = 2.22 (s, 3 H), 2.31 (br. s., 2 H), 2.39 (br. s., 2 H), 2.57 (s, 3 H), 3.48 (br. s., 2 H), 3.55 (d, 2 H), 5.47 (s, 2 H) 7.44 (d, 1 H), 7.62 (s, 2 H), 8.08 - 8.20 (m, 2 H), 8.26 - 8.32 (m, 2 H), 8.34 (s, 1 H), 8.68 (dd, 1 H), 9.43 (d, 1 H), 10.54 (s, 1 H).

### Beispiel 22

### 5'-Methyl-N-{2-[2-(4-methylpiperazin-1-yl)-2-oxoethyl]-2H-indazol-5-yl}-2,3'-bipyridin-6-carboxamid

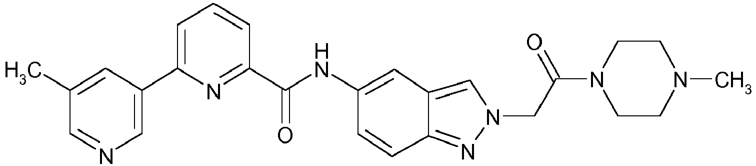

In Analogie zu Beispiel 21 wurden 75 mg (0.16 mmol) 6-Brom-N-{2-[2-(4-methylpiperazin-1-yl)-2-oxoethyl]-2H-indazol-5-yl}pyridin-2-carboxamid mit 45 mg (0.33 mmol) (5-Methylpyridin-3-yl)boronsäure, 13 mg (0.02 mmol) 1,1'-Bis(diphenylphosphino)ferrocen-palladium(II)dichlorid und 52 mg (0.49 mmol) Natriumcarbonat in einer entgasten Mischung aus 1.73 ml Dioxan und 0.25 ml Wasser 90 Minuten bei 105°C in der Mikrowelle gerührt. Nach Aufarbeitung und präparativer HPLC nach Methode P1 erhielt man 41 mg (53 % d.Th.) der Titelverbindung.
LC-MS (Methode A3): Rₜ = 0.51 min
MS (ESIpos): m/z = 470 (M+H)⁺
¹H NMR (300 MHz, DMSO-d6): δ = 2.23 (s, 3 H), 2.32 (br. s., 2 H), 2.41 (br. s., 2 H), 2.45 (s, 3 H), 3.48 (br. s., 2 H), 3.56 (br. s., 2 H), 5.47 (s, 2 H), 7.62 (s, 2 H), 8.11 - 8.23 (m, 2 H), 8.27 - 8.37 (m, 3 H), 8.55 (s, 1 H), 8.60 (s, 1 H), 9.38 (d, 1 H), 10.55 (s, 1 H).

### Beispiel 23

### 4'-Methyl-N-{2-[2-(4-methylpiperazin-1-yl)-2-oxoethyl]-2H-indazol-5-yl}-2,3'-bipyridin-6-carboxamid

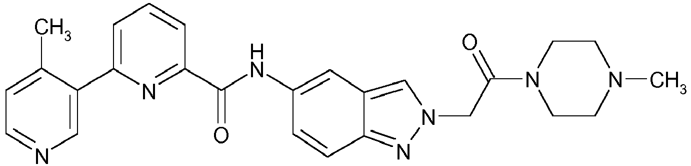

In Analogie zu Beispiel 21 wurden 75 mg (0.16 mmol) 6-Brom-N-{2-[2-(4-methylpiperazin-1-yl)-2-oxoethyl]-2H-indazol-5-yl}pyridin-2-carboxamid mit 45 mg (0.33 mmol) (4-Methylpyridin-3-yl)boronsäure, 13 mg (0.02 mmol) 1,1'-Bis(diphenylphosphino)ferrocen-palladium(II)dichlorid und 52 mg (0.49 mmol) Natriumcarbonat in einer entgasten Mischung aus 1.73 ml Dioxan und 0.25 ml Wasser 90 Minuten bei 105°C in der Mikrowelle gerührt. Nach Aufarbeitung und präparativer HPLC nach Methode P1 erhielt man 16 mg (21 % d.Th.) der Titelverbindung.
LC-MS (Methode A3): Rₜ = 0.45 min
MS (ESIpos): m/z = 470 (M+H)⁺
¹H NMR (300 MHz, DMSO-d6): δ = 2.21 (s, 3 H), 2.30 (br. s., 2 H), 2.38 (br. s., 2 H), 3.47 (br. s., 2 H), 3.54 (d, 2 H), 5.45 (s, 2 H), 7.42 (d, 1 H), 7.57 (d, 2 H), 7.91 (t, 1 H), 8.19 (d, 2 H), 8.28 (s, 1 H), 8.34 (s, 1 H), 8.54 (d, 1 H), 8.78 (s, 1 H), 10.41 (s, 1 H).

### Beispiel 24

### 6'-Methoxy-N-{2-[2-(4-methylpiperazin-1-yl)-2-oxoethyl]-2H-indazol-5-yl}-2,3'-bipyridin-6-carboxamid

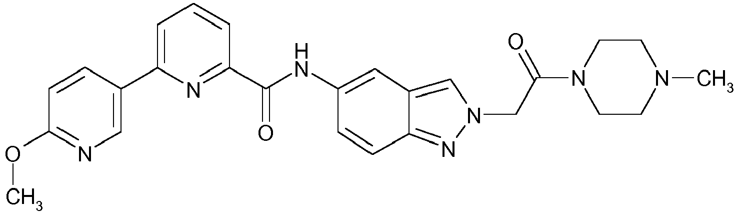

In Analogie zu Beispiel 21 wurden 50 mg (0.11 mmol) 6-Brom-N-{2-[2-(4-methylpiperazin-1-yl)-2-oxoethyl]-2H-indazol-5-yl}pyridin-2-carboxamid mit 33 mg (0.22 mmol) (6-Methoxypyridin-3-yl)boronsäure, 9 mg (0.01 mmol) 1,1'-Bis(diphenylphosphino)ferrocen-palladium(II)dichlorid und 35 mg (0.33 mmol) Natriumcarbonat in einer entgasten Mischung aus 1.15 ml Dioxan und 0.17 ml Wasser 90 Minuten bei 105°C in der Mikrowelle gerührt. Nach Aufarbeitung und präparativer HPLC nach Methode P1 erhielt man 28 mg (52 % d.Th.) der Titelverbindung.
LC-MS (Methode A3): Rₜ = 0.74 min
MS (ESIpos): m/z = 486 (M+H)⁺
¹H NMR (400 MHz, DMSO-d6): δ = 2.21 (s, 3 H), 2.30 (t, 2 H), 2.38 (t, 2 H), 3.45 - 3.52 (m, 2 H), 3.52 - 3.60 (m, 2 H), 3.96 (s, 3 H), 5.46 (s, 2 H), 6.96 - 7.01 (m, 1 H), 7.58 - 7.66 (m, 2 H), 8.07 - 8.11 (m, 1 H), 8.11 - 8.16 (m, 1 H), 8.24 (dd, 1 H), 8.30 (s, 1 H), 8.32 - 8.34 (m, 1 H), 8.74 (dd, 1 H), 9.22 (d, 1 H), 10.52 (s, 1 H).

### Beispiel 25

### 6'-Acetamido-N-{2-[2-(4-methylpiperazin-1-yl)-2-oxoethyl]-2H-indazol-5-yl}-2,3'-bipyridin-6-carboxamid

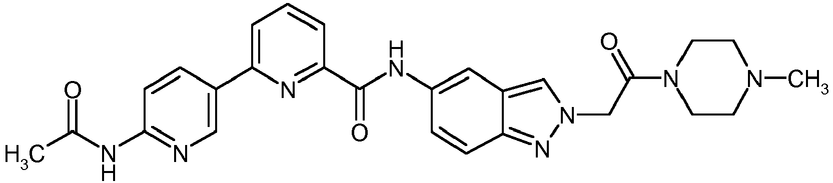

In Analogie zu Beispiel 21 wurden 50 mg (0.11 mmol) 6-Brom-N-{2-[2-(4-methylpiperazin-1-yl)-2-oxoethyl]-2H-indazol-5-yl}pyridin-2-carboxamid mit 57 mg (0.22 mmol) N-[5-(4,4,5,5-Tetramethyl-1,3,2-dioxaborolan-2-yl)pyridin-2-yl]acetamid, 9 mg (0.01 mmol) 1,1'-Bis(diphenylphosphino)ferrocen-palladium(II)dichlorid und 35 mg (0.33 mmol) Natriumcarbonat in einer entgasten Mischung aus 1.15 ml Dioxan und 0.17 ml Wasser 90 Minuten bei 105°C in der Mikrowelle gerührt. Nach Aufarbeitung und präparativer HPLC nach Methode P1 erhielt man 21 mg (37 % d.Th.) der Titelverbindung.
LC-MS (Methode A3): Rₜ = 0.59 min
MS (ESIpos): m/z = 513 (M+H)⁺
1H NMR (400 MHz, DMSO-d6): δ = 2.15 (s, 3 H), 2.21 (s, 3 H), 2.27 - 2.34 (m, 2 H), 2.35 - 2.41 (m, 2 H), 3.44 - 3.51 (m, 2 H), 3.52 - 3.58 (m, 2 H), 5.46 (s, 2 H), 7.58 - 7.68 (m, 2 H), 8.09 - 8.12 (m, 1 H), 8.12 - 8.17 (m, 1 H), 8.23 - 8.29 (m, 2 H), 8.30 (s, 1 H), 8.34 (s, 1 H), 8.79 (dd, 1 H), 9.32 (dd, 1 H), 10.53 (s, 1 H), 10.69 (s, 1 H).

### Beispiel 26

### N-{2-[2-(4-Methylpiperazm-1-yl)-2-oxoethyl]-2H-indazol-5-yl}-6'-nitro-2,3'-bipyridin-6-carboxamid

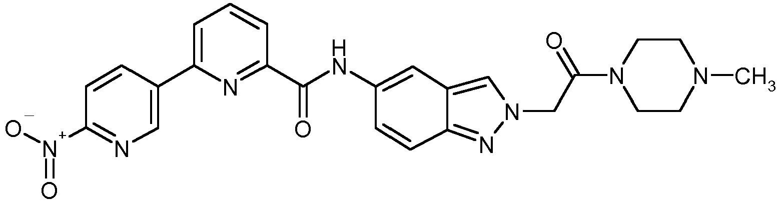

In Analogie zu Beispiel 21 wurden 75 mg (0.16 mmol) 6-Brom-N-{2-[2-(4-methylpiperazin-1-yl)-2-oxoethyl]-2H-indazol-5-yl}pyridin-2-carboxamid mit 82 mg (0.33 mmol) 2-Nitro-5-(4,4,5,5-tetramethyl-1,3,2-dioxaborolan-2-yl)pyridin, 13 mg (0.02 mmol) 1,1'-Bis(diphenylphosphino)ferrocen-palladium(II)dichlorid und 52 mg (0.49 mmol) Natriumcarbonat in einer entgasten Mischung aus 1.73 ml Dioxan und 0.25 ml Wasser 90 Minuten bei 105°C in der Mikrowelle gerührt. Nach Aufarbeitung und präparativer HPLC nach Methode P1 erhielt man 26 mg (32 % d.Th.) der Titelverbindung.
UPLC-MS (Methode A1): Rt = 0.78 min
MS (ESIpos): m/z = 501 (M+H)⁺
¹H NMR (300 MHz, DMSO-d6): δ = 2.22 (s, 3 H), 2.31 (br. s., 2 H), 2.39 (br. s., 2 H), 3.48 (br. s., 2 H), 3.56 (br. s., 2 H), 5.47 (s, 2 H), 7.63 (s, 2 H), 8.22 - 8.38 (m, 4 H), 8.45 - 8.55 (m, 2 H), 9.22 (dd, 1 H), 9.72 (d, 1 H), 10.63 (s, 1 H).

### Beispiel 27

### 6'-Amino-N-{2-[2-(4-methylpiperazin-1-yl)-2-oxoethyl]-2H-indazol-5-yl}-2,3'-bipyridin-6-carboxamid

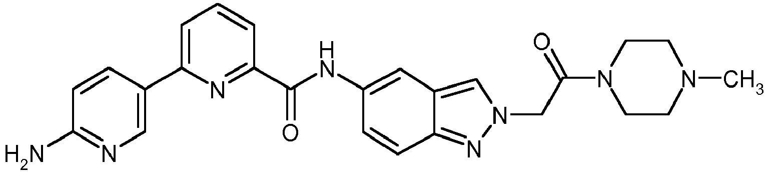

20 mg (0.04 mmol) N-{2-[2-(4-Methylpiperazin-1-yl)-2-oxoethyl]-2H-indazol-5-yl}-6'-nitro-2,3'-bipyridin-6-carboxamid wurden in 2.5 ml Methanol gelöst, mit 4 mg (0.004 mmol, 10%) Palladium auf Kohle versetzt und 4 h unter 1 bar Wasserstoff-Atmosphäre hydriert. Die Reaktionsmischung wurde über Celite abfiltriert, mehrmals mit Methanol gewaschen, das Filtrat eingeengt und im Vakuum getrocknet. Man erhielt 8 mg (43 % d.Th.) der Titelverbindung.
UPLC-MS (Methode A1): Rt = 0.81 min
MS (ESIpos): m/z = 471 (M+H)⁺
¹H NMR (400 MHz, METHANOL-d4): δ = 2.34 (s, 3 H), 2.43 - 2.49 (m, 2 H), 2.53 (br. s., 2 H), 3.66 (br. s., 4 H), 5.46 (s, 2 H), 6.73 (d, 1 H), 7.51 - 7.58 (m, 1 H), 7.60 - 7.67 (m, 1 H), 7.94 - 8.04 (m, 2 H), 8.07 (d, 1 H), 8.21 (s, 1 H), 8.34 (br. s., 2 H), 8.55 (s, 1 H), 8.77 (s, 1 H).

### Allgemeine Versuchsvorschrift 2a

1.0 Äquivalente des jeweiligen Intermediates wurden mit 1.0 Äquivalenten 1-Hydroxy-1H-benzotriazol Hydrat und 2.0 Äquivalenten 1-(3-Dimethylaminopropyl)-3-ethylcarbodiimidhydrochlorid in 3 ml Tetrahydrofuran und 0.33 ml Dimethylformamid 30 min bei 25°C gerührt. Dann wurden 1.5 Äquivalente des Amins zugegeben und 30 min bei 25°C gerührt. Es wurde auf 50 ml Wasser gegossen, abgesaugt, mit Wasser gewaschen und getrocknet.

### Allgemeine Versuchsvorschrift 2b

1.0 Äquivalente des jeweiligen Intermediates wurden mit 1.0 Äquivalenten 1-Hydroxy-1H-benzotriazol Hydrat, 2.0 Äquivalenten 1-(3-Dimethylaminopropyl)-3-ethylcarbodiimidhydrochlorid und 3.0 Äquivalenten Triethylamin in 1.5 ml *N*,*N*-Dimethylformamid 30 min bei 25°C gerührt. Dann wurden 1,2 Äquivalente des Amins zugegeben. Die Reaktionsmischung wurde mit weiteren 1.0 ml *N*,*N*-Dimethylformamid verdünnt und mit präparativer HPLC nach Methode P1 gereinigt.

### Allgemeine Versuchsvorschrift 2c

1.0 Äquivalente des jeweiligen Intermediates wurden mit 1.0 Äquivalenten 1-Hydroxy-1H-benzotriazol Hydrat, 2.0 Äquivalenten 1-(3-Dimethylaminopropyl)-3-ethylcarbodiimidhydrochlorid, 3.0 Äquivalenten Triethylamin und 1.2 Äquivalenten des Amins in Tetrahydrofuran 18 h bei 25°C gerührt. Das Reaktionsgemisch wurde mit Wasser versetzt. Der Feststoff wurde abgesaugt, mit Wasser und Diethylether gewaschen und getrocknet.

### Allgemeine Versuchsvorschrift 2d

1.0 Äquivalente des jeweiligen Intermediates wurden mit 1.0 Äquivalenten 1-Hydroxy-1H-benzotriazol Hydrat, 2,0 Äquivalenten 1-(3-Dimethylaminopropyl)-3-ethylcarbodiimidhydrochlorid, 3.0 Äquivalenten Triethylamin und 1.5 Äquivalenten des Amins in Tetrahydrofuran 18 h bei 25°C gerührt. Die Reaktionsmischung wurde mit Wasser verdünnt und mit Ethylacetat extrahiert. Die vereinigten organischen Phasen wurden eingeengt und das Rohprodukt mit präparativer HPLC nach Methode P4 gereinigt.

### Allgemeine Versuchsvorschrift 2e

1.0 Äquivalente des jeweiligen Intermediates wurden mit 1.0 Äquivalenten 1-Hydroxy-1H-benzotriazol Hydrat, 2.0 Äquivalenten 1-(3-Dimethylaminopropyl)-3-ethylcarbodiimidhydrochlorid, 3.0 Äquivalenten Triethylamin und 1.3 Äquivalenten des Amins in Tetrahydrofuran 18 h bei 25°C gerührt. Die Reaktionsmischung wurde mit Wasser verdünnt und mit Ethylacetat extrahiert. Die vereinigten organischen Phasen wurden eingeengt und mit 1 ml Dimethylsulfoxid versetzt. Der Feststoff wurde abgesaugt, dreimal mit je 0.5 ml Dimethylsulfoxid und dreimal mit Diethylether gewaschen und getrocknet. Das Filtrat wurde eingeengt und mit präparativer HPLC nach Methode P2 gereinigt. Die erhaltene Produktfraktion wurde mit dem Feststoff vereinigt.

### Allgemeine Versuchsvorschrift 2f

1.0 Äquivalente des jeweiligen Intermediates wurden mit 1.0 Äquivalenten 1-Hydroxy-1H-benzotriazol Hydrat, 2,0 Äquivalenten 1-(3-Dimethylaminopropyl)-3-ethylcarbodiimidhydrochlorid, 4.0 Äquivalenten Triethylamin und 1.2 Äquivalenten des Amins in Tetrahydrofuran 18 h bei 25°C gerührt. Der entstandene Niederschlag wurde abfiltriert und mit Tetrahydrofuran gewaschen. Der Feststoff wurde in Methyl-tert-butylether und Ethylacetat ausgerührt, dann in Dichlormethan gelöst und mit Wasser versetzt. Die wässrige Phase wurde mit Dichlormethan extrahiert und die vereinigten organischen Phasen mit gesättigter Natriumchloridlösung gewaschen und über Natriumsulfat getrocknet. Nach Filtration wurde die Lösung eingeengt und das erhaltene Produkt getrocknet.

### Allgemeine Versuchsvorschrift 2g

1.0 Äquivalente des jeweiligen Intermediates wurden mit 1.0 Äquivalenten 1-Hydroxy-1H-benzotriazol Hydrat, 2.0 Äquivalenten 1-(3-Dimethylaminopropyl)-3-ethylcarbodiimidhydrochlorid, 3.0 Äquivalenten Triethylamin und 1.3 Äquivalenten des Amins in Tetrahydrofuran 18 h bei 50°C gerührt. Das Reaktionsgemisch wurde mit Wasser und Ethylacetat versetzt. Der Feststoff wurde abgesaugt, mit Wasser und Diethylether gewaschen und getrocknet.

**Tabelle 2: Beispiele 28 - 71**

| Die Beispielverbindungen wurden aus den entsprechenden Intermediaten und Aminen nach den allgemeinen Versuchsvorschriften 2a-2g hergestellt. | | | | |
|---|---|---|---|---|
| Bsp Nr. | Struktur/Name | Hergestellt aus | * Siehe Lege nde | ¹H-NMR / LC-MS |
| 28 | 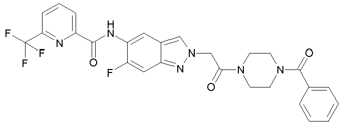 | 9-1 und Phenyl(piper azin-1-yl)methanon | 2a (94% ) | (300 MHz, DMSO-d6): δ = 3.38 - 3.75 (m, 8H), 5.51 (s, 2H), 7.40 - 7.56 (m, 6H), 8.19 - 8.26 (m, 1H),8.35 - 8.49 (m, 4H), 10.24 (m, 1H). |
| | N-{2-[2-(4-Benzoylpiperazin-1-yl)-2-oxoethyl]-6-fluor-2H-indazol-5-yl}-6-(trifluormethyl)pyridin-2-carboxamid | | | |
| | | | | UPLC-MS (Methode A1): Rt = 1.15 min |
| | | | | MS (ESIpos): m/z = 555 (M+H)+ |
| 29 | 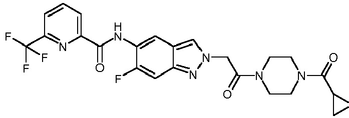 | 9-1 und Cyclopropyl( piperazin-1-yl)methanon | 2a (95% ) [a] | (300 MHz, DMSO-d6): δ = 0.69 - 0.81 (m, 4H), 2.00 (s br, 1H), 3.40 - 3.82 (m, 8H), 5.52 (s, 2H), 7.53 (d,1H), 8.22 (m, 1H), 8.36 - 8.49 (m, 4H), 10.25 (m, 1H). |
| | N-(2-{2-[4-(Cyclopropylcarbonyl)piperazin-1-yl]-2-oxoethyl}-6-fluor-2H-indazol-5-yl)-6-(trifluormethyl)pyridin-2-carboxamid | | | |
| | | | | UPLC-MS (Methode A1): Rt = 1.09 min |
| | | | | MS (ESIpos): m/z = 519 (M+H)+ |
| 30 | 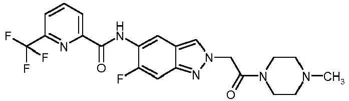 | 9-1 und 1-Methylpipera zin | 2a (41% ) | (300 MHz, DMSO-d6): δ = 2.21 (s, 3H), 2.29 (m, 2H), 2.38 (m, 2H), 3.47 (m, 2H), 3.55 (m, 2H), 5.47 (s, 2H), 7.52 (d, 1H), 8.22 (m, 1H), 8.34 - 8.48 (m, 4H), 10.24 (m, 1H). |
| | N-{6-Fluor-2-[2-(4-methylpiperazin-1-yl)-2-oxoethyl]-2H-indazol-5-yl}-6-(trifluormethy l)pyridin- 2-carboxamid | | | |
| | | | | UPLC-MS (Methode A1): Rt = 0.93 min |
| | | | | MS (ESIpos): m/z = 465 (M+H)+ |
| 31 | 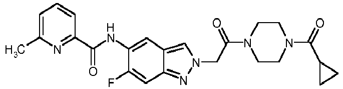 | 9-2 und Cyclopropyl( piperazin-1-yl)methanon | 2a (88% ) [a] | (300 MHz, DMSO-d6): δ = 0.68 - 0.82 (m, 4H), 2.01 (s br, 1H), 2.63 (s, 3H), 3.40 - 3.82 (m, 8H), 5.52 (s, 2H), 7.49 - 7.62 (m, 2H), 7.95 - 8.05 (m, 2H), 8.38 (s, 1H), 8.55 (d, 1H), 10.39 (d, 1H). |
| | N-(2-{2-[4-(Cyclopropylcarbonyl)piperazin-1-yl]-2-oxoethyl}-6-fluor-2H-indazol-5-yl)-6-methylpyridin-2-carboxamid | | | |
| | | | | UPLC-MS (Methode A1): Rt = 1.02 min |
| | | | | MS (ESIpos): m/z = 465 (M+H)+ |
| 32 | 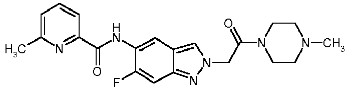 | 9-2 und 1-Methylpipera zin | 2a (68% ) | (300 MHz, DMSO-d6): δ = 2.21 (s, 3H), 2.29 (m, 2H), 2.38 (m, 2H), 2.63 (s, 3H), 3.46 (m, 2H), 3.53 (m, 2H), 5.45 (s, 2H), 7.47 - 7.62 (m, 2H), 7.93 (m, 2H), 8.36 (s, 1H), 8.55 (d, 1H), 10.55 (s, 1H). |
| | N-{6-Fluor-2-[2-(4-methylpiperazin-1-yl)-2-oxoethyl]-2H-indazol-5-yl}-6-methylpyridin-2-carboxamid | | | |
| | | | | UPLC-MS (Methode A1): Rt = 0.81 min |
| | | | | MS (ESIpos): m/z = 411 (M+H)+ |
| 33 | 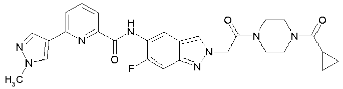 | 9-3 und Cyclopropyl( piperazin-1-yl)methanon | 2a (82% ) [a] | (300 MHz, DMSO-d6): δ = 0.68 - 0.83 (m, 4H), 2.02 (s br, 1H),3.42 -3.85 (m, 8H), 3.93 (s, 3H), 5.53 (s, 2H), 7.54 (d, 1H), 7.87 - 8.09 (m, 3H), 8.23 (s, 1H), 8.38 (m, 2H), 8.54 (s, 1H), 10.52 (s, 1H). |
| | N-(2-{2-[4-(Cyclopropylcarbonyl)piperazin-1-yl]-2-oxoethyl}-6-fluor-2H-indazol-5-yl)-6-(1-methyl-1H-pyrazol-4-yl)pyridin-2-carboxamid | | | |
| | | | | UPLC-MS (Methode A1): Rt = 0.93 min |
| | | | | MS (ESIpos): m/z = 531 (M+H)+ |
| 34 | 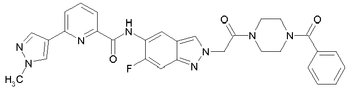 | 9-3 und Phenyl(piper azin-1-yl)methanon | 2a (47% ) [b] | (300 MHz, DMSO-d6): δ = 3.40 - 3.79 (m, 8H), 3.94 (s, 3H), 5.51 (s, 2H), 7.41 - 7.57 (m, 6H), 7.93 (t, 2H), 8.04 (t, 1H), 8.22 (s, 1H), 8.39 (m, 2H), 8.52 (s, 1H), 10.51 (s, 1H). |
| | N-{2-[2-(4-Benzoylpiperazin-l-yl)-2-oxoethyl]-6-fluor-2H-indazol-5-yl}-6-(1-methyl-1H-pyrazol-4-yl)pyridin-2-carboxamid | | | |
| | | | | UPLC-MS (Methode A1): Rt = 1.00 min |
| | | | | MS (ESIpos): m/z = 567 (M+H)+ |
| 35 | 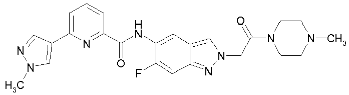 | 9-3 und 1-Methylpipera zin | 2a (14% ) [b] | (300 MHz, DMSO-d6): δ = 2.21 (s, 3H), 2.30 (m, 2H), 2.38 (m, 2H), 3.47 (m, 2H), 3.55 (m, 2H), 3.93 (s, 3H), 5.47 (s, 2H), 7.52 (d, 1H), 7.93 (t, 2H), 8.04 (t, 1H), 8.22 (s, 1H), 8.39 (m, 2H), 8.52 (s, 1H), 10.50 (s, 1H). |
| | N-{6-Fluor-2-[2-(4-methylpiperazin-1-yl)-2-oxoethyl]-2H-indazol-5-yl}-6-(1-methyl-1H-pyrazol-4-yl)pyridin-2-carboxamid | | | |
| | | | | UPLC-MS (Methode A1): Rt = 0.74 min |
| | | | | MS (ESIpos): m/z = 477 (M+H)+ |
| 36 | 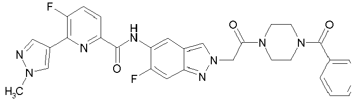 | 9-4 und Phenyl(piper azin-1-yl)methanon | 2a (64% ) | (300 MHz, DMSO-d6): δ = 3.38 - 3.76 (m, 8H), 3.96 (s, 3H), 5.51 (s, 2H), 7.41-7.56 (m, 6H), 7.93 - 8.05 (m, 2H), 8.25 - 8.31 (m, 2H), 8.40 (s, 1H), 8.53 (m, 1H), 10.40 (s, 1H). |
| | N-{2-[2-(4-Benzoylpiperazin-1-yl)-2-oxoethyl]-6-fluor-2H-indazol-5-yl}-5-fluor-6-(1-methyl-1H-pyrazol-4-yl)pyridin-2-carboxamid | | | |
| | | | | UPLC-MS (Methode A1): Rt = 1.07 min |
| | | | | MS (ESIpos): m/z = 585 (M+H)+ |
| 37 | 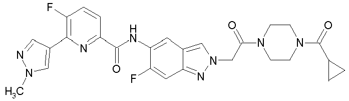 | 9-4 und Cyclopropyl( piperazin-1-yl)methanon | 2a (59% ) [a] | (300 MHz, DMSO-d6): δ = 0.69 - 0.83 (m, 4H), 2.01 (s br, 1H), 3.41 - 3.85 (m, 8H), 3.97 (s, 3H), 5.52 (s, 2H), 7.52 (d, 1H), 7.93 - 8.06 (m, 2H), 8.28 (m, 2H), 8.39 (s, 1H), 8.53 (s, 1H), 10.40 (s, 1H). |
| | N-(2-{2-[4-(Cyclopropylcarbonyl)piperazin-1-yl]-2-oxoethyl}-6-fluor-2H-indazol-5-yl)-5-fluor-6-(1-methyl-1H-pyrazol-4-yl)pyridin-2-carboxamid | | | |
| | | | | UPLC-MS (Methode A1): Rt = 1.00 min |
| | | | | MS (ESIpos): m/z = 549 (M+H)+ |
| 38 | 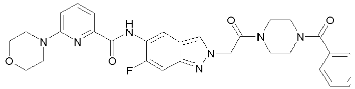 | 9-5 und Phenyl(piper azin-1-yl)methanon | 2a (78% ) | (300 MHz, DMSO-d6): δ = 3.41 - 3.82 (m, 16H), 5.50 (s, 2H), 7.15 (d, 1H), 7.47 |
| | N-{2-[2-(4-Benzoylpiperazin-1-yl)-2-oxoethyl]-6-fluor-2H-indazol-5-yl}-6-(morpholin-4-yl)pyridin-2-carboxamid | | | (m, 7H), 7.81 (t, 1H), 8.37 (s, 1H), 8.48 (d, 1H), 10.27 (m, 1H). |
| | | | | UPLC-MS (Methode A1): Rt = 1.06 min |
| | | | | MS (ESIpos): m/z = 572 (M+H)+ |
| 39 | 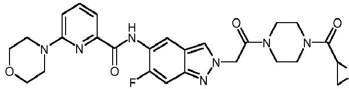 | 9-5 und Cyclopropyl( piperazin-1-yl)methanon | 2a (91% ) [a] | (300 MHz, DMSO-d6): δ = 0.67 - 0.83 (m, 4H), 2.01 (s br, 1H), 3.44 - 3.81 (m, 16H), 5.50 (s, 2H), 7.15 (d, 1H), 7.44 - 7.55 (m, 2H), 7.81 (t, 1H), 8.37 (s, 1H), 8.48 (d, 1H), 10.27 (m, 1H). |
| | N-(2-{2-[4-(Cyclopropylcarbonyl)piperazin-1-yl]-2-oxoethyl}-6-fluor-2H-indazol-5-yl)-6-(morpholin-4-yl)pyridin-2-carboxamid | | | |
| | | | | UPLC-MS (Methode A1): Rt = 1.01 min |
| | | | | MS (ESIpos): m/z = 536 (M+H)+ |
| 40 | 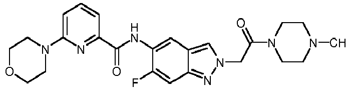 | 9-5 und 1-Methylpipera zin | 2a (64% ) | (300 MHz, DMSO-d6): δ = 2.21 (s, 3H), 2.30 (m, 2H), 2.38 (m, 2H), 3.47 (m, 2H), 3.55 (m, 2H), 3.59 (m, 4H), 3.76 (m, 4H), 5.45 (s, 2H), 7.15 (d, 1H), 7.44 - 7.53 (m, 2H), 7.81 (tr, 1H), 8.36 (s, 1H), 8.47 (d, 1H), 10.27 (m, 1H). |
| | N-{6-Fluor-2-[2-(4-methylpiperazin-1-yl)-2-oxoethyl]-2H-indazol-5-yl}-6-(morpholin-4-yl)pyridin-2-carboxamid | | | |
| | | | | UPLC-MS (Methode A1): Rt = 0.79 min |
| | | | | MS (ESIpos): m/z = 482 (M+H)+ |
| 41 | 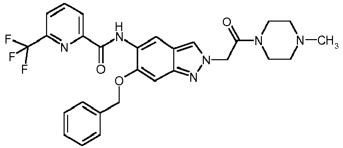 | 9-6 und 1-Methylpipera zin | 2b (72% ) | (400 MHz, DMSO-d6): δ = 2.21 (s, 3 H), 2.26 - 2.33 (m, 2 H), 2.34 - 2.43 (m, 2 H), 3.43 - 3.50 (m, 2 H), |
| | N-{6-(Benzyloxy)-2-[2-(4-methylpiperazin-1-yl)-2-oxoethyl]-2H-indazol-5-yl}-6-(trifluoromethy l)pyridin- 2-carboxamid | | | 3.51 - 3.59 (m, 2 H), 5.31 (s, 2 H), 5.39 (s, 2 H), 7.30 (s, 1 H), 7.34 - 7.45 (m, 3 H), 7.58 (dd, 2 H), 8.18 (dd, 1 H), 8.25 (s, 1 H), 8.35 - 8.44 (m, 1 H), 8.44 - 8.50 (m, 1 H), 8.80 (s, 1 H), 10.47 (s, 1 H). |
| | | | | LC-MS (Methode A3): Rt = 0.96 min |
| | | | | MS (ESIpos): m/z = 553 (M+H)+ |
| 42 | 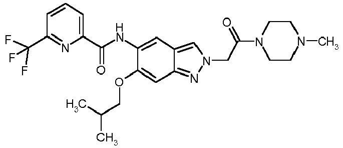 | 9-7 und 1-Methylpipera zin | 2b (53% ) | (300 MHz, DMSO-d6): δ = 1.11 (s, 3 H), 1.13 (s, 3 H), 2.17-2.23 (m, 1 H), 2.21 (s, 3 H), 2.25 - 2.33 (m, 2 H), 2.37 (br. 2 H), 3.46 (br. s., 2 H), 3.54 (br. s., 2 H), 3.96 (d, 2 H), 5.38 (s, 2 H), 7.07 (s, 1 H), 8.14 - 8.26 (m, 2 H), 8.34 - 8.45 (m, 1 H), 8.45 - 8.53 (m, 1 H), 8.78 (s, 1 H), 10.58 (s, 1 H). |
| | N-{6-Isobutoxy-2- [2-(4-methylpiperazin-1-yl)-2-oxoethyl]-2H-indazol-5-yl}-6-(trifluoromethy l)pyridin- 2-carboxamid | | | LC-MS (Methode A3): Rt = 1.06 min |
| | | | | MS (ESIpos): m/z = 519 (M+H)+ |
| 43 | 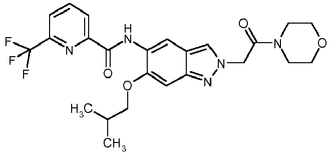 | 9-7 und Morpholin | 2b (54% ) | (300 MHz, DMSO-d6): δ = 3.47 (d, 2 H), 3.52 - 3.68 (m, 6 H), 3.96 (d, 2 H), 5.40 (s, 2 H), 7.07 (s, 1 H), 8.19 - 8.25 (m, 2 H), 8.36 - 8.45 (m, 1 H), 8.46 - 8.52 (m, 1 H), 8.78 (s, 1 H), 10.58 (s, 1 H). |
| | N-{6-isobutoxy-2-[2-(morpholin-4-yl)-2-oxoethyl]-2H-indazol-5-yl}-6-(trifluoromethyl)pyridin-2-carboxamid | | | |
| | | | | LC-MS (Methode A3): Rt = 1.32 min |
| | | | | MS (ESIpos): m/z = 506 (M+H)+ |
| 44 | 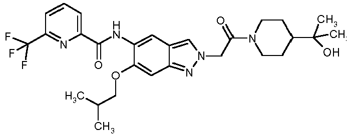 | 9-7 und 2-(Piperidin-4-yl)propan-2-ol | 2b (49% ) | (300 MHz, DMSO-d6): δ = 1.05 (s, 6 H), 1.11 (s, 3 H), 1.13 (s, 3 H), 1.19 - 1.33 (m, 3 H), 1.36 - 1.52 (m, 1 H), 1.75 (t, 2 H), 2.11 - 2.25 (m, 1 H), 2.90 - 3.09 (m, 1 H), 3.96 (d, 2 H), 4.02 (d, 1 H), 4.18 (s, 1 H), 4.42 (d, 1 H), 5.27 - 5.45 (m, 2 H), 7.07 (s, 1 H), 8.18 - 8.25 (m, 2 H), 8.35 - 8.45 (m, 1 H), 8.45 - 8.52 (m, 1 H), 8.77 (s, 1 H), 10.58 (s, 1 H). |
| | N-(2-{2-[4-(2-Hydroxypropan-2-yl)piperidin-1-yl]-2-oxoethyl}-6-isobutoxy-2H-indazol-5-yl)-6-(trifluoromethyl)pyridin-2-carboxamid | | | |
| | | | | LC-MS (Methode A3): Rt = 1.34 min |
| | | | | MS (ESIpos): m/z = 562 (M+H)+ |
| 45 | 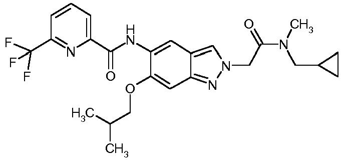 | 9-7 und 1-Cyclopropyl-N-methylmetha namin | 2b (54% ) | (400 MHz, DMSO-d6): δ = 0.18 - 0.25 (m, 1 H), 0.33 (q, 1 H), 0.41 - 0.49 (m, 1 H), 0.50 - 0.58 (m, 1 H), 0.92 - 1.02 (m, 1 H), 1.11 (s, 3 H), 1.13 (s, 3 H), 2.19 (dt, 1 H), 2.92 (s, 1 H) + 3.13 (s, 2 H), 3.20 (d, 1 H), 3.34 (d, 1 H), 3.96 (d, 2 H), 5.33 - 5.42 (m, 2 H), 7.07 (s, 1 H), 8.16 - 8.28 (m, 2 H), 8.41 (t, 1 H), 8.49 (d, 1 H), 8.78 (s, 1 H), 10.57 (s, 1 H). |
| | N-(2-{2-[(Cyclopropylmethyl)(methyl)amin o]-2-oxoethyl}-6-isobutoxy-2H-indazol-5-yl)-6-(trifluoromethyl)pyridin-2-carboxamid | | | |
| | | | | LC-MS (Methode A3): Rt = 1.45 min |
| | | | | MS (ESIpos): m/z = 504 (M+H)+ |
| 46 | 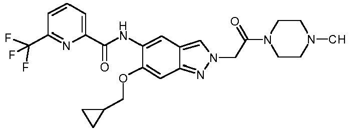 | 9-8 und 1-Methylpipera zin | 2b (75% ) | (400 MHz, DMSO-d6): δ = 0.39 - 0.51 (m, 2 H), 0.57 - 0.70 (m, 2 H), 1.27 - 1.43 (m, 1 H), 2.21 (s, 3 H), 2.29 (t, 2 H), 2.34 - 2.39 (m, 2 H), 3.43 - 3.49 (m, 2 H), 3.51 - 3.57 (m, 2 H), 4.03 (d, 2 H), 5.37 (s, 2 H), 7.05 (s, 1 H), 8.19 - 8.23 (m, 2 H), 8.41 (t, 1 H), 8.48 (d, 1 H), 8.76 (s, 1 H), 10.71 (s, 1 H). |
| | N-{6-(Cyclopropylmethoxy)-2-[2-(4-methylpiperazin-1-yl)-2-oxoethyl]-2H-indazol-5-yl}-6-(trifluoromethyl)pyridin-2-carboxamid | | | |
| | | | | LC-MS (Methode A3): Rt = 1.01 min |
| | | | | MS (ESIpos): m/z = 517 (M+H)+ |
| 47 | 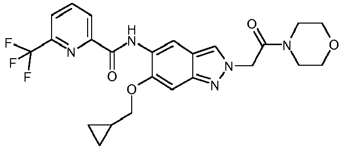 | 9-8 und Morpholin | 2b (73% ) | (400 MHz, DMSO-d6): δ = 0.40 - 0.48 (m, 2 H), 0.66 (dd, 2 H), 1.31 - 1.40 (m, 1 H), 3.43 - 3.50 (m, 2 H), 3.53 - 3.68 (m, 6 H), 4.03 (d, 2 H), 5.39 (s, 2 H), 7.05 (s, 1 H), 8.19 - 8.23 (m, 2 H), 8.38 - 8.44 (m, 1 H), 8.45 - 8.50 (m, 1 H), 8.76 (s, 1 H), 10.71 (s, 1 H). |
| | N-{6-(Cyclopropylmethoxy)-2-[2-(morpholin-4-yl)-2-oxoethyl]-2H-indazol-5-yl}-6-(trifluoromethyl)pyridin-2-carboxamid | | | |
| | | | | LC-MS (Methode A3): Rt = 1.25 min |
| | | | | MS (ESIpos): m/z = 504 (M+H)+ |
| 48 | 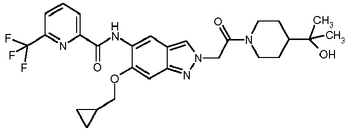 | 9-8 und 2-(Piperidin-4-yl)propan-2-ol | 2b (78% ) | (400 MHz, DMSO-d6): δ = 0.41 - 0.48 (m, 2 H), 0.62 - 0.69 (m, 2 H), 1.05 (s, 6 H), 1.09 (d, 1 H), 1.18 - 1.29 (m, 2 H), 1.29 - 1.40 (m, 1 H), 1.40 - 1.51 (m, 1 H), 1.75 (t, 2 H), 2.99 (t, 1 H), 4.02 (d, 3 H), 4.16 (s, 1 H), 4.42 (d, 1 H), 5.25 - 5.45 (m, 2 H), 7.04 (s, 1 H), 8.17 - 8.24 (m, 2 H), 8.41 (t, 1 H), 8.48 (d, 1 H), 8.75 (s, 1 H), 10.70 (s, 1 H). |
| | N-[6-(Cyclopropylmethoxy)-2- {2-[4-(2-hydroxypropan-2-yl)piperidin-1-yl]-2-oxoethyl}-2H-indazol-5-yl]-6-(trifluoromethyl)pyridin-2-carboxamid | | | |
| | | | | LC-MS (Methode A3): Rt = 1.27 min |
| | | | | MS (ESIpos): m/z = 560 (M+H)+ |
| 49 | 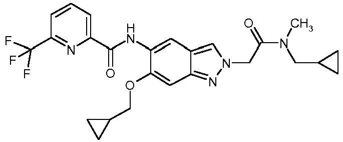 | 9-8 und 1-Cyclopropyl-N-methylmetha namin | 2b (54% ) | (400 MHz, DMSO-d6): δ = 0.22 (q, 1 H), 0.33 (d, 1 H), 0.41 - 0.48 (m, 3 H), 0.50 - 0.57 (m, 1 H), 0.62 - 0.69 (m, 2 H), 0.97 (br. s., 1 H), 1.30 - 1.41 (m, 1 H), 2.92 (s, 1 H) + 3.13 (s 2 H) 3.20 (d, 1 H), 3.34 (d, 1 H), 4.03 (d, 2 H), 5.29 - 5.43 (m, 2 H), 7.05 (s, 1 H), 8.19 - 8.25 (m, 2 H), 8.41 (t, 1 H), 8.48 (d, 1 H), 8.76 (s, 1 H), 10.70 (s, 1 H). |
| | N-[6-(Cyclopropylmethoxy)-2-{2-[(cyclopropylmethyl)(methyl)amin o]-2-oxoethyl}-2H-indazol-5-yl]-6-(trifluoromethyl)pyridin-2-carboxamid | | | |
| | | | | LC-MS (Methode A3): Rt = 1.38 min |
| | | | | MS (ESIpos): m/z = 502 (M+H)+ |
| 50 | 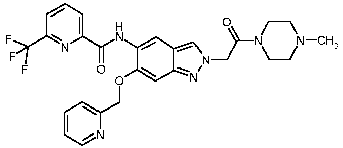 | 9-9 und 1-Methylpipera zin | 2b (75% ) | (300 MHz, DMSO-d6): δ = 2.21 (s, 3 H), 2.24 - 2.33 (m, 2 H), 2.37 (br. s., 2 H), 3.47 (br. s., 2 H), 3.55 (br. s., 2 H), 5.36 (s, 2 H), 5.40 (s, 2 H), 7.30 (s, 1 H), 7.36 - 7.47 (m, 1 H), 7.71 (d, 1 H), 7.79 - 7.90 (m, 1 H) 8.19 (dd, 1 H), 8.26 (s, 1 H), 8.34 - 8.44 (m, 1 H), 8.45 - 8.52 (m, 1 H), 8.62 (d, 1 H), 8.81 (s, 1 H), 10.50 (s, 1 H). |
| | N-{2-[2-(4-Methylpiperazin-1-yl)-2-oxoethyl]-6-(pyridin-2-ylmethoxy)-2H-indazol-5-yl}-6-(trifluoromethyl)pyridin-2-carboxamid | | | |
| | | | | LC-MS (Methode A3): Rt = 0.92 min |
| | | | | MS (ESIpos): m/z = 554 (M+H)+ |
| 51 | 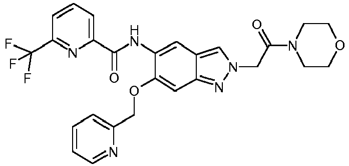 | 9-9 und Morpholin | 2b (23% ) | (400 MHz, DMSO-d6): δ = 3.40 - 3.52 (m, 2 H), 3.59 (d, 4 H), 3.62 - 3.68 (m, 2 H), 5.36 (s, 2 H), 5.41 (s, 2 H), 7.30 (s, 1 H), 7.42 (dd, 1 H), 7.70 (d, 1 H), 7.86 (td, 1 H), 8.15 - 8.23 (m, 1 H), 8.27 (s, 1 H), 8.39 (t, 1 H), 8.48 (d, 1 H), 8.62 (d, 1 H), 8.81 (s, 1 H), 10.50 (s, 1 H). |
| | N-{2-[2-(Morpholin-4-yl)-2-oxoethyl]-6-(pyridin-2-ylmethoxy)-2H-indazol-5-yl}-6-(trifluoromethyl)pyridin-2-carboxamid | | | |
| | | | | LC-MS (Methode A3): Rt = 1.11 min |
| | | | | MS (ESIpos): m/z = 541 (M+H)+ |
| 52 | 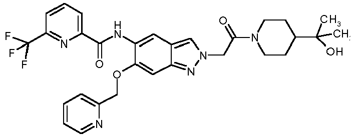 | 9-9 und 2-(Piperidin-4-yl)propan-2-ol | 2b (29% ) | (400 MHz, DMSO-d6): δ = 1.05 (s, 6 H), 1.17 - 1.34 (m, 3 H), 1.37 - 1.52 (m, 1 H), 1.76 (t, 2 H), 2.91 - 3.08 (m, 1 H), 4.04 (d, 1 H), 4.17 (s, 1 H), 4.42 (d, 1 H), 5.28 - 5.45 (m, 4 H), 7.29 (s, 1 H), 7.36 - 7.47 (m, 1 H), 7.70 (d, 1 H), 7.86 (td, 1 H), 8.19 (dd, 1 H), 8.26 (s, 1 H), 8.40 (t, 1 H), 8.48 (d, 1 H), 8.58 - 8.65 (m, 1 H), 8.81 (s, 1 H), 10.50 (s, 1 H). |
| | N-[2-{2-[4-(2-Hydroxypropan-2-yl)piperidin-1-yl]-2-oxoethyl}-6-(pyridin-2-ylmethoxy)-2H-indazol-5-yl]-6-(trifluoromethyl)pyridin-2-carb oxamid | | | |
| | | | | LC-MS (Methode A3): Rt = 1.15 min |
| | | | | MS (ESIpos): m/z = 597 (M+H)+ |
| 53 | 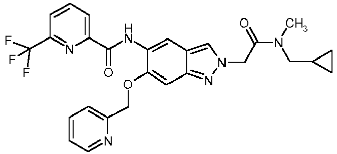 | 9-9 und 1-Cyclopropyl-N-methylmetha namin | 2b (44% ) | (400 MHz, DMSO-d6): δ = 0.19 - 0.26 (m, 1 H), 0.30 - 0.37 (m, 1 H), 0.40 - 0.49 (m, 1 H), 0.51 - 0.60 (m, 1 H), 0.91 - 1.02 (m, 1 H), 2.93 (s, 1 H)+ 3.14 (s, 2 H), 3.21 (d, 1 H), 3.35 (d, 1 H), 5.36 (s, 2 H), 5.39 (s, 2 H), 7.30 (s, 1 H), 7.37 - 7.45 (m, 1 H), 7.70 (d, 1 H), 7.86 (td, 1 H), 8.19 (dd, 1 H), 8.27 (d, 1 H), 8.40 (t, 1 H), 8.48 (d, 1 H), 8.62 (d, 1 H), 8.81 (s, 1 H), 10.50 (s, 1 H). |
| | N-[2-{2-[(cyclopropylmethyl)(methyl)amin o]-2-oxoethyl}-6-(pyridin-2-ylmethoxy)-2H-indazol-5-yl]-6-(trifluoromethyl)pyridin-2-carboxamid | | | |
| | | | | LC-MS (Methode A3): Rt = 1.24 min |
| | | | | MS (ESIpos): m/z = 539 (M+H)+ |
| 54 | 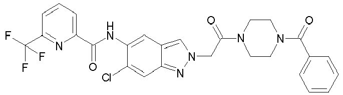 | 9-11 und Phenyl(piper azin-1-yl)methanon | 2c (95% ) | (300MHz, DMSO-d6): δ = 3.40 - 3.82 (m, 8H), 5.54 (br. s., 2H), 7.41 - 7.52 (m, 5H), 7.91 (s, 1H), 8.23 (dd, 1H), 8.37 - 8.49 (m, 3H), 8.64 (s, 1H), 10.5 (s, 1H). |
| | N-{2-[2-(4-Benzoylpiperazin-1-yl)-2-oxoethyl]-6-chlor-2H-indazol-5-yl}-6-(trifluormethyl)pyridin-2-carboxamid | | | |
| | | | | UPLC-MS (Methode A1): Rt = 1.22 min |
| | | | | MS (ESIpos): m/z = 571 (M+H)+ |
| 55 | 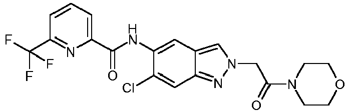 | 9-11 und Morpholin | 2d (44% ) | (400MHz, DMSO-d6): δ = 3.48 (d, 2H), 3.53 - 3.63 (m, 4H), 3.66 (d, 2H), 5.52 (s, 2H), 7.92 (s, 1H), 8.24 (d, 1H), 8.38 - 8.44 (m, 2H), 8.45 - 8.49 (m, 1H), 8.66 (s, 1H), 10.5 (br. s., 1H). |
| | N-{6-Chlor-2-[2-(morpholin-4-yl)-2-oxoethyl]-2H-indazol-5-yl}-6-(trifluormethyl)pyridin-2-carboxamid | | | |
| | | | | UPLC-MS (Methode A1): Rt = 1.16 min |
| | | | | MS (ESIpos): m/z = 468 (M+H)+ |
| 56 | 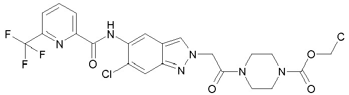 | 9-11 und Ethylpiperazi n-1-carboxylat | 2d (41% ) | (500MHz, DMSO-d6): δ = 1.21 (t, 3H), 3.37 - 3.63 (m, 9H), 4.08 (q, 2H), 5.54 (s, 2H), 7.92 (s, 1H), 8.24 (dd, 1H), 8.39 - 8.44 (m, 2H), 8.45 - 8.49 (m, 1H), 8.66 (s, 1H), 10.5 (s, 1H). |
| | Ethyl-4-{[6-chlor-5-({[6-(trifluormethyl)pyridin-2-yl]carbonyl}amino)-2H-indazol-2-yl]acetyl}piperazin-1-carboxylat | | | |
| | | | | UPLC-MS (Methode A1): Rt = 1.24 min |
| | | | | MS (ESIpos): m/z = 538 (M+H)+ |
| 57 | 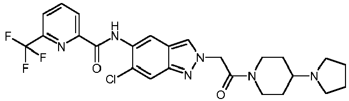 | 9-11 und 4-(Pyrrolidin-1-yl)piperidin | 2d (31% ) [c] | (400MHz, DMSO-d6): δ = 1.25 - 1.37 (m, 1H), 1.39 - 1.53 (m, 1H), 1.68 (br. s., 4H), 1.78 - 1.95 (m, 2H), 2.19 - 2.30 (m, 1H), 2.87 (t, 1H), 3.19 (t, 1H), 3.88 (d, 1H), 4.10 (d, 1H), 5.49 (d, 2H), 7.91 (s, 1H), 8.23 (d, 1H), 8.38 - 8.44 (m, 2H), 8.45 - 8.49 (m, 1H), 8.66 (s, 1H), 10.5 (br. s., 1H). |
| | N-(6-Chlor-2-{2-oxo-2-[4-(pyrrolidin-1-yl)piperidin-1-yl]ethyl}-2H-indazol-5-yl)-6-(trifluormethyl)pyridin-2-carboxamid | | | |
| | | | | UPLC-MS (Methode A1): Rt = 1.00 min |
| | | | | MS (ESIpos): m/z = 535 (M+H)+ |
| 58 | 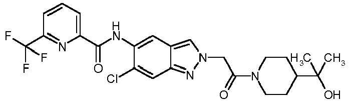 | 9-11 und 2-(Piperidin-4-yl)propan-2-ol | 2d (32% ) [d] | (400MHz, DMSO-d6, ausgewählte Signale): δ = 1.05 (s, 6H), 1.68 - 1.85 (m, 2H), 3.02 (t, 1H), 4.02 (d, 1H), 4.17 (s, 1H), 4.42 (d, 1H), 5.42 - 5.55 (m, 2H), 7.91 (s, 1H), 8.23 (d, 1H), 8.38 - 8.51 (m, 3H), 8.65 (s, 1H), 10.5 (s, 1H). |
| | N-(6-Chlor-2-{2-[4-(2-hydroxypropan-2-yl)piperidin-1-yl]-2-oxoethyl}-2H-indazol-5-yl)-6-(trifluormethyl)pyridin-2-carboxamid | | | |
| | | | | UPLC-MS (Methode A1): Rt = 1.18 min |
| | | | | MS (ESIpos): m/z = 524 (M+H)+ |
| 59 | 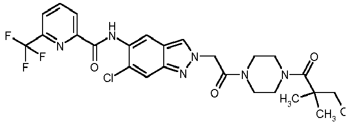 | 9-11 und 3-Hydroxy-2,2-dimethyl-1-(piperazin-1-yl)propan-1-on | 2d (39% ) [e] | (400MHz, DMSO-d6): δ = 1.18 (s, 6H), 3.39 - 3.72 (m, 10H), 4.61 (t, 1H), 5.54 (s, 2H), 7.92 (s, 1H), 8.24 (d, 1H), 8.38 - 8.44 (m, 2H), 8.45 - 8.50 (m, 1H), 8.66 (s, 1H), 10.5 (s, 1H). |
| | N-(6-Chlor-2-{2-[4-(3-hydroxy-2,2-dimethylpropanoyl)piperazin-1-yl]-2-oxoethyl}-2H-indazol-5-yl)-6-(trifluormethyl)pyridin-2-carboxamid | | | |
| | | | | UPLC-MS (Methode A1): Rt = 1.13 min |
| | | | | MS (ESIpos): m/z = 567 (M+H)+ |
| 60 | 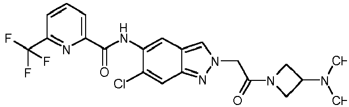 | 9-11 und N,N-Dimethylazet idin-3-amin | 2e (31% ) | (400MHz, DMSO-d6): δ = 2.08 (s, 6H), 3.07 - 3.14 (m, 1H), 3.70 (dd, 1H), 3.92 (dd, 1H), 4.02 (dd, 1H), 4.19 (t, 1H), 5.21 (s, 2H), 7.92 (s, 1H), 8.23 (dd, 1H), 8.37 - 8.49 (m, 3H), 8.64 (s, 1H), 10.5 (s, 1H). |
| | N-(6-Chlor-2-{2-[3-(dimethylamino)azetidin-1-yl]-2-oxoethyl}-2H-indazol-5-yl)-6-(trifluormethy l)pyridin- 2-carboxamid | | | |
| | | | | UPLC-MS (Methode A2): Rt = 1.13 min |
| | | | | MS (ESIpos): m/z = 481 (M+H)+ |
| 61 | 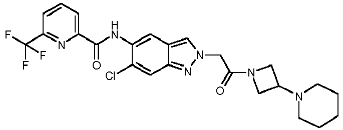 | 9-11 und 1-(Azetidin-3-yl)piperidin | 2e (53% ) [f] | (400MHz, CHLOROFORM-d): δ = 1.69 (br. s., 4H), 2.36 (br. s., 4H), 3.19 (br. s., 1H), 4.01 - 4.21 (m, 4H), 4.99 - 5.14 (m, 2H), 7.29 (s, 3H), 7.85 (s, 1H), 7.90 - 7.95 (m, 1H), 8.11 - 8.21 (m, 2H), 8.53 (d, 1H), 8.94 (s, 1H), 10.69 - 10.78 (m, 1H). |
| | N-(6-Chlor-2-{2-oxo-2-[3-(piperidin-1-yl)azetidin-1-yl]ethyl}-2H-indazol-5-yl)-6-(trifluormethyl)pyridin-2-carboxamid | | | |
| | | | | UPLC-MS (Methode A2): Rt = 1.28 min |
| | | | | MS (ESIpos): m/z = 521 (M+H)+ |
| 62 | 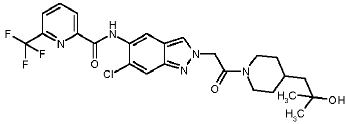 | 9-11 und 2-Methyl-1-(piperidin-4-yl)propan-2-ol | 2d (75% ) [g] | (300MHz, DMSO-d6): δ = 0.88 - 1.36 (m, 10H, enthält Singulett bei 1.11 ppm), 1.64 - 1.90 (m, 3H), 2.59 - 2.74 (m, überlagert durch DMSO-d6-Signal), 3.09 (t, 1H), 3.89 (d, 1H), 4.11 (s, 1H), 4.23 (d, 1H), 5.38 - 5.55 (m, 2H), 7.90 (s, 1H), 8.23 (dd, 1H), 8.37 - 8.49 (m, 3H), 8.63 (s, 1H), 10.5 (s, 1H). |
| | N-(6-Chlor-2-{2-[4-(2-hydroxy-2-methylpropyl)piperidin-1-yl]-2-oxoethyl}-2H-indazol-5-yl)-6-(trifluormethyl)pyridin-2-carboxamid | | | |
| | | | | UPLC-MS (Methode A1): Rt = 1.23 min |
| | | | | MS (ESIpos): m/z = 538 (M+H)+ |
| 63 | 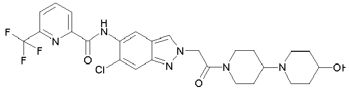 | 9-11 und 1,4'-Bipiperidin-4-ol | 2g (85% ) [h] | (300MHz, DMSO-d6): δ = 1.18 - 1.53 (m, 4H), 1.64 - 1.83 (m, 4H), 2.18 (t, 2H), 2.53 - 2.80 (m, 4H, überlagert durch DMSO-Signal), 3.06 (t, 1H), 3.36 - 3.46 (m, überlagert durch Wassersignal), 3.97 (d, 1H), 4.32 (d, 1H), 4.51 (d, 1H), 5.40 - 5.58 (m, 2H), 7.90 (s, 1H), 8.20 - 8.26 (m, 1H), 8.37 - 8.49 (m, 3H), 8.63 (s, 1H), 10.52 (s, 1 H). |
| | N-{6-Chlor-2-[2-(4-hydroxy-1,4'-bipiperidin-1'-yl)-2-oxoethyl]-2H-indazol-5-yl}-6-(trifluormethyl)pyridin-2-carboxamid | | | |
| | | | | UPLC-MS (Methode A1): Rt = 0.93 min |
| | | | | MS (ESIpos): m/z = 564 (M+H)+ |
| 64 | 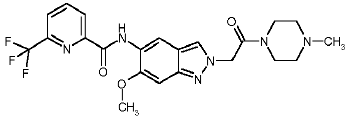 | 9-12 und 1-Methylpipera zin | 2f (57% ) | (400 MHz, DMSO-d6): δ = 2.21 (s, 3H), 2.30 (t, 2H), 2.37 (d, 2H), 3.47 (d, 2H), 3.51 - 3.59 (m, 2H), 3.99 (s, 3H), 5.39 (s, 2H), 7.11 (s, 1H), 8.18 - 8.26 (m, 2H), 8.37 - 8.43 (m, 1H), 8.44 - 8.49 (m, 1H), 8.71 (s, 1H), 10.51 (s, 1H). |
| | N-{6-Methoxy-2-[2-(4-methylpiperazin-1-yl)-2-oxoethyl]-2H-indazol-5-yl}-6-(trifluoromethyl)pyridine-2-carboxamid | | | |
| | | | | UPLC-MS (Methode A1): Rt = 0.91 min |
| | | | | MS (ESIpos): m/z = 477 (M+H)+ |
| 65 | 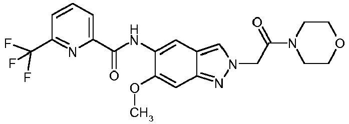 | 9-12 und Morpholin | 2b (85% ) | (300 MHz, DMSO-d6): δ = 3.42 - 3.51 (m, 2 H), 3.53 - 3.62 (m, 4 H), 3.62 - 3.68 (m, 2 H), 3.99 (s, 3 H), 5.40 (s, 2 H), 7.12 (s, 1 H), 8.19 - 8.25 (m, 2 H), 8.36 - 8.44 (m, 1 H), 8.44 - 8.50 (m, 1 H), 8.71 (s, 1 H), 10.51 (s, 1 H). |
| | N-{6-Methoxy-2-[2-(morpholin-4-yl)-2-oxoethyl]-2H-indazol-5-yl}-6-(trifluoromethyl)pyridin-2-carboxamid | | | |
| | | | | LC-MS (Methode A3): Rt = 1.14 min |
| | | | | MS (ESIpos): m/z = 464 (M+H)+ |
| 66 | 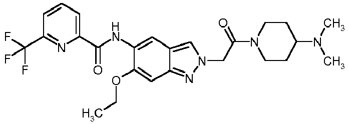 | 9-13 und N,N-Dimethylpip eridin-4-amin | 2g (63% ) | (400MHz, DMSO-d6): δ = 1.17 - 1.29 (m, 1H), 1.31 - 1.45 (m, 1H), 1.49 (t, 3H), 1.69 - 1.83 (m, 2H), 2.17 (s, 6H), 2.28 - 2.38 (m, 1H), 2.66 (t, 1H), 3.08 (t, 1H), 3.96 (d, 1H), 4.15 - 4.31 (m, 3H), 5.30 - 5.41 (m, 2H), 7.07 (s, 1H), 8.18 - 8.24 (m, 2H), 8.37 - 8.47 (m, 2H), 8.71 (s, 1H), 10.7 (s, 1H). |
| | N-(2-{2-[4-(Dimethylamino)piperidin-1-yl]-2-oxoethyl}-6-ethoxy-2H-indazol-5-yl)-6-(trifluormethyl)pyridin-2-carboxamid | | | |
| | | | | UPLC-MS (Methode A1): Rt = 0.93 min |
| | | | | MS (ESIpos): m/z = 519 (M+H)+ |
| 67 | 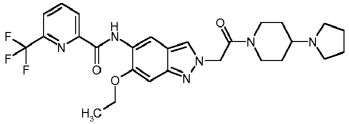 | 9-13 und 4-(Pyrrolidin-1-yl)piperidin | 2g (43% ) [i] | (400MHz, DMSO-d6): δ = 1.22 - 1.35 (m, 1H), 1.37 - 1.54 (m, 4H), 1.67 (br. s., 4H), 1.84 (t, 2H), 2.19 - 2.26 (m, 1H), 2.43 - 2.58 (überlagert durch DMSO-d6 Signal), 2.84 (t, 1H), 3.16 (t, 1H), 3.87 (d, 1H), 4.09 (d, 1H), 4.20 (q, 2H), 5.30 - 5.42 (m, 2H), 7.07 (s, 1H), 8.19 - 8.24 (m, 2H), 8.37 - 8.48 (m, 2H), 8.70 - 8.73 (m, 1H), 10.7 (s, 1H). |
| | N-(6-Ethoxy-2-{2-oxo-2-[4-(pyrrolidin-1-yl)piperidin-1-yl]ethyl}-2H-indazol-5-yl)-6-(trifluormethyl)pyridin-2-carboxamid | | | |
| | | | | UPLC-MS (Methode A1): Rt = 0.96 min |
| | | | | MS (ESIpos): m/z = 545 (M+H)+ |
| 68 | 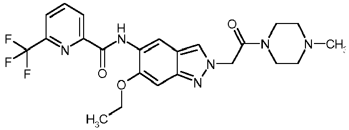 | 9-13 und 1-Methylpipera zin | 2g (51% ) [i] | (400MHz, DMSO-d6): δ = 1.49 (t, 3H), 2.20 (s, 3H), 2.26 - 2.41 (m, 4H), 3.42 - 3.58 (m, 4H), 4.20 (q, 2H), 5.37 (s, 2H), 7.07 (s, 1H), 8.18 - 8.24 (m, 2H), 8.37 - 8.47 (m, 2H), 8.71 (s, 1H), 10.7 (s, 1H). |
| | N-{6-Ethoxy-2-[2-(4-methylpiperazin-1-yl)-2-oxoethyl]-2H-indazol-5-yl}-6-(trifluormethyl)pyridin-2-carboxamid | | | |
| | | | | UPLC-MS (Methode A1): Rt = 0.92 min |
| | | | | MS (ESIpos): m/z = 491 (M+H)+ |
| 69 | 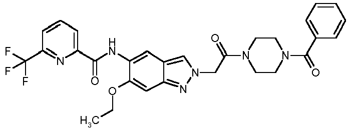 | 9-13 und Phenyl(piper azin-1-yl)methanon | 2g (71% ) | (400MHz, DMSO-d6): δ = 1.49 (t, 3H), 3.33 - 3.79 (m, 8H), 4.20 (q, 2H), 5.41 (br. s., 2H), 7.08 (s, 1H), 7.41 - 7.50 (m, 5H), 8.19 - 8.24 (m, 2H), 8.37 - 8.47 (m, 2H), 8.72 (s, 1H), 10.7 (s, 1H). |
| | N-{2-[2-(4-Benzoylpiperazin-1-yl)-2-oxoethyl]-6-ethoxy-2H-indazol-5-yl}-6-(trifluormethyl)pyridin-2-carboxamid | | | |
| | | | | UPLC-MS (Methode A1): Rt = 1.23 min |
| | | | | MS (ESIpos): m/z = 581 (M+H)+ |
| 70 | 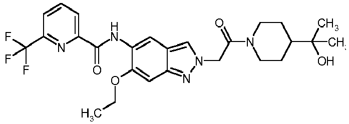 | 9-13 und 2-(Piperidin-4-yl)propan-2-ol | 2g (79% ) | (300MHz, DMSO-d6): δ = 0.97 - 1.29 (m, 8H, enthält Singulett bei 1.03), 1.37 - 1.56 (m, 4H), 1.74 (t, 2H), 2.42 - 2.63 (Signal verdeckt durch DMSO-d6 Signal) 2.98 (t, 1H), 4.02 (d, 1H), 4.14 - 4.25 (m, 3H), 4.40 (d, 1H), 5.27 - 5.43 (m, 2H), 7.07 (s, 1H), 8.17 - 8.24 (m, 2H), 8.36 - 8.48 (m, 2H), 8.71 (s, 1H), 10.7 (s, 1H). |
| | N-(6-Ethoxy-2-{2-[4-(2-hydroxypropan-2-yl)piperidin-1-yl]-2-oxoethyl}-2H-indazol-5-yl)-6-(trifluormethyl)pyridin-2-carboxamid | | | |
| | | | | UPLC-MS (Methode A1): Rt = 1.19 min |
| | | | | MS (ESIpos): m/z = 534 (M+H)+ |
| 71 | 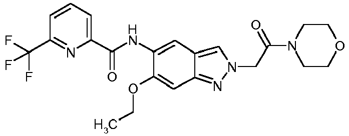 | 9-13 und Morpholin | 2g (89% ) | (300MHz, DMSO-d6): δ = 1.49 (t, 3H), 3.41 - 3.70 (m, 8H), 4.20 (q, 2H), 5.38 (s, 2H), 7.07 (s, 1H), 8.18 - 8.26 (m, 2H), 8.36 - 8.48 (m, 2H), 8.71 (s, 1H), 10.73 (s, 1H). |
| | N- {6-Ethoxy-2-[2-(morpholin-4-yl)-2-oxoethyl]-2H-indazol-5-yl}-6-(trifluormethyl)pyridin-2-carboxamid | | | |
| | | | | UPLC-MS (Methode A1): Rt = 1.16 min |
| | | | | MS (ESIpos): m/z = 478 (M+H)+ |

| | | | | |
|---|---|---|---|---|
| * Hergestellt nach angegebener Vorschrift, in Klammern ist die Ausbeute in % angegeben [a]: Das Piperazin wurde als Hydrochlorid eingesetzt. Zusätzlich zum Piperazin wurden 1.6 Äquivalente Triethylamin zur Reaktionsmischung gegeben. [b]: Das Produkt wurde mit präparativer HPLC nach Methode P1 gereinigt. [c]: Gradient für die präparative HPLC: iso. Ethanol / Methanol / Diethylamin 50:50:0.1; Flussrate: 35 ml/min [d]: Gradient für die präparative HPLC: iso. Hexan / Ethanol / Diethylamin 70:30:0.1; Flussrate: 40 ml/min [e]: Gradient für die präparative HPLC: iso. Hexan / Ethanol / Diethylamin 70:30:0.1; Flussrate: 31 ml/min [f]: Es wurde N,N-Dimethylformamid statt Dimethylsulfoxid verwendet. [g]: Die HPLC wurde nach Methode P1 durchgeführt. [h]: Es wurden 1.5 Äquivalente Piperazin eingesetzt. [i]: Das Produkt wurde in N,N-Dimethylformamid und Dimethylsulfoxid ausgerührt. | | | | |

### Beispiel 72

### N- {2-[2-(4-Benzoylpiperazin-1-yl)-2-oxoethyl]-3-methyl-2H-indazol-5-yl}-6-(trifluormethyl)pyridin-2-carboxamid

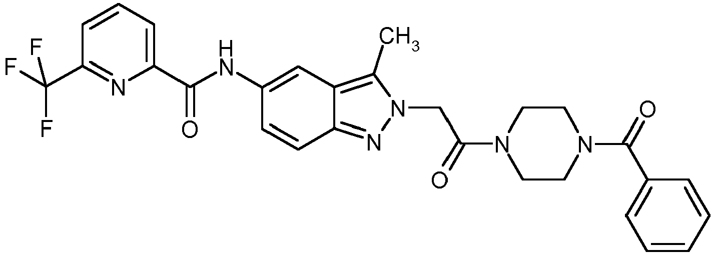

Analog zu Intermediat 8-1 wurden 103 mg (0.27 mmol) 2-(5-Amino-3-methyl-2H-indazol-2-yl)-1-(4-benzoylpiperazin-1-yl)ethanon (Intermediat 6-15, Rohprodukt) mit 78 mg (0.41 mmol) 6-(Trifluormethyl)pyridin-2-carbonsäure umgesetzt. Nach 24 h bei 25°C wurde mit Wasser versetzt. Der Feststoff wurde abfiltriert, mit Wasser und Diethylether gewaschen und im Vakuum getrocknet. Man erhielt 43 mg (29 % d.Th.) der Titelverbindung.
UPLC-MS (Methode A1): Rt = 1.12 min
MS (ESIpos): m/z = 551 (M+H)+.
¹H-NMR (300MHz, DMSO-d₆): δ = 3.34 - 3.73 (m, 8H), 5.48 (br. s., 2H), 7.42 - 7.58 (m, 7H), 8.14 - 8.23 (m, 2H), 8.32 - 8.43 (m, 2H), 10.35 (s, 1H).

### Beispiel 73

### N-{2-[3-(4-Benzoylpiperazin-1-yl)-3-oxopropyl]-2H-indazol-5-yl} -6-(trifluormethyl)pyridin-2-carboxamid

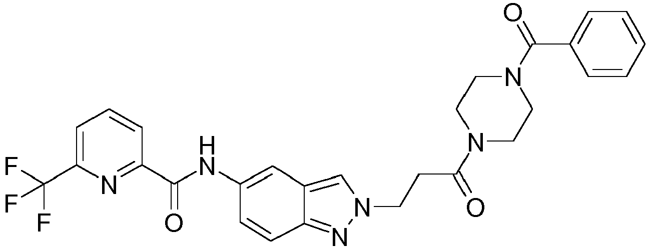

Analog zu Intermediat 8-1 wurden 80 mg (0.21 mmol) 3-[5-({[6-(Trifluormethyl)pyridin-2-yl]carbonyl}amino)-2H-indazol-2-yl]propansäure (Intermediat 9-15) in 0.3 ml *N,N-*Dimethylformamid und 2.9 ml Tetrahydrofuran mit 32 mg (0.21 mmol) 1-Hydroxy-1H-benzotriazol Hydrat und 81 mg (0.42 mmol) 1-(3-Dimethylaminopropyl)-3-ethylcarbodiimidhydrochlorid 30 Minuten gerührt. 60 mg (0.32 mmol) Phenyl(piperazin-1-yl)methanon wurden hinzugefügt. Die Reaktionsmischung wurde 2.5 h bei 25°C gerührt und in 50 ml Wasser getropft. Die wäßrige Phase wurde dreimal mit Ethylacetat extrahiert. Die vereinigten organischen Phasen wurden mit gesättigter Natriumchloridlösung gewaschen, über Natriumsulfat getrocknet, filtriert und eingeengt. Das Rohprodukt wurde in 2 ml Dimethylsulfoxid 30 min gerührt, filtriert und mit 30 ml Wasser gewaschen. Der Feststoff wurde mit präparativer HPLC nach Methode P1 gereinigt. Man erhielt 5 mg (4 % d.Th.) der Titelverbindung.
UPLC-MS (Methode A1): Rₜ = 1.10 min
MS (ESIpos): m/z = 551 (M+H)⁺
¹H NMR (400 MHz, DMSO-d6): δ = 3.10 (br. s., 2 H), 3.50 (br. s., 6 H), 4.65 (t, 2 H), 7.36 - 7.42 (m, 2 H), 7.42 - 7.47 (m, 3 H), 7.53 - 7.63 (m, 2 H), 8.17 (dd, 1 H), 8.28 (s, 1 H), 8.32 - 8.42 (m, 3 H), 10.35 (s, 1 H).

Die Beispielverbindungen der Tabellen 3 - 17 wurden im Rahmen einer Amidsynthese analog zu den Versuchsvorschriften 1a - 1g bzw. 2a - 2g oder durch eine in der Tabelle angegebene Methode synthetisiert und durch analytische LC-MS (Methode A4) analysiert.

**Tabelle 3: Beispiele 74 - 77**

| Die Beispielverbindungen wurden aus 2-(5-Amino-2H-indazol-2-yl)-1-[4-(cyclopropylcarbonyl)piperazin-1-yl]ethanon (Intermediat 6-10) und dem in der Tabelle angegebenen Edukt hergestellt. | | | |
|---|---|---|---|
| Beispiel | Struktur und Name | Edukt und Anmerkungen | LC-MS Retent ionszei t [min] |
| 74 | 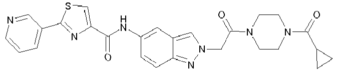 | 2-(Pyridin-3-yl)-1,3-thiazol-4-carbonsäure | 0.78 |
| | N-(2-{2-[4-(Cyclopropylcarbonyl)piperazin-1-yl]-2-oxoethyl}-2H-indazol-5-yl)-2-(pyridin-3-yl)-1,3-thiazol-4-carboxamid | | |
| 75 | 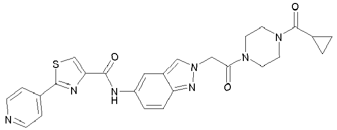 | 2-(Pyridin-4-yl)-1,3-thiazol-4-carbonsäure | 0.70 |
| | N-(2-{2-[4-(Cyclopropylcarbonyl)piperazin-1-yl]-2-oxoethyl}-2H-indazol-5-yl)-2-(pyridin-4-yl)-1,3-thiazol-4-carboxamid | | |
| 76 | 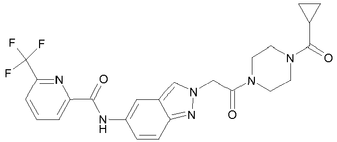 | 6-(Trifluormethyl)pyridin-2-carbonsäure | 0.94 |
| | N-(2-{2-[4-(Cyclopropylcarbonyl)piperazin-1-yl]-2-oxoethyl}-2H-indazol-5-yl)-6-(trifluormethyl)pyridin-2-carboxamid | | |
| 77 | 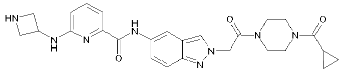 | Ausgehend von 2-(5-Amino-2H-indazol-2-yl)-1-[4-(cyclopropylcarbonyl)piperazin-1-yl]ethanon wurde mit 6-Fluorpyridin-2-carbonsäure umgesetzt. Man erhielt N-(2-{2-[4-(Cyclopropylcarbonyl)piperazin-1-yl]-2-oxoethyl} -2H-indazol-5-yl)-6-fluorpyridin-2-carboxamid, das mit 2 Äquiv. tert-Butyl-3-aminoazetidin-1-carboxylat und N-Ethyl-N-isopropylpropan-2-amin in NMP bei 100°C umgesetzt wurde. Das erhaltene Rohprodukt wurde dann mit Trifluoressigsäure in Dichlormethan umgesetzt. Nach Reinigung durch präparative HPLC erhielt man 14 mg der Beispielverbindung. | 0.54 |
| | 6-(Azetidin-3-ylamino)-N-(2-{2-[4-(cyclopropylcarbonyl)piperazin-1-yl]-2-oxoethyl}-2H-indazol-5-yl)pyridin-2-carboxamid | | |

**Tabelle 4: Beispiele 78 - 83**

| Die Beispielverbindungen wurden aus 2-(5-Amino-2H-indazol-2-yl)-1-(4-methylpiperazin-1-yl)ethanonund dem in der Tabelle angegebenen Edukt hergestellt. | | | |
|---|---|---|---|
| Beispiel | Struktur und Name | Edukt und Anmerkungen | LC-MS Retent ionszei t [min] |
| 78 | 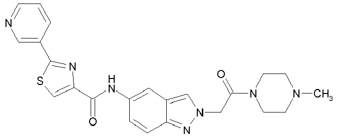 | 2-(Pyridin-3-yl)-1,3-thiazol-4-carbonsäure | 0.55 |
| | N-{2-[2-(4-Methylpiperazin-1-yl)-2-oxoethyl]-2H-indazol-5-yl} -2-(pyridin-3-yl)-1,3-thiazol-4-carboxamid | | |
| 79 | 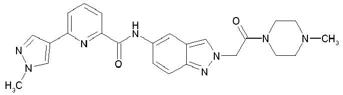 | 6-(1-Methyl-1H-pyrazol-4-yl)pyridin-2-carbonsäure | 0.60 |
| | N-{2-[2-(4-Methylpiperazin-1-yl)-2-oxoethyl]-2H-indazol-5-yl}-6-(1-methyl-1H-pyrazol-4-yl)pyridin-2-carboxamid | | |
| 80 | 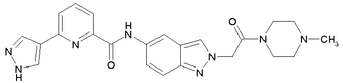 | 6-(1H-Pyrazol-4-yl)pyridin-2-carbonsäure | 0.54 |
| | N- {2-[2-(4-Methylpiperazin-1-yl)-2-oxoethyl]-2H-indazol-5-yl} -6-(1H-pyrazol-4-yl)pyridin-2-carboxamid | | |
| 81 | 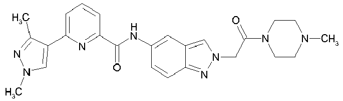 | 6-(1,3-Dimethyl-1H-pyrazol-4-yl)pyridin-2-carbonsäure | 0.64 |
| | 6-(1,3-Dimethyl-1H-pyrazol-4-yl)-N-{2-[2-(4-methylpiperazin-1-yl)-2-oxoethyl]-2H-indazol-5-yl}pyridin-2-carboxamid | | |
| 82 | 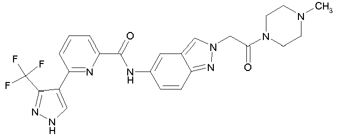 | 6-[3-(Trifluormethyl)-1H-pyrazol-4-yl]pyridin- 2-carbonsäure | 0.66 |
| | N- {2-[2-(4-Methylpiperazin-1-yl)-2-oxoethyl]-2H-indazol-5-yl}-6-[3-(trifluormethyl)-1H-pyrazol-4-yl]pyridin- 2-carboxamid | | |
| 83 | 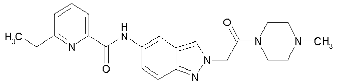 | 6-Ethylpyridin-2-carbonsäure | 0.66 |
| | 6-Ethyl-N- {2-[2-(4-methylpiperazin-1-yl)-2-oxoethyl]-2H-indazol-5-yl}pyridin-2-carboxamid | | |

**Tabelle 5: Beispiele 84 - 85**

| Die Beispielverbindungen wurden aus 2-(5-Amino-2H-indazol-2-yl)-1-[4-(2,2,2-trifluorethyl)piperazin-1-yl]ethanon (Intermediat 6-13) und dem in der Tabelle angegebenen Edukt hergestellt. | | | |
|---|---|---|---|
| Beispiel | Struktur und Name | Edukt und Anmerkungen | LC-MS Retent ionszei t [min] |
| 84 | 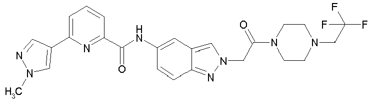 | 6-(1-Methyl-1H-pyrazol-4-yl)pyridin-2-carbonsäure | 0.97 |
| | 6-(1-Methyl-1H-pyrazol-4-yl)-N-(2- {2-oxo-2-[4-(2,2,2-trifluorethyl)piperazin-1-yl]ethyl}-2H-indazol-5-yl)pyridin-2-carboxamid | | |
| 85 | 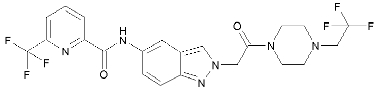 | 6-(Trifluormethyl)pyridin-2-carbonsäure | 1.10 |
| | N-(2-{2-Oxo-2-[4-(2,2,2-trifluorethyl)piperazin-1 -yl] ethyl}-2H-indazol-5-yl)-6-(trifluormethyl)pyridin-2-carboxamid | | |

**Tabelle 6: Beispiel 86**

| Die Beispielverbindung wurde aus 4-[(5-Amino-2H-indazol-2-yl)acetyl]-1-ethylpiperazin-2-on und dem in der Tabelle angegebenen Edukt hergestellt. | | | |
|---|---|---|---|
| Beispiel | Name und Struktur | Edukt und Anmerkungen | LC-MS Retent ionszei t [min] |
| 86 | 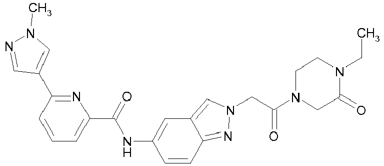 | 6-(1-Methyl-1H-pyrazol-4-yl)pyridin-2-carbonsäure | 0.79 |
| | N-{2-[2-(4-Ethyl-3-oxopiperazin-1-yl)-2-oxoethyl]-2H-indazol-5-yl}-6-(1-methyl-1H-pyrazol-4-yl)pyridin-2-carboxamid | | |

**Tabelle 7: Beispiele 87 - 121**

| Die Beispielverbindungen wurden aus 2-(5-Amino-2H-indazol-2-yl)-1-(4-benzoylpiperazin-1-yl)ethanon (Intermediat 6-11) und dem in der Tabelle angegebenen Edukt hergestellt. | | | |
|---|---|---|---|
| Beispiel | Name und Struktur | Edukt und Anmerkungen | LC-MS Retent ionszei t [min] |
| 87 | 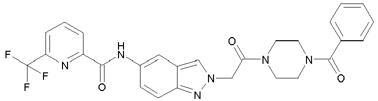 | 6-(Trifluormethyl)pyridin-2-carbonsäure | 1.02 |
| | N-{2-[2-(4-Benzoylpiperazin-1-yl)-2-oxoethyl]-2H-indazol-5-yl} -6-(trifluormethyl)pyridin-2-carboxamid | | |
| 88 | 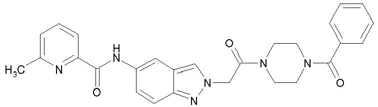 | 6-Methylpyridin-2-carbonsäure | 0.93 |
| | N-2-[2-(4-Benzoylpiperazin-1-yl)-2-oxoethyl]-2H-indazol-5-yl} -6-methylpyridin-2-carboxamid | | |
| 89 | 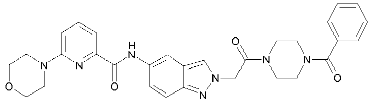 | 6-(Morpholin-4-yl)pyridin-2-carbonsäure | 0.94 |
| | N-{2-[2-(4-Benzoylpiperazin-1-yl)-2-oxoethyl]-2H-indazol-5-yl}-6-(morpholin-4-yl)pyridin-2-carboxamid | | |
| 90 | 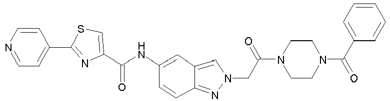 | 2-(Pyridin-4-yl)-1,3-thiazol-4-carbonsäure | 0.79 |
| | N-{2-[2-(4-Benzoylpiperazin-1-yl)-2-oxoethyl]-2H-indazol-5-yl}-2-(pyridin-4-yl)-1,3-thiazol-4-carboxamid | | |
| 91 | 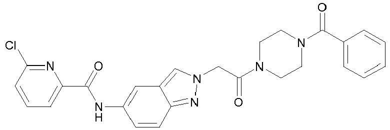 | 6-Chlorpyridin-2-carbonsäure | 0.96 |
| | N-{2-[2-(4-Benzoylpiperazin-1-yl)-2-oxoethyl]-2H-indazol-5-yl}-6-chlorpyridin-2-carboxamid | | |
| 92 | 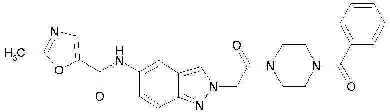 | 2-Methyl-1,3-oxazol-5-carbonsäure | 0.77 |
| | N-{2-[2-(4-Benzoylpiperazin-1-yl)-2-oxoethyl]-2H-indazol-5-yl}-2-methyl-1,3-oxazol-5-carboxamid | | |
| 93 | 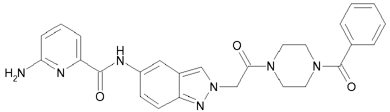 | 6-Aminopyridin-2-carbonsäure | 0.69 |
| | 6-Amino-N-{2-[2-(4-benzoylpiperazin-1-yl)-2-oxoethyl]-2H-indazol-5-yl}pyridin-2-carboxamid | | |
| 94 | 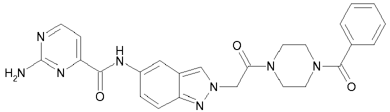 | 2-Aminopyrimidin-4-carbonsäure | 0.71 |
| | 2-Amino-N-{2-[2-(4-benzoylpiperazin-1-yl)-2-oxoethyl]-2H-indazol-5-yl}pyrimidin-4-carboxamid | | |
| 95 | 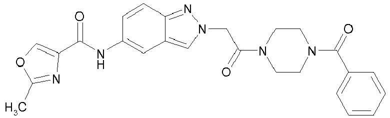 | 2-Methyl-1,3-oxazol-4-carbonsäure | 0.77 |
| | N-{2-[2-(4-Benzoylpiperazin-1-yl)-2-oxoethyl]-2H-indazol-5-yl}-2-methyl-1,3-oxazol-4-carboxamid | | |
| 96 | 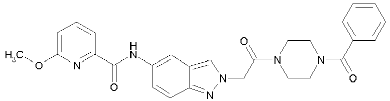 | 6-Methoxypyridin-2-carbonsäure | 0.96 |
| | N-{2-[2-(4-Benzoylpiperazin-1-yl)-2-oxoethyl]-2H-indazol-5-yl}-6-methoxypyridin-2-carboxamid | | |
| 97 | 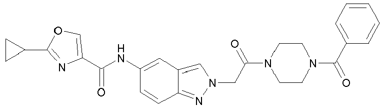 N-{2-[2-(4-Benzoylpiperazin-1-yl)-2-oxoethyl]-2H-indazol-5-yl}-2-cyclopropyl-1,3-oxazol-4-carboxamid | 2-Cyclopropyl-1,3-oxazol-4-carbonsäure | 0.89 |
| 98 | 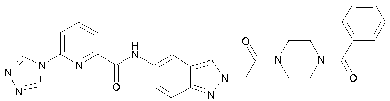 | 6-(4H-1,2,4-Triazol-4-yl)pyridin-2-carbonsäure | 0.74 |
| | N-{2-[2-(4-Benzoylpiperazin-1-yl)-2-oxoethyl]-2H-indazol-5-yl}-6-(4H-1,2,4-triazol-4-yl)pyridin-2-carboxamid | | |
| 99 | 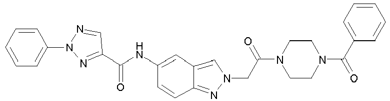 | 2-Phenyl-2H-1,2,3-triazol-4-carbonsäure | 1.04 |
| | N-{2-[2-(4-Benzoylpiperazin-1-yl)-2-oxoethyl]-2H-indazol-5-yl}-2-phenyl-2H-1,2,3-triazol-4-carboxamid | | |
| 100 | 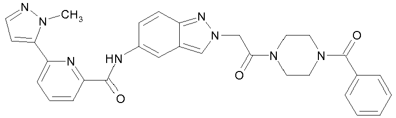 | 6-(1-Methyl-1H-pyrazol-5-yl)pyridin-2-carbonsäure | 0.93 |
| | N-{2-[2-(4-Benzoylpiperazin-1-yl)-2-oxoethyl]-2H-indazol-5-yl}-6-(1-methyl-1H-pyrazol-5-yl)pyridin-2-carboxamid | | |
| 101 | 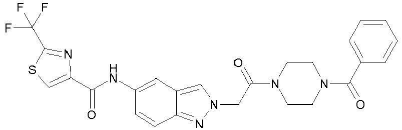 | 2-(Trifluormethyl)-1,3-thiazol-4-carbonsäure | 0.99 |
| | N-{2-[2-(4-Benzoylpiperazin-1-yl)-2-oxoethyl]-2H-indazol-5-yl}-2-(trifluormethyl)-1,3-thiazol-4-carboxamid | | |
| 102 | 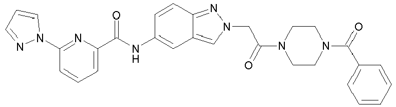 | 6-(1H-Pyrazol-1-yl)pyridin-2-carbonsäure | 0.97 |
| | N-{2-[2-(4-Benzoylpiperazin-1-yl)-2-oxoethyl]-2H-indazol-5-yl}-6-(1H-pyrazol-1-yl)pyridin-2-carboxamid | | |
| 103 | 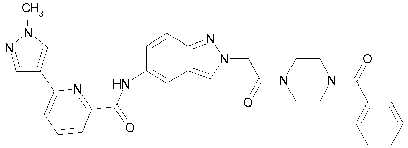 | 6-(1-Methyl-1H-pyrazol-4-yl)pyridin-2-carbonsäure | 0.91 |
| | N-{2-[2-(4-Benzoylpiperazin-1-yl)-2-oxoethyl]-2H-indazol-5-yl}-6-(1-methyl-1H-pyrazol-4-yl)pyridin-2-carboxamid | | |
| 104 | 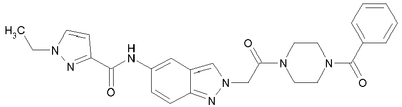 | 1-Ethyl-1H-pyrazol-3-carbonsäure | 0.81 |
| | N-{2-[2-(4-Benzoylpiperazin-1-yl)-2-oxoethyl]-2H-indazol-5-yl}-1 -ethyl-1H-pyrazol-3-carboxamid | | |
| 105 | 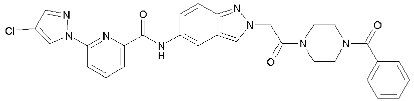 | 6-(4-Chlor-1H-pyrazol-1-yl)pyridin-2-carbonsäure | 1.11 |
| | N-{2-[2-(4-Benzoylpiperazin-1-yl)-2-oxoethyl]-2H-indazol-5-yl}-6-(4-chlor-1H-pyrazol-1-yl)pyridin-2-carboxamid | | |
| 106 | 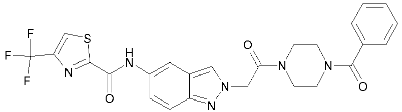 | 4-(Trifluormethyl)-1,3-thiazol-2-carbonsäure | 1.02 |
| | N-{2-[2-(4-Benzoylpiperazin-1-yl)-2-oxoethyl]-2H-indazol-5-yl}-4-(trifluormethyl)-1,3-thiazol-2-carboxamid | | |
| 107 | 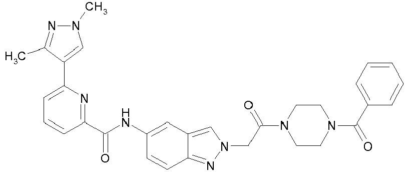 | 6-(1,3-Dimethyl-1H-pyrazol-4-yl)pyridin-2-carbonsäure | 0.95 |
| | N-{2-[2-(4-Benzoylpiperazin-1-yl)-2-oxoethyl]-2H-indazol-5-yl}-6-(1,3-dimethyl-1H-pyrazol-4-yl)pyridin-2-carboxamid | | |
| 108 | 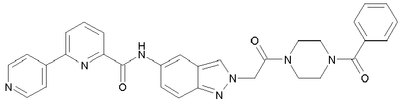 | 2,4'-Bipyridin-6-carbonsäure | 0.73 |
| | N-{2-[2-(4-Benzoylpiperazin-1-yl)-2-oxoethyl]-2H-indazol-5-yl}-2,4'-bipyridin-6-carboxamid | | |
| 109 | 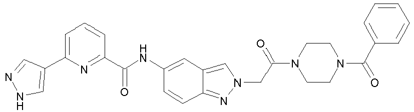 | 6-(1H-Pyrazol-4-yl)pyridin-2-carbonsäure | 0.84 |
| | N-{2-[2-(4-Benzoylpiperazin-1-yl)-2-oxoethyl]-2H-indazol-5-yl}-6-(1H-pyrazol-4-yl)pyridin-2-carboxamid | | |
| 110 | 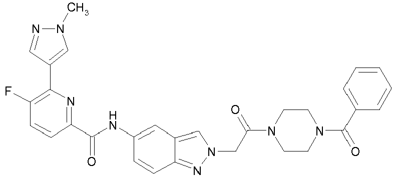 | 5-Fluor-6-(1-methyl-1H-pyrazol-4-yl)pyridin-2-carbonsäure | 0.96 |
| | N-{2-[2-(4-Benzoylpiperazin-1-yl)-2-oxoethyl]-2H-indazol-5-yl}-5-fluor-6-(1-methyl-1H-pyrazol-4-yl)pyridin-2-carboxamid | | |
| 111 | 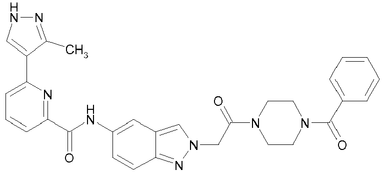 | 6-(3-Methyl-1H-pyrazol-4-yl)pyridin-2-carbonsäure | 0.88 |
| | N-{2-[2-(4-Benzoylpiperazin-1-yl)-2-oxoethyl]-2H-indazol-5-yl}-6-(3-methyl-1H-pyrazol-4-yl)pyridin-2-carboxamid | | |
| 112 | 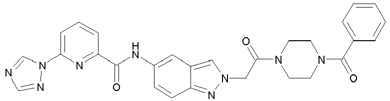 | 6-(1H-1,2,4-Triazol-1-yl)pyridin-2-carbonsäure | 0.85 |
| | N-{2-[2-(4-Benzoylpiperazin-1-yl)-2-oxoethyl]-2H-indazol-5-yl}-6-(1H-1,2,4-triazol-1-yl)pyridin-2-carboxamid | | |
| 113 | 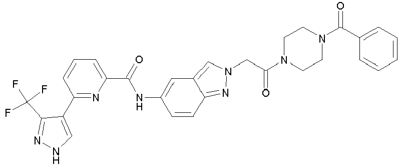 | 6-[3-(Trifluormethyl)-1H-pyrazol-4-yl]pyridin-2-carbonsäure | 0.97 |
| | N-{2-[2-(4-Benzoylpiperazin-1-yl)-2-oxoethyl]-2H-indazol-5-yl}-6-[3-(trifluormethyl)-1H-pyrazol-4-yl]pyridin-2-carboxamid | | |
| 114 | 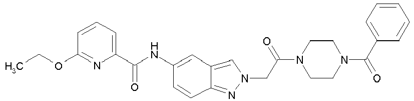 | 6-Ethoxypyridin-2-carbonsäure | 1.04 |
| | N-{2-[2-(4-Benzoylpiperazin-1-yl)-2-oxoethyl]-2H-indazol-5-yl}-6-ethoxypyridin- 2-carboxamid | | |
| 115 | 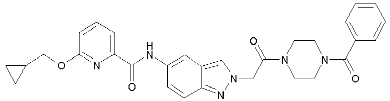 | 6-(Cyclopropylmethoxy)pyridin-2-carbonsäure | 1.11 |
| | N-{2-[2-(4-Benzoylpiperazin-1-yl)-2-oxoethyl]-2H-indazol-5-yl}-6-(cyclopropylmethoxy)pyridin-2-carboxamid | | |
| 116 | 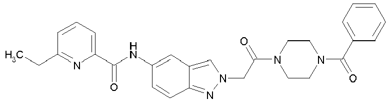 | 6-Ethylpyridin-2-carbonsäure | 1.03 |
| | N-{2-[2-(4-Benzoylpiperazin-1-yl)-2-oxoethyl]-2H-indazol-5-yl} -6-ethylpyridin-2-carboxamid | | |
| 117 | 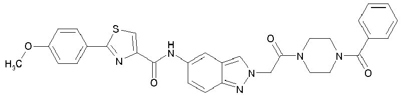 | 2-(4-Methoxyphenyl)-1,3-thiazol-4-carbonsäure | 1.12 |
| | N-{2-[2-(4-Benzoylpiperazin-1-yl)-2-oxoethyl]-2H-indazol-5-yl}-2-(4-methoxyphenyl)-1,3-thiazol-4-carboxamid | | |
| 118 | 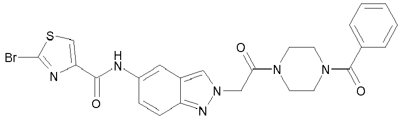 | 2-Brom-1,3-thiazol-4-carbonsäure | 0.93 |
| | N-{2-[2-(4-Benzoylpiperazin-1-yl)-2-oxoethyl]-2H-indazol-5-yl}-2-brom-1,3-thiazol-4-carboxamid | | |
| 119 | 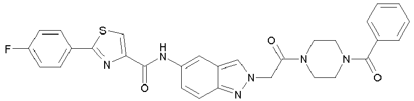 | 2-(4-Fluorphenyl)-1,3-thiazol-4-carbonsäure | 1.13 |
| | N-{2-[2-(4-Benzoylpiperazin-1-yl)-2-oxoethyl]-2H-indazol-5-yl}-2-(4-fluorphenyl)-1,3-thiazol-4-carboxamid | | |
| 120 | 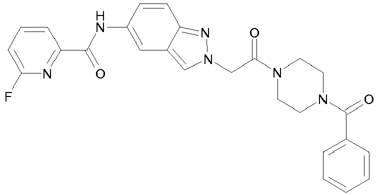 | 6-Fluorpyridin-2-carbonsäure | 0.89 |
| | N-{2-[2-(4-Benzoylpiperazin-1-yl)-2-oxoethyl]-2H-indazol-5-yl}-6-fluorpyridin-2-carboxamid | | |
| 121 | 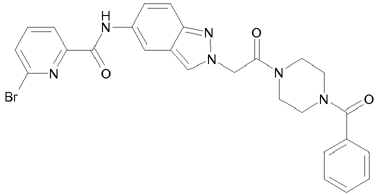 | 6-Brompyridin-2-carbonsäure | 0.98 |
| | N-{2-[2-(4-Benzoylpiperazin-1-yl)-2-oxoethyl]-2H-indazol-5-yl}-6-brompyridin-2-carboxamid | | |

**Tabelle 8: Beispiele 122 - 200**

| Die Beispielverbindungen wurden aus [5-({[6-(Trifluormethyl)pyridin-2-yl]carbonyl}amino)-2H-indazol-2-yl]essigsäure (Intermediat 9-14) und dem in der Tabelle angegebenen Edukt hergestellt. | | | |
|---|---|---|---|
| Beispiel | Struktur und Name | Edukt und Anmerkungen | LC-MS Retent ionszei t [min] |
| 122 | 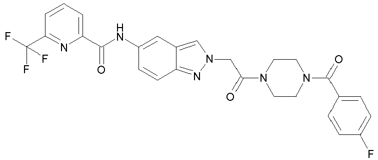 | (4-Fluorphenyl)(piperazin-1-yl)methanon | 1.05 |
| | N-(2-{2-[4-(4-Fluorbenzoyl)piperazin-1-yl]-2-oxoethyl}-2H-indazol-5-yl)-6-(trifluormethyl)pyridin-2-carboxamid | | |
| 123 | 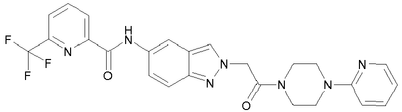 | 1-(Pyridin-2-yl)piperazin | 0.75 |
| | N-(2-{2-Oxo-2-[4-(pyridin-2-yl)piperazin-1-yl]ethyl}-2H-indazol-5-yl)-6-(trifluormethy l)pyridin- 2-carboxamid | | |
| 124 | 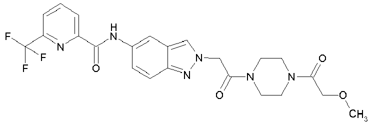 | 2-Methoxy-1 -(piperazin-1 - yl)ethanon | 0.86 |
| | N-(2-{2-[4-(Methoxyacetyl)piperazin-1-yl]-2-oxoethyl}-2H-indazol-5-yl)-6-(trifluormethy l)pyridin- 2-carboxamid | | |
| 125 | 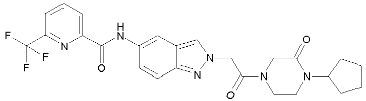 | 1-Cyclopentylpiperazin-2-on | 1.03 |
| | N-{2-[2-(4-Cyclopentyl-3-oxopiperazin-1-yl)-2-oxoethyl]-2H-indazol-5-yl}-6-(trifluormethyl)pyridin-2-carboxamid | | |
| 126 | 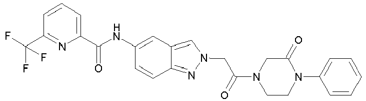 | 1-Phenylpiperazin-2-on | 1.01 |
| | N-{2-[2-Oxo-2-(3-oxo-4-phenylpiperazin-1 -yl)ethyl]-2H-indazol-5-yl} -6-(trifluormethy l)pyridin- 2-carboxamid | | |
| 127 | 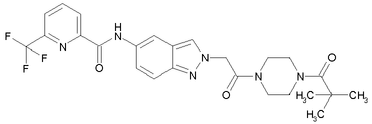 | 2,2-Dimethyl-1-(piperazin-1-yl)propan-1-on | 1.03 |
| | N-(2- {2-[4-(2,2-Dimethylpropanoyl)piperazin-1-yl]-2-oxoethyl}-2H-indazol-5-yl)-6-(trifluormethy l)pyridin- 2-carboxamid | | |
| 128 | 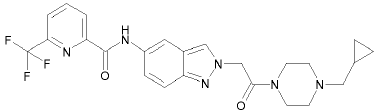 | 1-(Cyclopropylmethyl)-piperazin | 0.70 |
| | N-(2-{2-[4-(Cyclopropylmethyl)piperazin-1-yl]-2-oxoethyl} -2H-indazol-5-yl)-6-(trifluormethy l)pyridin- 2-carboxamid | | |
| 129 | 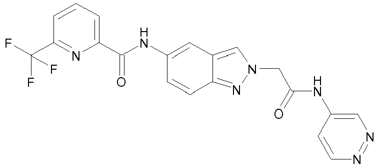 | Pyridazin-4-amin | 0.86 |
| | N-{2-[2-Oxo-2-(pyridazin-4-ylamino)ethyl]-2H-indazol-5-yl}-6-(trifluormethy l)pyridin- 2-carboxamid | | |
| 130 | 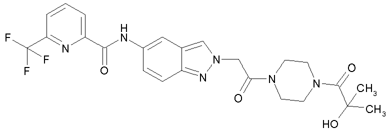 N-(2-{2-[4-(2-Hydroxy-2-methylpropanoyl)piperazin-1-yl]-2-oxoethyl}-2H-indazol-5-yl)-6-(trifluormethy l)pyridin- 2-carboxamid | 2-Hydroxy-2-methyl-1 - (piperazin-1-yl)propan-1-on | 0.89 |
| 131 | 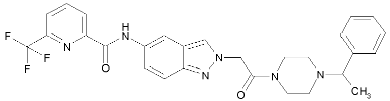 | 1-(1-Phenylethyl)piperazin | 0.79 |
| | N-(2- {2-Oxo-2-[4-(1-phenylethyl)piperazin-1-yl] ethyl}-2H-indazol-5-yl)-6-(trifluormethyl)pyridin-2-carboxamid | | |
| 132 | 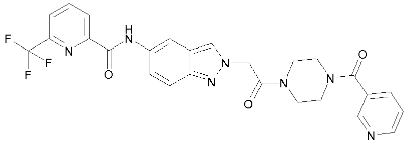 | Piperazin-1-yl(pyridin-3-yl)methanon | 0.86 |
| | N-(2-{2-Oxo-2-[4-(pyridin- 3-ylcarbonyl)piperazin-1-yl]ethyl}-2H-indazol-5-yl)-6-(trifluormethyl)pyridin-2-carboxamid | | |
| 133 | 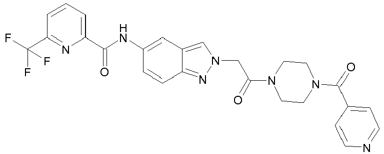 | Piperazin-1-yl(pyridin-4-yl)methanon | 0.83 |
| | N- {2-[2-(4-Isonicotinoylpiperazin-1-yl)-2-oxoethyl]-2H-indazol-5-yl}-6-(trifluormethy l)pyridin- 2-carboxamid | | |
| 134 | 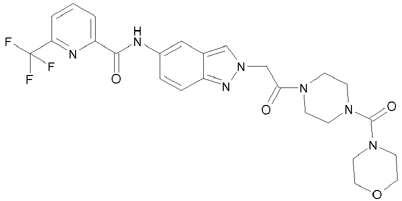 | Morpholin-4-yl(piperazin-1-yl)methanon | 0.90 |
| | N-(2-{2-[4-(Morpholin-4-ylcarbonyl)piperazin-1-yl]-2-oxoethyl}-2H-indazol-5-yl)-6-(trifluormethyl)pyridin-2-carboxamid | | |
| 135 | 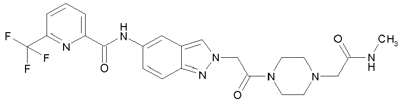 | N-Methyl-2-(piperazin-1-yl)acetamid | 0.69 |
| | N-[2-(2-{4-[2-(Methylamino)-2-oxoethyl]piperazin-1-yl}-2-oxoethyl)-2H-indazol-5-yl]-6-(trifluormethyl)pyridin-2-carboxamid | | |
| 136 | 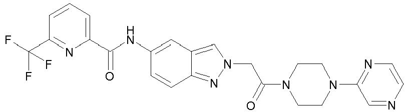 | 2-(Piperazin-1 -yl)pyrazin | 0.97 |
| | N-(2-{2-Oxo-2-[4-(pyrazin-2-yl)piperazin-1-yl]ethyl}-2H-indazol-5-yl)-6-(trifluormethy l)pyridin- 2-carboxamid | | |
| 137 | 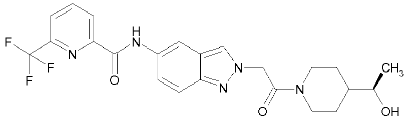 | (1R)-1-(Piperidin-4-yl)ethanol | 0.92 |
| | N-(2- {2- [4-(1-Hydroxyethyl)piperidin-1 - yl]-2-oxoethyl}-2H-indazol-5-yl)-6-(trifluormethy l)pyridin- 2-carboxamid | | |
| 138 | 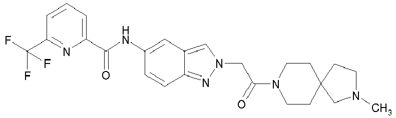 | 2-Methyl-2,8-diazaspiro[4.5]decan | 0.69 |
| | N-{2-[2-(2-Methyl-2,8-diazaspiro[ 4.5]dec-8-yl)-2-oxoethyl]-2H-indazol-5-yl}-6-(trifluormethy l)pyridin- 2-carboxamid | | |
| 139 | 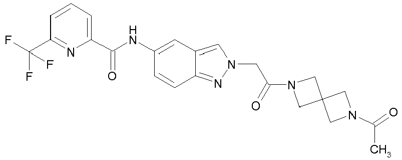 | 1-(2,6-Diazaspiro[3.3]hept-2-yl)ethanon | 0.84 |
| | N-{2-[2-(6-Acetyl-2,6-diazaspiro[3.3]hept-2-yl)-2-oxoethyl]-2H-indazol-5-yl}-6-(trifluormethy l)pyridin- 2-carboxamid | | |
| 140 | 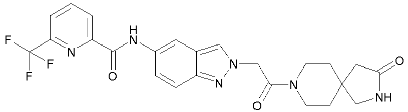 | 2,8-Diazaspiro[4.5]decan-3-on | 0.85 |
| | N-{2-[2-Oxo-2-(3-oxo-2,8-diazaspiro[4.5]dec-8-yl)ethyl]-2H-indazol-5-yl}-6-(trifluormethyl)pyridin-2-carboxamid | | |
| 141 | 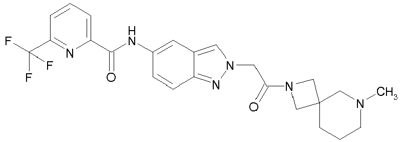 | 6-Methyl-2,6-diazaspiro[3.5]nonan | 0.67 |
| | N-{2-[2-(6-Methyl-2,6-diazaspiro[3.5]non-2-yl)-2-oxoethyl]-2H -indazol-5-yl}-6-(trifluormethy l)pyridin- 2-carboxamid | | |
| 142 | 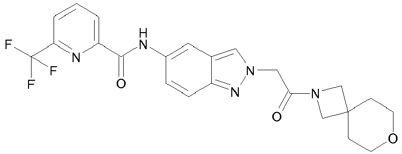 | 7-Oxa-2-azaspiro[3.5]nonan | 0.94 |
| | N-{2-[2-(7-Oxa-2-azaspiro[3.5]non-2-yl)-2-oxoethyl]-2H-indazol-5-yl} -6-(trifluormethy l)pyridin- 2-carboxamid | | |
| 143 | 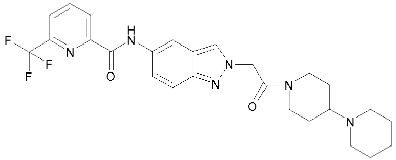 | 1,4'-Bipiperidin | 0.71 |
| | N-{2-[2-(1,4'-Bipiperidin-1'-yl)-2-oxoethyl]-2H-indazol-5-yl}-6-(trifluormethy l)pyridin- 2-carboxamid | | |
| 144 | 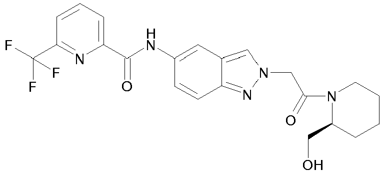 | (2S)- Piperidin-2-ylmethanol | 0.94 |
| | N-(2-{2-[2-(Hydroxymethyl)piperidin-1-yl]-2-oxoethyl}-2H-indazol-5-yl)-6-(trifluormethyl)pyridin-2-carboxamid | | |
| 145 | 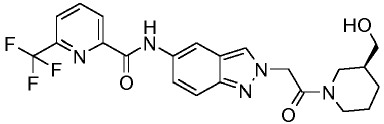 | (3 S)-Piperidin-3 -ylmethanol | 0.91 |
| | N-(2-{2-[3-(Hydroxymethyl)piperidin-1-yl]-2-oxoethyl}-2H-indazol-5-yl)-6-(trifluormethyl)pyridin-2-carboxamid | | |
| 146 | 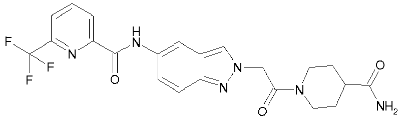 | Piperidin-4-carboxamid | 0.82 |
| | N-{2-[2-(4-Carbamoylpiperidin-1-yl)-2-oxoethyl]-2H-indazol-5-yl}-6-(trifluormethy l)pyridin- 2-carboxamid | | |
| 147 | 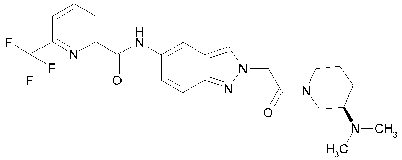 | (3R)-N,N-Dimethylpiperidin-3-amin | 0.68 |
| | N-(2-{2-[3-(Dimethylamino)piperidin-1-yl]-2-oxoethyl}-2H-indazol-5-yl)-6-(trifluormethy l)pyridin- 2-carboxamid | | |
| 148 | 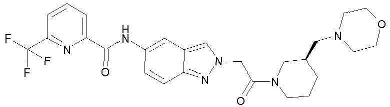 | 4-[(3S)-Piperidin-3-ylmethyl]morpholin | 0.71 |
| | N-(2-{2-[3-(Morpholin-4-ylmethyl)piperidin-1-yl]-2-oxoethyl}-2H-indazol-5-yl)-6-(trifluormethyl)pyridin-2-carboxamid | | |
| 149 | 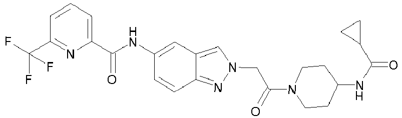 | N-(Piperidin-4-yl)cyclopropancarboxamid | 0.94 |
| | N-[2-(2- {4-[(Cyclopropylcarbonyl)amino]piperidin-1-yl}-2-oxoethyl)-2H-indazol-5-yl]-6-(trifluormethyl)pyridin-2-carboxamid | | |
| 150 | 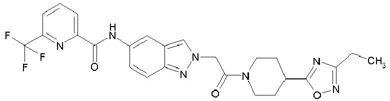 | 4-(3-Ethyl-1,2,4-oxadiazol-5-yl)piperidin | 1.08 |
| | N-(2-{2-[4-(3-Ethyl-1,2,4-oxadiazol-5-yl)piperidin-1-yl]-2-oxoethyl} -2H-indazol-5-yl)-6-(trifluormethyl)pyridin-2-carboxamid | | |
| 151 | 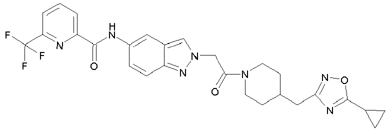 | 4-[(5-Cyclopropyl-1,2,4-oxadiazol-3-yl)methyl]piperidin | 1.12 |
| | N-[2-(2-{4-[(5-Cyclopropyl-1,2,4-oxadiazol-3-yl)methyl]piperidin-1-yl}-2-oxoethyl)-2H-indazol-5-yl]-6-(trifluormethyl)pyridin-2-carboxamid | | |
| 152 | 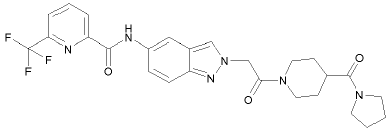 | Piperidin-4-yl(pyrrolidin-1-yl)methanon | 0.96 |
| | N-(2-{2-Oxo-2-[4-(pyrrolidin-1-ylcarbonyl)piperidin-1-yl]ethyl}-2H-indazol-5-yl)-6-(trifluormethyl)pyridin-2-carboxamid | | |
| 153 | 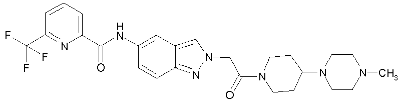 | 1-Methyl-4-(piperidin-4-yl)piperazin | 0.64 |
| | N-(2-{2-[4-(4-Methylpiperazin-1- yl)piperidin-1-yl]-2-oxoethyl} -2H-indazol-5-yl)-6-(trifluormethyl)pyridin-2-carboxamid | | |
| 154 | 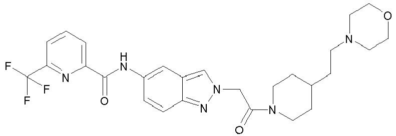 | 4-[2-(Piperidin-4-yl)ethyl]morpholin | 0.71 |
| | N-[2-(2-{4-[2-(Morpholin-4-yl)ethyl]piperidin-1-yl}-2-oxoethyl)-2H-indazol-5-yl]-6-(trifluormethyl)pyridin-2-carboxamid | | |
| 155 | 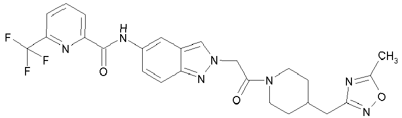 | 4-[(5-Methyl-1,2,4-oxadiazol-3-yl)methyl]piperidin | 1.03 |
| | N-[2-(2-{4-[(5-Methyl-1,2,4-oxadiazol-3-yl)methyl]piperidin-1-yl} -2-oxoethyl)-2H-indazol-5-yl]-6-(trifluormethyl)pyridin-2-carboxamid | | |
| 156 | 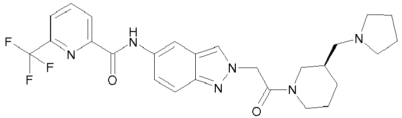 | (3S)-3-(Pyrrolidin-1-ylmethyl)piperidin | 0.72 |
| | N-(2-{2-Oxo-2-[3-(pyrrolidin-1-ylmethyl)piperidin-1-yl]ethyl}-2H-indazol-5-yl)-6-(trifluormethyl)pyridin-2-carboxamid | | |
| 157 | 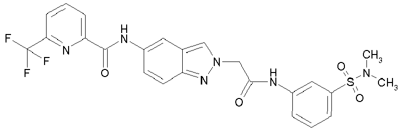 | 3-Amino-N,N-dimethylbenzolsulfonamid | 1.11 |
| | N-[2-(2-{[3-(Dimethylsulfamoyl)phenyl]amino}-2-oxoethyl)-2H-indazol-5-yl]-6-(trifluormethyl)pyridin-2-carboxamid | | |
| 158 | 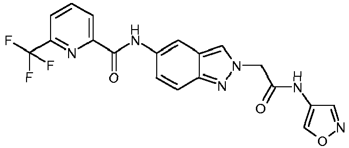 | 1,2-Oxazol-4-amin | 0.97 |
| | N-{2-[2-(1,2-Oxazol-4-ylamino)-2-oxoethyl]-2H-indazol-5-yl}-6-(trifluormethyl)pyridin-2-carboxamid | | |
| 159 | 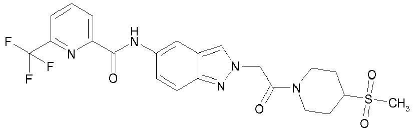 | 4-(Methylsulfonyl)piperidin | 0.89 |
| | N-(2-{2-[4-(Methylsulfonyl)piperidin-1-yl]-2-oxoethyl}-2H-indazol-5-yl)-6-(trifluormethyl)pyridin-2-carboxamid | | |
| 160 | 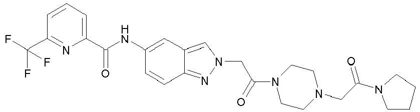 | 2-(Piperazin-1-yl)-1-(pyrrolidin-1-yl)ethanon | 0.71 |
| | N-[2-(2-Oxo-2-{4-[2-oxo-2-(pyrrolidin-1-yl)ethyl]piperazin-1-yl}ethyl)-2H-indazol-5-yl]-6-(trifluormethyl)pyridin-2-carboxamid | | |
| 161 | 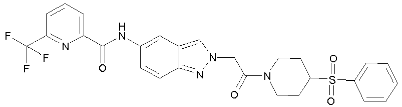 | 4-(Phenylsulfonyl)piperidin | 1.08 |
| | N-(2-{2-Oxo-2-[4-(phenylsulfonyl)piperidin-1-yl]ethyl}-2H-indazol-5-yl)-6-(trifluormethyl)pyridin-2-carboxamid | | |
| 162 | 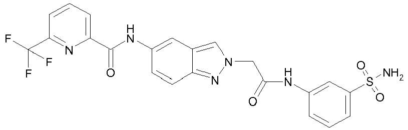 | 3-Aminobenzolsulfonamid | 0.96 |
| | N-(2-{2-Oxo-2-[(3-sulfamoylphenyl)amino]ethyl}-2H-indazol-5-yl)-6-(trifluormethyl)pyridin-2-carboxamid | | |
| 163 | 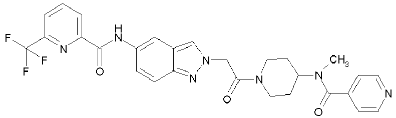 | N-Methyl-N-(piperidin-4-yl)isonicotinamid | 0.85 |
| | N-[2-(2-{4-[Isonicotinoyl(methyl)amino]piperidin-1-yl}-2-oxoethyl)-2H-indazol-5-yl]-6-(trifluormethyl)pyridin-2-carboxamid | | |
| 164 | 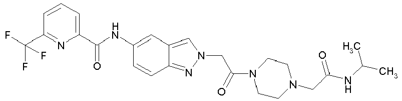 | N-Isopropyl-2-(piperazin-1-yl)acetamid | 0.74 |
| | N-[2-(2-{4-[2-(Isopropylamino)-2-oxoethyl]piperazin-1-yl}-2-oxoethyl)-2H-indazol-5-yl]-6-(trifluormethyl)pyridin-2-carboxamid | | |
| 165 | 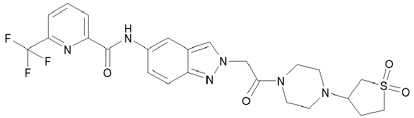 | 1-(1,1-Dioxidotetrahydrothiophen-3- yl)piperazin | 0.82 |
| | N-(2-{2-[4-(1,1-Dioxidotetrahydrothiophen-3-yl)piperazin-1-yl]-2-oxoethyl}-2H-indazol-5-yl)-6-(trifluormethyl)pyridin-2-carboxamid | | |
| 166 | 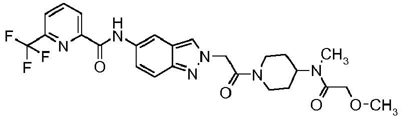 | 2-Methoxy-N-methyl-N-(piperidin-4-yl)acetamid | 0.90 |
| | N-[2-(2-{4-[(Methoxyacetyl)(methyl)amino]piperidin-1-yl}-2-oxoethyl)-2H-indazol-5-yl]-6-(trifluormethyl)pyridin-2-carboxamid | | |
| 167 | 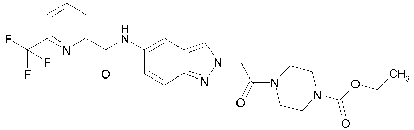 | Ethylpiperazin-1-carboxylat | 1.01 |
| | Ethyl-4-{[5-({[6-(trifluormethyl)pyridin-2-yl]carbonyl} amino)-2H-indazol-2-yl] acetyl}piperazin-1-carboxylat | | |
| 168 | 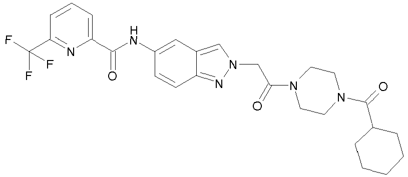 | Cyclohexyl(piperazin-1-yl)methanon | 1.10 |
| | N-(2-{2-[4-(Cyclohexylcarbonyl)piperazin-1-yl]-2-oxoethyl}-2H-indazol-5-yl)-6-(trifluormethyl)pyridin-2-carboxamid | | |
| 169 | 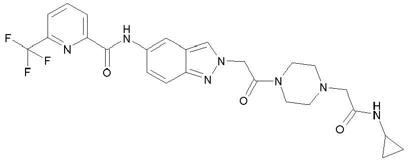 | N-Cyclopropyl-2-(piperazin-1- yl)acetamid | 0.72 |
| | N-[2-(2-{4-[2-(Cyclopropylamino)-2-oxoethyl]piperazin-1-yl}-2-oxoethyl)-2H-indazol-5-yl]-6-(trifluormethyl)pyridin-2-carboxamid | | |
| 170 | 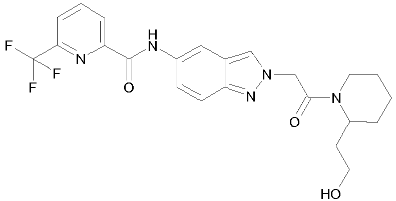 | 2-(Piperidin-2-yl)ethanol | 0.98 |
| | N-(2-{2-[2-(2-Hydroxyethyl)piperidin-1-yl]-2-oxoethyl1-2H-indazol-5-yl)-6-(trifluormethyl)pyridin-2-carboxamid | | |
| 171 | 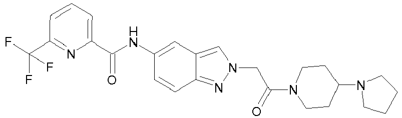 | 4-(Pyrrolidin-1-yl)piperidin | 0.69 |
| | N-(2-{2-Oxo-2-[4-(pyrrolidin-1-yl)piperidin-1 -yl]ethyl}-2H-indazol-5-yl)-6-(trifluormethyl)pyridin-2-carboxamid | | |
| 172 | 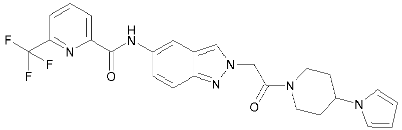 | 4-(1H-Pyrrol-1-yl)piperidin | 1.13 |
| 173 | 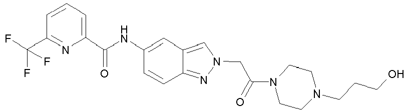 | 3 -(Piperazin-1 -yl)propan-1-ol | 0.65 |
| | N-(2-{2-[4-(3-Hydroxypropyl)piperazin-l - yl]-2-oxoethyl}-2H-indazol-5-yl)-6-(trifluormethyl)pyridin-2-carboxamid | | |
| 174 | 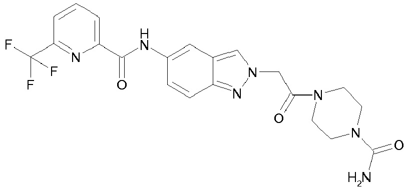 | Piperazin-1 -carboxamid | 0.81 |
| | 4- {[5-({[6-(Trifluormethyl)pyridin-2-yl]carbonyl}amino)-2H-indazol-2-yl]acetyl} piperazin-1-carboxamid | | |
| 175 | 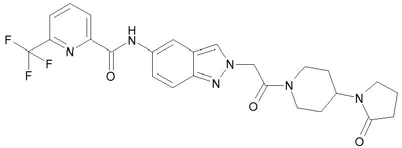 | 1-(Piperidin-4-yl)pyrrolidin-2-on | 0.92 |
| | N-(2-{2-Oxo-2-[4-(2-oxopyrrolidin-1-yl)piperidin-1 -yl]ethyl}-2H-indazol-5-yl)-6-(trifluormethyl)pyridin-2-carboxamid | | |
| 176 | 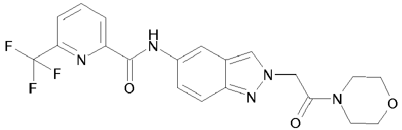 | Morpholin | 0.90 |
| | N-{2-[2-(Morpholin-4-yl)-2-oxoethyl]-2H-indazol-5-yl}-6-(trifluormethyl)pyridin-2-carboxamid | | |
| 177 | 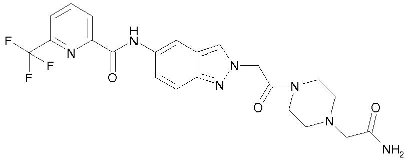 | 2-(Piperazin-1 -yl)acetamid | 0.67 |
| | N-(2-{2-[4-(2-Amino-2-oxoethyl)piperazin-1-yl]-2-oxoethyl} -2H-indazol-5-yl)-6-(trifluormethy l)pyridin- 2-carboxamid | | |
| 178 | 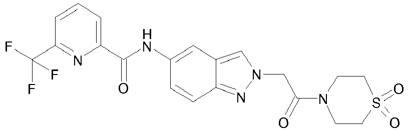 | Thiomorpholin-1,1-dioxid | 0.89 |
| | N-{2-[2-(1,1-Dioxidothiomorpholin-4-yl)-2-oxoethyl]-2H-indazol-5-yl} -6-(trifluormethy l)pyridin- 2-carboxamid | | |
| 179 | 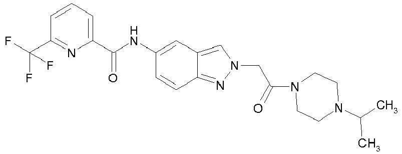 | 1-Isopropylpiperazin | 0.69 |
| | N-{2-[2-(4-Isopropylpiperazin-1-yl)-2-oxoethyl]-2H-indazol-5-yl}-6-(trifluormethyl)pyridin-2-carboxamid | | |
| 180 | 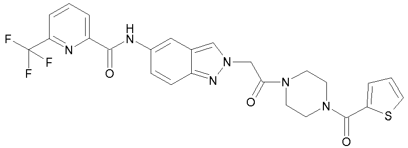 | Piperazin-1-yl(2-thienyl)methanon | 1.01 |
| | N-(2-{2-Oxo-2-[4-(2-thienylcarbonyl)piperazin-1-yl]ethyl}-2H-indazol-5-yl)-6-(trifluormethyl)pyridin-2-carboxamid | | |
| 181 | 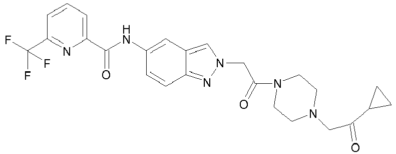 | 1-Cyclopropyl-2-(piperazin-1-yl)ethanon | 0.72 |
| | N-(2-{2-[4-(2-Cyclopropyl-2-oxoethyl)piperazin-1-yl]-2-oxoethyl}-2H-indazol-5-yl)-6-(trifluormethyl)pyridin-2-carboxamid | | |
| 182 | 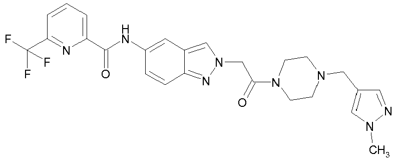 | 1-[(1-Methyl-1H-pyrazol-4-yl)methyl]piperazin | 0.68 |
| | N-[2-(2-{4-[(1-Methyl-1H-pyrazol-4-yl)methyl]piperazin-1-yl}-2-oxoethyl)-2H-indazol-5-yl]-6-(trifluormethyl)pyridin-2-carboxamid | | |
| 183 | 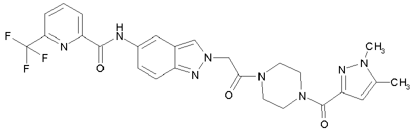 | (1,5-Dimethyl-1H-pyrazol-3-yl)(piperazin-1-yl)methanon | 0.95 |
| | N-[2-(2-{4-[(1,5-Dimethyl-1H-pyrazol-3-yl)carbonyl]piperazin-1-yl}-2-oxoethyl)-2H-indazol-5-yl]-6-(trifluormethyl)pyridin-2-carboxamid | | |
| 184 | 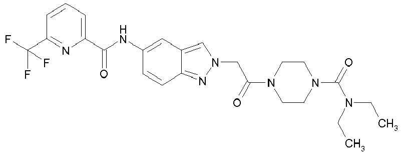 | N,N-Diethylpiperazin-1-carboxamid | 1.04 |
| | N,N-Diethyl-4-{[5-({[6-(trifluormethyl)pyridin-2-yl]carbonyl} amino)-2H-indazol-2-yl]acetyl}piperazin-1-carboxamid | | |
| 185 | 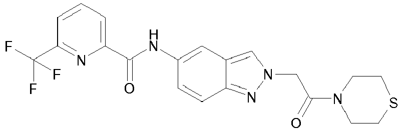 | Thiomorpholin | 1.01 |
| | N-{2-[2-Oxo-2-(thiomorpholin-4-yl)ethyl]-2H-indazol-5-yl}-6-(trifluormethyl)pyridin-2-carboxamid | | |
| 186 | 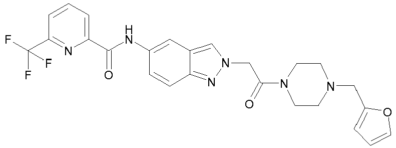 | 1-(2-Furylmethyl)piperazin | 0.74 |
| | N-(2-{2-[4-(2-Furylmethyl)piperazin-1-yl]-2-oxoethyl}-2H-indazol-5-yl)-6-(trifluormethyl)pyridin-2-carboxamid | | |
| 187 | 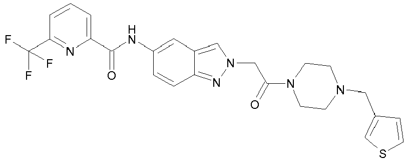 | 1-(3-Thienylmethyl)piperazin | 0.76 |
| | N-(2-{2-Oxo-2-[4-(3-thienylmethyl)piperazin-1-yl]ethyl}-2H-indazol-5-yl)-6-(trifluormethyl)pyridin-2-carboxamid | | |
| 188 | 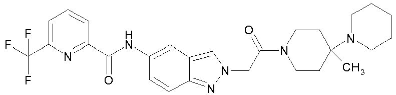 | 4'-Methyl-1,4'-bipiperidin | 0.72 |
| | N-{2-[2-(4'-Methyl-1,4'-bipiperidin-1'-yl)-2-oxoethyl]-2H-indazol-5-yl}-6-(trifluormethyl)pyridin-2-carboxamid | | |
| 189 | 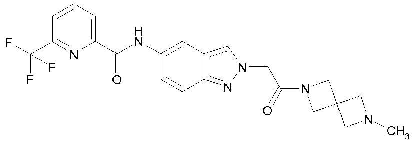 | 2-Methyl-2,6-diazaspiro[3.3]heptan | 0.65 |
| | N-{2-[2-(6-Methyl-2,6-diazaspiro[3.3]hept-2-yl)-2-oxoethyl]-2H-indazol-5-yl}-6-(trifluormethyl)pyridin-2-carboxamid | | |
| 190 | 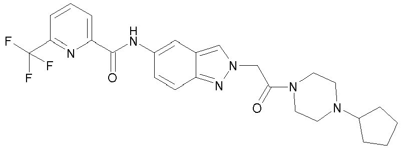 | 1-Cyclopentylpiperazin | 0.72 |
| | N-{2-[2-(4-Cyclopentylpiperazin-1-yl)-2-oxoethyl]-2H-indazol-5-yl}-6-(trifluormethyl)pyridin-2-carboxamid | | |
| 191 | 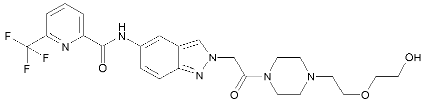 | 2-[2-(Piperazin-1-yl)ethoxy]ethanol | 0.66 |
| | N-[2-(2-{4-[2-(2-Hydroxyethoxy)ethyl]piperazin-1-yl}-2-oxoethyl)-2H-indazol-5-yl]-6-(trifluormethyl)pyridin-2-carboxamid | | |
| 192 | 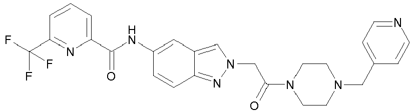 | 1-(Pyridin-4-ylmethyl)piperazin | 0.70 |
| | N-(2-{2-Oxo-2-[4-(pyridin-4-ylmethyl)piperazin-1-yl]ethyl}-2H-indazol-5-yl)-6-(trifluormethyl)pyridin-2-carboxamid | | |
| 193 | 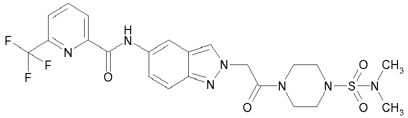 | N,N-Dimethylpiperazin-1-sulfonamid | 1.01 |
| | N-(2-{2-[4-(Dimethylsulfamoyl)piperazin-1-yl]-2-oxoethyl}-2H-indazol-5-yl)-6-(trifluormethyl)pyridin-2-carboxamid | | |
| 194 | 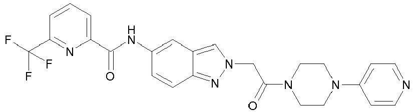 | 1-(Pyridin-4-yl)piperazin | 0.70 |
| | N-(2-{2-Oxo-2-[4-(pyridin-4-yl)piperazin-1-yl]ethyl}-2H-indazol-5-yl)-6-(trifluormethyl)pyridin-2-carboxamid | | |
| 195 | 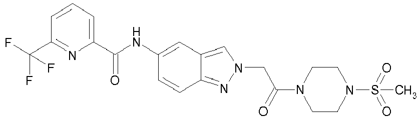 | 1-(Methylsulfonyl)piperazin | 0.92 |
| | N-(2-{2-[4-(Methylsulfonyl)piperazin-1- yl]-2-oxoethyl}-2H-indazol-5-yl)-6-(trifluormethyl)pyridin-2-carboxamid | | |
| 196 | 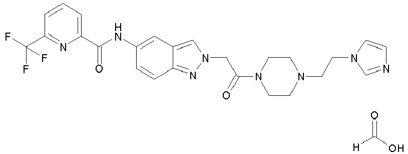 | 1-[2-(1H-Imidazol-1- yl)ethyl]piperazin | 0.64 |
| | Ameisensäure-N-[2-(2-{4-[2-(1H-imidazol-1-yl)ethyl]piperazin-1-yl}-2-oxoethyl)-2H-indazol-5-yl]-6-(trifluormethyl)pyridin-2-carboxamid(l:l) | | |
| 197 | 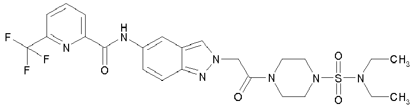 | N,N-Diethylpiperazin-1- sulfonamid | 1.11 |
| | N-(2-{2-[4-(Diethylsulfamoyl)piperazin- yl]-2-oxoethyl}-2H-indazol-5-yl)-6-(trifluormethyl)pyridin-2-carboxamid | | |
| 198 | 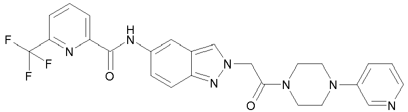 | 1-(Pyridin-3-yl)piperazin | 0.72 |
| | N-(2-{2-Oxo-2-[4-(pyridin-3-yl)piperazin-1-yl]ethyl}-2H-indazol-5-yl)-6-(trifluormethyl)pyridin-2-carboxamid | | |
| 199 | 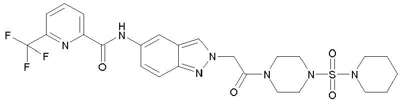 | 1-(Piperidin-1-ylsulfonyl)piperazin | 1.14 |
| | N-(2-{2-Oxo-2-[4-(piperidin-1- ylsulfonyl)piperazin-1-yl]ethyl}-2H-indazol-5-yl)-6-(trifluormethyl)pyridin-2-carboxamid | | |
| 200 | 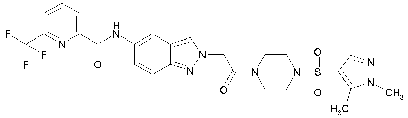 | 1-[(1,5-Dimethyl-1H-pyrazol-4-yl)sulfonyl]piperazin | 1.00 |
| | N-[2-(2-{4-[(1,5-Dimethyl-1H-pyrazol-4-yl)sulfonyl]piperazin-1-yl}-2-oxoethyl)-2H-indazol-5-yl]-6-(trifluormethyl)pyridin-2-carboxamid | | |

**Tabelle 9: Beispiele 201 -205**

| Die Beispielverbindungen wurden aus [5-({[6-(1-Methyl-1H-pyrazol-4-yl)pyridin-2-yl]carbonyl}amino)-2H-indazol-2-yl]essigsäure (Intermediat9-10) und dem in der Tabelle angegebenen Edukt hergestellt. | | | |
|---|---|---|---|
| Beispiel | Name und Struktur | Edukt und Anmerkungen | LC-MS Retent ionszei t [min] |
| 201 | 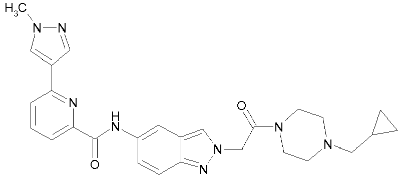 | 1-(Cyclopropylmethyl)-piperazin | 0.64 |
| | N-(2-{2-[4-(Cyclopropylmethyl)piperazin-1-yl]-2-oxoethyl}-2H-indazol-5-yl)-6-(1-methyl-1H-pyrazol-4-yl)pyridin-2-carboxamid | | |
| 202 | 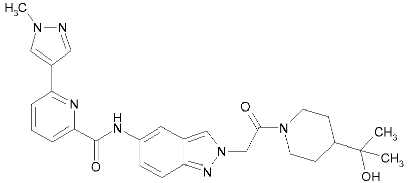 | 2-(Piperidin-4-yl)propan-2-ol | 0.85 |
| | N-(2-{2-[4-(2-Hydroxypropan-2-yl)piperidin-1-yl]-2-oxoethyl} -2H-indazol-5-yl)-6-(1-methyl-1H-pyrazol-4-yl)pyridin-2-carboxamid | | |
| 203 | 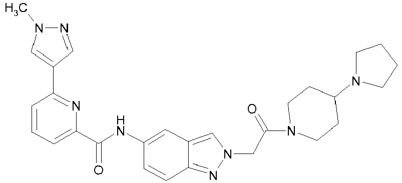 | 4-(Pyrrolidin-1-yl)piperidin | 0.63 |
| | 6-(1-Methyl-1H-pyrazol-4-yl)-N-(2- {2-oxo-2-[4-(pyrrolidin-1-yl)piperidin-1-yl]ethyl}-2H-indazol-5-yl)pyridin-2-carboxamid | | |
| 204 | 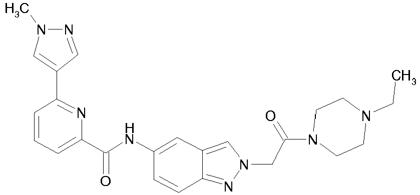 | 1-Ethylpiperazin | 0.61 |
| | N-{2-[2-(4-Ethylpiperazin-1-yl)-2-oxoethyl]-2H-indazol-5-yl}-6-(1-methyl-1H-pyrazol-4-yl)pyridin-2-carboxamid | | |
| 205 | 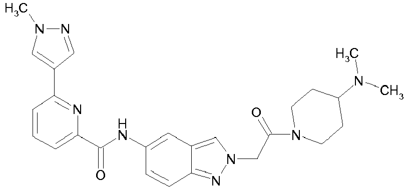 | N,N-Dimethylpiperidin-4-amin | 0.61 |
| | N-(2-{2-[4-(Dimethylamino)piperidin-1-yl]-2-oxoethyl}-2H-indazol-5-yl)-6-(1-methyl-1H-pyrazol-4-yl)pyridin-2-carboxamid | | |

**Tabelle 10: Beispiele 206 - 208**

| Die Beispielverbindungen wurden aus den in der Tabelle angegebenen Intermediaten hergestellt. | | | |
|---|---|---|---|
| Beispiel | Name und Struktur | Edukte und Anmerkungen | LC-MS Retentionszei t [min] |
| 206 | 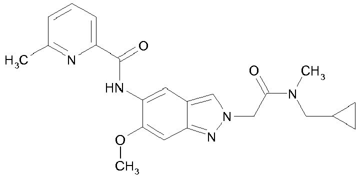 | Die Beispielverbindung wurde hergestellt aus 2-(5-Amino-6-methoxy-2H-indazol-2-yl)-N-(cyclopropylmethyl)-N-methylacetamid und 6-Methylpyridin-2-carbonsäure. | 1.07 |
| | N-(2-{2-[(Cyclopropylmethyl)(methyl)amino] -2-oxoethyl}-6-methoxy-2H-indazol-5-yl)-6-methylpyridin-2-carboxamid | ¹H-NMR (300MHz, DMSO-d6): δ = 0.17 - 0.57 (m, 4H), 0.91 - 1.11 (m, 1H), 2.61 (s), 2.91 (s), 3.12 (s), 3.19 (d), 3. (s, 3H), 5.33 - 5.40 (m, 2H), 7.09 (s, 1H), 7.55 (dd, 1H), 7.93 - 8.02 (m, 2H), 8.18 - 8.24 (m, 1H), 8.71 (s, 1H), 10.71 (s, 1H). | |
| 207 | 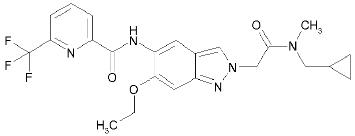 | Die Beispielverbindung wurde hergestellt ausgehend von 105 mg (0.26 mmol) [6-Ethoxy-5-({[6-(trifluormethyl)pyridin-2-yl]carbonyl} amino)-2H-indazol-2-yl]essigsäure und 33 mg (1.5 eq.) 1-Cyclopropyl-N-methylmethanamin. Man erhielt 87 mg der Beispielverbindung. | 1.24 |
| | N-(2-{2-[(Cyclopropylmethyl)(methyl)amino] -2-oxoethyl}-6-ethoxy-2H-indazol-5-yl)-6-(trifluormethyl)pyridin-2-carboxamid | | |
| | | ¹H-NMR (300MHz, DMSO-d6): δ = 0.17 - 0.57 (m, 4H), 0.88 - 1.12 (m, 1H), 1.49 (t, 3H), 2.91 (s, 1H), 3.09 - 3.24 (m, 3H), 3.34 (br. s., 1H), 4.20 (q, 2H), 5.32 - 5.40 (m, 2H), 7.08 (s, 1H), 8.17 - 8.26 (m, 2H), 8.36 - 8.48 (m, 2H), 8.71 (s, 1H), 10.7 (s, 1H). | |
| 208 | 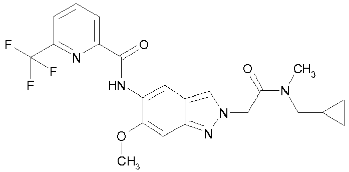 | Die Beispielverbindung wurde hergestellt aus 2-(5-Amino-6-methoxy-2H-indazol-2-yl)-N-(cyclopropylmethyl)- N-methylacetamid und 6-(Trifluormethyl)pyridin-2-carbonsäure. 1H-NMR (300MHz, DMSO-d6): δ = | 1.16 |
| | N-(2-{2-[(Cyclopropylmethyl)(methyl)amino] | | |
| | -2-oxoethyl}-6-methoxy-2H-indazol-5-yl)-6-(trifluormethyl)pyridin-2-carboxamid | 0.16 - 0.59 (m, 4H), 0.88 - 1.14 (m, 1H), 2.91 (s, 1H), 3.10 - 3.23 (m, 3H), 3.98 (s, 3H), 5.33 - 5.42 (m, 2H), 7.11 (s, 1H), 8.17 - 8.28 (m, 2H), 8.35 - 8.49 (m, 2H), 8.70 (s, 1H), 10.50 (s, 1H). | |

**Tabelle 11: Beispiele 209 - 210**

| Die Beispielverbindungen (Bsp.) wurden aus 2-(5-Amino-6-methoxy-2H-indazol-2-yl)-1-[4-(2-hydroxypropan-2-yl)piperidin-1-yl]ethanon (Intermediat 6-5) hergestellt. | | | |
|---|---|---|---|
| Bsp. | Name und Struktur | Edukte und Anmerkungen | LC-MS Retent ionszei t [min] |
| 209 | 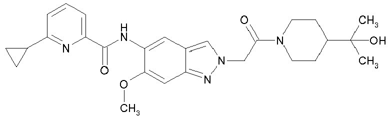 | Hergestellt aus 100 mg 2-(5-Amino-6-methoxy-2H-indazol-2-yl)-1-[4-(2-hydroxypropan-2-yl)piperidin-1-yl]ethanon und 6-Cyclopropylpyridin-2-carbonsäure. | 1.08 |
| | 6-Cyclopropyl-N-(2- {2-[4-(2-hydroxypropan-2-yl)piperidin-1-yl]-2-oxoethyl}-6-methoxy-2H-indazol-5-yl)pyridin-2-carboxamid | 1H-NMR (400MHz, DMSO-d6): δ = 0.99 - 1.14 (m, 11H), 1.14 - 1.28 (m, 1H), 1.38 - 1.49 (m, 1H), 1.74 (t, 2H), 2.21 - 2.30 (m, 1H), 2.98 (t, 1H), 3.97 - 4.08 (m, 4H), 4.15 (s, 1H), 4.41 (d, 1H), 5.26 - 5.43 (m, 2H), 7.08 (s, 1H), 7.58 - 7.64 (m, 1H), 7.87 - 7.96 (m, 2H), 8.17 - 8.23 (m, 1H), 8.65 (s, 1H), 10.80 (s, 1H). | |
| 210 | 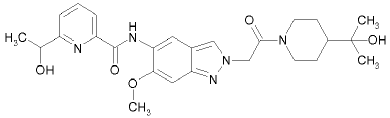 | Hergestellt aus 150 mg 2-(5-Amino-6-methoxy-2H-indazol-2-yl)-1-[4-(2-hydroxypropan-2-yl)piperidin-1-yl]ethanon und 133 mg Kalium-6-(1-hydroxyethyl)pyridin-2-carboxylat (Intermediat 19-1) | 0.82 |
| | 6-(1-Hydroxyethyl)-N-(2-{2-[4-(2-hydroxypropan-2-yl)piperidin-1-yl]-2-oxoethyl}-6-methoxy-2H-indazol-5-yl)pyridin-2-carboxamid | | |
| | | 1H-NMR (400MHz, DMSO-d6): δ = 0.99 - 1.13 (m, 7H), 1.15 - 1.29 (m, 1H), 1.34 - 1.48 (m, 1H), 1.51 (d, 3H), 1.74 (t, 2H), 2.99 (t, 1H), 3.95 - 4.07 (m, 4H), 4.16 (s, 1H), 4.41 (d, 1H), 4.81 - 4.90 (m, 1H), 5.28 - 5.43 (m, 2H), 5.58 (d, 1H), 7.08 (s, 1H), 7.79 (dd, 1H), 8.01 - 8.10 (m, 2H), 8.20 (s, 1H), 8.67 (s, 1H), 10.78 (s, 1H). | |

**Tabelle 12: Beispiele 211 - 213**

| Die Beispielverbindungen wurden aus 2-(5-Amino-2H-indazol-2-yl)-1-(4-benzoylpiperazin-1-yl)ethanon (Intermediat 6-11) hergestellt. | | | |
|---|---|---|---|
| Bsp. | Name und Struktur | Herstellung und Anmerkungen | LC-MS Retent ionszei t [min] |
| 211 | 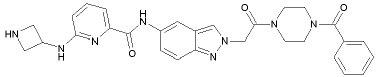 | 96 mg 2-(5-Amino-2H-indazol-2-yl)-1 -(4-benzoylpiperazin-1 -yl)ethanon und 202 mg 6-{[1-(tert-Butoxycarbonyl)azetidin-3-yl]amino}pyridin-2-carbonsäure (Intermediat 19-12) wurden mit EDC, HOBt und Triethylamin umgesetzt. Nach wässriger Aufarbeitung erhielt man 252 mg tert-Butyl-3-{[6-({2-[2-(4-benzoylpiperazin-1 -yl)-2-oxoethyl] - 2H - indazol-5-yl} carbamoyl)pyridin-2-yl] amino} azetidin-1 -carboxylat als Rohprodukt, welches mit Trifluoressigsäure in Dichlormethan umgesetzt wurde. Nach Reinigung durch HPLC nach der Methode P2 wurden 19 mg der Titelverbindung erhalten. | 0.60 |
| | 6-(Azetidin-3-ylamino)-N- {2-[2-(4-benzoylpiperazin-1-yl)-2-oxoethyl]-2H-indazol-5-yl}pyridin-2-carboxamid | | |
| 212 | 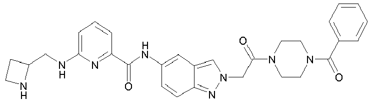 | 100 mg 2-(5-Amino-2H-indazol-2-yl)-1 -(4-benzoylpiperazin-1 -yl)ethanon und 265 mg Kalium-6-({[1-(tert-butoxycarbonyl)azetidin-2-yl]methyl} amino)pyridin-2-carboxylat (Intermediat 19-13) wurden mit EDC, HOBt und Triethylamin umgesetzt. Nach wässriger Aufarbeitung und HPLC erhielt man 93 mg tert-Butyl-2-({[6-({2- [2-(4-benzoylpiperazin-1 -yl)-2-oxoethyl]-2H - indazol-5-yl}carbamoyl)pyridin-2-yl] amino}methyl)azetidin-1-carboxylat, welches mit Trifluoressigsäure in Dichlormethan umgesetzt wurde. Nach HPLC Aufreinigung erhielt man 50 mg der Titelverbindung. ¹H-NMR (400MHz, DMSO-d6, ausgewählte Signale): δ = 2.20 - 2.42 (m, 2H), 4.37 - 4.49 (m, 1H), 5.48 (br. s., 2H), 6.77 (d, 1H), 7.24 (t, 1H), 7.32 (d, 1H), 7.39 - 7.53 (m, 6H), 7.53 - 7.65 (m, 2H), 8.28 (d, 2H), 10.17 (br. s., 1H). | 0.61 |
| | 6- [(Azetidin-2-ylmethyl)amino] -N- {2-[2-(4-benzoylpiperazin-1 -yl)-2-oxoethyl]-2H-indazol-5-yl}pyridin-2-carboxamid | | |
| 213 | 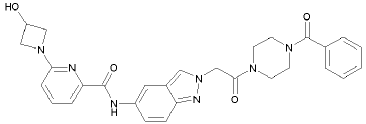 | 85 mg N-{2-[2-(4-Benzoylpiperazin-1-yl)-2-oxoethyl]-2H-indazol-5-yl}-6-chlorpyridin-2-carboxamid (Beispiel 91) wurden mit 3 Äquiv. Azetidin-3-olhydrochlorid (1:1) und 115 µl N-Ethyl-N-isopropylpropan-2-amin in 2 ml NMP bei 100°C umgesetzt. Nach Reinigung durch HPLC wurden 2 mg der Titelverbindung erhalten. | 0.81 |
| | N-{2-[2-(4-Benzoylpiperazin-1-yl)-2-oxoethyl]-2H-indazol-5-yl}-6-(3-hydroxyazetidin-1-yl)pyridin-2-carboxamid | | |
| | | ¹H-NMR (400MHz, DMSO-d6, ausgewählte Signale): δ = 3.82 (dd, 2H), 4.30 (t, 2H), 4.58 - 4.66 (m, 1H), 5.49 (br. s., 2H), 5.70 (d, 1H), 6.63 (d, 1H), 7.38 (d, 1H), 7.42 - 7.52 (m), 7.56 - 7.61 (m, 1H), 7.71 (t, 1H), 8.27 (s, 1H), 8.31 (s, 1H), 10.11 (s, 1H). | |

**Tabelle 13: Beispiele 214 - 216**

| Bsp. | Name und Struktur | Herstellung und Anmerkungen | LC-MS Retent ionszei t [min] |
|---|---|---|---|
| 214 | 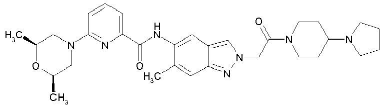 | 75 mg 6-[(2R,6S)-2,6-Dimethylmorpholin-4-yl]pyridin-2-carbonsäure (Intermediat 19-14) wurden mit 118 mg 2-(5-Amino-6-methyl-2H-indazol-2-yl)-1-[4-(pyrrolidin-1-yl)piperidin-1-yl]ethanon (Intermediat 6-2) umgesetzt. | 0.78 |
| | 6-[(2R,6S)-2,6-Dimethylmorpholin-4-yl]-N-(6-methyl-2-{2-oxo-2-[4-(pyrrolidin-1-yl)piperidin-1-yl]ethyl}-2H-indazol-5-yl)pyridin-2-carboxamid | | |
| | | ¹H-NMR (400MHz, DMSO-d6): δ = 1.18 (d, 6H), 1.21 - 1.48 (m, 2H), 1.67 (br. s., 4H), 1.84 (t, 2H), 2.20 - 2.28 (m, 1H), 2.28 - 2.39 (m, 1H), 2.84 (t, 1H), 3.17 (t), 3.61 - 3.71 (m, 2H), 3.88 (d, 1H), 4.10 (d, 1H), 4.29 (d, 2H), 5.34 - 5.46 (m, 2H), 7.14 (d, 1H), 7.41 - 7.50 (m, 2H), 7.77 (dd, 1H), 8.22 (s, 1H), 8.36 (s, 1H), 10.18 (s, 1H). | |
| 215 | 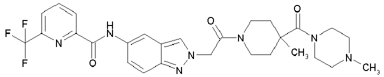 | 400 mg [5-({[6-(Trifluormethy l)pyridin- 2-yl]carbonyl}amino)-2H-indazol-2-yl]essigsäure wurden mit 296 mg Ethyl-4-methylpiperidin-4-carboxylathydrochlorid (1:1) in Gegenwart von EDC, HOBt und Triethylamin umgesetzt. Es wurden 544 mg Ethyl-4-methyl-1-{[5-({[6-(trifluormethy l)pyridin- 2-yl]carbonyl}amino)-2H-indazol-2-yl]acetyl} piperidin-4-carboxylat als Rohprodukt erhalten. Es wurde mit Ethanol und THF und 348 mg Lithiumhydroxid Monohydrat in Wasser versetzt, über Nacht gerührt | 0.71 |
| | N-[2-(2-{4-Methyl-4-[(4-methylpiperazin-1-yl)carbonyl]piperidin-1-yl}-2-oxoethyl)-2H-indazol-5-yl]-6-(trifluormethy l)pyridin- 2-carboxamid | | |
| | | und mit Zitronensäurelösung angesäuert. Nach Extraktion mit Ethylacetat und HPLC-Aufreinigung wurden 89 mg 4-Methyl-1-{[5-({[6-(trifluormethy l)pyridin- 2-yl]carbonyl}amino)-2H-indazol-2-yl] acetyl} piperidin-4-carbonsäure erhalten. 49 mg davon wurden mit 15 mg 1-Methylpiperazin in Gegenwart von EDC, HOBt und Triethylamin in THF umgesetzt. Nach HPLC-Aufreinigung wurden 29 mg N-[2-(2-{4-Methyl-4-[(4-methylpiperazin-1-yl)carbonyl]piperidin-1-yl} -2-oxoethyl)-2H-indazol-5-yl]-6-(trifluormethy l)pyridin- 2-carboxamid erhalten. | |
| | | ¹H-NMR (300MHz, DMSO-d6, ausgewählte Signale): δ = 1.25 (s, 3H), 1.36 - 1.57 (m, 2H), 1.98 - 2.22 (m, 5H), 2.27 (br. s., 4H), 3.13 (t), 3.54 (s), 3.60 - 3.80 (m, 2H), 5.35 - 5.50 (m, 2H), 7.51 - 7.63 (m, 2H), 8.17 (dd, 1H), 8.26 - 8.42 (m, 4H), 10.37 (s, 1H). | |
| 216 | 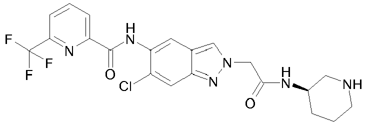 | 100 mg ([6-Chlor-5-({[6-(trifluormethy l)pyridin- 2-yl]carbonyl} amino)-2H-indazol-2-yl]essigsäure (Intermediat 9-11) wurden mit 65 mg tert-Butyl-(3R)-3-aminopiperidin-1-carboxylat in Gegenwart von EDC, HOBt und Triethylamin in THF umgesetzt. Nach Zugabe von Wasser und Extraktion mit Ethylacetat wurden nach dem Einengen 148 mg tert-Butyl-(3R)-3-({[6-chlor-5-({[6-(trifluormethyl)pyridin-2-yl]carbonyl}amino)-2H-indazol-2-yl] acetyl} amino)piperidin-1 - carboxylat als Rohprodukt erhalten. Nach Zugabe von Dichlormethan und Trifluoressigsäure wurde über Nacht gerührt, eingeengt und durch HPLC gereinigt. Es wurden 105 mg N-(6-Chlor-2-{2-oxo-2-[(3R)-piperidin-3-ylamino]ethyl}-2H-indazol-5-yl)-6-(trifluormethy l)pyridin- 2-carboxamid erhalten. | 0.79 |
| | N-(6-Chlor-2-{2-oxo-2-[(3R)-piperidin-3-ylamino]ethyl}-2H-indazol-5-yl)-6-(trifluormethy l)pyridin- 2-carboxamid | | |
| | | ¹H-NMR (300MHz, DMSO-d6, ausgewählte Signale): δ = 1.39 - 1.64 (m, 2H), 1.74 - 1.90 (m, 2H), 2.56 - 2.67 (m, 1H), 2.68 - 2.80 (m, 1H), 2.98 - 3.21 (m, überlagert), 3.10 - 3.21 (m, 2H), 5.07 - 5.22 (m, 2H), 7.92 (s, 1H), 8.18 - 8.27 (m, 1H), 8.36 - 8.53 (m, 4H), 8.64 (s, 1H), 10.53 (s, 1H). | |

**Tabelle 14: Beispiele 217 - 222**

| Die Beispielverbindungen wurden aus [6-Isopropoxy-5-({[6-(trifluoromethyl)pyridin-2-yl]carbonyl}amino)-2H-indazol-2-yl]essigsäure (Intermediat 9-16) oder (6-Isopropoxy-5-{[(6-methylpyridin-2-yl)carbonyl]amino}-2H-indazol-2-yl)essigsäure (Intermediat 9-17) und dem in der Tabelle angegebenen Edukt nach der allgemeinen Vorschrift 2a hergestellt. | | | | |
|---|---|---|---|---|
| Bsp. | Struktur/Name | Hergestellt aus | Ausbeute [%] | ¹H-NMR / LC-MS |
| 217 | 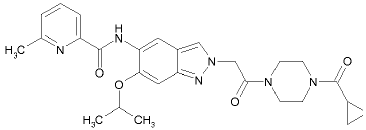 | Cyclopropy l(piperazin-1-yl)methano n | 91 | (300 MHz, DMSO-d6): δ = 0.67 - 0.82 (m, 4H), 1.45 (d, 6H), 1.92 - 2.09 (m, 1H), 2.62 (s, 3H), 3.38 - 3.86 (m, 8H), 4.76 - 4.90 (m, 1H), 5.42 (s, 2H), 7.13 (s, 1H), 7.53 -7.60 (m, 1H), 7.93 - 8.02 (m, 2H), 8.21 (s, 1H), 8.72 (s, 1H), 10.99 H). UPLC-MS (Methode A1): Rt = 1.14 min MS (ESIpos): m/z = 505 (M+H)+ |
| | N-(2-{2-[4-(Cyclopropylcarbonyl)piperazin-1-yl]-2-oxoethyl}-6-isopropoxy-2H-indazol-5-yl)-6-methylpyridin-2-carboxamid | | | |
| 218 | 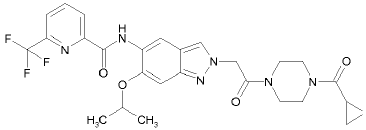 | Cyclopropy l(piperazin-1-yl)methano n | 75 | (300 MHz, DMSO-d6): δ = 0.67 - 0.82 (m, 4H), 1.41 (d, 6H), 1.92 - 2.08 (m, 1H), 3.38 - 3.88 (m, 8H), 4.79 - 4.93 (m, 1H), 5.43 (s, 2H), 7.16 (s, 1H), 8.18 - 8.27 (m, 2H), 8.36 - 8.51 (m, 2H), 8.75 (s, 1H), 10.75 (s, 1H). |
| | N-(2-{2-[4-(Cyclopropylcarbonyl)piperazin-1-yl]-2-oxoethyl}-6-isopropoxy-2H-indazol-5-yl)-6-(trifluormethyl)pyridin-2-carboxamid | | | |
| | | | | UPLC-MS (Methode A1): Rt = 1.20 min |
| | | | | MS (ESIpos): m/z = 559 (M+H)+ |
| 219 | 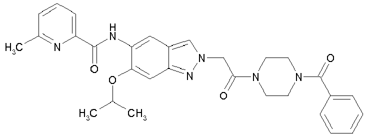 | Phenyl(pip erazin-1-yl)methano n | 82 | (300 MHz, DMSO-d6): δ = 1.45 (d, 6H), 2.62 (s, 3H), 3.37 - 3.86 (m, 8H), 4.76 - 4.92 (m, 1H), 5.41 (s, 2H), 7.13 (s, 1H), 7.39 - 7.51 (m, 5H), 7.53 - 7.61 (m, 1H), 7.92 - 8.04 (m, 2H), 8.20 (s, 1H), 8.72 (s, 1H), 10.98 (s, 1H). |
| | N-{2-[2-(4-Benzoylpiperazin-1-yl)-2-oxoethyl]-6-isopropoxy-2H-indazol-5-yl}-6-methylpyridin-2-carboxamid | | | |
| | | | | UPLC-MS (Methode A1): Rt = 1.21 min |
| | | | | MS (ESIpos): m/z = 541 (M+H)+ |
| 220 | 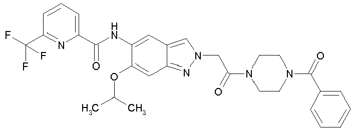 | Phenyl(pip erazin-1-yl)methano n | 98 | (300 MHz, DMSO-d6): δ = 1.41 (d, 6H), 3.38 - 3.93 (m, 8H), 4.79 - 4.93 (m, 1H), 5.42 (s, 2H), 7.15 (s, 1H), 7.40 - 7.53 (m, 5H), 8.17 - 8.26 (m, 2H), 8.35 - 8.51 (m, 2H), 8.75 (s, 1H), 10.74 (s, 1H). |
| | N-{2-[2-(4-Benzoylpiperazin-1-yl)-2-oxoethyl]-6-isopropoxy-2H-indazol-5-yl}-6-(trifluormethyl)pyridin-2-carboxamid | | | |
| | | | | UPLC-MS (Methode A1): Rt = 1.26 min |
| | | | | MS (ESIpos): m/z = 595 (M+H)+ |
| 221 | 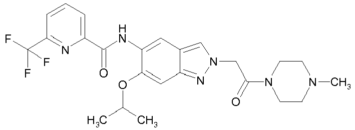 | 1-Methylpipe razin | 36 | (300 MHz, DMSO-d6): δ = 1.41 (d, 6H), 2.12 - 2.70 (m, 4H), , 3.37 - 3.78 (m, 4H), 4.80 - 4.91 (m, 1H), 5.40 (s, 2H), 7.15 (s, 1H), 8.18 - 8.26 (m, 2H), 8.36 - 8.49 (m, 2H), 8.74 (s, 1H), 10.75 (s, 1H). |
| | N-{6-Isopropoxy-2-[2-(4-methylpiperazin-1-yl)-2-oxoethyl]-2H-indazol-5-yl}-6-(trifluormethy l)pyridin- 2-carboxamid | | | |
| | | | | UPLC-MS (Methode A1): Rt = 1.01 min |
| | | | | MS (ESIpos): m/z = 505 (M+H)+ |
| 222 | 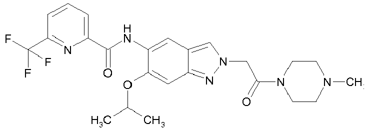 | 1-Methylpipe razin | 63 | (300 MHz, DMSO-d6): δ = 1.45 (d, 6H), 2.18 - 2.70 (m, 4H), 2.62 (s, 3H), 3.34 - 3.87 (m, 4H), 4.77 - 4.89 (m, 1H), 5.39 (s, 2H), 7.12 (s, 1H), 7.53 - 7.58 (m, 1H), 7.93 - 8.02 (m, 2H), 8.20 (s, 1H), 8.72 (s, 1H), 10.98 (s, 1H). |
| | N-{6-Isopropoxy-2-[2-(4-methylpiperazin-1-yl)-2-oxoethyl]-2H-indazol-5-yl}-6-methylpyridin-2-carboxamid | | | |
| | | | | UPLC-MS (Methode A1): Rt = 0.95 min |
| | | | | MS (ESIpos): m/z = 451 (M+H)+ |

**Tabelle 15: Beispiele 223 - 226**

| Die Beispielverbindungen wurden aus N-{2-[2-Oxo-2-(piperazin-1-yl)ethyl]-2H-indazol-5-yl}-6-(trifluormethyl)pyridin-2-carboxamid (Intermediat 22-1) und dem in der Tabelle angegebenen Edukt analog zu den vorigen Beispielen durch eine Amidsynthese hergestellt. | | | |
|---|---|---|---|
| Beispiel | Name und Struktur | Edukt und Anmerkungen | LC-MS Retent ionszei t [min] |
| 223 | 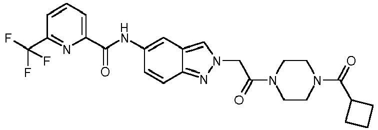 | Cyclobutantancarbonsäure | 1.0 |
| | N-(2-{2-[4-(Cyclobutylcarbonyl)piperazin-1-yl] -2-oxoethyl}-2H-indazol-5-yl)-6-(trifluormethy l)pyridin- 2-carboxamid | | |
| 224 | 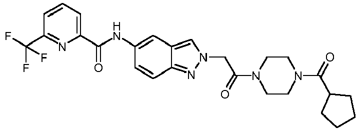 | Cyclopentancarbonsäure | 1.06 |
| | N-(2-{2-[4-(Cyclopentylcarbonyl)piperazin-1-yl]-2-oxoethyl}-2H-indazol-5-yl)-6-(trifluormethy l)pyridin- 2-carboxamid | | |
| 225 | 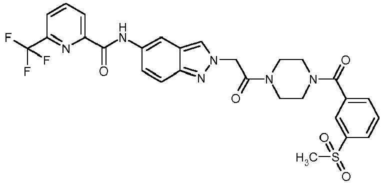 | 3 -(Methylsulfonyl)benzoesäure | 0.95 |
| | N-[2-(2- {4-[3-(Methylsulfonyl)benzoyl]piperazin-1-yl}-2-oxoethyl)-2H-indazol-5-yl]-6-(trifluormethy l)pyridin- 2-carboxamid | | |
| 226 | 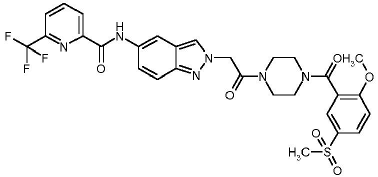 | 2-Methoxy-5-(methylsulfonyl) benzoesäure | 0.7 |
| | N-[2-(2-{4-[2-Methoxy-5-(methylsulfonyl)benzoyl]piperazin-1-yl}-2-oxoethyl)-2H-indazol-5-yl]-6-(trifluormethy l)pyridin- 2-carboxamid | | |

**Tabelle 16: Beispiele 227 - 244**

| Die Beispielverbindungen wurden aus den in der Tabelle angegebenen Intermediaten und Edukten hergestellt. | | | | |
|---|---|---|---|---|
| Bsp | Name und Struktur | Inter medi at | Edukt, Herstellung und 1H NMR | LC-MS Retent ionszei t[min] |
| 227 | 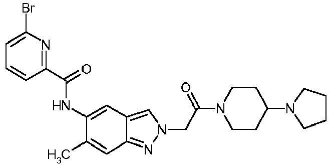 | 6-2 | 6-Brompyridin-2-carbonsäure ¹H NMR (400 MHz, DMSO-d6, ausgewählte Signale): δ = 1.21 - 1.36 (m, 1 H), 1.38 - 1.52 (m, 1 H), 1.69 (br. s., 4 H), 1.78 - 1.95 (m, 2 H), 2.21 - 2.36 (m, 1 H), 2.39 (s, 3 H), 2.80 - 2.91 (m, 1 H), 3.18 (t, 1 H), 3.82 - 3.96 (m, 1 H), 4.04 - 4.18 (m, 1 H), 5.45 (d, 1 H), 5.40 (d, 1 H), 7.48 (s, 1 H), 7.91 - 7.97 (m, 1 H), 8.02 (t, 1 H), 8.09 (s, 1 H), 8.17 (dd, 1 H), 8.23 - 8.27 (m, 1 H), 10.05 (s, 1 H). | 0.7 |
| | | | | |
| | 6-Brom-N-(6-methyl-2-{2-oxo-2-[4-(pyrrolidin-1-yl)piperidin-1-yl]ethyl}-2H-indazol-5-yl)pyridin-2-carboxamid | | | |
| 228 | 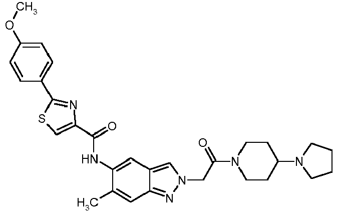 | 6-2 | 2-(4-Methoxyphenyl)-1,3-thiazol-4-carbonsäure ¹H NMR (400 MHz, DMSO-d6, ausgewählte Signale): δ = 1.21 - 1.36 (m, 1 H), 1.37 - 1.52 (m, 1 H), 1.68 (br. s., 4 H), 1.85 (t, 2 H), 2.18 - 2.28 (m, 1 H), 2.42 (s, 3 H), 2.80 - 2.92 (m, 1 H), 3.18 (t, 1 H), 3.85 (s, 3 H), 3.88 - 3.94 (m, 1 H), 4.05 - 4.15 (m, 1 H), 5.40 (d, 1 H), 5.45 (d, 1 H), 7.06 - 7.15 (m, 2 H), 7.49 (s, 1 H), 8.02 - 8.09 (m, 3 H), 8.25 (s, 1 H), 8.36 (s, 1 H), 9.86 (s, 1 H). | 0.82 |
| | | | | |
| | 2-(4-Methoxyphenyl)-N-(6-methyl-2-{2-oxo-2-[4-(pyrrolidin-1-yl)piperidin-1-yl]ethyl}-2H-indazol-5-yl)-1,3-thiazol-4-carboxamid | | | |
| 229 | 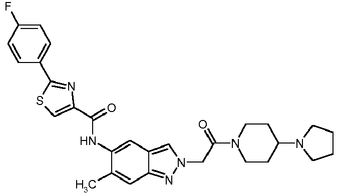 | 6-2 | 2-(4-Fluorphenyl)-1,3-thiazol-4-carbonsäure | 0.83 |
| | | | ¹H NMR (400 MHz, CHLOROFORM-d): δ = 1.26 (s, 1 H), 1.36 (t, 1 H), 1.44 - 1.59 (m, 1 H), 1.82 (br. s., 2 H), 1.94 (d, 2 H), 2.33 (br. s., 1 H), 2.56 (s, 3 H), 2.63 (br. s., 4 H), 2.81 - 2.92 (m, 1 H), 3.01 (d, 1 H), 3.16 (t, 1 H), 3.98 (d, 1 H), 4.44 (d, 1 H), 5.23 - 5.29 (m, 2 H), 7.16 - 7.24 (m, 2 H), 7.53 - 7.58 (m, 1 H), 7.96 - 8.04 (m, 3 H), 8.20 (s, 1 H), 8.55 (s, 1 H), 9.44 (s, 1 H). | |
| | 2-(4-Fluorphenyl)-N-(6-methyl-2-{2-oxo-2-[4-(pyrrolidin-1-yl)piperidin-1-yl]ethyl}-2H-indazol-5-yl)-1,3-thiazol-4-carboxamid | | | |
| 230 | 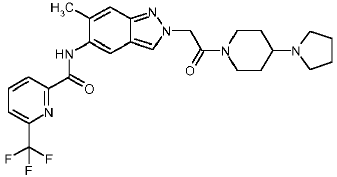 | 6-2 | 6-(Trifluormethyl)pyridin-2-carbonsäure | 0.75 |
| | | | ¹H NMR (300 MHz, DMSO-d6): δ = 1.15 - 1.36 (m, 1 H), 1.42 - 1.45 (m, 1 H), 1.68 (br. s., 4 H), 1.86 (t, 2 H), 2.19 - 2.32 (m, 1 H), 2.41 (s, 3 H), 2.85 (t, 1 H), 3.10 - 3.24 (m, 1 H), 3.89 (d, 1 H), 4.11 (d, 1 H), 5.43 (s, 2 H), 7.49 (s, 1 H), 8.16 - 8.24 (m, 2 H), 8.26 (s, 1 H), 8.33 - 8.49 (m, 2 H), 10.15 (s, 1 H). | |
| | N-(6-Methyl-2-{2-oxo-2-[4-(pyrrolidin-1-yl)piperidin-1-yl]ethyl}-2H-indazol-5-yl)-6-(trifluormethy l)pyridin- 2-carboxamid | | | |
| 231 | 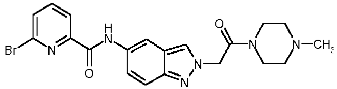 | 6-12 | 6-Brompyridin-2-carbonsäure | 0.61 |
| | | | ¹H NMR (300 MHz, DMSO-d6): δ = 2.21 (s, 3 H), 2.24 - 2.34 (m, 2 H), 2.34 - 2.42 (m, 2 H), 3.43 - 3.52 (m, 2 H), 3.54 (d, 2 H), 5.45 (s, 2 H), 7.52 - 7.63 (m, 2 H), 7.84 - 7.96 (m, 1 H), 8.01 (t, 1 H), 8.15 (dd, 1 H), 8.26 - 8.36 (m, 2 H), 10.38 (s, 1 H). | |
| | 6-Brom-N-{2-[2-(4-methylpiperazin-1-yl)-2-oxoethyl]-2H-indazol-5-yl}pyridin-2-carboxamid | | | |
| 232 | 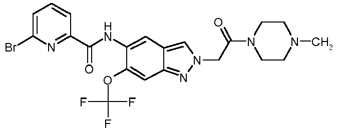 | 6-8 | 6-Brompyridin-2-carbonsäure | 0.82 |
| | | | ¹H NMR (300 MHz, DMSO-d6): δ = 2.21 (s, 3 H), 2.25 - 2.33 (m, 2 H), 2.39 (br. s., 2 H), 3.47 (br. s., 2 H), 3.54 (d, 2 H), 5.52 (s, 2 H), 7.74 (s, 1 H), 7.94 - 8.01 (m, 1 H), 8.01 - 8.10 (m, 1 H), 8.19 (d, 1 H), 8.45 (s, 1 H), 8.55 (s, 1 H), 10.28 (s, 1 H) | |
| | 6-Brom-N-{2-[2-(4-methylpiperazin-1-yl)-2-oxoethyl]-6-(trifluormethoxy)-2H-indazol-5-yl}pyridin-2-carboxamid | | | |
| 233 | 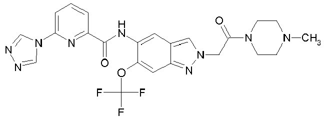 | 6-8 | 6-(4H-1,2,4- Triazol-4-yl)pyridin-2-carbonsäure | 0.56 |
| | N- {2-[2-(4-Methylpiperazin-1-yl)-2-oxoethyl]-6-(trifluormethoxy)-2H-indazol-5-yl}-6-(4H-1,2,4-triazol-4-yl)pyridin-2-carboxamid | | ¹H NMR (400 MHz, DMSO-d6): δ = 2.22 (s, 3 H), 2.27 - 2.35 (m, 2 H), 2.39 (br. s., 2 H), 3.43 - 3.52 (m, 2 H), 3.52 - 3.60 (m, 2 H), 5.53 (s, 2 H), 7.72 (s, 1 H), 8.18 (dd, 2 H), 8.28 (s, 1 H), 8.34 (d, 1 H), 8.47 (s, 1 H), 9.62 (s, 2 H), 10.56 (s, 1 H). | |
| 234 | 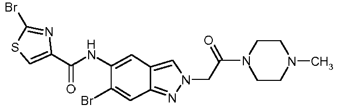 | 6-9 | 2-Brom-1,3-thiazol-4-carbonsäure | 0.72 |
| | | | ¹H NMR (300 MHz, DMSO-d6): δ = 2.21 (s, 3 H), 2.25 - 2.34 (m, 2 H), 2.38 (br. s., 2 H), 3.47 (br. s., 2 H), 3.54 (br. s., 2 H), 5.49 (s, 2 H), 8.03 (s, 1 H), 8.25 (s, 1 H), 8.35 - 8.42 (m, 1 H), 8.49 (s, 1 H), 9.96 (s, 1 H). | |
| | 2-Brom-N- {6-brom-2-[2-(4-methylpiperazin-1-yl)-2-oxoethyl]-2H-indazol-5-yl}-1,3-thiazol-4-carboxamid | | | |
| 235 | 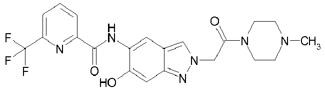 | - | Analog zu Intermediat 8-7 wurden 25 mg (0.05mmol) N-{6-(Benzyloxy)-2-[2-(4-methylpiperazin-1-yl)-2-oxoethyl]-2H-indazol-5-yl}-6-(trifluoromethyl)pyridin-2-carboxamid (Beispiel 41) in 7 ml Ethanol gelöst, mit 4.8 mg Palladium auf Kohle versetzt und bei Normaldruck Wasserstoff für 6 h hydriert. Nach Aufarbeitung erhielt man 5 mg (34 % d. Th.) Produkt. | 0.67 |
| | N- {6-Hydroxy-2-[2-(4-methylpiperazin-1-yl)-2-oxoethyl]-2H-indazol-5-yl}-6-(trifluormethyl)pyridin-2-carboxamid | | | |
| | | | ¹H NMR (400 MHz, DMSO-d6): δ = 2.20 (s, 3 H), 2.28 (br. s., 2 H), 2.35 (br. s., 2 H), 3.46 (br. s., 2 H), 3.53 (br. s., 2 H), 5.31 (s, 2 H), 6.88 (s, 1 H), 8.13 (s, 1 H), 8.20 (d, 1 H), 8.40 (t, 1 H), 8.47 (d, 1 H), 8.66 (s, 1 H), 10.64 (br. s., 1 H). | |
| 236 | 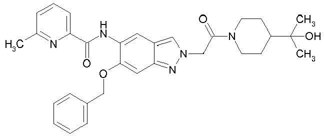 | 9-18 | 2-(Piperidin-4-yl)propan-2-ol | 1.19 |
| | | | ¹H NMR (300 MHz, DMSO-d6): δ = 0.96 - 1.17 (m, 1 H), 1.05 (s, 6 H), 1.18 - 1.34 (m, 2 H), 1.36 - 1.54 (m, 1 H), 1.76 (t, 2 H), 2.43 (s, 3 H), 3.00 (t, 1 H), 4.04 (d, 1 H), 4.19 (s, 1 H), 4.42 (d, 1 H), 5.30 (s, 2H), 5.38 (s, 1 H), 5.37 (s, 1 H), 7.27 (s, 1 H), 7.37 - 7.54 (m, 4 H), 7.66 (d, 2 H), 7.87 - 8.02 (m, 2 H). 8.23 (s, 1 H) 8.78 (s, 1 H) 10.87 (s, 1 H). | |
| | N-[6-(Benzyloxy)-2-{2-[4-(2-hydroxypropan-2-yl)piperidin-1-yl]-2-oxoethyl}-2H-indazol-5-yl]-6-methylpyridin-2-carboxamid | | | |
| 237 | 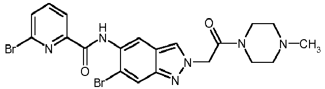 | 6-9 | 6-Brompyridin-2-carbonsäure | 0.77 |
| | 6-Brom-N-{6-brom-2-[2-(4-methylpiperazin-1-yl)-2-oxoethyl]-2H-indazol-5-yl}pyridin-2-carboxamid | | ¹H NMR (300 MHz, DMSO-d6): δ = 2.20 (s, 3 H), 2.23 - 2.33 (m, 2 H), 2.37 (br. s., 2 H), 3.46 (d, 2 H), 3.53 (br. s., 2 H), 5.49 (s, 2 H), 7.94 - 7.99 (m, 1 H), 8.01 - 8.08 (m, 2 H), 8.16 - 8.23 (m, 1 H), 8.39 (s, 1 H), 8.51 (s, 1 H), 10.38 (s, 1 H). | |
| 238 | 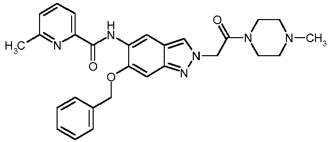 | 9-18 | 1-Methylpiperazin | 0.87 |
| | N-{6-(Benzyloxy)-2-[2-(4-methylpiperazin-1-yl)-2-oxoethyl]-2H-indazol-5-yl}-6-methylpyridin-2-carboxamid | | ¹H NMR (300 MHz, DMSO-d6): δ = 2.21 (s, 3 H), 2.23 - 2.33 (m, 2 H), 2.37 (br. s., 2 H), 2.42 (s, 3 H), 3.47 (br. s., 2 H), 3.55 (br. s., 2 H), 5.30 (s, 2 H), 5.39 (s, 2 H), 7.27 (s, 1 H), 7.36 - 7.56 (m, 4 H), 7.62 - 7.69 (m, 2 H), 7.90 - 8.02 (m, 2 H), 8.23 (s, 1 H), 8.78 (s, 1 H), 10.87 (s, 1 H). | |
| 239 | 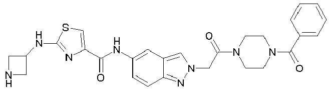 | 6-11 | Nach der allgemeinen Vorschrift 1c wurden 35 mg (0.09 mmol) des Intermediates 6-11 mit 32 mg (0.1 mmol) 2-{[1-(tert-butoxycarbonyl)azetidin-3-yl]amino}-1,3-thiazol-4-carbonsäure umgesetzt. Es wurden 10 mg (0.01 mmol) tert-Butyl-3-{[4-({2-[2-(4-benzoylpiperazin-1-yl)-2-oxoethyl]-2H-indazol-5-yl}carbamoyl)-1,3-thiazol-2-yl]amino}azetidin-1-carboxylat erhalten. | 0.56 |
| | 2-(Azetidin-3-ylamino)-N-{2-[2-(4-benzoylpiperazin-1-yl)-2-oxoethyl]-2H-indazol-5-yl}-1,3-thiazol-4-carboxamid | | | |
| | | | ¹H NMR (400 MHz, DMSO-d6): δ = 1.39 (s, 9 H), 3.55 (br. s., 4 H), 3.64 (br. s., 4 H), 3.78 (dd, 2 H), 4.25 (t, 2 H), 4.57 - 4.69 (m, 1 H), 5.48 (br. s., 2 H), 7.39 - 7.52 (m, 7 H), 7.54 - 7.59 (m, 1 H), 8.22 (d, 1 H), 8.25 - 8.29 (m, 1 H), 8.43 (d, 1 H), 9.55 (s, 1 H). 50 mg (0.07 mmol) tert-Butyl-3-{[4-({2-[2-(4-benzoylpiperazin-1-yl)-2-oxoethyl]-2H-indazol-5-yl}carbamoyl)-1,3-thiazol-2-yl] amino}azetidin-1-carboxylat wurden in 200 µl 4 M Chlorwasserstoff in Dioxan gelöst und 24 h bei Raumtemperatur gerührt. Die Reaktionsmischung wurde eingeengt und in Dichlormethan aufgenommen, mit gesättigter Natriumhydrogencarbonatlösung und gesättigter Natriumchloridlösung gewaschen, über Natriumsulfat getrocknet, filtriert und eingeengt. Nach Trocknen wurden 11 mg (0.02 mmol) 2-(Azetidin-3-ylamino)-N-{2-[2-(4-benzoylpiperazin-1-yl)-2-oxoethyl]-2H-indazol-5-yl}-1,3-thiazol-4-carboxamid erhalten. | |
| 240 | 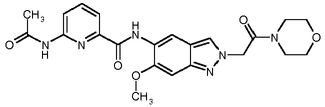 | 6-16 | 6-Acetamidopyridin-2-carbonsäure | 0.76 |
| | 6-Acetamido-N-{6-methoxy-2-[2-(morpholin-4-yl)-2-oxoethyl]-2H-indazol-5-yl}pyridin-2-carboxamid | | ¹H NMR (400 MHz, DMSO-d6): δ = 2.21 (s, 3 H), 3.47 (d, 2 H), 3.51 - 3.62 (m, 4 H), 3.64 (d, 2 H), 4.03 (s, 3 H), 5.39 (s, 2 H), 7.10 (s, 1 H), 7.87 (dd, 1 H), 8.04 (t, 1 H), 8.22 (s, 1 H), 8.28 (d, 1 H), 8.69 (s, 1 H), 10.34 (s, 1 H), 10.65 (s, 1 H). | |
| 241 | 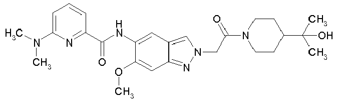 | 6-5 | 6-(Dimethylamino)pyridin-2-carbonsäure ¹H NMR (400 MHz, DMSO-d6): δ = 1.01 - 1.14 (m, 1 H), 1.05 (s, 6 H), 1.18 - 1.30 (m, 1 H), 2.50-2.52 (m, 1 H) (Signal unter DMSO),1.44 (t, 1 H), 1.75 (t, 2 H), 2.99 (t, 1 H), 3.16 (s, 6 H), 3.97 (s, 3 H), 4.03 (d, 1 H), 4.16 (s, 1 H), 4.42 (d, 1 H), 5.27 - 5.41 (m, 2 H), 6.94 (d, 1 H), 7.07 (s, 1 H), 7.36 (d, 1 H), 7.74 (dd, 1 H), 8.19 (s, 1 H), 8.67 (s, 1 H), 10.88 (s, 1 H). | 1.03 |
| | 6-(Dimethylamino)-N-(2- {2-[4-(2- hydroxypropan-2-yl)piperidin-1-yl]-2-oxoethyl}-6-methoxy-2H-indazol-5-yl)pyridin-2-carboxamid | | | |
| 242 | 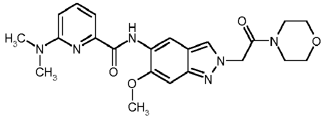 | 6-16 | 6-(Dimethylamino)pyridin-2-carbonsäure ¹H NMR (400 MHz, DMSO-d6): δ = 3.16 (s, 6 H), 3.47 (d, 2 H), 3.53 - 3.61 (m, 4 H), 3.64 (d, 2 H), 3.98 (s, 3 H), 5.38 (s, 2 H), 6.94 (d, 1 H), 7.08 (s, 1 H), 7.36 (d, 1 H), 7.74 (dd, 1 H), 8.17 - 8.21 (m, 1 H), 8.67 (s, 1 H), 10.88 (s, 1 H). | 0.99 |
| | 6-(Dimethylamino)-N-{6-methoxy-2-[2-(morpholin-4-yl)-2-oxoethyl]-2H-indazol-5-yl}pyridin-2-carboxamid | | | |
| 243 | 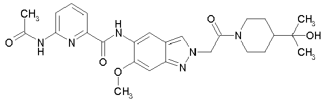 | 6-5 | 6-Acetamidopyridin-2-carbonsäure ¹H NMR (400 MHz, DMSO-d6): δ = 0.98 - 1.12 (m, 1 H), 1.05 (s, 6 H), 1.15 - 1.31 (m, 1 H), 1.39 - 1.50 (m, 1 H), 1.75 (t, 2 H), 2.21 (s, 3 H), 2.52 - 2.57 (m, 1 H), 2.99 (br. s., 1 H), 3.98 - 4.08 (m, 1 H), 4.03 (s, 3 H), 4.16 (s, 1 H), 4.42 (d, 1 H), 5.36 (d, 2 H), 7.09 (s, 1 H), 7.87 (dd, 1 H), 8.04 (t, 1 H), 8.19 - 8.23 (m, 1 H), 8.28 (d, 1 H), 8.69 (s, 1 H), 10.34 (s, 1 H), 10.65 (s, 1 H). | 0.83 |
| | 6-Acetamido-N-(2- {2-[4-(2-hydroxypropan-2-yl)piperidin-1-yl]-2-oxoethyl}-6-methoxy-2H-indazol-5-yl)pyridin-2-carboxamid | | | |
| 244 | 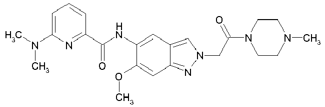 | 6-17 | 6-(Dimethylamino)pyridin-2-carbonsäure ¹H NMR (400 MHz, DMSO-d6): δ = 2.21 (s, 3 H), 2.27 - 2.31 (m, 2 H), 2.34 - 2.39 (m, 2 H), 3.16 (s, 6 H), 3.43 - 3.51 (m, 2 H), 3.51 - 3.58 (m, 2 H), 3.97 (s, 3 H), 5.36 (s, 2 H), 6.94 (d, 1 H), 7.08 (s, 1 H), 7.36 (d, 1 H), 7.74 (dd, 1 H), 8.19 (s, 1 H), 8.67 (s, 1 H), 10.88 (s, 1 H). | 0.74 |
| | 6-(Dimethylamino)-N-{6-methoxy-2-[2-(4-methylpiperazin-1-yl)-2-oxoethyl]-2H-indazol-5-yl}pyridin-2-carboxamid | | | |

**Tabelle 17: Beispiele 245 - 247**

| Bsp. | Name und Struktur | Herstellung und Anmerkungen | LC-MS Retent ionszei t [min] |
|---|---|---|---|
| 245 | 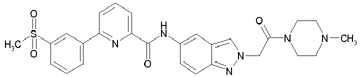 | 80 mg 6-[3-(Methylsulfonyl)phenyl]pyridin-2-carbonsäure (Intermediat 19-8) und 95 mg 2-(5-Amino-2H-indazol-2-yl)-1-(4-methylpiperazin-1-yl)ethanon (Intermediat 6-12, Rohprodukt) wurden mit EDC, HOBt und Triethylamin in THF bei Raumtemperatur über Nacht umgesetzt. Es wurde mit Wasser und Ethylacetat verdünnt. Der Feststoff wurde abgesaugt, mit Wasser und Diethylether gewaschen und getrocknet. Es wurden 48 mg der Beispielverbindung erhalten. | 0.65 |
| | N-{2-[2-(4-Methylpiperazin-1-yl)-2-oxoethyl]-2H-indazol-5-yl}-6-[3-(methylsulfonyl)phenyl]pyridin-2-carboxamid | | |
| | | ¹H-NMR (400MHz, DMSO-d6): δ = 2.20 (s, 3H), 2.24 - 2.42 (m, 4H), 3.36 (s, 3H), 3.42 - 3.51 (m, 2H), 3.51 - 3.61 (m, 2H), 5.45 (s, 2H), 7.56 - 7.64 (m, 2H), 7.84 (t, 1H), 8.05 (d, 1H), 8.16 - 8.24 (m, 2H), 8.30 (s, 1H), 8.34 - 8.40 (m, 2H), 8.75 - 8.80 (m, 2H), 10.56 (s, 1H). | |
| 246 | 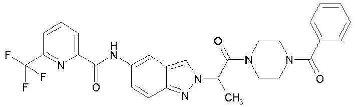 | 100 mg 2-(5-Amino-2H-indazol-2-yl)-1-(4-benzoylpiperazin-1-yl)propan-1-on (Intermediat 6-19) und 76 mg 6-(Trifluormethyl)pyridin-2-carbonsäure wurden mit EDC, HOBt und Triethylamin in THF über Nacht bei Raumtemperatur umgesetzt. Nach Zugabe von Wasser, Extraktion mit Ethylacetat und Einengen wurde durch präparative HPLC nach Methode P1 gereinigt. Es wurden 98 mg der Beispielverbindung erhalten. | 1.08 |
| | N-{2-[1-(4-Benzoylpiperazin-1-yl)-1-oxopropan-2-yl]-2H-indazol-5-yl}-6-(trifluormethy l)pyridin- 2-carboxamid | | |
| | | ¹H-NMR (400MHz, DMSO-d6): δ = 1.68 (d, 3H), 3.1 - 3.7 (breite Signale, überlagert), 5.91 (br. s., 1H), 7.34 - 7.45 (m, 5H), 7.52 - 7.61 (m, 2H), 8.14 (dd, 1H), 8.26 - 8.39 (m, 3H), 8.43 (s, 1H), 10.34 (s, 1H). | |
| 247 | 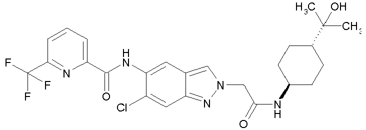 | 80 mg [6-Chlor-5-({[6-(trifluormethy l)pyridin- 2-yl]carbonyl} amino)-2H-indazol-2-yl]essigsäure und 41 mg 2-(trans-4-Aminocyclohexyl)propan-2-ol wurden mit EDC, HOBt und Triethylamin in THF über Nacht bei Raumtemperatur und 7 h bei 50°C umgesetzt. Es wurde mit Wasser und Ethylacetat versetzt. Der Feststoff wurde abgesaugt, mit Wasser und Diethylether gewaschen und getrocknet. Es wurden 92 mg der Beispielverbindung erhalten. | 1.16 |
| | N-[6-Chlor-2-(2-{[trans-4-(2-hydroxypropan-2-yl)cyclohexyl] amino}-2-oxoethyl)-2H-indazol-5-yl]-6-(trifluormethy l)pyridin- 2-carboxamid | | |
| | | ¹H-NMR (300MHz, DMSO-d6): δ = 0.92 - 1.25 (11H, enthält Singulett bei 1.01 ppm), 1.73 - 1.91 (m, 4H), 3.36 - 3.60 (m), 4.02 (s, 1H), 5.08 (s, 2H), 7.91 (s, 1H), 8.23 (d, 2H), 8.37 - 8.50 (m, 3H), 8.63 (s, 1H), 10.52 (s, 1H). | |

**Tabelle 18: Beispiele 248 - 260**

| Die Beispielverbindungen wurden aus den entsprechenden Intermediaten und Aminen nach den allgemeinen Versuchsvorschriften 2a-2g hergestellt. | | | |
|---|---|---|---|
| Bsp Nr. | Struktur/Name | Hergestellt aus | ¹H-NMR / LC-MS |
| 248 | 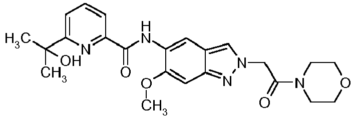 | Intermediat 9-23 und Morpholin | (400 MHz, DMSO-d6): δ = 1.57 (s, 6 H), 3.42 - 3.52 (m, 2 H), 3.52 - 3.62 (m, 4 H), 3.62 - 3.68 (m, 2 H), 3.99 (s, 3 H), 5.39 (s, 2 H), 5.47 (s, 1 H), 7.10 (s, 1 H), 7.93 (dd, 1 H), 7.99 - 8.10 (m, 2 H), 8.19 - 8.23 (m, 1 H), 8.68 (s, 1 H), 10.93 (s, 1H). |
| | 6-(2-Hydroxypropan-2-yl)-N- {6-methoxy-2-[2-(morpholin-4-yl)-2-oxoethyl]-2H-indazol-5-yl}pyridin-2-carboxamid | | |
| | | | UPLC-MS (Methode A1): Rt = 0.88 min |
| | | | MS (ESIpos): m/z = 454 (M+H)⁺ |
| 249 | 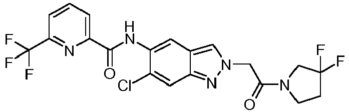 | Intermediat 9-11 und 3,3-Difluorpyrr olidin | (400 MHz, DMSO-d6): δ = 2.38 - 2.46 (m, 1 H), 2.54 - 2.61 (m, 1 H), 3.58 (t, 1 H), 3.77 (t, 1 H), 3.87 (t, 1 H), 4.11 (t, 1 H), 5.42 (s, 1 H), 5.48 (s, 1 H), 7.93 (s, 1 H), 8.24 (dd, 1 H), 8.38 - 8.45 (m, 2 H), 8.45 - 8.50 (m, 1 H), 8.64 - 8.67 (m, 1 H), 10.53 (s, 1 H). |
| | N-{6-Chlor-2-[2-(3,3-difluorpyrrolidin-1-yl)-2-oxoethyl] - 2H-indazol-5-yl}-6-(trifluormethy l)pyridin- 2-carboxamid | | |
| 250 | 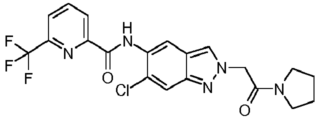 | Intermediat 9-11 und Pyrrolidin | UPLC-MS (Methode A2): Rt = 1.21 min |
| | | | MS (ESIpos): m/z = 452 (M+H)⁺ |
| | N-{6-Chlor-2-[2-oxo-2-(pyrrolidin-1-yl)ethyl]-2H-indazol-5-yl}-6-(trifluormethy l)pyridin- 2-carboxamid | | |
| 251 | 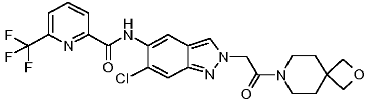 | Intermediat 9-11 und 2-Oxa-7-azaspiro[3. 5]nonan (CAS24182 0-91-7) | (300 MHz, DMSO-d6): δ = 1.75 (br. s., 2 H), 1.87 (br. s., 2 H), 3.37 - 3.51 (m, 4 H), 4.24 - 4.41 (m, 4 H), 5.50 (s, 2 H), 7.91 (s, 1 H), 8.24 (d, 1 H), 8.36 - 8.51 (m, 3 H), 8.64 (s, 1 H), 10.53 (s, 1 H). |
| | N-{6-Chlor-2-[2-(2-oxa-7-azaspiro[3.5]non-7-yl)-2-oxoethyl]-2H-indazol-5-yl}-6-(trifluormethy l)pyridin- 2-carboxamid | | |
| | | | UPLC-MS (Methode A1): Rt = 1.22 min |
| | | | MS (ESIpos): m/z = 508 (M+H)⁺ |
| 252 | 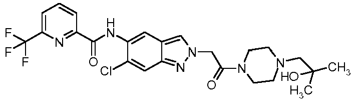 | Intermediat 9-11 und 2-Methyl-1-(piperazin-1-yl)propan-2-ol | (300 MHz, DMSO-d6, ausgewählte Signale): δ = 1.11 (s, 6 H), 2.23 (s, 2 H), 2.59 (br. s., 2 H), 3.46 (br. s., 2 H), 3.53 (br. s., 2 H), 4.16 (s, 1 H), 5.49 (s, 2 H), 7.92 (s, 1 H), 8.24 (dd, J=7.4, 1.2 Hz, 1 H), 8.37 - 8.51 (m, 3 H), 8.64 (s, 1 H), 10.53 (s, 1 H). |
| | N-(6-Chlor-2-{2-[4-(2-hydroxy-2-methylpropyl)piperazin-1-yl]-2-oxoethyl}-2H-indazol-5-yl)-6-(trifluormethy l)pyridin- 2-carboxamid | | |
| 253 | 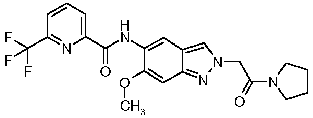 | Intermediat 9-12 und Pyrrolidin | (400 MHz, DMSO-d6): δ = 1.81 (s, 2 H), 1.91 - 1.98 (m, 2 H), 3.33 - 3.37 (m, 2 H), 3.54 (t, 2 H), 3.99 (s, 3 H), 5.27 (s, 2 H), 7.12 (s, 1 H), 8.19 - 8.25 (m, 2 H), 8.42 (d, 1 H), 8.44 - 8.49 (m, 1 H), 8.71 (s, 1 H), 10.51 (s, 1 H). |
| | N-{6-Methoxy-2-[2-oxo-2-(pyrrolidin-1-yl)ethyl]-2H-indazol-5-yl}-6-(trifluormethyl)pyridin-2-carboxamid | | |
| | | | UPLC-MS (Methode A2): Rt = 1.14 min |
| | | | MS (ESIpos): m/z = 448 (M+H)⁺ |
| 254 | 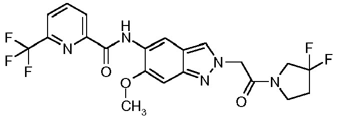 | Intermediat 9-12 und 3,3-Difluorpyrr olidin | (400 MHz, DMSO-d6): δ = 2.37 - 2.44 (m, 1 H), 2.54 - 2.61 (m, 1 H), 3.58 (t, 1 H), 3.76 (t, 1 H), 3.86 (t, 1 H), 3.99 (s, 3 H), 4.10 (t, 1 H), 5.27 - 5.34 (m, 1 H), 5.37 (s, 1 H), 7.12 (s, 1 H), 8.15 - 8.30 (m, 2 H), 8.37 - 8.45 (m, 1 H), 8.45 - 8.51 (m, 1 H), 8.71 (s, 1 H), 10.51 (s, 1 H). |
| | N-{2-[2-(3,3-Difluorpyrrolidin-1-yl)-2-oxoethyl]-6-methoxy-2H-indazol-5-yl}-6-(trifluormethyl)pyridin-2-carboxamid | | |
| | | | UPLC-MS (Methode A2): Rt = 1.17 min |
| | | | MS (ESIpos): m/z = 484 (M+H)⁺ |
| 255 | 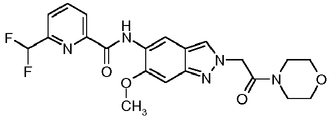 | Intermediat 9-24 und Morpholin | (400 MHz, DMSO-d6): δ = 3.40 - 3.49 (m, 2 H), 3.53 - 3.62 (m, 4 H), 3.62 - 3.68 (m, 2 H), 4.00 (s, 3 H), 5.40 (s, 2 H), 7.16 (t, 1 H), 7.11 (s, 1 H), 8.00 (d, 1 H), 8.23 (s, 1 H), 8.27 - 8.38 (m, 2 H), 8.71 (s, 1 H), 10.56 (s, 1 H). |
| | 6-(Difluormethyl)-N- {6-methoxy-2-[2-(morpholin-4-yl)-2-oxoethyl]-2H-indazol-5-yl}pyridin-2-carboxamid | | |
| | | | UPLC-MS (Methode A1): Rt = 0.99 min |
| | | | MS (ESIpos): m/z = 446 (M+H)⁺ |
| 256 | 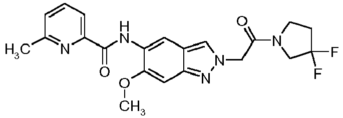 | Intermediat 9-19 und 3,3-Difluorpyrr olidin | (400 MHz, DMSO-d6): δ = 2.35 - 2.48 (m, 1 H), 2.54 - 2.60 (m, 1 H), 2.63 (s, 3 H), 3.58 (t, 1 H), 3.76 (s, 1 H), 3.86 (t, 1 H), 4.01 (s, 3 H), 4.04 - 4.16 (m, 1 H), 5.30 (s, 1 H), 5.36 (s, 1 H), 7.10 (s, 1 H), 7.56 (dd, 1 H), 7.94 - 8.02 (m, 2 H), 8.20 - 8.24 (m, 1 H), 8.72 (s, 1 H), 10.71 (s, 1 H). |
| | N-{2-[2-(3,3-Difluorpyrrolidin-1-yl)-2-oxoethyl]-6-methoxy-2H-indazol-5-yl}-6-methylpyridin-2-carboxamid | | |
| | | | UPLC-MS (Methode A1): Rt = 1.17 min |
| | | | MS (ESIpos): m/z = 430 (M+H)⁺ |
| 257 | 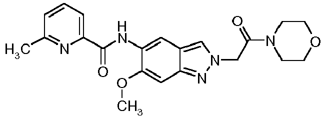 | Intermediat 9-19 und Morpholin | (400 MHz, DMSO-d6): δ = 2.63 (s, 3 H), 3.47 (s, 2 H), 3.53 - 3.62 (m, 4 H), 3.64 (s, 2 H), 4.01 (s, 3 H), 5.39 (s, 2 H), 7.09 (s, 1 H), 7.56 (dd, J=7.1, 1.5 Hz, 1 H), 7.93 - 8.03 (m, 2 H), 8.21 (s, 1 H), 8.72 (s, 1 H), 10.71 (s, 1 H). |
| | N-{6-Methoxy-2-[2-(morpholin-4-yl)-2-oxoethyl]-2H-indazol-5-yl}-6-methylpyridin-2-carboxamid | | |
| | | | UPLC-MS (Methode A1): Rt = 1.00 min |
| | | | MS (ESIpos): m/z = 410 (M+H)⁺ |
| 258 | 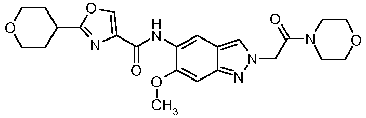 | Intermediat 9-20 und Morpholin | (400 MHz, DMSO-d6): δ = 1.71 - 1.84 (m, 2 H), 1.92 - 2.02 (m, 2 H), 3.16 - 3.28 (m, 1 H), 3.42 - 3.50 (m, 4 H), 3.53 - 3.62 (m, 4 H), 3.64 (d, 2 H), 3.88 - 3.95 (m, 2 H), 3.97 (s, 3 H), 5.38 (s, 2 H), 7.08 (s, 1 H), 8.20 (s, 1 H), 8.56 (s, 1 H), 8.74 (s, 1 H), 9.41 (s, 1 H). |
| | N-{6-Methoxy-2-[2-(morpholin-4-yl)-2-oxoethyl]-2H-indazol-5-yl}-2-(tetrahydro-2H-pyran-4-yl)-1,3-oxazol-4-carboxamid | | |
| | | | UPLC-MS (Methode A1): Rt = 0.88 min |
| | | | MS (ESIpos): m/z = 470 (M+H)⁺ |
| 259 | 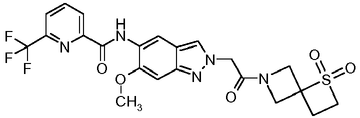 | Intermediat 9-12 und 1-Thia-6-azaspiro[3. 3] heptan-1,1-dioxid (CAS13525 46-75-8) | UPLC-MS (Methode A2): Rt = 1.12 min |
| | N-{2-[2-(1,1-Dioxido-1-thia-6-azaspiro[3.3]hept-6-yl)-2-oxoethyl]-6-methoxy-2H-indazol-5-yl}-6-(trifluormethyl)pyridin-2-carboxamid | | MS (ESIpos): m/z = 524 (M+H)⁺ |
| 260 | 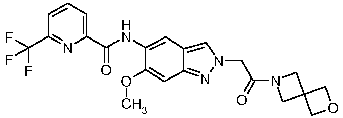 | Intermediat 9-12 und 2-Oxa-6-azaspiro[3. 3]heptan (CAS 174-78-7) | (300 MHz, DMSO-d6): δ = 3.99 (s, 3 H), 4.10 (s, 2 H), 4.32 (s, 2 H), 4.67 (s, 4 H), 5.06 (s, 2 H), 7.12 (s, 1 H), 8.22 (d, J=7.6 Hz, 1 H), 8.25 (s, 1 H), 8.41 (s, 1 H), 8.46 (s, 1 H), 8.70 (s, 1 H), 10.51 (s, 1 H). |
| | N-{6-Methoxy-2-[2-(2-oxa-6-azaspiro[3.3]hept-6-yl)-2-oxoethyl]-2H-indazol-5-yl}-6-(trifluormethy l)pyridin- 2-carboxamid | | |
| | | | UPLC-MS (Methode A2): Rt = 1.08 min |
| | | | MS (ESIpos): m/z = 476 (M+H)⁺ |

### Beispiel 261

### N-{6-(3-Hydroxy-2,2-dimethylpropoxy)-2-[2-(morpholin-4-yl)-2-oxoethyl]-2H-indazol-5-yl}-6-(trifluormethyl)pyridin-2-carboxamid

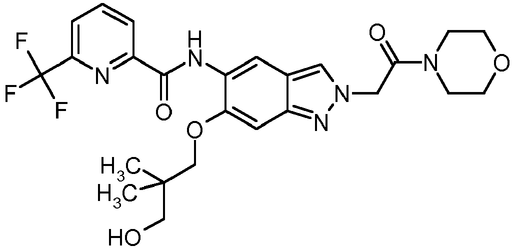

### Stufe A:

### N-{6-(3-{[tert-Butyl(dimethyl)silyl]oxy}-2,2-dimethylpropoxy)-2-[2-(morpholin-4-yl)-2-oxoethyl]-2H-indazol-5-yl}-6-(trifluormethyl)pyridin-2-carboxamid

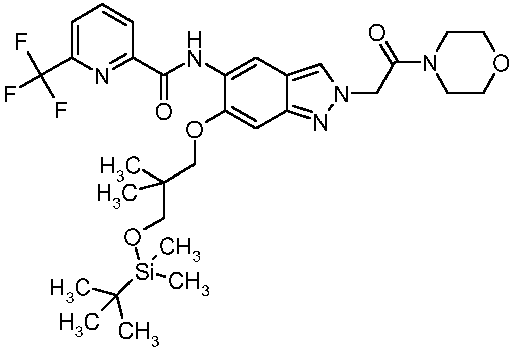

100 mg (0.22 mmol) N-{6-Hydroxy-2-[2-(morpholin-4-yl)-2-oxoethyl]-2H-indazol-5-yl}-6-(trifluormethyl)pyridin-2-carboxamid (Beispiel 287) wurde in 2.0 ml *N*,*N-*Dimethylformamid gelöst und unter Rühren mit 46 mg (0.33 mmol) Kaliumcarbonat versetzt. Die Suspension wurde 10 Minuten bei 25°C gerührt und anschließend wurden 94 mg (0.33 mmol) (3-Brom-2,2-dimethylpropoxy)(tert-butyl)dimethylsilan hinzugegeben. Die Reaktionsmischung wurde für 1 h bei 100°C in der Mikrowelle gerührt. Anschließend wurde die Reaktionsmischung filtriert und mit präparativer HPLC. Man erhielt 34 mg (24 % d.Th.) der Titelverbindung.
UPLC-MS (Methode A1): Rₜ = 1.70 min
MS (ESIpos): m/z = 650 (M+H)⁺
¹H NMR (400 MHz, DMSO-d6): δ = -0.17 - -0.09 (m, 6 H), 0.75 (s, 9 H), 1.07 (s, 6 H), 3.42 - 3.51 (m, 2 H), 3.54 - 3.64 (m, 2 H), 3.54 - 3.64 (m, 4 H), 3.64 - 3.71 (m, 2 H), 3.88 (s, 2 H), 5.40 (s, 2 H), 7.05 (s, 1 H), 8.17 - 8.27 (m, 2 H), 8.42 (t, 1 H), 8.49 - 8.56 (m, 1 H), 8.79 (s, 1 H), 10.42 (s, 1 H).

### Stufe B:

40 mg (0.06 mmol) N-{6-(3-{[tert-Butyl(dimethyl)silyl]oxy}-2,2-dimethylpropoxy)-2-[2-(morpholin-4-yl)-2-oxoethyl]-2H-indazol-5-yl}-6-(trifluormethyl)pyridin-2-carboxamid wurden in 2.5 ml Tetrahydrofuran gelöst, mit 185 µl (0.18 mmol) einer 1 M Lösung von Tetrabutylammoniumfluoridund in Tetrahydrofuran versetzt und für 2 h bei 25°C gerührt. Die Reaktionsmischung wurde mit 5 ml Wasser versetzt und eingeengt. Der entstandene Niederschlag wurde abgesaugt, mit Wasser und Diethylether gewaschen und im Vakuum getrocknet. Man erhielt 26 mg (48% der Theorie) N-{6-(3-Hydroxy-2,2-dimethylpropoxy)-2-[2-(morpholin-4-yl)-2-oxoethyl]-2H-indazol-5-yl}-6-(trifluormethyl)pyridin-2-carboxamid.
UPLC-MS (Methode A1): Rₜ = 1.09 min
MS (ESIpos): m/z = 536 (M+H)⁺
¹H-NMR (400MHz, DMSO-d6): δ = 1.06 (s, 6 H), 3.42 (d, Hz, 2 H), 3.45 - 3.51 (m, 2 H), 3.54 - 3.63 (m, 4 H), 3.63 - 3.68 (m, 2 H), 3.90 (s, 2 H), 4.63 - 4.69 (m, 1 H), 5.40 (s, 2 H), 7.05 (s, 1 H), 8.17 - 8.25 (m, 2 H), 8.41 (t, 1 H), 8.51 (d, 1 H), 8.81 (s, 1 H), 10.44 (s, 1 H).

### Beispiel 262

### 6-Ethyl-N-{6-methoxy-2-[2-(morpholin-4-yl)-2-oxoethyl]-2H-indazol-5-yl}pyridin-2-carboxamid

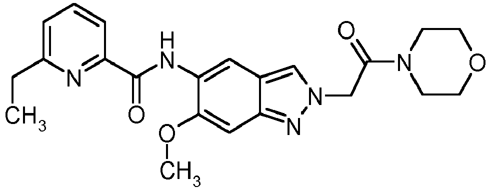

50 mg (0.11 mmol) 6-Brom-N-{6-methoxy-2-[2-(morpholin-4-yl)-2-oxoethyl]-2H-indazol-5-yl}pyridin-2-carboxamid (Intermediat 15-2) wurden in 750 µl trockenem Dioxan suspendiert, mit 86 µl (0.09 mmol) einer 1.1 M Lösung von Diethylzink in Toluol und 4 mg (0.01 mmol) 1,1'-Bis(diphenylphospino)ferrocen-palladium(II)dichlorid Dichlormethan Komplex versetzt und für 24 h bei 40°C gerührt. Es wurden erneut 86 µl (0.09 mmol) einer 1.1 M Lösung von Diethylzink in Toluol und 4 mg (0.01 mmol) 1,1'-Bis(diphenylphospino)ferrocen-palladium(II)dichlorid Dichlormethan Komplex hinzugegeben und für weitere 24 h bei 60°C gerührt. Es wurden nochmals 86 µl (0.09 mmol) einer 1.1 M Lösung von Diethylzink in Toluol und 4 mg (0.01 mmol) 1,1'-Bis(diphenylphospino)ferrocen-palladium(II)dichlorid Dichlormethan Komplex hinzugegeben und für weitere 24 h bei 60°C gerührt Die Reaktionsmischung wurde filtriert und das Filtrat eingeengt. Das Rohprodukt wurde in 2,5 ml Dimethylsulfoxid gelöst und mit präparativer HPLC nach Methode P1 gereinigt. Die Produktfraktion wurde lyophilisiert. Man erhielt 5.8 mg (11 % d.Th.) der Titelverbindung.
UPLC-MS (Methode A1): Rₜ = 1.11 min
MS (ESIpos): m/z = 424 (M+H)⁺
¹H-NMR (300MHz, DMSO-d6): δ = 1.37 (t, 3 H), 2.92 (q, 2 H), 3.42 - 3.50 (m, 2 H), 3.58 (br. s., 4 H), 3.62 - 3.69 (m, 2 H), 4.00 (s, 3 H), 5.39 (s, 2 H), 7.10 (s, 1 H), 7.57 (dd, 2 H), 7.97 - 8.02 (m, 2 H), 8.21 (s, 1 H), 8.71 (s, 1 H), 10.88 (s, 1 H).

### Beispiel 263

### 6-Isobutyl-N-{6-methoxy-2-[2-(morpholin-4-yl)-2-oxoethyl]-2H-indazol-5-yl}pyridin-2-carboxamid

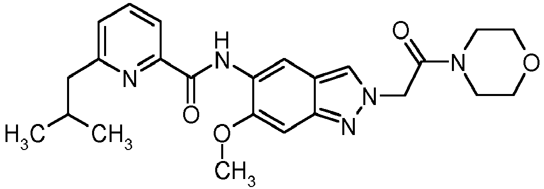

50 mg (0.11 mmol) 6-Brom-N-{6-methoxy-2-[2-(morpholin-4-yl)-2-oxoethyl]-2H-indazol-5-yl}pyridin-2-carboxamid (Intermediat 15-2) wurden in 1.5 ml Tetrahydrofuran gelöst, mit 316 µl (0.16 mmol) einer 0.5 M Lösung von 2-Methylpropylzinkbromid in Tetrahydrofuran und 3 mg (0.01 mmol) Bis(tri-tert-butylphosphin)palladium(0) versetzt und für 48 h bei 25°C gerührt. Die Reaktionsmischung wurde filtriert und das Filtrat eingeengt. Das Rohprodukt wurde in 2,5 ml Dimethylsulfoxid gelöst und mit präparativer HPLC nach Methode P1 gereinigt. Die Produktfraktion wurde lyophilisiert. Man erhielt 2.8 mg (6 % d.Th.) der Titelverbindung.
UPLC-MS (Methode A1): Rₜ = 1.27 min
MS (ESIpos): m/z = 452 (M+H)⁺
¹H-NMR (300MHz, DMSO-d6): δ = 0.99 (d, 6 H) 2.15 - 2.29 (m, 1 H) 2.77 (d, 2 H) 3.47 (d, 2 H) 3.53 - 3.69 (m, 6 H) 3.99 (s, 3 H) 5.39 (s, 2 H) 7.09 (s, 1 H) 7.53 (dd, 1 H) 7.94 - 8.03 (m, 2 H) 8.21 (s, 1 H) 8.71 (s, 1 H) 10.85 (s, 1 H).

### Beispiel 264

### Methyl-2-[2-(morpholin-4-yl)-2-oxoethyl]-5-({[6-(trifluormethyl)pyridin-2-yl]carbonyl}amino)-2H-indazol-6-carboxylat

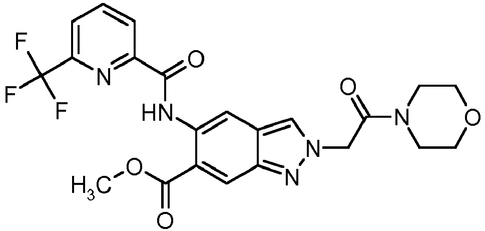

100 mg (0.60 mmol) Methyl-5-({[6-(trifluormethyl)pyridin-2-yl]carbonyl}amino)-1H-indazol-6-carboxylat (Intermediat 14-9) wurden in 1 ml Tetrahydrofuran gelöst, mit 228 mg (1.10 mmol) 2-Brom-1-(morpholin-4-yl)ethanon und 235 µl (1.10 mmol) *N,N-*Dicyclohexylmethylamin versetzt und für 24 h bei 75°C gerührt. Die Reaktionsmischung wurde mittels Membranfilter filtriert, das Filtrat mit 1 ml Dimethylsulfoxid verdünnt und per präparativer HPLC gereinigt. Die Produktfraktion wurden lyophilisiert. Man erhielt 15 mg (11 % d.Th.) der Titelverbindung.
UPLC-MS (Methode A2): Rₜ = 1.10 min
MS (ESIpos): m/z = 492 (M+H)⁺
¹H NMR (400 MHz, CHLOROFORM-d): δ = 3.45 - 3.50 (m, 2 H), 3.54 - 3.64 (m, 4 H), 3.64 - 3.70 (m, 2 H), 3.97 (s, 3 H), 5.59 (s, 2 H), 8.21 (dd, 1 H), 8.36 - 8.43 (m, 1 H), 8.44 - 8.49 (m, 3 H), 9.08 (s, 1 H), 12.52 (s, 1 H).

### Beispiel 265

### Methyl-5- {[(6-methylpyridin-2-yl)carbonyl]amino}-2-[2-(morpholin-4-yl)-2-oxoethyl]-2H-indazol-6-carboxylat

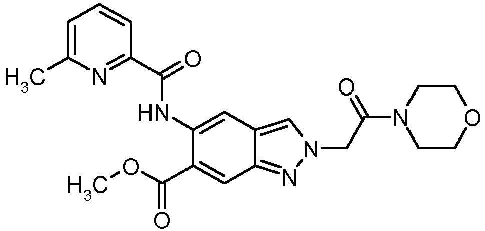

50 mg (0.16 mmol) (Intermediat 14-10) wurden in 2.5 ml Tetrahydrofuran gelöst, mit 134 mg (0.64 mmol) 2-Brom-1-(morpholin-4-yl)ethanon und 138 µl (0.64 mmol) *N,N-*Dicyclohexylmethylamin versetzt und für 16 h bei 80°C gerührt. Die Reaktionsmischung wurde filtriert und das Filtrat eingeengt. Der Rückstand wurde in Dichlormethan gelöst und drei Mal mit 1 M Salzsäurelösung und drei Mal mit gesättigter Natriumchloridlösung gewaschen, über einen wasserabweisenden Filter filtriert und unter Einengen auf Isolute® HM-N (Biotage) adsorbiert. Die Isolute wurde auf eine mit Hexan vorequilibrierte Kartusche (40 g; Puriflash) gegeben und mittels Isolera® Flash-Aufreinigungssystem (Biotage) die Chromatographie durchgeführt (Laufmittel: Hexan-Ethylacetat; Flussrate: 25 ml/mim; Gradient: 90:10->25:75). Die vereinigten Produktfraktionen wurden eingeengt und getrocknet. Man erhielt 20 mg (28% der Theorie) der Titelverbindung.UPLC-MS (Methode A2): Rₜ = 1.05 min
MS (ESIpos): m/z = 438 (M+H)⁺
¹H NMR (400 MHz, CHLOROFORM-d): δ = 2.65 (s, 3 H), 3.48 (d, 2 H), 3.59 (dd, 4 H), 3.67 (d, 2 H), 3.99 (s, 3 H), 5.58 (s, 2 H), 7.55 (dd, 1 H), 7.81 - 8.04 (m, 2 H), 8.38 - 8.47 (m, 2 H), 9.09 (s, 1 H), 12.57 (s, 1 H).

**Tabelle 19: Beispiele 266 - 286**

| Die Beispielverbindungen wurden aus den entsprechenden Intermediaten und Carbonsäuren nach den allgemeinen Versuchsvorschriften 1a - 1e hergestellt. | | | |
|---|---|---|---|
| Bsp Nr. | Struktur/Name | Hergestellt aus | ¹H-NMR / LC-MS |
| 266 | 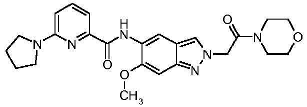 | Intermediat 6-16 und 6-(Pyrrolidin-1-yl)pyridin-2-carbonsäure (CAS 450368-20-4) | (400MHz, DMSO-d6): δ = 1.98 - 2.06 (m, 4 H), 3.44 - 3.49 (m, 2 H), 3.51 (br. s., 4 H), 3.54 - 3.61 (m, 4 H), 3.61 - 3.68 (m, 2 H), 3.98 (s, 3 H), 5.38 (s, 2 H), 6.73 (d, 1 H), 7.07 (s, 1 H), 7.33 (d, 1 H), 7.72 (dd, 1 H), 8.19 (s, 1 H), 8.67 (s, 1 H), 10.93 (s, 1 H). |
| | N-{6-Methoxy-2-[2-(morpholin-4-yl)-2-oxoethyl]-2H-indazol-5-yl}-6-(pyrrolidin-1-yl)pyridin-2-carboxamid | | |
| | | | UPLC-MS (Methode A1): Rt = 1.16 min |
| | | | MS (ESIpos): m/z = 465 (M+H)+ |
| 267 | 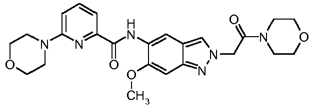 | Intermediat 6-16 und 6-(Morpholin-4-yl)pyridin-2-carbonsäure (CAS 554405-17-3) | (400MHz, DMSO-d6): δ = 3.42 - 3.50 (m, 2 H), 3.53 - 3.68 (m, 10 H), 3.75 - 3.84 (m, 4 H), 3.97 (s, 3 H), 5.38 (s, 2 H), 7.09 (s, 1 H), 7.15 (d, 1 H), 7.46 (d, 1 H), 7.81 (dd, 1 H), 8.17 - 8.21 (m, 1 H), 8.66 (s, 1 H), 10.79 (s, 1 H). |
| | N-{6-Methoxy-2-[2-(morpholin-4-yl)-2-oxoethyl]-2H-indazol-5-yl}-6-(morpholin-4-yl)pyridin-2-carboxamid | | |
| | | | UPLC-MS (Methode A1): Rt = 0.96 min |
| | | | MS (ESIpos): m/z = 481 (M+H)+ |
| 268 | 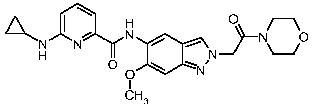 | Intermediat 6-16 und 6-(Cyclopropyl amino)pyridi n-2-carbonsäure (hergestellt nach Synthesesche ma 8, erhältlich von Ukrorgsyntez Ltd., Bestellnumm er BBV-33897980) | (300MHz, DMSO-d6): δ = 0.40 - 0.58 (m, 2 H), 0.74 - 0.89 (m, 2 H), 2.60 - 2.79 (m, 1 H), 3.41 - 3.53 (m, 2 H), 3.53 - 3.69 (m, 6 H), 3.96 (s, 3 H), 5.38 (s, 2 H), 6.78 (d, 1 H), 7.06 (s, 1 H), 7.26 (s, 1 H), 7.36 (d, 1 H), 7.64 (d, 1 H), 8.19 (s, 1 H), 8.72 (s, 1 H), 10.80 (s, 1 H). |
| | 6-(Cyclopropylamino)-N-{6-methoxy-2-[2-(morpholin-4-yl)-2-oxoethyl]-2H-indazol-5-yl}pyridin-2-carboxamid | | |
| | | | LC-MS (Methode A3): Rt = 0.95 min |
| | | | MS (ESIpos): m/z = 451 (M+H)+ |
| 269 | 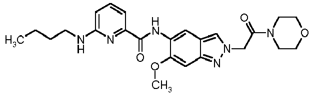 | Intermediat 6-16 und 6-(Butylamino) pyridin-2-carbonsäure (CAS 1250403-97-4) | (300MHz, DMSO-d6): δ = 0.95 (t, 3 H), 1.44 (dq, 2 H), 1.63 (quin, 2 H), 3.36 - 3.44 (m, 2 H), 3.44 - 3.51 (m, 2 H), 3.58 (br. s., 4 H), 3.62 - 3.70 (m, 2 H), 3.98 (s, 3 H), 5.38 (s, 2 H), 6.72 (d, 1 H), 7.02 (t, 1 H), 7.07 (s, 1 H), 7.26 (d, 1 H), 7.50 - 7.62 (m, 1 H), 8.19 (s, 1 H), 8.69 (s, 1 H), 10.82 (s, 1 H). |
| | 6-(Butylamino)-N- {6-methoxy-2-[2-(morpholin-4-yl)-2-oxoethyl]-2H-indazol-5-yl}pyridin-2-carboxamid | | |
| | | | LC-MS (Methode A3): Rt = 1.07 min |
| | | | MS (ESIpos): m/z = 467 (M+H)+ |
| 270 | 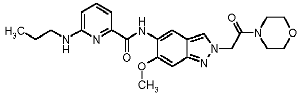 | Intermediat 6-16 und 6-(Propylamin o)pyridin-2-carbonsäure (hergestellt nach Synthesesche ma 8, erhältlich von Ukrorgsyntez Ltd., Bestellnumm er BBV-33897968) | (300MHz, DMSO-d6): δ = 1.00 (t, 3 H), 1.59 - 1.74 (m, 2 H), 3.34 - 3.42 (m, 2 H), 3.42 - 3.50 (m, 2 H), 3.58 (br. s., 4 H), 3.62 - 3.68 (m, 2 H), 3.98 (s, 3 H), 5.38 (s, 2 H), 6.72 (d, 1 H), 7.03 - 7.12 (m, 2 H), 7.26 (d, 1 H), 7.50 - 7.66 (m, 1 H), 8.19 (s, 1 H), 8.69 (s, 1 H), 10.83 (s, 1 H). |
| | N-{6-Methoxy-2-[2-(morpholin-4-yl)-2-oxoethyl]-2H-indazol-5-yl}-6-(propylamino)pyridin-2-carboxamid | | |
| | | | UPLC-MS (Methode A1): Rt = 1.05 min |
| | | | MS (ESIpos): m/z = 453 (M+H)⁺ |
| 271 | 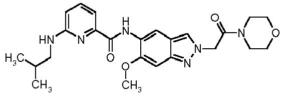 | Intermediat 6-16 und Kalium-6-(isobutylami no)pyridin-2-carboxylat (Intermediat 19-15) | (300MHz, DMSO-d6): δ = 1.00 (d, 6 H) 1.84 - 2.02 (m, 1 H) 3.25 (t, 2 H) 3.47 (d, 2 H) 3.52 - 3.72 (m, 6 H) 3.99 (s, 3 H) 5.38 (s, 2 H) 6.75 (d, 1 H) 6.98 - 7.13 (m, 2 H) 7.26 (d, 1 H) 7.57 (dd, 1 H) 8.19 (s, 1 H) 8.70 (s, 1 H) 10.75 (s, 1 H). |
| | 6-(Isobutylamino)-N-{6-methoxy-2-[2-(morpholin-4-yl)-2-oxoethyl]-2H-indazol-5-yl}pyridin-2-carboxamid | | |
| | | | UPLC-MS (Methode A1): Rt = 1.12 min |
| | | | MS (ESIpos): m/z = 467 (M+H)⁺ |
| 272 | 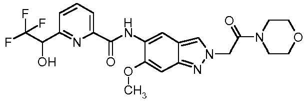 | Intermediat 6-16 und Kalium-6-(2,2,2-trifluor-1-hydroxyethyl )pyridin-2-carboxylat (Intermediat 19-10) | (300MHz, DMSO-d6): δ = 3.42 - 3.52 (m, 2 H), 3.58 (br. s., 4 H), 3.62 - 3.67 (m, 2 H), 3.97 (s, 3 H), 5.29 - 5.39 (m, 1 H), 5.40 (s, 2 H), 7.09 (s, 1 H), 7.31 (d, J=6.0 Hz, 1 H), 7.91 (t, J=4.5 Hz, 1 H), 8.16 - 8.25 (m, 3 H), 8.67 (s, 1 H), 10.70 (s, 1 H). |
| | N-{6-Methoxy-2-[2-(morpholin-4-yl)-2-oxoethyl]-2H-indazol-5-yl}-6-(2,2,2-trifluor-1-hydroxyethyl)pyridin-2-carboxamid (Enantiomer 1)* | | |
| | | | UPLC-MS (Methode A2): Rt = 0.95 min |
| | | | MS (ESIpos): m/z = 494 (M+H)⁺ |
| 273 | 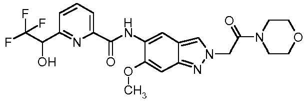 | Intermediat 6-16 und Kalium-6-(2,2,2-trifluor-1-hydroxyethyl )pyridin-2-carboxylat (Intermediat 19-10) | (300MHz, DMSO-d6): δ = 3.42 - 3.52 (m, 2 H), 3.58 (br. s., 4 H), 3.62 - 3.67 (m, 2 H), 3.97 (s, 3 H), 5.29 - 5.39 (m, 1 H), 5.40 (s, 2 H), 7.09 (s, 1 H), 7.31 (d, J=6.0 Hz, 1 H), 7.91 (t, J=4.5 Hz, 1 H), 8.16 - 8.25 (m, 3 H), 8.67 (s, 1 H), 10.70 (s, 1 H). |
| | N-{6-Methoxy-2-[2-(morpholin-4-yl)-2-oxoethyl]-2H-indazol-5-yl}-6-(2,2,2-trifluor-1-hydroxyethyl)pyridin-2-carboxamid (Enantiomer 2)* | | |
| | | | UPLC-MS (Methode A2): Rt = 0.95 min |
| | | | MS (ESIpos): m/z = 494 (M+H)⁺ |
| 274 | 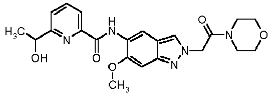 | Intermediat 6-16 und Kalium-6-(1-hydroxyethyl )pyridin-2-carboxylat (Intermediat 19-1) | (300MHz, DMSO-d6): δ = 1.52 (d, 3 H), 3.41 - 3.51 (m, 2 H), 3.58 (br. s., 4 H), 3.63 - 3.69 (m, 2 H), 4.00 (s, 2 H), 4.80 - 4.92 (m, 1 H), 5.39 (s, 2 H), 5.59 (d, 1 H), 7.10 (s, 1 H), 7.80 (dd, 1 H), 8.00 - 8.13 (m, 2 H), 8.21 (s, 1 H), 8.69 (s, 1 H), 10.79 (s, 1 H). |
| | 6-(1-Hydroxyethyl)-N-{6-methoxy-2-[2-(morpholin-4-yl)-2-oxoethyl]-2H-indazol-5-yl}pyridin-2-carboxamid | | |
| | | | UPLC-MS (Methode A1): Rt = 0.85 min |
| | | | MS (ESIpos): m/z = 440 (M+H)⁺ |
| 275 | 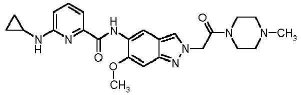 | Intermediat 6-17 und 6-(Cyclopropyl amino)pyridi n-2-carbonsäure | (400MHz, DMSO-d6): δ = 0.44 - 0.56 (m, 2 H), 0.77 - 0.87 (m, 3 H), 2.20 (s, 3 H), 2.25 - 2.31 (m, 2 H), 2.36 (br. s., 2 H), 2.63 - 2.74 (m, 1 H), 3.46 (br. s., 2 H), 3.54 (br. s., 2 H), 3.96 (s, 3 H), 5.36 (s, 2 H), 6.78 (d, 1 H), 7.06 (s, 1 H), 7.24 (s, 1 H), 7.36 (d, 1 H), 7.65 (t, 1 H), 8.18 (s, 1 H), 8.71 (s, 1 H), 10.79 (s, 1 H). |
| | 6-(Cyclopropylamino)-N-{6-methoxy-2-[2-(4-methylpiperazin-1-yl)-2-oxoethyl]-2H-indazol-5-yl}pyridin-2-carboxamid | | |
| | | | UPLC-MS (Methode A1): Rt = 0.89 min |
| | | | MS (ESIpos): m/z = 464 (M+H)⁺ |
| 276 | 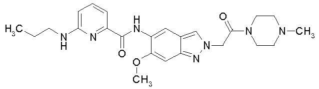 | Intermediat 6-17 und 6-(Propylamin o)pyridin-2-carbonsäure | (300MHz, DMSO-d6, ausgewählte Signale): δ = 1.00 (t, 3 H), 1.59 - 1.74 (m, 2 H), 2.28 (s, 3 H), 2.41 (br. s., 2 H) 3.21 - 3.43 (m, 2 H), 3.49 (br. s., 2 H), 3.58 (br. s., 2 H), 3.98 (s, 3 H), 5.38 (s, 2 H), 6.72 (d, 1 H), 7.00 - 7.09 (m, 2 H), 7.26 (d, 1 H), 7.57 (dd, 1 H), 8.18 (s, 1 H), 8.69 (s, 1 H), 10.82 (s, 1 H). |
| | N-{6-Methoxy-2-[2-(4-methylpiperazin-1-yl)-2-oxoethyl]-2H-indazol-5-yl}-6-(propylamino)pyridin-2-carboxamid | | |
| | | | UPLC-MS (Methode A1): Rt = 0.86 min |
| | | | MS (ESIpos): m/z = 466 (M+H)⁺ |
| 277 | 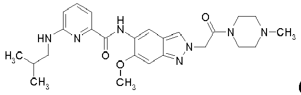 | Intermediat 6-17 und Kalium-6-(isobutylami no)pyridin-2-carboxylat (Intermediat 19-15) | (400MHz, DMSO-d6): δ = 1.00 (d, 6 H) 1.94 (dt, 1 H) 2.20 (s, 3 H) 2.25 - 2.33 (m, 2 H) 2.33 - 2.41 (m, 2 H) 3.25 (t, 2 H) 3.41 - 3.50 (m, 2 H) 3.50 - 3.59 (m, 2 H) 3.99 (s, 3 H) 5.36 (s, 2 H) 6.71 - 6.80 (m, 1 H) 7.00 - 7.11 (m, 2 H) 7.22 - 7.30 (m, 1 H) 7.57 (dd, 1 H) 8.18 (s, 1 H) 8.70 (s, 1 H) 10.75 (s, 1 H). |
| | 5-(Isobutylamino)-N-{6-methoxy-2-[2-(4-methylpiperazin-1-yl)-2-oxoethyl]-2H-indazol-5-yl}pyridin-2-carboxamid | | |
| | | | UPLC-MS (Methode A1): Rt = 0.90 min |
| | | | MS (ESIpos): m/z = 480 (M+H)⁺ |
| 278 | 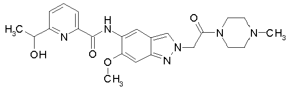 | Intermediat 6-17 und Kalium-6-(1-hydroxyethyl )pyridin-2-carboxylat (Intermediat 19-1) | (400MHz, DMSO-d6): δ = 1.52 (d, 3 H), 2.21 (s, 3 H), 2.26 - 2.32 (m, 2 H), 2.34 - 2.39 (m, 2 H), 3.41 - 3.50 (m, 2 H), 3.51 - 3.58 (m, 2 H), 4.00 (s, 3 H), 4.87 (dd, 1 H), 5.37 (s, 2 H), 5.59 (d, 1 H), 7.09 (s, 1 H), 7.80 (dd, 1 H), 7.98 - 8.12 (m, 2 H), 8.21 (s, 1 H), 8.68 (s, 1 H), 10.79 (s, 1 H). |
| | 6-(1-Hydroxyethyl)-N- {6-methoxy-2-[2-(4-methylpiperazin-1-yl)-2-oxoethyl]-2H-indazol-5-yl}pyridin-2-carboxamid | | |
| | | | UPLC-MS (Methode A1): Rt = 0.69 min |
| | | | MS (ESIpos): m/z = 453 (M+H)⁺ |
| 279 | 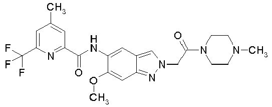 | Intermediat 6-17 und 4-Methyl-6-(trifluormeth yl)pyridin-2-carbonsäure (erhältlich von Anichem Inc. USA) | (300MHz, DMSO-d6): δ = 2.21 (s, 3 H), 2.25 - 2.33 (m, 2 H), 2.37 (br. s., 2 H), 2.58 (s, 3 H), 3.46 (br. s., 2 H), 3.54 (br. s., 2 H), 3.98 (s, 3 H), 5.38 (s, 2 H), 7.11 (s, 1 H), 8.08 (s, 1 H), 8.23 (s, 1 H), 8.30 (s, 1 H), 8.70 (s, 1 H), 10.52 (s, 1 H). |
| | N-{6-Methoxy-2-[2-(4-methylpiperazin-1-yl)-2-oxoethyl]-2H-indazol-5-yl}-4-methyl-6-(trifluormethyl)pyridin-2-carboxamid | | |
| | | | UPLC-MS (Methode A1): Rt = 0.99 min |
| | | | MS (ESIpos): m/z = 491 (M+H)⁺ |
| 280 | 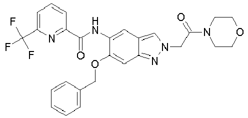 | Intermediat 6-21 und 2-[5-Amino-6-(benzyloxy)-2H-indazol-2-yl]-1-(morpholin-4-yl)ethanon (Intermediat 6-20) | (300MHz, DMSO-d6): δ = 3.43 - 3.53 (m, 2 H), 3.54 - 3.63 (m, 4 H), 3.63 - 3.69 (m, 2 H), 5.31 (s, 2 H), 5.41 (s, 2 H), 7.30 (s, 1 H), 7.41 (d, 3 H), 7.58 (d, 2 H), 8.18 (d, 1 H), 8.26 (s, 1 H), 8.35 - 8.44 (m, 1 H), 8.44 - 8.52 (m, 1 H), 8.81 (s, 1 H), 10.47 (s, 1 H). |
| | N-{6-(Benzyloxy)-2-[2-(morpholin-4-yl)-2-oxoethyl]-2H-indazol-5-yl}-6-(trifluormethyl)pyridin-2-carboxamid | | |
| | | | UPLC-MS (Methode A1): Rt = 1.26 min |
| | | | MS (ESIpos): m/z = 540 (M+H)⁺ |
| 281 | 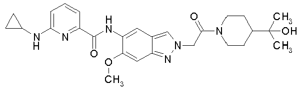 | Intermediat 6-5 und 6-(Cyclopropyl amino)pyridi n-2-carbonsäure | (400MHz, DMSO-d6): δ = 0.47 - 0.55 (m, 2 H), 0.76 - 0.86 (m, 2 H), 1.00 - 1.11 (m, 7 H), 1.16 - 1.31 (m, 1 H), 1.37 - 1.50 (m, 1 H), 1.75 (t, 2 H), 2.64 - 2.77 (m, 1 H), 2.99 (t, 1 H), 3.95 (s, 3 H), 4.03 (d, 1 H), 4.18 (s, 1 H), 4.42 (d, 1 H), 5.32 (d, 1 H), 5.38 (d, 1 H), 6.78 (d, 1 H), 7.06 (s, 1 H), 7.25 (d, 1 H), 7.36 (d, 1 H), 7.65 (dd, 1 H), 8.19 (s, 1 H), 8.71 (s, 1 H), 10.79 (s, 1 H). |
| | 6-(Cyclopropylamino)-N-(2-{2-[4-(2-hydroxypropan-2-yl)piperidin-1-yl]-2-oxoethyl}-6-methoxy-2H-indazol-5-yl)pyridin-2-carboxamid | | |
| | | | LC-MS (Methode A3): Rt = 1.10 min |
| | | | MS (ESIpos): m/z = 507 (M+H)⁺ |
| 282 | 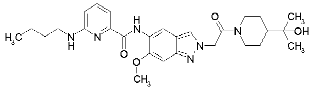 | Intermediat 6-5 und 6-(Butylamino) pyridin-2-carbonsäure | (300MHz, DMSO-d6): δ = 0.95 (t, 3 H), 1.04 (s, 7 H), 1.18 - 1.31 (m, 1 H), 1.37 - 1.51 (m, 3 H), 1.55 - 1.68 (m, 2 H), 1.68 - 1.81 (m, 2 H), 2.87 - 3.05 (m, 1 H), 3.36 - 3.47 (m, 2 H), 3.97 (s, 3 H), 4.05 (br. s., 1 H), 4.18 (s, 1 H), 4.41 (d, 1 H), 5.38 (d, 1 H), 5.32 (d, 1 H), 6.72 (d, 1 H), 7.02 (t, 1 H), 7.07 (s, 1 H), 7.26 (d, 1 H), 7.50 - 7.63 (m, 1 H), 8.19 (s, 1 H), 8.69 (s, 1 H), 10.81 (s, 1 H). |
| | 6-(Butylamino)-N-(2-{2-[4-(2-hydroxypropan-2-yl)piperidin-1-yl]-2-oxoethyl}-6-methoxy-2H-indazol-5-yl)pyridin-2-carboxamid | | |
| | | | LC-MS (Methode A3): Rt = 1.12 min |
| | | | MS (ESIpos): m/z = 523 (M+H)⁺ |
| 283 | 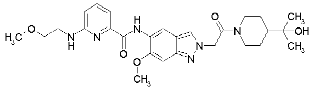 | Intermediat 6-5 und 6-[(2-Methoxyethy l)amino]pyri din-2-carbonsäure (hergestellt nach Synthesesche ma 8, erhältlich von Ukrorgsyntez Ltd., Bestellnumm er BBV-33897975) | (300MHz, DMSO-d6, ausgewählte Signale): δ = 1.04 (s, 7 H), 1.17 - 1.31 (m, 1 H), 1.35 - 1.51 (m, 1 H), 1.75 (t, 2 H), 2.99 (t, 1 H), 3.60 (s, 4 H), 3.94 - 4.09 (m, 1 H), 3.99 (s, 3 H), 4.18 (s, 1 H), 4.42 (d, 1 H), 5.38 (d, 1 H), 5.32 (d, 1 H), 6.77 (d, 1 H), 7.07 (s, 1 H), 7.14 (br. s., 1 H), 7.28 (d, 1 H), 7.52 - 7.64 (m, 1 H), 8.19 (s, 1 H), 8.68 (s, 1 H), 10.82 (s, 1 H). |
| | N-(2-{2-[4-(2-Hydroxypropan-2-yl)piperidin-1-yl]-2-oxoethyl}-6-methoxy-2H-indazol-5-yl)-6-[(2-methoxyethyl)amino]pyridin-2-carboxamid | | |
| | | | LC-MS (Methode A3): Rt = 0.90 min |
| | | | MS (ESIpos): m/z = 525 (M+H)⁺ |
| 284 | 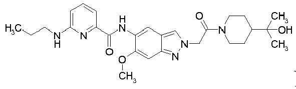 | Intermediat 6-5 und 6-(Propylamin o)pyridin-2-carbonsäure | (300MHz, DMSO-d6): δ = 1.04 (s, 6 H), 1.00 (t, 3 H), 1.12 (br. s., 1 H), 1.15 - 1.30 (m, 1 H), 1.34 - 1.51 (m, 1 H), 1.58 - 1.83 (m, 2 H), 1.67 (sxt, 2 H), 2.91 - 3.07 (m, 1 H), 3.35 - 3.42 (m, 2 H), 3.98 (s, 4 H), 4.05 (br. s., 1 H), 4.18 (s, 1 H), 4.42 (d, 1 H), 5.35 (d, 2 H), 6.72 (d, 1 H), 7.02 - 7.10 (m, 2 H), 7.26 (d, 1 H), 7.57 (dd, 1 H), 8.19 (s, 1 H), 8.69 (s, 1 H), 10.82 (s, 1 H). |
| | N-(2- {2-[4-(2-Hydroxypropan-2-yl)piperidin-1-yl]-2-oxoethyl}-6-methoxy-2H-indazol-5-yl)-6-(propylamino)pyridin-2-carboxamid | | |
| | | | UPLC-MS (Methode A1): Rt = 1.09 min |
| | | | MS (ESIpos): m/z = 509 (M+H)⁺ |
| 285 | 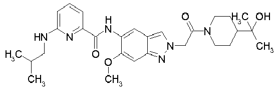 | Intermediat 6-5 und 6-(Isobutylami no)pyridin-2-carbonsäure | (300MHz, DMSO-d6): δ = 0.99 (s, 3 H), 1.01 (s, 3 H), 1.03 - 1.08 (m, 7 H), 1.19 - 1.28 (m, 1 H), 1.38 - 1.50 (m, 1 H), 1.75 (t, 2 H), 1.94 (dt, 1 H), 2.99 (br. s., 1 H), 3.25 (t, 2 H), 3.99 (s, 3 H), 4.03 (d, 1 H), 4.16 (s, 1 H), 4.42 (d, 1 H), 5.38 (d, 1 H), 5.32 (d, 1 H), 6.75 (dd, 1 H), 7.07 (s, 1 H), 7.05 (t, 1 H), 7.23 - 7.29 (m, 1 H), 7.57 (dd, 1 H), 8.15 - 8.20 (m, 1 H), 8.70 (s, 1 H), 10.75 (s, 1 H). |
| | N-(2- {2-[4-(2-Hydroxypropan-2-yl)piperidin-1-yl]-2-oxoethyl}-6-methoxy-2H-indazol-5-yl)-6-(isobutylamino)pyridin-2 - carboxamid | | |
| | | | UPLC-MS (Methode A1): Rt = 1.16 min |
| | | | MS (ESIpos): m/z = 523 (M+H)⁺ |
| 286 | 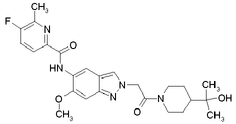 | Intermediat 6-5 und 5-Fluor-6-methylpyridi n-2-carbonsäure | (300MHz, DMSO-d6): δ = 1.04 (s, 6 H), 1.07 - 1.15 (m, 1 H), 1.17 - 1.35 (m, 1 H), 1.36 - 1.52 (m, 1 H), 1.75 (t, 2 H), 2.59 (d, 3 H), 2.99 (t, 1 H), 3.96 - 4.08 (m, 1 H), 4.00 (s, 3 H), 4.19 (s, 1 H), 4.41 (d, 1 H), 5.33 (d, 1 H), 5.39 (d, 1 H), 7.09 (s, 1 H), 7.90 (t, 1 H), 8.09 (dd, 1 H), 8.21 (s, 1 H), 8.68 (s, 1 H), 10.52 (s, 1 H). |
| | 5-Fluor-N-(2-{2-[4-(2-hydroxypropan-2-yl)piperidin-1-yl]-2-oxoethyl}-6-methoxy-2H-indazol-5-yl)-6-methylpyridin-2-carboxamid | | |

| | | | |
|---|---|---|---|
| * Nach der Reaktion von Intermediat 6-16 und Kalium-6-(2,2,2-trifluor-1-hydroxyethyl)pyridin-2-carboxylat erhielt man N-{6-Methoxy-2-[2-(morpholin-4-yl)-2-oxoethyl]-2H-indazol-5-yl}-6-(2,2,2-trifluor-1-hydroxyethyl)pyridin-2-carboxamid als racemisches Gemisch. Diese Mischung wurde durch eine präparative chirale HPLC mit den folgenden Bedingungen in die reinen Enantiomere aufgetrennt: | | | |

| | | | | |
|---|---|---|---|---|
| *System:* | Agilent: Prep 1200, 2xPrep Pump, DLA, MWD, Gilson: Liquid Handler 215 | | | |
| *Säule:* | Chiralpak IC 5µm 250x30 mm | | | |
| *Solvent:* | Ethanol / Methanol 50:50 (v/v) | | | |
| *Fluss:* | 35 mL/min | | | |
| *Temperatur:* | Raumtemperatur | | | |
| *Lösung:* | 401 mg / 8 mL Dichlormethan/MeOH | | | |
| *Injektion:* | 10 x 0.8 mL | | | |

| | | | | |
|---|---|---|---|---|
| *Detektion:* | UV 280 nm | | | |
| **Fraktion** | Rt in min | Reinheit in % | Menge in mg | Peakzuordnung |
| **entspricht Beispiel 273** | 8.0 - 8.7 | 98.8 | 70 | Peak 2 - 2.88 min |
| **entspricht Beispiel 272** | 10.1 - 11.1 | 99.1 | 59 | Peak 4 - 3.81 min |
| *Aufarbeitung:* | Die Fraktionen wurden eingedampft, mit tBuOH versetzt, bei -65°C tiefgekühlt und abschließend gefriergetrocknet. | | | |

### Beispiel 287

### N-{6-Hydroxy-2-[2-(morpholin-4-yl)-2-oxoethyl]-2H-indazol-5-yl}-6-(trifluormethyl)pyridin-2-carboxamid

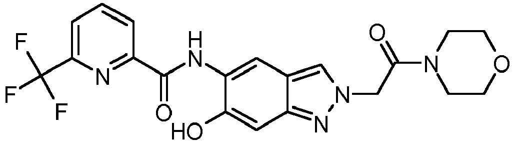

2.43 g (4.50 mmol) N-{6-(Benzyloxy)-2-[2-(morpholin-4-yl)-2-oxoethyl]-2H-indazol-5-yl}-6-(trifluormethyl)pyridin-2-carboxamid (Beispiel 280) wurden in 470 ml Tetrahydrofuran suspendiert und der Kolben evakuiert und anschließend mit Stickstoff gespült (Vorgang noch zwei Mal wiederholt). 958 mg (0.9 mmol, 10%) Palladium auf Kohle und 95 ml (370.6 mmol) einer 25%igen Ammoniumformiatlösung wurden zugegeben und es wurde für 40 Minuten bei 25°C kräftig gerührt. Die Reaktionsmischung wurde über Celite filtriert und eingeengt. Der entstandene Niederschlag wurde abgesagt und mehrmals mit Wasser gewaschen und im Trockenschrank bei 50°C im Vakuum getrocknet. Man erhielt 2.01 g (90 % d. Th.) der Titelverbindung.
UPLC-MS (Methode A2): Rt = 0.64 min
MS (ESIpos): m/z = 450 (M+H)⁺
¹H NMR (400 MHz, DMSO-d6): δ = 3.40 - 3.50 (m, 2 H), 3.52 - 3.61 (m, 4 H), 3.61 - 3.67 (m, 2 H), 5.36 (s, 2 H), 6.91 (s, 1 H), 8.16 - 8.23 (m, 2 H), 8.40 (t, 1 H), 8.47 (d, 1 H), 8.69 (s, 1 H), 10.55 (s, 1 H), 10.65 (s, 1 H).

### Allgemeine Versuchsvorschrift 3a

1.0 Äquivalente N-{6-Hydroxy-2-[2-(morpholin-4-yl)-2-oxoethyl]-2H-indazol-5-yl}-6-(trifluormethyl)pyridin-2-carboxamid wurden mit 1.5 Äquivalenten des entsprechenden Halogenids und 3.0 Äquivalenten Kaliumcarbonat in *N,N*-Dimethylformamid für 1 h bei 100°C in der Mikrowelle gerührt. Die Reaktionsmischung wurde mit Wasser versetzt und der entstandene Niederschlag wurde abfiltriert, mit Wasser und Diethylether gewaschen und getrocknet.

### Allgemeine Versuchsvorschrift 3b

1.0 Äquivalente N-{6-Hydroxy-2-[2-(morpholin-4-yl)-2-oxoethyl]-2H-indazol-5-yl}-6-(trifluormethyl)pyridin-2-carboxamid wurden mit 1.5 Äquivalenten des entsprechenden Halogenids und 3.0 Äquivalenten Kaliumcarbonat in *N,N*-Dimethylformamid für 1 h bei 100°C in der Mikrowelle gerührt. Die Reaktionsmischung wurde filtriert, mit Dimethylsulfoxid versetzt und per präparativer HPLC nach der Methode P1 gereinigt.

### Allgemeine Versuchsvorschrift 3c

1.0 Äquivalente N-{6-Hydroxy-2-[2-(morpholin-4-yl)-2-oxoethyl]-2H-indazol-5-yl}-6-(trifluormethyl)pyridin-2-carboxamid wurden mit 3.0 Äquivalenten des entsprechenden Halogenids, 5.0 Äquivalenten Kaliumcarbonat und 0.1 Äquivalenten Kaliumiodid in *N,N-*Dimethylformamid für 1 h bei 150°C in der Mikrowelle gerührt. Die Reaktionsmischung wurde filtriert, mit Dimethylsulfoxid versetzt und per präparativer HPLC nach der Methode P5 (Gradient: 0 - 15 min 10 - 50% B) gereinigt.

**Tabelle 20: Beispiele 304 - 328**

| Die Beispielverbindungen wurden aus N-{6-Hydroxy-2-[2-(morpholin-4-yl)-2-oxoethyl]-2H-indazol-5-yl}-6-(trifluormethyl)pyridin-2-carboxamid und den in der Tabelle angegebenen Halogeniden nach den allgemeinen Versuchsvorschriften 3a, 3b oder 3c hergestellt. | | | | |
|---|---|---|---|---|
| Bsp Nr. | Struktur/Name | Hergestellt durch Umsetzung mit | * Siehe Lege nde | ¹H-NMR / LC-MS |
| 288 | 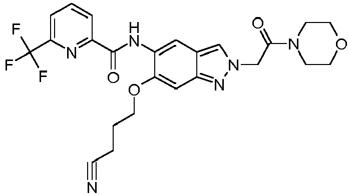 | 4-Brombutanni tril | 3a (87% ) | (400 MHz, DMSO-d6): δ = 2.21 (quin, 2 H), 2.77 (t, 2 H), 3.43 - 3.51 (m, 2 H), 3.53 - 3.62 (m, 4 H), 3.63 - 3.69 (m, 2 H), 4.25 (t, 2 H), 5.40 (s, 2 H), 7.13 (s, 1 H), |
| | N-{6-(3-Cyanpropoxy)-2-[2-(morpholin-4-yl)-2-oxoethyl]-2H-indazol-5-yl}-6-(trifluormethyl)pyridin-2-carboxamid | | | 8.22 (dd, 1 H), 8.24 - 8.26 (m, 1 H), 8.37 - 8.45 (m, 1 H), 8.45 - 8.50 (m, 1 H), 8.74 (s, 1 H), 10.55 (s, 1 H). |
| | | | | UPLC-MS (Methode A1): Rt = 1.05 min |
| | | | | MS (ESIpos): m/z = 517 (M+H)+ |
| 289 | 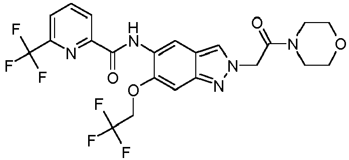 | 1,1,1-Trifluor-2-iodethan | 3b^{[b]} (21% ) | (300 MHz, DMSO-d6): δ = 3.43 - 3.50 (m, 2 H), 3.53 - 3.63 (m, 5 H), 3.63 - 3.69 (m, 2 H), 5.01 (d, 1 H), 4.95 (d, 1 H), 5.43 (s, 2 H), |
| | N-{2-[2-(Morpholin-4-yl)-2-oxoethyl]-6-(2,2,2-trifluorethoxy)-2H-indazol-5-yl}-6-(trifluormethyl)pyridin-2-carboxamid | | | 7.30 (s, 1 H), 8.21 (d, 1 H), 8.30 (s, 1 H), 8.41 (t, 1 H), 8.45 - 8.51 (m, 1 H), 8.81 (s, 1 H), 10.53 (s, 1 H). |
| | | | | UPLC-MS (Methode A1): Rt = 1.16 min |
| | | | | MS (ESIpos): m/z = 532 (M+H)+ |
| 290 | 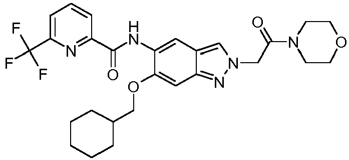 | (Brommethyl )cyclohexan | 3b (70% ) | (300 MHz, DMSO-d6): δ = 1.07 - 1.37 (m, 6 H), 1.64 - 1.81 (m, 2 H), 1.86 - 2.02 (m, 3 H), 3.42 - 3.50 (m, 2 H), 3.53 - 3.69 (m, 6 H), 3.97 (d, 2 H), 5.39 (s, 2 H), 7.08 (s, 1 H), 8.18 - 8.25 (m, 2 H), 8.40 (t, 1 H), 8.48 (d, 1 H), 8.79 (s, 1 H), 10.48 (s, 1 H). |
| | N-{6-(Cyclohexylmethoxy)-2-[2-(morpholin-4-yl)-2-oxoethyl]-2H-indazol-5-yl}-6-(trifluormethyl)pyridin-2-carboxamid | | | |
| | | | | LC-MS (Methode A3): Rt = 1.40 min |
| | | | | MS (ESIpos): m/z = 546 (M+H)+ |
| 291 | 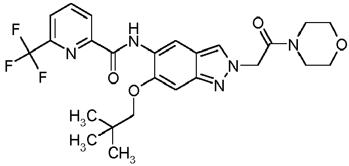 | 1-Iod-2,2-dimethylprop an | 3b (22% ) | (300 MHz, DMSO-d6): δ = 1.03 - 1.21 (m, 9 H), 3.39 - 3.53 (m, 2 H), 3.53 - 3.73 (m, 6 H), 3.84 (s, 2 H), 5.40 (s, 2 H), 7.07 (s, 1 H), 8.18 - 8.26 (m, 2 H), 8.41 (t, 1 H), 8.51 (d, 1 H), 8.82 (s, 1 H), 10.47 (s, 1 H). |
| | N-{6-(2,2-Dimethylpropoxy)-2-[2-(morpholin-4-yl)-2-oxoethyl]-2H-indazol-5-yl}-6-(trifluormethyl)pyridin-2-carboxamid | | | |
| | | | | LC-MS (Methode A3): Rt = 1.30 min |
| | | | | MS (ESIpos): m/z = 520 (M+H)+ |
| 292 | 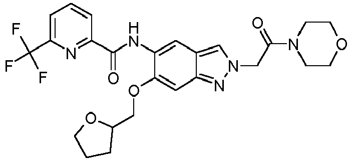 | 2-(Brommethyl )tetrahydrofu ran | 3b (41% ) | (300 MHz, DMSO-d6): δ = 1.70 - 2.00 (m, 3 H), 2.05 - 2.20 (m, 1 H), 3.47 (d, 2 H), 3.53 - 3.68 (m, 7 H), 3.68 - 3.77 (m, 1 H), 3.77 - 3.89 (m, 1 H), 4.07 - 4.22 (m, 2 H), 4.22 - 4.34 (m, 1 H), 5.40 (s, 2 H), 7.14 (s, 1 H), 8.17 - 8.29 (m, 2 H), 8.41 (t, 1 H), 8.48 (d, 1 H), 8.75 (s, 1 H), 10.51 (s, 1 H). |
| | N-{2-[2-(Morpholin-4-yl)-2-oxoethyl]-6-(tetrahydrofuran-2-ylmethoxy)-2H-indazol-5-yl}-6-(trifluormethyl)pyridin-2-carboxamid | | | |
| | | | | LC-MS (Methode A3): Rt = 1.07 min |
| | | | | MS (ESIpos): m/z = 534 (M+H)+ |
| 293 | 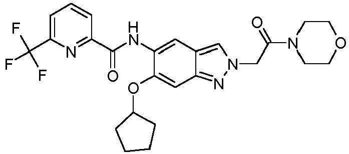 | Iodcyclopent an | 3b^{[c]} (23% ) | (300 MHz, DMSO-d6): δ = 1.67 (dd, 2 H), 1.80 (dd, 2 H), 1.86 - 1.96 (m, 2 H), 1.97 - 2.10 (m, 2 H), 3.47 (d, 2 H), 3.54 - 3.62 (m, 4 H), 3.65 (d, 2 H), 5.07 (t, 1 H), 5.38 (s, 2 H), 7.07 (s, 1 H), 8.18 - 8.24 (m, 2 H), 8.40 (t, 1 H), 8.47 (d, 1 H), 8.75 (s, 1 H), 10.62 (s, 1 H). |
| | N-{6-(Cyclopentyloxy)-2-[2-(morpholin-4-yl)-2-oxoethyl]-2H-indazol-5-yl}-6-(trifluormethyl)pyridin-2-carboxamid | | | |
| | | | | UPLC-MS (Methode A2): Rt = 1.29 min |
| | | | | MS (ESIpos): m/z = 518 (M+H)+ |
| 294 | 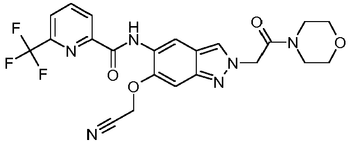 | Bromacetonit ril | 3b^{[d]} (10% ) | (300 MHz, DMSO-d6): δ = 3.47 (d, 2 H), 3.55 - 3.62 (m, 4 H), 3.65 (d, 2 H), 5.39 (s, 2 H), 5.44 (s, 2 H), 7.31 (s, 1 H), 8.23 (dd, 1 H), 8.30 (s, 1 H), 8.42 (d, 1 H), 8.46 (s, 1 H), 8.73 (s, 1 H), 10.41 (s, 1 H). |
| | N-{6-(Cyanmethoxy)-2-[2-(morpholin-4-yl)-2-oxoethyl]-2H-indazol-5-yl}-6-(trifluormethyl)pyridin-2-carboxamid | | | |
| | | | | UPLC-MS (Methode A2): Rt = 1.01 min |
| | | | | MS (ESIpos): m/z = 489 (M+H)+ |
| 295 | 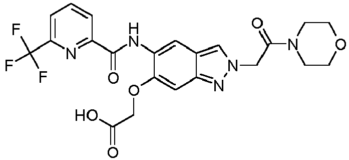 | Bromessigsä ure | 3c (9%) | (300 MHz, DMSO-d6): δ = 3.47 (d, 2 H), 3.52 - 3.71 (m, 6 H), 4.90 (s, 2 H), 5.41 (s, 2 H), 7.12 (s, 1 H), 8.16 - 8.29 (m, 2 H), 8.40 (t, 1 H), 8.48 (d, 1 H), 8.77 (s, 1 H), 10.58 (s, 1 H), 13.20 (br. s., 1 H). |
| | ({2-[2-(Morpholin-4-yl)-2-oxoethyl]-5-({[6-(trifluormethyl)pyridin-2-yl]carbonyl}amino)-2H-indazol-6-yl}oxy)essigsäure | | | |
| | | | | UPLC-MS (Methode A2): Rt = 0.61 min |
| | | | | MS (ESIpos): m/z = 508 (M+H)+ |
| 296 | 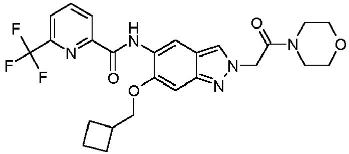 | (Brommethyl )cyclobutan | 3a (59% ) | (300 MHz, DMSO-d6): δ = 1.82 - 2.04 (m, 4 H), 2.09 - 2.24 (m, 2 H), 2.78 - 2.95 (m, 1 H), 3.47 (d, 2 H), 3.52 - 3.73 (m, 6 H), 4.15 (d, 2 H), 5.40 (s, 2 H), 7.10 (s, 1 H), 8.16 - 8.28 (m, 2 H), 8.40 (t, 1 H), 8.48 (d, 1 H), 8.76 (s, 1 H), 10.52 (s, 1 H). |
| | N-{6-(Cyclobutylmethoxy)-2-[2-(morpholin-4-yl)-2-oxoethyl]-2H-indazol-5-yl}-6-(trifluormethyl)pyridin-2-carboxamid | | | |
| | | | | UPLC-MS (Methode A1): Rt = 1.32 min |
| | | | | MS (ESIpos): m/z = 518 (M+H)+ |
| 297 | 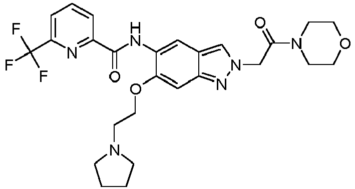 | 1-(2-Chlorethyl)p yrrolidinhydr ochlorid | 3a (54% ) | (400 MHz, DMSO-d6): δ = 1.65 (dt, 4 H), 2.57 (br. s., 4 H), 2.97 (t, 2 H), 3.42 - 3.50 (m, 2 H), 3.54 - 3.61 (m, 4 H), 3.61 - 3.68 (m, 2 H), 4.28 (t, 2 H), 5.39 (s, 2 H), 7.13 (s, 1 H), 8.19 - 8.25 (m, 2 H), 8.41 (t, 1 H), 8.48 (d, 1 H), 8.75 (s, 1 H), 10.58 (s, 1 H). |
| | N-{2-[2-(Morpholin-4-yl)-2-oxoethyl]-6-[2-(pyrrolidin-1-yl)ethoxy]-2H-indazol-5-yl}-6-(trifluormethyl)pyridin-2-carboxamid | | | |
| | | | | UPLC-MS (Methode A1): Rt = 0.77 min |
| | | | | MS (ESIpos): m/z = 547 (M+H)+ |
| 298 | 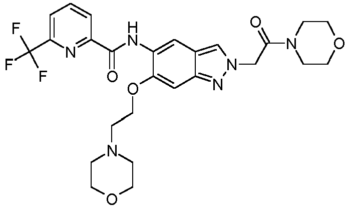 | 4-(2-Chlorethyl)m orpholinhydr ochlorid | 3a (64% ) | (400 MHz, DMSO-d6, ausgewählte Signale): δ = 2.84 - 2.93 (m, 2 H), 3.43 - 3.51 (m, 2 H), 3.51 - 3.62 (m, 8 H), 3.62 - 3.69 (m, 2 H), 4.31 (t, 2 H), 5.40 (s, 2 H), 7.16 (s, 1 H), 8.18 - 8.26 (m, 2 H), 8.41 (t, 1 H), 8.48 (d, 1 H), 8.74 (s, 1 H), 10.57 (s, 1 H). |
| | N-{6-[2-(Morpholin-4-yl)ethoxy]-2-[2-(morpholin-4-yl)-2-oxoethyl]-2H-indazol-5-yl}-6-(trifluormethy l)pyridin- 2-carboxamid | | | |
| | | | | UPLC-MS (Methode A1): Rt = 0.75 min |
| | | | | MS (ESIpos): m/z = 563 (M+H)+ |
| 299 | 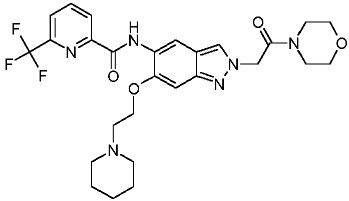 | 1-(2-Chlorethyl)pi peridinhydro chlorid | 3a (72% ) | (300 MHz, DMSO-d6, ausgewählte Signale): δ = 1.29 - 1.40 (m, 2 H), 1.40 - 1.53 (m, 4 H), 2.73 (s, 1 H), 2.84 (t, 2 H), 3.42 - 3.51 (m, 2 H), 3.58 (br. s., 4 H), 3.62 - 3.68 (m, 2 H), 4.27 (t, 2 H), 5.40 (s, 2 H), 7.14 (s, 1 H), 8.19 - 8.25 (m, 2 H), 8.41 (t, 1 H), 8.48 (d, 1 H), 8.74 (s, 1 H), 10.58 (s, 1H). |
| | N-{2-[2-(Morpholin-4-yl)-2-oxoethyl]-6-[2-(piperidin-1-yl)ethoxy]-2H-indazol-5-yl}-6-(trifluormethyl)pyridin-2-carboxamid | | | |
| | | | | UPLC-MS (Methode A1): Rt = 0.83 min |
| | | | | MS (ESIpos): m/z = 561 (M+H)+ |
| 300 | 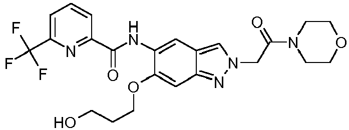 | (3-Brompropox y)(tert-butyl)dimeth ylsilan | 3a (72% ) [f] (49% ) | (300 MHz, DMSO-d6): δ = 1.93 - 2.08 (m, 2 H), 3.43 - 3.52 (m, 2 H), 3.52 - 3.77 (m, 8 H), 4.24 (t, 2 H), 4.59 (t, 1 H), 5.40 (s, 2 H), 7.10 (s, 1 H), 8.17 - 8.25 (m, 2 H), 8.36 - 8.44 (m, 1 H), 8.45 - 8.50 (m, 1 H), 8.75 (s, 1 H), 10.64 (s, 1 H). |
| | N-{6-(3-Hydroxypropoxy)-2-[2-(morpholin-4-yl)-2-oxoethyl]-2H-indazol-5-yl}-6-(trifluormethyl)pyridin-2-carboxamid | | | |
| | | | | UPLC-MS (Methode A1): Rt = 0.97 min |
| | | | | MS (ESIpos): m/z = 508 (M+H)+ |
| 301 | 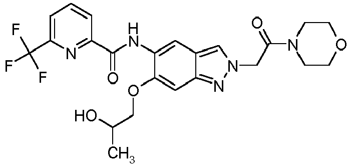 | [(1-Brompropan-2-yl)oxy] (tert-butyl)dimeth ylsilan | 3a (66% ) [f] (46% ) | (300 MHz, DMSO-d6): δ = 1.32 (d, 3 H), 3.42 - 3.51 (m, 2 H), 3.51 - 3.61 (m, 4 H), 3.61 - 3.69 (m, 2 H), 3.86 - 3.95 (m, 1 H), 4.05 - 4.16 (m, 2 H), 4.96 (d, 1 H), 5.40 (s, 2 H), 7.09 (s, 1 H), 8.18 - 8.25 (m, 2 H), 8.35 - 8.44 (m, 1 H), 8.44 - 8.52 (m, 1 H), 8.76 (s, 1 H), 10.54 (s, 1 H). |
| | N-{6-(2-Hydroxypropoxy)-2-[2-(morpholin-4-yl)-2-oxoethyl]-2H-indazol-5-yl}-6-(trifluormethyl)pyridin-2-carboxamid | | | |
| | | | | UPLC-MS (Methode A2): Rt = 0.98 min |
| | | | | MS (ESIpos): m/z = 508 (M+H)+ |
| 302 | 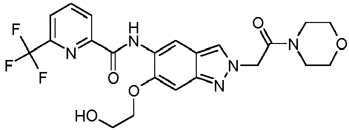 | tert-Butyl(2-iodethoxy)di methylsilan | 3a (52% ) [f] (77% ) | (300 MHz, DMSO-d6): δ = 3.42 - 3.51 (m, 2 H), 3.58 (br. s., 4 H), 3.62 - 3.68 (m, 2 H), 3.89 (q, 2 H), 4.20 (t, 2 H), 4.89 (t, 1 H), 5.40 (s, 2 H), 7.12 (s, 1 H), 8.21 (dd, 1 H), 8.24 (s, 1 H), 8.35 - 8.44 (m, 1 H), 8.44 - 8.49 (m, 1 H), 8.73 (s, 1 H), 10.66 (s, 1 H). |
| | N-{6-(2-Hydroxyethoxy)-2-[2-(morpholin-4-yl)-2-oxoethyl]-2H-indazol-5-yl}-6-(trifluormethyl)pyridin-2-carboxamid | | | |
| | | | | LC-MS (Methode A3): Rt = 0.84 min |
| | | | | MS (ESIpos): m/z = 494 (M+H)+ |
| 303 | 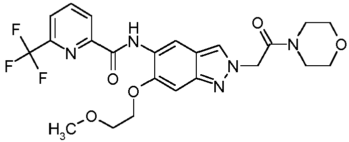 | 1-Brom-2-methoxyetha n | 3b^{[h]} (27% ) | (300 MHz, DMSO-d6): δ = 3.36 (s, 3 H), 3.42 - 3.51 (m, 2 H), 3.54 - 3.62 (m, 4 H), 3.62 - 3.69 (m, 2 H), 3.82 (dd, 2 H), 4.30 (dd, 2 H), 5.40 (s, 2 H), 7.13 (s, 1 H), 8.20 - 8.25 (m, 2 H), 8.37 - 8.44 (m, 1 H), 8.45 - 8.50 (m, 1 H), 8.77 (s, 1 H), 10.58 (s, 1 H). |
| | N-{6-(2-Methoxyethoxy)-2-[2-(morpholin-4-yl)-2-oxoethyl]-2H-indazol-5-yl}-6-(trifluormethyl)pyridin-2-carboxamid | | | |
| | | | | LC-MS (Methode A3): Rt = 1.01 min |
| | | | | MS (ESIpos): m/z = 508 (M+H)+ |
| 304 | 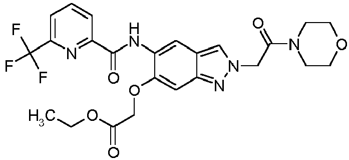 | Ethylbromacetat | 3b^{[h]} (59% ) | (300 MHz, DMSO-d6): δ = 1.21 (t, 3 H), 3.42 - 3.50 (m, 2 H), 3.52 - 3.62 (m, 4 H), 3.63 - 3.68 (m, 2 H), 4.20 (q, 2 H), 5.02 (s, 2 H), 5.41 (s, 2 H), 8.22 (dd, 1 H), 8.26 (s, 1 H), 8.36 - 8.45 (m, 1 H), 8.46 - 8.51 (m, 1 H), 8.76 (s, 1 H), 10.56 (s, 1 H). |
| | Ethyl-( {2-[2-(morpholin-4-yl)-2-oxoethyl]-5-( {[6-(trifluormethy l)pyridin- 2-yl]carbonyl} amino)-2H-indazol-6-yl} oxy)acetat | | | |
| | | | | LC-MS (Methode A3): Rt = 1.05 min |
| | | | | MS (ESIpos): m/z = 536 (M+H)+ |
| 305 | 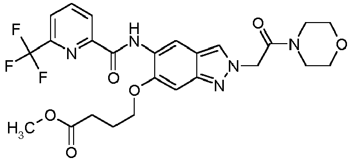 | Methyl-4-brombutanoa t | 3b^{[h]} (46% ) | (300 MHz, DMSO-d6): δ = 2.07 - 2.23 (m, 2 H), 2.62 (t, 2 H), 3.40 - 3.51 (m, 2 H), 3.59 (s, 3 H), 3.53 - 3.63 (m, 4 H), 3.65 (br. s., 2 H), 4.19 (t, 2 H), 5.40 (s, 2 H), 7.08 (s, 1 H), 8.21 (dd, 1 H), 8.23 (s, 1 H), 8.34 - 8.52 (m, 1 H), 8.40 (t, 1 H), 8.74 (s, 1 H), 10.58 (s, 1 H). |
| | Methyl-4-( {2- [2-(morpholin-4-yl)-2-oxoethyl]-5-( {[6-(trifluormethy l)pyridin- 2-yl]carbonyl} amino)-2H-indazol-6-yl}oxy)butanoat | | | |
| | | | | LC-MS (Methode A3): Rt = 1.05 min |
| | | | | MS (ESIpos): m/z = 550 (M+H)+ |
| 306 | 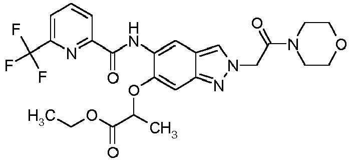 | Ethyl-2-brompropano at | 3b^{[h]} (46% ) | (300 MHz, DMSO-d6): δ = 1.13 (t, 3 H), 1.68 (d, 3 H), 3.43 - 3.52 (m, 2 H), 3.53 - 3.62 (m, 4 H), 3.62 - 3.69 (m, 2 H), 4.13 (q, 2 H), 5.28 (q, 1 H), 5.40 (s, 2 H), 7.07 (s, 1 H), 8.22 (dd, 1 H), 8.26 (s, 1 H), 8.38 - 8.44 (m, 1 H), 8.46 - 8.51 (m, 1 H), 8.75 (s, 1 H), 10.81 (s, 1 H). |
| | Ethyl-2-( {2-[2-(morpholin-4-yl)-2-oxoethyl]-5-( {[6-(trifluormethy l)pyridin- 2-yl]carbonyl} amino)-2H-indazol-6-yl} oxy)propanoat | | | |
| | | | | LC-MS (Methode A3): Rt = 1.12 min |
| | | | | MS (ESIpos): m/z = 550 (M+H)+ |
| 307 | 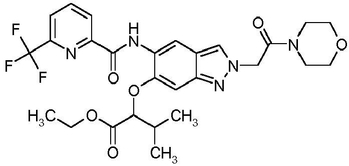 | Ethyl-2-brompropano at | 3b^{[h]} (51% ) | (300 MHz, DMSO-d6): δ = 1.09 - 1.18 (m, 9 H), 2.38 (d, 1 H), 3.43 - 3.49 (m, 2 H), 3.53 - 3.62 (m, 4 H), 3.62 - 3.67 (m, 2 H), 4.16 (q, 2 H), 4.99 (d, 1 H), 5.40 (s, 2 H), 6.99 (s, 1 H), 8.22 (dd, 1 H), 8.26 (s, 1 H), 8.42 (t, 1 H), 8.50 - 8.54 (m, 1 H), 8.82 (s, 1 H), 10.53 - 10.56 (m, 1 H). |
| | Ethyl-3-methyl-2-({2-[2-(morpholin-4-yl)-2-oxoethyl]-5-({[6-(trifluormethyl)pyridin-2-yl]carbonyl}amino)-2H-indazol-6-yl}oxy)butanoat | | | |
| | | | | LC-MS (Methode A3): Rt = 1.24 min |
| | | | | MS (ESIpos): m/z = 578 (M+H)+ |

| | | | | |
|---|---|---|---|---|
| * Hergestellt nach angegebener Vorschrift, in Klammern ist die Ausbeute in % angegeben [a]: Nach 60 Minuten wurde nochmals 1 Äquivalent des Halogenids hinzugegeben und für weitere 60 Minuten bei 120°C in der Mikorwelle gerührt. [b]: Das Rohprodukt wurde per präparativer HPLCgereinigt. [c]: Das Rohprodukt wurde per präparativer HPLC nach der Methode P5 (Gradient: 0 - 15 min 30 - 70% B) gereinigt. [d]: Die Reaktionsmischung wurde auf Wasser gegeben, der Niederschlag abgesaugt und mit Diethylether gewaschen. Das Rohprodukt wurde per präparativer HPLC nach der Methode P5 (Gradient: 0 - 15 min 15 - 55% B) gereinigt. [e]: Das Rohprodukt wurde per präparativer HPLC nach der Methode P5 (Gradient: 0 - 15 min 15 - 55% B) gereinigt. [f]: Folgende Vorschrift wurde für die Entschützung des alkylierten Intermediate verwendet (die in der Tabelle angegebene 2. Ausbeute in % bezieht sich auf die Entschützung): 1 Äquivalent des silylgeschützten Intermediates wurde in Tetrahydrofuran gelöst, mit 3 Äquivalenten einer 1 M Lösung von Tetrabutylammoniumfluorid in Tetrahydrofuran versetzt und für 24 h bei 25°C gerührt. Die Reaktionsmischung wurde mit Wasser versetzt, der entstandene Niederschlag abgesaugt, mit Wasser gewaschen und im Trockenschrank bei 50°C im Vakuum getrocknet. [g]: Es wurden noch 0.1 Äquivalente Kaliumiodid zu der Reaktionsmischung gegeben. [h]: Das Rohprodukt wurde per präparativer HPLC (Säule: XBridge C18 5µm 100x30 mm) gereinigt. | | | | |

### Beispiel 308

### 2-({2-[2-(Morpholin-4-yl)-2-oxoethyl]-5-({[6-(trifluormethyl)pyridin-2-yl]carbonyl}amino)-2H-indazol-6-yl} oxy)propansäure

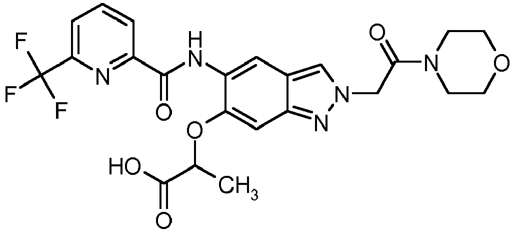

Analog zu Intermediat 4-1 wurden 50 mg (0.09 mmol) Ethyl-2-({2-[2-(morpholin-4-yl)-2-oxoethyl]-5-({[6-(trifluormethyl)pyridin-2-yl]carbonyl}amino)-2H-indazol-6-yl}oxy)propanoat (Beispiel 306) in 0.5 ml Tetrahydrofuran gelöst und mit einer Lösung von 11 mg (0.45 mmol) Lithiumhydroxid Monohydrat in 164 µl Wasser versetzt und 24 h bei 25°C gerührt. Die Reaktionsmischung wurde filtriert, mit Dimethylsulfoxid versetzt und per präparativer HPLC (Säule: XBridge C18 5µm 100x30 mm) gereinigt. Man erhielt 7 mg (15 % d.Th.) der Titelverbindung.
UPLC-MS (Methode A2): Rt = 0.67 min
MS (ESIpos): m/z = 522 (M+H)⁺
¹H NMR (400 MHz, DMSO-d6): δ = 1.64 (d, 3 H), 3.46 (br. s., 2 H), 3.58 (br. s., 4 H), 3.64 (d, 2 H), 4.99 (d, 1 H), 5.38 (s, 2 H), 6.97 (s, 1 H), 8.15 - 8.25 (m, 2 H), 8.35 - 8.45 (m, 1 H), 8.45 - 8.51 (m, 1 H), 8.73 (s, 1 H), 10.82 (s, 1 H).

### Beispiel 309

### N-{6-(2-Hydroxypropan-2-yl)-2-[2-(morpholin-4-yl)-2-oxoethyl]-2H-indazol-5-yl}-6-(trifluormethyl)pyridin-2-carboxamid

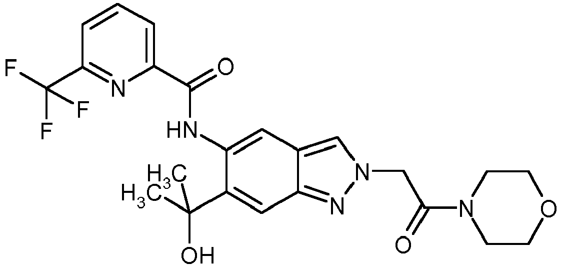

Zu einer Mischung aus 250 mg (0.69 mmol) N-[6-(2-Hydroxypropan-2-yl)-1H-indazol-5-yl]-6-(trifluormethyl)pyridin-2-carboxamid (Intermediat 14-11) und 0.59 ml N,N-Dicyclohexylmethylamin in 1.5 ml THF wurden 536 mg (4 Äquiv.) 2-Brom-1-(morpholin-4-yl)ethanon gegeben und man rührte die Mischung bei 70°C über Nacht. Man versetzte mit Wasser, extrahierte mit Ethylacetat, wusch mit gesättigter wässriger Kochsalzlösung, filtrierte über einen wasserabweisenden Filter und engte ein. Der Rückstand wurde säulenchromatographisch an Kieselgel gereinigt (Dichlormethan / Methanol). Man erhielt 46 mg (14% der Theorie) der Titelverbindung.
¹H-NMR (400MHz, DMSO-d₆): δ [ppm]= 1.62 (s, 6H), 3.46 (d, 3H), 3.42 - 3.69 (m), 5.45 (s, 2H), 5.95 (s, 1H), 7.54 (s, 1H), 8.15 (dd, 1H), 8.25 (s, 1H), 8.36 (t, 1H), 8.45 (d, 1H), 8.73 (s, 1H), 12.35 (s, 1H).
UPLC-MS (Methode A2): Rt = 0.99 min (UV-TIC), gefundene Masse 491.00.

### Beispiel 310

### N-{6-Chlor-2-[2-(4-methylpiperazin-1-yl)-2-oxoethyl]-2H-indazol-5-yl}-6-(difluormethyl)pyridin-2-carboxamid

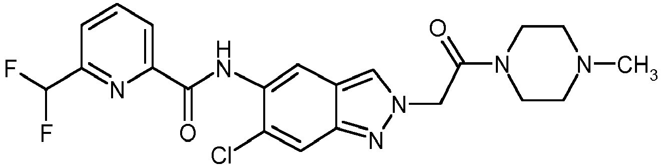

98 mg (0.32 mmol) 2-(5-Amino-6-chlor-2H-indazol-2-yl)-1-(4-methylpiperazin-1-yl)ethanon (Intermediat 6-21) und 82 mg 6-(Difluormethyl)pyridin-2-carbonsäure wurden in 3.0 ml THF vorgelegt, man addierte 49 mg 1-Hydroxy-1H-benzotriazol Hydrat, 121 mg 1-(3-Dimethylaminopropyl)-3-ethylcarbodiimidhydrochlorid und 0.13 ml Triethylamin und rührte 19.5 h bei Raumtemperatur. Man verdünnte mit Wasser, filtrierte den ausgefallenen Feststoff ab, wusch zweimal mit Wasser und dreimal mit Diethylether und trocknete im Vakuum. Man erhielt 129 mg der Titelverbindung.
¹H-NMR (300MHz, DMSO-d₆): δ [ppm]= 2.21 (s, 3H), 2.25 - 2.42 (m, 4H), 3.42 - 3.59 (m, 4H), 5.50 (s, 2H), 7.14 (t, 1H), 7.91 (s, 1H), 8.02 (dd, 1H), 8.29 - 8.44 (m, 3H), 8.64 (s, 1H), 10.60 (s, 1H).
UPLC-MS (Methode A2): Rt = 1.06 min (UV-TIC), gefundene Masse 462.00.

### Beispiel 311

### N-{6-Chlor-2-[2-(morpholin-4-yl)-2-oxoethyl]-2H-indazol-5-yl}-6-(difluormethyl)pyridin-2-carboxamid

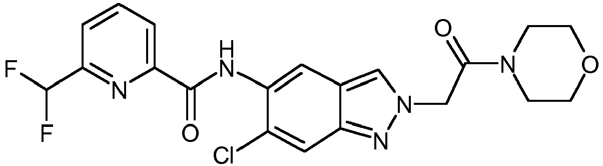

Analog zur Herstellung von Beispiel 310 wurden 137 mg 2-(5-Amino-6-chlor-2H-indazol-2-yl)-1-(morpholin-4-yl)ethanon (Intermediat 6-22) mit 70 mg 6-(Difluormethyl)pyridin-2-carbonsäure in 68 h bei Raumtemperatur umgesetzt. Man versetzte mit Wasser, saugte den Feststoff ab, wusch mit Aceton, Wasser und Diethylether und trocknete im Vakuum. Man erhielt 91 mg der Titelverbindung.
UPLC-MS (Methode A1): Rt = 1.06 min (UV-TIC), gefundene Masse 449.00.
¹H-NMR (400MHz, DMSO-d₆): δ [ppm]= 3.42 - 3.68 (m, 8H), 5.50 (s, 2H), 7.90 (s, 1H), 8.01 (dd, 1H), 8.29 - 8.36 (m, 2H), 8.41 (d, 1H), 8.64 (s, 1H), 10.59 (s, 1H).

### Bewertung der physiologischen Wirksamkeit

Die in vitro-Wirkung der erfindungsgemäßen Verbindungen kann in folgenden Assays gezeigt werden:

### Irak4- Kinaseassay

Die Irak4-inhibitorische Aktivität der erfindungsgemäßen Substanzen dieser Erfindung wurde in dem in den folgenden Absätzen beschriebenen Irak4-TR-FRET-Assay gemessen (TR-FRET = Time Resolved Fluorescence Resonance Energy Transfer).

Rekombinantes Fusionsprotein aus N-terminalem GST (Glutathion-S-Transferase) und humanem Irak4, exprimiert in Bakulovirus-infizierten Insektenzellen (Hi5, BTI-TN-5B1-4, Zelllinie gekauft von Invitrogen, Katalog-Nr. B855-02) und gereinigt via Affinitätschromatographie, wurde als Enzym verwendet. Als Substrat für die Kinasereaktion wurde das biotinylierte Peptid Biotin-Ahx-KKARFSRFAGSSPSQASFAEPG (C-Terminus in Amid-Form) verwendet, das z.B. bei der Firma Biosyntan GmbH (Berlin-Buch) gekauft werden kann.
Für den Assay wurden 11 verschiedene Konzentrationen im Bereich von 20 µM bis 0,073 nM aus einer 2 mM Lösung der Testsubstanz in DMSO hergestellt. 50 nl der jeweiligen Lösung wurden in eine schwarze low-volume 384well-Mikrotiterplatte (Greiner Bio-One, Frickenhausen, Deutschland) pipettiert, 2 µl einer Lösung von Irak4 in Assaypuffer [50 mM HEPES pH 7.5, 5 mM MgC12, 1.0 mM Dithiothreitol, 30 µM aktiviertes Natriumorthovanadat, 0,1 % (w/v) bovines gamma-Globulin (BGG) 0,04% (v/v) Nonidet-P40 (Sigma)] hinzugegeben und die Mischung für 15 min inkubiert, um eine Vorbindung der Substanzen an das Enzym vor der Kinasereaktion zu ermöglichen. Dann wurde die Kinasereaktion gestartet durch Zugabe von 3 µl einer Lösung von Adenosine-tri-phosphat (ATP, 1,67 mM =Endkonzentration in 5 µl Assayvolumen ist 1 mM) und Peptidsubstrat (0,83 µM =Endkonzentration in 5 µl Assayvolumen ist 0,5 µM) in Assaypuffer und die resultierende Mischung für die Reaktionszeit von 45 min bei 22°C inkubiert. Die Konzentration des Irak4 wurde an die jeweilige Aktivität des Enzyms angepasst und so eingestellt, dass der Assay im linearen Bereich arbeitete. Typische Konzentrationen lagen in der Größenordnung von etwa 0,2 nM. Die Reaktion wurde gestoppt durch Zugabe von 5 µl einer Lösung von TR-FRET-Detektionsreagentien [0,1 µM Streptavidin-XL665 (Cisbio Bioassays; Frankreich, Katalog-Nr. 610SAXLG) und 1,5 nM Anti-phosho-Serin Antikörper [Merck Millipore, "STK Antibody", Katalog-Nr. 35-002] und 0,6 nM LANCE EU-W1024-markierter anti-Maus-IgG-Antikörper (Perkin-Elmer, Produkt-Nr. AD0077, alternativ kann ein Terbium-Kryptat-markierter anti-Maus-IgG-Antikörper von Cisbio Bioassays verwendet werden) in wässriger EDTA-Lösung (100 mM EDTA, 0.4 % [w/v] bovines Serumalbumin [BSA] in 25 mM HEPES pH 7,5).

Die resultierende Mischung wurde 1 h bei 22°C inkubiert, um die Bildung eines Komplexes aus dem biotinylierten phosphorylierten Substrat und den Detektionsreagentien zu ermöglichen. Anschließend wurde die Menge des phosphorylierten Substrates ausgewertet durch eine Messung des Resonanz-Energietransfers vom Europium-Chelat markierten anti-Maus-IgG-Antikörper zum Streptavidin-XL665. Hierzu wurden in einem TR-FRET-Meßgerät, z.B. einem Rubystar (BMG Labtechnologies, Offenburg, Germany) oder einem Viewlux (Perkin-Elmer), die Fluoreszenz-Emissionen bei 620 nm and 665 nm nach Anregung bei 350 nm gemessen. Das Verhältnis der Emissionen bei 665 nm und 622 nm wurde als Maß für die Menge des phosphorylierten Substrates genommen. Die Daten wurden normalisiert (Enzymreaktion ohne Testsubstanz = 0 % Inhibition, alle anderen Assaykomponenten aber kein Enzym = 100 % Inhibition). Üblicherweise wurden die Testsubstanzen auf derselben Mikrotiterplatten bei 11 verschiedenen Konzentrationen im Bereich von 20 µM bis 0,073 nM getestet (20 µM, 5,7 µM, 1,6 µM, 0,47 µM, 0,13 µM, 38 nM, 11 nM, 3,1 nM, 0,89 nM, 0,25 nM und 0,073 nM). Die Verdünnungsreihen wurden vor dem Assay hergestellt (2 mM bis 7,3 nM in 100 % DMSO) durch serielle Verdünnungen. Die IC50-Werte wurden kalkuliert mit einem 4-Parameter-Fit.

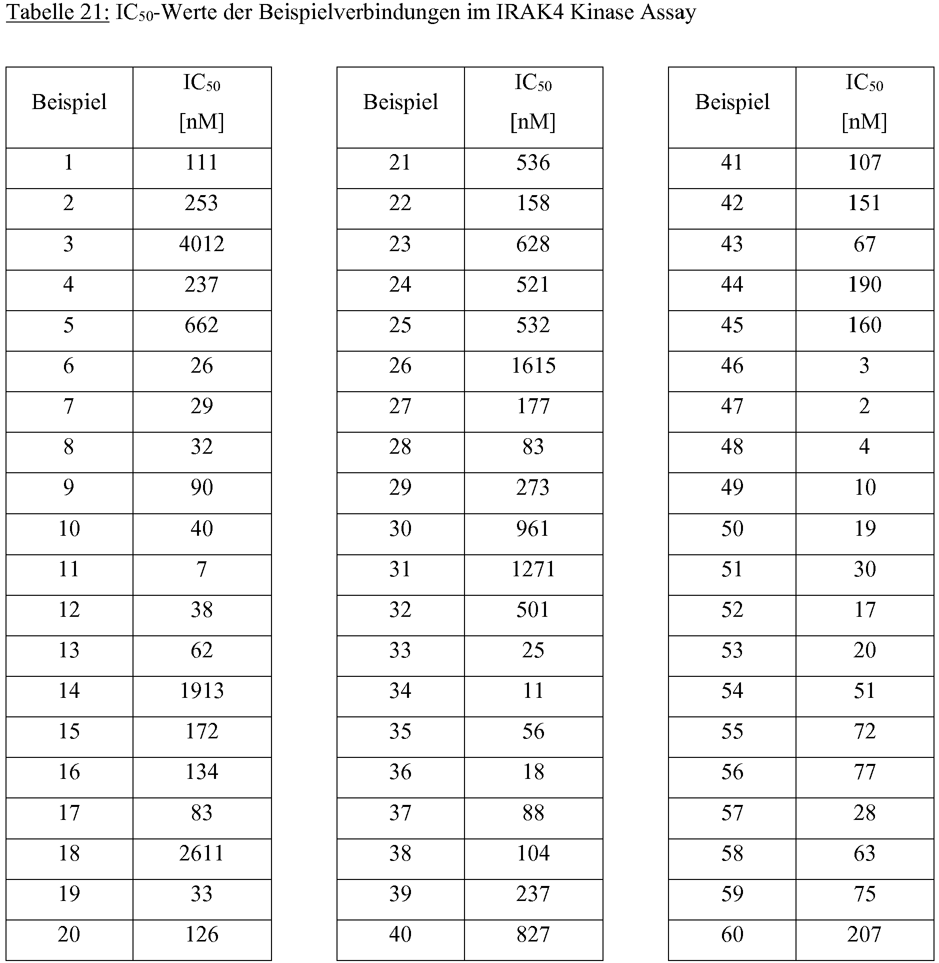

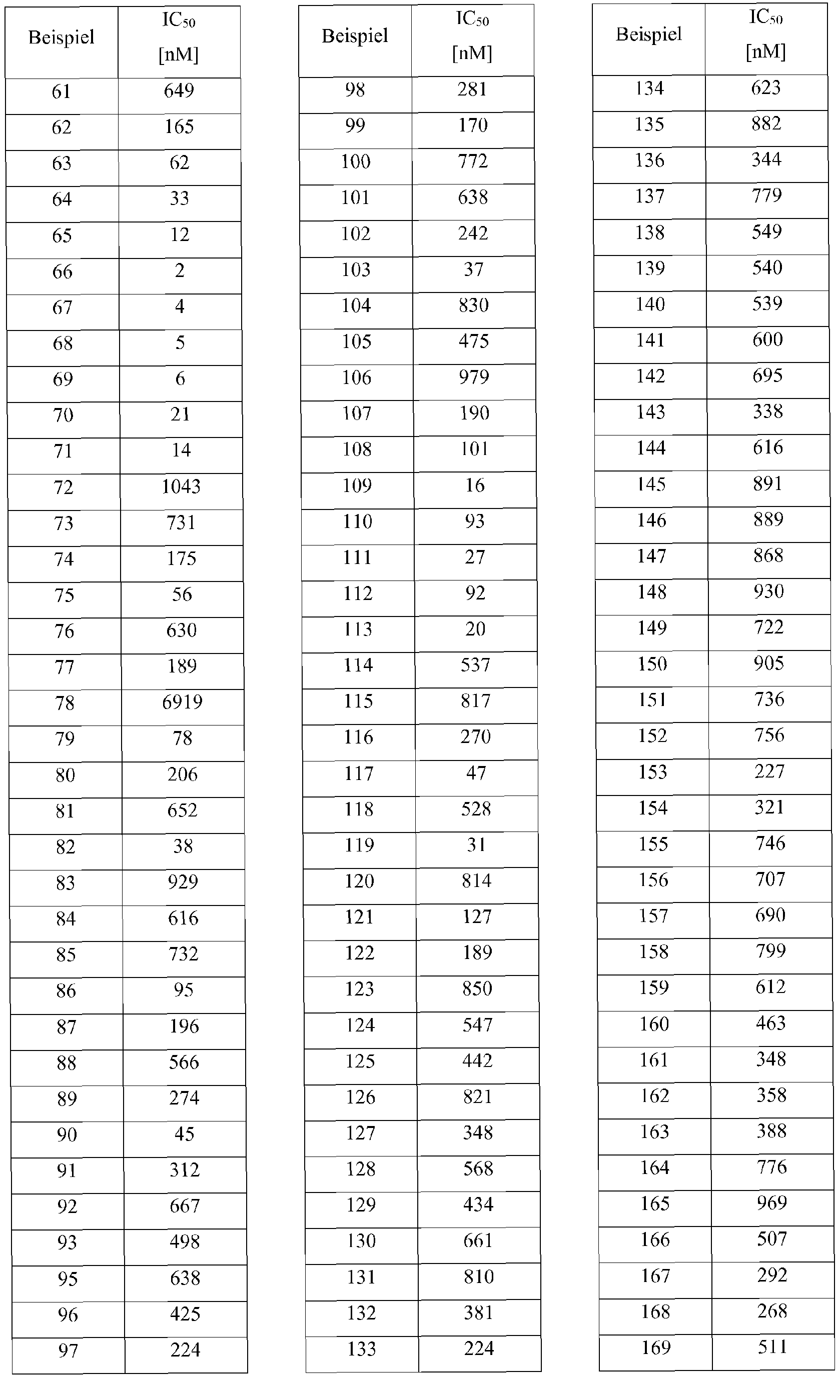

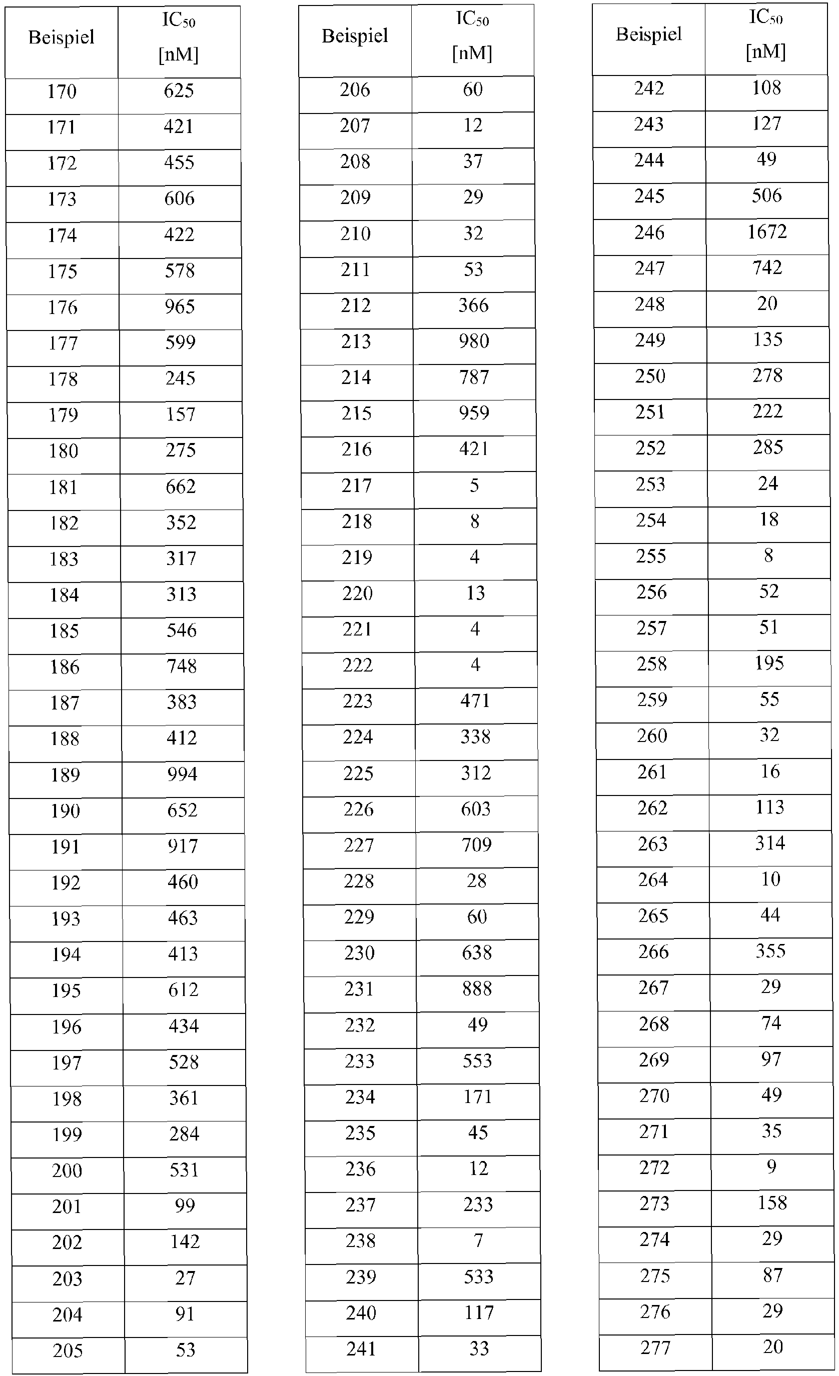

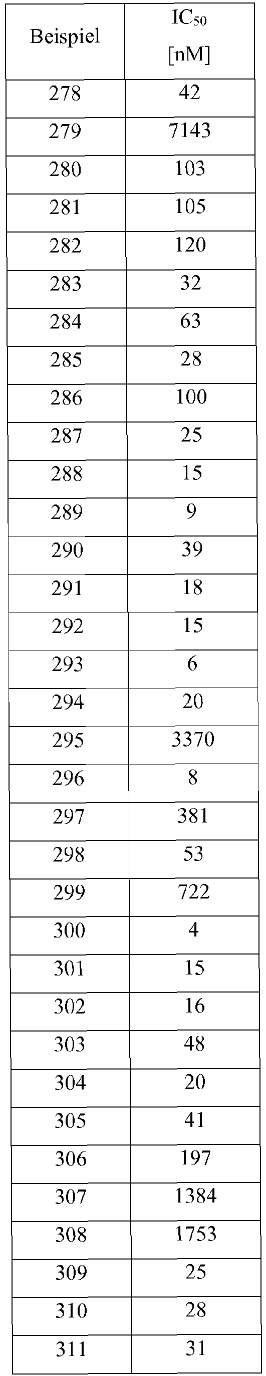

### TNF-α Ausschüttung in THP-1 Zellen

Mithilfe dieses Tests können Substanzen auf ihre Fähigkeit hin getestet werden, die TNF-α (Tumornekrosefaktor-alpha) Ausschüttung in THP-1 Zellen (humane monozytische akute Leukämie-Zellinie) zu inhibieren. TNF-α ist ein Zytokin, welches in inflammatorischen Prozessen beteiligt ist. Die TNF-α Ausschüttung wird in diesem Test ausgelöst durch Inkubation mit bakteriellem Lipopolysaccharid (LPS).
THP-1 Zellen werden in kontinuierlicher Suspensions-Zellkultur [RPMI 1460 Medium mit L-Glutamax (Gibco, Kat-Nr. 61870-044) supplemetiert mit foetalem Kälberserum (FCS) 10% (Invitrogen, Kat-Nr. 10082-147), 1% Penicillin/Streptomycin (Gibco BRL, Kat-Nr. 15140-114)] gehalten und sollten eine Zellkonzentration von 1x10⁶ Zellen/ml nicht überschreiten.
Der Assay erfolgte im Zellkulturmedium (RPMI 1460 Medium mit L-Glutamax supplemetiert mit FCS 10%).
Jeweils 2-2.5 µl der Zellsuspension (entspricht 4000 Zellen) wurden pro well in eine 384-well Testplatte (Greiner, Kat-Nr. 784076) dispensiert, in der sich jeweils 40-50 nl Substanz in 100% DMSO gelöst befanden. Hierbei wurden jeweils 10 verschiedene Konzentrationen im Bereich von 20 µM bis 0,073 nM je Substanz eingesetzt. Die Zellen wurden 15 min bei Raumtemperatur inkubiert. Anschließend wurden 2-2.5 µl pro well 0.1 µg/ml LPS (Sigma, *Escherichia coli* 055:B5, Kat-Nr. L5418) gelöst in Zellkulturmedium dispensiert (finale Konzentration 0.05 µg/ml). Als Neutralkontrolle wurden Zellen mit 0.05 µg/ml LPS und 1% DMSO und als Inhibitorkontrolle nur mit 1% DMSO behandelt.
Die Platten wird 30 sec bei 80g zentrifugiert und 17 h bei 37°C, 5% CO₂ und 95% Luftfeuchtigkeit inkubiert. Zur Bestimmung der Menge an TNF-α wurde der TNF-alpha HTRF Detection Kit (Cisbio, Kat-Nr. 62TNFPEB/C) verwendet. Hierzu wurden jeweils 2 µl der Detektionslösung, bestehend aus Anti-TNF-α-XL665 Konjugat und Anti-TNF-α-Kryptat Konjugat gelöst nach Anweisung des Herstellers im Rekonstitutionspuffer, für den HTRF (Homogeneous Time-Resolved Fluorescence) Test zugegeben. Nach Zugabe wurde entweder 3 h bei Raumtemperatur oder über Nacht bei 4°C inkubiert. Anschließend wurden die Signale mit einem HTRF-fähigen wie dem BMG PheraStar bei 620/665nm Messgerät ausgelesen.

Die Wirkung der Substanzen wird als Verhältnis zwischen Neutral- und Inhibitorkontrolle in Prozent ausgedrückt. Die IC₅₀-Werte wurden mit einem 4-Parameter-Fit kalkuliert.

### Tabelle 22: IC₅₀-Werte der Beispielverbindungen bezüglich der TNF-α Ausschüttung in THP-1 Zellen

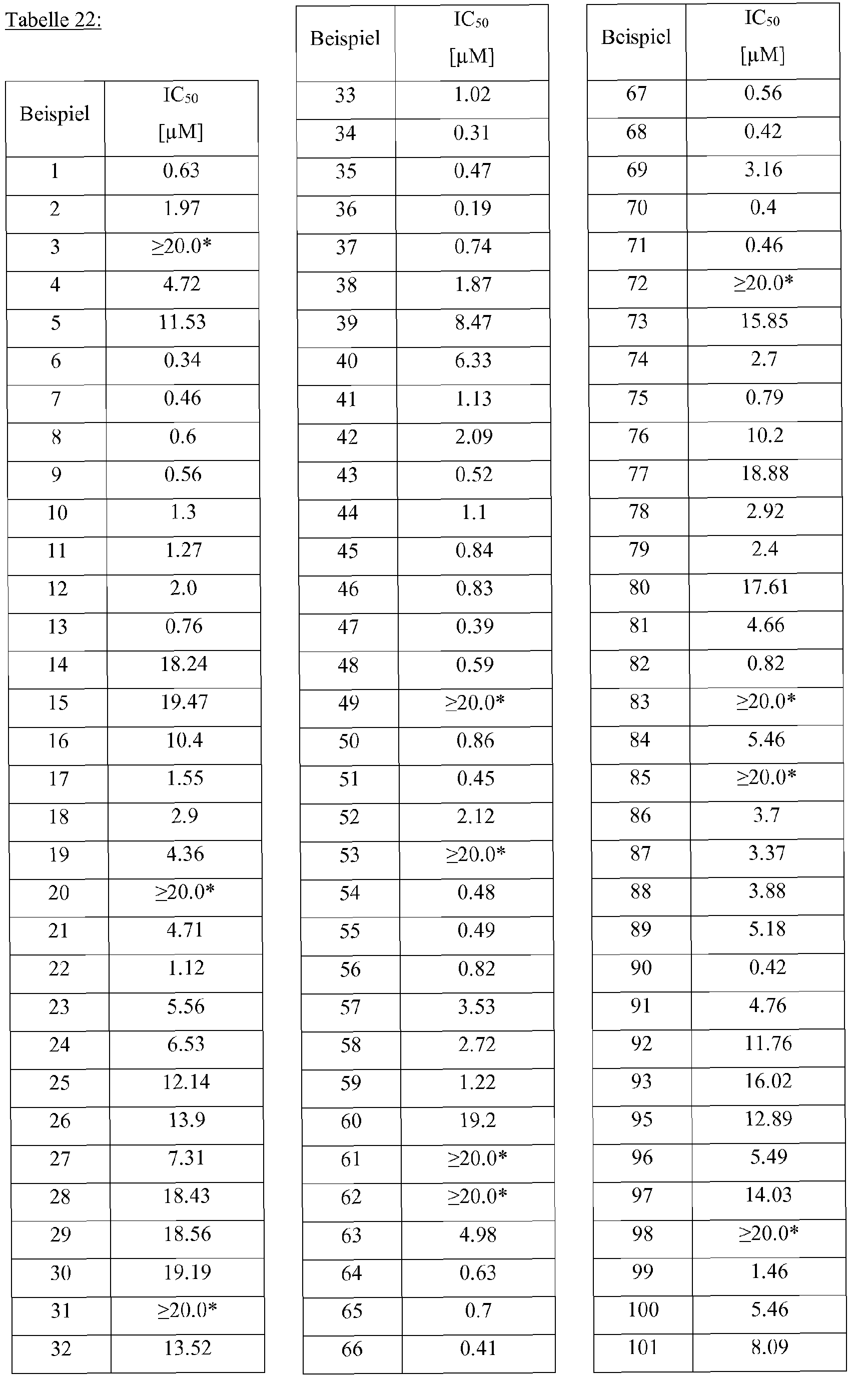

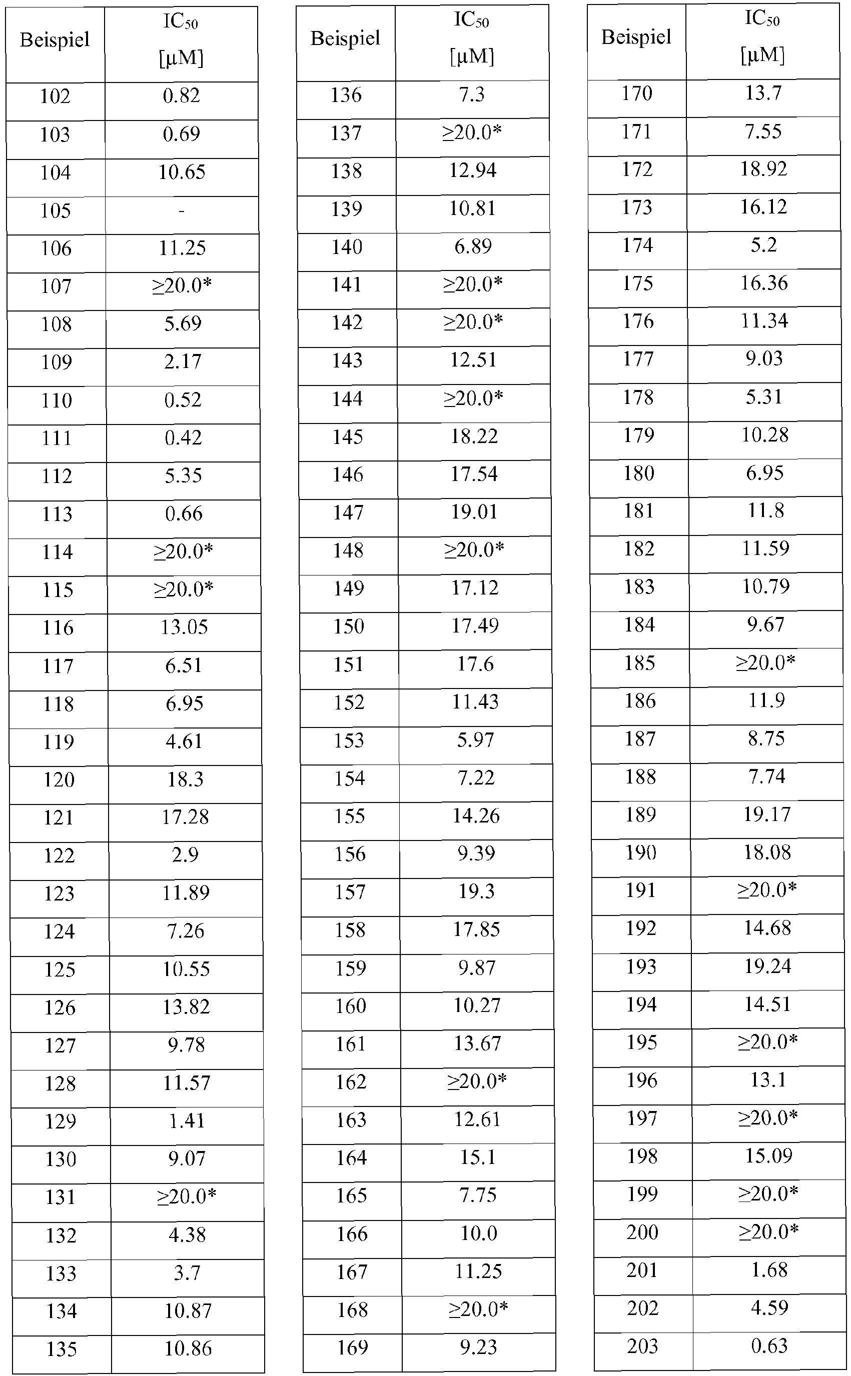

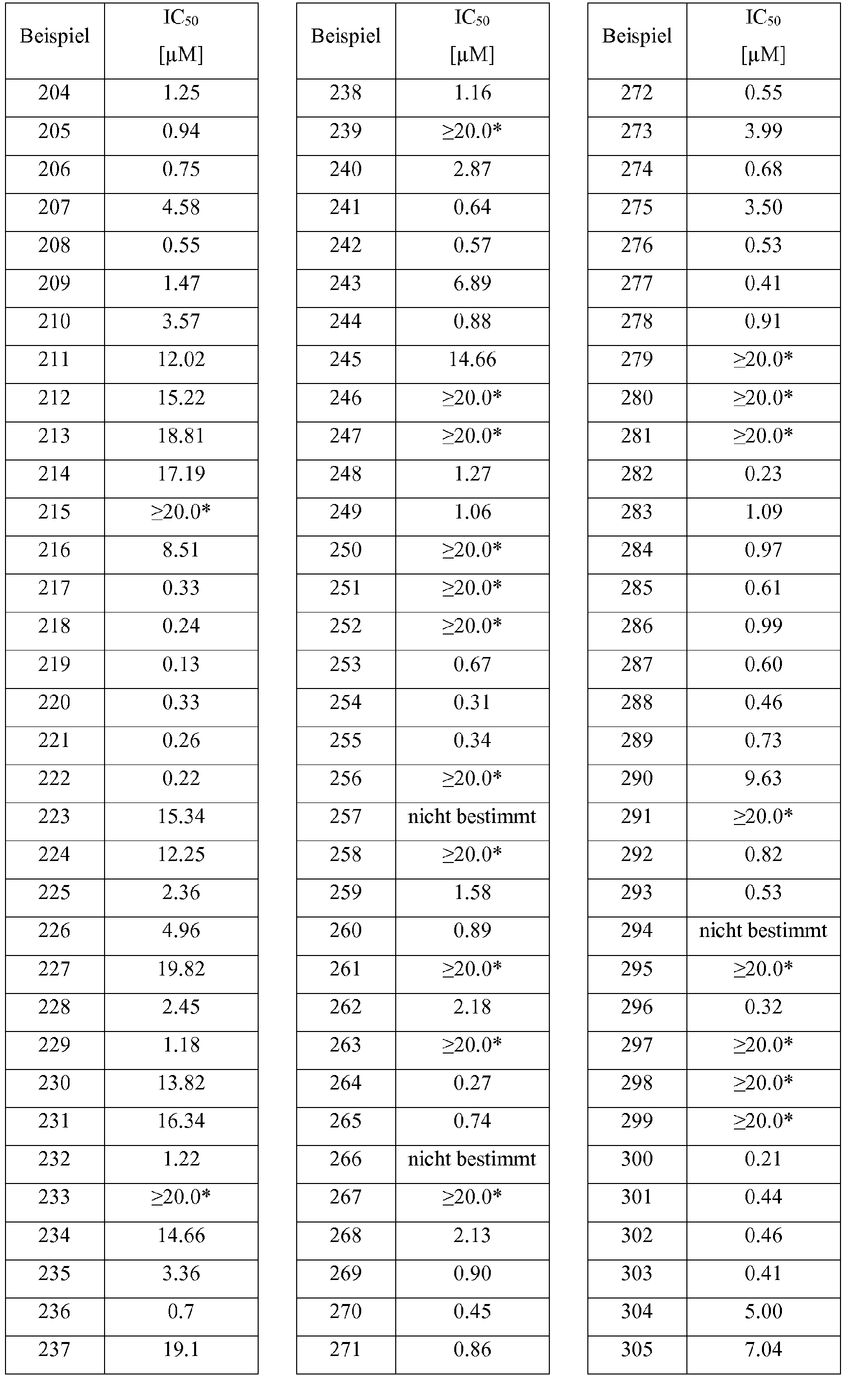

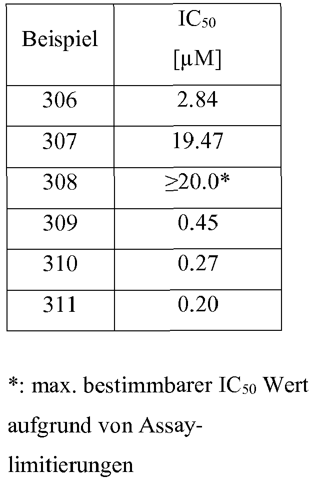

### In Vitro LPS (Lipopolysaccharide)-induzierte Zytokinproduktion in humanen PBMCs (peripheral blood mononuclear cells)

Die Wirkung der erfindungsgemäßen Verbindungen auf die induzierte Zytokinproduktion in humanen PBMCs wurde untersucht. Hierbei erfolgte die Induktion der Zytokinproduktion durch LPS, einen TLR4 Liganden, welcher zur Aktivierung des IRAK4-vermittelten Signalweges führt. Die Gewinnung der humanen PBMCs erfolgte aus anti-koaguliertem humanem Vollbut. Hierzu wurde in Leucosep-Röhrchen 15 ml Ficoll-Paque (Biochrom, Kat-Nr. L6115) vorgelegt und 20 ml Humanblut hinzugefügt. Nach Zentrifugation des Blutes bei 800g für 15 min bei Raumtemperatur wurde Plasma inklusive der Thrombozyten abgenommen und verworfen. Die PBMCs wurden in Zentrifugenröhrchen überführt und mit PBS (phosphatgepufferte Salzlösung) (Gibco, Kat-Nr. 14190) aufgefüllt. Die Zellsuspension wurde bei 250g für 10 min bei Raumtemperatur zentrifugiert und der Überstand verworfen. Die Resuspension der PBMCs erfolgte in Komplettmedium (RPMI 1640, ohne L-Glutamin (PAA, Kat-Nr. E15-039), 10% FCS; 50 U/ml Penicillin, 50 µg/ml Streptomycin (PAA, Kat-Nr. P11-010) und 1% L-Glutamin (Sigma, Kat-Nr. G7513)).
Auch der Assay erfolgte in Komplettmedium. Die PBMCs wurden in 96-well Platten mit einer Zelldichte von 2.5x10⁵ Zellen/well ausgesät. Die erfindungsgemäßen Verbindungen wurden in einem konstanten Volumen von 100% DMSO seriell verdünnt und in dem Assay mit 8 verschiedenen Konzentrationen im Bereich von 10 µM bis 3 nM so eingesetzt, so dass die finale DMSO Konzentration 0.4% DMSO betrug. Die Zellen wurden damit für 30 min vor der eigentlichen Stimulation vorinkubiert. Zur Induktion der Zytokinsekretion erfolgte eine Stimulation mit 0.1 µg/ml LPS (Sigma, Kat-Nr. L4516) für 24 Stunden. Die Bestimmung der Zellviabilität erfolgte durch Verwendung des CellTiter-Glo Luminescent Assay (Promega, Kat-Nr. G7571 (G755/G756A)) nach Anweisung des Herstellers. Die Bestimmung der Menge an sekretiertem TNF-α im Zellkulturüberstand erfolgte mittels Human ProInflammatory 9-Plex Tissue Culture Kit (MSD, Kat.-Nr. K15007B) nach Anweisung des Herstellers. Exemplarisch sind die Beispielverbindung 1 mit einer Aktivität zwischen 1 und 10 µM und die Beispielverbindungen 47, 64 und 71 mit einer Aktivität ≤ 1 µM genannt.

### In vitro Tumor-assozierte NF-kB-Reporter Aktivität

Die Wirkung der erfindungsgemäßen Verbindungen auf den NF-kB Signalweg wurde in humanen DLBCL (diffuses großzelliges B-Zell-Lymphom) Zelllinien untersucht. TMD-8, HBL-1, U2932 , HT und WSU-DLCL2 Zellen wurden mit einem lentiviralen NF-kB Reporter Konstrukt (Cignal Lenti NPκB Reporter (luc) Kit: CLS-013L, Qiagen) stabil transduziert und somit TMD-8-NF-kB-luc , HBL-1-NF-kB-luc , U2932-NF-kB-luc , HT-NF-kB-luc und WSU-DLCL2-NF-kB-luc Reporter Zell-Linien generiert. 10,000 Zellen wurden in 30 µL/Kavität in Wachstumsmedium ((RPMI (Biochrom, Kat-Nr. FG 1215), 20% FCS (Biochrom, Kat-Nr. S 0615)) oder in RPMI 1640 Medium supplementiert mit 10% FCS in einer 384- Kavitäten Platte (Perkin Elmer, weiß) überführt und über Nacht bei 37°C inkubiert. Nach 24h wurden Zellen mit Testsubstanzen behandelt und für 6h und 24h bei 37°C inkubiert. Die Testsubstanzen wurden in einer 7-fachen Verdünnung - entweder allein oder als Kombination zweier Testsubstanzen von verschiedenen Konzentrationen (Ratios Substanz 1 und Substanz 2: 1:0, 0,85:0,15; 0,7:0,3; 0,5:0,5; 0,3:0,7; 0,15:0,85; 0:1) mittels eines HP D300 Digital Dispensers zu den Zellen gegeben. Als Kontrolle wurden Zellen mit Vehikel (DMSO) behandelt. Nach 6h und 24h wurden die Zellen mit 30 µL/Kavität One-Glo Lösung (Promega, Kat-Nr. E6110) behandelt, für 10 min bei Raumtemperatur inkubiert und die Lumineszenz mittels eines VICTOR V (Perkin Elmer) gemessen, um die NF-KB Reporter Aktivität zum Ende der Behandlung zu bestimmen. Die prozentuale Beeinflussung der NF-KB Reporter Aktivität und die daraus abgeleiteten IC₅₀-Werte wurden für jede Testsubstanz bestimmt. Die ICso-Werte wurden mit einem 4-Parameter-Fit kalkuliert.
Exemplarisch ist die Beispielverbindung 289 mit einer Aktivität zwischen 1 und 10 µM bei den Zelllinien TMD-8-NF-kB-luc , HBL-1-NF-kB-luc , U2932-NF-kB-luc und WSU-DLCL2-NF-kB-luc genannt

### In vitro Tumor-assozierte Sekretion von Interleukin-6 und Interleukin-10

Die Wirkung der erfindungsgemäßen Verbindungen auf die Sekretion von Interleukin-6 und Interleukin-10 wurde in humanen TMD-8 DLBCL Zellen untersucht. 15000 Zellen/Kavität wurden in 100µl frischem Wachstumsmedium ((RPMI (Biochrom, Kat-Nr. FG 1215), 20% FCS (Biochrom, Kat-Nr. S 0615)) in einer 96-Kavitäten Platte (Perkin Elmer) ausgesät. Testsubstanzen wurden in einer 7-fachen Verdünnung mittels eines HP D300 Digital Dispensers zu den Zellen gegeben und für 24h inkubiert. Nach Ende der Inkubationszeit wurden die Überstände gesammelt und die Interleukin Konzentration mittels des Human IL-6/IL-10 Elisa Kit, (Life Technologies, Kat-Nr. KHC0062, KHC0101) nach Vorgaben des Herstellers bestimmt. Die prozentuale Beeinflussung der Interleukin- Sekretion wurde für jede Testsubstanz bestimmt.

Exemplarisch ist die Beispielverbindung 289 mit einer Aktivität zwischen 1 und 10 µM auf die Sekretion von Interleukin-6 und Interleukin-10 genannt.

Die Eignung der erfindungsgemäßen Verbindungen zur Behandlung von inflammatorischen Erkrankungen, Tumorerkrankungen sowie ophthalmologischen Erkrankungen wie der feuchten AMD (Altersbedingte Makuladegeneration) kann in folgenden Tiermodellen gezeigt werden:

### In Vivo TLR-vermitteltes Inflammationsmodel

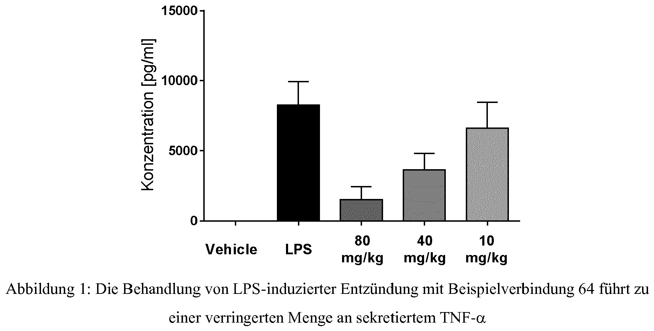

Die erfindungsgemäßen Verbindungen wurden in einem *in vivo* TLR-vermittelten Inflammationsmodell bezüglich ihrer in vivo Wirksamkeit untersucht. Dieses mechanistische Modell zeigt insbesondere die potentielle Wirkung der erfindungsgemäßen Verbindungen auf TLR4-vermittelte Erkrankungen, da ein LPS-vermitteltes Inflammationsmodell verwendet wurde. Hierbei wurden weibliche Balb/c Mäuse (ca. 8 Wochen alt; Charles River Laboratories, Deutschland) in Gruppen von jeweils 5 Tieren aufgeteilt. Die Kontrollgruppe wurde mit dem Vehikel, in welchem die Substanz gelöst wurde (Substanzvehikel), als auch mit dem Vehikel, in welchem das LPS gelöst wurde, behandelt. Neben den Substanzbehandlungsgruppen wurde auch der Positivkontrollgruppe jeweils 0,2 mg LPS/kg Körpergewicht (Sigma, Kat-Nr. L4391) (Lipopolysaccharides from E. coli 0111:B4) intraperitoneal (i.p.) verabreicht. Die Positivkontrollgruppe erhielt außerdem das oben beschriebene Substanzvehikel. Die Substanzgabe erfolgte oral 8 Stunden vor Induktion der Inflammation durch die Gabe von LPS. Zur Untersuchung der Wirkung der erfindungsgemäßen Verbindungen auf die Inflammation erfolgte bei den Tieren nach 1,5 Stunden die finale Blutentnahme. Die Bestimmung der Konzentration bestimmter Zytokine erfolgte im Plasma durch Verwendung des Mouse ProInflammatory 7-Plex Tissue Culture Kit (MSD, Kat.-Nr. K15012B) nach Anweisung des Herstellers. Abb. 1 zeigt die Menge an TNF-α im Plasma, die im Vergleich zur LPS-induzierten Konzentration durch die Gabe von Beispielverbindung 64 Dosis-abhängig reduziert ist.

### In Vivo IL-1β-vermitteltes Inflammationsmodel

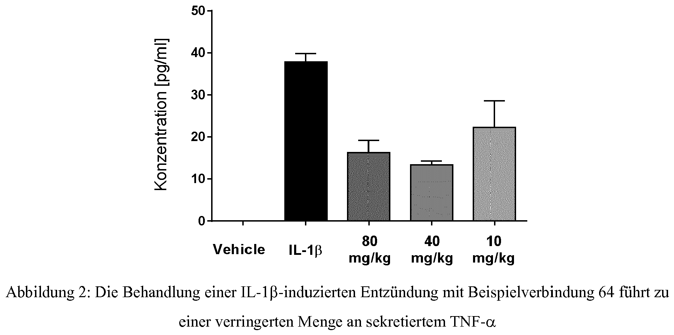

Zur Erfassung der potentiellen Wirksamkeit der erfindungsgemäßen Verbindungen in IL-1β-vermittelten Erkrankungen, wurde weiblichen Balb/c Mäusen (ca. 8 Wochen, Charles River Laboratories, Deutschland) IL-1β i.p. appliziert und die Wirkung der erfindungsgemäßen Verbindungen auf die IL-1β-vermittelte Zytokinsekretion untersucht. Die Gruppengröße betrug jeweils 5 Tiere. Die Kontrollgruppe wurde mit den Vehikeln, welche zur Lösung der Substanz und des IL-1β eingesetzt wurden, behandelt. Den Substanzbehandlungsgruppen und der Positivkontrollgruppe wurde jeweils 90 µg IL-1β /kg Körpergewicht (R&D, Kat-Nr.401-ML/CF) i.p verabreicht. Die Substanz bzw. dessen Vehikel in der Positivkontrollgruppe wurde 4 Stunden vor der IL-1β Verabreichung appliziert. Die Bestimmung von TNF-α im Plasma nach finaler Blutentnahme erfolgte 2 Stunden nach Verabreichung des IL-1β mittels des Mouse ProInflammatory 7-Plex Tissue Culture Kit (MSD, Kat.-Nr. K15012B) nach Anweisung des Herstellers. Die Gabe von IL-1β führt zu einer erhöhten Konzentration an TNF-α im Plasma, welches durch die Behandlung mit Beispielverbindung 64 inhibiert wird. Dies wird durch Abbildung 2 verdeutlicht.

### Sauerstoff-induziertes in vivo Retinopathie-Model (OIR)

Es wurde gezeigt, dass eine durch Sauerstoff induzierte Retinopathie ein wertvolles Tiermodell für die Untersuchung der pathologischen retinalen Angiogenese darstellt. Dieses Modell basiert auf der Beobachtung, dass Hyperoxie während der frühen postnatalen Entwicklung in der Retina zum Anhalten oder Verlangsamung des Wachstums von normalen retinalen Blutgefäßen führt. Sobald die Tiere nach einer 7-tägigen Hyperoxiephase, zur normoxischen Raumluft zurückkehren, ist dieses gleichbedeutend einer relativen Hypoxie. Die dadurch erzeugte ischämische Situation führt zu einer abnormen Neovaskularisation, die Ähnlichkeiten mit der pathophysiologischen Neovaskularisation in Augenerkrankungen wie der feuchten AMD (Altersbedingte Makuladegeneration) aufzeigt. Darüber hinaus ist die hervorgerufene Neovaskularisierung sehr reproduzierbar, quantifizierbar und ein wichtiger Parameter für die Untersuchung der Krankheitsmechanismen und einer möglichen Behandlungen für verschiedenste Formen von Netzhauterkrankungen.Aufgrund dessen ist dieses Modell zur Untersuchung der Wirkung der erfindungsgemäßen Verbindungen auf diesen pathologischen Prozess geeignet.
Hierzu werden 7 Tage alte murine Jungtiere beispielsweise C57B1/6 Jungtiere für 5 Tage einer hyperoxischen Umgebung (70% Sauerstoff) ausgesetzt. Von Tag 12 bis Tag 17 erfolgt die Haltung unter normoxischen Bedingungen. Normoxisch bedeutet Raumluft mit 21% Sauerstoff. Innerhalb dieses Zeitraumes werden die Tiere dann in Substanzbehandlungsgruppen und Vehikelgruppe aufgeteilt und entsprechend der Gruppe behandelt. Nach Tötung der Tiere am Tag 17 werden die Augen entnommen und in 4% Formalin fixiert. Nach dem Waschen in Phosphat-gepufferter Kochsalzlösung werden die Netzhaut präpariert, ein Flachpräperat davon erzeugt und dieses mit Isolectin B4 Antikörper gefärbt (Tual-Chalot, Allinson, et al., J. Vis. Exp., 2013). Die Quantifizierung der neugewachsenen Gefäße wird unter Verwendung eines Zeiss ApoTome durchgeführt.

### Laser-induziertes in vivo choroidales Neovaskularisationsmodell

Diese Studie dient dem Zweck, die Wirksamkeit einer Testsubstanz auf die Reduktion der Extravasation/Ödembildung und/oder der choroidalen Neovaskularisierung im Rattenmodell der Laser-induzierten choroidalen Neovaskularisierung zu untersuchen. Die Laser-vermittelte Photokoagulation führt im Tiermodell zu einer Zerstörung der Bruch-Membran, was mit einer Schädigung der Gefäße und zur entzündungsassoziierten Neovaskularisierung einhergeht. Beide Prozesse entsprechen dem Pathomechanismus der Makuladegeneration (Grossniklaus, Kang, Berglin, Prog Retin Eye Res., 2010).
Zur Bestimmung der Wirkung der erfindungsgemäßen Verbindungen werden Brown Norway Ratten (Charles River Laboratories) in die entsprechenden Gruppen (Substanz und Vehikel) aufgeteilt, betäubt und die Augen mit 0.5% Tropicamid zur Pupillenerweiterung beträufelt. Die choroidale Neovaskularisierung wird unter Betäubung der Tiere (15 mg/kg Xylazine und 80 mg/kg Ketamine) in jeweils einem Auge per Tier durch das Brennen von sechs Löchern in die Retina mittels 532 nm Argon Laser ausgelöst (Läsionsgröße: 50 µm - 75 µm; Laserintensität: 150 mW; Dauer: 100 ms). Die Behandlung der Tiere mit der erfindungsgemäßen Substanz bzw. dem entsprechenden Vehikel erfolgt entweder vom ersten bzw. siebten Tag an bis einschließlich Tag 23. Am Tag 21 wird eine Angiographie durchgeführt. Hierzu werden die Tiere jeweils betäubt, die Pupillen erweitert und 10% Natrium-Fluorescein Lösung subkutan injiziert. Das Angiogramm wird maximal 10 Minuten nach Injektion aufgenommen und von 3 Prüfern verblindet unter Verwendung eines Scoresystems (0= keine Färbung = keine Gefäßverletzung, 1= leichte Färbung = leichte Gefäßverletzung, 2= moderate Färbung = moderate Gefäßverletzung, 3= maximale Färbung = maximale Gefäßverletzung) bewertet. Nach Tötung der Tiere am Tag 23 werden die Augen entnommen und in 4%iger Paraform-Aldehyd-Lösung für eine Stunde bei Raumtemperatur fixiert. Nach einem Waschdurchgang wird die Retina vorsichtig herausgeschält, und der Sklera-Choroidea-Komplex wird mit einem FITC-Isolektin B4 Antikörper angefärbt und anschließend flach auf einen Objetträger aufgebracht. Die so erhaltenen Präparate werden mittels eines Fluoreszenz-Mikroskops (Apotom, Zeiss) bei einer Anregungs-Wellenlänge von 488 nm ausgewertet. Das Volumen bzw die Fläche der choroidalen Neovaskularisierung wird durch morphometrische Analyse mittels Axiovision 4.6 Software berechnet.

### Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der Verbindung von Beispiel 64, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Lösung:

### Zusammensetzung

500 mg der Verbindung von Beispiel 64, 2.5 g Polysorbat und 97 g Polyethylenglycol 400. Einer Einzeldosis von 100 mg der erfindungsgemäßen Verbindung entsprechen 20 g orale Lösung.

### Herstellung

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### Zusammensetzung:

1 mg der Verbindung von Beispiel 64, 15 g Polyethylenglykol 400 und 250 g Wasser für Injektionszwecke.

### Herstellung:

Die erfindungsgemäße Verbindung wird zusammen mit Polyethylenglykol 400 in dem Wasser unter Rühren gelöst. Die Lösung wird sterilfiltriert (Porendurchmesser 0,22 µm) und unter aseptischen Bedingungen in hitzesterilisierte Infusionsflaschen abgefüllt. Diese werden mit Infusionsstopfen und Bördelkappen verschlossen.

## Patentansprüche

1. Verbindungen der allgemeinen Formel (I) 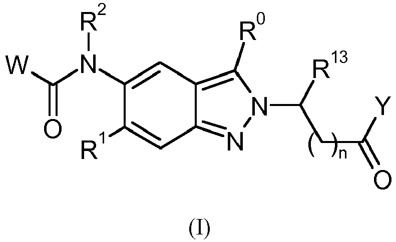 in der:
R⁰ für Wasserstoff oder C₁-C₄-Alkyl steht, wobei der C₁-C₄-Alkylrest gegebenenfalls ein- oder mehrfach, gleich oder verschieden mit Hydroxy oder Halogen substituiert sein kann;
R¹ für Wasserstoff, Halogen, Cyano, C(=O)OH, C(=O)OR^{a}, C(=O)NH₂, C(=O)N(H)R^{a}, C(=O)N(R^{a})R^{b}, C(=O)R^{d}, Hydroxy oder C₁-C₆-Alkyl steht, wobei der C₁-C₆-Alkylrest gegebenenfalls ein- oder mehrfach, gleich oder verschieden substituiert sein kann mit
Hydroxy, Halogen, Cyano, C(=O)OH, C(=O)OR^{a}, S(=O)₂-C₁-C₆-Alkyl, NH₂, NHR^{a}, N(R^{a})R^{b}, einem gegebenenfalls ein- oder mehrfach, gleich oder verschieden mit Halogen substituiertem C₁-C₆-Alkoxy, einem gegebenenfalls ein- oder mehrfach, gleich oder verschieden mit Halogen substituiertem C₃-C₈-Cycloalkoxy, einem ein- oder mehrfach, gleich oder verschieden mit R^{c} substituiertem Heterocycloalkyl,
oder für C₁-C₆-Alkoxy steht, wobei der C₁-C₆-Alkoxyrest gegebenenfalls ein- oder mehrfach, gleich oder verschieden substituiert sein kann mit
Hydroxy, Halogen, Cyano, C(=O)OH, C(=O)OR^{a}, S(=O)₂-C₁-C₆-Alkyl, NH₂, NHR^{a}, N(R^{a})R^{b}, einem gegebenenfalls ein- oder mehrfach, gleich oder verschieden mit Halogen substituiertem C₃-C₈-Cycloalkyl, einem gegebenenfalls ein- oder mehrfach, gleich oder verschieden mit Halogen substituiertem C₁-C₆-Alkoxy, einem gegebenenfalls ein- oder mehrfach, gleich oder verschieden mit Halogen substituiertem C₃-C₈-Cycloalkoxy, einem gegebenenfalls ein- oder mehrfach, gleich oder verschieden mit R^{c} substituiertem Heterocycloalkyl, einem gegebenenfalls ein- oder mehrfach, gleich oder verschieden mit R^{c} subsituiertem Aryl oder einem gegebenenfalls ein- oder mehrfach, gleich oder verschieden mit R^{c} substituiertem 5- oder 6-gliedrigen Heteroaryl,
oder für C₃-C₈-Cycloalkoxy oder Heterocycloalkoxy steht, welche gegebenenfalls ein- oder mehrfach, gleich oder verschieden substituiert sein können mit Hydroxy, Halogen, Cyano oder C₁-C₆-Alkyl,
oder für Aryloxy oder 5- oder 6-gliedriges Heteroaryloxy steht, worin Aryloxy und 5- oder 6-gliedriges Heteroaryloxy gegebenenfalls ein- oder mehrfach, gleich oder verschieden, substituiert sein können mit Hydroxy, Halogen, Cyano, C(=O)OH, C(=O)OR^{a}, C₁-C₆-Alkyl oder C₁-C₆-Alkoxy,
oder für C₃-C₈-Cycloalkyl oder Heterocycloalkyl steht, welche gegebenenfalls ein- oder mehrfach, gleich oder verschieden substituiert sein können mit Hydroxy, Halogen, Cyano oder C₁-C₆-Alkyl,
oder für C₂-C₆-Alkenyl oder C₂-C₆-Alkinyl steht,
oder für Aryl, 5- bis 10-gliedriges Heteroaryl, Aryl-C₁-C₄-alkyl oder 5- oder 6-gliedriges Heteroaryl-C₁-C₄-alkyl steht, worin Aryl und Heteroaryl gegebenenfalls ein- oder mehrfach, gleich oder verschieden substituiert sein können mit Halogen, Hydroxy, Cyano, C(=O)OH, C(=O)OR^{a}, C₁-C₆-Alkyl, C₃-C₈-Cycloalkyl oder C₁-C₆- Alkoxy;
R^{a} für C₁-C₆-Alkyl, C₃-C₁₀-Cycloalkyl, Heterocycloalkyl, Aryl oder Heteroaryl steht, worin Alkyl, Cycloalkyl, Heterocycloalkyl, Aryl und Heteroaryl gegebenenfalls ein- oder mehrfach, gleich oder verschieden substituiert sein können mit Halogen, Hydroxy, Cyano, C₁-C₃-Alkyl, C₁-C₃-Alkoxy, Heterocycloalkyl, -C(=O)O-C₁-C₆-Alkyl oder S(=O)₂-C₁-C₆-Alkyl;
R^{b} für C₁-C₆-Alkyl oder C₃-C₁₀-Cycloalkyl steht;
oder R^{a} und R^{b} bilden gemeinsam mit dem Stickstoffatom einen 5- oder 6-gliedrigen Heterocyclus, welcher gegebenenfalls ein- oder mehrfach, gleich oder verschieden substituiert sein kann mit Hydroxy, Halogen, Cyano oder C₁-C₆-Alkyl;
R^{c} für Hydroxy, Halogen, Cyano, C₁-C₃-Alkyl oder C₁-C₃-Alkoxy steht;
R^{d} für Wasserstoff, C₁-C₆-Alkyl oder C₃-C₁₀-Cycloalkyl steht;
R² für Wasserstoff, C₁-C₆-Alkyl oder C₃-C₆-Cycloalkyl steht;
R¹³ für Wasserstoff oder C₁-C₆-Alkyl steht;
W für 5-gliedriges Heteroaryl steht, welches ein bis drei Heteroatome ausgewählt aus der Gruppe N, O und S enthält und gegebenenfalls einfach mit R³ und gegebenenfalls ein- oder mehrfach, gleich oder verschieden mit R⁴ substituiert sein kann oder
W für Pyridyl, Pyrazinyl, Pyridazinyl, 1,2,4-Triazinyl oder 1,3,5-Triazinyl steht, welche gegebenenfalls einfach mit R³ und gegebenenfalls ein- oder mehrfach, gleich oder verschieden mit R⁴ substituiert sein können;
R³ für Wasserstoff, Halogen, Cyano, C(=O)R^{a}, NH₂, NHR^{a}, N(R^{a})R^{b}, N(H)C(=O)R^{a} oder C₁-C₆-Alkyl steht, wobei
C₁-C₆-Alkyl gegebenenfalls ein- oder mehrfach, gleich oder verschieden substituiert sein kann mit Hydroxy, Halogen, Cyano, C(=O)R^{a}, C(=O)OH, C(=O)OR^{a}, S(=O)₂-C₁-C₆-Alkyl, NH₂, NHR^{a}, N(R^{a})R^{b}, C₁-C₆-Alkoxy, C₃-C₈-Cycloalkoxy,
worin C₁-C₆-Alkoxy und C₃-C₈-Cycloalkoxy gegebenenfalls ein- oder mehrfach, gleich oder verschieden substituiert sein können mit Halogen;
oder C₁-C₆-Alkyl ist gegebenenfalls ein- oder mehrfach, gleich oder verschieden substituiert mit C₃-C₆-Cycloalkyl oder Heterocycloalkyl,
worin C₃-C₆-Cycloalkyl und Heterocycloalkyl optional ein-, zwei- oder dreimal, gleich oder verschieden, substituiert sein können mit Halogen, Cyano, C₁-C₃-Alkyl oder C₁-C₃-Alkoxy,
oder C₁-C₆-Alkyl ist gegebenenfalls ein- oder mehrfach, gleich oder verschieden substituiert mit Aryl oder 5- oder 6-gliedrigem Heteroaryl,
worin Aryl und 5- oder 6-gliedriges Heteroaryl optional ein-, zwei- oder dreimal, gleich oder verschieden, substituiert sein können mit Halogen, Cyano, C₁-C₃-Alkyl oder C₁-C₃-Alkoxy,
oder
R³ für C₁-C₆-Alkoxy steht, wobei C₁-C₆-Alkoxy gegebenenfalls ein- oder mehrfach, gleich oder verschieden substituiert sein kann mit Hydroxy, Halogen, Cyano, C(=O)OR^{a}, S(=O)₂-C₁-C₆-Alkyl, N(R^{a})R^{b}, C₃-C₈-Cycloalkyl, C₁-C₄-Alkoxy, C₃-C₈-Cycloalkoxy,
oder für C₃-C₆-Cycloalkyl, Heterocycloalkyl oder C₅-C₁₁-Spirocycloalkyl steht, wobei
Cycloalkyl, Heterocycloalkyl und Spirocycloalkyl gegebenenfalls ein- oder mehrfach, gleich oder verschieden substituiert sein können mit Hydroxy, Halogen, Cyano, C(=O)R^{a}, C(=O)OH, C(=O)OR^{a}, C₁-C₆-Alkyl oder C₁-C₄-Alkoxy;
oder für Aryl oder 5- bis 10-gliedriges Heteroaryl steht, worin
Aryl und Heteroaryl gegebenenfalls ein- oder mehrfach, gleich oder verschieden substituiert sein können mit Halogen, Hydroxy, Cyano, C(=O)OR^{a}, S(=O)₂-C₁-C₆-Alkyl, NO₂, NH₂, NHR^{a}, N(R^{a})R^{b}, N(H)C(=O)R^{a}, C₃-C₈-Cycloalkyl, C₁-C₃-Alkoxy oder C₁-C₃-Alkyl, wobei C₁-C₃-Alkyl gegebenenfalls ein- oder mehrfach, gleich oder verschieden substituiert sein kann mit Halogen;
R⁴ für Halogen, Hydroxy, Cyano oder C₁-C₆-Alkyl steht, worin C₁-C₆-Alkyl gegebenenfalls ein- oder mehrfach, gleich oder verschieden substituiert sein kann mit Halogen, C₁-C₆-Alkoxy, worin C₁-C₆-Alkoxy gegebenenfalls ein- oder mehrfach, gleich oder verschieden substituiert sein kann mit Halogen, C₂-C₆-Alkenyl, C₂-C₆-Alkinyl, C₃-C₁₀-Cycloalkyl, 3- bis 10-gliedriges Heterocycloalkyl oder Aryl, wobei Aryl gegebenenfalls ein- oder mehrfach, gleich oder verschieden mit R substituiert sein kann,
oder
R⁴ für Aryl oder Heteroaryl steht, welche gegebenenfalls ein- oder mehrfach, gleich oder verschieden substituiert sein können mit R,
oder
R⁴ für C(=O)R^{a}, C(=O)NH₂, C(=O)N(H)R^{a}, C(=O)N(R^{a})R^{b}, C(=O)OR^{a}, NH₂, NHR^{a}, N(R^{a})R^{b}, N(H)C(=O)R^{a}, N(R^{a})C(=O)R^{a}, N(H)C(=O)NH₂, N(H)C(=O)NHR^{a}, N(H)C(=O)N(R^{a})R^{b}, N(R^{a})C(=O)NH₂, N(R^{a})C(=O)NHR^{a}, N(R^{a})C(=O)N(R^{a})R^{b}, N(H)C(=O)OR^{a}, N(R^{a})C(=O)OR^{a}, NO₂, N(H)S(=O)R^{a}, N(R^{a})S(=O)R^{a}, N(H)S(=O)₂R^{a}, N(R^{a})S(=O)₂R^{a}, N=S(=O)(R^{a})R^{b}, OC(=O)R^{a}, OC(=O)NH₂, OC(=O)NHR^{a}, OC(=O)N(R^{a})R^{b}, SH, SR^{a}, S(=O)R^{a}, S(=O)₂R^{a}, S(=O)₂NH₂, S(=O)₂NHR^{a}, S(=O)₂N(R^{a})R^{b} oder S(=O)(=N-R^{a})R^{b} steht;
R für Halogen, Cyano, C₁-C₆-Alkyl, C₂-C₆-Alkenyl,C₂-C₆-Alkinyl, C₃-C₁₀-Cycloalkyl, 3- bis 10-gliedriges Heterocycloalkyl, Aryl, Heteroaryl, C(=O)R^{a}, C(=O)NH₂, C(=O)N(H)R^{a},C(=O)N(R^{a})R^{b}, C(=O)OR^{a}, NH₂, NHR^{a}, N(R^{a})R^{b}, N(H)C(=O)R^{a}, N(R^{a})C(=O)R^{a}, N(H)C(=O)NH₂, N(H)C(=O)NHR^{a}, N(H)C(=O)N(R^{a})R^{b}, N(R^{a})C(=O)NH₂, N(R^{a})C(=O)NHR^{a}, N(R^{a})C(=O)N(R^{a})R^{b}, N(H)C(=O)OR^{a}, N(R^{a})C(=O)OR^{a}, NO₂, N(H)S(=O)R^{a}, N(R^{a})S(=O)R^{a}, N(H)S(=O)₂R^{a}, N(R^{a})S(=O)₂R^{a}, N=S(=O)(R^{a})R^{b}, OH, C₁-C₆-Alkoxy, OC(=O)R^{a}, OC(=O)NH₂, OC(=O)NHR^{a}, OC(=O)N(R^{a})R^{b}, SH, SR^{a}, S(=O)R^{a}, S(=O)₂R^{a}, S(=O)₂NH₂, S(=O)₂NHR^{a}, S(=O)₂N(R^{a})R^{b} oder S(=O)(=NR^{a})R^{b} steht;
n für 0 oder 1 steht;
Y für eine Gruppe steht, ausgewählt aus: 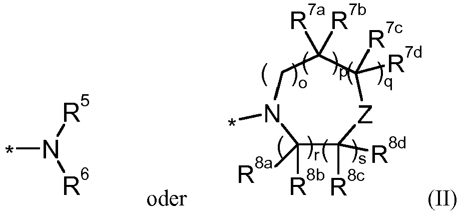
wobei * für die Verknüpfungsstelle der Gruppe mit dem Rest des Moleküls steht;.
R⁵ für Wasserstoff, C₁-C₆-Alkyl oder C₃-C₁₀-Cycloalkyl steht, wobei
C₁-C₆-Alkyl gegebenenfalls ein- oder mehrfach, gleich oder verschieden substituiert sein kann mit Hydroxy, Halogen, Cyano, C(=O)OH, C(=O)OR^{a}, S(=O)₂-C₁-C₆-Alkyl, N(R^{a})R^{b}, C₁-C₄-Alkoxy oder C₃-C₈-Cycloalkyl;
R⁶ für Wasserstoff oder C₁-C₆-Alkyl steht, wobei
C₁-C₆-Alkyl gegebenenfalls ein- oder mehrfach, gleich oder verschieden substituiert sein kann mit Hydroxy, Halogen, Cyano, C₃-C₁₀-Cycloalkyl, C(=O)R^{a}, C(=O)OH, C(=O)OR^{a}, S(=O)₂-C₁-C₆-Alkyl, N(R^{a})R^{b}, C₁-C₄-Alkoxy oder C₃-C₈-Cycloalkoxy,
oder für C₃-C₁₀-Cycloalkyl steht, wobei C₃-C₁₀-Cycloalkyl gegebenenfalls ein- oder mehrfach, gleich oder verschieden substituiert sein kann mit Hydroxy, Halogen, Cyano oder C₁-C₆-Alkyl, wobei
C₁-C₆-Alkyl gegebenenfalls mit Hydroxy substituiert sein kann,
oder für Heterocycloalkyl steht, worin
Heterocycloalkyl gegebenenfalls ein- oder mehrfach, gleich oder verschieden substituiert sein kann mit Halogen, Cyano, C₁-C₃-Alkyl oder C₁-C₃-Alkoxy,
oder für Aryl oder 5- oder 6-gliedriges Heteroaryl steht, worin Aryl und 5- oder 6-gliedriges Heteroaryl gegebenenfalls ein- oder mehrfach, gleich oder verschieden substituiert sein können mit Halogen, Cyano, C₁-C₃-Alkyl, C₁-C₃-Alkoxy, S(=O)₂NH₂, S(=O)₂NHR^{a} oder S(=O)₂N(R^{a})R^{b};
R^{7a} für Wasserstoff, Halogen, N(R^{a})R^{b}, C₁-C₆-Alkyl oder C₃-C₁₀-Cycloalkyl steht, wobei
C₁-C₆-Alkyl gegebenenfalls ein- oder mehrfach, gleich oder verschieden substituiert sein kann mit Hydroxy, Halogen, Cyano, C(=O)OH, C(=O)OR^{a}, S(=O)₂-C₁-C₆-Alkyl, N(R^{a})R^{b}, C₁-C₄-Alkoxy, C₃-C₈-Cycloalkyl oder Heterocycloalkyl;
R^{7b} für Wasserstoff, Halogen oder C₁-C₆-Alkyl steht, wobei
C₁-C₆-Alkyl gegebenenfalls ein- oder mehrfach, gleich oder verschieden substituiert sein kann mit Hydroxy, Halogen, Cyano, C(=O)OH, C(=O)OR^{a}, S(=O)₂-C₁-C₆-Alkyl, N(R^{a})R^{b}, C₁-C₄-Alkoxy, C₃-C₈-Cycloalkyl oder Heterocycloalkyl;
oder R^{7a} und R^{7b} bilden gemeinsam mit dem Kohlenstoffatom ein C₃-C₆-Cycloalkyl, welches gegebenenfalls ein- oder mehrfach, gleich oder verschieden substituiert sein kann mit Hydroxy, Halogen, Cyano oder C₁-C₆-Alkyl,
oder R^{7a} und R^{7b} stehen gemeinsam für eine Oxo-Gruppe;
R^{7c} für Wasserstoff, Halogen, N(R^{a})R^{b}, C₁-C₆-Alkyl oder C₃-C₁₀-Cycloalkyl steht, wobei C₁-C₆-Alkyl gegebenenfalls ein- oder mehrfach, gleich oder verschieden substituiert sein kann mit Hydroxy, Halogen, Cyano, C(=O)OH, C(=O)OR^{a}, S(=O)₂-C₁-C₆-Alkyl, N(R^{a})R^{b}, C₁-C₄-Alkoxy, C₃-C₈-Cycloalkyl oder Heterocycloalkyl;
R^{7d} für Wasserstoff, Halogen oder C₁-C₆-Alkyl steht, wobei
C₁-C₆-Alkyl gegebenenfalls ein- oder mehrfach, gleich oder verschieden substituiert sein kann mit Hydroxy, Halogen, Cyano, C(=O)OH, C(=O)OR^{a}, S(=O)₂-C₁-C₆-Alkyl, N(R^{a})R^{b}, C₁-C₄-Alkoxy, C₃-C₈-Cycloalkyl oder Heterocycloalkyl;
oder R^{7c} und R^{7d} bilden gemeinsam mit dem Kohlenstoffatom ein C₃-C₆-Cycloalkyl, welches gegebenenfalls ein- oder mehrfach, gleich oder verschieden substituiert sein kann mit Hydroxy, Halogen, Cyano oder C₁-C₆-Alkyl,
oder R^{7c} und R^{7d} stehen gemeinsam für eine Oxo-Gruppe;
R^{8a} für Wasserstoff, Halogen, N(R^{a})R^{b}, C₁-C₆-Alkyl oder C₃-C₁₀-Cycloalkyl steht, wobei
C₁-C₆-Alkyl gegebenenfalls ein- oder mehrfach, gleich oder verschieden substituiert sein kann mit Hydroxy, Halogen, Cyano, C(=O)OH, C(=O)OR^{a}, S(=O)₂-C₁-C₆-Alkyl, N(R^{a})R^{b}, C₁-C₄-Alkoxy, C₃-C₈-Cycloalkyl oder Heterocycloalkyl;
R^{8b} für Wasserstoff, Halogen oder C₁-C₆-Alkyl steht, wobei C₁-C₆-Alkyl gegebenenfalls ein- oder mehrfach, gleich oder verschieden substituiert sein kann mit Hydroxy, Halogen, Cyano, C(=O)OH, C(=O)OR^{a}, S(=O)₂-C₁-C₆-Alkyl, N(R^{a})R^{b}, C₁-C₄-Alkoxy, C₃-C₈-Cycloalkyl oder Heterocycloalkyl;
oder R^{8a} und R^{8b} bilden gemeinsam mit dem Kohlenstoffatom ein C₃-C₆-Cycloalkyl, welches gegebenenfalls ein- oder mehrfach, gleich oder verschieden substituiert sein kann mit Hydroxy, Halogen, Cyano oder C₁-C₆-Alkyl,
R^{8c} für Wasserstoff, Halogen, N(R^{a})R^{b}, C₁-C₆-Alkyl oder C₃-C₁₀-Cycloalkyl steht, wobei C₁-C₆-Alkyl gegebenenfalls ein- oder mehrfach, gleich oder verschieden substituiert sein kann mit Hydroxy, Halogen, Cyano, C(=O)OH, C(=O)OR^{a}, S(=O)₂-C₁-C₆-Alkyl, N(R^{a})R^{b}, C₁-C₄-Alkoxy, C₃-C₈-Cycloalkyl oder Heterocycloalkyl;
R^{8d} für Wasserstoff, Halogen oder C₁-C₆-Alkyl steht, wobei
C₁-C₆-Alkyl gegebenenfalls ein- oder mehrfach, gleich oder verschieden substituiert sein kann mit Hydroxy, Halogen, Cyano, C(=O)OH, C(=O)OR^{a}, S(=O)₂-C₁-C₆-Alkyl, N(R^{a})R^{b}, C₁-C₄-Alkoxy, C₃-C₈-Cycloalkyl oder Heterocycloalkyl;
oder R^{8c} und R^{8d} bilden gemeinsam mit dem Kohlenstoffatom ein C₃-C₆-Cycloalkyl, welches gegebenenfalls ein- oder mehrfach, gleich oder verschieden substituiert sein kann mit Hydroxy, Halogen, Cyano oder C₁-C₆-Alkyl,
oder R^{8c} und R^{8d} stehen gemeinsam für eine Oxo-Gruppe;
o für 0, 1 oder 2 steht,
p für 0, 1 oder 2 steht,
q für 0, 1 oder 2 steht,
r für 0, 1 oder 2 steht,
s für 0, 1 oder 2 steht,
wobei o, p, q, r und s nicht gleichzeitig 0 bedeuten;
Z für eine Gruppe steht ausgewählt aus C(=O), CR⁹R¹⁰, NR¹¹, O, S, S(=O) oder S(=O)₂;
R⁹ für Wasserstoff oder C₁-C₆-Alkyl steht,
R¹⁰ für Wasserstoff, Halogen, Cyano, C(=O)R^{a}, C(=O)OH, C(=O)OR^{a}, C(=O)NH₂, C(=O)N(H)R^{a},C(=O)N(R^{a})R^{b}, N(H)C(=O)R^{a}, N(R^{b})C(=O)R^{a}, S(=O)₂R^{a}, Hydroxy, N(R^{a})R^{b} oder C₁-C₆-Alkyl steht, wobei
C₁-C₆-Alkyl gegebenenfalls ein- oder mehrfach, gleich oder verschieden substituiert sein kann mit Hydroxy, Halogen, Cyano, C(=O)R^{a}, C(=O)OH, C(=O)OR^{a}, S(=O)₂-C₁-C₆-Alkyl, N(R^{a})R^{b}, C₁-C₄-Alkoxy oder C₃-C₈-Cycloalkoxy,
oder für C₁-C₆-Alkoxy steht, wobei C₁-C₆-Alkoxy gegebenenfalls ein- oder mehrfach, gleich oder verschieden substituiert sein kann mit Hydroxy, Halogen, Cyano, C(=O)OH, C(=O)OR^{a}, S(=O)₂-C₁-C₆-Alkyl, N(R^{a})R^{b}, C₃-C₈-Cycloalkyl, C₁-C₄-Alkoxy, C₃-C₈-Cycloalkoxy, Heterocycloalkyl, Aryl oder 5- oder 6-gliedriges Heteroaryl, worin
Aryl und 5- oder 6-gliedriges Heteroaryl gegebenenfalls ein- oder mehrfach, gleich oder verschieden substituiert sein können mit Halogen, Cyano, C₁-C₃-Alkyl oder C₁-C₃-Alkoxy,
oder für Aryloxy oder 5- oder 6-gliedriges Heteroaryloxy steht, worin Aryloxy und 5- oder 6-gliedriges Heteroaryloxy gegebenenfalls ein- oder mehrfach, gleich oder verschieden substituiert sein können mit Hydroxy, Halogen, Cyano, C(=O)OH, C(=O)OR^{a}, C₁-C₃-Alkyl oder C₁-C₃-Alkoxy,
oder für C₃-C₈-Cycloalkyl, C₃-C₈-Cycloalkyl-C₁-C₄-alkyl, Heterocycloalkyl oder Heterocycloalkyl-C₁-C₄-alkyl steht, welche gegebenenfalls ein- oder mehrfach, gleich oder verschieden substituiert sein können mit Hydroxy, Halogen, Cyano, C(=O)R^{a}, C(=O)OH, C(=O)OR^{a}, C₁-C₆-Alkyl oder C₁-C₆-Alkoxy, wobei
C₁-C₆-Alkoxy gegebenenfalls ein- oder mehrfach, gleich oder verschieden substituiert sein kann mit Halogen oder einer Oxo-Gruppe,
oder für C₂-C₆-Alkenyl oder C₂-C₆-Alkinyl steht,
oder für Aryl, 5- bis 10-gliedriges Heteroaryl, Aryl-C₁-C₄-alkyl oder 5- oder 6-gliedriges Heteroaryl-C₁-C₄-alkyl steht, worin
Aryl und Heteroaryl gegebenenfalls ein- oder mehrfach, gleich oder verschieden substituiert sein können mit Halogen, Hydroxy, Cyano, C(=O)OH, C(=O)OR^{a}, NHR^{a}, N(R^{a})R^{b}, C₁-C₃-Alkyl, C₃-C₈-Cycloalkyl oder C₁-C₃-Alkoxy;
oder R⁹ und R¹⁰ bilden gemeinsam mit dem Kohlenstoffatom ein C₃-C₈-Cycloalkyl oder einen 4- bis 6-gliedrigen Heterocyclus, wobei
C₃-C₈-Cycloalkylrest oder der 4- bis 6-gliedrige Heterocyclus gegebenenfalls ein- oder mehrfach, gleich oder verschieden substituiert sein können mit Hydroxy, Halogen, Cyano, C₁-C₆-Alkyl, C(=O)R^{a} oder einer Oxo-Gruppe;
R¹¹ für Wasserstoff, C(=O)R^{a}, C(=O)OR^{a}, C(=O)NH₂, C(=O)N(H)R^{a},C(=O)N(R^{a})R^{b}, S(=O)₂R^{a}, S(=O)₂N(R^{a})R^{b} oder C₁-C₆-Alkyl steht, wobei
C₁-C₆-Alkyl gegebenenfalls ein- oder mehrfach, gleich oder verschieden substituiert sein kann mit Hydroxy, Halogen, Cyano, C(=O)R^{a}, C(=O)OR^{a}, C(=O)NH₂, C(=O)N(H)R^{a},C(=O)N(R^{a})R^{b}, S(=O)₂-C₁-C₆-Alkyl, N(R^{a})R^{b}, C₃-C₈-Cycloalkyl, C₁-C₄-Alkoxy oder C₃-C₈-Cycloalkoxy, worin
C₃-C₈-Cycloalkyl, C₁-C₄-Alkoxy und C₃-C₈-Cycloalkoxy gegebenenfalls ein- oder mehrfach, gleich oder verschieden substituiert sein können mit Hydroxy oder Halogen,
oder für C₃-C₈-Cycloalkyl, Heterocycloalkyl oder Heterocycloalkyl-C₁-C₄-alkyl steht, welche gegebenenfalls ein- oder mehrfach, gleich oder verschieden substituiert sein können mit Hydroxy, Halogen, Cyano, C₁-C₆-Alkyl, C₁-C₆-Alkoxy, wobei Alkyl und Alkoxy gegebenenfalls ein- oder mehrfach, gleich oder verschieden substituiert sein können mit Halogen oder einer Oxo-Gruppe,
oder für C₂-C₆-Alkenyl oder C₂-C₆-Alkinyl steht, oder für Aryl, 5- bis 10-gliedriges Heteroaryl, Aryl-C₁-C₄-alkyl oder 5- oder 6-gliedriges Heteroaryl-C₁-C₄-alkyl steht, worin
Aryl und Heteroaryl gegebenenfalls ein- oder mehrfach, gleich oder verschieden substituiert sein können mit Halogen, Hydroxy, Cyano, C(=O)OH, C(=O)OR^{a}, C₁-C₃-Alkyl, C₃-C₈-Cycloalkyl oder C₁-C₃-Alkoxy;
und ihre Diastereomere, Enantiomere, ihre Salze, ihre Solvate oder die Solvate ihrer Salze.

2. Verbindungen gemäß Anspruch 1, worin R¹ Wasserstoff, Halogen, Hydroxy, Cyano, C₁-C₆-Alkyl, C₁-C₅-Alkyl substituiert mit Hydroxy, C₁-C₆-Alkoxy, C₁-C₆-Alkoxy substituiert mit C₃-C₈-Cycloalkyl, C₁-C₆-Alkoxy substituiert mit bis zu drei Fluoratomen, C₁-C₆-Alkoxy substituiert mit einem gegebenenfalls ein- oder mehrfach, gleich oder verschieden mit R^{c} subsituiertem Aryl oder einem gegebenenfalls ein- oder mehrfach, gleich oder verschieden mit R^{c} substituiertem 5- oder 6-gliedrigen Heteroaryl ist.

3. Verbindungen gemäß Anspruch 1, worin W eine Gruppe ausgewählt aus den Formeln (III) bis (IX) ist: 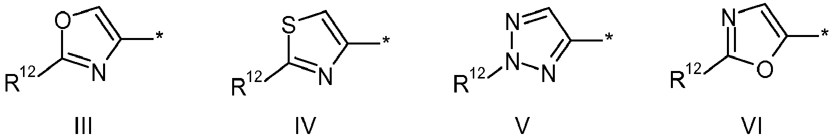 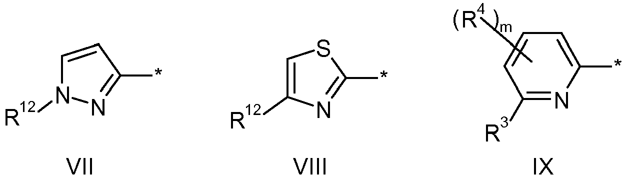 worin
R¹² für Wasserstoff, Halogen, ein gegebenenfalls ein- oder mehrfach, gleich oder verschieden mit Halogen substituiertes C₁-C₆-Alkyl, ein gegebenenfalls ein- oder mehrfach, gleich oder verschieden mit Halogen substituiertes C₃-C₆-Cycloalkyl, ein gegebenenfalls ein- oder mehrfach, gleich oder verschieden mit R^{c} subsituiertes Aryl, ein gegebenenfalls ein- oder mehrfach, gleich oder verschieden mit R^{c} substituiertes 5- oder 6-gliedriges Heteroaryl oder NHR^{a} steht;
m für 0, 1, 2 oder 3 steht und
R³ und R⁴ die weiter oben angegebenen Bedeutungen haben und
* für die Verknüpfungsstelle der Gruppe mit dem Rest des Moleküls steht.

4. Verbindungen gemäß Anspruch 1, worin W für eine Gruppe der allgemeinen Formel (X) steht, 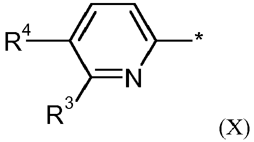 und R³ und R⁴ die in Anspruch 1 angegebene Bedeutung haben.

5. Verbindungen gemäß Anspruch 1, worin Y eine Gruppe der allgemeinen Formel (II) ist mit den in Anspruch 1 angegeben Definitionen für R^{7a}, R^{7b}, R^{7c}, R^{7d}, R^{8a}, R^{8b}, R^{8c} und R^{8d}:

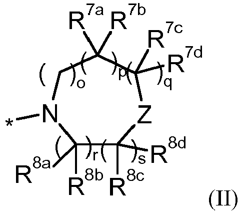

6. Verbindungen gemäß Anspruch 1, worin Y ein Rest NR⁵R⁶ ist mit den in Anspruch 1 angegeben Definitionen für R⁵ und R⁶.

7. Verbindungen gemäß Anspruch 1, worin W für eine Gruppe der allgemeinen Formel (IX) steht, 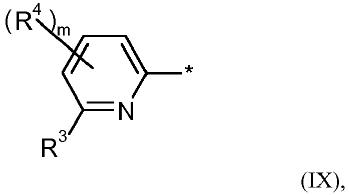 worin
m für 0 steht und R², R⁰ sowie R¹³ gleichzeitig Wasserstoff sowie R³ Trifluormethyl, Ethyl, Methyl, Cyclopropyl, 2,2,2-Trifluor-1-hydroxyethyl oder 1-Hydroxyethyl bedeuten; Y für 4-Methylpiperazin-1-yl, 4-Ethylpiperazin-1-yl oder Morpholin-4-yl steht, n für 0 und R¹ für Cyclopropylmethoxy, Trifluormethoxy, 2,2,2-Trifluorethoxy, Chlor, Ethoxy, Methoxy, 2-Hydroxypropan-2-yl oder 3-Hydroxypentan-3-yl steht.

8. Verbindungen gemäß Anspruch 7, worin R¹ für Cyclopropylmethoxy, Methoxy, Ethoxy oder 2-Hydroxypropan-2-yl steht.

9. Verbindungen gemäß Anspruch 7, worin R³ ein Trifluormethyl- oder ein Cyclopropylrest ist.

10. Verbindungen gemäß Anspruch 1 nämlich
N- {2-[2-(4-Benzoylpiperazin-1-yl)-2-oxoethyl]-6-methyl-2H-indazol-5-yl}-6-(trifluormethyl)pyridin-2-carboxamid
6-Ethyl-N-(6-methyl-2-{2-oxo-2-[4-(pyrrolidin-1-yl)piperidin-1-yl]ethyl}-2H-indazol-5-yl)pyridin-2-carboxamid
5-Fluor-N-(6-methyl-2-{2-oxo-2-[4-(pyrrolidin-1-yl)piperidin-1-yl]ethyl}-2H-indazol-5-yl)pyridin-2-carboxamid
N-(2-{2-[4-(3-Hydroxy-2,2-dimethylpropanoyl)piperazin-1-yl]-2-oxoethyl}-6-methyl-2H-indazol-5-yl)-6-(trifluormethyl)pyridin-2-carboxamid
N-(2-{2-[4-(Methoxyacetyl)piperazin-1-yl]-2-oxoethyl}-6-methyl-2H-indazol-5-yl)-6-(trifluormethyl)pyridin-2-carboxamid
N-(2-{2-[4-(2-Hydroxypropan-2-yl)piperidin-1-yl]-2-oxoethyl}-6-methoxy-2H-indazol-5-yl)-6-(trifluormethyl)pyridin-2-carboxamid
N-(2-{2-[4-(2-Hydroxypropan-2-yl)piperidin-1-yl]-2-oxoethyl}-6-methoxy-2H-indazol-5-yl)-6-methylpyridin-2-carboxamid
N-(2-{2-[4-(Cyclopropylmethyl)piperazin-1-yl]-2-oxoethyl}-6-methoxy-2H-indazol-5-yl)-6-(trifluormethyl)pyridin-2-carboxamid
N-(2-{2-[4-(Cyclopropylmethyl)piperazin-1-yl]-2-oxoethyl}-6-methoxy-2H-indazol-5-yl)-6-methylpyridin- 2-carboxamid
N-{2-[2-(4-Benzoylpiperazin-1-yl)-2-oxoethyl]-6-methoxy-2H-indazol-5-yl}-6-cyclopropylpyridin- 2-carboxamid
N-{2-[2-(4-Benzoylpiperazin-1-yl)-2-oxoethyl]-6-methoxy-2H-indazol-5-yl}-6-(1-hydroxyethyl)pyridin-2-carboxamid
N-{2-[2-(4-Methylpiperazin-1-yl)-2-oxoethyl]-6-(trifluormethoxy)-2H-indazol-5-yl}-6-(trifluormethyl)pyridin-2-carboxamid
6-Methyl-N-{2-[2-(4-methylpiperazin-1-yl)-2-oxoethyl]-6-(trifluormethoxy)-2H-indazol-5-yl}pyridin-2-carboxamid
tert-Butyl-3-{[4-({2-[2-(4-methylpiperazin-1-yl)-2-oxoethyl]-6-(trifluormethoxy)-2H-indazol-5-yl}carbamoyl)-1,3-thiazol-2-yl]amino}azetidin-1-carboxylat
N-{6-Brom-2-[2-(4-methylpiperazin-1-yl)-2-oxoethyl]-2H-indazol-5-yl}-6-(trifluormethyl)pyridin-2-carboxamid
N-{6-Brom-2-[2-(4-methylpiperazin-1-yl)-2-oxoethyl]-2H-indazol-5-yl}-6-methylpyridin-2-carboxamid
N-{6-Brom-2-[2-(4-methylpiperazin-1-yl)-2-oxoethyl]-2H-indazol-5-yl}-2-cyclopropyl-1,3-oxazol-4-carboxamid
tert-Butyl-3-{[4-({6-brom-2-[2-(4-methylpiperazin-1-yl)-2-oxoethyl]-2H-indazol-5-yl}carbamoyl)-1,3-thiazol-2-yl]amino}azetidin-1-carboxylat
2-(Azetidin-3-ylamino)-N-{2-[2-(4-methylpiperazin-1-yl)-2-oxoethyl]-6-(trifluormethoxy)-2H-indazol-5-yl}-1,3-thiazol-4-carboxamid
N-{6-Cyan-2-[2-(4-methylpiperazin-1-yl)-2-oxoethyl]-2H-indazol-5-yl}-6-(trifluormethyl)pyridin-2-carboxamid
6'-Methyl-N-{2-[2-(4-methylpiperazin-1-yl)-2-oxoethyl]-2H-indazol-5-yl}-2,3'-bipyridin-6-carboxamid
5'-Methyl-N-{2-[2-(4-methylpiperazin-1-yl)-2-oxoethyl]-2H-indazol-5-yl}-2,3'-bipyridin-6-carboxamid
4'-Methyl-N-{2-[2-(4-methylpiperazin-1-yl)-2-oxoethyl]-2H-indazol-5-yl}-2,3'-bipyridin-6-carboxamid
6'-Methoxy-N-{2-[2-(4-methylpiperazin-1-yl)-2-oxoethyl]-2H-indazol-5-yl}-2,3'-bipyridin-6-carboxamid
6'-Acetamido-N-{2-[2-(4-methylpiperazin-1-yl)-2-oxoethyl]-2H-indazol-5-yl}-2,3'-bipyridin-6-carboxamid
N-{2-[2-(4-Methylpiperazin-1-yl)-2-oxoethyl]-2H-indazol-5-yl}-6'-nitro-2,3'-bipyridin-6-carboxamid
6'-Amino-N-{2-[2-(4-methylpiperazin-1-yl)-2-oxoethyl]-2H-indazol-5-yl}-2,3'-bipyridin-6-carboxamid
N-{2-[2-(4-Benzoylpiperazin-1-yl)-2-oxoethyl]-6-fluor-2H-indazol-5-yl}-6-(trifluormethyl)pyridin-2-carboxamid
N-(2-{2-[4-(Cyclopropylcarbonyl)piperazin-1-yl]-2-oxoethyl}-6-fluor-2H-indazol-5-yl)-6-(trifluormethyl)pyridin-2-carboxamid
N-{6-Fluor-2-[2-(4-methylpiperazin-1-yl)-2-oxoethyl]-2H-indazol-5-yl}-6-(trifluormethyl)pyridin-2-carboxamid
N-(2-{2-[4-(Cyclopropylcarbonyl)piperazin-1-yl]-2-oxoethyl}-6-fluor-2H-indazol-5-yl)-6-methylpyridin-2-carboxamid
N-{6-Fluor-2-[2-(4-methylpiperazin-1-yl)-2-oxoethyl]-2H-indazol-5-yl}-6-methylpyridin-2-carboxamid
N-(2-{2-[4-(Cyclopropylcarbonyl)piperazin-1-yl]-2-oxoethyl}-6-fluor-2H-indazol-5-yl)-6-(1-methyl-1H-pyrazol-4-yl)pyridin-2-carboxamid
N-{2-[2-(4-Benzoylpiperazin-1-yl)-2-oxoethyl]-6-fluor-2H-indazol-5-yl}-6-(1-methyl-1H-pyrazol-4-yl)pyridin-2-carboxamid
N-{6-Fluor-2-[2-(4-methylpiperazin-1-yl)-2-oxoethyl]-2H-indazol-5-yl}-6-(1-methyl-1H-pyrazol-4-yl)pyridin-2-carboxamid
N-{2-[2-(4-Benzoylpiperazin-1-yl)-2-oxoethyl]-6-fluor-2H-indazol-5-yl}-5-fluor-6-(1-methyl-1H-pyrazol-4-yl)pyridin-2-carboxamid
N-(2-{2-[4-(Cyclopropylcarbonyl)piperazin-1-yl]-2-oxoethyl}-6-fluor-2H-indazol-5-yl)-5-fluor-6-(1-methyl-1H-pyrazol-4-yl)pyridin-2-carboxamid
N-{2-[2-(4-Benzoylpiperazin-1-yl)-2-oxoethyl]-6-fluor-2H-indazol-5-yl}-6-(morpholin-4-yl)pyridin-2-carboxamid
N-(2-{2-[4-(Cyclopropylcarbonyl)piperazin-1-yl]-2-oxoethyl}-6-fluor-2H-indazol-5-yl)-6-(morpholin-4-yl)pyridin-2-carboxamid
N-{6-Fluor-2-[2-(4-methylpiperazin-1-yl)-2-oxoethyl]-2H-indazol-5-yl}-6-(morpholin-4-yl)pyridin-2-carboxamid
N-{6-(Benzyloxy)-2-[2-(4-methylpiperazin-1-yl)-2-oxoethyl]-2H-indazol-5-yl}-6-(trifluormethyl)pyridin-2-carboxamid
N-{6-Isobutoxy-2-[2-(4-methylpiperazin-1-yl)-2-oxoethyl]-2H-indazol-5-yl}-6-(trifluormethyl)pyridin-2-carboxamid
N-{6-Isobutoxy-2-[2-(morpholin-4-yl)-2-oxoethyl]-2H-indazol-5-yl}-6-(trifluormethyl)pyridin-2-carboxamid
N-(2-{2-[4-(2-Hydroxypropan-2-yl)piperidin-1-yl]-2-oxoethyl}-6-isobutoxy-2H-indazol-5-yl)-6-(trifluormethyl)pyridin-2-carboxamid
N-(2-{2-[(Cyclopropylmethyl)(methyl)amino]-2-oxoethyl}-6-isobutoxy-2H-indazol-5-yl)-6-(trifluormethyl)pyridin-2-carboxamid
N-{6-(Cyclopropylmethoxy)-2-[2-(4-methylpiperazin-1-yl)-2-oxoethyl]-2H-indazol-5-yl}-6-(trifluormethyl)pyridin-2-carboxamid
N-{6-(Cyclopropylmethoxy)-2-[2-(morpholin-4-yl)-2-oxoethyl]-2H-indazol-5-yl}-6-(trifluormethyl)pyridin-2-carboxamid
N-[6-(Cyclopropylmethoxy)-2-{2-[4-(2-hydroxypropan-2-yl)piperidin-1-yl]-2-oxoethyl}-2H-indazol-5-yl]-6-(trifluormethyl)pyridin-2-carboxamid
N-[6-(Cyclopropylmethoxy)-2-{2-[(cyclopropylmethyl)(methyl)amino]-2-oxoethyl}-2H-indazol-5-yl]-6-(trifluormethyl)pyridin-2-carboxamid
N-{2-[2-(4-Methylpiperazin-1-yl)-2-oxoethyl]-6-(pyridin-2-ylmethoxy)-2H-indazol-5-yl}-6-(trifluormethyl)pyridin-2-carboxamid
N-{2-[2-(Morpholin-4-yl)-2-oxoethyl]-6-(pyridin-2-ylmethoxy)-2H-indazol-5-yl}-6-(trifluormethyl)pyridin-2-carboxamid
N-[2-{2-[4-(2-Hydroxypropan-2-yl)piperidin-1-yl]-2-oxoethyl}-6-(pyridin-2-ylmethoxy)-2H-indazol-5-yl]-6-(trifluormethyl)pyridin-2-carboxamid
N-[2-{2-[(Cyclopropylmethyl)(methyl)amino]-2-oxoethyl}-6-(pyridin-2-ylmethoxy)-2H-indazol-5-yl]-6-(trifluormethyl)pyridin-2-carboxamid
N-{2-[2-(4-Benzoylpiperazin-1-yl)-2-oxoethyl]-6-chlor-2H-indazol-5-yl}-6-(trifluormethyl)pyridin-2-carboxamid
N-{6-Chlor-2-[2-(morpholin-4-yl)-2-oxoethyl]-2H-indazol-5-yl}-6-(trifluormethyl)pyridin-2-carboxamid
Ethyl-4-{[6-chlor-5-({[6-(trifluormethyl)pyridin-2-yl]carbonyl}amino)-2H-indazol-2-yl] acetyl} piperazin-1-carboxylat
N-(6-Chlor-2-{2-oxo-2-[4-(pyrrolidin-1-yl)piperidin-1-yl]ethyl}-2H-indazol-5-yl)-6-(trifluormethyl)pyridin-2-carboxamid
N-(6-Chlor-2-{2-[4-(2-hydroxypropan-2-yl)piperidin-1-yl]-2-oxoethyl}-2H-indazol-5-yl)-6-(trifluormethyl)pyridin-2-carboxamid
N-(6-Chlor-2-{2-[4-(3-hydroxy-2,2-dimethylpropanoyl)piperazin-1-yl]-2-oxoethyl}-2H-indazol-5-yl)-6-(trifluormethyl)pyridin-2-carboxamid
N-(6-Chlor-2-{2-[3-(dimethylamino)azetidin-1-yl]-2-oxoethyl}-2H-indazol-5-yl)-6-(trifluormethyl)pyridin-2-carboxamid
N-(6-Chlor-2-{2-oxo-2-[3-(piperidin-1-yl)azetidin-1-yl]ethyl}-2H-indazol-5-yl)-6-(trifluormethyl)pyridin-2-carboxamid
N-(6-Chlor-2-{2-[4-(2-hydroxy-2-methylpropyl)piperidin-1-yl]-2-oxoethyl}-2H-indazol-5-yl)-6-(trifluormethyl)pyridin-2-carboxamid
N-{6-Chlor-2-[2-(4-hydroxy-1,4'-bipiperidin-1'-yl)-2-oxoethyl]-2H-indazol-5-yl}-6-(trifluormethyl)pyridin-2-carboxamid
N-{6-Methoxy-2-[2-(4-methylpiperazin-1-yl)-2-oxoethyl]-2H-indazol-5-yl}-6-(trifluormethyl)pyridin-2-carboxamid
N-{6-Methoxy-2-[2-(morpholin-4-yl)-2-oxoethyl]-2H-indazol-5-yl}-6-(trifluormethyl)pyridin-2-carboxamid
N-(2-{2-[4-(Dimethylamino)piperidin-1-yl]-2-oxoethyl}-6-ethoxy-2H-indazol-5-yl)-6-(trifluormethyl)pyridin-2-carboxamid
N-(6-Ethoxy-2-{2-oxo-2-[4-(pyrrolidin-1-yl)piperidin-1-yl]ethyl}-2H-indazol-5-yl)-6-(trifluormethyl)pyridin-2-carboxamid
N-{6-Ethoxy-2-[2-(4-methylpiperazin-1-yl)-2-oxoethyl]-2H-indazol-5-yl}-6-(trifluormethyl)pyridin-2-carboxamid
N-{2-[2-(4-Benzoylpiperazin-1-yl)-2-oxoethyl]-6-ethoxy-2H-indazol-5-yl}-6-(trifluormethyl)pyridin-2-carboxamid
N-(6-Ethoxy-2-{2-[4-(2-hydroxypropan-2-yl)piperidin-1 -yl]-2-oxoethyl}-2H-indazol-5-yl)-6-(trifluormethyl)pyridin-2-carboxamid
N-{6-Ethoxy-2-[2-(morpholin-4-yl)-2-oxoethyl]-2H-indazol-5-yl}-6-(trifluormethyl)pyridin-2-carboxamid
N-{2-[2-(4-Benzoylpiperazin-1-yl)-2-oxoethyl]-3-methyl-2H-indazol-5-yl}-6-(trifluormethyl)pyridin-2-carboxamid
N-{2-[3-(4-Benzoylpiperazin-1-yl)-3-oxopropyl]-2H-indazol-5-yl}-6-(trifluormethyl)pyridin-2-carboxamid
N-(2-{2-[4-(Cyclopropylcarbonyl)piperazin-1-yl]-2-oxoethyl}-2H-indazol-5-yl)-2-(pyridin-3-yl)-1,3-thiazol-4-carboxamid
N-(2-{2-[4-(Cyclopropylcarbonyl)piperazin-1-yl]-2-oxoethyl}-2H-indazol-5-yl)-2-(pyridin-4-yl)-1,3-thiazol-4-carboxamid
N-(2-{2-[4-(Cyclopropylcarbonyl)piperazin-1-yl]-2-oxoethyl}-2H-indazol-5-yl)-6-(trifluormethyl)pyridin-2-carboxamid
6-(Azetidin-3-ylamino)-N-(2-{2-[4-(cyclopropylcarbonyl)piperazin-1-yl]-2-oxoethyl}-2H-indazol-5-yl)pyridin-2-carboxamid
N-{2-[2-(4-Methylpiperazin-1-yl)-2-oxoethyl]-2H-indazol-5-yl}-2-(pyridin-3-yl)-1,3-thiazol-4-carboxamid
N-{2-[2-(4-Methylpiperazin-1-yl)-2-oxoethyl]-2H-indazol-5-yl}-6-(1-methyl-1H-pyrazol-4-yl)pyridin-2-carboxamid
N-{2-[2-(4-Methylpiperazin-1-yl)-2-oxoethyl]-2H-indazol-5-yl}-6-(1H-pyrazol-4-yl)pyridin-2-carboxamid
6-(1,3-Dimethyl-1H-pyrazol-4-yl)-N-{2-[2-(4-methylpiperazin-1-yl)-2-oxoethyl]-2H-indazol-5-yl}pyridin-2-carboxamid
N-{2-[2-(4-Methylpiperazin-1-yl)-2-oxoethyl]-2H-indazol-5-yl}-6-[3-(trifluormethyl)-1H-pyrazol-4-yl]pyridin-2-carboxamid
6-Ethyl-N-{2-[2-(4-methylpiperazin-1-yl)-2-oxoethyl]-2H-indazol-5-yl}pyridin-2-carboxamid
6-(1-Methyl-1H-pyrazol-4-yl)-N-(2-{2-oxo-2-[4-(2,2,2-trifluorethyl)piperazin-1-yl]ethyl}-2H-indazol-5-yl)pyridin-2-carboxamid
N-(2-{2-Oxo-2-[4-(2,2,2-trifluorethyl)piperazin-1-yl]ethyl}-2H-indazol-5-yl)-6-(trifluormethyl)pyridin-2-carboxamid
N-{2-[2-(4-Ethyl-3-oxopiperazin-1-yl)-2-oxoethyl]-2H-indazol-5-yl}-6-(1-methyl-1H-pyrazol-4-yl)pyridin-2-carboxamid
N-{2-[2-(4-Benzoylpiperazin-1-yl)-2-oxoethyl]-2H-indazol-5-yl}-6-(trifluormethyl)pyridin-2-carboxamid
N-{2-[2-(4-Benzoylpiperazin-1-yl)-2-oxoethyl]-2H-indazol-5-yl}-6-methylpyridin-2-carboxamid
N-{2-[2-(4-Benzoylpiperazin-1-yl)-2-oxoethyl]-2H-indazol-5-yl}-6-(morpholin-4-yl)pyridin-2-carboxamid
N-{2-[2-(4-Benzoylpiperazin-1-yl)-2-oxoethyl]-2H-indazol-5-yl}-2-(pyridin-4-yl)-1,3-thiazol-4-carboxamid
N-{2-[2-(4-Benzoylpiperazin-1-yl)-2-oxoethyl]-2H-indazol-5-yl}-6-chlorpyridin-2-carboxamid
N-{2-[2-(4-Benzoylpiperazin-1-yl)-2-oxoethyl]-2H-indazol-5-yl}-2-methyl-1,3-oxazol-5-carboxamid
6-Amino-N-{2-[2-(4-benzoylpiperazin-1-yl)-2-oxoethyl]-2H-indazol-5-yl}pyridin-2-carboxamid
N-{2-[2-(4-Benzoylpiperazin-1-yl)-2-oxoethyl]-2H-indazol-5-yl}-2-methyl-1,3-oxazol-4-carboxamid
N-{2-[2-(4-Benzoylpiperazin-1-yl)-2-oxoethyl]-2H-indazol-5-yl}-6-methoxypyridin-2-carboxamid
N-{2-[2-(4-Benzoylpiperazin-1-yl)-2-oxoethyl]-2H-indazol-5-yl}-2-cyclopropyl-1,3-oxazol-4-carboxamid
N-{2-[2-(4-Benzoylpiperazin-1-yl)-2-oxoethyl]-2H-indazol-5-yl}-6-(4H-1,2,4-triazol-4-yl)pyridin-2-carboxamid
N-{2-[2-(4-Benzoylpiperazin-1-yl)-2-oxoethyl]-2H-indazol-5-yl}-2-phenyl-2H-1,2,3-triazol-4-carboxamid
N-{2-[2-(4-Benzoylpiperazin-1-yl)-2-oxoethyl]-2H-indazol-5-yl}-6-(1-methyl-1H-pyrazol-5-yl)pyridin-2-carboxamid
N-{2-[2-(4-Benzoylpiperazin-1-yl)-2-oxoethyl]-2H-indazol-5-yl} -2-(trifluormethyl)-1,3-thiazol-4-carboxamid
N-{2-[2-(4-Benzoylpiperazin-1-yl)-2-oxoethyl]-2H-indazol-5-yl}-6-(1H-pyrazol-1-yl)pyridin-2-carboxamid
N-{2-[2-(4-Benzoylpiperazin-1-yl)-2-oxoethyl]-2H-indazol-5-yl}-6-(1-methyl-1H-pyrazol-4-yl)pyridin-2-carboxamid
N-{2-[2-(4-Benzoylpiperazin-1-yl)-2-oxoethyl]-2H-indazol-5-yl}-1-ethyl-1H-pyrazol-3-carboxamid
N-{2-[2-(4-Benzoylpiperazin-1-yl)-2-oxoethyl]-2H-indazol-5-yl}-6-(4-chlor-1H-pyrazol-1-yl)pyridin-2-carboxamid
N-{2-[2-(4-Benzoylpiperazin-1-yl)-2-oxoethyl]-2H-indazol-5-yl}-4-(trifluormethyl)-1,3-thiazol-2-carboxamid
N-{2-[2-(4-Benzoylpiperazin-1-yl)-2-oxoethyl]-2H-indazol-5-yl}-6-(1,3-dimethyl-1H-pyrazol-4-yl)pyridin-2-carboxamid
N-{2-[2-(4-Benzoylpiperazin-1-yl)-2-oxoethyl]-2H-indazol-5-yl}-2,4'-bipyridin-6-carboxamid
N-{2-[2-(4-Benzoylpiperazin-1-yl)-2-oxoethyl]-2H-indazol-5-yl}-6-(1H-pyrazol-4-yl)pyridin-2-carboxamid
N-{2-[2-(4-Benzoylpiperazin-1-yl)-2-oxoethyl]-2H-indazol-5-yl}-5-fluor-6-(1-methyl-1H-pyrazol-4-yl)pyridin-2-carboxamid
N-{2-[2-(4-Benzoylpiperazin-1-yl)-2-oxoethyl]-2H-indazol-5-yl}-6-(3-methyl-1H-pyrazol-4-yl)pyridin-2-carboxamid
N-{2-[2-(4-Benzoylpiperazin-1-yl)-2-oxoethyl]-2H-indazol-5-yl}-6-(1H-1,2,4-triazol-1-yl)pyridin-2-carboxamid
N-{2-[2-(4-Benzoylpiperazin-1-yl)-2-oxoethyl]-2H-indazol-5-yl}-6-[3-(trifluormethyl)-1H-pyrazol-4-yl]pyridin-2-carboxamid
N-{2-[2-(4-Benzoylpiperazin-1-yl)-2-oxoethyl]-2H-indazol-5-yl}-6-ethoxypyridin-2-carboxamid
N-{2-[2-(4-Benzoylpiperazin-1-yl)-2-oxoethyl]-2H-indazol-5-yl}-6-(cyclopropylmethoxy)pyridin-2-carboxamid
N-{2-[2-(4-Benzoylpiperazin-1-yl)-2-oxoethyl]-2H-indazol-5-yl}-6-ethylpyridin-2-carboxamid
N-{2-[2-(4-Benzoylpiperazin-1-yl)-2-oxoethyl]-2H-indazol-5-yl}-2-(4-methoxyphenyl)-1,3-thiazol-4-carboxamid
N-{2-[2-(4-Benzoylpiperazin-1-yl)-2-oxoethyl]-2H-indazol-5-yl}-2-brom-1,3-thiazol-4-carboxamid
N-{2-[2-(4-Benzoylpiperazin-1-yl)-2-oxoethyl]-2H-indazol-5-yl}-2-(4-fluorphenyl)-1,3-thiazol-4-carboxamid
N-{2-[2-(4-Benzoylpiperazin-1-yl)-2-oxoethyl]-2H-indazol-5-yl}-6-fluorpyridin-2-carboxamid
N-{2-[2-(4-Benzoylpiperazin-1-yl)-2-oxoethyl]-2H-indazol-5-yl}-6-brompyridin-2-carboxamid
N-(2-{2-[4-(4-Fluorbenzoyl)piperazin-1-yl]-2-oxoethyl}-2H-indazol-5-yl)-6-(trifluormethyl)pyridin-2-carboxamid
N-(2-{2-Oxo-2-[4-(pyridin-2-yl)piperazin-1-yl]ethyl}-2H-indazol-5-yl)-6-(trifluormethyl)pyridin-2-carboxamid
N-(2-{2-[4-(Methoxyacetyl)piperazin-1-yl]-2-oxoethyl}-2H-indazol-5-yl)-6-(trifluormethyl)pyridin-2-carboxamid
N-{2-[2-(4-Cyclopentyl-3-oxopiperazin-1-yl)-2-oxoethyl]-2H-indazol-5-yl}-6-(trifluormethyl)pyridin-2-carboxamid
N-{2-[2-Oxo-2-(3-oxo-4-phenylpiperazin-1-yl)ethyl]-2H-indazol-5-yl}-6-(trifluormethyl)pyridin-2-carboxamid
N-(2-{2-[4-(2,2-Dimethylpropanoyl)piperazin-1-yl]-2-oxoethyl}-2H-indazol-5-yl)-6-(trifluormethyl)pyridin-2-carboxamid
N-(2-{2-[4-(Cyclopropylmethyl)piperazin-1-yl]-2-oxoethyl}-2H-indazol-5-yl)-6-(trifluormethyl)pyridin-2-carboxamid
N-{2-[2-Oxo-2-(pyridazin-4-ylamino)ethyl]-2H-indazol-5-yl}-6-(trifluormethyl)pyridin-2-carboxamid
N-(2-{2-[4-(2-Hydroxy-2-methylpropanoyl)piperazin-1-yl]-2-oxoethyl}-2H-indazol-5-yl)-6-(trifluormethyl)pyridin-2-carboxamid
N-(2-{2-Oxo-2-[4-(1-phenylethyl)piperazin-1-yl]ethyl}-2H-indazol-5-yl)-6-(trifluormethyl)pyridin-2-carboxamid
N-(2-{2-Oxo-2-[4-(pyridin-3-ylcarbonyl)piperazin-1-yl]ethyl}-2H-indazol-5-yl)-6-(trifluormethyl)pyridin-2-carboxamid
N-{2-[2-(4-Isonicotinoylpiperazin-1-yl)-2-oxoethyl]-2H-indazol-5-yl}-6-(trifluormethyl)pyridin-2-carboxamid
N-(2-{2-[4-(Morpholin-4-ylcarbonyl)piperazin-1-yl]-2-oxoethyl}-2H-indazol-5-yl)-6-(trifluormethyl)pyridin-2-carboxamid
N-[2-(2-{4-[2-(Methylamino)-2-oxoethyl]piperazin-1-yl}-2-oxoethyl)-2H-indazol-5-yl]-6-(trifluormethyl)pyridin-2-carboxamid
N-(2-{2-Oxo-2-[4-(pyrazin-2-yl)piperazin-1-yl]ethyl}-2H-indazol-5-yl)-6-(trifluormethyl)pyridin-2-carboxamid
N-(2-{2-[4-(1-Hydroxyethyl)piperidin-1-yl]-2-oxoethyl}-2H-indazol-5-yl)-6-(trifluormethyl)pyridin-2-carboxamid
N-{2-[2-(2-Methyl-2,8-diazaspiro[4.5]dec-8-yl)-2-oxoethyl]-2H-indazol-5-yl}-6-(trifluormethyl)pyridin-2-carboxamid
N-{2-[2-(6-Acetyl-2,6-diazaspiro[3.3]hept-2-yl)-2-oxoethyl]-2H-indazol-5-yl}-6-(trifluormethyl)pyridin-2-carboxamid
N-{2-[2-Oxo-2-(3-oxo-2,8-diazaspiro[4.5]dec-8-yl)ethyl]-2H-indazol--5yl}-6-(trifluormethyl)pyridin-2-carboxamid
N-{2-[2-(6-Methyl-2,6-diazaspiro[3.S]non-2-yl)-2-oxoethyl]-2H-indazol-5-yl}-6-(trifluormethyl)pyridin-2-carboxamid
N-{2-[2-(7-Oxa-2-azaspiro[3.5]non-2-yl)-2-oxoethyl]-2H-indazol-5-yl}-6-(trifluormethyl)pyridin-2-carboxamid
N-{2-[2-(1,4'-Bipiperidin-1'-yl)-2-oxoethyl]-2H-indazol-5-yl}-6-(trifluormethyl)pyridin-2-carboxamid
N-(2-{2-[2-(Hydroxymethyl)piperidin-1-yl]-2-oxoethyl} -2H-indazol-5-yl)-6-(trifluormethyl)pyridin-2-carboxamid
N-(2-{2-[3-(Hydroxymethyl)piperidin-1-yl]-2-oxoethyl}-2H-indazol-5-yl)-6-(trifluormethyl)pyridin-2-carboxamid
N-{2-[2-(4-Carbamoylpiperidin-1-yl)-2-oxoethyl]-2H-indazol-5-yl}-6-(trifluormethyl)pyridin-2-carboxamid
N-(2-{2-[3-(Dimethylamino)piperidin-1-yl]-2-oxoethyl}-2H-indazol-5-yl)-6-(trifluormethyl)pyridin-2-carboxamid
N-(2-{2-[3-(Morpholin-4-ylmethyl)piperidin-1-yl]-2-oxoethyl}-2H-indazol-5-yl)-6-(trifluormethyl)pyridin-2-carboxamid
N-[2-(2-{4-[(Cyclopropylcarbonyl)amino]piperidin-1-yl}-2-oxoethyl)-2H-indazol-5-yl]-6-(trifluormethyl)pyridin-2-carboxamid
N-(2-{2-[4-(3-Ethyl-1,2,4-oxadiazol-5-yl)piperidin-1-yl]-2-oxoethyl}-2H-indazol-5-yl)-6-(trifluormethyl)pyridin-2-carboxamid
N-[2-(2-{4-[(5-Cyclopropyl-1,2,4-oxadiazol-3-yl)methyl]piperidin-1-yl}-2-oxoethyl)-2H-indazol-5-yl]-6-(trifluormethyl)pyridin-2-carboxamid
N-(2-{2-Oxo-2-[4-(pyrrolidin-1-ylcarbonyl)piperidin-1-yl]ethyl}-2H-indazol-5-yl)-6-(trifluormethyl)pyridin-2-carboxamid
N-(2-{2-[4-(4-Methylpiperazin-1-yl)piperidin-1-yl]-2-oxoethyl}-2H-indazol-5-yl)-6-(trifluormethyl)pyridin-2-carboxamid
N-[2-(2-{4-[2-(Morpholin-4-yl)ethyl]piperidin-1-yl}-2-oxoethyl)-2H-indazol-5-yl]-6-(trifluormethyl)pyridin-2-carboxamid
N-[2-(2-{4-[(5-Methyl-1,2,4-oxadiazol-3-yl)methyl]piperidin-1-yl}-2-oxoethyl)-2H-indazol-5-yl]-6-(trifluormethyl)pyridin-2-carboxamid
N-(2-{2-Oxo-2-[3-(pyrrolidin-1-ylmethyl)piperidin-1-yl]ethyl}-2H-indazol-5-yl)-6-(trifluormethyl)pyridin-2-carboxamid
N-[2-(2-{[3-(Dimethylsulfamoyl)phenyl]amino}-2-oxoethyl)-2H-indazol-5-yl]-6-(trifluormethyl)pyridin-2-carboxamid
N-{2-[2-(1,2-Oxazol-4-ylamino)-2-oxoethyl]-2H-indazol-5-yl}-6-(trifluormethyl)pyridin-2-carboxamid
N-(2-{2-[4-(Methylsulfonyl)piperidin-1-yl]-2-oxoethyl}-2H-indazol-5-yl)-6-(trifluormethyl)pyridin-2-carboxamid
N-[2-(2-Oxo-2-{4-[2-oxo-2-(pyrrolidin-1-yl)ethyl]piperazin-1-yl}ethyl)-2H-indazol-5-yl]-6-(trifluormethyl)pyridin-2-carboxamid
N-(2-{2-Oxo-2-[4-(phenylsulfonyl)piperidin-1-yl]ethyl}-2H-indazol-5-yl)-6-(trifluormethyl)pyridin-2-carboxamid
N-(2-{2-Oxo-2-[(3-sulfamoylphenyl)amino]ethyl}-2H-indazol-5-yl)-6-(trifluormethyl)pyridin-2-carboxamid
N-[2-(2-{4-[Isonicotinoyl(methyl)amino]piperidin-1-yl}-2-oxoethyl)-2H-indazol-5-yl]-6-(trifluormethyl)pyridin-2-carboxamid
N-[2-(2-{4-[2-(Isopropylamino)-2-oxoethyl]piperazin-1-yl}-2-oxoethyl)-2H-indazol-5-yl]-6-(trifluormethyl)pyridin-2-carboxamid
N-(2-{2-[4-(1,1-Dioxidotetrahydrothiophen-3 -yl)piperazin-1-yl]-2-oxoethyl}-2H-indazol-5-yl)-6-(trifluormethyl)pyridin-2-carboxamid
N-[2-(2-{4-[(Methoxyacetyl)(methyl)amino]piperidin-1-yl}-2-oxoethyl)-2H-indazol-5-yl]-6-(trifluormethyl)pyridin-2-carboxamid
Ethyl-4-{[5-({[6-(trifluormethyl)pyridin-2-yl]carbonyl}amino)-2H-indazol-2-yl] acetyl} piperazin-1-carboxylat
N-(2-{2-[4-(Cyclohexylcarbonyl)piperazin-1-yl]-2-oxoethyl}-2H-indazol-5-yl)-6-(trifluormethyl)pyridin-2-carboxamid
N-[2-(2-{4-[2-(Cyclopropylamino)-2-oxoethyl]piperazin-1-yl}-2-oxoethyl)-2H-indazol-5-yl]-6-(trifluormethyl)pyridin-2-carboxamid
N-(2-{2-[2-(2-Hydroxyethyl)piperidin-1-yl]-2-oxoethyl}-2H-indazol-5-yl)-6-(trifluormethyl)pyridin-2-carboxamid
N-(2-{2-Oxo-2-[4-(pyrrolidin-1-yl)piperidin-1-yl]ethyl}-2H-indazol-5-yl)-6-(trifluormethyl)pyridin-2-carboxamid
N-(2-{2-Oxo-2-[4-(1H-pyrrol-1-yl)piperidin-1-yl]ethyl}-2H-indazol-5-yl)-6-(trifluormethyl)pyridin-2-carboxamid
N-(2-{2-[4-(3-Hydroxypropyl)piperazin-1-yl]-2-oxoethyl}-2H-indazol-5-yl)-6-(trifluormethyl)pyridin-2-carboxamid
4-{[5-({[6-(Trifluormethyl)pyridin-2-yl]carbonyl}amino)-2H-indazol-2-yl]acetyl}piperazin-1-carboxamid
N-(2-{2-Oxo-2-[4-(2-oxopyrrolidin-1-yl)piperidin-1-yl]ethyl}-2H-indazol-5-yl)-6-(trifluormethyl)pyridin-2-carboxamid
N-{2-[2-(Morpholin-4-yl)-2-oxoethyl]-2H-indazol-5-yl}-6-(trifluormethyl)pyridin-2-carboxamid
N-(2-{2-[4-(2-Amino-2-oxoethyl)piperazin-1-yl]-2-oxoethyl}-2H-indazol-5-yl)-6-(trifluormethyl)pyridin-2-carboxamid
N-{2-[2-(1,1-Dioxidothiomorpholin-4-yl)-2-oxoethyl]-2H-indazol-5-yl}-6-(trifluormethyl)pyridin-2-carboxamid
N-{2-[2-(4-Isopropylpiperazin-1-yl)-2-oxoethyl]-2H-indazol-5-yl}-6-(trifluormethyl)pyridin-2-carboxamid
N-(2-{2-Oxo-2-[4-(2-thienylcarbonyl)piperazin-1-yl]ethyl}-2H-indazol-5-yl)-6-(trifluormethyl)pyridin-2-carboxamid
N-(2-{2-[4-(2-Cyclopropyl-2-oxoethyl)piperazin-1-yl]-2-oxoethyl}-2H-indazol-5-yl)-6-(trifluormethyl)pyridin-2-carboxamid
N-[2-(2-{4-[(1-Methyl-1H-pyrazol-4-yl)methyl]piperazin-1-yl} -2-oxoethyl)-2H-indazol-5-yl]-6-(trifluormethyl)pyridin-2-carboxamid
N-[2-(2- {4-[(1,5-Dimethyl-1H-pyrazol-3-yl)carbonyl]piperazin-1-yl} -2-oxoethyl)-2H-indazol-5-yl]-6-(trifluormethyl)pyridin-2-carboxamid
N,N-Diethyl-4-{[5-({[6-(trifluormethyl)pyridin-2-yl]carbonyl}amino)-2H-indazol-2-yl] acetyl} piperazin-1 -carboxamid
N-{2-[2-Oxo-2-(thiomorpholin-4-yl)ethyl]-2H-indazol-5-yl}-6-(trifluormethyl)pyridin-2-carboxamid
N-(2-{2-[-yl]-2-oxoethyl} -2H-indazol-5-yl)-6-(trifluormethyl)pyridin-2-carboxamid
N-(2-{2-Oxo-2-[4-(3-thienylmethyl)piperazin-1-yl]ethyl}-2H-indazol-5-yl)-6-(trifluormethyl)pyridin-2-carboxamid
N-{2-[2-(4'-Methyl-1,4'-bipiperidin-1'-yl)-2-oxoethyl]-2H-indazol-5-yl}-6-(trifluormethyl)pyridin-2-carboxamid
N-{2-[2-(6-Methyl-2,6-diazaspiro[3.3]hept-2-yl)-2-oxoethyl]-2H-indazol-5-yl}-6-(trifluormethyl)pyridin-2-carboxamid
N-{2-[2-(4-Cyclopentylpiperazin-1-yl)-2-oxoethyl]-2H-indazol-5-yl}-6-(trifluormethyl)pyridin-2-carboxamid
N-[2-(2-{4-[2-(2-Hydroxyethoxy)ethyl]piperazin-1-yl}-2-oxoethyl)-2H-indazol-5-yl]-6-(trifluormethyl)pyridin-2-carboxamid
N-(2-{2-Oxo-2-[4-(pyridin-4-ylmethyl)piperazin-1-yl]ethyl}-2H-indazol-5-yl)-6-(trifluormethyl)pyridin-2-carboxamid
N-(2-{2-[4-(Dimethylsulfamoyl)piperazin-1-yl]-2-oxoethyl}-2H-indazol-5-yl)-6-(trifluormethyl)pyridin-2-carboxamid
N-(2-{2-Oxo-2-[4-(pyridin-4-yl)piperazin-1-yl]ethyl}-2H-indazol-5-yl)-6-(trifluormethyl)pyridin-2-carboxamid
N-(2-{2-[4-(Methylsulfonyl)piperazin-1-yl]-2-oxoethyl}-2H-indazol-5-yl)-6-(trifluormethyl)pyridin-2-carboxamid
Ameisensäure--N-[2-(2-{4-[2-(1H-imidazol-1-yl)ethyl]piperazin-1-yl}-2-oxoethyl)-2H-indazol-5-yl]-6-(trifluormethyl)pyridin-2-carboxamid(1:1)
N-(2-{2-[4-(Diethylsulfamoyl)piperazin-1-yl]-2-oxoethyl}-2H-indazol-5-yl)-6-(trifluormethyl)pyridin-2-carboxamid
N-(2-{2-Oxo-2-[-yl]ethyl}-2H-indazol-5-yl)-6-(trifluormethyl)pyridin-2-carboxamid
N-(2-{2-Oxo-2-[4-(piperidin-1-ylsulfonyl)piperazin-1-yl]ethyl}-2H-indazol-5-yl)-6-(trifluormethyl)pyridin-2-carboxamid
N-[2-(2-{4-[(1,5-Dimethyl-1H-pyrazol-4-yl)sulfonyl]piperazin-1-yl} -2-oxoethyl)-2H-indazol-5-yl]-6-(trifluormethyl)pyridin-2-carboxamid
N-(2-{2-[4-(Cyclopropylmethyl)piperazin-1-yl]-2-oxoethyl}-2H-indazol-5-yl)-6-(1-methyl-1H-pyrazol-4-yl)pyridin-2-carboxamid
N-(2-{2-[4-(2-Hydroxypropan-2-yl)piperidin-1-yl]-2-oxoethyl}-2H-indazol-5-yl)-6-(1-methyl-1H-pyrazol-4-yl)pyridin-2-carboxamid
6-(1-Methyl-1H-pyrazol-4-yl)-N-(2-{2-oxo-2-[4-(pyrrolidin-1-yl)piperidin-1-yl]ethyl}-2H-indazol-5-yl)pyridin-2-carboxamid
N-{2-[2-(4-Ethylpiperazin-1-yl)-2-oxoethyl]-2H-indazol-5-yl}-6-(1-methyl-1H-pyrazol-4-yl)pyridin-2-carboxamid
N-(2-{2-[4-(Dimethylamino)piperidin-1-yl]-2-oxoethyl}-2H-indazol-5-yl)-6-(1-methyl-1H-pyrazol-4-yl)pyridin-2-carboxamid
N-(2-{2-[(Cyclopropylmethyl)(methyl)amino]-2-oxoethyl}-6-methoxy-2H-indazol-5-yl)-6-methylpyridin-2-carboxamid
N-(2-{2-[(Cyclopropylmethyl)(methyl)amino]-2-oxoethyl}-6-ethoxy-2H-indazol-5-yl)-6-(trifluormethyl)pyridin-2-carboxamid
N-(2-{2-[(Cyclopropylmethyl)(methyl)amino]-2-oxoethyl}-6-methoxy-2H-indazol-5-yl)-6-(trifluormethyl)pyridin-2-carboxamid
6-Cyclopropyl-N-(2-{2-[4-(2-hydroxypropan-2-yl)piperidin-1-yl]-2-oxoethyl}-6-methoxy-2H-indazol-5-yl)pyridin-2-carboxamid
6-(1-Hydroxyethyl)-N-(2-{2-[4-(2-hydroxypropan-2-yl)piperidin-1-yl]-2-oxoethyl}-6-methoxy-2H-indazol-5-yl)pyridin-2-carboxamid
6-(Azetidin-3-ylamino)-N-{2-[2-(4-benzoylpiperazin-1-yl)-2-oxoethyl]-2H-indazol-5-yl}pyridin-2-carboxamid
6-[(Azetidin-2-ylmethyl)amino]-N-{2-[2-(4-benzoylpiperazin-1-yl)-2-oxoethyl]-2H-indazol-5-yl}pyridin-2-carboxamid
N-{2-[2-(4-Benzoylpiperazin-1-yl)-2-oxoethyl]-2H-indazol-5-yl}-6-(3-hydroxyazetidin-1-yl)pyridin-2-carboxamid
6-[(2R,6S)-2,6-Dimethylmorpholin-4-yl]-N-(6-methyl-2-{2-oxo-2-[4-(pyrrolidin-1-yl)piperidin-1-yl]ethyl}-2H-indazol-5-yl)pyridin-2-carboxamid
N-[2-(2-{4-Methyl-4-[(4-methylpiperazin-1-yl)carbonyl]piperidin-1-yl}-2-oxoethyl)-2H-indazol-5-yl]-6-(trifluormethyl)pyridin-2-carboxamid
N-(6-Chlor-2-{2-oxo-2-[(3R)-piperidin-3-ylamino]ethyl}-2H-indazol-5-yl)-6-(trifluormethyl)pyridin-2-carboxamid
N-(2-{2-[4-(Cyclopropylcarbonyl)piperazin-1-yl]-2-oxoethyl}-6-isopropoxy-2H-indazol-5-yl)-6-methylpyridin-2-carboxamid
N-(2-{2-[4-(Cyclopropylcarbonyl)piperazin-1-yl]-2-oxoethyl}-6-isopropoxy-2H-indazol-5-yl)-6-(trifluormethyl)pyridin-2-carboxamid
N-{2-[2-(4-Benzoylpiperazin-1-yl)-2-oxoethyl]-6-isopropoxy-2H-indazol-5-yl}-6-methylpyridin-2-carboxamid
N-{2-[2-(4-Benzoylpiperazin-1-yl)-2-oxoethyl]-6-isopropoxy-2H-indazol-5-yl}-6-(trifluormethyl)pyridin-2-carboxamid
N-{6-Isopropoxy-2-[2-(4-methylpiperazin-1-yl)-2-oxoethyl]-2H-indazol-5-yl}-6-(trifluormethyl)pyridin-2-carboxamid
N-{6-Isopropoxy-2-[2-(4-methylpiperazin-1-yl)-2-oxoethyl]-2H-indazol-5-yl}-6-methylpyridin-2-carboxamid
N-(2-{2-[4-(Cyclobutylcarbonyl)piperazin-1-yl]-2-oxoethyl}-2H-indazol-5-yl)-6-(trifluormethyl)pyridin-2-carboxamid
N-(2-{2-[4-(Cyclopentylcarbonyl)piperazin-1-yl]-2-oxoethyl}-2H-indazol-5-yl)-6-(trifluormethyl)pyridin-2-carboxamid
N-[2-(2-{4-[3-(Methylsulfonyl)benzoyl]piperazin-1-yl}-2-oxoethyl)-2H-indazol-5-yl]-6-(trifluormethyl)pyridin-2-carboxamid
N-[2-(2-{4-[2-Methoxy-5-(methylsulfonyl)benzoyl]piperazin-1-yl}-2-oxoethyl)-2H-indazol-5-yl]-6-(trifluormethyl)pyridin-2-carboxamid
6-Brom-N-(6-methyl-2-{2-oxo-2-[4-(pyrrolidin-1-yl)piperidin-1-yl]ethyl}-2H-indazol-5-yl)pyridin-2-carboxamid
2-(4-Methoxyphenyl)-N-(6-methyl-2-{2-oxo-2-[4-(pyrrolidin-1-yl)piperidin-1-yl]ethyl}-2H-indazol-5-yl)-1,3-thiazol-4-carboxamid
2-(4-Fluorphenyl)-N-(6-methyl-2-{2-oxo-2-[4-(pyrrolidin-1-yl)piperidin-1-yl] ethyl}-2H-indazol-5-yl)-1,3-thiazol-4-carboxamid
N-(6-Methyl-2-{2-oxo-2-[4-(pyrrolidin-1-yl)piperidin-1-yl]ethyl}-2H-indazol-5-yl)-6-(trifluormethyl)pyridin-2-carboxamid
6-Brom-N-{2-[2-(4-methylpiperazin-1-yl)-2-oxoethyl]-2H-indazol-5-yl}pyridin-2-carboxamid
6-Brom-N- {2-[2-(4-methylpiperazin-1-yl)-2-oxoethyl] -6-(trifluormethoxy)-2H-indazol-5-yl}pyridin-2-carboxamid
N-{2-[2-(4-Methylpiperazin-1-yl)-2-oxoethyl]-6-(trifluormethoxy)-2H-indazol-5-yl}-6-(4H-1,2,4-triazol-4-yl)pyridin-2-carboxamid
2-Brom-N-{6-brom-2-[2-(4-methylpiperazin-1-yl)-2-oxoethyl]-2H-indazol-5-yl}-1,3-thiazol-4-carboxamid
N-{6-Hydroxy-2-[2-(4-methylpiperazin-1-yl)-2-oxoethyl]-2H-indazol-5-yl}-6-(trifluormethyl)pyridin-2-carboxamid
N-[6-(Benzyloxy)-2-{2-[4-(2-hydroxypropan-2-yl)piperidin-1-yl]-2-oxoethyl}-2H-indazol-5-yl]-6-methylpyridin-2-carboxamid
6-Brom-N- {6-brom-2-[2-(4-methylpiperazin-1-yl)-2-oxoethyl]-2H-indazol-5-yl}pyridin-2-carboxamid
N-{6-(Benzyloxy)-2-[2-(4-methylpiperazin-1-yl)-2-oxoethyl]-2H-indazol-5-yl}-6-methylpyridin-2-carboxamid
2-(Azetidin-3-ylamino)-N-{2-[2-(4-benzoylpiperazin-1-yl)-2-oxoethyl]-2H-indazol-5-yl}-1,3-thiazol-4-carboxamid
6-Acetamido-N-{6-methoxy-2-[2-(morpholin-4-yl)-2-oxoethyl]-2H-indazol-5-yl}pyridin-2-carboxamid
6-(Dimethylamino)-N-(2-{2-[4-(2-hydroxypropan-2-yl)piperidin-1-yl]-2-oxoethyl}-6-methoxy-2H-indazol-5-yl)pyridin-2-carboxamid
6-(Dimethylamino)-N-{6-methoxy-2-[2-(morpholin-4-yl)-2-oxoethyl]-2H-indazol-5-yl}pyridin-2-carboxamid
6-Acetamido-N-(2-{2-[4-(2-hydroxypropan-2-yl)piperidin-1-yl]-2-oxoethyl}-6-methoxy-2H-indazol-5-yl)pyridin-2-carboxamid
6-(Dimethylamino)-N-{6-methoxy-2-[2-(4-methylpiperazin-1-yl)-2-oxoethyl]-2H-indazol-5-yl}pyridin-2-carboxamid
N-{2-[2-(4-Methylpiperazin-1-yl)-2-oxoethyl]-2H-indazol-5-yl}-6-[3-(methylsulfonyl)phenyl] pyridin-2-carboxamid
N-{2-[1-(4-Benzoylpiperazin-1-yl)-1-oxopropan-2-yl]-2H-indazol-5-yl}-6-(trifluormethyl)pyridin-2-carboxamid
N-[6-Chlor-2-(2-{[trans-4-(2-hydroxypropan-2-yl)cyclohexyl]amino}-2-oxoethyl)-2H-indazol-5-yl]-6-(trifluormethyl)pyridin-2-carboxamid
6-(2-Hydroxypropan-2-yl)-N-{6-methoxy-2-[2-(morpholin-4-yl)-2-oxoethyl]-2H-indazol-5-yl} pyridin-2-carboxamid
N-{6-Chlor-2-[2-(3,3-difluorpyrrolidin-1-yl)-2-oxoethyl]-2H-indazol-5-yl}-6-(trifluormethyl)pyridin-2-carboxamid
N-{6-Chlor-2-[2-oxo-2-(pyrrolidin-1-yl)ethyl]-2H-indazol-5-yl}-6-(trifluormethyl)pyridin-2-carboxamid
N-{6-Chlor-2-[2-(2-oxa-7-azaspiro[3.5]non-7-yl)-2-oxoethyl]-2H-indazol-5-yl}-6-(trifluormethyl)pyridin-2-carboxamid
N-(6-Chlor-2-{2-[4-(2-hydroxy-2-methylpropyl)piperazin-1-yl]-2-oxoethyl}-2H-indazol-5-yl)-6-(trifluormethyl)pyridin-2-carboxamid
N-{6-Methoxy-2-[2-oxo-2-(pyrrolidin-1-yl)ethyl]-2H-indazol-5-yl}-6-(trifluormethyl)pyridin-2-carboxamid
N-{2-[2-(3,3-Difluorpyrrolidin-1-yl)-2-oxoethyl]-6-methoxy-2H-indazol-5-yl}-6-(trifluormethyl)pyridin-2-carboxamid
6-(Difluormethyl)-N-{6-methoxy-2-[2-(morpholin-4-yl)-2-oxoethyl]-2H-indazol-5-yl}pyridin-2-carboxamid
N-{2-[2-(3,3-Difluorpyrrolidin-1-yl)-2-oxoethyl]-6-methoxy-2H-indazol-5-yl}-6-methylpyridin-2-carboxamid
N-{6-Methoxy-2-[2-(morpholin-4-yl)-2-oxoethyl]-2H-indazol-5-yl}-6-methylpyridin-2-carboxamid
N-{6-Methoxy-2-[2-(morpholin-4-yl)-2-oxoethyl]-2H-indazol-5-yl}-2-(tetrahydro-2H-pyran-4-yl)-1,3-oxazol-4-carboxamid
N-{2-[2-(1,1-Dioxido-1-thia-6-azaspiro[3.3]hept-6-yl)-2-oxoethyl]-6-methoxy-2H-indazol-5-yl}-6-(trifluormethyl)pyridin-2-carboxamid
N-{6-Methoxy-2-[2-(2-oxa-6-azaspiro[3.3]hept-6-yl)-2-oxoethyl]-2H-indazol-5-yl}-6-(trifluormethyl)pyridin-2-carboxamid
N-{6-(3-Hydroxy-2,2-dimethylpropoxy)-2-[2-(morpholin-4-yl)-2-oxoethyl]-2H-indazol-5-yl}-6-(trifluormethyl)pyridin-2-carboxamid
6-Ethyl-N-{6-methoxy-2-[2-(morpholin-4-yl)-2-oxoethyl]-2H-indazol-5-yl}pyridin-2-carboxamid
6-Isobutyl-N-{6-methoxy-2-[2-(morpholin-4-yl)-2-oxoethyl]-2H-indazol-5-yl}pyridin-2-carboxamid
Methyl-2-[2-(morpholin-4-yl)-2-oxoethyl]-5-({[6-(trifluormethyl)pyridin-2-yl]carbonyl}amino)-2H-indazol-6-carboxylat
Methyl-5-{[(6-methylpyridin-2-yl)carbonyl]amino}-2-[2-(morpholin-4-yl)-2-oxoethyl]-2H-indazol-6-carboxylat
N-{6-Methoxy-2-[2-(morpholin-4-yl)-2-oxoethyl]-2H-indazol-5-yl}-6-(pyrrolidin-1-yl)pyridin-2-carboxamid
N-{6-Methoxy-2-[2-(morpholin-4-yl)-2-oxoethyl]-2H-indazol-5-yl}-6-(morpholin-4-yl)pyridin-2-carboxamid
6-(Cyclopropylamino)-N-{6-methoxy-2-[2-(morpholin-4-yl)-2-oxoethyl]-2H-indazol-5-yl}pyridin-2-carboxamid
6-(Butylamino)-N-{6-methoxy-2-[2-(morpholin-4-yl)-2-oxoethyl]-2H-indazol-5-yl}pyridin-2-carboxamid
N-{6-Methoxy-2-[2-(morpholin-4-yl)-2-oxoethyl]-2H-indazol-5-yl}-6-(propylamino)pyridin-2-carboxamid
6-(Isobutylamino)-N-{6-methoxy-2-[2-(morpholin-4-yl)-2-oxoethyl]-2H-indazol-5-yl}pyridin-2-carboxamid
R-N-{6-Methoxy-2-[2-(morpholin-4-yl)-2-oxoethyl]-2H-indazol-5-yl}-6-(2,2,2-trifluor-1-hydroxyethyl)pyridin-2-carboxamid
S-N-{6-Methoxy-2-[2-(morpholin-4-yl)-2-oxoethyl]-2H-indazol-5-yl}-6-(2,2,2-trifluor-1-hydroxyethyl)pyridin-2-carboxamid
6-(1-Hydroxyethyl)-N-{6-methoxy-2-[2-(morpholin-4-yl)-2-oxoethyl]-2H-indazol-5-yl}pyridin-2-carboxamid
6-(Cyclopropylamino)-N-{6-methoxy-2-[2-(4-methylpiperazin-1-yl)-2-oxoethyl]-2H-indazol-5-yl}pyridin-2-carboxamid
N-{6-Methoxy-2-[2-(4-methylpiperazin-1-yl)-2-oxoethyl]-2H-indazol-5-yl}-6-(propylamino)pyridin-2-carboxamid
6-(Isobutylamino)-N-{6-methoxy-2-[2-(4-methylpiperazin-1-yl)-2-oxoethyl]-2H-indazol-5-yl}pyridin-2-carboxamid
6-(1-Hydroxyethyl)-N-{6-methoxy-2-[2-(4-methylpiperazin-1-yl)-2-oxoethyl]-2H-indazol-5-yl}pyridin-2-carboxamid
N-{6-Methoxy-2-[2-(4-methylpiperazin-1-yl)-2-oxoethyl]-2H-indazol-5-yl}-4-methyl-6-(trifluormethyl)pyridin-2-carboxamid
N-{6-(Benzyloxy)-2-[2-(morpholin-4-yl)-2-oxoethyl]-2H-indazol-5-yl}-6-(trifluormethyl)pyridin-2-carboxamid
6-(Cyclopropylamino)-N-(2-{2-[4-(2-hydroxypropan-2-yl)piperidin-1-yl]-2-oxoethyl}-6-methoxy-2H-indazol-5-yl)pyridin-2-carboxamid
6-(Butylamino)-N-(2-{2-[4-(2-hydroxypropan-2-yl)piperidin-1-yl]-2-oxoethyl}-6-methoxy-2H-indazol-5-yl)pyridin-2-carboxamid
N-(2-{2-[4-(2-Hydroxypropan-2-yl)piperidin-1-yl]-2-oxoethyl}-6-methoxy-2H-indazol-5-yl)-6-[(2-methoxyethyl)amino]pyridin-2-carboxamid
N-(2-{2-[4-(2-Hydroxypropan-2-yl)piperidin-1-yl]-2-oxoethyl}-6-methoxy-2H-indazol-5-yl)-6-(propylamino)pyridin-2-carboxamid
N-(2-{2-[4-(2-Hydroxypropan-2-yl)piperidin-1-yl]-2-oxoethyl}-6-methoxy-2H-indazol-5-yl)-6-(isobutylamino)pyridin-2-carboxamid
5-Fluor-N-(2-{2-[4-(2-hydroxypropan-2-yl)piperidin-1-yl]-2-oxoethyl}-6-methoxy-2H-indazol-5-yl)-6-methylpyridin-2-carboxamid
N-{6-Hydroxy-2-[2-(morpholin-4-yl)-2-oxoethyl]-2H-indazol-5-yl}-6-(trifluormethyl)pyridin-2-carboxamid
N-{6-(3-Cyanpropoxy)-2-[2-(morpholin-4-yl)-2-oxoethyl]-2H-indazol-5-yl}-6-(trifluormethyl)pyridin-2-carboxamid
N-{2-[2-(Morpholin-4-yl)-2-oxoethyl]-6-(2,2,2-trifluorethoxy)-2H-indazol-5-yl}-6-(trifluormethyl)pyridin-2-carboxamid
N-{6-(Cyclohexylmethoxy)-2-[2-(morpholin-4-yl)-2-oxoethyl]-2H-indazol-5-yl}-6-(trifluormethyl)pyridin-2-carboxamid
N-{6-(2,2-Dimethylpropoxy)-2-[2-(morpholin-4-yl)-2-oxoethyl]-2H-indazol-5-yl}-6-(trifluormethyl)pyridin-2-carboxamid
N-{2-[2-(Morpholin-4-yl)-2-oxoethyl]-6-(tetrahydrofuran-2-ylmethoxy)-2H-indazol-5-yl}-6-(trifluormethyl)pyridin-2-carboxamid
N-{6-(Cyclopentyloxy)-2-[2-(morpholin-4-yl)-2-oxoethyl]-2H-indazol-5-yl}-6-(trifluormethyl)pyridin-2-carboxamid
N-{6-(Cyanmethoxy)-2-[2-(morpholin-4-yl)-2-oxoethyl]-2H-indazol-5-yl}-6-(trifluormethyl)pyridin-2-carboxamid
({2-[2-(Morpholin-4-yl)-2-oxoethyl]-5-({[6-(trifluormethyl)pyridin-2-yl]carbonyl}amino)-2H-indazol-6-yl}oxy)essigsäure
N-{6-(Cyclobutylmethoxy)-2-[2-(morpholin-4-yl)-2-oxoethyl]-2H-indazol-5-yl}-6-(trifluormethyl)pyridin-2-carboxamid
N-{2-[2-(Morpholin-4-yl)-2-oxoethyl]-6-[2-(pyrrolidin-1-yl)ethoxy]-2H-indazol-5-yl}-6-(trifluormethyl)pyridin-2-carboxamid
N-{6-[2-(Morpholin-4-yl)ethoxy]-2-[2-(morpholin-4-yl)-2-oxoethyl]-2H-indazol-5-yl}-6-(trifluormethyl)pyridin-2-carboxamid
N-{2-[2-(Morpholin-4-yl)-2-oxoethyl]-6-[2-(piperidin-1-yl)ethoxy]-2H-indazol-5-yl}-6-(trifluormethyl)pyridin-2-carboxamid
N-{6-(3-Hydroxypropoxy)-2-[2-(morpholin-4-yl)-2-oxoethyl]-2H-indazol-5-yl}-6-(trifluormethyl)pyridin-2-carboxamid
N-{6-(2-Hydroxypropoxy)-2-[2-(morpholin-4-yl)-2-oxoethyl]-2H-indazol-5-yl}-6-(trifluormethyl)pyridin-2-carboxamid
N-{6-(2-Hydroxyethoxy)-2-[2-(morpholin-4-yl)-2-oxoethyl]-2H-indazol-5-yl}-6-(trifluormethyl)pyridin-2-carboxamid
N-{6-(2-Methoxyethoxy)-2-[2-(morpholin-4-yl)-2-oxoethyl]-2H-indazol-5-yl}-6-(trifluormethyl)pyridin-2-carboxamid
Ethyl-({2-[2-(morpholin-4-yl)-2-oxoethyl]-5-({[6-(trifluormethyl)pyridin-2-yl]carbonyl}amino)-2H-indazol-6-yl} oxy)acetat
Methyl-4-({2-[2-(morpholin-4-yl)-2-oxoethyl]-5-({[6-(trifluormethyl)pyridin-2-yl]carbonyl}amino)-2H-indazol-6-yl}oxy)butanoat
Ethyl-2-({2-[2-(morpholin-4-yl)-2-oxoethyl]-5-({[6-(trifluormethyl)pyridin-2-yl]carbonyl}amino)-2H-indazol-6-yl}oxy)propanoat
Ethyl-3-methyl-2-({2-[2-(morpholin-4-yl)-2-oxoethyl]-5-({[6-(trifluormethyl)pyridin-2-yl]carbonyl}amino)-2H-indazol-6-yl}oxy)butanoat
2-({2-[2-(Morpholin-4-yl)-2-oxoethyl]-5-({[6-(trifluormethyl)pyridin-2-yl]carbonyl}amino)-2H-indazol-6-yl}oxy)propansäure
N-{6-(2-Hydroxypropan-2-yl)-2-[2-(morpholin-4-yl)-2-oxoethyl]-2H-indazol-5-yl}-6-(trifluormethyl)pyridin-2-carboxamid
N-{6-Chlor-2-[2-(4-methylpiperazin-1-yl)-2-oxoethyl]-2H-indazol-5-yl}-6-(difluormethyl)pyridin-2-carboxamid
N-{6-Chlor-2-[2-(morpholin-4-yl)-2-oxoethyl]-2H-indazol-5-yl}-6-(difluormethyl)pyridin-2-carboxamid

11. Verbindung der Formel (I), wie in einem der Ansprüche 1 bis 10 definiert, zur Behandlung und/oder Prophylaxe von Krankheiten.

12. Verbindung der Formel (I), wie in einem der Ansprüche 1 bis 10 definiert, zur Verwendung in einem Verfahren zur Behandlung und/oder Prophylaxe von Tumorerkrankungen, dermatologischen Erkrankungen, gynäkologischen Erkrankungen, kardiovaskulären Erkrankungen, pulmonalen Erkrankungen, ophthalmologischen Erkrankungen, neurologischen Erkrankungen,, Stoffwechselerkrankungen, inflammatorischen Erkrankungen, Autoimmunerkrankungen und Schmerz.

13. Verbindung der Formel (I), wie in einem der Ansprüche 1 bis 10 definiert, zur Verwendung in einem Verfahren zur Behandlung und/oder Prophylaxe von Lymphomen, Makuladegeneration, Endometriose, Psoriasis, Lupus erythematodes, Multipler Sklerose, COPD, rheumatoide Arthritis.

14. Verwendung einer Verbindung der Formel (I), wie in einem der Ansprüche 1 bis 10 definiert, zur Herstellung eines Arzneimittels zur Behandlung und/oder Prophylaxe von Tumorerkrankungen, dermatologischen Erkrankungen, gynäkologischen Erkrankungen, kardiovaskulären Erkrankungen, pulmonalen Erkrankungen, ophthalmologischen Erkrankungen, neurologischen Erkrankungen, Stoffwechselerkrankungen, inflammatorischen Erkrankungen, Autoimmunerkrankungen und Schmerz.

15. Verbindung der Formel (I), wie in einem der Ansprüche 1 bis 10 definiert, zur Herstellung eines Arzneimittels zur Behandlung und/oder Prophylaxe von Lymphomen, Makuladegeneration, Endometriose, Psoriasis, Lupus erythematodes, Multipler Sklerose, COPD, rheumatoide Arthritis.

16. Arzneimittel enthaltend eine Verbindung der Formel (I), wie in einem der Ansprüche 1 bis 10 definiert, in Kombination mit einem inerten, nicht-toxischen, pharmazeutisch geeigneten Hilfsstoff.

17. Verfahren zur Herstellung von Verbindungen der allgemeinen Formel (III) aus der Verbindung der allgemeinen Formel (II), 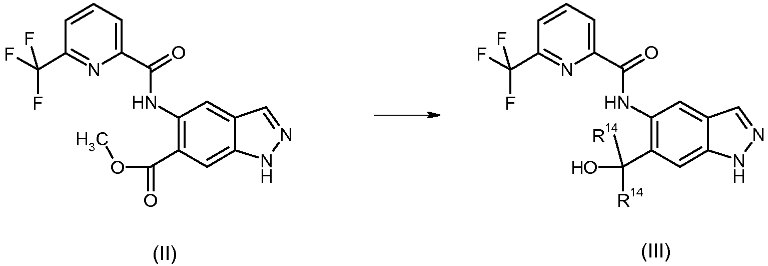 worin R¹⁴ entweder ein Methyl- oder Ethylrest ist durch Grignardreaktion mit Methyl- oder Ethylmagnesiumbromid.

18. Verbindungen der allgemeinen Formel (III) 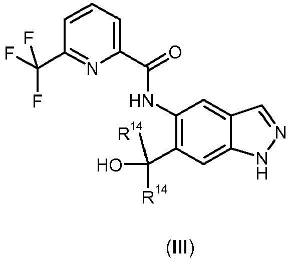 worin R¹⁴ entweder ein Methyl- oder Ethylrest ist.

## Claims

1. Compounds of the general formula (I) 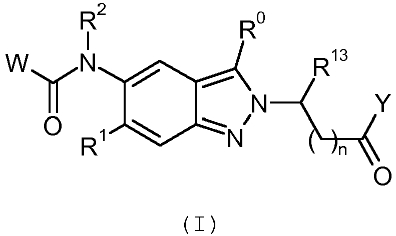 in which:
R⁰ represents hydrogen or C₁-C₄-alkyl, where the C₁-C₄-alkyl radical may optionally be mono- or polysubstituted by identical or different radicals from the group consisting of hydroxy and halogen;
R¹ represents hydrogen, halogen, cyano, C(=O)OH, C(=O)OR^{a}, C(=O)NH₂, C(=O)N(H)R^{a}, C(=O)N(R^{a})R^{b}, C(=O)R^{d}, hydroxy or C₁-C₆-alkyl, where the C₁-C₆-alkyl radical is optionally mono- or polysubstituted by identical or different radicals from the group consisting of
hydroxy, halogen, cyano, C(=O)OH, C(=O)OR^{a}, S(=O)₂-C₁-C₆-alkyl, NH₂, NHR^{a}, N(R^{a})R^{b}, C₁-C₆-alkoxy which is optionally mono- or polysubstituted by identical or different halogen atoms, C₃-C₈-cycloalkoxy which is optionally mono- or polysubstituted by identical or different halogen atoms, heterocycloalkyl which is optionally mono- or polysubstituted by identical or different radicals R^{C} ,
or represents C₁-C₆-alkoxy, where the C₁-C₆-alkoxy radical may optionally be mono- or polysubstituted by identical or different radicals from the group consisting of
hydroxy, halogen, cyano, C(=O)OH, C(=O)OR^{a}, S(=O)₂-C₁-C₆-alkyl, NH₂, NHR^{a}, N(R^{a})R^{b}, C₃-C₈-cycloalkyl which is optionally mono- or polysubstituted by identical or different halogen atoms, C₁-C₆-alkoxy which is optionally mono- or polysubstituted by identical or different halogen atoms, C₃-C₈-cycloalkoxy which is optionally mono- or polysubstituted by identical or different halogen atoms, heterocycloalkyl which is optionally mono- or polysubstituted by identical or different radicals R^{C} , aryl which is optionally mono- or polysubstituted by identical or different radicals R^{C} , and 5- or 6-membered heteroaryl which is optionally mono- or polysubstituted by identical or different radicals R^{C} ,
or represents C₃-C₈-cycloalkoxy or heterocycloalkoxy which may optionally be mono- or polysubstituted by identical or different radicals from the group consisting of hydroxy, halogen, cyano and C₁-C₆-alkyl,
or represents aryloxy or 5- or 6-membered heteroaryloxy in which aryloxy and
5- or 6-membered heteroaryloxy may optionally be mono- or polysubstituted by identical or different radicals from the group consisting of hydroxy, halogen, cyano, C(=O)OH, C(=O)OR^{a}, C₁-C₆-alkyl and C₁-C₆-alkoxy,
or represents C₃-C₈-cycloalkyl or heterocycloalkyl which may optionally be mono- or polysubstituted by identical or different radicals from the group consisting of hydroxy, halogen, cyano and C₁-C₆-alkyl,
or represents C₂-C₆-alkenyl or C₂-C₆-alkynyl,
or represents aryl, 5- to 10-membered heteroaryl, aryl-C₁-C₄-alkyl or 5- or 6-membered heteroaryl-C₁-C₄-alkyl, where aryl and heteroaryl may optionally be mono- or polysubstituted by identical or different radicals from the group consisting of halogen, hydroxy, cyano, C(=O)OH, C(=O)OR^{a}, C₁-C₆-alkyl, C₃-C₈-cycloalkyl and C₁-C₆-alkoxy;
R^{a} represents C₁-C₆-alkyl, C₃-C₁₀-cycloalkyl, heterocycloalkyl, aryl or heteroaryl, where alkyl, cycloalkyl, heterocycloalkyl, aryl and heteroaryl may optionally be mono- or polysubstituted by identical or different radicals from the group consisting of halogen, hydroxy, cyano, C₁-C₃-alkyl, C₁-C₃-alkoxy, heterocycloalkyl, -C(=O)O-C₁-C₆-alkyl and S(=O)₂-C₁-C₆-alkyl;
R^{b} represents C₁-C₆-alkyl or C₃-C₁₀-cycloalkyl;
or R^{a} and R^{b} together with the nitrogen atom form a 5- or 6-membered heterocycle which may optionally be mono- or polysubstituted by identical or different radicals from the group consisting of hydroxy, halogen, cyano, and C₁-C₆-alkyl;
R^{C} represents hydroxy, halogen, cyano, C₁-C₃-alkyl or C₁-C₃-alkoxy;
R^{d} represents hydrogen, C₁-C₆-alkyl or C₃-C₁₀-cycloalkyl;
R² represents hydrogen, C₁-C₆-alkyl or C₃-C₆-cycloalkyl;
R¹³ represents hydrogen or C₁-C₆-alkyl;
W represents 5-membered heteroaryl which contains one to three heteroatoms selected from the group consisting of N, O and S and may optionally be monosubstituted by R³ and optionally be mono- or polysubstituted by identical or different radicals R⁴ or
W represents pyridyl, pyrazinyl, pyridazinyl, 1,2,4-triazinyl or 1,3,5-triazinyl which may optionally be monosubstituted by R³ and optionally be mono- or polysubstituted by identical or different radicals R⁴;
R³ represents hydrogen, halogen, cyano, C(=O)R^{a}, NH₂, NHR^{a}, N(R^{a})R^{b}, N(H)C(=O)R^{a} or C₁-C₆-alkyl, where
C₁-C₆-alkyl may optionally be mono- or polysubstituted by identical or different radicals from the group consisting of hydroxy, halogen, cyano, C(=O)R^{a}, C(=O)OH, C(=O)OR^{a}, S(=O)₂-C₁-C₆-alkyl, NH₂, NHR^{a}, N(R^{a})R^{b}, C₁-C₆-alkoxy, C₃-C₈-cycloalkoxy, where C₁-C₆-alkoxy and C₃-C₈-cycloalkoxy may optionally be mono- or polysubstituted by identical or different halogen atoms;
or C₁-C₆-alkyl is optionally mono- or polysubstituted by identical or different radicals from the group consisting of C₃-C₆-cycloalkyl and heterocycloalkyl,
where C₃-C₆-cycloalkyl and heterocycloalkyl may optionally be mono-, di- or trisubstituted by identical or different radicals from the group consisting of halogen, cyano, C₁-C₃-alkyl and C₁-C₃-alkoxy,
or C₁-C₆-alkyl is optionally mono- or polysubstituted by identical or different radicals from the group consisting of aryl and 5- or 6-membered heteroaryl,
where aryl and 5- or 6-membered heteroaryl may optionally be mono-, di- or trisubstituted by identical or different radicals from the group consisting of halogen, cyano, C₁-C₃-alkyl and C₁-C₃-alkoxy,
or
R³ represents C₁-C₆-alkoxy, where
C₁-C₆-alkoxy may optionally be mono- or polysubstituted by identical or different radicals from the group consisting of hydroxy, halogen, cyano, C(=O)OR^{a}, S(=O)₂-C₁-C₆-alkyl, N(R^{a})R^{b}, C₃-C₈-cycloalkyl, C₁-C₄-alkoxy, C₃-C₈-cycloalkoxy,
or represents C₃-C₆-cycloalkyl, heterocycloalkyl or C₅-C₁₁-spirocycloalkyl, where cycloalkyl, heterocycloalkyl and spirocycloalkyl may optionally be mono- or polysubstituted by identical or different radicals from the group consisting of hydroxy, halogen, cyano, C(=O)R^{a}, C(=O)OH, C(=O)OR^{a}, C₁-C₆-alkyl and C₁-C₄-alkoxy;
or represents aryl or 5- to 10-membered heteroaryl, where
aryl and heteroaryl may optionally be mono- or polysubstituted by identical or different radicals from the group consisting of halogen, hydroxy, cyano, C(=O)OR^{a}, S(=O)₂-C₁-C₆-alkyl, NO₂, NH₂, NHR^{a}, N(R^{a})R^{b}, N(H)C(=O)R^{a}, C₃-C₈-cycloalkyl, C₁-C₃-alkoxy and C₁-C₃-alkyl, where C₁-C₃-alkyl may optionally be mono- or polysubstituted by identical or different halogen atoms;
R⁴ represents halogen, hydroxy, cyano or C₁-C₆-alkyl, where C₁-C₆-alkyl may optionally be mono- or polysubstituted by identical or different halogen atoms, C₁-C₆-alkoxy, where C₁-C₆-alkoxy may optionally be mono- or polysubstituted by identical or different halogen atoms , C₂-C₆-alkenyl, C₂-C₆-alkynyl, C₃-C₁₀-cycloalkyl, 3- to 10-membered heterocycloalkyl and aryl, where aryl may optionally be mono- or polysubstituted by identical or different radicals R,
or
R⁴ represents aryl or heteroaryl which may optionally be mono- or polysubstituted by identical or different radicals R,
or
R⁴ represents C(=O)R^{a}, C(=O)NH₂, C(=O)N(H)R^{a}, C(=O)N(R^{a})R^{b}, C(=O)OR^{a}, NH₂, NHR^{a}, N(R^{a})R^{b}, N(H)C(=O)R^{a}, N(R^{a})C(=O)R^{a}, N(H)C(=O)NH₂, N(H)C(=O)NHR^{a}, N(H)C(=O)N(R^{a})R^{b}, N(R^{a})C(=O)NH₂, N(R^{a})C(=O)NHR^{a}, N(R^{a})C(=O)N(R^{a})R^{b}, N(H)C(=O)OR^{a}, N(R^{a})C(=O)OR^{a}, NO₂, N(H)S(=O)R^{a}, N(R^{a})S(=O)R^{a}, N(H)S(=O)₂R^{a}, N(R^{a})S(=O)₂R^{a}, N=S(=O)(R^{a}) R^{b}, OC(=O)R^{a}, OC(=O)NH₂, OC(=O)NHR^{a}, OC(=O)N(R^{a})R^{b}, SH, SR^{a}, S(=O)R⁻, S(=O)₂R^{a}, S(=O)₂NH₂, S(=O)₂NHR^{a}, S(=O)₂N(R^{a})R^{b} or S(=O)(=N-R^{a})R^{b};
R represents halogen, cyano, C₁-C₆-alkyl, C₂-C₆-alkenyl, C₂-C₆-alkynyl, C₃-C₁₀-cycloalkyl, 3- to 10-membered heterocycloalkyl, aryl, heteroaryl, C(=O)R^{a}, C(=O)NH₂, C(=O)N(H)R^{a}, C(=O)N(R^{a})R^{b}, C(=O)OR^{a}, NH₂, NHR^{a}, N(R^{a})R^{b}, N(H)C(=O)R^{a}, N(R^{a})C(=O)R^{a}, N(H)C(=O)NH₂, N(H)C(=O)NHR^{a}, N(H)C(=O)N(R^{a})R^{b}, N(R^{a})C(=O)NH₂, N(R^{a})C(=O)NHR^{a}, N(R^{a})C(=O)N(R^{a})R^{b}, N(H)C(=O)OR^{a}, N(R^{a})C(=O)OR^{a}, NO₂, N(H)S(=O)R^{a}, N(R^{a})S(=O)R^{a}, N(H)S(=O)₂R^{a}, N(R^{a})S(=O)₂R^{a}, N=S(=O)(R^{a})R^{b}, OH, C₁-C₆-alkOxy, OC(=O)R^{a}, OC(=O)NH₂, OC(=O)NHR^{a}, OC(=O)N(R^{a})R^{b}, SH, SR^{a}, S(=O)R^{a}, S(=O)₂R^{a}, S(=O)₂NH₂, S(=O)₂NHR^{a}, S(=O)₂N(R^{a})R^{b} or S(=O)(=NR^{a})R^{b};
n represents 0 or 1;
Y represents a group selected from: 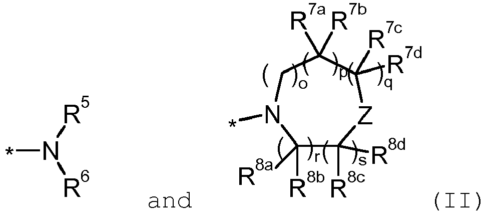
where * represents the point of attachment of the group to the remainder of the molecule;
R⁵ represents hydrogen, C₁-C₆-alkyl or C₃-C₁₀-cycloalkyl, where
C₁-C₆-alkyl may optionally be mono- or polysubstituted by identical or different radicals from the group consisting of hydroxy, halogen, cyano, C(=O)OH, C(=O)OR^{a}, S(=O)₂-C₁-C₆-alkyl, N(R^{a})R^{b}, C₁-C₄-alkoxy and C₃-C₈-cycloalkyl;
R⁶ represents hydrogen or C₁-C₆-alkyl, where
C₁-C₆-alkyl may optionally be mono- or polysubstituted by identical or different radicals from the group consisting of hydroxy, halogen, cyano, C₃-C₁₀-cycloalkyl, C(=O)R^{a}, C(=O)OH, C(=O)OR^{a}, S(=O)₂-C₁-C₆-alkyl, N(R^{a})R^{b}, C₁-C₄-alkoxy and C₃-C₈-cycloalkoxy,
or represents C₃-C₁₀-cycloalkyl, where
C₃-C₁₀-cycloalkyl may optionally be mono- or polysubstituted by identical or different radicals from the group consisting of hydroxy, halogen, cyano and C₁-C₆-alkyl, where
C₁-C₆-alkyl may optionally be substituted by hydroxy,
or represents heterocycloalkyl, where
heterocycloalkyl may optionally be mono- or polysubstituted by identical or different radicals from the group consisting of halogen, cyano, C₁-C₃-alkyl and C₁-C₃-alkoxy,
or represents aryl or 5- or 6-membered heteroaryl, where
aryl and 5- or 6-membered heteroaryl may optionally be mono- or polysubstituted by identical or different radicals from the group consisting of halogen, cyano, C₁-C₃-alkyl, C₁-C₃-alkoxy, S(=O)₂NH₂, S(=O)₂NHR^{a} and S(=O)₂N(R^{a})R^{b};
R^{7a} represents hydrogen, halogen, N(R^{a})R^{b}, C₁-C₆-alkyl or C₃-C₁₀-cycloalkyl, where
C₁-C₆-alkyl may optionally be mono- or polysubstituted by identical or different radicals from the group consisting of hydroxy, halogen, cyano, C(=O)OH, C(=O)OR^{a}, S(=O)₂-C₁-C₆-alkyl, N(R^{a})R^{b}, C₁-C₄-alkoxy, C₃-C₈-cycloalkyl and heterocycloalkyl;
R^{7b} represents hydrogen, halogen or C₁-C₆-alkyl, where
C₁-C₆-alkyl may optionally be mono- or polysubstituted by identical or different radicals from the group consisting of hydroxy, halogen, cyano, C(=O)OH, C(=O)OR^{a}, S(=O)₂-C₁-C₆-alkyl, N(R^{a})R^{b}, C₁-C₄-alkoxy, C₃-C₈-cycloalkyl and heterocycloalkyl;
or R^{7a} and R^{7b} together with the carbon atom form C₃-C₆-cycloalkyl which may optionally be mono- or polysubstituted by identical or different radicals from the group consisting of hydroxy, halogen, cyano and C₁-C₆-alkyl,
or R^{7a} and R^{7b} together represent an oxo group;
R^{7c} represents hydrogen, halogen, N(R^{a})R^{b}, C₁-C₆-alkyl or C₃-C₁₀-cycloalkyl, where
C₁-C₆-alkyl may optionally be mono- or polysubstituted by identical or different radicals from the group consisting of hydroxy, halogen, cyano, C(=O)OH, C(=O)OR^{a}, S(=O)₂-C₁-C₆-alkyl, N(R^{a})R^{b}, C₁-C₄-alkoxy, C₃-C₈-cycloalkyl and heterocycloalkyl;
R^{7d} represents hydrogen, halogen or C₁-C₆-alkyl, where
C₁-C₆-alkyl may optionally be mono- or polysubstituted by identical or different radicals from the group consisting of hydroxy, halogen, cyano, C(=O)OH, C(=O)OR^{a}, S(=O)₂-C₁-C₆-alkyl, N(R^{a})R^{b}, C₁-C₄-alkoxy, C₃-C₈-cycloalkyl and heterocycloalkyl;
or R^{7c} and R^{7d} together with the carbon atom form C₃-C₆-cycloalkyl which may optionally be mono- or polysubstituted by identical or different radicals from the group consisting of hydroxy, halogen, cyano and C₁-C₆-alkyl,
or R^{7c} and R^{7d} together represent an oxo group;
R^{8a} represents hydrogen, halogen, N(R^{a})R^{b}, C₁-C₆-alkyl or C₃-C₁₀-cycloalkyl, where
C₁-C₆-alkyl may optionally be mono- or polysubstituted by identical or different radicals from the group consisting of hydroxy, halogen, cyano, C(=O)OH, C(=O)OR^{a}, S(=O)₂-C₁-C₆-alkyl, N(R^{a})R^{b}, C₁-C₄-alkoxy, C₃-C₈-cycloalkyl and heterocycloalkyl;
R^{8b} represents hydrogen, halogen or C₁-C₆-alkyl, where
C₁-C₆-alkyl may optionally be mono- or polysubstituted by identical or different radicals from the group consisting of hydroxy, halogen, cyano, C(=O)OH, C(=O)OR^{a}, S(=O)₂-C₁-C₆-alkyl, N(R^{a})R^{b}, C₁-C₄-alkoxy, C₃-C₈-cycloalkyl and heterocycloalkyl;
or R^{8a} and R^{8b} together with the carbon atom form C₃-C₆-cycloalkyl which may optionally be mono- or polysubstituted by identical or different radicals from the group consisting of hydroxy, halogen, cyano and C₁-C₆-alkyl,
R^{8c} represents hydrogen, halogen, N(R^{a})R^{b}, C₁-C₆-alkyl or C₃-C₁₀-cycloalkyl, where
C₁-C₆-alkyl may optionally be mono- or polysubstituted by identical or different radicals from the group consisting of hydroxy, halogen, cyano, C(=O)OH, C(=O)OR^{a}, S(=O)₂-C₁-C₆-alkyl, N(R^{a})R^{b}, C₁-C₄-alkoxy, C₃-C₈-cycloalkyl and heterocycloalkyl;
R^{8d} represents hydrogen, halogen or C₁-C₆-alkyl, where
C₁-C₆-alkyl may optionally be mono- or polysubstituted by identical or different radicals from the group consisting of hydroxy, halogen, cyano, C(=O)OH, C(=O)OR^{a}, S(=O)₂-C₁-C₆-alkyl, N(R^{a})R^{b}, C₁-C₄-alkoxy, C₃-C₈-cycloalkyl and heterocycloalkyl;
or R^{8c} and R^{8d} together with the carbon atom form C₃-C₆-cycloalkyl which may optionally be mono- or polysubstituted by identical or different radicals from the group consisting of hydroxy, halogen, cyano and C₁-C₆-alkyl,
or R^{8c} and R^{8d} together represent an oxo group;
o represents 0, 1 or 2,
p represents 0, 1 or 2,
q represents 0, 1 or 2,
r represents 0, 1 or 2,
s represents 0, 1 or 2,
where o, p, q, r and s do not simultaneously represent 0;
Z represents a group selected from C(=O), CR⁹R¹⁰, NR¹¹, O, S, S(=O) and S(=O)₂;
R⁹ represents hydrogen or C₁-C₆-alkyl,
R¹⁰ represents hydrogen, halogen, cyano, C(=O)R^{a}, C(=O)OH, C(=O)OR^{a}, C(=O)NH₂, C(=O)N(H)R^{a}, C(=O)N(R^{a})R^{b}, N(H)C(=O)R^{a}, N(R^{b})C(=O)R^{a}, S(=O)₂R^{a}, hydroxy, N(R^{a})R^{b} or C₁-C₆-alkyl, where
C₁-C₆-alkyl may optionally be mono- or polysubstituted by identical or different radicals from the group consisting of hydroxy, halogen, cyano, C(=O)R^{a}, C(=O)OH, C(=O)OR^{a}, S(=O)₂-C₁-C₆-alkyl, N(R^{a})R^{b}, C₁-C₄-alkoxy and C₃-C₈-cycloalkoxy,
or represents C₁-C₆-alkoxy, where
C₁-C₆-alkoxy may optionally be mono- or polysubstituted by identical or different radicals from the group consisting of hydroxy, halogen, cyano, C(=O)OH, C(=O)OR^{a}, S(=O)₂-C₁-C₆-alkyl, N(R^{a})R^{b}, C₃-C₈-cycloalkyl, C₁-C₄-alkoxy, C₃-C₈-cycloalkoxy, heterocycloalkyl, aryl and 5- or 6-membered heteroaryl, where
aryl and 5- or 6-membered heteroaryl may optionally be mono- or polysubstituted by identical or different radicals from the group consisting of halogen, cyano, C₁-C₃-alkyl and C₁-C₃-alkoxy,
or represents aryloxy or 5- or 6-membered heteroaryloxy in which
aryloxy and 5- or 6-membered heteroaryloxy may optionally be mono- or polysubstituted by identical or different radicals from the group consisting of hydroxy, halogen, cyano, C(=O)OH, C(=O)OR^{a}, C₁-C₃-alkyl and C₁-C₃-alkoxy,
or represents C₃-C₈-cycloalkyl, C₃-C₈-Cycloalkyl-C₁-C₄-alkyl, heterocycloalkyl or heterocycloalkyl-C₁-C₄-alkyl, which may optionally be mono- or polysubstituted by identical or different radicals from the group consisting of hydroxy, halogen, cyano, C(=O)R^{a}, C(=O)OH, C(=O)OR^{a}, C₁-C₆-alkyl and C₁-C₆-alkoxy, where
C₁-C₆-alkoxy may optionally be mono- or polysubstituted by identical or different halogen atoms or an oxo group;
or represents C₂-C₆-alkenyl or C₂-C₆-alkynyl, or represents aryl, 5- to 10-membered heteroaryl, aryl-C₁-C₄-alkyl or 5- or 6-membered heteroaryl-C₁-C₄-alkyl, where
aryl and heteroaryl may optionally be mono- or polysubstituted by identical or different radicals from the group consisting of halogen, hydroxy, cyano, C(=O)OH, C(=O)OR^{a}, NHR^{a}, N(R^{a})R^{b}, C₁-C₃-alkyl, C₃-C₈-cycloalkyl and C₁-C₃-alkoxy;
or R⁹ and R¹⁰ together with the carbon atom form C₃-C₈-cycloalkyl or a 4- to 6-membered heterocycle, where
the C₃-C₈-cycloalkyl radical or the 4- to 6-membered heterocycle may optionally be mono-or polysubstituted by identical or different radicals from the group consisting of hydroxy, halogen, cyano, C₁-C₆-alkyl, C(=O)R^{a} and an oxo group;
R¹¹ represents hydrogen, C(=O)R^{a}, C(=O)OR^{a}, C(=O)NH₂, C(=O)N(H)R^{a}, C(=O)N(R^{a})R^{b}, S(=O)₂R^{a}, S(=O)₂N(R^{a})R^{b} or C₁-C₆-alkyl, where
C₁-C₆-alkyl may optionally be mono- or polysubstituted by identical or different radicals from the group consisting of hydroxy, halogen, cyano, C(=O)R^{a}, C(=O)OR^{a}, C(=O)NH₂, C(=O)N(H)R^{a}, C(=O)N(R^{a})R^{b}, S(=O)₂-C₁-C₆-alkyl, N(R^{a})R^{b}, C₃-C₈-cycloalkyl, C₁-C₄-alkoxy and C₃-C₈-cycloalkoxy, where
C₃-C₈-cycloalkyl, C₁-C₄-alkoxy and C₃-C₈-cycloalkoxy may optionally be mono- or polysubstituted by identical or different radicals from the group consisting of hydroxy and halogen;
or represents C₃-C₈-cycloalkyl, heterocycloalkyl or heterocycloalkyl-C₁-C₄-alkyl, which may optionally be mono- or polysubstituted by identical or different radicals from the group consisting of hydroxy, halogen, cyano, C₁-C₆-alkyl and C₁-C₆-alkoxy, where alkyl and alkoxy may optionally be mono- or polysubstituted by identical or different radicals from the group consisting of halogen and an oxo group,
or represents C₂-C₆-alkenyl or C₂-C₆-alkynyl,
or represents aryl, 5- to 10-membered heteroaryl, aryl-C₁-C₄-alkyl or 5- or 6-membered heteroaryl-C₁-C₄-alkyl, where
aryl and heteroaryl may optionally be mono- or polysubstituted by identical or different radicals from the group consisting of halogen, hydroxy, cyano, C(=O)OH, C(=O)OR^{a}, C₁-C₃-alkyl, C₃-C₈-cycloalkyl and C₁-C₃-alkoxy;
and their diastereomers, enantiomers, their salts, their solvates or the solvates of their salts.

2. Compounds according to Claim 1 in which R¹ represents hydrogen, halogen, hydroxy, cyano, C₁-C₆-alkyl, C₁-C₅-alkyl substituted by hydroxy, C₁-C₆-alkoxy, C₁-C₆-alkoxy substituted by C₃-C₈-cycloalkyl, C₁-C₆-alkoxy substituted by up to three fluorine atoms, C₁-C₆-alkoxy substituted by aryl which is optionally mono- or polysubstituted by identical or different radicals R^{c} or represents 5- or 6-membered heteroaryl which is optionally mono- or polysubstituted by identical or different radicals R^{c}.

3. Compounds according to Claim 1 in which W represents a group selected from formulae (III) to (IX): 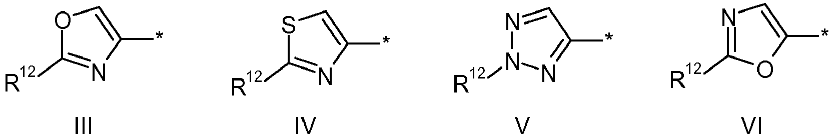 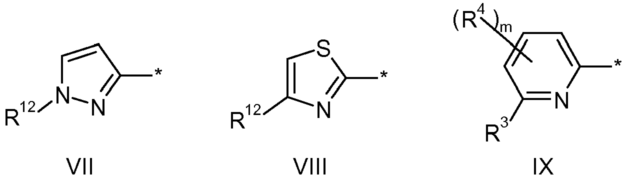 in which
R¹² represents hydrogen, halogen, C₁-C₆-alkyl which is optionally mono- or polysubstituted by identical or different halogen atoms, C₃-C₆-cycloalkyl which is optionally mono- or polysubstituted by identical or different halogen atoms, aryl which is optionally mono- or polysubstituted by identical or different radicals R^{c} or 5- or 6-membered heteroaryl which is optionally mono- or polysubstituted by identical or different radicals R^{c} or represents NHR^{a};
m represents 0, 1, 2 or 3 and
R³ and R⁴ have the meanings given above and
* represents the point of attachment of the group to the remainder of the molecule.

4. Compounds according to Claim 1 in which W represents a group of the general formula (X) 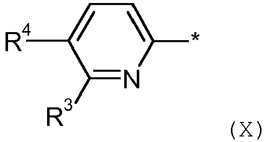 and R³ and R⁴ have the meaning given in Claim 1.

5. Compounds according to Claim 1 in which Y is a group of the general formula (II) where R^{7a}, R^{7b}, R^{7c}, R^{7d}, R^{8a}, R^{8b}, R^{8c} and R^{8d} are as defined in Claim 1:

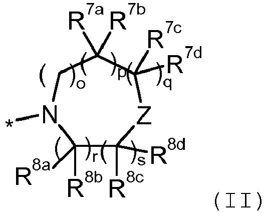

6. Compounds according to Claim 1 in which Y is a radical NR⁵R⁶ where R⁵ and R⁶ are as defined in Claim 1.

7. Compounds according to Claim 1 in which W represents a group of the general formula (IX) 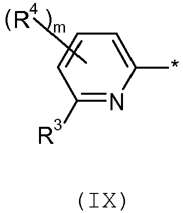 in which
m represents 0 and R², R⁰ and R¹³ all represent hydrogen and R³ represents trifluoromethyl, ethyl, methyl, cyclopropyl, 2,2,2-trifluoro-1-hydroxyethyl or 1-hydroxyethyl; Y represents 4-methylpiperazin-1-yl, 4-ethylpiperazin-1-yl or morpholin-4-yl, n represents 0 and R¹ represents cyclopropylmethoxy, trifluoromethoxy, 2,2,2-trifluoroethoxy, chlorine, ethoxy, methoxy, 2-hydroxypropan-2-yl or 3-hydroxypentan-3-yl.

8. Compounds according to Claim 7 in which R¹ represents cyclopropylmethoxy, methoxy, ethoxy or 2-hydroxypropan-2-yl.

9. Compounds according to Claim 7 in which R³ is a trifluoromethyl or a cyclopropyl radical.

10. Compounds according to Claim 1, specifically
N-{2-[2-(4-benzoylpiperazin-1-yl)-2-oxoethyl]-6-methyl-2H-indazol-5-yl}-6-(trifluoromethyl)pyridine-2-carboxamide
6-ethyl-N-(6-methyl-2-{2-oxo-2-[4-(pyrrolidin-1-yl)piperidin-1-yl]ethyl}-2H-indazol-5-yl)pyridine-2-carboxamide
5-fluoro-N-(6-methyl-2-{2-oxo-2-[4-(pyrrolidin-1-yl)piperidin-1-yl]ethyl}-2H-indazol-5-yl)pyridine-2-carboxamide
N-(2-{2-[4-(3-hydroxy-2,2-dimethylpropanoyl)piperazin-1-yl]-2-oxoethyl}-6-methyl-2H-indazol-5-yl)-6-(trifluoromethyl)pyridine-2-carboxamide
N-(2-{2-[4-(methoxyacetyl)piperazin-1-yl]-2-oxoethyl}-6-methyl-2H-indazol-5-yl)-6-(trifluoromethyl)pyridine-2-carboxamide
N-(2-{2-[4-(2-hydroxypropan-2-yl)piperidin-1-yl]-2-oxoethyl}-6-methoxy-2H-indazol-5-yl)-6-(trifluoromethyl)pyridine-2-carboxamide
N-(2-{2-[4-(2-hydroxypropan-2-yl)piperidin-1-yl]-2-oxoethyl}-6-methoxy-2H-indazol-5-yl)-6-methylpyridine-2-carboxamide
N-(2-{2-[4-(cyclopropylmethyl)piperazin-1-yl]-2-oxoethyl}-6-methoxy-2H-indazol-5-yl)-6-(trifluoromethyl)pyridine-2-carboxamide
N-(2-{2-[4-(cyclopropylmethyl)piperazin-1-yl]-2-oxoethyl}-6-methoxy-2H-indazol-5-yl)-6-methylpyridine-2-carboxamide
N-{2-[2-(4-benzoylpiperazin-1-yl)-2-oxoethyl]-6-methoxy-2H-indazol-5-yl}-6-cyclopropylpyridine-2-carboxamide
N-{2-[2-(4-benzoylpiperazin-1-yl)-2-oxoethyl]-6-methoxy-2H-indazol-5-yl}-6-(1-hydroxyethyl)pyridine-2-carboxamide
N-{2-[2-(4-methylpiperazin-1-yl)-2-oxoethyl]-6-(trifluoromethoxy)-2H-indazol-5-yl}-6-(trifluoromethyl)pyridine-2-carboxamide
6-methyl-N-{2-[2-(4-methylpiperazin-1-yl)-2-oxoethyl]-6-(trifluoromethoxy)-2H-indazol-5-yl}pyridine-2-carboxamide
tert-butyl 3-{[4-({2-[2-(4-methylpiperazin-1-yl)-2-oxoethyl]-6-(trifluoromethoxy)-2H-indazol-5-yl}carbamoyl)-1,3-thiazol-2-yl]amino}azetidine-1-carboxylate
N-{6-bromo-2-[2-(4-methylpiperazin-1-yl)-2-oxoethyl]-2H-indazol-5-yl}-6-(trifluoromethyl)pyridine-2-carboxamide
N-{6-bromo-2-[2-(4-methylpiperazin-1-yl)-2-oxoethyl]-2H-indazol-5-yl}-6-methylpyridine-2-carboxamide
N-{6-bromo-2-[2-(4-methylpiperazin-1-yl)-2-oxoethyl]-2H-indazol-5-yl}-2-cyclopropyl-1,3-oxazole-4-carboxamide
tert-butyl 3-{[4-({6-bromo-2-[2-(4-methylpiperazin-1-yl)-2-oxoethyl]-2H-indazol-5-yl}carbamoyl)-1,3-thiazol-2-yl]amino}azetidine-1-carboxylate
2-(azetidin-3-ylamino)-N-{2-[2-(4-methylpiperazin-1-yl)-2-oxoethyl]-6-(trifluoromethoxy)-2H-indazol-5-yl}-1,3-thiazole-4-carboxamide
N-{6-cyano-2-[2-(4-methylpiperazin-1-yl)-2-oxoethyl]-2H-indazol-5-yl}-6-(trifluoromethyl)pyridine-2-carboxamide
6'-methyl-N-{2-[2-(4-methylpiperazin-1-yl)-2-oxoethyl]-2H-indazol-5-yl}-2,3'-bipyridine-6-carboxamide
5'-methyl-N-{2-[2-(4-methylpiperazin-1-yl)-2-oxoethyl]-2H-indazol-5-yl}-2,3'-bipyridine-6-carboxamide
4'-methyl-N-{2-[2-(4-methylpiperazin-1-yl)-2-oxoethyl]-2H-indazol-5-yl}-2,3'-bipyridine-6-carboxamide
6'-methoxy-N-{2-[2-(4-methylpiperazin-1-yl)-2-oxoethyl]-2H-indazol-5-yl}-2,3'-bipyridine-6-carboxamide
6'-acetamido-N-{2-[2-(4-methylpiperazin-1-yl)-2-oxoethyl]-2H-indazol-5-yl}-2,3'-bipyridine-6-carboxamide
N-{2-[2-(4-methylpiperazin-1-yl)-2-oxoethyl]-2H-indazol-5-yl}-6'-nitro-2,3'-bipyridine-6-carboxamide
6'-amino-N-{2-[2-(4-methylpiperazin-1-yl)-2-oxoethyl]-2H-indazol-5-yl}-2,3'-bipyridine-6-carboxamide
N-{2-[2-(4-benzoylpiperazin-1-yl)-2-oxoethyl]-6-fluoro-2H-indazol-5-yl}-6-(trifluoromethyl)pyridine-2-carboxamide
N-(2-{2-[4-(cyclopropylcarbonyl)piperazin-1-yl]-2-oxoethyl}-6-fluoro-2H-indazol-5-yl)-6-(trifluoromethyl)pyridine-2-carboxamide
N-{6-fluoro-2-[2-(4-methylpiperazin-1-yl)-2-oxoethyl]-2H-indazol-5-yl}-6-(trifluoromethyl)pyridine-2-carboxamide
N-(2-{2-[4-(cyclopropylcarbonyl)piperazin-1-yl]-2-oxoethyl}-6-fluoro-2H-indazol-5-yl)-6-methylpyridine-2-carboxamide
N-{6-fluoro-2-[2-(4-methylpiperazin-1-yl)-2-oxoethyl]-2H-indazol-5-yl}-6-methylpyridine-2-carboxamide
N-(2-{2-[4-(cyclopropylcarbonyl)piperazin-1-yl]-2-oxoethyl}-6-fluoro-2H-indazol-5-yl)-6-(1-methyl-1H-pyrazol-4-yl)pyridine-2-carboxamide
N-{2-[2-(4-benzoylpiperazin-1-yl)-2-oxoethyl]-6-fluoro-2H-indazol-5-yl}-6-(1-methyl-1H-pyrazol-4-yl)pyridine-2-carboxamide
N-{6-fluoro-2-[2-(4-methylpiperazin-1-yl)-2-oxoethyl]-2H-indazol-5-yl}-6-(1-methyl-1H-pyrazol-4-yl)pyridine-2-carboxamide
N-{2-[2-(4-benzoylpiperazin-1-yl)-2-oxoethyl]-6-fluoro-2H-indazol-5-yl}-5-fluoro-6-(1-methyl-1H-pyrazol-4-yl)pyridine-2-carboxamide
N-(2-{2-[4-(cyclopropylcarbonyl)piperazin-1-yl]-2-oxoethyl}-6-fluoro-2H-indazol-5-yl)-5-fluoro-6-(1-methyl-1H-pyrazol-4-yl)pyridine-2-carboxamide
N-{2-[2-(4-benzoylpiperazin-1-yl)-2-oxoethyl]-6-fluoro-2H-indazol-5-yl}-6-(morpholin-4-yl)pyridine-2-carboxamide
N-(2-{2-[4-(cyclopropylcarbonyl)piperazin-1-yl]-2-oxoethyl}-6-fluoro-2H-indazol-5-yl)-6-(morpholin-4-yl)pyridine-2-carboxamide
N-{6-fluoro-2-[2-(4-methylpiperazin-1-yl)-2-oxoethyl]-2H-indazol-5-yl}-6-(morpholin-4-yl)pyridine-2-carboxamide
N-{6-(benzyloxy)-2-[2-(4-methylpiperazin-1-yl)-2-oxoethyl]-2H-indazol-5-yl}-6-(trifluoromethyl)pyridine-2-carboxamide
N-{6-isobutoxy-2-[2-(4-methylpiperazin-1-yl)-2-oxoethyl]-2H-indazol-5-yl}-6-(trifluoromethyl)pyridine-2-carboxamide
N-{6-isobutoxy-2-[2-(morpholin-4-yl)-2-oxoethyl]-2H-indazol-5-yl}-6-(trifluoromethyl)pyridine-2-carboxamide
N-(2-{2-[4-(2-hydroxypropan-2-yl)piperidin-1-yl]-2-oxoethyl}-6-isobutoxy-2H-indazol-5-yl)-6-(trifluoromethyl)pyridine-2-carboxamide
N-(2-{2-[(cyclopropylmethyl)(methyl)amino]-2-oxoethyl}-6-isobutoxy-2H-indazol-5-yl)-6-(trifluoromethyl)pyridine-2-carboxamide
N-{6-(cyclopropylmethoxy)-2-[2-(4-methylpiperazin-1-yl)-2-oxoethyl]-2H-indazol-5-yl}-6-(trifluoromethyl)pyridine-2-carboxamide
N-{6-(cyclopropylmethoxy)-2-[2-(morpholin-4-yl)-2-oxoethyl]-2H-indazol-5-yl}-6-(trifluoromethyl)pyridine-2-carboxamide
N-[6-(cyclopropylmethoxy)-2-{2-[4-(2-hydroxypropan-2-yl)piperidin-1-yl]-2-oxoethyl}-2H-indazol-5-yl]-6-(trifluoromethyl)pyridine-2-carboxamide
N-[6-(cyclopropylmethoxy)-2-{2-[(cyclopropylmethyl)(methyl)amino]-2-oxoethyl}-2H-indazol-5-yl]-6-(trifluoromethyl)pyridine-2-carboxamide
N-{2-[2-(4-methylpiperazin-1-yl)-2-oxoethyl]-6-(pyridin-2-ylmethoxy)-2H-indazol-5-yl}-6-(trifluoromethyl)pyridine-2-carboxamide
N-{2-[2-(morpholin-4-yl)-2-oxoethyl]-6-(pyridin-2-ylmethoxy)-2H-indazol-5-yl}-6-(trifluoromethyl)pyridine-2-carboxamide
N-[2-{2-[4-(2-hydroxypropan-2-yl)piperidin-1-yl]-2-oxoethyl}-6-(pyridin-2-ylmethoxy)-2H-indazol-5-yl]-6-(trifluoromethyl)pyridine-2-carboxamide
N-[2-{2-[(cyclopropylmethyl)(methyl)amino]-2-oxoethyl}-6-(pyridin-2-ylmethoxy)-2H-indazol-5-yl]-6-(trifluoromethyl)pyridine-2-carboxamide
N-{2-[2-(4-benzoylpiperazin-1-yl)-2-oxoethyl]-6-chloro-2H-indazol-5-yl}-6-(trifluoromethyl)pyridine-2-carboxamide
N-{6-chloro-2-[2-(morpholin-4-yl)-2-oxoethyl]-2H-indazol-5-yl}-6-(trifluoromethyl)pyridine-2-carboxamide
ethyl 4-{[6-chloro-5-({[6-(trifluoromethyl)pyridin-2-yl]carbonyl}amino)-2H-indazol-2-yl]acetyl}piperazine-1-carboxylate
N-(6-chloro-2-{2-oxo-2-[4-(pyrrolidin-1-yl)piperidin-1-yl]ethyl}-2H-indazol-5-yl)-6-(trifluoromethyl)pyridine-2-carboxamide
N-(6-chloro-2-{2-[4-(2-hydroxypropan-2-yl)piperidin-1-yl]-2-oxoethyl}-2H-indazol-5-yl)-6-(trifluoromethyl)pyridine-2-carboxamide
N-(6-chloro-2-{2-[4-(3-hydroxy-2,2-dimethylpropanoyl)piperazin-1-yl]-2-oxoethyl}-2H-indazol-5-yl)-6-(trifluoromethyl)pyridine-2-carboxamide
N-(6-chloro-2-{2-[3-(dimethylamino)azetidin-1-yl]-2-oxoethyl}-2H-indazol-5-yl)-6-(trifluoromethyl)pyridine-2-carboxamide
N-(6-chloro-2-{2-oxo-2-[3-(piperidin-1-yl)azetidin-1-yl]ethyl}-2H-indazol-5-yl)-6-(trifluoromethyl)pyridine-2-carboxamide
N-(6-chloro-2-{2-[4-(2-hydroxy-2-methylpropyl)piperidin-1-yl]-2-oxoethyl}-2H-indazol-5-yl)-6-(trifluoromethyl)pyridine-2-carboxamide
N-{6-chloro-2-[2-(4-hydroxy-1,4'-bipiperidin-1'-yl)-2-oxoethyl]-2H-indazol-5-yl}-6-(trifluoromethyl)pyridine-2-carboxamide
N-{6-methoxy-2-[2-(4-methylpiperazin-1-yl)-2-oxoethyl]-2H-indazol-5-yl}-6-(trifluoromethyl)pyridine-2-carboxamide
N-{6-methoxy-2-[2-(morpholin-4-yl)-2-oxoethyl]-2H-indazol-5-yl}-6-(trifluoromethyl)pyridine-2-carboxamide
N-(2-{2-[4-(dimethylamino)piperidin-1-yl]-2-oxoethyl}-6-ethoxy-2H-indazol-5-yl)-6-(trifluoromethyl)pyridine-2-carboxamide
N-(6-ethoxy-2-{2-oxo-2-[4-(pyrrolidin-1-yl)piperidin-1-yl]ethyl}-2H-indazol-5-yl)-6-(trifluoromethyl)pyridine-2-carboxamide
N-{6-ethoxy-2-[2-(4-methylpiperazin-1-yl)-2-oxoethyl]-2H-indazol-5-yl}-6-(trifluoromethyl)pyridine-2-carboxamide
N-{2-[2-(4-benzoylpiperazin-1-yl)-2-oxoethyl]-6-ethoxy-2H-indazol-5-yl}-6-(trifluoromethyl)pyridine-2-carboxamide
N-(6-ethoxy-2-{2-[4-(2-hydroxypropan-2-yl)piperidin-1-yl]-2-oxoethyl}-2H-indazol-5-yl)-6-(trifluoromethyl)pyridine-2-carboxamide
N-{6-ethoxy-2-[2-(morpholin-4-yl)-2-oxoethyl]-2H-indazol-5-yl}-6-(trifluoromethyl)pyridine-2-carboxamide
N-{2-[2-(4-benzoylpiperazin-1-yl)-2-oxoethyl]-3-methyl-2H-indazol-5-yl}-6-(trifluoromethyl)pyridine-2-carboxamide
N-{2-[3-(4-benzoylpiperazin-1-yl)-3-oxopropyl]-2H-indazol-5-yl}-6-(trifluoromethyl)pyridine-2-carboxamide
N-(2-{2-[4-(cyclopropylcarbonyl)piperazin-1-yl]-2-oxoethyl}-2H-indazol-5-yl)-2-(pyridin-3-yl)-1,3-thiazole-4-carboxamide
N-(2-{2-[4-(cyclopropylcarbonyl)piperazin-1-yl]-2-oxoethyl}-2H-indazol-5-yl)-2-(pyridin-4-yl)-1,3-thiazole-4-carboxamide
N-(2-{2-[4-(cyclopropylcarbonyl)piperazin-1-yl]-2-oxoethyl}-2H-indazol-5-yl)-6-(trifluoromethyl)pyridine-2-carboxamide
6-(azetidin-3-ylamino)-N-(2-{2-[4-(cyclopropylcarbonyl)piperazin-1-yl]-2-oxoethyl}-2H-indazol-5-yl)pyridine-2-carboxamide
N-{2-[2-(4-methylpiperazin-1-yl)-2-oxoethyl]-2H-indazol-5-yl}-2-(pyridin-3-yl)-1,3-thiazole-4-carboxamide
N-{2-[2-(4-methylpiperazin-1-yl)-2-oxoethyl]-2H-indazol-5-yl}-6-(1-methyl-1H-pyrazol-4-yl)pyridine-2-carboxamide
N-{2-[2-(4-methylpiperazin-1-yl)-2-oxoethyl]-2H-indazol-5-yl}-6-(1H-pyrazol-4-yl)pyridine-2-carboxamide
6-(1,3-dimethyl-1H-pyrazol-4-yl)-N-{2-[2-(4-methylpiperazin-1-yl)-2-oxoethyl]-2H-indazol-5-yl}pyridine-2-carboxamide
N-{2-[2-(4-methylpiperazin-1-yl)-2-oxoethyl]-2H-indazol-5-yl}-6-[3-(trifluoromethyl)-1H-pyrazol-4-yl]pyridine-2-carboxamide
6-ethyl-N-{2-[2-(4-methylpiperazin-1-yl)-2-oxoethyl]-2H-indazol-5-yl}pyridine-2-carboxamide
6-(1-methyl-1H-pyrazol-4-yl)-N-(2-{2-oxo-2-[4-(2,2,2-trifluoroethyl)piperazin-1-yl]ethyl}-2H-indazol-5-yl)pyridine-2-carboxamide
N-(2-{2-oxo-2-[4-(2,2,2-trifluoroethyl)piperazin-1-yl]ethyl}-2H-indazol-5-yl)-6-(trifluoromethyl)pyridine-2-carboxamide
N-{2-[2-(4-ethyl-3-oxopiperazin-1-yl)-2-oxoethyl]-2H-indazol-5-yl}-6-(1-methyl-1H-pyrazol-4-yl)pyridine-2-carboxamide
N-{2-[2-(4-benzoylpiperazin-1-yl)-2-oxoethyl]-2H-indazol-5-yl}-6-(trifluoromethyl)pyridine-2-carboxamide
N-{2-[2-(4-benzoylpiperazin-1-yl)-2-oxoethyl]-2H-indazol-5-yl}-6-methylpyridine-2-carboxamide
N-{2-[2-(4-benzoylpiperazin-1-yl)-2-oxoethyl]-2H-indazol-5-yl}-6-(morpholin-4-yl)pyridine-2-carboxamide
N-{2-[2-(4-benzoylpiperazin-1-yl)-2-oxoethyl]-2H-indazol-5-yl}-2-(pyridin-4-yl)-1,3-thiazole-4-carboxamide
N-{2-[2-(4-benzoylpiperazin-1-yl)-2-oxoethyl]-2H-indazol-5-yl}-6-chloropyridine-2-carboxamide
N-{2-[2-(4-benzoylpiperazin-1-yl)-2-oxoethyl]-2H-indazol-5-yl}-2-methyl-1,3-oxazole-5-carboxamide
6-amino-N-{2-[2-(4-benzoylpiperazin-1-yl)-2-oxoethyl]-2H-indazol-5-yl}pyridine-2-carboxamide
N-{2-[2-(4-benzoylpiperazin-1-yl)-2-oxoethyl]-2H-indazol-5-yl}-2-methyl-1,3-oxazole-4-carboxamide
N-{2-[2-(4-benzoylpiperazin-1-yl)-2-oxoethyl]-2H-indazol-5-yl}-6-methoxypyridine-2-carboxamide
N-{2-[2-(4-benzoylpiperazin-1-yl)-2-oxoethyl]-2H-indazol-5-yl}-2-cyclopropyl-1,3-oxazole-4-carboxamide
N-{2-[2-(4-benzoylpiperazin-1-yl)-2-oxoethyl]-2H-indazol-5-yl}-6-(4H-1,2,4-triazol-4-yl)pyridine-2-carboxamide
N-{2-[2-(4-benzoylpiperazin-1-yl)-2-oxoethyl]-2H-indazol-5-yl}-2-phenyl-2H-1,2,3-triazole-4-carboxamide
N-{2-[2-(4-benzoylpiperazin-1-yl)-2-oxoethyl]-2H-indazol-5-yl}-6-(1-methyl-1H-pyrazol-5-yl)pyridine-2-carboxamide
N-{2-[2-(4-benzoylpiperazin-1-yl)-2-oxoethyl]-2H-indazol-5-yl}-2-(trifluoromethyl)-1,3-thiazole-4-carboxamide
N-{2-[2-(4-benzoylpiperazin-1-yl)-2-oxoethyl]-2H-indazol-5-yl}-6-(1H-pyrazol-1-yl)pyridine-2-carboxamide
N-{2-[2-(4-benzoylpiperazin-1-yl)-2-oxoethyl]-2H-indazol-5-yl}-6-(1-methyl-1H-pyrazol-4-yl)pyridine-2-carboxamide
N-{2-[2-(4-benzoylpiperazin-1-yl)-2-oxoethyl]-2H-indazol-5-yl}-1-ethyl-1H-pyrazole-3-carboxamide
N-{2-[2-(4-benzoylpiperazin-1-yl)-2-oxoethyl]-2H-indazol-5-yl}-6-(4-chloro-1H-pyrazol-1-yl)pyridine-2-carboxamide
N-{2-[2-(4-benzoylpiperazin-1-yl)-2-oxoethyl]-2H-indazol-5-yl}-4-(trifluoromethyl)-1,3-thiazole-2-carboxamide
N-{2-[2-(4-benzoylpiperazin-1-yl)-2-oxoethyl]-2H-indazol-5-yl}-6-(1,3-dimethyl-1H-pyrazol-4-yl)pyridine-2-carboxamide
N-{2-[2-(4-benzoylpiperazin-1-yl)-2-oxoethyl]-2H-indazol-5-yl}-2,4'-bipyridine-6-carboxamide
N-{2-[2-(4-benzoylpiperazin-1-yl)-2-oxoethyl]-2H-indazol-5-yl}-6-(1H-pyrazol-4-yl)pyridine-2-carboxamide
N-{2-[2-(4-benzoylpiperazin-1-yl)-2-oxoethyl]-2H-indazol-5-yl}-5-fluoro-6-(1-methyl-1H-pyrazol-4-yl)pyridine-2-carboxamide
N-{2-[2-(4-benzoylpiperazin-1-yl)-2-oxoethyl]-2H-indazol-5-yl}-6-(3-methyl-1H-pyrazol-4-yl)pyridine-2-carboxamide
N-{2-[2-(4-benzoylpiperazin-1-yl)-2-oxoethyl]-2H-indazol-5-yl}-6-(1H-1,2,4-triazol-1-yl)pyridine-2-carboxamide
N-{2-[2-(4-benzoylpiperazin-1-yl)-2-oxoethyl]-2H-indazol-5-yl}-6-[3-(trifluoromethyl)-1H-pyrazol-4-yl]pyridine-2-carboxamide
N-{2-[2-(4-benzoylpiperazin-1-yl)-2-oxoethyl]-2H-indazol-5-yl}-6-ethoxypyridine-2-carboxamide
N-{2-[2-(4-benzoylpiperazin-1-yl)-2-oxoethyl]-2H-indazol-5-yl}-6-(cyclopropylmethoxy)pyridine-2-carboxamide
N-{2-[2-(4-benzoylpiperazin-1-yl)-2-oxoethyl]-2H-indazol-5-yl}-6-ethylpyridine-2-carboxamide
N-{2-[2-(4-benzoylpiperazin-1-yl)-2-oxoethyl]-2H-indazol-5-yl}-2-(4-methoxyphenyl)-1,3-thiazole-4-carboxamide
N-{2-[2-(4-benzoylpiperazin-1-yl)-2-oxoethyl]-2H-indazol-5-yl}-2-bromo-1,3-thiazole-4-carboxamide
N-{2-[2-(4-benzoylpiperazin-1-yl)-2-oxoethyl]-2H-indazol-5-yl}-2-(4-fluorophenyl)-1,3-thiazole-4-carboxamide
N-{2-[2-(4-benzoylpiperazin-1-yl)-2-oxoethyl]-2H-indazol-5-yl}-6-fluoropyridine-2-carboxamide
N-{2-[2-(4-benzoylpiperazin-1-yl)-2-oxoethyl]-2H-indazol-5-yl}-6-bromopyridine-2-carboxamide
N-(2-{2-[4-(4-fluorobenzoyl)piperazin-1-yl]-2-oxoethyl}-2H-indazol-5-yl)-6-(trifluoromethyl)pyridine-2-carboxamide
N-(2-{2-oxo-2-[4-(pyridin-2-yl)piperazin-1-yl]ethyl}-2H-indazol-5-yl)-6-(trifluoromethyl)pyridine-2-carboxamide
N-(2-{2-[4-(methoxyacetyl)piperazin-1-yl]-2-oxoethyl}-2H-indazol-5-yl)-6-(trifluoromethyl)pyridine-2-carboxamide
N-{2-[2-(4-cyclopentyl-3-oxopiperazin-1-yl)-2-oxoethyl]-2H-indazol-5-yl}-6-(trifluoromethyl)pyridine-2-carboxamide
N-{2-[2-oxo-2-(3-oxo-4-phenylpiperazin-1-yl)ethyl]-2H-indazol-5-yl}-6-(trifluoromethyl)pyridine-2-carboxamide
N-(2-{2-[4-(2,2-dimethylpropanoyl)piperazin-1-yl]-2-oxoethyl}-2H-indazol-5-yl)-6-(trifluoromethyl)pyridine-2-carboxamide
N-(2-{2-[4-(cyclopropylmethyl)piperazin-1-yl]-2-oxoethyl}-2H-indazol-5-yl)-6-(trifluoromethyl)pyridine-2-carboxamide
N-{2-[2-oxo-2-(pyridazin-4-ylamino)ethyl]-2H-indazol-5-yl}-6-(trifluoromethyl)pyridine-2-carboxamide
N-(2-{2-[4-(2-hydroxy-2-methylpropanoyl)piperazin-1-yl]-2-oxoethyl}-2H-indazol-5-yl)-6-(trifluoromethyl)pyridine-2-carboxamide
N-(2-{2-oxo-2-[4-(1-phenylethyl)piperazin-1-yl]ethyl}-2H-indazol-5-yl)-6-(trifluoromethyl)pyridine-2-carboxamide
N-(2-{2-oxo-2-[4-(pyridin-3-ylcarbonyl)piperazin-1-yl]ethyl}-2H-indazol-5-yl)-6-(trifluoromethyl)pyridine-2-carboxamide
N-{2-[2-(4-isonicotinoylpiperazin-1-yl)-2-oxoethyl]-2H-indazol-5-yl}-6-(trifluoromethyl)pyridine-2-carboxamide
N-(2-{2-[4-(morpholin-4-ylcarbonyl)piperazin-1-yl]-2-oxoethyl}-2H-indazol-5-yl)-6-(trifluoromethyl)pyridine-2-carboxamide
N-[2-(2-{4-[2-(methylamino)-2-oxoethyl]piperazin-1-yl}-2-oxoethyl)-2H-indazol-5-yl]-6-(trifluoromethyl)pyridine-2-carboxamide
N-(2-{2-oxo-2-[4-(pyrazin-2-yl)piperazin-1-yl]ethyl}-2H-indazol-5-yl)-6-(trifluoromethyl)pyridine-2-carboxamide
N-(2-{2-[4-(1-hydroxyethyl)piperidin-1-yl]-2-oxoethyl}-2H-indazol-5-yl)-6-(trifluoromethyl)pyridine-2-carboxamide
N-{2-[2-(2-methyl-2,8-diazaspiro[4.5]dec-8-yl)-2-oxoethyl]-2H-indazol-5-yl}-6-(trifluoromethyl)pyridine-2-carboxamide
N-{2-[2-(6-acetyl-2,6-diazaspiro[3.3]hept-2-yl)-2-oxoethyl]-2H-indazol-5-yl}-6-(trifluoromethyl)pyridine-2-carboxamide
N-{2-[2-oxo-2-(3-oxo-2,8-diazaspiro[4.5]dec-8-yl)ethyl]-2H-indazol-5-yl}-6-(trifluoromethyl)pyridine-2-carboxamide
N-{2-[2-(6-methyl-2,6-diazaspiro[3.5]non-2-yl)-2-oxoethyl]-2H-indazol-5-yl}-6-(trifluoromethyl)pyridine-2-carboxamide
N-{2-[2-(7-oxa-2-azaspiro[3.5]non-2-yl)-2-oxoethyl]-2H-indazol-5-yl}-6-(trifluoromethyl)pyridine-2-carboxamide
N-{2-[2-(1,4'-bipiperidin-1'-yl)-2-oxoethyl]-2H-indazol-5-yl}-6-(trifluoromethyl)pyridine-2-carboxamide
N-(2-{2-[2-(hydroxymethyl)piperidin-1-yl]-2-oxoethyl}-2H-indazol-5-yl)-6-(trifluoromethyl)pyridine-2-carboxamide
N-(2-{2-[3-(hydroxymethyl)piperidin-1-yl]-2-oxoethyl}-2H-indazol-5-yl)-6-(trifluoromethyl)pyridine-2-carboxamide
N-{2-[2-(4-carbamoylpiperidin-1-yl)-2-oxoethyl]-2H-indazol-5-yl}-6-(trifluoromethyl)pyridine-2-carboxamide
N-(2-{2-[3-(dimethylamino)piperidin-1-yl]-2-oxoethyl}-2H-indazol-5-yl)-6-(trifluoromethyl)pyridine-2-carboxamide
N-(2-{2-[3-(morpholin-4-ylmethyl)piperidin-1-yl]-2-oxoethyl}-2H-indazol-5-yl)-6-(trifluoromethyl)pyridine-2-carboxamide
N-[2-(2-{4-[(cyclopropylcarbonyl)amino]piperidin-1-yl}-2-oxoethyl)-2H-indazol-5-yl]-6-(trifluoromethyl)pyridine-2-carboxamide
N-(2-{2-[4-(3-ethyl-1,2,4-oxadiazol-5-yl)piperidin-1-yl]-2-oxoethyl}-2H-indazol-5-yl)-6-(trifluoromethyl)pyridine-2-carboxamide
N-[2-(2-{4-[(5-cyclopropyl-1,2,4-oxadiazol-3-yl)methyl]piperidin-1-yl}-2-oxoethyl)-2H-indazol-5-yl]-6-(trifluoromethyl)pyridine-2-carboxamide
N-(2-{2-oxo-2-[4-(pyrrolidin-1-ylcarbonyl)piperidin-1-yl]ethyl}-2H-indazol-5-yl)-6-(trifluoromethyl)pyridine-2-carboxamide
N-(2-{2-[4-(4-methylpiperazin-1-yl)piperidin-1-yl]-2-oxoethyl}-2H-indazol-5-yl)-6-(trifluoromethyl)pyridine-2-carboxamide
N-[2-(2-{4-[2-(morpholin-4-yl)ethyl]piperidin-1-yl}-2-oxoethyl)-2H-indazol-5-yl]-6-(trifluoromethyl)pyridine-2-carboxamide
N-[2-(2-{4-[(5-methyl-1,2,4-oxadiazol-3-yl)methyl]piperidin-1-yl}-2-oxoethyl)-2H-indazol-5-yl]-6-(trifluoromethyl)pyridine-2-carboxamide
N-(2-{2-oxo-2-[3-(pyrrolidin-1-ylmethyl)piperidin-1-yl]ethyl}-2H-indazol-5-yl)-6-(trifluoromethyl)pyridine-2-carboxamide
N-[2-(2-{[3-(dimethylsulphamoyl)phenyl]amino}-2-oxoethyl)-2H-indazol-5-yl]-6-(trifluoromethyl)pyridine-2-carboxamide
N-{2-[2-(1,2-oxazol-4-ylamino)-2-oxoethyl]-2H-indazol-5-yl}-6-(trifluoromethyl)pyridine-2-carboxamide
N-(2-{2-[4-(methylsulphonyl)piperidin-1-yl]-2-oxoethyl}-2H-indazol-5-yl)-6-(trifluoromethyl)pyridine-2-carboxamide
N-[2-(2-oxo-2-{4-[2-oxo-2-(pyrrolidin-1-yl)ethyl]piperazin-1-yl}ethyl)-2H-indazol-5-yl]-6-(trifluoromethyl)pyridine-2-carboxamide
N-(2-{2-oxo-2-[4-(phenylsulphonyl)piperidin-1-yl]ethyl}-2H-indazol-5-yl)-6-(trifluoromethyl)pyridine-2-carboxamide
N-(2-{2-oxo-2-[(3-sulphamoylphenyl)amino]ethyl}-2H-indazol-5-yl)-6-(trifluoromethyl)pyridine-2-carboxamide
N-[2-(2-{4-[isonicotinoyl(methyl)amino]piperidin-1-yl}-2-oxoethyl)-2H-indazol-5-yl]-6-(trifluoromethyl)pyridine-2-carboxamide
N-[2-(2-{4-[2-(isopropylamino)-2-oxoethyl]piperazin-1-yl}-2-oxoethyl)-2H-indazol-5-yl]-6-(trifluoromethyl)pyridine-2-carboxamide
N-(2-{2-[4-(1,1-dioxidotetrahydrothiophen-3-yl)piperazin-1-yl]-2-oxoethyl}-2H-indazol-5-yl)-6-(trifluoromethyl)pyridine-2-carboxamide
N-[2-(2-{4-[(methoxyacetyl)(methyl)amino]piperidin-1-yl}-2-oxoethyl)-2H-indazol-5-yl]-6-(trifluoromethyl)pyridine-2-carboxamide
ethyl 4-{[5-({[6-(trifluoromethyl)pyridin-2-yl]carbonyl}amino)-2H-indazol-2-yl]acetyl}piperazine-1-carboxylate
N-(2-{2-[4-(cyclohexylcarbonyl)piperazin-1-yl]-2-oxoethyl}-2H-indazol-5-yl)-6-(trifluoromethyl)pyridine-2-carboxamide
N-[2-(2-{4-[2-(cyclopropylamino)-2-oxoethyl]piperazin-1-yl}-2-oxoethyl)-2H-indazol-5-yl]-6-(trifluoromethyl)pyridine-2-carboxamide
N-(2-{2-[2-(2-hydroxyethyl)piperidin-1-yl]-2-oxoethyl}-2H-indazol-5-yl)-6-(trifluoromethyl)pyridine-2-carboxamide
N-(2-{2-oxo-2-[4-(pyrrolidin-1-yl)piperidin-1-yl]ethyl}-2H-indazol-5-yl)-6-(trifluoromethyl)pyridine-2-carboxamide
N-(2-{2-oxo-2-[4-(1H-pyrrol-1-yl)piperidin-1-yl]ethyl}-2H-indazol-5-yl)-6-(trifluoromethyl)pyridine-2-carboxamide
N-(2-{2-[4-(3-hydroxypropyl)piperazin-1-yl]-2-oxoethyl}-2H-indazol-5-yl)-6-(trifluoromethyl)pyridine-2-carboxamide
4-{[5-({[6-(trifluoromethyl)pyridin-2-yl]carbonyl}amino)-2H-indazol-2-yl]acetyl}piperazine-1-carboxamide
N-(2-{2-oxo-2-[4-(2-oxopyrrolidin-1-yl)piperidin-1-yl]ethyl}-2H-indazol-5-yl)-6-(trifluoromethyl)pyridine-2-carboxamide
N-{2-[2-(morpholin-4-yl)-2-oxoethyl]-2H-indazol-5-yl}-6-(trifluoromethyl)pyridine-2-carboxamide
N-(2-{2-[4-(2-amino-2-oxoethyl)piperazin-1-yl]-2-oxoethyl}-2H-indazol-5-yl)-6-(trifluoromethyl)pyridine-2-carboxamide
N-{2-[2-(1,1-dioxidothiomorpholin-4-yl)-2-oxoethyl]-2H-indazol-5-yl}-6-(trifluoromethyl)pyridine-2-carboxamide
N-{2-[2-(4-isopropylpiperazin-1-yl)-2-oxoethyl]-2H-indazol-5-yl}-6-(trifluoromethyl)pyridine-2-carboxamide
N-(2-{2-oxo-2-[4-(2-thienylcarbonyl)piperazin-1-yl]ethyl}-2H-indazol-5-yl)-6-(trifluoromethyl)pyridine-2-carboxamide
N-(2-{2-[4-(2-cyclopropyl-2-oxoethyl)piperazin-1-yl]-2-oxoethyl}-2H-indazol-5-yl)-6-(trifluoromethyl)pyridine-2-carboxamide
N-[2-(2-{4-[(1-methyl-1H-pyrazol-4-yl)methyl]piperazin-1-yl}-2-oxoethyl)-2H-indazol-5-yl]-6-(trifluoromethyl)pyridine-2-carboxamide
N-[2-(2-{4-[(1,5-dimethyl-1H-pyrazol-3-yl)carbonyl]piperazin-1-yl}-2-oxoethyl)-2H-indazol-5-yl]-6-(trifluoromethyl)pyridine-2-carboxamide
N,N-diethyl-4-{ [5-({ [6-(trifluoromethyl)pyridin-2-yl]carbonyl}amino)-2H-indazol-2-yl]acetyl}piperazine-1-carboxamide
N-{2-[2-oxo-2-(thiomorpholin-4-yl)ethyl]-2H-indazol-5-yl}-6-(trifluoromethyl)pyridine-2-carboxamide
N-(2-{2-[4-(2-furylmethyl)piperazin-1-yl]-2-oxoethyl}-2H-indazol-5-yl)-6-(trifluoromethyl)pyridine-2-carboxamide
N-(2-{2-oxo-2-[4-(3-thienylmethyl)piperazin-1-yl]ethyl}-2H-indazol-5-yl)-6-(trifluoromethyl)pyridine-2-carboxamide
N-{2-[2-(4'-methyl-1,4'-bipiperidin-1'-yl)-2-oxoethyl]-2H-indazol-5-yl}-6-(trifluoromethyl)pyridine-2-carboxamide
N-{2-[2-(6-methyl-2,6-diazaspiro[3.3]hept-2-yl)-2-oxoethyl]-2H-indazol-5-yl}-6-(trifluoromethyl)pyridine-2-carboxamide
N-{2-[2-(4-cyclopentylpiperazin-1-yl)-2-oxoethyl]-2H-indazol-5-yl}-6-(trifluoromethyl)pyridine-2-carboxamide
N-[2-(2-{4-[2-(2-hydroxyethoxy)ethyl]piperazin-1-yl}-2-oxoethyl)-2H-indazol-5-yl]-6-(trifluoromethyl)pyridine-2-carboxamide
N-(2-{2-oxo-2-[4-(pyridin-4-ylmethyl)piperazin-1-yl]ethyl}-2H-indazol-5-yl)-6-(trifluoromethyl)pyridine-2-carboxamide
N-(2-{2-[4-(dimethylsulphamoyl)piperazin-1-yl]-2-oxoethyl}-2H-indazol-5-yl)-6-(trifluoromethyl)pyridine-2-carboxamide
N-(2-{2-oxo-2-[4-(pyridin-4-yl)piperazin-1-yl]ethyl}-2H-indazol-5-yl)-6-(trifluoromethyl)pyridine-2-carboxamide
N-(2-{2-[4-(methylsulphonyl)piperazin-1-yl]-2-oxoethyl}-2H-indazol-5-yl)-6-(trifluoromethyl)pyridine-2-carboxamide
formic acid N-[2-(2-{4-[2-(1H-imidazol-1-yl)ethyl]piperazin-1-yl}-2-oxoethyl)-2H-indazol-5-yl]-6-(trifluoromethyl)pyridine-2-carboxamide (1:1)
N-(2-{2-[4-(diethylsulphamoyl)piperazin-1-yl]-2-oxoethyl}-2H-indazol-5-yl)-6-(trifluoromethyl)pyridine-2-carboxamide
N-(2-{2-oxo-2-[4-(pyridin-3-yl)piperazin-1-yl]ethyl}-2H-indazol-5-yl)-6-(trifluoromethyl)pyridine-2-carboxamide
N-(2-{2-oxo-2-[4-(piperidin-1-ylsulphonyl)piperazin-1-yl]ethyl}-2H-indazol-5-yl)-6-(trifluoromethyl)pyridine-2-carboxamide
N-[2-(2-{4-[(1,5-dimethyl-1H-pyrazol-4-yl)sulphonyl]piperazin-1-yl}-2-oxoethyl)-2H-indazol-5-yl]-6-(trifluoromethyl)pyridine-2-carboxamide
N-(2-{2-[4-(cyclopropylmethyl)piperazin-1-yl]-2-oxoethyl}-2H-indazol-5-yl)-6-(1-methyl-1H-pyrazol-4-yl)pyridine-2-carboxamide
N-(2-{2-[4-(2-hydroxypropan-2-yl)piperidin-1-yl]-2-oxoethyl}-2H-indazol-5-yl)-6-(1-methyl-1H-pyrazol-4-yl)pyridine-2-carboxamide
6-(1-methyl-1H-pyrazol-4-yl)-N-(2-{2-oxo-2-[4-(pyrrolidin-1-yl)piperidin-1-yl]ethyl}-2H-indazol-5-yl)pyridine-2-carboxamide
N-{2-[2-(4-ethylpiperazin-1-yl)-2-oxoethyl]-2H-indazol-5-yl}-6-(1-methyl-1H-pyrazol-4-yl)pyridine-2-carboxamide
N-(2-{2-[4-(dimethylamino)piperidin-1-yl]-2-oxoethyl}-2H-indazol-5-yl)-6-(1-methyl-1H-pyrazol-4-yl)pyridine-2-carboxamide
N-(2-{2-[(cyclopropylmethyl)(methyl)amino]-2-oxoethyl}-6-methoxy-2H-indazol-5-yl)-6-methylpyridine-2-carboxamide
N-(2-{2-[(cyclopropylmethyl)(methyl)amino]-2-oxoethyl}-6-ethoxy-2H-indazol-5-yl)-6-(trifluoromethyl)pyridine-2-carboxamide
N-(2-{2-[(cyclopropylmethyl)(methyl)amino]-2-oxoethyl}-6-methoxy-2H-indazol-5-yl)-6-(trifluoromethyl)pyridine-2-carboxamide
6-cyclopropyl-N-(2-{2-[4-(2-hydroxypropan-2-yl)piperidin-1-yl]-2-oxoethyl}-6-methoxy-2H-indazol-5-yl)pyridine-2-carboxamide
6-(1-hydroxyethyl)-N-(2-{2-[4-(2-hydroxypropan-2-yl)piperidin-1-yl]-2-oxoethyl}-6-methoxy-2H-indazol-5-yl)pyridine-2-carboxamide
6-(azetidin-3-ylamino)-N-{2-[2-(4-benzoylpiperazin-1-yl)-2-oxoethyl]-2H-indazol-5-yl}pyridine-2-carboxamide
6-[(azetidin-2-ylmethyl)amino]-N-{2-[2-(4-benzoylpiperazin-1-yl)-2-oxoethyl]-2H-indazol-5-yl}pyridine-2-carboxamide
N-{2-[2-(4-benzoylpiperazin-1-yl)-2-oxoethyl]-2H-indazol-5-yl}-6-(3-hydroxyazetidin-1-yl)pyridine-2-carboxamide
6-[(2R,6S)-2,6-dimethylmorpholin-4-yl]-N-(6-methyl-2-{2-oxo-2-[4-(pyrrolidin-1-yl)piperidin-1-yl]ethyl}-2H-indazol-5-yl)pyridine-2-carboxamide
N-[2-(2-{4-methyl-4-[(4-methylpiperazin-1-yl)carbonyl]piperidin-1-yl}-2-oxoethyl)-2H-indazol-5-yl]-6-(trifluoromethyl)pyridine-2-carboxamide
N-(6-chloro-2-{2-oxo-2-[(3R)-piperidin-3-ylamino]ethyl}-2H-indazol-5-yl)-6-(trifluoromethyl)pyridine-2-carboxamide
N-(2-{2-[4-(cyclopropylcarbonyl)piperazin-1-yl]-2-oxoethyl}-6-isopropoxy-2H-indazol-5-yl)-6-methylpyridine-2-carboxamide
N-(2-{2-[4-(cyclopropylcarbonyl)piperazin-1-yl]-2-oxoethyl}-6-isopropoxy-2H-indazol-5-yl)-6-(trifluoromethyl)pyridine-2-carboxamide
N-{2-[2-(4-benzoylpiperazin-1-yl)-2-oxoethyl]-6-isopropoxy-2H-indazol-5-yl}-6-methylpyridine-2-carboxamide
N-{2-[2-(4-benzoylpiperazin-1-yl)-2-oxoethyl]-6-isopropoxy-2H-indazol-5-yl}-6-(trifluoromethyl)pyridine-2-carboxamide
N-{6-isopropoxy-2-[2-(4-methylpiperazin-1-yl)-2-oxoethyl]-2H-indazol-5-yl}-6-(trifluoromethyl)pyridine-2-carboxamide
N-{6-isopropoxy-2-[2-(4-methylpiperazin-1-yl)-2-oxoethyl]-2H-indazol-5-yl}-6-methylpyridine-2-carboxamide
N-(2-{2-[4-(cyclobutylcarbonyl)piperazin-1-yl]-2-oxoethyl}-2H-indazol-5-yl)-6-(trifluoromethyl)pyridine-2-carboxamide
N-(2-{2-[4-(cyclopentylcarbonyl)piperazin-1-yl]-2-oxoethyl}-2H-indazol-5-yl)-6-(trifluoromethyl)pyridine-2-carboxamide
N-[2-(2-{4-[3-(methylsulphonyl)benzoyl]piperazin-1-yl}-2-oxoethyl)-2H-indazol-5-yl]-6-(trifluoromethyl)pyridine-2-carboxamide
N-[2-(2-{4-[2-methoxy-5-(methylsulphonyl)benzoyl]piperazin-1-yl}-2-oxoethyl)-2H-indazol-5-yl]-6-(trifluoromethyl)pyridine-2-carboxamide
6-bromo-N-(6-methyl-2-{2-oxo-2-[4-(pyrrolidin-1-yl)piperidin-1-yl]ethyl}-2H-indazol-5-yl)pyridine-2-carboxamide
2-(4-methoxyphenyl)-N-(6-methyl-2-{2-oxo-2-[4-(pyrrolidin-1-yl)piperidin-1-yl]ethyl}-2H-indazol-5-yl)-1,3-thiazole-4-carboxamide
2-(4-fluorophenyl)-N-(6-methyl-2-{2-oxo-2-[4-(pyrrolidin-1-yl)piperidin-1-yl]ethyl}-2H-indazol-5-yl)-1,3-thiazole-4-carboxamide
N-(6-methyl-2-{2-oxo-2-[4-(pyrrolidin-1-yl)piperidin-1-yl]ethyl}-2H-indazol-5-yl)-6-(trifluoromethyl)pyridine-2-carboxamide
6-bromo-N-{2-[2-(4-methylpiperazin-1-yl)-2-oxoethyl]-2H-indazol-5-yl}pyridine-2-carboxamide
6-bromo-N-{2-[2-(4-methylpiperazin-1-yl)-2-oxoethyl]-6-(trifluoromethoxy)-2H-indazol-5-yl}pyridine-2-carboxamide
N-{2-[2-(4-methylpiperazin-1-yl)-2-oxoethyl]-6-(trifluoromethoxy)-2H-indazol-5-yl}-6-(4H-1,2,4-triazol-4-yl)pyridine-2-carboxamide
2-bromo-N-{6-bromo-2-[2-(4-methylpiperazin-1-yl)-2-oxoethyl]-2H-indazol-5-yl}-1,3-thiazole-4-carboxamide
N-{6-hydroxy-2-[2-(4-methylpiperazin-1-yl)-2-oxoethyl]-2H-indazol-5-yl}-6-(trifluoromethyl)pyridine-2-carboxamide
N-[6-(benzyloxy)-2-{2-[4-(2-hydroxypropan-2-yl)piperidin-1-yl]-2-oxoethyl}-2H-indazol-5-yl]-6-methylpyridine-2-carboxamide
6-bromo-N-{6-bromo-2-[2-(4-methylpiperazin-1-yl)-2-oxoethyl]-2H-indazol-5-yl}pyridine-2-carboxamide
N-{6-(benzyloxy)-2-[2-(4-methylpiperazin-1-yl)-2-oxoethyl]-2H-indazol-5-yl}-6-methylpyridine-2-carboxamide
2-(azetidin-3-ylamino)-N-{2-[2-(4-benzoylpiperazin-1-yl)-2-oxoethyl]-2H-indazol-5-yl}-1,3-thiazole-4-carboxamide
6-acetamido-N-{6-methoxy-2-[2-(morpholin-4-yl)-2-oxoethyl]-2H-indazol-5-yl}pyridine-2-carboxamide
6-(dimethylamino)-N-(2-{2-[4-(2-hydroxypropan-2-yl)piperidin-1-yl]-2-oxoethyl}-6-methoxy-2H-indazol-5-yl)pyridine-2-carboxamide
6-(dimethylamino)-N-{6-methoxy-2-[2-(morpholin-4-yl)-2-oxoethyl]-2H-indazol-5-yl}pyridine-2-carboxamide
6-acetamido-N-(2-{2-[4-(2-hydroxypropan-2-yl)piperidin-1-yl]-2-oxoethyl}-6-methoxy-2H-indazol-5-yl)pyridine-2-carboxamide
6-(dimethylamino)-N-{6-methoxy-2-[2-(4-methylpiperazin-1-yl)-2-oxoethyl]-2H-indazol-5-yl}pyridine-2-carboxamide
N-{2-[2-(4-methylpiperazin-1-yl)-2-oxoethyl]-2H-indazol-5-yl}-6-[3-(methylsulphonyl)phenyl]pyridine-2-carboxamide
N-{2-[1-(4-benzoylpiperazin-1-yl)-1-oxopropan-2-yl]-2H-indazol-5-yl}-6-(trifluoromethyl)pyridine-2-carboxamide
N-[6-chloro-2-(2-{[trans-4-(2-hydroxypropan-2-yl)cyclohexyl]amino}-2-oxoethyl)-2H-indazol-5-yl]-6-(trifluoromethyl)pyridine-2-carboxamide
6-(2-hydroxypropan-2-yl)-N-{6-methoxy-2-[2-(morpholin-4-yl)-2-oxoethyl]-2H-indazol-5-yl}pyridine-2-carboxamide
N-{6-chloro-2-[2-(3,3-difluoropyrrolidin-1-yl)-2-oxoethyl]-2H-indazol-5-yl}-6-(trifluoromethyl)pyridine-2-carboxamide
N-{6-chloro-2-[2-oxo-2-(pyrrolidin-1-yl)ethyl]-2H-indazol-5-yl}-6-(trifluoromethyl)pyridine-2-carboxamide
N-{6-chloro-2-[2-(2-oxa-7-azaspiro[3.5]non-7-yl)-2-oxoethyl]-2H-indazol-5-yl}-6-(trifluoromethyl)pyridine-2-carboxamide
N-(6-chloro-2-{2-[4-(2-hydroxy-2-methylpropyl)piperazin-1-yl]-2-oxoethyl}-2H-indazol-5-yl)-6-(trifluoromethyl)pyridine-2-carboxamide
N-{6-methoxy-2-[2-oxo-2-(pyrrolidin-1-yl)ethyl]-2H-indazol-5-yl}-6-(trifluoromethyl)pyridine-2-carboxamide
N-{2-[2-(3,3-difluoropyrrolidin-1-yl)-2-oxoethyl]-6-methoxy-2H-indazol-5-yl}-6-(trifluoromethyl)pyridine-2-carboxamide
6-(difluoromethyl)-N-{6-methoxy-2-[2-(morpholin-4-yl)-2-oxoethyl]-2H-indazol-5-yl}pyridine-2-carboxamide
N-{2-[2-(3,3-difluoropyrrolidin-1-yl)-2-oxoethyl]-6-methoxy-2H-indazol-5-yl}-6-methylpyridine-2-carboxamide
N-{6-methoxy-2-[2-(morpholin-4-yl)-2-oxoethyl]-2H-indazol-5-yl}-6-methylpyridine-2-carboxamide
N-{6-methoxy-2-[2-(morpholin-4-yl)-2-oxoethyl]-2H-indazol-5-yl}-2-(tetrahydro-2H-pyran-4-yl)-1,3-oxazole-4-carboxamide
N-{2-[2-(1,1-dioxido-1-thia-6-azaspiro[3.3]hept-6-yl)-2-oxoethyl]-6-methoxy-2H-indazol-5-yl}-6-(trifluoromethyl)pyridine-2-carboxamide
N-{6-methoxy-2-[2-(2-oxa-6-azaspiro[3.3]hept-6-yl)-2-oxoethyl]-2H-indazol-5-yl}-6-(trifluoromethyl)pyridine-2-carboxamide
N-{6-(3-hydroxy-2,2-dimethylpropoxy)-2-[2-(morpholin-4-yl)-2-oxoethyl]-2H-indazol-5-yl}-6-(trifluoromethyl)pyridine-2-carboxamide
6-ethyl-N-{6-methoxy-2-[2-(morpholin-4-yl)-2-oxoethyl]-2H-indazol-5-yl}pyridine-2-carboxamide
6-isobutyl-N-{6-methoxy-2-[2-(morpholin-4-yl)-2-oxoethyl]-2H-indazol-5-yl}pyridine-2-carboxamide
methyl 2-[2-(morpholin-4-yl)-2-oxoethyl]-5-({[6-(trifluoromethyl)pyridin-2-yl]carbonyl}amino)-2H-indazole-6-carboxylate
methyl 5-{[(6-methylpyridin-2-yl)carbonyl]amino}-2-[2-(morpholin-4-yl)-2-oxoethyl]-2H-indazole-6-carboxylate
N-{6-methoxy-2-[2-(morpholin-4-yl)-2-oxoethyl]-2H-indazol-5-yl}-6-(pyrrolidin-1-yl)pyridine-2-carboxamide
N-{6-methoxy-2-[2-(morpholin-4-yl)-2-oxoethyl]-2H-indazol-5-yl}-6-(morpholin-4-yl)pyridine-2-carboxamide
6-(cyclopropylamino)-N-{6-methoxy-2-[2-(morpholin-4-yl)-2-oxoethyl]-2H-indazol-5-yl}pyridine-2-carboxamide
6-(butylamino)-N-{6-methoxy-2-[2-(morpholin-4-yl)-2-oxoethyl]-2H-indazol-5-yl}pyridine-2-carboxamide
N-{6-methoxy-2-[2-(morpholin-4-yl)-2-oxoethyl]-2H-indazol-5-yl}-6-(propylamino)pyridine-2-carboxamide
6-(isobutylamino)-N-{6-methoxy-2-[2-(morpholin-4-yl)-2-oxoethyl]-2H-indazol-5-yl}pyridine-2-carboxamide
R-N-{6-methoxy-2-[2-(morpholin-4-yl)-2-oxoethyl]-2H-indazol-5-yl}-6-(2,2,2-trifluoro-1-hydroxyethyl)pyridine-2-carboxamide
S-N-{6-methoxy-2-[2-(morpholin-4-yl)-2-oxoethyl]-2H-indazol-5-yl}-6-(2,2,2-trifluoro-1-hydroxyethyl)pyridine-2-carboxamide
6-(1-hydroxyethyl)-N-{6-methoxy-2-[2-(morpholin-4-yl)-2-oxoethyl]-2H-indazol-5-yl}pyridine-2-carboxamide
6-(cyclopropylamino)-N-{6-methoxy-2-[2-(4-methylpiperazin-1-yl)-2-oxoethyl]-2H-indazol-5-yl}pyridine-2-carboxamide
N-{6-methoxy-2-[2-(4-methylpiperazin-1-yl)-2-oxoethyl]-2H-indazol-5-yl}-6-(propylamino)pyridine-2-carboxamide
6-(isobutylamino)-N-{6-methoxy-2-[2-(4-methylpiperazin-1-yl)-2-oxoethyl]-2H-indazol-5-yl}pyridine-2-carboxamide
6-(1-hydroxyethyl)-N-{6-methoxy-2-[2-(4-methylpiperazin-1-yl)-2-oxoethyl]-2H-indazol-5-yl}pyridine-2-carboxamide
N-{6-methoxy-2-[2-(4-methylpiperazin-1-yl)-2-oxoethyl]-2H-indazol-5-yl}-4-methyl-6-(trifluoromethyl)pyridine-2-carboxamide
N-{6-(benzyloxy)-2-[2-(morpholin-4-yl)-2-oxoethyl]-2H-indazol-5-yl}-6-(trifluoromethyl)pyridine-2-carboxamide
6-(cyclopropylamino)-N-(2-{2-[4-(2-hydroxypropan-2-yl)piperidin-1-yl]-2-oxoethyl}-6-methoxy-2H-indazol-5-yl)pyridine-2-carboxamide
6-(butylamino)-N-(2-{2-[4-(2-hydroxypropan-2-yl)piperidin-1-yl]-2-oxoethyl}-6-methoxy-2H-indazol-5-yl)pyridine-2-carboxamide
N-(2-{2-[4-(2-hydroxypropan-2-yl)piperidin-1-yl]-2-oxoethyl}-6-methoxy-2H-indazol-5-yl)-6-[(2-methoxyethyl)amino]pyridine-2-carboxamide
N-(2-{2-[4-(2-hydroxypropan-2-yl)piperidin-1-yl]-2-oxoethyl}-6-methoxy-2H-indazol-5-yl)-6-(propylamino)pyridine-2-carboxamide
N-(2-{2-[4-(2-hydroxypropan-2-yl)piperidin-1-yl]-2-oxoethyl}-6-methoxy-2H-indazol-5-yl)-6-(isobutylamino)pyridine-2-carboxamide
5-fluoro-N-(2-{2-[4-(2-hydroxypropan-2-yl)piperidin-1-yl]-2-oxoethyl}-6-methoxy-2H-indazol-5-yl)-6-methylpyridine-2-carboxamide
N-{6-hydroxy-2-[2-(morpholin-4-yl)-2-oxoethyl]-2H-indazol-5-yl}-6-(trifluoromethyl)pyridine-2-carboxamide
N-{6-(3-cyanopropoxy)-2-[2-(morpholin-4-yl)-2-oxoethyl]-2H-indazol-5-yl}-6-(trifluoromethyl)pyridine-2-carboxamide
N-{2-[2-(morpholin-4-yl)-2-oxoethyl]-6-(2,2,2-trifluoroethoxy)-2H-indazol-5-yl}-6-(trifluoromethyl)pyridine-2-carboxamide
N-{6-(cyclohexylmethoxy)-2-[2-(morpholin-4-yl)-2-oxoethyl]-2H-indazol-5-yl}-6-(trifluoromethyl)pyridine-2-carboxamide
N-{6-(2,2-dimethylpropoxy)-2-[2-(morpholin-4-yl)-2-oxoethyl]-2H-indazol-5-yl}-6-(trifluoromethyl)pyridine-2-carboxamide
N-{2-[2-(morpholin-4-yl)-2-oxoethyl]-6-(tetrahydrofuran-2-ylmethoxy)-2H-indazol-5-yl}-6-(trifluoromethyl)pyridine-2-carboxamide
N-{6-(cyclopentyloxy)-2-[2-(morpholin-4-yl)-2-oxoethyl]-2H-indazol-5-yl}-6-(trifluoromethyl)pyridine-2-carboxamide
N-{6-(cyanomethoxy)-2-[2-(morpholin-4-yl)-2-oxoethyl]-2H-indazol-5-yl}-6-(trifluoromethyl)pyridine-2-carboxamide
({2-[2-(morpholin-4-yl)-2-oxoethyl]-5-({[6-(trifluoromethyl)pyridin-2-yl]carbonyl}amino)-2H-indazol-6-yl}oxy)acetic acid
N-{6-(cyclobutylmethoxy)-2-[2-(morpholin-4-yl)-2-oxoethyl]-2H-indazol-5-yl}-6-(trifluoromethyl)pyridine-2-carboxamide
N-{2-[2-(morpholin-4-yl)-2-oxoethyl]-6-[2-(pyrrolidin-1-yl)ethoxy]-2H-indazol-5-yl}-6-(trifluoromethyl)pyridine-2-carboxamide
N-{6-[2-(morpholin-4-yl)ethoxy]-2-[2-(morpholin-4-yl)-2-oxoethyl]-2H-indazol-5-yl}-6-(trifluoromethyl)pyridine-2-carboxamide
N-{2-[2-(morpholin-4-yl)-2-oxoethyl]-6-[2-(piperidin-1-yl)ethoxy]-2H-indazol-5-yl}-6-(trifluoromethyl)pyridine-2-carboxamide
N-{6-(3-hydroxypropoxy)-2-[2-(morpholin-4-yl)-2-oxoethyl]-2H-indazol-5-yl}-6-(trifluoromethyl)pyridine-2-carboxamide
N-{6-(2-hydroxypropoxy)-2-[2-(morpholin-4-yl)-2-oxoethyl]-2H-indazol-5-yl}-6-(trifluoromethyl)pyridine-2-carboxamide
N-{6-(2-hydroxyethoxy)-2-[2-(morpholin-4-yl)-2-oxoethyl]-2H-indazol-5-yl}-6-(trifluoromethyl)pyridine-2-carboxamide
N-{6-(2-methoxyethoxy)-2-[2-(morpholin-4-yl)-2-oxoethyl]-2H-indazol-5-yl}-6-(trifluoromethyl)pyridine-2-carboxamide
ethyl ({2-[2-(morpholin-4-yl)-2-oxoethyl]-5-({[6-(trifluoromethyl)pyridin-2-yl]carbonyl}amino)-2H-indazol-6-yl}oxy)acetate
methyl 4-({2-[2-(morpholin-4-yl)-2-oxoethyl]-5-({[6-(trifluoromethyl)pyridin-2-yl]carbonyl}amino)-2H-indazol-6-yl}oxy)butanoate
ethyl 2-({2-[2-(morpholin-4-yl)-2-oxoethyl]-5-({[6-(trifluoromethyl)pyridin-2-yl]carbonyl}amino)-2H-indazol-6-yl}oxy)propanoate
ethyl 3-methyl-2-({2-[2-(morpholin-4-yl)-2-oxoethyl]-5-({[6-(trifluoromethyl)pyridin-2-yl]carbonyl}amino)-2H-indazol-6-yl}oxy)butanoate
2-({2-[2-(morpholin-4-yl)-2-oxoethyl]-5-({[6-(trifluoromethyl)pyridin-2-yl]carbonyl}amino)-2H-indazol-6-yl}oxy)propanoic acid
N-{6-(2-hydroxypropan-2-yl)-2-[2-(morpholin-4-yl)-2-oxoethyl]-2H-indazol-5-yl}-6-(trifluoromethyl)pyridine-2-carboxamide
N-{6-chloro-2-[2-(4-methylpiperazin-1-yl)-2-oxoethyl]-2H-indazol-5-yl}-6-(difluoromethyl)pyridine-2-carboxamide
N-{6-chloro-2-[2-(morpholin-4-yl)-2-oxoethyl]-2H-indazol-5-yl}-6-(difluoromethyl)pyridine-2-carboxamide.

11. Compound of the formula (I) as defined in any of Claims 1 to 10 for the treatment and/or prophylaxis of diseases.

12. Compound of the formula (I) as defined in any of Claims 1 to 10 for use in a method for the treatment and/or prophylaxis of tumour disorders, dermatological disorders, gynaecological disorders, cardiovascular disorders, pulmonary disorders, ophthalmological disorders, neurological disorders, metabolic disorders, inflammatory disorders, autoimmune disorders and pain.

13. Compound of the formula (I) as defined in any of Claims 1 to 10 for use in a method for the treatment and/or prophylaxis of lymphomas, macular degeneration, endometriosis, psoriasis, lupus erythematosus, multiple sclerosis, COPD, rheumatoid arthritis.

14. Use of a compound of the formula (I) as defined in any of Claims 1 to 10 for preparing a medicament for the treatment and/or prophylaxis of tumour disorders, dermatological disorders, gynaecological disorders, cardiovascular disorders, pulmonary disorders, ophthalmological disorders, neurological disorders, metabolic disorders, inflammatory disorders, autoimmune disorders and pain.

15. Compound of the formula (I) as defined in any of Claims 1 to 10 for preparing a medicament for the treatment and/or prophylaxis of lymphomas, macular degeneration, endometriosis, psoriasis, lupus erythematosus, multiple sclerosis, COPD, rheumatoid arthritis.

16. Medicament comprising a compound of the formula (I) as defined in any of Claims 1 to 10 in combination with an inert, non-toxic, pharmaceutically suitable excipient.

17. Process for preparing compounds of the general formula (III) from the compounds of the general formula (II) 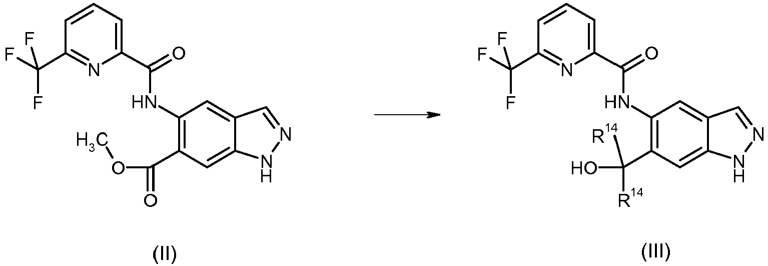 in which R¹⁴ is either a methyl or an ethyl radical by Grignard reaction with methyl- or ethylmagnesium bromide.

18. Compounds of the general formula (III) 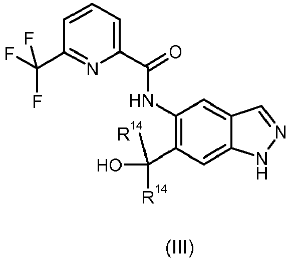 in which R¹⁴ is either a methyl or an ethyl radical.

## Revendications

1. Composés de formule générale (I) 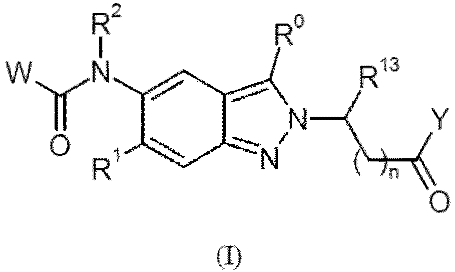 dans laquelle :
R⁰ représente hydrogène ou alkyle en C₁-C₄, le radical alkyle en C₁-C₄ pouvant éventuellement être substitué une ou plusieurs fois, de manière identique ou différente, avec hydroxy ou halogène ;
R¹ représente hydrogène, halogène, cyano, C(=O)OH, C(=O)OR^{a}, C(=O)NH₂, C(=O)N(H)R^{a}, C(=O)N(R^{a})R^{b}, C(=O)R^{d}, hydroxy ou alkyle en C₁-C₆, le radical alkyle en C₁-C₆ pouvant éventuellement être substitué une ou plusieurs fois, de manière identique ou différente, avec
hydroxy, halogène, cyano, C(=O)OH, C(=O)OR^{a}, S(=O)₂-alkyle en C₁-C₆, NH₂, NHR^{a}, N(R^{a})R^{b}, alcoxy en C₁-C₆ éventuellement substitué une ou plusieurs fois, de manière identique ou différente, avec halogène, cycloalcoxy en C₃-C₈ éventuellement substitué une ou plusieurs fois, de manière identique ou différente, avec halogène, hétérocycloalkyle substitué une ou plusieurs fois, de manière identique ou différente, avec R^{c},
ou représente alcoxy en C₁-C₆, le radical alcoxy en C₁-C₆ pouvant éventuellement être substitué une ou plusieurs fois, de manière identique ou différente, avec
hydroxy, halogène, cyano, C(=O)OH, C(=O)OR^{a}, S(=O)₂-alkyle en C₁-C₆, NH₂, NHR^{a}, N(R^{a})R^{b}, cycloalkyle en C₃-C₈ éventuellement substitué une ou plusieurs fois, de manière identique ou différente, avec halogène, alcoxy en C₁-C₆ éventuellement substitué une ou plusieurs fois, de manière identique ou différente, avec halogène, cycloalcoxy en C₃-C₈ éventuellement substitué une ou plusieurs fois, de manière identique ou différente, avec halogène, hétérocycloalkyle éventuellement substitué une ou plusieurs fois, de manière identique ou différente, avec R^{c}, aryle éventuellement substitué une ou plusieurs fois, de manière identique ou différente, avec R^{c}, ou hétéroaryle à 5 ou 6 chaînons éventuellement substitué une ou plusieurs fois, de manière identique ou différente, avec R^{c},
ou représente cycloalcoxy en C₃-C₈ ou hétérocycloalcoxy, qui peuvent éventuellement être substitués une ou plusieurs fois, de manière identique ou différente, avec hydroxy, halogène, cyano ou alkyle en C₁-C₆,
ou représente aryloxy ou hétéroaryloxy à 5 ou 6 chaînons, l'aryloxy et l'hétéroaryloxy à 5 ou 6 chaînons pouvant éventuellement être substitués une ou plusieurs fois, de manière identique ou différente, avec hydroxy, halogène, cyano, C(=O)OH, C(=O)OR^{a}, alkyle en C₁-C₆ ou alcoxy en C₁-C₆,
ou représente cycloalkyle en C₃-C₈ ou hétérocycloalkyle, qui peuvent éventuellement être substitués une ou plusieurs fois, de manière identique ou différente, avec hydroxy, halogène, cyano ou alkyle en C₁-C₆,
C₆, ou représente alcényle en C₂-C₆ ou alcynyle en C₂-
ou représente aryle, hétéroaryle de 5 à 10 chaînons, aryl-alkyle en C₁-C₄ ou hétéroaryle à 5 ou 6 chaînons-alkyle en C₁-C₄, l'aryle et l'hétéroaryle pouvant éventuellement être substitués une ou plusieurs fois, de manière identique ou différente, avec halogène, hydroxy, cyano, C(=O)OH, C(=O)OR^{a}, alkyle en C₁-C₆, cycloalkyle en C₃-C₈ ou alcoxy en C₁-C₆ ;
R^{a} représente alkyle en C₁-C₆, cycloalkyle en C₃-C₁₀, hétérocycloalkyle, aryle ou hétéroaryle, l'alkyle, le cycloalkyle, l'hétérocycloalkyle, l'aryle et l'hétéroaryle pouvant éventuellement être substitués une ou plusieurs fois, de manière identique ou différente, avec halogène, hydroxy, cyano, alkyle en C₁-C₃, alcoxy en C₁-C₃, hétérocycloalkyle, -C(=O)O-alkyle en C₁-C₆ ou S(=O)₂-alkyle en C₁-C₆ ;
R^{b} représente alkyle en C₁-C₆ ou cycloalkyle en C₃-C₁₀ ;
ou R^{a} et R^{b} forment ensemble avec l'atome d'azote un hétérocycle à 5 ou 6 chaînons, qui peut éventuellement être substitué une ou plusieurs fois, de manière identique ou différente, avec hydroxy, halogène, cyano ou alkyle en C₁-C₆ ;
R^{c} représente hydroxy, halogène, cyano, alkyle en C₁-C₃ ou alcoxy en C₁-C₃ ;
R^{d} représente hydrogène, alkyle en C₁-C₆ ou cycloalkyle en C₃-C₁₀ ;
R² représente hydrogène, alkyle en C₁-C₆ ou cycloalkyle en C₃-C₆ ;
R¹³ représente hydrogène ou alkyle en C₁-C₆ ;
W représente hétéroaryle à 5 chaînons, qui contient un à trois hétéroatomes choisis dans le groupe constitué par N, O et S, et peut éventuellement être substitué une fois avec R³ et éventuellement une ou plusieurs fois, de manière identique ou différente, avec R⁴, ou
W représente pyridyle, pyrazinyle, pyridazinyle, 1,2,4-triazinyle ou 1,3,5-triazinyle, qui peuvent éventuellement être substitués une fois avec R³ et éventuellement une ou plusieurs fois, de manière identique ou différente, avec R⁴ ;
R³ représente hydrogène, halogène, cyano, C(=O)R^{a}, NH₂, NHR^{a}, N(R^{a})R^{b}, N(H)C(=O)R^{a} ou alkyle en C₁-C₆,
l'alkyle en C₁-C₆ pouvant éventuellement être substitué une ou plusieurs fois, de manière identique ou différente, avec hydroxy, halogène, cyano, C(=O)R^{a}, C(=O)OH, C(=O)OR^{a}, S(=O)₂-alkyle en C₁-C₆, NH₂, NHR^{a}, N(R^{a})R^{b}, alcoxy en C₁-C₆, cycloalcoxy en C₃-C₈,
l'alcoxy en C₁-C₆ et le cycloalcoxy en C₃-C₈ pouvant éventuellement être substitués une ou plusieurs fois, de manière identique ou différente, avec halogène ;
ou l'alkyle en C₁-C₆ est éventuellement substitué une ou plusieurs fois, de manière identique ou différente, avec cycloalkyle en C₃-C₆ ou hétérocycloalkyle,
le cycloalkyle en C₃-C₆ et l'hétérocycloalkyle pouvant éventuellement être substitués une, deux ou trois fois, de manière identique ou différente, avec halogène, cyano, alkyle en C₁-C₃ ou alcoxy en C₁-C₃,
ou l'alkyle en C₁-C₆ est éventuellement substitué une ou plusieurs fois, de manière identique ou différente, avec aryle ou hétéroaryle à 5 ou 6 chaînons,
l'aryle et l'hétéroaryle à 5 ou 6 chaînons pouvant éventuellement être substitués une, deux ou trois fois, de manière identique ou différente, avec halogène, cyano, alkyle en C₁-C₃ ou alcoxy en C₁-C₃, ou
R³ représente alcoxy en C₁-C₆,
l'alcoxy en C₁-C₆ pouvant éventuellement être substitué une ou plusieurs fois, de manière identique ou différente, avec hydroxy, halogène, cyano, C(=O)OR^{a}, S(=O)₂-alkyle en C₁-C₆, N(R^{a})R^{b}, cycloalkyle en C₃-C₈, alcoxy en C₁-C₄, cycloalcoxy en C₃-C₈,
ou représente cycloalkyle en C₃-C₆, hétérocycloalkyle ou spirocycloalkyle en C₅-C₁₁,
le cycloalkyle, l'hétérocycloalkyle et le spirocycloalkyle pouvant éventuellement être substitués une ou plusieurs fois, de manière identique ou différente, avec hydroxy, halogène, cyano, C(=O)R^{a}, C(=O)OH, C(=O)OR^{a}, alkyle en C₁-C₆ ou alcoxy en C₁-C₄ ;
ou représente aryle ou hétéroaryle de 5 à 10 chaînons,
l'aryle et l'hétéroaryle pouvant éventuellement être substitués une ou plusieurs fois, de manière identique ou différente, avec halogène, hydroxy, cyano, C(=O)OR^{a}, S(=O)₂-alkyle en C₁-C₆, NO₂, NH₂, NHR^{a}, N(R^{a})R^{b}, N(H)C(=O)R^{a}, cycloalkyle en C₃-C₈, alcoxy en C₁-C₃ ou alkyle en C₁-C₃, l'alkyle en C₁-C₃ pouvant éventuellement être substitué une ou plusieurs fois, de manière identique ou différente, avec halogène ;
R⁴ représente halogène, hydroxy, cyano ou alkyle en C₁-C₆, l'alkyle en C₁-C₆ pouvant éventuellement être substitué une ou plusieurs fois, de manière identique ou différente, avec halogène, alcoxy en C₁-C₆, l'alcoxy en C₁-C₆ pouvant éventuellement être substitué une ou plusieurs fois, de manière identique ou différente, avec halogène, alcényle en C₂-C₆, alcynyle en C₂-C₆, cycloalkyle en C₃-C₁₀, hétérocycloalkyle de 3 à 10 chaînons ou aryle, l'aryle pouvant éventuellement être substitué une ou plusieurs fois, de manière identique ou différente, avec R,
ou
R⁴ représente aryle ou hétéroaryle, qui peuvent éventuellement être substitués une ou plusieurs fois, de manière identique ou différente, avec R,
ou
R⁴ représente C(=O)R^{a}, C(=O)NH₂, C(=O)N(H)R^{a}, C(=O)N(R^{a})R^{b}, C(=O)OR^{a}, NH₂, NHR^{a}, N(R^{a})R^{b}, N(H)C(=O)R^{a}, N(R^{a})C(=O)R^{a}, N(H)C(=O)NH₂, N(H)C(=O)NHR^{a}, N(H)C(=O)N(R^{a})R^{b}, N(R^{a})C(=O)NH₂, N(R^{a})C(=O)NHR^{a}, N(R^{a})C(=O)N(R^{a})R^{b}, N(H)C(=O)OR^{a}, N(R^{a})C(=O)OR^{a}, NO₂, N(H)S(=O)R^{a}, N(R^{a})S(=O)R^{a}, N(H)S(=O)₂R^{a}, N(R^{a})S(=O)₂R^{a}, N=S(=O)(R^{a})R^{b}, OC(=O)R^{a}, OC(=O)NH₂, OC(=O)NHR^{a}, OC(=O)N(R^{a})R^{b}, SH, SR^{a}, S(=O)R^{a}, S(=O)₂R^{a}, S(=O)₂NH₂, S(=O)₂NHR^{a}, S(=O)₂N(R^{a})R^{b} ou S(=O)(=N-R^{a})R^{b} ;
R représente halogène, cyano, alkyle en C₁-C₆, alcényle en C₂-C₆, alcynyle en C₂-C₆, cycloalkyle en C₃-C₁₀, hétérocycloalkyle de 3 à 10 chaînons, aryle, hétéroaryle, C(=O)R^{a}, C(=O)NH₂, C(=O)N(H)R^{a}, C(=O)N(R^{a})R^{b}, C(=O)OR^{a}, NH₂, NHR^{a}, N(R^{a})R^{b}, N(H)C(=O)R^{a}, N(R^{a})C(=O)R^{a}, N(H)C(=O)NH₂, N(H)C(=O)NHR^{a}, N(H)C(=O)N(R^{a})R^{b}, N(R^{a})C(=O)NH₂, N(R^{a})C(=O)NHR^{a}, N(R^{a})C(=O)N(R^{a})R^{b}, N(H)C(=O)OR^{a}, N(R^{a})C(=O)OR^{a}, NO₂, N(H)S(=O)R^{a}, N(R^{a})S(=O)R^{a}, N (H) S(=O)₂R^{a}, N(R^{a})S(=O)₂R^{a}, N=S(=O)(R^{a})R^{b}, OH, alcoxy en C₁-C₆, OC(=O)R^{a}, OC(=O)NH₂, OC(=O)NHR^{a}, OC(=O)N(R^{a})R^{b}, SH, SR^{a}, S(=O)R^{a}, S(=O)₂R^{a}, S(=O)₂NH₂, S(=O)₂NHR^{a}, S(=O)₂N(R^{a})R^{b} ou S(=O)(=NR^{a})R^{b} ;
n représente 0 ou 1 ;
Y représente un groupe choisi parmi : 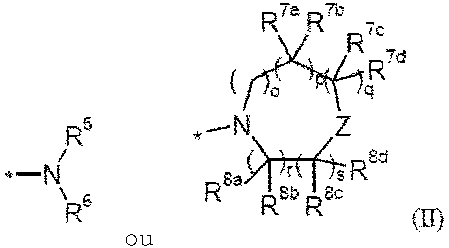 * représentant l'emplacement de liaison du groupe avec le reste de la molécule ;
R⁵ représente hydrogène, alkyle en C₁-C₆ ou cycloalkyle en C₃-C₁₀,
l'alkyle en C₁-C₆ pouvant éventuellement être substitué une ou plusieurs fois, de manière identique ou différente, avec hydroxy, halogène, cyano, C(=O)OH, C(=O)OR^{a}, S(=O)₂-alkyle en C₁-C₆, N(R^{a})R^{b}, alcoxy en C₁-C₄ ou cycloalkyle en C₃-C₈ ;
R⁶ représente hydrogène ou alkyle en C₁-C₆,
l'alkyle en C₁-C₆ pouvant éventuellement être substitué une ou plusieurs fois, de manière identique ou différente, avec hydroxy, halogène, cyano, cycloalkyle en C₃-C₁₀, C(=O)R^{a}, C(=O)OH, C(=O)OR^{a}, S(=O)₂-alkyle en C₁-C₆, N(R^{a})R^{b}, alcoxy en C₁-C₄ ou cycloalcoxy en C₃-C₈,
ou représente cycloalkyle en C₃-C₁₀,
le cycloalkyle en C₃-C₁₀ pouvant éventuellement être substitué une ou plusieurs fois, de manière identique ou différente, avec hydroxy, halogène, cyano ou alkyle en C₁-C₆,
l'alkyle en C₁-C₆ pouvant éventuellement être substitué avec hydroxy,
ou représente hétérocycloalkyle,
l'hétérocycloalkyle pouvant éventuellement être substitué une ou plusieurs fois, de manière identique ou différente, avec halogène, cyano, alkyle en C₁-C₃ ou alcoxy en C₁-C₃,
ou représente aryle ou hétéroaryle à 5 ou 6 chaînons,
l'aryle et l'hétéroaryle à 5 ou 6 chaînons pouvant éventuellement être substitués une ou plusieurs fois, de manière identique ou différente, avec halogène, cyano, alkyle en C₁-C₃, alcoxy en C₁-C₃, S(=O)₂NH₂, S(=O)₂NHR^{a} ou S(=O)₂N(R^{a})R^{b} ;
R^{7a} représente hydrogène, halogène, N(R^{a})R^{b}, alkyle en C₁-C₆ ou cycloalkyle en C₃-C₁₀,
l'alkyle en C₁-C₆ pouvant éventuellement être substitué une ou plusieurs fois, de manière identique ou différente, avec hydroxy, halogène, cyano, C(=O)OH, C(=O)OR^{a}, S(=O)₂-alkyle en C₁-C₆, N(R^{a})R^{b}, alcoxy en C₁-C₄, cycloalkyle en C₃-C₈ ou hétérocycloalkyle ;
R^{7b} représente hydrogène, halogène ou alkyle en C₁-C₆,
l'alkyle en C₁-C₆ pouvant éventuellement être substitué une ou plusieurs fois, de manière identique ou différente, avec hydroxy, halogène, cyano, C(=O)OH, C(=O)OR^{a}, S(=O)₂-alkyle en C₁-C₆, N(R^{a})R^{b}, alcoxy en C₁-C₄, cycloalkyle en C₃-C₈ ou hétérocycloalkyle ;
ou R^{7a} et R^{7b} forment ensemble avec l'atome de carbone un cycloalkyle en C₃-C₆, qui peut éventuellement être substitué une ou plusieurs fois, de manière identique ou différente, avec hydroxy, halogène, cyano ou alkyle en C₁-C₆,
ou R^{7a} et R^{7b} représentent ensemble un groupe oxo ;
R^{7c} représente hydrogène, halogène, N(R^{a})R^{b}, alkyle en C₁-C₆ ou cycloalkyle en C₃-C₁₀,
l'alkyle en C₁-C₆ pouvant éventuellement être substitué une ou plusieurs fois, de manière identique ou différente, avec hydroxy, halogène, cyano, C(=O)OH, C(=O)OR^{a}, S(=O)₂-alkyle en C₁-C₆, N(R^{a})R^{b}, alcoxy en C₁-C₄, cycloalkyle en C₃-C₈ ou hétérocycloalkyle ;
R^{7d} représente hydrogène, halogène ou alkyle en C₁-C₆,
l'alkyle en C₁-C₆ pouvant éventuellement être substitué une ou plusieurs fois, de manière identique ou différente, avec hydroxy, halogène, cyano, C(=O)OH, C(=O)OR^{a}, S(=O)₂-alkyle en C₁-C₆, N(R^{a})R^{b}, alcoxy en C₁-C₄, cycloalkyle en C₃-C₈ ou hétérocycloalkyle ;
ou R^{7c} et R^{7d} forment ensemble avec l'atome de carbone un cycloalkyle en C₃-C₆, qui peut éventuellement être substitué une ou plusieurs fois, de manière identique ou différente, avec hydroxy, halogène, cyano ou alkyle en C₁-C₆,
ou R^{7c} et R^{7d} représentent ensemble un groupe oxo ;
R^{8a} représente hydrogène, halogène, N(R^{a})R^{b}, alkyle en C₁-C₆ ou cycloalkyle en C₃-C₁₀,
l'alkyle en C₁-C₆ pouvant éventuellement être substitué une ou plusieurs fois, de manière identique ou différente, avec hydroxy, halogène, cyano, C(=O)OH, C(=O)OR^{a}, S(=O)₂-alkyle en C₁-C₆, N(R^{a})R^{b}, alcoxy en C₁-C₄, cycloalkyle en C₃-C₈ ou hétérocycloalkyle ;
R^{8b} représente hydrogène, halogène ou alkyle en C₁-C₆,
l'alkyle en C₁-C₆ pouvant éventuellement être substitué une ou plusieurs fois, de manière identique ou différente, avec hydroxy, halogène, cyano, C(=O)OH, C(=O)OR^{a}, S(=O)₂-alkyle en C₁-C₆, N(R^{a})R^{b}, alcoxy en C₁-C₄, cycloalkyle en C₃-C₈ ou hétérocycloalkyle ;
ou R^{8a} et R^{8b} forment ensemble avec l'atome de carbone un cycloalkyle en C₃-C₆, qui peut éventuellement être substitué une ou plusieurs fois, de manière identique ou différente, avec hydroxy, halogène, cyano ou alkyle en C₁-C₆,
R^{8c} représente hydrogène, halogène, N(R^{a})R^{b}, alkyle en C₁-C₆ ou cycloalkyle en C₃-C₁₀,
l'alkyle en C₁-C₆ pouvant éventuellement être substitué une ou plusieurs fois, de manière identique ou différente, avec hydroxy, halogène, cyano, C(=O)OH, C(=O)OR^{a}, S(=O)₂-alkyle en C₁-C₆, N(R^{a})R^{b}, alcoxy en C₁-C₄, cycloalkyle en C₃-C₈ ou hétérocycloalkyle ;
R^{8d} représente hydrogène, halogène ou alkyle en C₁-C₆,
l'alkyle en C₁-C₆ pouvant éventuellement être substitué une ou plusieurs fois, de manière identique ou différente, avec hydroxy, halogène, cyano, C(=O)OH, C(=O)OR^{a}, S(=O)₂-alkyle en C₁-C₆, N(R^{a})R^{b}, alcoxy en C₁-C₄, cycloalkyle en C₃-C₈ ou hétérocycloalkyle ;
ou R^{8c} et R^{8d} forment ensemble avec l'atome de carbone un cycloalkyle en C₃-C₆, qui peut éventuellement être substitué une ou plusieurs fois, de manière identique ou différente, avec hydroxy, halogène, cyano ou alkyle en C₁-C₆,
ou R^{8c} et R^{8d} représentent ensemble un groupe oxo ;
o représente 0, 1 ou 2,
p représente 0, 1 ou 2,
q représente 0, 1 ou 2,
r représente 0, 1 ou 2,
s représente 0, 1 ou 2,
o, p, q, r et s ne signifiant pas simultanément 0 ;
Z représente un groupe choisi parmi C(=O), CR⁹R¹⁰, NR¹¹, O, S, S(=O) ou S(=O)₂ ;
R⁹ représente hydrogène ou alkyle en C₁-C₆,
R¹⁰ représente hydrogène, halogène, cyano, C(=O)R^{a}, C(=O)OH, C(=O)OR^{a}, C(=O)NH₂, C(=O)N(H)R^{a}, C(=O)N(R^{a})R^{b}, N(H)C(=O)R^{a}, N(R^{b})C(=O)R^{a}, S(=O)₂R^{a}, hydroxy, N(R^{a})R^{b} ou alkyle en C₁-C₆,
l'alkyle en C₁-C₆ pouvant éventuellement être substitué une ou plusieurs fois, de manière identique ou différente, avec hydroxy, halogène, cyano, C(=O)R^{a}, C(=O)OH, C(=O)OR^{a}, S(=O)₂-alkyle en C₁-C₆, N(R^{a})R^{b}, alcoxy en C₁-C₄ ou cycloalcoxy en C₃-C₈,
ou représente alcoxy en C₁-C₆,
l'alcoxy en C₁-C₆ pouvant éventuellement être substitué une ou plusieurs fois, de manière identique ou différente, avec hydroxy, halogène, cyano, C(=O)OH, C(=O)OR^{a}, S(=O)₂-alkyle en C₁-C₆, N(R^{a})R^{b}, cycloalkyle en C₃-C₈, alcoxy en C₁-C₄, cycloalcoxy en C₃-C₈, hétérocycloalkyle, aryle ou hétéroaryle à 5 ou 6 chaînons,
l'aryle et l'hétéroaryle à 5 ou 6 chaînons pouvant éventuellement être substitués une ou plusieurs fois, de manière identique ou différente, avec halogène, cyano, alkyle en C₁-C₃ ou alcoxy en C₁-C₃,
ou représente aryloxy ou hétéroaryloxy à 5 ou 6 chaînons,
l'aryloxy et l'hétéroaryloxy à 5 ou 6 chaînons pouvant éventuellement être substitués une ou plusieurs fois, de manière identique ou différente, avec hydroxy, halogène, cyano, C(=O)OH, C(=O)OR^{a}, alkyle en C₁-C₃ ou alcoxy en C₁-C₃,
ou représente cycloalkyle en C₃-C₈, cycloalkyle en C₃-C₈-alkyle en C₁-C₄, hétérocycloalkyle ou hétérocycloalkyle-alkyle en C₁-C₄, qui peuvent éventuellement être substitués une ou plusieurs fois, de manière identique ou différente, avec hydroxy, halogène, cyano, C(=O)R^{a}, C(=O)OH, C(=O)OR^{a}, alkyle en C₁-C₆ ou alcoxy en C₁-C₆,
l'alcoxy en C₁-C₆ pouvant éventuellement être substitué une ou plusieurs fois, de manière identique ou différente, avec halogènee ou un groupe oxo,
ou représente alcényle en C₂-C₆ ou alcynyle en C₂-C₆,
ou représente aryle, hétéroaryle de 5 à 10 chaînons, aryl-alkyle en C₁-C₄ ou hétéroaryle à 5 ou 6 chaînons-alkyle en C₁-C₄,
l'aryle et l'hétéroaryle pouvant éventuellement être substitués une ou plusieurs fois, de manière identique ou différente, avec halogène, hydroxy, cyano, C(=O)OH, C(=O)OR^{a}, NHR^{a}, N(R^{a})R^{b}, alkyle en C₁-C₃, cycloalkyle en C₃-C₈ ou alcoxy en C₁-C₃ ;
ou R⁹ et R¹⁰ forment ensemble avec l'atome de carbone un cycloalkyle en C₃-C₈ ou un hétérocycle de 4 à 6 chaînons,
le radical cycloalkyle en C₃-C₈ ou l'hétérocyle de 4 à 6 chaînons pouvant éventuellement être substitués une ou plusieurs fois, de manière identique ou différente, avec hydroxy, halogène, cyano, alkyle en C₁-C₆, C(=O)R^{a} ou un groupe oxo;
R¹¹ représente hydrogène, C(=O)R^{a}, C(=O)OR^{a}, C(=O)NH₂, C(=O)N(H)R^{a}, C(=O)N(R^{a})R^{b}, S(=O)₂R^{a} S(=O)₂N(R^{a})R^{b} ou alkyle en C₁-C₆,
l'alkyle en C₁-C₆ pouvant éventuellement être substitué une ou plusieurs fois, de manière identique ou différente, avec hydroxy, halogène, cyano, C(=O)R^{a}, C(=O)OR^{a}, C(=O)NH₂, C(=O)N(H)R^{a}, C(=O)N(R^{a})R^{b}, S(=O)₂-alkyle en C₁-C₆, N(R^{a})R^{b}, cycloalkyle en C₃-C₈, alcoxy en C₁-C₄ ou cycloalcoxy en C₃-C₈,
le cycloalkyle en C₃-C₈, l'alcoxy en C₁-C₄ et le cycloalcoxy en C₃-C₈ pouvant éventuellement être substitués une ou plusieurs fois, de manière identique ou différente, avec hydroxy ou halogène ;
ou représente cycloalkyle en C₃-C₈, hétérocycloalkyle ou hétérocycloalkyle-alkyle en C₁-C₄, qui peuvent éventuellement être substitués une ou plusieurs fois, de manière identique ou différente, avec hydroxy, halogène, cyano, alkyle en C₁-C₆, alcoxy en C₁-C₆, l'alkyle et l'alcoxy pouvant éventuellement être substitués une ou plusieurs fois, de manière identique ou différente, avec halogène ou un groupe oxo,
ou représente alcényle en C₂-C₆ ou alcynyle en C₂-C₆,
ou représente aryle, hétéroaryle de 5 à 10 chaînons, aryl-alkyle en C₁-C₄ ou hétéroaryle à 5 ou 6 chaînons-alkyle en C₁-C₄,
l'aryle et l'hétéroaryle pouvant éventuellement être substitués une ou plusieurs fois, de manière identique ou différente, avec halogène, hydroxy, cyano, C(=O)OH, C(=O)OR^{a}, alkyle en C₁-C₃, cycloalkyle en C₃-C₈ ou alcoxy en C₁-C₃ ;
et leurs diastéréomères, énantiomères, leurs sels, leurs solvates et les solvates de leurs sels.

2. Composés selon la revendication 1, dans lesquels R¹ représente hydrogène, halogène, hydroxy, cyano, alkyle en C₁-C₆, alkyle en C₁-C₅ substitué avec hydroxy, alcoxy en C₁-C₆, alcoxy en C₁-C₆ substitué avec cycloalkyle en C₃-C₈, alcoxy en C₁-C₆ substité avec jusqu'à trois atomes de fluor, alcoxy en C₁-C₆ substitué avec un aryle éventuellement substitué une ou plusieurs fois, de manière identique ou différente, avec R^{c}, ou un hétéroaryle à 5 ou 6 chaînons éventuellement substitué une ou plusieurs fois, de manière identique ou différente, avec R^{c}.

3. Composés selon la revendication 1, dans lesquels W est un groupe choisi parmi les formules (III) à (IX) : 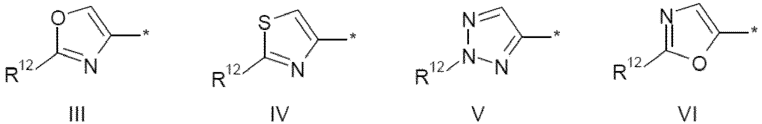 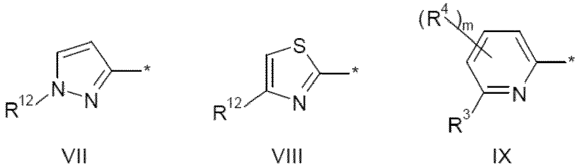 dans lesquelles
R¹² représente hydrogène, halogène, un alkyle en C₁-C₆ éventuellement substitué une ou plusieurs fois, de manière identique ou différente, avec halogène, un cycloalkyle en C₃-C₆ éventuellement substitué une ou plusieurs fois, de manière identique ou différente, avec halogène, un aryle éventuellement substitué une ou plusieurs fois, de manière identique ou différente,
avec R^{c}, un hétéroaryle à 5 ou 6 chaînons éventuellement substitué une ou plusieurs fois, de manière identique ou différente, avec R^{c}, ou NHR^{a} ;
m représente 0, 1, 2 ou 3, et
R³ et R⁴ ont les significations indiquées précédemment, et
* représente l'emplacement de liaison du groupe avec le reste de la molécule.

4. Composés selon la revendication 1, dans lesquels W représente un groupe de formule générale (X) 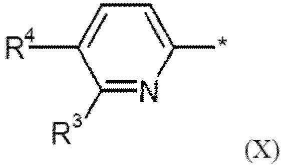 et R³ et R⁴ ont la signification indiquée dans la revendication 1.

5. Composés selon la revendication 1, dans lesquels Y est un groupe de formule générale (II) avec les définitions indiquées dans la revendication 1 pour R^{7a}, R^{7b}, R^{7c}, R^{7d}, R^{8a}, R^{8b}, R^{8c} et R^{8d}

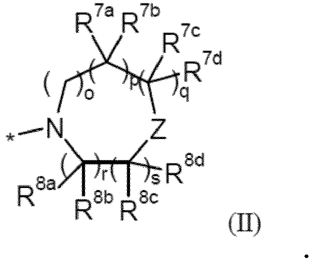

6. Composés selon la revendication 1, dans lesquels Y est un radical NR⁵R⁶ avec les définitions indiquées dans la revendication 1 pour R⁵ et R⁶.

7. Composés selon la revendication 1, dans lesquels W est un groupe de formule générale (IX) 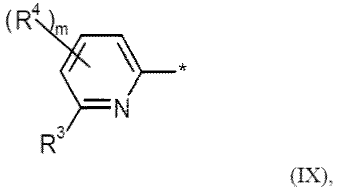 dans laquelle
m représente 0 et R², R⁰ et R¹³ signifient simultanémnt hydrogène, et R³ signifie trifluorométhyle, éthyle, méthyle, cyclopropyle, 2,2,2-trifluoro-1-hydroxyéthyle ou 1-hydroxyéthyle ; Y représente 4-méthylpipérazin-1-yle, 4-éthylpipérazin-1-yle ou morpholin-4-yle, n représente 0 et R¹ représente cyclopropylméthoxy, trifluorométhoxy, 2,2,2-trifluoroéthoxy, chlore, éthoxy, méthoxy, 2-hydroxypropan-2-yle ou 3-hydroxypentan-3-yle.

8. Composés selon la revendication 7, dans lesquels R¹ représente cyclopropylméthoxy, méthoxy, éthoxy ou 2-hydroxypropan-2-yle.

9. Composés selon la revendication 7, dans lesquels R³ est un radical trifluorométhyle ou cyclopropyle.

10. Composés selon la revendication 1, à savoir
N-{2-[2-(4-benzoylpipérazin-1-yl)-2-oxoéthyl]-6-méthyl-2H-indazol-5-yl}-6-(trifluorométhyl)pyridine-2-carboxamide
6-éthyl-N-(6-méthyl-2-{2-oxo-2-[4-(pyrrolidin-1-yl)pipéridin-1-yl]éthyl}-2H-indazol-5-yl)pyridine-2-carboxamide
5-fluoro-N-(6-méthyl-2-{2-oxo-2-[4-(pyrrolidin-1-yl)pipéridin-1-yl]éthyl}-2H-indazol-5-yl)pyridine-2-carboxamide
N-(2-{2-[4-(3-hydroxy-2,2-diméthylpropanoyl)pipérazin-1-yl]-2-oxoéthyl}-6-méthyl-2H-indazol-5-yl)-6-(trifluorométhyl)pyridine-2-carboxamide
N-(2-{2-[4-(méthoxyacétyl)pipérazin-1-yl]-2-oxoéthyl}-6-méthyl-2H-indazol-5-yl)-6-(trifluorométhyl)pyridine-2-carboxamide
N-(2-{2-[4-(2-hydroxypropan-2-yl)pipéridin-1-yl]-2-oxoéthyl}-6-méthoxy-2H-indazol-5-yl)-6-(trifluorométhyl)pyridine-2-carboxamide
N-(2-{2-[4-(2-hydroxypropan-2-yl)pipéridin-1-yl]-2-oxoéthyl}-6-méthoxy-2H-indazol-5-yl)-6-méthylpyridine-2-carboxamide
N-(2-{2-[4-(cyclopropylméthyl)pipérazin-1-yl]-2-oxoéthyl}-6-méthoxy-2H-indazol-5-yl)-6-(trifluorométhyl)pyridine-2-carboxamide
N-(2-{2-[4-(cyclopropylméthyl)pipérazin-1-yl]-2-oxoéthyl}-6-méthoxy-2H-indazol-5-yl)-6-méthylpyridine-2-carboxamide
N-{2-[2-(4-benzoylpipérazin-1-yl)-2-oxoéthyl]-6-méthoxy-2H-indazol-5-yl}-6-cyclopropylpyridine-2-carboxamide
N-{2-[2-(4-benzoylpipérazin-1-yl)-2-oxoéthyl]-6-méthoxy-2H-indazol-5-yl}-6-(1-hydroxyéthyl)pyridine-2-carboxamide
N-{2-[2-(4-méthylpipérazin-1-yl)-2-oxoéthyl]-6-(trifluorométhoxy)-2H-indazol-5-yl}-6-(trifluorométhyl)pyridine-2-carboxamide
6-méthyl-N-{2-[2-(4-méthylpipérazin-1-yl)-2-oxoéthyl]-6-(trifluorométhoxy)-2H-indazol-5-yl}pyridine-2-carboxamide
3-{[4-({2-[2-(4-méthylpipérazin-1-yl)-2-oxoéthyl]-6-(trifluorométhoxy)-2H-indazol-5-yl}carbamoyl)-1,3-thiazol-2-yl]amino}azétidine-1-carboxylate de tert-butyle
N-{6-bromo-2-[2-(4-méthylpipérazin-1-yl)-2-oxoéthyl]-2H-indazol-5-yl}-6-(trifluorométhyl)pyridine-2-carboxamide
N-{6-bromo-2-[2-(4-méthylpipérazin-1-yl)-2-oxoéthyl]-2H-indazol-5-yl}-6-méthylpyridine-2-carboxamide
N-{6-bromo-2-[2-(4-méthylpipérazin-1-yl)-2-oxoéthyl]-2H-indazol-5-yl}-2-cyclopropyl-1,3-oxazole-4-carboxamide
3-{[4-({6-bromo-2-[2-(4-méthylpipérazin-1-yl)-2-oxoéthyl]-2H-indazol-5-yl}carbamoyl)-1,3-thiazol-2-yl]amino}azétidin-1-carboxylate de tert-butyle
2-(azétidin-3-ylamino)-N-{2-[2-(4-méthylpipérazin-1-yl)-2-oxoéthyl]-6-(trifluorométhoxy)-2H-indazol-5-yl}-1,3-thiazole-4-carboxamide
N-{6-cyano-2-[2-(4-méthylpipérazin-1-yl)-2-oxoéthyl]-2H-indazol-5-yl}-6-(trifluorométhyl)pyridine-2-carboxamide
6'-méthyl-N-{2-[2-(4-méthylpipérazin-1-yl)-2-oxoéthyl]-2H-indazol-5-yl}-2,3'-bipyridine-6-carboxamide
5'-méthyl-N-{2-[2-(4-méthylpipérazin-1-yl)-2-oxoéthyl]-2H-indazol-5-yl}-2,3'-bipyridine-6-carboxamide
4'-méthyl-N-{2-[2-(4-méthylpipérazin-1-yl)-2-oxoéthyl]-2H-indazol-5-yl}-2,3'-bipyridine-6-carboxamide
6'-méthoxy-N-{2-[2-(4-méthylpipérazin-1-yl)-2-oxoéthyl]-2H-indazol-5-yl}-2,3'-bipyridine-6-carboxamide
6'-acétamido-N-{2-[2-(4-méthylpipérazin-1-yl)-2-oxoéthyl]-2H-indazol-5-yl}-2,3'-bipyridine-6-carboxamide
N-{2-[2-(4-méthylpipérazin-1-yl)-2-oxoéthyl]-2H-indazol-5-yl}-6'-nitro-2,3'-bipyridine-6-carboxamide
6'-amino-N-{2-[2-(4-méthylpipérazin-1-yl)-2-oxoéthyl]-2H-indazol-5-yl}-2,3'-bipyridine-6-carboxamide
N-{2-[2-(4-benzoylpipérazin-1-yl)-2-oxoéthyl]-6-fluoro-2H-indazol-5-yl}-6-(trifluorométhyl)pyridine-2-carboxamide
N-(2-{2-[4-(cyclopropylcarbonyl)pipérazin-1-yl]-2-oxoéthyl}-6-fluoro-2H-indazol-5-yl)-6-(trifluorométhyl)pyridine-2-carboxamide
N-{6-fluoro-2-[2-(4-méthylpipérazin-1-yl)-2-oxoéthyl]-2H-indazol-5-yl}-6-(trifluorométhyl)pyridine-2-carboxamide
N-(2-{2-[4-(cyclopropylcarbonyl)pipérazin-1-yl]-2-oxoéthyl}-6-fluoro-2H-indazol-5-yl)-6-méthylpyridine-2-carboxamide
N-{6-fluoro-2-[2-(4-méthylpipérazin-1-yl)-2-oxoéthyl]-2H-indazol-5-yl}-6-méthylpyridine-2-carboxamide
N-(2-{2-[4-(cyclopropylcarbonyl)pipérazin-1-yl]-2-oxoéthyl}-6-fluoro-2H-indazol-5-yl)-6-(1-méthyl-1H-pyrazol-4-yl)pyridine-2-carboxamide
N-{2-[2-(4-benzoylpipérazin-1-yl)-2-oxoéthyl]-6-fluoro-2H-indazol-5-yl}-6-(1-méthyl-1H-pyrazol-4-yl)pyridine-2-carboxamide
N-{6-fluoro-2-[2-(4-méthylpipérazin-1-yl)-2-oxoéthyl]-2H-indazol-5-yl}-6-(1-méthyl-1H-pyrazol-4-yl)pyridine-2-carboxamide
N-{2-[2-(4-benzoylpipérazin-1-yl)-2-oxoéthyl]-6-fluoro-2H-indazol-5-yl}-5-fluoro-6-(1-méthyl-1H-pyrazol-4-yl)pyridine-2-carboxamide
N-(2-{2-[4-(cyclopropylcarbonyl)pipérazin-1-yl]-2-oxoéthyl}-6-fluoro-2H-indazol-5-yl)-5-fluoro-6-(1-méthyl-1H-pyrazol-4-yl)pyridine-2-carboxamide
N-{2-[2-(4-benzoylpipérazin-1-yl)-2-oxoéthyl]-6-fluoro-2H-indazol-5-yl}-6-(morpholin-4-yl)pyridine-2-carboxamide
N-(2-{2-[4-(cyclopropylcarbonyl)pipérazin-1-yl]-2-oxoéthyl}-6-fluoro-2H-indazol-5-yl)-6-(morpholin-4-yl)pyridine-2-carboxamide
N-{6-fluoro-2-[2-(4-méthylpipérazin-1-yl)-2-oxoéthyl]-2H-indazol-5-yl}-6-(morpholin-4-yl)pyridine-2-carboxamide
N-{6-(benzyloxy)-2-[2-(4-méthylpipérazin-1-yl)-2-oxoéthyl]-2H-indazol-5-yl}-6-(trifluorométhyl)pyridine-2-carboxamide
N-{6-isobutoxy-2-[2-(4-méthylpipérazin-1-yl)-2-oxoéthyl]-2H-indazol-5-yl}-6-(trifluorométhyl)pyridine-2-carboxamide
N-{6-isobutoxy-2-[2-(morpholin-4-yl)-2-oxoéthyl]-2H-indazol-5-yl}-6-(trifluorométhyl)pyridine-2-carboxamide
N-(2-{2-[4-(2-hydroxypropan-2-yl)pipéridin-1-yl]-2-oxoéthyl}-6-isobutoxy-2H-indazol-5-yl)-6-(trifluorométhyl)pyridine-2-carboxamide
N-(2-{2-[(cyclopropylméthyl)(méthyl)amino]-2-oxoéthyl}-6-isobutoxy-2H-indazol-5-yl)-6-(trifluorométhyl)pyridine-2-carboxamide
N-{6-(cyclopropylméthoxy)-2-[2-(4-méthylpipérazin-1-yl)-2-oxoéthyl]-2H-indazol-5-yl}-6-(trifluorométhyl)pyridine-2-carboxamide
N-{6-(cyclopropylméthoxy)-2-[2-(morpholin-4-yl)-2-oxoéthyl]-2H-indazol-5-yl}-6-(trifluorométhyl)pyridine-2-carboxamide
N-[6-(cyclopropylméthoxy)-2-{2-[4-(2-hydroxypropan-2-yl)pipéridin-1-yl]-2-oxoéthyl}-2H-indazol-5-yl]-6-(trifluorométhyl)pyridine-2-carboxamide
N-[6-(cyclopropylméthoxy)-2-{2-[(cyclopropylméthyl) (méthyl)amino]-2-oxoéthyl}-2H-indazol-5-yl]-6-(trifluorométhyl)pyridine-2-carboxamide
N-{2-[2-(4-méthylpipérazin-1-yl)-2-oxoéthyl]-6-(pyridin-2-ylméthoxy)-2H-indazol-5-yl}-6-(trifluorométhyl)pyridine-2-carboxamide
N-{2-[2-(morpholin-4-yl)-2-oxoéthyl]-6-(pyridin-2-ylméthoxy)-2H-indazol-5-yl}-6-(trifluorométhyl)pyridine-2-carboxamide
N-[2-{2-[4-(2-hydroxypropan-2-yl)pipéridin-1-yl]-2-oxoéthyl}-6-(pyridin-2-ylméthoxy)-2H-indazol-5-yl]-6-(trifluorométhyl)pyridine-2-carboxamide
N-[2-{2-[(cyclopropylméthyl)(méthyl)amino]-2-oxoéthyl}-6-(pyridin-2-ylméthoxy)-2H-indazol-5-yl]-6-(trifluorométhyl)pyridine-2-carboxamide
N-{2-[2-(4-benzoylpipérazin-1-yl)-2-oxoéthyl]-6-chloro-2H-indazol-5-yl}-6-(trifluorométhyl)pyridine-2-carboxamide
N-{6-chloro-2-[2-(morpholin-4-yl)-2-oxoéthyl]-2H-indazol-5-yl}-6-(trifluorométhyl)pyridine-2-carboxamide
4-{[6-chloro-5-({[6-(trifluorométhyl)pyridin-2-yl]carbonyl}amino)-2H-indazol-2-yl]acétyl}pipérazine-1-carboxylate d'éthyle
N-(6-chloro-2-{2-oxo-2-[4-(pyrrolidin-1-yl)pipéridin-1-yl]éthyl}-2H-indazol-5-yl)-6-(trifluorométhyl)pyridine-2-carboxamide
N-(6-chloro-2-{2-[4-(2-hydroxypropan-2-yl)pipéridin-1-yl]-2-oxoéthyl}-2H-indazol-5-yl)-6-(trifluorométhyl)pyridine-2-carboxamide
N-(6-chloro-2-{2-[4-(3-hydroxy-2,2-diméthylpropanoyl)pipérazin-1-yl]-2-oxoéthyl}-2H-indazol-5-yl)-6-(trifluorométhyl)pyridine-2-carboxamide
N-(6-chloro-2-{2-[3-(diméthylamino)azétidin-1-yl]-2-oxoéthyl}-2H-indazol-5-yl)-6-(trifluorométhyl)pyridine-2-carboxamide
N-(6-chloro-2-{2-oxo-2-[3-(pipéridin-1-yl)azétidin-1-yl]éthyl}-2H-indazol-5-yl)-6-(trifluorométhyl)pyridine-2-carboxamide
N-(6-chloro-2-{2-[4-(2-hydroxy-2-méthylpropyl)pipéridin-1-yl]-2-oxoéthyl}-2H-indazol-5-yl)-6-(trifluorométhyl)pyridine-2-carboxamide
N-{6-chloro-2-[2-(4-hydroxy-1,4'-bipipéridin-1'-yl)-2-oxoéthyl]-2H-indazol-5-yl}-6-(trifluorométhyl)pyridine-2-carboxamide
N-{6-méthoxy-2-[2-(4-méthylpipérazin-1-yl)-2-oxoéthyl]-2H-indazol-5-yl}-6-(trifluorométhyl)pyridine-2-carboxamide
N-{6-méthoxy-2-[2-(morpholin-4-yl)-2-oxoéthyl]-2H-indazol-5-yl}-6-(trifluorométhyl)pyridine-2-carboxamide
N-(2-{2-[4-(diméthylamino)pipéridin-1-yl]-2-oxoéthyl}-6-éthoxy-2H-indazol-5-yl)-6-(trifluorométhyl)pyridine-2-carboxamide
N-(6-éthoxy-2-{2-oxo-2-[4-(pyrrolidin-1-yl)pipéridin-1-yl]éthyl}-2H-indazol-5-yl)-6-(trifluorométhyl)pyridine-2-carboxamide
N-{6-éthoxy-2-[2-(4-méthylpipérazin-1-yl)-2-oxoéthyl]-2H-indazol-5-yl}-6-(trifluorométhyl)pyridine-2-carboxamide
N-{2-[2-(4-benzoylpipérazin-1-yl)-2-oxoéthyl]-6-éthoxy-2H-indazol-5-yl}-6-(trifluorométhyl)pyridine-2-carboxamide
N-(6-éthoxy-2-{2-[4-(2-hydroxypropan-2-yl)pipéridin-1-yl]-2-oxoéthyl}-2H-indazol-5-yl)-6-(trifluorométhyl)pyridine-2-carboxamide
N-{6-éthoxy-2-[2-(morpholin-4-yl)-2-oxoéthyl]-2H-indazol-5-yl}-6-(trifluorométhyl)pyridine-2-carboxamide
N-{2-[2-(4-benzoylpipérazin-1-yl)-2-oxoéthyl]-3-méthyl-2H-indazol-5-yl}-6-(trifluorométhyl)pyridine-2-carboxamide
N-{2-[3-(4-benzoylpipérazin-1-yl)-3-oxopropyl]-2H-indazol-5-yl}-6-(trifluorométhyl)pyridine-2-carboxamide
N-(2-{2-[4-(cyclopropylcarbonyl)pipérazin-1-yl]-2-oxoéthyl}-2H-indazol-5-yl)-2-(pyridin-3-yl)-1,3-thiazole-4-carboxamide
N-(2-{2-[4-(cyclopropylcarbonyl)pipérazin-l-yl]-2-oxoéthyl}-2H-indazol-5-yl)-2-(pyridin-4-yl)-1,3-thiazole-4-carboxamide
N-(2-{2-[4-(cyclopropylcarbonyl)pipérazin-1-yl]-2-oxoéthyl}-2H-indazol-5-yl)-6-(trifluorométhyl)pyridine-2-carboxamide
6-(azétidin-3-ylamino)-N-(2-{2-[4-(cyclopropylcarbonyl)pipérazin-1-yl]-2-oxoéthyl}-2H-indazol-5-yl)pyridine-2-carboxamide
N-{2-[2-(4-méthylpipérazin-1-yl)-2-oxoéthyl]-2H-indazol-5-yl}-2-(pyridin-3-yl)-1,3-thiazole-4-carboxamide
N-{2-[2-(4-méthylpipérazin-1-yl)-2-oxoéthyl]-2H-indazol-5-yl}-6-(1-méthyl-1H-pyrazol-4-yl)pyridin-2-carboxamide
N-{2-[2-(4-méthylpipérazin-1-yl)-2-oxoéthyl]-2H-indazol-5-yl}-6-(1H-pyrazol-4-yl)pyridine-2-carboxamide
6-(1,3-diméthyl-1H-pyrazol-4-yl)-N-{2-[2-(4-méthylpipérazin-1-yl)-2-oxoéthyl]-2H-indazol-5-yl}pyridine-2-carboxamide
N-{2-[2-(4-méthylpipérazin-1-yl)-2-oxoéthyl]-2H-indazol-5-yl}-6-[3-(trifluorométhyl)-1H-pyrazol-4-yl]pyridine-2-carboxamide
6-éthyl-N-{2-[2-(4-méthylpipérazin-1-yl)-2-oxoéthyl]-2H-indazol-5-yl}pyridine-2-carboxamide
6-(1-méthyl-1H-pyrazol-4-yl)-N-(2-{2-oxo-2-[4-(2,2,2-trifluoroéthyl)pipérazin-1-yl]éthyl}-2H-indazol-5-yl)pyridine-2-carboxamide
N-(2-{2-oxo-2-[4-(2,2,2-trifluoroéthyl)pipérazin-1-yl]éthyl}-2H-indazol-5-yl)-6-(trifluorométhyl)pyridine-2-carboxamide
N-{2-[2-(4-éthyl-3-oxopipérazin-1-yl)-2-oxoéthyl]-2H-indazol-5-yl}-6-(1-méthyl-1H-pyrazol-4-yl)pyridine-2-carboxamide
N-{2-[2-(4-benzoylpipérazin-1-yl)-2-oxoéthyl]-2H-indazol-5-yl}-6-(trifluorométhyl)pyridine-2-carboxamide
N-{2-[2-(4-benzoylpipérazin-1-yl)-2-oxoéthyl]-2H-indazol-5-yl}-6-méthylpyridine-2-carboxamide
N-{2-[2-(4-benzoylpipérazin-1-yl)-2-oxoéthyl]-2H-indazol-5-yl}-6-(morpholin-4-yl)pyridine-2-carboxamide
N-{2-[2-(4-benzoylpipérazin-1-yl)-2-oxoéthyl]-2H-indazol-5-yl}-2-(pyridin-4-yl)-1,3-thiazole-4-carboxamide
N-{2-[2-(4-benzoylpipérazin-1-yl)-2-oxoéthyl]-2H-indazol-5-yl}-6-chloropyridine-2-carboxamide
N-{2-[2-(4-benzoylpipérazin-1-yl)-2-oxoéthyl]-2H-indazol-5-yl}-2-méthyl-1,3-oxazole-5-carboxamide
6-amino-N-{2-[2-(4-benzoylpipérazin-1-yl)-2-oxoéthyl]-2H-indazol-5-yl}pyridine-2-carboxamide
N-{2-[2-(4-benzoylpipérazin-1-yl)-2-oxoéthyl]-2H-indazol-5-yl}-2-méthyl-1,3-oxazole-4-carboxamide
N-{2-[2-(4-benzoylpipérazin-1-yl)-2-oxoéthyl]-2H-indazol-5-yl}-6-méthoxypyridine-2-carboxamide
N-{2-[2-(4-benzoylpipérazin-1-yl)-2-oxoéthyl]-2H-indazol-5-yl}-2-cyclopropyl-1,3-oxazole-4-carboxamide
N-{2-[2-(4-benzoylpipérazin-1-yl)-2-oxoéthyl]-2H-indazol-5-yl}-6-(4H-1,2,4-triazol-4-yl)pyridine-2-carboxamide
N-{2-[2-(4-benzoylpipérazin-1-yl)-2-oxoéthyl]-2H-indazol-5-yl}-2-phényl-2H-1,2,3-triazole-4-carboxamide
N-{2-[2-(4-benzoylpipérazin-1-yl)-2-oxoéthyl]-2H-indazol-5-yl}-6-(1-méthyl-1H-pyrazol-5-yl)pyridine-2-carboxamide
N-{2-[2-(4-benzoylpipérazin-1-yl)-2-oxoéthyl]-2H-indazol-5-yl}-2-(trifluorométhyl)-1,3-thiazole-4-carboxamide
N-{2-[2-(4-benzoylpipérazin-1-yl)-2-oxoéthyl]-2H-indazol-5-yl}-6-(1H-pyrazol-1-yl)pyridine-2-carboxamide
N-{2-[2-(4-benzoylpipérazin-1-yl)-2-oxoéthyl]-2H-indazol-5-yl}-6-(1-méthyl-1H-pyrazol-4-yl)pyridine-2-carboxamide
N-{2-[2-(4-benzoylpipérazin-1-yl)-2-oxoéthyl]-2H-indazol-5-yl}-1-éthyl-1H-pyrazole-3-carboxamide
N-{2-[2-(4-benzoylpipérazin-1-yl)-2-oxoéthyl]-2H-indazol-5-yl}-6-(4-chloro-1H-pyrazol-1-yl)pyridine-2-carboxamide
N-{2-[2-(4-benzoylpipérazin-1-yl)-2-oxoéthyl]-2H-indazol-5-yl}-4-(trifluorométhyl)-1,3-thiazole-2-carboxamide
N-{2-[2-(4-benzoylpipérazin-1-yl)-2-oxoéthyl]-2H-indazol-5-yl}-6-(1,3-diméthyl-1H-pyrazol-4-yl)pyridine-2-carboxamide
N-{2-[2-(4-benzoylpipérazin-1-yl)-2-oxoéthyl]-2H-indazol-5-yl}-2,4'-bipyridine-6-carboxamide
N-{2-[2-(4-benzoylpipérazin-1-yl)-2-oxoéthyl]-2H-indazol-5-yl}-6-(1H-pyrazol-4-yl)pyridine-2-carboxamide
N-{2-[2-(4-benzoylpipérazin-1-yl)-2-oxoéthyl]-2H-indazol-5-yl}-5-fluoro-6-(1-méthyl-1H-pyrazol-4-yl)pyridine-2-carboxamide
N-{2-[2-(4-benzoylpipérazin-1-yl)-2-oxoéthyl]-2H-indazol-5-yl}-6-(3-méthyl-1H-pyrazol-4-yl)pyridine-2-carboxamide
N-{2-[2-(4-benzoylpipérazin-1-yl)-2-oxoéthyl]-2H-indazol-5-yl}-6-(1H-1,2,4-triazol-1-yl)pyridine-2-carboxamide
N-{2-[2-(4-benzoylpipérazin-1-yl)-2-oxoéthyl]-2H-indazol-5-yl}-6-[3-(trifluorométhyl)-1H-pyrazol-4-yl]pyridine-2-carboxamide
N-{2-[2-(4-benzoylpipérazin-1-yl)-2-oxoéthyl]-2H-indazol-5-yl}-6-éthoxypyridine-2-carboxamide
N-{2-[2-(4-benzoylpipérazin-1-yl)-2-oxoéthyl]-2H-indazol-5-yl}-6-(cyclopropylméthoxy)pyridine-2-carboxamide
N-{2-[2-(4-benzoylpipérazin-1-yl)-2-oxoéthyl]-2H-indazol-5-yl}-6-éthylpyridine-2-carboxamide
N-{2-[2-(4-benzoylpipérazin-1-yl)-2-oxoéthyl]-2H-indazol-5-yl}-2-(4-méthoxyphényl)-1,3-thiazole-4-carboxamide
N-{2-[2-(4-benzoylpipérazin-1-yl)-2-oxoéthyl]-2H-indazol-5-yl}-2-bromo-1,3-thiazole-4-carboxamide
N-{2-[2-(4-benzoylpipérazin-1-yl)-2-oxoéthyl]-2H-indazol-5-yl}-2-(4-fluorophényl)-1,3-thiazole-4-carboxamide
N-{2-[2-(4-benzoylpipérazin-1-yl)-2-oxoéthyl]-2H-indazol-5-yl}-6-fluoropyridine-2-carboxamide
N-{2-[2-(4-benzoylpipérazin-1-yl)-2-oxoéthyl]-2H-indazol-5-yl}-6-bromopyridine-2-carboxamide
N-(2-{2-[4-(4-fluorobenzoyl)pipérazin-1-yl]-2-oxoéthyl}-2H-indazol-5-yl)-6-(trifluorométhyl)pyridine-2-carboxamide
N-(2-{2-oxo-2-[4-(pyridin-2-yl)pipérazin-1-yl]éthyl}-2H-indazol-5-yl)-6-(trifluorométhyl)pyridine-2-carboxamide
N-(2-{2-[4-(méthoxyacétyl)pipérazin-1-yl]-2-oxoéthyl}-2H-indazol-5-yl)-6-(trifluorométhyl)pyridine-2-carboxamide
N-{2-[2-(4-cyclopentyl-3-oxopipérazin-1-yl)-2-oxoéthyl]-2H-indazol-5-yl}-6-(trifluorométhyl)pyridine-2-carboxamide
N-{2-[2-oxo-2-(3-oxo-4-phénylpipérazin-1-yl)éthyl]-2H-indazol-5-yl}-6-(trifluorométhyl)pyridine-2-carboxamide
N-(2-{2-[4-(2,2-diméthylpropanoyl)pipérazin-1-yl]-2-oxoéthyl}-2H-indazol-5-yl)-6-(trifluorométhyl)pyridine-2-carboxamide
N-(2-{2-[4-(cyclopropylméthyl)pipérazin-1-yl]-2-oxoéthyl}-2H-indazol-5-yl)-6-(trifluorométhyl)pyridine-2-carboxamide
N-{2-[2-oxo-2-(pyridazin-4-ylamino)éthyl]-2H-indazol-5-yl}-6-(trifluorométhyl)pyridine-2-carboxamide
N-(2-{2-[4-(2-hydroxy-2-méthylpropanoyl)pipérazin-1-yl]-2-oxoéthyl}-2H-indazol-5-yl)-6-(trifluorométhyl)pyridine-2-carboxamide
N-(2-{2-oxo-2-[4-(1-phényléthyl)pipérazin-1-yl]éthyl}-2H-indazol-5-yl)-6-(trifluorométhyl)pyridine-2-carboxamide
N-(2-{2-oxo-2-[4-(pyridin-3-ylcarbonyl)pipérazin-1-yl]éthyl}-2H-indazol-5-yl)-6-(trifluorométhyl)pyridine-2-carboxamide
N-{2-[2-(4-isonicotinoylpipérazin-1-yl)-2-oxoéthyl]-2H-indazol-5-yl}-6-(trifluorométhyl)pyridine-2-carboxamide
N-(2-{2-[4-(morpholin-4-ylcarbonyl)pipérazin-1-yl]-2-oxoéthyl}-2H-indazol-5-yl)-6-(trifluorométhyl)pyridine-2-carboxamide
N-[2-(2-{4-[2-(méthylamino)-2-oxoéthyl]pipérazin-1-yl}-2-oxoéthyl)-2H-indazol-5-yl]-6-(trifluorométhyl)pyridine-2-carboxamide
N-(2-{2-oxo-2-[4-(pyrazin-2-yl)pipérazin-1-yl]éthyl}-2H-indazol-5-yl)-6-(trifluorométhyl)pyridin-2-carboxamide
N-(2-{2-[4-(1-hydroxyéthyl)pipéridin-1-yl]-2-oxoéthyl}-2H-indazol-5-yl)-6-(trifluorométhyl)pyridine-2-carboxamide
N-{2-[2-(2-méthyl-2,8-diazaspiro[4.5]déc-8-yl)-2-oxoéthyl]-2H-indazol-5-yl}-6-(trifluorométhyl)pyridine-2-carboxamide
N-{2-[2-(6-acétyl-2,6-diazaspiro[3.3]hept-2-yl)-2-oxoéthyl]-2H-indazol-5-yl}-6-(trifluorométhyl)pyridine-2-carboxamide
N-{2-[2-oxo-2-(3-oxo-2,8-diazaspiro[4.5]déc-8-yl)éthyl]-2H-indazol-5-yl}-6-(trifluorométhyl)pyridine-2-carboxamide
N-{2-[2-(6-méthyl-2,6-diazaspiro[3.5]non-2-yl)-2-oxoéthyl]-2H-indazol-5-yl}-6-(trifluorométhyl)pyridine-2-carboxamide
N-{2-[2-(7-oxa-2-azaspiro[3.5]non-2-yl)-2-oxoéthyl]-2H-indazol-5-yl}-6-(trifluorométhyl)pyridine-2-carboxamide
N-t2-[2-(1,4"-bipipéridin-1"-yl)-2-oxoéthyl]-2H-indazol-5-yl}-6-(trifluorométhyl)pyridine-2-carboxamide
N-(2-{2-[2-(hydroxyméthyl)pipéridin-1-yl]-2-oxoéthyl}-2H-indazol-5-yl)-6-(trifluorométhyl)pyridine-2-carboxamide
N-(2-{2-[3-(hydroxyméthyl)pipéridin-1-yl]-2-oxoéthyl}-2H-indazol-5-yl)-6-(trifluorométhyl)pyridine-2-carboxamide
N-{2-[2-(4-carbamoylpipéridin-1-yl)-2-oxoéthyl]-2H-indazol-5-yl}-6-(trifluorométhyl)pyridine-2-carboxamide
N-(2-{2-[3-(diméthylamino)pipéridin-1-yl]-2-oxoéthyl}-2H-indazol-5-yl)-6-(trifluorométhyl)pyridine-2-carboxamide
N-(2-{2-[3-(morpholin-4-ylméthyl)pipéridin-1-yl]-2-oxoéthyl}-2H-indazol-5-yl)-6-(trifluorométhyl)pyridine-2-carboxamide
N-[2-(2-{4-[(cyclopropylcarbonyl)amino]pipéridin-1-yl}-2-oxoéthyl)-2H-indazol-5-yl]-6-(trifluorométhyl)pyridine-2-carboxamide
N-(2-{2-[4-(3-éthyl-1,2,4-oxadiazol-5-yl)pipéridin-1-yl]-2-oxoéthyl}-2H-indazol-5-yl)-6-(trifluorométhyl)pyridine-2-carboxamide
N-[2-(2-{4-[(5-cyclopropyl-1,2,4-oxadiazol-3-yl)méthyl]pipéridin-1-yl}-2-oxoéthyl)-2H-indazol-5-yl]-6-(trifluorométhyl)pyridine-2-carboxamide
N-(2-{2-oxo-2-[4-(pyrrolidin-1-ylcarbonyl)pipéridin-1-yl]éthyl}-2H-indazol-5-yl)-6-(trifluorométhyl)pyridine-2-carboxamide
N-(2-{2-[4-(4-méthylpipérazin-1-yl)pipéridin-1-yl]-2-oxoéthyl}-2H-indazol-5-yl)-6-(trifluorométhyl)pyridine-2-carboxamide
N-[2-(2-{4-[2-(morpholin-4-yl)éthyl]pipéridin-1-yl}-2-oxoéthyl)-2H-indazol-5-yl]-6-(trifluorométhyl)pyridine-2-carboxamide
N-[2-(2-{4-[(5-méthyl-1,2,4-oxadiazol-3-yl)méthyl]pipéridin-1-yl}-2-oxoéthyl)-2H-indazol-5-yl]-6-(trifluorométhyl)pyridine-2-carboxamide
N-(2-{2-oxo-2-[3-(pyrrolidin-1-ylméthyl)pipéridin-1-yl]éthyl}-2H-indazol-5-yl)-6-(trifluorométhyl)pyridine-2-carboxamide
N-[2-(2-{[3-(diméthylsulfamoyl)phényl]amino}-2-oxoéthyl)-2H-indazol-5-yl]-6-(trifluorométhyl)pyridine-2-carboxamide
N-{2-[2-(1,2-oxazol-4-ylamino)-2-oxoéthyl]-2H-indazol-5-yl}-6-(trifluorométhyl)pyridine-2-carboxamide
N-(2-{2-[4-(méthylsulfonyl)pipéridin-1-yl]-2-oxoéthyl}-2H-indazol-5-yl)-6-(trifluorométhyl)pyridine-2-carboxamide
N-[2-(2-oxo-2-{4-[2-oxo-2-(pyrrolidin-1-yl)éthyl]pipérazin-1-yl}éthyl)-2H-indazol-5-yl]-6-(trifluorométhyl)pyridine-2-carboxamide
N-(2-{2-oxo-2-[4-(phénylsulfonyl)pipéridin-1-yl]éthyl}-2H-indazol-5-yl)-6-(trifluorométhyl)pyridine-2-carboxamide
N-(2-{2-oxo-2-[(3-sulfamoylphényl)amino]éthyl}-2H-indazol-5-yl)-6-(trifluorométhyl)pyridine-2-carboxamide
N-[2-(2-{4-[isonicotinoyl(méthyl)amino]pipéridin-1-yl}-2-oxoéthyl)-2H-indazol-5-yl]-6-(trifluorométhyl)pyridine-2-carboxamide
N-[2-(2-{4-[2-(isopropylamino)-2-oxoéthyl]pipérazin-1-yl}-2-oxoéthyl)-2H-indazol-5-yl]-6-(trifluorométhyl)pyridine-2-carboxamide
N-(2-{2-[4-(1,1-dioxydotétrahydrothiophén-3-yl)pipérazin-1-yl]-2-oxoéthyl}-2H-indazol-5-yl)-6-(trifluorométhyl)pyridine-2-carboxamide
N-[2-(2-{4-[(méthoxyacétyl)(méthyl)amino]pipéridin-1-yl}-2-oxoéthyl)-2H-indazol-5-yl]-6-(trifluorométhyl)pyridine-2-carboxamide
4-{[5-({[6-(trifluorométhyl)pyridin-2-yl]carbonyl}amino)-2H-indazol-2-yl]acétyl}pipérazine-1-carboxylate d'éthyle
N-(2-{2-[4-(cyclohexylcarbonyl)pipérazin-1-yl]-2-oxoéthyl}-2H-indazol-5-yl)-6-(trifluorométhyl)pyridine-2-carboxamide
N-[2-(2-{4-[2-(cyclopropylamino)-2-oxoéthyl]pipérazin-1-yl}-2-oxoéthyl)-2H-indazol-5-yl]-6-(trifluorométhyl)pyridine-2-carboxamide
N-(2-{2-[2-(2-hydroxyéthyl)pipéridin-1-yl]-2-oxoéthyl}-2H-indazol-5-yl)-6-(trifluorométhyl)pyridine-2-carboxamide
N-(2-{2-oxo-2-[4-(pyrrolidin-1-yl)pipéridin-1-yl]éthyl}-2H-indazol-5-yl)-6-(trifluorométhyl)pyridine-2-carboxamide
N-(2-{2-oxo-2-[4-(1H-pyrrol-1-yl)pipéridin-1-yl]éthyl}-2H-indazol-5-yl)-6-(trifluorométhyl)pyridine-2-carboxamide
N-(2-{2-[4-(3-hydroxypropyl)pipérazin-1-yl]-2-oxoéthyl}-2H-indazol-5-yl)-6-(trifluorométhyl)pyridine-2-carboxamide
4-{[5-({[6-(trifluorométhyl)pyridin-2-yl]carbonyl}amino)-2H-indazol-2-yl)acétyl}pipérazine-1-carboxamide
N-(2-{2-oxo-2-[4-(2-oxopyrrolidin-1-yl)pipéridin-1-yl]éthyl}-2H-indazol-5-yl)-6-(trifluorométhyl)pyridine-2-carboxamide
N-{2-[2-(morpholin-4-yl)-2-oxoéthyl]-2H-indazol-5-yl}-6-(trifluorométhyl)pyridine-2-carboxamide
N-(2-{2-[4-(2-amino-2-oxoéthyl)pipérazin-1-yl]-2-oxoéthyl}-2H-indazol-5-yl)-6-(trifluorométhyl)pyridine-2-carboxamide
N-{2-[2-(1,1-dioxydothiomorpholin-4-yl)-2-oxoéthyl]-2H-indazol-5-yl}-6-(trifluorométhyl)pyridine-2-carboxamide
N-{2-[2-(4-isopropylpipérazin-1-yl)-2-oxoéthyl]-2H-indazol-5-yl}-6-(trifluorométhyl)pyridine-2-carboxamide
N-(2-{2-oxo-2-[4-(2-thiénylcarbonyl)pipérazin-1-yl]éthyl}-2H-indazol-5-yl)-6-(trifluorométhyl)pyridine-2-carboxamide
N-(2-{2-[4-(2-cyclopropyl-2-oxoéthyl)pipérazin-1-yl]-2-oxoéthyl}-2H-indazol-5-yl)-6-(trifluorométhyl)pyridine-2-carboxamide
N-[2-(2-{4-[(1-méthyl-1H-pyrazol-4-yl)méthyl]pipérazin-1-yl}-2-oxoéthyl)-2H-indazol-5-yl]-6-(trifluorométhyl)pyridine-2-carboxamide
N-[2-(2-{4-[(1,5-diméthyl-1H-pyrazol-3-yl)carbonyl]pipérazin-1-yl}-2-oxoéthyl)-2H-indazol-5-yl]-6-(trifluorométhyl)pyridine-2-carboxamide
N,N-diéthyl-4-{[5-({[6-(trifluorométhyl)pyridin-2-yl]carbonyl}amino)-2H-indazol-2-yl]acétyl}pipérazine-1-carboxamide
N-{2-[2-oxo-2-(thiomorpholin-4-yl)éthyl]-2H-indazol-5-yl}-6-(trifluorométhyl)pyridine-2-carboxamide
N-(2-{2-[4-(2-furylméthyl)pipérazin-1-yl]-2-oxoéthyl}-2H-indazol-5-yl)-6-(trifluorométhyl)pyridine-2-carboxamide
N-(2-{2-oxo-2-[4-(3-thiénylméthyl)pipérazin-1-yl]éthyl}-2H-indazol-5-yl)-6-(trifluorométhyl)pyridine-2-carboxamide
N-{2-[2-(4'-méthyl-1,4'-bipipéridin-1'-yl)-2-oxoéthyl]-2H-indazol-5-yl}-6-(trifluorométhyl)pyridine-2-carboxamide
N-{2-[2-(6-méthyl-2,6-diazaspiro[3.3]hept-2-yl)-2-oxoéthyl]-2H-indazol-5-yl}-6-(trifluorométhyl)pyridine-2-carboxamide
N-{2-[2-(4-cyclopentylpipérazin-1-yl)-2-oxoéthyl]-2H-indazol-5-yl}-6-(trifluorométhyl)pyridine-2-carboxamide
N-[2-(2-t4-[2-(2-hydroxyéthoxy)éthyllpipérazin-1-yll-2-oxoéthyl)-2H-indazol-5-yl]-6-(trifluorométhyl)pyridine-2-carboxamide
N-(2-{2-oxo-2-[4-(pyridin-4-ylméthyl)pipérazin-1-yl]éthyl}-2H-indazol-5-yl)-6-(trifluorométhyl)pyridine-2-carboxamide
N-(2-{2-[4-(diméthylsulfamoyl)pipérazin-1-yl]-2-oxoéthyl}-2H-indazol-5-yl)-6-(trifluorométhyl)pyridine-2-carboxamide
N-(2-{2-oxo-2-[4-(pyridin-4-yl)pipérazin-1-yl]éthyl}-2H-indazol-5-yl)-6-(trifluorométhyl)pyridine-2-carboxamide
N-(2-{2-[4-(méthylsulfonyl)pipérazin-1-yl]-2-oxoéthyl}-2H-indazol-5-yl)-6-(trifluorométhyl)pyridine-2-carboxamide
N-[2-(2-{4-[2-(1H-imidazol-1-yl)éthyl]pipérazin-1-yl}-2-oxoéthyl)-2H-indazol-5-yl]-6-(trifluorométhyl)pyridine-2-carboxamide de l'acide formique (1:1)
N-(2-{2-[4-(diéthylsulfamoyl)pipérazin-1-yl]-2-oxoéthyl}-2H-indazol-5-yl)-6-(trifluorométhyl)pyridine-2-carboxamide
N-(2-{2-oxo-2-[4-(pyridin-3-yl)pipérazin-1-yl]éthyl}-2H-indazol-5-yl)-6-(trifluorométhyl)pyridine-2-carboxamide
N-(2-{2-oxo-2-[4-(pipéridin-1-ylsulfonyl)pipérazin-1-yl]éthyl}-2H-indazol-5-yl)-6-(trifluorométhyl)pyridine-2-carboxamide
N-[2-(2-{4-[(1,5-diméthyl-1H-pyrazol-4-yl)sulfonyl]pipérazin-1-yl}-2-oxoéthyl)-2H-indazol-5-yl]-6-(trifluorométhyl)pyridine-2-carboxamide
N-(2-{2-[4-(cyclopropylméthyl)pipérazin-1-yl]-2-oxoéthyl}-2H-indazol-5-yl)-6-(1-méthyl-1H-pyrazol-4-yl)pyridine-2-carboxamide
N-(2-{2-[4-(2-hydroxypropan-2-yl)pipéridin-1-yl]-2-oxoéthyl}-2H-indazol-5-yl)-6-(1-méthyl-1H-pyrazol-4-yl)pyridine-2-carboxamide
6-(1-méthyl-1H-pyrazol-4-yl)-N-(2-{2-oxo-2-[4-(pyrrolidin-1-yl)pipéridin-1-yl]éthyl}-2H-indazol-5-yl)pyridine-2-carboxamide
N-{2-[2-(4-éthylpipérazin-1-yl)-2-oxoéthyl]-2H-indazol-5-yl}-6-(1-méthyl-1H-pyrazol-4-yl)pyridine-2-carboxamide
N-(2-{2-[4-(diméthylamino)pipéridin-1-yl]-2-oxoéthyl}-2H-indazol-5-yl)-6-(1-méthyl-1H-pyrazol-4-yl)pyridine-2-carboxamide
N-(2-{2-[(cyclopropylméthyl) (méthyl)amino]-2-oxoéthyl}-6-méthoxy-2H-indazol-5-yl)-6-méthylpyridine-2-carboxamide
N-(2-{2-[(cyclopropylméthyl) (méthyl)amino]-2-oxoéthyl}-6-éthoxy-2H-indazol-5-yl)-6-(trifluorométhyl)pyridine-2-carboxamide
N-(2-{2-[(cyclopropylméthyl(méthyl)amino]-2-oxoéthyl}-6-méthoxy-2H-indazol-5-yl)-6-(trifluorométhyl)pyridine-2-carboxamide
6-cyclopropyl-N-(2-{2-[4-(2-hydroxypropan-2-yl)pipéridin-1-yl]-2-oxoéthyl}-6-méthoxy-2H-indazol-5-yl)pyridine-2-carboxamide
6-(1-hydroxyéthyl)-N-(2-{2-[4-(2-hydroxypropan-2-yl)pipéridin-1-yl]-2-oxoéthyl}-6-méthoxy-2H-indazol-5-yl)pyridine-2-carboxamide
6-(azétidin-3-ylamino)-N-{2-[2-(4-benzoylpipérazin-1-yl)-2-oxoéthyl]-2H-indazol-5-yl}pyridine-2-carboxamide
6-[(azétidin-2-ylméthyl)amino]-N-{2-[2-(4-benzoylpipérazin-1-yl)-2-oxoéthyl]-2H-indazol-5-yl}pyridine-2-carboxamide
N-{2-[2-(4-benzoylpipérazin-1-yl)-2-oxoéthyl]-2H-indazol-5-yl}-6-(3-hydroxyazétidin-1-yl)pyridine-2-carboxamide
6-[(2R,6S)-2,6-diméthylmorpholin-4-yl]-N-(6-méthyl-2-{2-oxo-2-[4-(pyrrolidin-1-yl)pipéridin-1-yl]éthyl}-2H-indazol-5-yl)pyridine-2-carboxamide
N-[2-(2-{4-méthyl-4-[(4-méthylpipérazin-1-yl)carbonyl]pipéridin-1-yl}-2-oxoéthyl)-2H-indazol-5-yl]-6-(trifluorométhyl)pyridine-2-carboxamide
N-(6-chloro-2-{2-oxo-2-[(3R)-pipéridin-3-ylamino]éthyl}-2H-indazol-5-yl)-6-(trifluorométhyl)pyridine-2-carboxamide
N-(2-{2-[4-(cyclopropylcarbonyl)pipérazin-1-yl]-2-oxoéthyl}-6-isopropoxy-2H-indazol-5-yl)-6-méthylpyridine-2-carboxamide
N-(2-{2-[4-(cyclopropylcarbonyl)pipérazin-l-yl]-2-oxoéthyl}-6-isopropoxy-2H-indazol-5-yl)-6-(trifluorométhyl)pyridine-2-carboxamide
N-{2-[2-(4-benzoylpipérazin-1-yl)-2-oxoéthyl]-6-isopropoxy-2H-indazol-5-yl}-6-méthylpyridine-2-carboxamide
N-{2-[2-(4-benzoylpipérazin-1-yl)-2-oxoéthyl]-6-isopropoxy-2H-indazol-5-yl}-6-(trifluorométhyl)pyridine-2-carboxamide
N-{6-isopropoxy-2-[2-(4-méthylpipérazin-1-yl)-2-oxoéthyl]-2H-indazol-5-yl}-6-(trifluorométhyl)pyridine-2-carboxamide
N-{6-isopropoxy-2-[2-(4-méthylpipérazin-1-yl)-2-oxoéthyl]-2H-indazol-5-yl}-6-méthylpyridine-2-carboxamide
N-(2-{2-[4-(cyclobutylcarbonyl)pipérazin-1-yl]-2-oxoéthyl}-2H-indazol-5-yl)-6-(trifluorométhyl)pyridine-2-carboxamide
N-(2-{2-[4-(cyclopentylcarbonyl)pipérazin-1-yl]-2-oxoéthyl}-2H-indazol-5-yl)-6-(trifluorométhyl)pyridine-2-carboxamide
N-[2-(2-{4-[3-(méthylsulfonyl)benzoyl]pipérazin-1-yl}-2-oxoéthyl)-2H-indazol-5-yl]-6-(trifluorométhyl)pyridine-2-carboxamide
N-[2-(2-{4-[2-méthoxy-5-(méthylsulfonyl)benzoyl]pipérazin-1-yl}-2-oxoéthyl)-2H-indazol-5-yl]-6-(trifluorométhyl)pyridine-2-carboxamide
6-bromo-N-(6-méthyl-2-{2-oxo-2-[4-(pyrrolidin-1-yl)pipéridin-1-yl]éthyl}-2H-indazol-5-yl)pyridine-2-carboxamide
2-(4-méthoxyphényl)-N-(6-méthyl-2-{2-oxo-2-[4-(pyrrolidin-1-yl)pipéridin-1-yl]éthyl}-2H-indazol-5-yl)-1,3-thiazole-4-carboxamide
2-(4-fluorophényl)-N-(6-méthyl-2-{2-oxo-2-[4-(pyrrolidin-1-yl)pipéridin-1-yl]éthyl}-2H-indazol-5-yl)-1,3-thiazole-4-carboxamide
N-(6-méthyl-2-{2-oxo-2-[4-(pyrrolidin-1-yl)pipéridin-1-yl]éthyl}-2H-indazol-5-yl)-6-(trifluorométhyl)pyridine-2-carboxamide
6-bromo-N-{2-[2-(4-méthylpipérazin-1-yl)-2-oxoéthyl]-2H-indazol-5-yl}pyridine-2-carboxamide
6-bromo-N-{2-[2-(4-méthylpipérazin-1-yl)-2-oxoéthyl]-6-(trifluorométhoxy)-2H-indazol-5-yl}pyridine-2-carboxamide
N-{2-[2-(4-méthylpipérazin-1-yl)-2-oxoéthyl]-6-(trifluorométhoxy)-2H-indazol-5-yl}-6-(4H-1,2,4-triazol-4-yl)pyridine-2-carboxamide
2-bromo-N-{6-bromo-2-[2-(4-méthylpipérazin-1-yl)-2-oxoéthyl]-2H-indazol-5-yl}-1,3-thiazole-4-carboxamide
N-{6-hydroxy-2-[2-(4-méthylpipérazin-1-yl)-2-oxoéthyl]-2H-indazol-5-yl}-6-(trifluorométhyl)pyridine-2-carboxamide
N-[6-(benzyloxy)-2-{2-[4-(2-hydroxypropan-2-yl)pipéridin-1-yl]-2-oxoéthyl}-2H-indazol-5-yl]-6-méthylpyridine-2-carboxamide
6-bromo-N-{6-bromo-2-[2-(4-méthylpipérazin-1-yl)-2-oxoéthyl]-2H-indazol-5-yl}pyridine-2-carboxamide
N-{6-(benzyloxy)-2-[2-(4-méthylpipérazin-1-yl)-2-oxoéthyl]-2H-indazol-5-yl}-6-méthylpyridine-2-carboxamide
2-(azétidin-3-ylamino)-N-{2-[2-(4-benzoylpipérazin-1-yl)-2-oxoéthyl]-2H-indazol-5-yl}-1,3-thiazole-4-carboxamide
6-acétamideo-N-{6-méthoxy-2-[2-(morpholin-4-yl)-2-oxoéthyl]-2H-indazol-5-yl}pyridine-2-carboxamide
6-(diméthylamino)-N-(2-{2-[4-(2-hydroxypropan-2-yl)pipéridin-1-yl]-2-oxoéthyl}-6-méthoxy-2H-indazol-5-yl)pyridine-2-carboxamide
6-(diméthylamino)-N-{6-méthoxy-2-[2-(morpholin-4-yl)-2-oxoéthyl]-2H-indazol-5-yl}pyridine-2-carboxamide
6-acétamido-N-(2-{2-[4-(2-hydroxypropan-2-yl)pipéridin-1-yl]-2-oxoéthyl}-6-méthoxy-2H-indazol-5-yl)pyridine-2-carboxamide
6-(diméthylamino)-N-{6-méthoxy-2-[2-(4-méthylpipérazin-1-yl)-2-oxoéthyl]-2H-indazol-5-yl}pyridine-2-carboxamide
N-{2-[2-(4-méthylpipérazin-1-yl)-2-oxoéthyl]-2H-indazol-5-yl}-6-[3-(méthylsulfonyl)phényl]pyridine-2-carboxamide
N-{2-[1-(4-benzoylpipérazin-1-yl)-1-oxopropan-2-yl]-2H-indazol-5-yl}-6-(trifluorométhyl)pyridine-2-carboxamide
N-[6-chloro-2-(2-{[trans-4-(2-hydroxypropan-2-yl)cyclohexyl]amino}-2-oxoéthyl)-2H-indazol-5-yl]-6-(trifluorométhyl)pyridine-2-carboxamide
6-(2-hydroxypropan-2-yl)-N-{6-méthoxy-2-[2-(morpholin-4-yl)-2-oxoéthyl]-2H-indazol-5-yl}pyridine-2-carboxamide
N-{6-chloro-2-[2-(3,3-difluoropyrrolidin-1-yl)-2-oxoéthyl]-2H-indazol-5-yl}-6-(trifluorométhyl)pyridine-2-carboxamide
N-{6-chloro-2-[2-oxo-2-(pyrrolidin-1-yl)éthyl]-2H-indazol-5-yl}-6-(trifluorométhyl)pyridine-2-carboxamide
N-{6-chloro-2-[2-(2-oxa-7-azaspiro[3.5]non-7-yl)-2-oxoéthyl]-2H-indazol-5-yl}-6-(trifluorométhyl)pyridine-2-carboxamide
N-(6-chloro-2-{2-[4-(2-hydroxy-2-méthylpropyl)pipérazin-1-yl]-2-oxoéthyl}-2H-indazol-5-yl)-6-(trifluorométhyl)pyridine-2-carboxamide
N-{6-méthoxy-2-[2-oxo-2-(pyrrolidin-1-yl)éthyl]-2H-indazol-5-yl}-6-(trifluorométhyl)pyridine-2-carboxamide
N-{2-[2-(3,3-difluoropyrrolidin-1-yl)-2-oxoéthyl]-6-méthoxy-2H-indazol-5-yl}-6-(trifluorométhyl)pyridine-2-carboxamide
6-(difluorométhyl)-N-{6-méthoxy-2-[2-(morpholin-4-yl)-2-oxoéthyl]-2H-indazol-5-yl}pyridine-2-carboxamide
N-{2-[2-(3,3-difluoropyrrolidin-1-yl)-2-oxoéthyl]-6-méthoxy-2H-indazol-5-yl}-6-méthylpyridine-2-carboxamide
N-{6-méthoxy-2-[2-(morpholin-4-yl)-2-oxoéthyl]-2H-indazol-5-yl}-6-méthylpyridine-2-carboxamide
N-{6-méthoxy-2-[2-(morpholin-4-yl)-2-oxoéthyl]-2H-indazol-5-yl}-2-(tétrahydro-2H-pyran-4-yl)-1,3-oxazole-4-carboxamide
N-{2-[2-(1,1-dioxydo-1-thia-6-azaspiro[3.3]hept-6-yl)-2-oxoéthyl]-6-méthoxy-2H-indazol-5-yl}-6-(trifluorométhyl)pyridine-2-carboxamide
N-{6-méthoxy-2-[2-(2-oxa-6-azaspiro[3.3]hept-6-yl)-2-oxoéthyl]-2H-indazol-5-yl}-6-(trifluorométhyl)pyridine-2-carboxamide
N-{6-(3-hydroxy-2,2-diméthylpropoxy)-2-[2-(morpholin-4-yl)-2-oxoéthyl]-2H-indazol-5-yl}-6-(trifluorométhyl)pyridine-2-carboxamide
6-éthyl-N-{6-méthoxy-2-[2-(morpholin-4-yl)-2-oxoéthyl]-2H-indazol-5-yl}pyridine-2-carboxamide
6-isobutyl-N-{6-méthoxy-2-[2-(morpholin-4-yl)-2-oxoéthyl]-2H-indazol-5-yl}pyridine-2-carboxamide
2-[2-(morpholin-4-yl)-2-oxoéthyl]-5-({[6-(trifluorométhyl)pyridin-2-yl]carbonyl}amino)-2H-indazole-6-carboxylate de méthyle
5-{[(6-méthylpyridin-2-yl)carbonyl]amino}-2-[2-(morpholin-4-yl)-2-oxoéthyl]-2H-indazole-6-carboxylate de méthyle
N-{6-méthoxy-2-[2-(morpholin-4-yl)-2-oxoéthyl]-2H-indazol-5-yl}-6-(pyrrolidin-1-yl)pyridine-2-carboxamide
N-{6-méthoxy-2-[2-(morpholin-4-yl)-2-oxoéthyl]-2H-indazol-5-yl}-6-(morpholin-4-yl)pyridine-2-carboxamide
6-(cyclopropylamino)-N-{6-méthoxy-2-[2-(morpholin-4-yl)-2-oxoéthyl]-2H-indazol-5-yl}pyridine-2-carboxamide
6-(butylamino)-N-{6-méthoxy-2-[2-(morpholin-4-yl)-2-oxoéthyl]-2H-indazol-5-yl}pyridine-2-carboxamide
N-{6-méthoxy-2-[2-(morpholin-4-yl)-2-oxoéthyl]-2H-indazol-5-yl}-6-(propylamino)pyridine-2-carboxamide
6-(isobutylamino)-N-{6-méthoxy-2-[2-(morpholin-4-yl)-2-oxoéthyl]-2H-indazol-5-yl}pyridine-2-carboxamide
R-N-{6-méthoxy-2-[2-(morpholin-4-yl)-2-oxoéthyl]-2H-indazol-5-yl}-6-(2,2,2-trifluoro-1-hydroxyéthyl)pyridine-2-carboxamide
S-N-{6-méthoxy-2-[2-(morpholin-4-yl)-2-oxoéthyl]-2H-indazol-5-yl}-6-(2,2,2-trifluoro-1-hydroxyéthyl)pyridine-2-carboxamide
6-(1-hydroxyéthyl)-N-{6-méthoxy-2-[2-(morpholin-4-yl)-2-oxoéthyl]-2H-indazol-5-yl}pyridine-2-carboxamide
6-(cyclopropylamino)-N-{6-méthoxy-2-[2-(4-méthylpipérazin-1-yl)-2-oxoéthyl]-2H-indazol-5-yl}pyridine-2-carboxamide
N-{6-méthoxy-2-[2-(4-méthylpipérazin-1-yl)-2-oxoéthyl]-2H-indazol-5-yl}-6-(propylamino)pyridine-2-carboxamide
6-(isobutylamino)-N-{6-méthoxy-2-[2-(4-méthylpipérazin-1-yl)-2-oxoéthyl]-2H-indazol-5-yl}pyridine-2-carboxamide
6-(1-hydroxyéthyl)-N-{6-méthoxy-2-[2-(4-méthylpipérazin-1-yl)-2-oxoéthyl]-2H-indazol-5-yl}pyridine-2-carboxamide
N-{6-méthoxy-2-[2-(4-méthylpipérazin-1-yl)-2-oxoéthyl]-2H-indazol-5-yl}-4-méthyl-6-(trifluorométhyl)pyridine-2-carboxamide
N-{6-(benzyloxy)-2-[2-(morpholin-4-yl)-2-oxoéthyl]-2H-indazol-5-yl}-6-(trifluorométhyl)pyridine-2-carboxamide
6-(cyclopropylamino)-N-(2-{2-[4-(2-hydroxypropan-2-yl)pipéridin-1-yl]-2-oxoéthyl}-6-méthoxy-2H-indazol-5-yl)pyridine-2-carboxamide
6-(butylamino)-N-(2-{2-[4-(2-hydroxypropan-2-yl)pipéridin-1-yl]-2-oxoéthyl}-6-méthoxy-2H-indazol-5-yl)pyridine-2-carboxamide
N-(2-{2-[4-(2-hydroxypropan-2-yl)pipéridin-1-yl]-2-oxoéthyl}-6-méthoxy-2H-indazol-5-yl)-6-[(2-méthoxyéthyl)amino]pyridine-2-carboxamide
N-(2-{2-[4-(2-hydroxypropan-2-yl)pipéridin-1-yl]-2-oxoéthyl}-6-méthoxy-2H-indazol-5-yl)-6-(propylamino)pyridine-2-carboxamide
N-(2-{2-[4-(2-hydroxypropan-2-yl)pipéridin-1-yl]-2-oxoéthyl}-6-méthoxy-2H-indazol-5-yl)-6-(isobutylamino)pyridine-2-carboxamide
5-fluoro-N-(2-{2-[4-(2-hydroxypropan-2-yl)pipéridin-1-yl]-2-oxoéthyl}-6-méthoxy-2H-indazol-5-yl)-6-méthylpyridine-2-carboxamide
N-{6-hydroxy-2-[2-(morpholin-4-yl)-2-oxoéthyl]-2H-indazol-5-yl}-6-(trifluorométhyl)pyridine-2-carboxamide
N-{6-(3-cyanopropoxy)-2-[2-(morpholin-4-yl)-2-oxoéthyl]-2H-indazol-5-yl}-6-(trifluorométhyl)pyridine-2-carboxamide
N-{2-[2-(morpholin-4-yl)-2-oxoéthyl]-6-(2,2,2-trifluoroéthoxy)-2H-indazol-5-yl}-6-(trifluorométhyl)pyridine-2-carboxamide
N-{6-(cyclohexylméthoxy)-2-[2-(morpholin-4-yl)-2-oxoéthyl]-2H-indazol-5-yl}-6-(trifluorométhyl)pyridine-2-carboxamide
N-{6-(2,2-diméthylpropoxy)-2-[2-(morpholin-4-yl)-2-oxoéthyl]-2H-indazol-5-yl}-6-(trifluorométhyl)pyridine-2-carboxamide
N-{2-[2-(morpholin-4-yl)-2-oxoéthyl]-6-(tétrahydrofuran-2-ylméthoxy)-2H-indazol-5-yl}-6-(trifluorométhyl)pyridine-2-carboxamide
N-{6-(cyclopentyloxy)-2-[2-(morpholin-4-yl)-2-oxoéthyl]-2H-indazol-5-yl}-6-(trifluorométhyl)pyridine-2-carboxamide
N-{6-(cyanométhoxy)-2-[2-(morpholin-4-yl)-2-oxoéthyl]-2H-indazol-5-yl}-6-(trifluorométhyl)pyridine-2-carboxamide
acide ({2-[2-(morpholin-4-yl)-2-oxoéthyl]-5-({[6-(trifluorométhyl)pyridin-2-yl]carbonyl}amino)-2H-indazol-6-yl}oxy) acétique
N-{6-(cyclobutylméthoxy)-2-[2-(morpholin-4-yl)-2-oxoéthyl]-2H-indazol-5-yl}-6-(trifluorométhyl)pyridine-2-carboxamide
N-{2-[2-(morpholin-4-yl)-2-oxoéthyl]-6-[2-(pyrrolidin-1-yl)éthoxy]-2H-indazol-5-yl}-6-(trifluorométhyl)pyridine-2-carboxamide
N-{6-[2-(morpholin-4-yl)éthoxy]-2-[2-(morpholin-4-yl)-2-oxoéthyl]-2H-indazol-5-yl}-6-(trifluorométhyl)pyridine-2-carboxamide
N-{2-[2-(morpholin-4-yl)-2-oxoéthyl]-6-[2-(pipéridin-1-yl)éthoxy]-2H-indazol-5-yl}-6-(trifluorométhyl)pyridine-2-carboxamide
N-{6-(3-hydroxypropoxy)-2-[2-(morpholin-4-yl)-2-oxoéthyl]-2H-indazol-5-yl}-6-(trifluorométhyl)pyridine-2-carboxamide
N-{6-(2-hydroxypropoxy)-2-[2-(morpholin-4-yl)-2-oxoéthyl]-2H-indazol-5-yl}-6-(trifluorométhyl)pyridine-2-carboxamide
N-{6-(2-hydroxyéthoxy)-2-[2-(morpholin-4-yl)-2-oxoéthyl]-2H-indazol-5-yl}-6-(trifluorométhyl)pyridine-2-carboxamide
N-{6-(2-méthoxyéthoxy)-2-[2-(morpholin-4-yl)-2-oxoéthyl]-2H-indazol-5-yl}-6-(trifluorométhyl)pyridine-2-carboxamide
({2-[2-(morpholin-4-yl)-2-oxoéthyl]-5-({[6-(trifluorométhyl)pyridin-2-yl]carbonyl}amino)-2H-indazol-6-yl}oxy)acétate d'éthyle
4-({2-[2-(morpholin-4-yl)-2-oxoéthyl]-5-({[6-(trifluorométhyl)pyridin-2-yl]carbonyl}amino)-2H-indazol-6-yl}oxy)butanoate de méthyle
2-({2-[2-(morpholin-4-yl)-2-oxoéthyl]-5-({[6-(trifluorométhyl)pyridin-2-yl]carbonyl}amino)-2H-indazol-6-yl}oxy)propanoate d'éthyle
3-méthyl-2-({2-[2-(morpholin-4-yl)-2-oxoéthyl]-5-({[6-(trifluorométhyl)pyridin-2-yl]carbonyl}amino)-2H-indazol-6-yl}oxy)butanoate d'éthyle
acide 2-({2-[2-(morpholin-4-yl)-2-oxoéthyl]-5-({[6-(trifluorométhyl)pyridin-2-yl]carbonyl}amino)-2H-indazol-6-yl}oxy)propanoïque
N-{6-(2-hydroxypropan-2-yl)-2-[2-(morpholin-4-yl)-2-oxoéthyl]-2H-indazol-5-yl}-6-(trifluorométhyl)pyridine-2-carboxamide
N-{6-chloro-2-[2-(4-méthylpipérazin-1-yl)-2-oxoéthyl]-2H-indazol-5-yl}-6-(difluorométhyl)pyridine-2-carboxamide
N-{6-chloro-2-[2-(morpholin-4-yl)-2-oxoéthyl]-2H-indazol-5-yl}-6-(difluorométhyl)pyridine-2-carboxamide.

11. Composé de formule (I), tel que défini dans l'une quelconque des revendications 1 à 10, pour le traitement et/ou la prophylaxie de maladies.

12. Composé de formule (I), tel que défini dans l'une quelconque des revendications 1 à 10, destiné à une utilisation dans un procédé de traitement et/ou de prophylaxie de maladies tumorales, de maladies dermatologiques, de maladies gynécologiques, de maladies cardiovasculaires, de maladies pulmonaires, de maladies ophtalmologiques, de maladies neurologiques, de maladies métaboliques, de maladies inflammatoires, de maladies autoimmunes et de la douleur.

13. Composé de formule (I), tel que défini dans l'une quelconque des revendications 1 à 10, destiné à une utilisation dans un procédé de traitement et/ou de prophylaxie de lymphomes, de la dégénération maculaire, de l'endométriose, du psoriasis, du lupus érythémateux, de la sclérose en plaques, de la BPCO, de la polyarthrite rhumatoïde.

14. Utilisation d'un composé de formule (I), tel que défini dans l'une quelconque des revendications 1 à 10, pour la fabrication d'un médicament pour le traitement et/ou la prophylaxie de maladies tumorales, de maladies dermatologiques, de maladies gynécologiques, de maladies cardiovasculaires, de maladies pulmonaires, de maladies ophtalmologiques, de maladies neurologiques, de maladies métaboliques, de maladies inflammatoires, de maladies autoimmunes et de la douleur.

15. Composé de formule (I), tel que défini dans l'une quelconque des revendications 1 à 10, pour la fabrication d'un médicament pour le traitement et/ou la prophylaxie de lymphomes, de la dégénération maculaire, de l'endométriose, du psoriasis, du lupus érythémateux, de la sclérose en plaques, de la BPCO, de la polyarthrite rhumatoïde.

16. Médicament, contenant un composé de formule (I), tel que défini dans l'une quelconque des revendications 1 à 10, en combinaison avec un adjuvant inerte, non toxique, pharmaceutiquement approprié.

17. Procédé de fabrication de composés de formule générale (III) à partir du composé de formule générale (II) 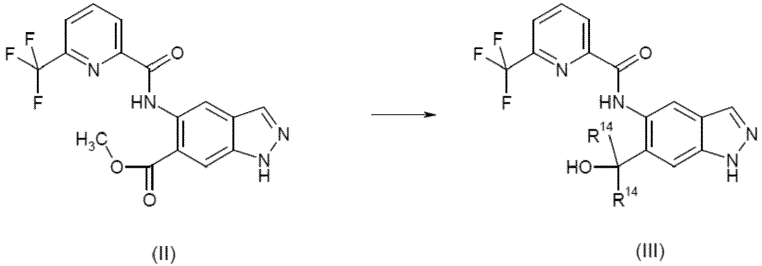 R¹⁴ étant un radical méthyle ou éthyle, par réaction de Grignard avec du bromure de méthyl- ou d'éthylmagnésium.

18. Composés de formule générale (III) 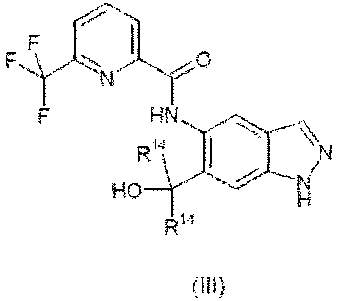 R¹⁴ étant un radical méthyle ou éthyle.
